# EUROPEAN PATENT SPECIFICATION

(11) **EP 3 169 149 B1**
(45) Date of publication and mention of the grant of the patent: **17.01.2024**
(21) Application number: 15738907.3
(22) Date of filing: 16.07.2015
(51) Int. Cl.: C12N 9/10, C12N 9/48, C12N 9/50, C12N 15/82, A24B 3/12

(54) **TOBACCO PROTEASE GENES**
TABAKPROTEASEGENE
GÈNES DE LA PROTÉASE DU TABAC

(30) Priority: 18.07.2014 EP 14177715
(43) Date of publication of application: 24.05.2017
(73) Proprietor: Philip Morris Products S.A., 2000 Neuchâtel (CH)
(72) Inventor: BOVET, Lucien, 2300 La-chaux-de-Fon (CH); FLORACK, Dion, 2525 Le Landeron (CH); BATTEY, James, 2000 Neuchatel (CH)
(74) Representative: Script IP Limited
(86) International application number: PCT/EP2015/066341
(87) International publication number: WO 2016/009006

(56) References cited:
- US-A1- 2004 106 198
- CATHERINE NAVARRE ET AL: "Identification, gene cloning and expression of serine proteases in the extracellular medium ofcells", PLANT CELL REPORTS, SPRINGER, BERLIN, DE, vol. 31, no. 10, 17 July 2012 (2012-07-17) , pages 1959-1968, XP035112133, ISSN: 1432-203X, DOI: 10.1007/S00299-012-1308-Y
- G. BEYENE: "Two new cysteine proteinases with specific expression patterns in mature and senescent tobacco (Nicotiana tabacum L.) leaves", JOURNAL OF EXPERIMENTAL BOTANY, vol. 57, no. 6, 1 March 2006 (2006-03-01), pages 1431-1443, XP55220617, GB ISSN: 0022-0957, DOI: 10.1093/jxb/erj123
- Bovet, L. et al.: "Gene expression changes during tobacco curing", , 2013, XP002746297, Retrieved from the Internet: URL:https://www.pmiscience.com/system/file s/asset/files/2013_bovet_l_swissplant13_po ster.pdf [retrieved on 2015-10-15]
- KAWASHIMA N ET AL: "STUDIES ON PROTEIN METABOLISM IN HIGHER PLANTS V SOME PROPERTIES OF A TOBACCO-D LEAF ENZ PROTEASE INCREASED DURING CURING INST COLUMN CHROMATOGRAPHY", AGRICULTURAL AND BIOLOGICAL CHEMISTRY, vol. 32, no. 9, 1968, pages 1141-1145, XP002746298, ISSN: 0002-1369
- FLORIAN MARTIN ET AL: "Design of a tobacco exon array with application to investigate the differential cadmium accumulation property in two tobacco varieties", BMC GENOMICS, BIOMED CENTRAL LTD, LONDON, UK, vol. 13, no. 1, 28 November 2012 (2012-11-28), page 674, XP021140791, ISSN: 1471-2164, DOI: 10.1186/1471-2164-13-674 cited in the application

## Description

### FIELD OF THE INVENTION

The present invention concerns tobacco plant and plant cells comprising proteases which can alter the characteristics of cured tobacco products. In particular, the invention provides method for preparing tobacco plants with modulated expression of one or more tobacco protease genes.

### BACKGROUND OF THE INVENTION

Tobacco curing is a process of physical and biochemical changes that bring out the aroma and flavor of each variety of tobacco. After tobacco has been harvested, it is necessary to cure it and then age it before consumption, to improve its flavour. There are four common methods of curing, and the method used depends on the type of tobacco and its intended use. Air-cured tobacco is sheltered from wind and sun in a well-ventilated chamber, where it air-dries for six to eight weeks. Air-cured tobacco is low in sugar, which gives the tobacco smoke a light, sweet flavor, and high in nicotine. Cigar and burley tobaccos are air cured.

In fire curing, smoke from a low-burning fire permeates the leaves. This gives the leaves a distinctive smokey aroma and flavour. Fire curing takes three to ten weeks and produces a tobacco low in sugar and high in nicotine. Pipe tobacco, chewing tobacco, and snuff are fire cured.

Flue-cured tobacco is kept in an enclosed heated area, but it is not directly exposed to smoke. This method produces cigarette tobacco that is high in sugar and has medium to high levels of nicotine. It is the fastest method of curing, requiring about a week. Virginia tobacco that has been flue cured is also called bright tobacco, because flue curing turns its leaves gold, orange, or yellow.

Sun-cured tobacco dries uncovered in the sun. This method is used in Turkey, Greece and other Mediterranean countries to produce oriental tobacco. Sun-cured tobacco is low in sugar and nicotine and is used in cigarettes.

Curing produces various compounds in the tobacco leaves that give cured tobacco its specific flavour and taste, such as for example a sweet hay, tea, rose oil, or fruity aromatic flavor.

During the first phase of curing, corresponding to the so-called yellowing phase and also known as color curing, the chlorophyll content is reduced. This phase takes between 2 and 8 days depending on the tobacco type. During this phase leaf metabolic activities are drastically changed. Not only is chlorophyll degraded but also, for example, starch and proteins.

To date, the only methods for altering the curing process which have been proposed are base on altering the actual conditions to which the tobacco is exposed in the chosen curing procedure. Very little is known about gene expression in tobacco during curing, and moreover few data have been reported on the activities of proteases in tobacco leaf and their resulting products.

We have identified 80 protease genes that are activated during leaf curing in the three main tobacco types, Burley, Virginia and Oriental. We have found that specific protease expression is associated with particular flavour profiles in tobacco.

### SUMMARY OF THE INVENTION

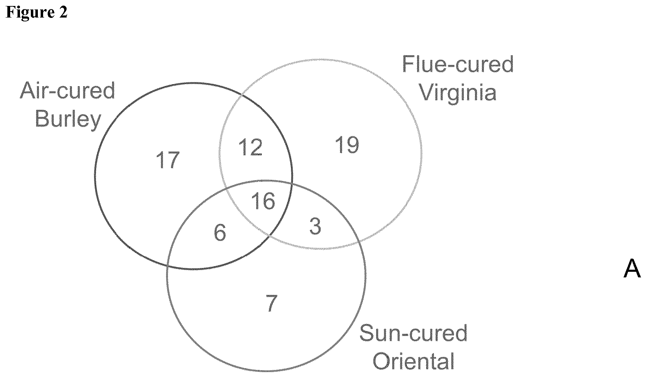

The present invention is defined in the appended claims.
80 protease genes (SEQ ID NO: 1-80) were identified that are up-regulated in Burley tobacco upon air curing, Virginia tobacco upon flue-curing and Oriental tobacco upon sun curing. Details on such up-regulation in one or more of the different tobacco types are summarised in Figure 2 and Table 1 & 2.

Such gene sequences and their regulatory sequences can be used to modulate or modify protease activity during curing. The polynucleotide sequences SEQ ID NO: 1-80 include exon and intron sequences. The protein sequences relating to the coding sequence part of the polynucleotide sequences SEQ ID NO: 1-80, are depicted in SEQ ID NO: 81-160.

Accordingly, there is provided a mutant tobacco plant cell or a transgenic tobacco plant cell comprising:
(i) a polynucleotide comprising, consisting or consisting essentially of a sequence encoding a functional protease and having at least 96% sequence identity to a protease selected from:
   at least one of SEQ ID NO: 1 to 16; or
   at least one of SEQ ID NO: 30 to 41; or
   at least one of SEQ ID NO: 17 to 22; or
   at least one of SEQ ID NO: 42 to 44; or
   at least one of SEQ ID NO: 45 to 61; or
   at least one of SEQ ID NO: 23 to 29;
   or
(ii) (ii) a construct, vector or expression vector comprising the isolated polynucleotide set forth in (i),
and wherein the mutagenesis or transgenesis results in the insertion of one or more mutations into the polynucleotide set forth in (i) and/or into a regulatory region thereof, to create a mutant tobacco plant wherein expression or activity of said protease is up-regulated or downregulated as compared to a control tobacco plant cell, wherein the control tobacco plant cell has the same genotype as the starting material for the genetic alteration that resulted in the mutant tobacco plant cell or the transgenic tobacco plant cell.

Furthermore, there is provided a mutant tobacco plant or a transgenic tobacco plant or component or part thereof comprising the plant cell described above.

Furthermore, there is provided plant material consisting of biomass, stem, flowers or leaves, from the tobacco plant described above.

Furthermore, there is provided a tobacco product comprising the tobacco plant cell the tobacco plant or the plant material described above.

Also herein is provided a method for preparing a mutant tobacco plant with modulated levels of protease as compared to a control tobacco plant, wherein the control tobacco plant has the same genotype as the starting material for the genetic alteration that resulted in the mutant tobacco plant, said method comprising the steps of:
(a) providing a tobacco plant comprising (i) a polynucleotide comprising, consisting or consisting essentially of a sequence encoding a functional protease and having at least 96% sequence identity to a protease selected from:
   at least one of SEQ ID NO: 1 to 16; or
   at least one of SEQ ID NO: 30 to 41; or
   at least one of SEQ ID NO: 17 to 22; or
   at least one of SEQ ID NO: 42 to 44; or
   at least one of SEQ ID NO: 45 to 61; or
   at least one of SEQ ID NO: 23 to 29; or
(b) inserting one or more mutations into said polynucleotide of said tobacco plant to create a mutant tobacco plant; and
(c) curing the tobacco plant material.

Also herein is provided a method for producing cured plant material, preferably cured leaves, or flowers with an altered flavour profile as compared to control plant material comprising the steps of:
(a) providing a tobacco plant described above or the plant material described above;
(b) optionally harvesting the plant material therefrom; and
(c) curing the plant material for a period of time such that the levels of at least one protease are modulated compared to control cured plant material.
Also herein is provided the use of
(i) a polynucleotide comprising, consisting or consisting essentially of a sequence encoding a functional protease and having at least 96% sequence identity to
   a protease selected from:
   at least one of SEQ ID NO: 1 to 16; or
   at least one of SEQ ID NO: 30 to 41; or
   at least one of SEQ ID NO: 17 to 22; or
   at least one of SEQ ID NO: 42 to 44; or
   at least one of SEQ ID NO: 45 to 61; or
   at least one of SEQ ID NO: 23 to 29; or
(ii) a construct, vector or expression vector comprising the isolated polynucleotide set forth in (i),
for the up-regulation or down-regulation of the expression or activity of one or more proteases in a mutant tobacco plant or a transgenic tobacco plant during a tobacco curing procedure as compared to a control tobacco plant, wherein the control tobacco plant has the same genotype as the starting material for the genetic alteration that resulted in the mutant tobacco plant or a transgenic tobacco plant,

### BRIEF DESCRIPTION OF THE DRAWINGS

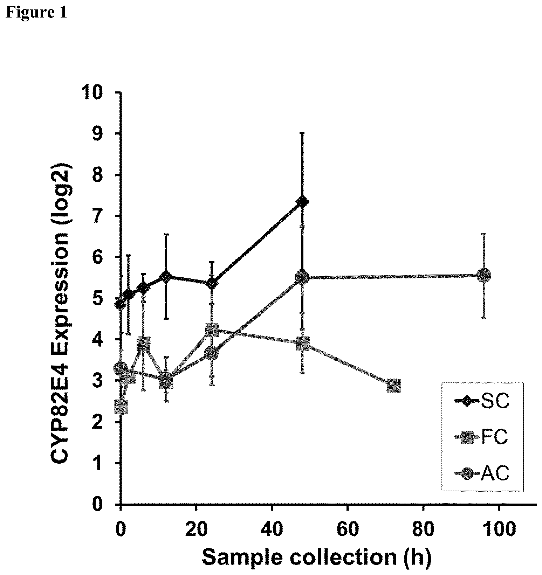

**Figure 1****.** Expression of CYP82E4 (AGD93125.1 / GI:444237502) increased after 48h curing in the three main tobacco types, SC, sun-cured; FC, flue-cured; AC, air-cured.
**Figure 2****.** The expression of 80 senescence-activated protease genes increased in the three main tobacco types

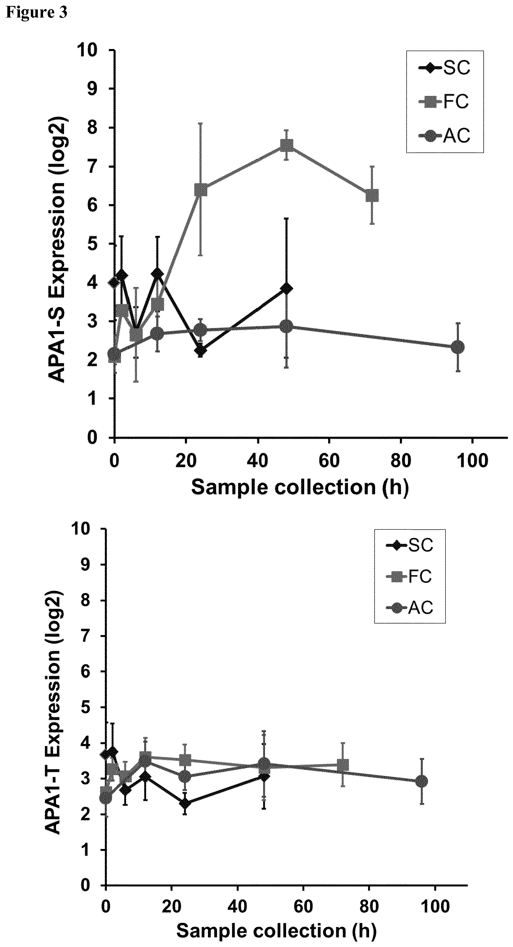

**Figure 3****.** One *APA1* tobacco gene (SEQ 68) is only expressed during Virginia Curing.

### DEFINITIONS

The technical terms and expressions used within the scope of this application are generally to be given the meaning commonly applied to them in the pertinent art of plant and molecular biology. All of the following term definitions apply to the complete content of this application. The word "comprising" does not exclude other elements or steps, and the indefinite article "a" or "an" does not exclude a plurality. A single step may fulfil the functions of several features recited in the claims. The terms "about", "essentially" and "approximately" in the context of a given numerate value or range refers to a value or range that is within 20%, within 10%, or within 5%, 4%, 3%, 2% or 1% of the given value or range.

The term "isolated" refers to any entity that is taken from its natural milieu, but the term does not connote any degree of purification.

An "expression vector" is a nucleic acid vehicle that comprises a combination of nucleic acid components for enabling the expression of nucleic acid. Suitable expression vectors include episomes capable of extra-chromosomal replication such as circular, double-stranded nucleic acid plasmids; linearized double-stranded nucleic acid plasmids; and other functionally equivalent expression vectors of any origin. An expression vector comprises at least a promoter positioned upstream and operably-linked to a nucleic acid, nucleic acid constructs or nucleic acid conjugate, as defined below.

The term "construct" refers to a double-stranded, recombinant nucleic acid fragment comprising one or more polynucleotides. The construct comprises a "template strand" base-paired with a complementary "sense or coding strand." A given construct can be inserted into a vector in two possible orientations, either in the same (or sense) orientation or in the reverse (or anti-sense) orientation with respect to the orientation of a promoter positioned within a vector - such as an expression vector.

A "vector" refers to a nucleic acid vehicle that comprises a combination of nucleic acid components for enabling the transport of nucleic acid, nucleic acid constructs and nucleic acid conjugates and the like. Suitable vectors include episomes capable of extra-chromosomal replication such as circular, double-stranded nucleic acid plasmids; linearized double-stranded nucleic acid plasmids; and other vectors of any origin.

A "promoter" refers to a nucleic acid element/sequence, typically positioned upstream and operably-linked to a double-stranded DNA fragment. Promoters can be derived entirely from regions proximate to a native gene of interest, or can be composed of different elements derived from different native promoters or synthetic DNA segments.

The terms "homology, identity or similarity" refer to the degree of sequence similarity between two polypeptides or between two nucleic acid molecules compared by sequence alignment. The degree of homology between two discrete nucleic acid sequences being compared is a function of the number of identical, or matching, nucleotides at comparable positions. The percent identity may be determined by visual inspection and mathematical calculation. Alternatively, the percent identity of two nucleic acid sequences may be determined by comparing sequence information using a computer program such as - ClustalW, BLAST, FASTA or Smith-Waterman.

A "variant" means a substantially similar sequence. A variant can have a similar function or substantially similar function as a wild-type sequence. For a protease, a similar function is at least about 50%, 60%, 70%, 80% or 90% of wild-type enzyme function under the same conditions. For a protease, a substantially similar function is at least about 90%, 95%, 96%, 97%, 98% or 99% of wild-type enzyme function under the same conditions. For example, wild-type protease sequences are set forth in SEQ ID Nos: 81-160. The variants can have one or more mutations that result in the enzyme having a reduced level of protease activity as compared to the wild-type protease. The variants can have one or more mutations that result in their protease activity being knocked out (i.e. a 100% inhibition, and thus a non-functional polypeptide). Variants can also have increased activity, leading to a more active protease enzyme function.

The term "plant" refers to any plant or part of a plant at any stage of its life cycle or development, and its progenies. In one embodiment, the plant is a "tobacco plant", which refers to a plant belonging to the genus Nicotiana. Preferred species of tobacco plant are described herein.

"Plant parts" include plant cells, plant protoplasts, plant cell tissue cultures from which a whole plant can be regenerated, plant calli, plant clumps and plant cells that are intact in plants or parts of plants such as embryos, pollen, anthers, ovules, seeds, leaves, flowers, stems, branches, fruit, roots, root tips and the like. Progeny, variants and mutants of regenerated plants are also included within the scope of the disclosure, provided that they comprise the introduced polynucleotides described herein.

A "plant cell" refers to a structural and physiological unit of a plant. The plant cell may be in the form of a protoplast without a cell wall, an isolated single cell or a cultured cell, or as a part of higher organized unit such as but not limited to, plant tissue, a plant organ, or a whole plant. The term "plant material" refers to any solid, liquid or gaseous composition, or a combination thereof, obtainable from a plant, including biomass, leaves, stems, roots, flowers or flower parts, fruits, pollen, egg cells, zygotes, seeds, cuttings, secretions, extracts, cell or tissue cultures, or any other parts or products of a plant. In one embodiment, the plant material comprises or consists of biomass, stem, seed or leaves. In another embodiment, the plant material comprises or consists of leaves.

The term "variety" refers to a population of plants that share constant characteristics which separate them from other plants of the same species. While possessing one or more distinctive traits, a variety is further characterized by a very small overall variation between individuals within that variety. A variety is often sold commercially.

A "type" of tobacco is defined by origin and curing method. Flue-cured tobacco, which accounts for 40% of global production, is also known as "Bright" and "Virginia" tobacco. It is used almost entirely in cigarette blends. Some of the heavier leaves may be used in mixtures for pipe smoking. Some English cigarettes are 100% flue-cured. Flue-cured leaf is characterized by a high sugar: nitrogen ratio. This ratio is enhanced by the picking of the leaf in an advanced stage of ripeness, and by the unique curing process which allows certain chemical changes to occur in the leaf. Cured leaves vary from lemon to orange to mahogany in colour.

Burley is light air-cured type derived from the White Burley which arose as a mutant on a farm in Ohio in 1864. Burley is used primarily in cigarette blends. Some of the heavier leaf is sued in pipe blends and also for chewing.

Cured burley leaf is characterized by low sugar content and a very low sugar to nitrogen ratio (high nicotine). This is enhanced by high Nitrogen fertilizer, harvesting at an early stage of senescence, and the air curing process which allows oxidation of any sugars which may have occurred.

Maryland is another light air-cured type. It is used to some extent in American blended cigarettes and to a greater extent in certain Swiss cigarette blends.

Maryland tobacco is extremely fluffy, has good burning properties, low nicotine, and neutral aroma.

Dark air-cured tobacco encompasses a number of types used mainly for chewing, snuff, cigar, and pipe blends. Most of the world production is confined to the tropics.

Oriental tobacco gives a mild smoke with very characteristic aroma. Resins, waxes and gum exuded by glandular hairs (trichomes) furnish the aroma. Nicotine is low averaging around 1.0%.

Dark-fired tobacco is used in the production of snuff, chewing tobacco, and pipe blends. Dark-fired leaves are subjected to smoke from smoldering wood during the early stage of curing. The type of wood used is very important in determining taste and grown. Cured leaves are very dark in color and are long and heavy bodied.

The term "modulating" may refer to reducing, inhibiting, increasing or otherwise affecting the expression or activity of a polypeptide. The term may also refer to reducing, inhibiting, increasing or otherwise affecting the activity of a gene encoding a polypeptide which can include, but is not limited to, modulating transcriptional activity.

The term "reduce" or "reduced" as used herein, refers to a reduction of from about 10% to about 99%, or a reduction of at least 10%, at least 20%, at least 25%, at least 30%, at least 40%, at least 50%, at least 60%, at least 70%, at least 75%, at least 80%, at least 90%, at least 95%, at least 98%, at least 99%, or at least 100% or more of a quantity or an activity, such as but not limited to polypeptide activity, transcriptional activity and protein expression. The term "inhibit" or "inhibited" as used herein, refers to a reduction of from about 98% to about 100%, or a reduction of at least 98%, at least 99%, but particularly of 100%, of a quantity or an activity, such as but not limited to polypeptide activity, transcriptional activity and protein expression.

The term "increase" or "increased" as used herein, refers to an increase of from about 5% to about 99%, or an increase of at least 5%, at least 10%, at least 20%, at least 25%, at least 30%, at least 40%, at least 50%, at least 60%, at least 70%, at least 75%, at least 80%, at least 90%, at least 95%, at least 98%, at least 99%, at least 100%, at least 500% or at least 1000% or more of a quantity or an activity, such as but not limited to polypeptide activity, transcriptional activity and protein expression.

The term "control" in the context of a control plant means a plant or plant cell in which the expression or activity of an enzyme has not been modified (for example, increased or reduced) and so it can provide a comparison with a plant in which the expression or activity of the enzyme has been modified. The control plant may comprise an empty vector. The control plant or plant cell may correspond to a wild-type plant or wild-type plant cell. For example, the control plant or plant cell can be the same genotype as the starting material for the genetic alteration that resulted in the subject plant. In all such cases, the subject plant and the control plant are cultured and harvested using the same protocols for comparative purposes. Changes in levels, ratios, activity, or distribution of the genes or polypeptides described herein, or changes in tobacco plant phenotype, particularly reduced production of proteases, can be measured by comparing a subject plant to the control plant, where the subject plant and the control plant have been cultured, harvested and cured using the same protocols. The control plant can provide a reference point for measuring changes in phenotype of the subject plant. The measurement of changes in phenotype can be measured at any time in a plant, including during plant development, senescence, or preferably after curing. Measurement of changes in phenotype can be measured in plants grown under any conditions, including from plants grown in growth chamber, greenhouse, or in a field. Changes in phenotype can be measured by determining the expression or activity of proteases identified herein in SEQ ID Nos 81-160.

### DETAILED DESCRIPTION

In one embodiment, there is a mutant tobacco plant cell or a transgenic tobacco plant cell comprising apolynucleotide comprising, consisting or consisting essentially a sequence encoding a functional protease having at least 96% sequence identity to any of the sequences selected from
at least one of SEQ ID NO: 1 to 16; or
at least one of SEQ ID NO: 30 to 41; or
at least one of SEQ ID NO: 17 to 22; or
at least one of SEQ ID NO: 42 to 44; or
at least one of SEQ ID NO: 45 to 61; or
at least one of SEQ ID NO: 23 to 29

These polynucleotides are shown in the sequence lisiting. Suitably, the isolated polynucleotide comprises, consists or consists essentially of a sequence having at least 95%, 96%, 97%, 98%, 99% or 100% sequence identity thereto.

Suitably, as described herein but not claimed, the polynucleotide(s) described herein encode a protein with protease activity that is at least about 50%, 60%, 70%, 80%, 90% 95%, 96%, 97%, 98%, 99% 100% or more of the activity of the protein set forth in SEQ ID NOs: 81-160. A polynucleotide as described herein can include a polymer of nucleotides, which may be unmodified or modified deoxyribonucleic acid (DNA) or ribonucleic acid (RNA). Accordingly, a polynucleotide can be, without limitation, a genomic DNA, complementary DNA (cDNA), mRNA, or antisense RNA or a fragment(s) thereof. Moreover, a polynucleotide can be single-stranded or double-stranded DNA, DNA that is a mixture of single-stranded and double-stranded regions, a hybrid molecule comprising DNA and RNA, or a hybrid molecule with a mixture of single-stranded and double-stranded regions or a fragment(s) thereof. In addition, the polynucleotide can be composed of triple-stranded regions comprising DNA, RNA, or both or a fragment(s) thereof. A polynucleotide can contain one or more modified bases, such as phosphothioates, and can be a peptide nucleic acid. Generally, polynucleotides can be assembled from isolated or cloned fragments of cDNA, genomic DNA, oligonucleotides, or individual nucleotides, or a combination of the foregoing. Although the polynucleotide sequences described herein are shown as DNA sequences, the sequences include their corresponding RNA sequences, and their complementary (for example, completely complementary) DNA or RNA sequences, including the reverse complements thereof.

A polynucleotide as described herein will generally contain phosphodiester bonds, although in some cases, polynucleotide analogues are included that may have alternate backbones, comprising, for example, phosphoramidate, phosphorothioate, phosphorodithioate, or O-methylphophoroamidite linkages; and peptide polynucleotide backbones and linkages. Other analogue polynucleotides include those with positive backbones; non-ionic backbones, and non-ribose backbones. Modifications of the ribose-phosphate backbone may be done for a variety of reasons, for example, to increase the stability and half-life of such molecules in physiological environments or as probes on a biochip. Mixtures of naturally occurring polynucleotides and analogues can be made; alternatively, mixtures of different polynucleotide analogues, and mixtures of naturally occurring polynucleotides and analogues may be made. A variety of polynucleotide analogues are known, including, for example, phosphoramidate, phosphorothioate, phosphorodithioate, O-methylphophoroamidite linkages and peptide polynucleotide backbones and linkages. Other analogue polynucleotides include those with positive backbones, non-ionic backbones and non-ribose backbones. Polynucleotides containing one or more carbocyclic sugars are also included.

Other analogues , described herein but not claimed, include peptide polynucleotides which are peptide polynucleotide analogues. These backbones are substantially non-ionic under neutral conditions, in contrast to the highly charged phosphodiester backbone of naturally occurring polynucleotides. This may result in advantages. First, the peptide polynucleotide backbone may exhibit improved hybridization kinetics. Peptide polynucleotides have larger changes in the melting temperature for mismatched versus perfectly matched base pairs. DNA and RNA typically exhibit a 2-4 °C drop in melting temperature for an internal mismatch. With the non-ionic peptide polynucleotide backbone, the drop is closer to 7-9 °C. Similarly, due to their non-ionic nature, hybridization of the bases attached to these backbones is relatively insensitive to salt concentration. In addition, peptide polynucleotides may not be degraded or degraded to a lesser extent by cellular enzymes, and thus may be more stable.

Among the uses, described herein but not claimed, of the disclosed polynucleotides, and fragments thereof, is the use of fragments as probes in nucleic acid hybridisation assays or primers for use in nucleic acid amplification assays. Such fragments generally comprise at least about 10, 11, 12, 13, 14, 15, 16, 17, 18, 19 or 20 or more contiguous nucleotides of a DNA sequence. In other embodiments, a DNA fragment comprises at least about 10, 15, 20, 30, 40, 50 or 60 or more contiguous nucleotides of a DNA sequence. Thus, in one aspect, there is also provided a method for detecting a polynucleotide encoding a protein with nicotine N-demethylase activity member or encoding a nicotine N-demethylase enzyme comprising the use of the probes or primers or both.

The basic parameters affecting the choice of hybridization conditions and guidance for devising suitable conditions are described, but not herein claimed, by Sambrook, J., E. F. Fritsch, and T. Maniatis (1989, Molecular Cloning: A Laboratory Manual, Cold Spring Harbor Laboratory Press, Cold Spring Harbor, N.Y.). Using knowledge of the genetic code in combination with the amino acid sequences described herein, sets of degenerate oligonucleotides can be prepared. Such oligonucleotides are useful as primers, for example, in polymerase chain reactions (PCR), whereby DNA fragments are isolated and amplified. In certain embodiments, degenerate primers can be used as probes for genetic libraries. Such libraries would include but are not limited to cDNA libraries, genomic libraries, and even electronic express sequence tag or DNA libraries. Homologous sequences identified by this method would then be used as probes to identify homologues of the sequences identified herein.

Also of potential use, and described herein but not claimed, are polynucleotides and oligonucleotides (for example, primers or probes) that hybridize under reduced stringency conditions, typically moderately stringent conditions, and commonly highly stringent conditions to the polynucleotide(s) as described herein. The basic parameters affecting the choice of hybridization conditions and guidance for devising suitable conditions are set forth by Sambrook, J., E. F. Fritsch, and T. Maniatis (1989, Molecular Cloning: A Laboratory Manual, Cold Spring Harbor Laboratory Press, Cold Spring Harbor, N.Y. and can be readily determined by those having ordinary skill in the art based on, for example, the length or base composition of the polynucleotide. One way of achieving moderately stringent conditions involves the use of a prewashing solution containing 5x Standard Sodium Citrate, 0.5% Sodium Dodecyl Sulphate, 1.0 mM Ethylenediaminetetraacetic acid (pH 8.0), hybridization buffer of about 50% formamide, 6x Standard Sodium Citrate, and a hybridization temperature of about 55 °C (or other similar hybridization solutions, such as one containing about 50% formamide, with a hybridization temperature of about 42°C), and washing conditions of about 60°C, in 0.5x Standard Sodium Citrate, 0.1% Sodium Dodecyl Sulphate. Generally, highly stringent conditions are defined as hybridization conditions as above, but with washing at approximately 68 °C, 0.2x Standard Sodium Citrate, 0.1% Sodium Dodecyl Sulphate. SSPE (1 x SSPE is 0.15 M sodium chloride, 10 mM sodium phosphate, and 1.25 mM Ethylenediaminetetraacetic acid, pH 7.4) can be substituted for Standard Sodium Citrate (1 x Standard Sodium Citrate is 0.15 M sodium chloride and 15 mM sodium citrate) in the hybridization and wash buffers; washes are performed for 15 minutes after hybridization is complete. It should be understood that the wash temperature and wash salt concentration can be adjusted as necessary to achieve a desired degree of stringency by applying the basic principles that govern hybridization reactions and duplex stability, as known to those skilled in the art and described further below (see, for example, Sambrook, J., E. F. Fritsch, and T. Maniatis (1989, Molecular Cloning: A Laboratory Manual, Cold Spring Harbor Laboratory Press, Cold Spring Harbor, N.Y). When hybridizing a polynucleotide to a target polynucleotide of unknown sequence, the hybrid length is assumed to be that of the hybridizing polynucleotide. When polynucleotides of known sequence are hybridized, the hybrid length can be determined by aligning the sequences of the polynucleotides and identifying the region or regions of optimal sequence complementarity. The hybridization temperature for hybrids anticipated to be less than 50 base pairs in length should be 5 to 10 °C less than the melting temperature of the hybrid, where melting temperature is determined according to the following equations. For hybrids less than 18 base pairs in length, melting temperature (°C)=2(number of A+T bases)+4(number of G+C bases). For hybrids above 18 base pairs in length, melting temperature (°C)=81.5+16.6(Iog10 [Na+])+0.41(% G+C)-(600/N), where N is the number of bases in the hybrid, and [Na+] is the concentration of sodium ions in the hybridization buffer ([Na+] for 1x Standard Sodium Citrate=0.165M). Typically, each such hybridizing polynucleotide has a length that is at least 25% (commonly at least 50%, 60%, or 70%, and most commonly at least 80%) of the length of a polynucleotide to which it hybridizes, and has at least 60% sequence identity (for example, at least 70%, 75%, 80%, 85%, 90%, 95%, 96%, 97%, 98%, 99% or 100%) with a polynucleotide to which it hybridizes.

As will be understood by the person skilled in the art, a linear DNA has two possible orientations: the 5'-to-3' direction and the 3'-to-5' direction. For example, if a reference sequence is positioned in the 5'-to-3' direction, and if a second sequence is positioned in the 5'-to-3' direction within the same polynucleotide molecule/strand, then the reference sequence and the second sequence are orientated in the same direction, or have the same orientation. Typically, a promoter sequence and a gene of interest under the regulation of the given promoter are positioned in the same orientation. However, with respect to the reference sequence positioned in the 5'-to-3' direction, if a second sequence is positioned in the 3'-to-5' direction within the same polynucleotide molecule/strand, then the reference sequence and the second sequence are orientated in anti-sense direction, or have anti-sense orientation. Two sequences having anti-sense orientations with respect to each other can be alternatively described as having the same orientation, if the reference sequence (5'-to-3' direction) and the reverse complementary sequence of the reference sequence (reference sequence positioned in the 5'-to-3') are positioned within the same polynucleotide molecule/strand. The sequences set forth herein are shown in the 5'-to-3' direction.

Recombinant constructs provided herein can be used to transform plants or plant cells in order to modulate protein expression and/or activity levels. A recombinant polynucleotide construct can comprise a polynucleotide encoding one or more polynucleotides as described herein, operably linked to a regulatory region suitable for expressing the polypeptide. Thus, a polynucleotide can comprise a coding sequence that encodes the polypeptide as described herein. Plants or plant cells in which protein expression and/or activity levels are modulated can include mutant, non-naturally occurring, transgenic, man-made or genetically engineered plants or plant cells. Suitably, the transgenic plant or plant cell comprises a genome that has been altered by the stable integration of recombinant DNA. Recombinant DNA includes DNA which has been genetically engineered and constructed outside of a cell and includes DNA containing naturally occurring DNA or cDNA or synthetic DNA. A transgenic plant can include a plant regenerated from an originally-transformed plant cell and progeny transgenic plants from later generations or crosses of a transformed plant. Suitably, the transgenic modification alters the expression or activity of the polynucleotide or the polypeptide described herein as compared to a control plant.

The polypeptide encoded by a recombinant polynucleotide can be a native polypeptide, or can be heterologous to the cell. In some cases, the recombinant construct contains a polynucleotide that modulates expression, operably linked to a regulatory region. Examples of suitable regulatory regions are described herein.

Vectors containing recombinant polynucleotide constructs such as those described herein are also provided. Suitable vector backbones include, for example, those routinely used in the art such as plasmids, viruses, artificial chromosomes, bacterial artificial chromosomes, yeast artificial chromosomes, or bacteriophage artificial chromosomes. Suitable expression vectors include, without limitation, plasmids and viral vectors derived from, for example, bacteriophage, baculoviruses, and retroviruses. Numerous vectors and expression systems are commercially available. The vectors can include, for example, origins of replication, scaffold attachment regions or markers. A marker gene can confer a selectable phenotype on a plant cell. For example, a marker can confer biocide resistance, such as resistance to an antibiotic (for example, kanamycin, G418, bleomycin, or hygromycin), or an herbicide (for example, glyphosate, chlorsulfuron or phosphinothricin). In addition, an expression vector can include a tag sequence designed to facilitate manipulation or detection (for example, purification or localization) of the expressed polypeptide. Tag sequences, such as luciferase, beta-glucuronidase, green fluorescent protein, glutathione S-transferase, polyhistidine, c-myc or hemagglutinin sequences typically are expressed as a fusion with the encoded polypeptide. Such tags can be inserted anywhere within the polypeptide, including at either the carboxyl or amino terminus.

A plant or plant cell can be transformed by having the recombinant polynucleotide integrated into its genome to become stably transformed. The plant or plant cell described herein can be stably transformed. Stably transformed cells typically retain the introduced polynucleotide with each cell division. A plant or plant cell can be transiently transformed such that the recombinant polynucleotide is not integrated into its genome. Transiently transformed cells typically lose all or some portion of the introduced recombinant polynucleotide with each cell division such that the introduced recombinant polynucleotide cannot be detected in daughter cells after a sufficient number of cell divisions.

A number of methods are available in the art for transforming a plant cell which are all encompassed herein, including biolistics, gene gun techniques, Agrobacterium-mediated transformation, viral vector-mediated transformation and electroporation. The Agrobacterium system for integration of foreign DNA into plant chromosomes has been extensively studied, modified, and exploited for plant genetic engineering. Naked recombinant DNA molecules comprising DNA sequences corresponding to the subject purified tobacco protein operably linked, in the sense or antisense orientation, to regulatory sequences are joined to appropriate T-DNA sequences by conventional methods. These are introduced into tobacco protoplasts by polyethylene glycol techniques or by electroporation techniques, both of which are standard. Alternatively, such vectors comprising recombinant DNA molecules encoding the subject purified tobacco protein are introduced into live Agrobacterium cells, which then transfer the DNA into the tobacco plant cells. Transformation by naked DNA without accompanying T-DNA vector sequences can be accomplished via fusion of tobacco protoplasts with DNA-containing liposomes or via electroporation. Naked DNA unaccompanied by T-DNA vector sequences can also be used to transform tobacco cells via inert, high velocity microprojectiles.

If a cell or cultured tissue is used as the recipient tissue for transformation, plants can be regenerated from transformed cultures if desired, by techniques known to those skilled in the art.

The choice of regulatory regions to be included in a recombinant construct depends upon several factors, including, but not limited to, efficiency, selectability, inducibility, desired expression level, and cell- or tissue-preferential expression. It is a routine matter for one of skill in the art to modulate the expression of a coding sequence by appropriately selecting and positioning regulatory regions relative to the coding sequence. Transcription of a polynucleotide can be modulated in a similar manner. Some suitable regulatory regions initiate transcription only, or predominantly, in certain cell types. Methods for identifying and characterizing regulatory regions in plant genomic DNA are known in the art.

Suitable promoters include tissue-specific promoters recognized by tissue-specific factors present in different tissues or cell types (for example, root-specific promoters, shoot-specific promoters, xylem-specific promoters), or present during different developmental stages, or present in response to different environmental conditions. Suitable promoters include constitutive promoters that can be activated in most cell types without requiring specific inducers. Examples of suitable promoters for controlling RNAi polypeptide production include the cauliflower mosaic virus 35S (CaMV/35S), SSU, OCS, lib4, usp, STLS1, B33, nos or ubiquitin- or phaseolin-promoters. Persons skilled in the art are capable of generating multiple variations of recombinant promoters.

Tissue-specific promoters are transcriptional control elements that are only active in particular cells or tissues at specific times during plant development, such as in vegetative tissues or reproductive tissues. Tissue-specific expression can be advantageous, for example, when the expression of polynucleotides in certain tissues is preferred. Examples of tissue-specific promoters under developmental control include promoters that can initiate transcription only (or primarily only) in certain tissues, such as vegetative tissues, for example, roots or leaves, or reproductive tissues, such as fruit, ovules, seeds, pollen, pistols, flowers, or any embryonic tissue. Reproductive tissue-specific promoters may be, for example, anther-specific, ovule-specific, embryo-specific, endosperm-specific, integument-specific, seed and seed coat-specific, pollen-specific, petal-specific, sepal-specific, or combinations thereof.

Suitable leaf-specific promoters include pyruvate, orthophosphate dikinase (PPDK) promoter from C4 plant (maize), cab-m1Ca+2 promoter from maize, the Arabidopsis thaliana myb-related gene promoter (Atmyb5), the ribulose biphosphate carboxylase (RBCS) promoters (for example, the tomato RBCS 1, RBCS2 and RBCS3A genes expressed in leaves and light-grown seedlings, RBCS1 and RBCS2 expressed in developing tomato fruits or ribulose bisphosphate carboxylase promoter expressed almost exclusively in mesophyll cells in leaf blades and leaf sheaths at high levels).

Suitable senescence-specific promoters include a tomato promoter active during fruit ripening, senescence and abscission of leaves, a maize promoter of gene encoding a cysteine protease, the promoter of 82E4 and the promoter of SAG genes. Suitable anther-specific promoters can be used. Suitable root-preferred promoters known to persons skilled in the art may be selected. Suitable seed-preferred promoters include both seed-specific promoters (those promoters active during seed development such as promoters of seed storage proteins) and seed-germinating promoters (those promoters active during seed germination). Such seed-preferred promoters include, but are not limited to, Cim1 (cytokinin-induced message); cZ19B1 (maize 19 kDa zein); milps (myo-inositol-1-phosphate synthase); mZE40-2, also known as Zm-40; nuclc; and celA (cellulose synthase). Gama-zein is an endosperm-specific promoter. Glob-1 is an embryo-specific promoter. For dicots, seed-specific promoters include, but are not limited to, bean beta-phaseolin, napin, β-conglycinin, soybean lectin, cruciferin, and the like. For monocots, seed-specific promoters include, but are not limited to, a maize 15 kDa zein promoter, a 22 kDa zein promoter, a 27 kDa zein promoter, a g-zein promoter, a 27 kDa gamma-zein promoter (such as gzw64A promoter, see Genbank Accession number S78780), a waxy promoter, a shrunken 1 promoter, a shrunken 2 promoter, a globulin 1 promoter (see Genbank Accession number L22344), an Itp2 promoter, cim1 promoter, maize end1 and end2 promoters, nuc1 promoter, Zm40 promoter, eep1 and eep2; lec1, thioredoxin H promoter; mlip15 promoter, PCNA2 promoter; and the shrunken-2 promoter.

Examples of inducible promoters include promoters responsive to pathogen attack, anaerobic conditions, elevated temperature, light, drought, cold temperature, or high salt concentration. Pathogen-inducible promoters include those from pathogenesis-related proteins (PR proteins), which are induced following infection by a pathogen (for example, PR proteins, SAR proteins, beta-1,3-glucanase, chitinase).

In addition to plant promoters, other suitable promoters may be derived from bacterial origin for example, the octopine synthase promoter, the nopaline synthase promoter and other promoters derived from Ti plasmids, or may be derived from viral promoters (for example, 35S and 19S RNA promoters of cauliflower mosaic virus (CaMV), constitutive promoters of tobacco mosaic virus, cauliflower mosaic virus (CaMV) 19S and 35S promoters, or figwort mosaic virus 35S promoter).

Preferred promoters include the control elements provided herein, as part of SEQ ID Nos. 1-80, which demonstrate desirable expression during curing procedures in tobacco leaf.

In another aspect, there is described herein but not claimed an isolated polypeptide comprising, consisting or consisting essentially of a polypeptide sequence having at least 95% sequence identity to any of the polypeptide sequences described herein, including any of the polypeptides shown in the sequence lisiting. Suitably, the isolated polypeptide comprises, consists or consists essentially of a sequence having at least 95% 96%, 97%, 98%, 99%, 99.1%, 99.2%, 99.3%, 99.4%, 99.5%, 99.6%, 99.7%, 99.8%, 99.9% or 100% sequence identity thereto.

The polypeptide can include sequences comprising a sufficient or substantial degree of identity or similarity to SEQ ID NOs: 81-160 to function as proteases. Fragments of the polypeptide(s) typically retain some or all of the activity of the full length sequence.

As discussed herein, the polypeptides also include mutants produced by introducing any type of alterations (for example, insertions, deletions, or substitutions of amino acids; changes in glycosylation states; changes that affect refolding or isomerizations, three-dimensional structures, or self-association states), which can be deliberately engineered or isolated naturally provided that they still have some or all of their function or activity as a protease. Suitably, the function or activity as a protease is modulated, increased or reduced. Polypeptides include variants produced by introducing any type of alterations (for example, insertions, deletions, or substitutions of amino acids; changes in glycosylation states; changes that affect refolding or isomerizations, three-dimensional structures, or self-association states), which can be deliberately engineered or isolated naturally. The variant may have alterations which produce a silent change and result in a functionally equivalent protein. Deliberate amino acid substitutions may be made on the basis of similarity in polarity, charge, solubility, hydrophobicity, hydrophilicity and the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine. Conservative substitutions may be made, for example according to the Table below. Amino acids in the same block in the second column and preferably in the same line in the third column may be substituted for each other:

| | | |
|---|---|---|
| ALIPHATIC | Non-polar | Gly Ala Pro |
| | | Ile Leu Val |
| | Polar - uncharged | Cys Ser Thr Met |
| | | Asn Gly |
| | Polar - charged | Asp Glu |
| | | Lys Arg |
| AROMATIC | | His Phe TrpTyr |

The polypeptide may be a mature protein or an immature protein or a protein derived from an immature protein. Polypeptides may be in linear form or cyclized using known methods. Polypeptides typically comprise at least 10, at least 20, at least 30, or at least 40 contiguous amino acids.

A tobacco plant or plant cell comprising a mutation in a gene encoding a protease as described herein is disclosed, wherein said mutation results in modulated expression or modulated function of said protease. The expression or function of the protease(s) may be enhanced. Aside from one or more mutations in said protease, the mutant plants or plant cells can have one or more further mutations in one or more other genes or polypeptides. In certain embodiments, aside from the one or more mutations in a protease gene, the mutants can have one or more further mutations in one or more other genes or polypeptides - such as one or more other protease genes or polypeptides as described in the Sequence Listing. Suitably, a protease is expressed in the leaves of the mutant plant during the curing procedure.

There is also provided a method for modulating the level of a protease in a (cured) tobacco plant or in (cured) tobacco plant material said method comprising introducing into the genome of said plant one or more mutations that modulate expression of at least one protease gene, wherein said at least one protease gene is selected from SEQ ID Nos: 1-80.

There is also described herein but not claimed a method for identifying a tobacco plant with increased levels of protease, said method comprising screening a nucleic acid sample from a tobacco plant of interest for the presence of one or more mutations in SEQ ID NOs:1-80, and optionally correlating the identified mutation(s) with mutation(s) that are known to modulate levels of protease.

There is also disclosed a tobacco plant or plant cell that is heterozygous or homozygous for mutations in a gene encoding a protease, wherein said mutation results in modulated (enhanced or reduced) expression or function of said protease.

A number of approaches can be used to combine mutations in one plant including sexual crossing. A plant having one or more favourable heterozygous or homozygous mutations in a protease gene that enhances or reduces protease expression or activity can be crossed with a plant having one or more favourable heterozygous or homozygous mutations in one or more other protease genes that enhance or reduce protease activity. In one embodiment, crosses are made in order to introduce one or more favourable heterozygous or homozygous mutations within a protease gene within the same plant.

The activity of one or more protease polypeptides in a tobacco plant is reduced or enhanced according to the present disclosure if the protease activity is statistically lower or higher than the protease activity of the same protease(s) in a tobacco plant that has not been modified to inhibit the activity of that protease polypeptide and which has been cultured, harvested and cured using the same protocols.

In some embodiments, the mutation(s) is introduced into a tobacco plant or plant cell using a mutagenesis approach, and the introduced mutation is identified or selected using methods known to those of skill in the art - such as Southern blot analysis, DNA sequencing, PCR analysis, or phenotypic analysis. Mutations that impact gene expression or that interfere with the function of the encoded protein can be determined using methods that are well known in the art. Insertional mutations in gene exons usually result in null-mutants. Mutations in conserved residues can be particularly effective in inhibiting the metabolic function of the encoded protein.

Methods for obtaining mutant polynucleotides and polypeptides are also disclosed but not claimed. Any plant of interest, including a plant cell or plant material can be genetically modified by various methods known to induce mutagenesis, including site-directed mutagenesis, oligonucleotide-directed mutagenesis, chemically-induced mutagenesis, irradiation-induced mutagenesis, mutagenesis utilizing modified bases, mutagenesis utilizing gapped duplex DNA, double-strand break mutagenesis, mutagenesis utilizing repair-deficient host strains, mutagenesis by total gene synthesis, DNA shuffling and other equivalent methods.

Fragments of protease polynucleotides and polypeptides encoded thereby are also disclosed but not claimed. Fragments of a polynucleotide may encode protein fragments that retain the biological activity of the native protein and hence are involved in the metabolic conversion of nicotine to nornicotine. Alternatively, fragments of a polynucleotide that are useful as hybridization probes or PCR primers generally do not encode fragment proteins retaining biological activity. Furthermore, fragments of the disclosed nucleotide sequences include those that can be assembled within recombinant constructs as discussed herein. Fragments of a polynucleotide sequence may range from at least about 25 nucleotides, about 50 nucleotides, about 75 nucleotides, about 100 nucleotides about 150 nucleotides, about 200 nucleotides, about 250 nucleotides, about 300 nucleotides, about 400 nucleotides, about 500 nucleotides, about 600 nucleotides, about 700 nucleotides, about 800 nucleotides, about 900 nucleotides, about 1000 nucleotides, about 1100 nucleotides, about 1200 nucleotides, about 1300 nucleotides or about 1400 nucleotides and up to the full-length polynucleotide encoding the polypeptides described herein. Fragments of a polypeptide sequence may range from at least about 25 amino acids, about 50 amino acids, about 75 amino acids, about 100 amino acids about 150 amino acids, about 200 amino acids, about 250 amino acids, about 300 amino acids, about 400 amino acids, about 500 amino acids, and up to the full-length polypeptide described herein.

Mutant polypeptide variants can be used to create mutant, non-naturally occurring or transgenic plants (for example, mutant, non-naturally occurring, transgenic, man-made or genetically engineered plants) or plant cells comprising one or more mutant polypeptide variants. Suitably, mutant polypeptide variants retain the activity of the unmutated polypeptide. The activity of the mutant polypeptide variant may be higher, lower or about the same as the unmutated polypeptide.

Mutations in the nucleotide sequences and polypeptides described herein can include man-made mutations or synthetic mutations or genetically engineered mutations. Mutations in the nucleotide sequences and polypeptides described herein can be mutations that are obtained or obtainable via a process which includes an *in vitro* or an *in vivo* manipulation step. Mutations in the nucleotide sequences and polypeptides described herein can be mutations that are obtained or obtainable via a process which includes intervention by man. By way of example, the process may include mutagenesis using exogenously added chemicals - such as mutagenic, teratogenic, or carcinogenic organic compounds, for example ethyl methanesulfonate (EMS), that produce random mutations in genetic material. By way of further example, the process may include one or more genetic engineering steps - such as one or more of the genetic engineering steps that are described herein or combinations thereof. By way of further example, the process may include one or more plant crossing steps. A polypeptide may be prepared by culturing transformed or recombinant host cells under culture conditions suitable to express a polypeptide. The resulting expressed polypeptide may then be purified from such culture using known purification processes. The purification of the polypeptide may include an affinity column containing agents which will bind to the polypeptide; one or more column steps over such affinity resins; one or more steps involving hydrophobic interaction chromatography; or immunoaffinity chromatography. Alternatively, the polypeptide may also be expressed in a form that will facilitate purification. For example, it may be expressed as a fusion polypeptide, such as those of maltose binding polypeptide, glutathione-5-transferase, his-tag or thioredoxin. Kits for expression and purification of fusion polypeptides are commercially available. The polypeptide may be tagged with an epitope and subsequently purified by using a specific antibody directed to such epitope. One or more liquid chromatography steps - such as reverse-phase high performance liquid chromatography can be employed to further purify the polypeptide. Some or all of the foregoing purification steps, in various combinations, can be employed to provide a substantially homogeneous recombinant polypeptide. The polypeptide thus purified may be substantially free of other polypeptides and is defined herein as a "substantially purified polypeptide"; such purified polypeptides include polypeptides, fragments, variants, and the like. Expression, isolation, and purification of the polypeptides and fragments can be accomplished by any suitable technique, including but not limited to the methods described herein.

It is also possible to utilise an affinity column such as a monoclonal antibody generated against polypeptides, to affinity-purify expressed polypeptides. These polypeptides can be removed from an affinity column using conventional techniques, for example, in a high salt elution buffer and then dialyzed into a lower salt buffer for use or by changing pH or other components depending on the affinity matrix utilized, or be competitively removed using the naturally occurring substrate of the affinity moiety.

Isolated or substantially purified polynucleotides or protein compositions are disclosed but not claimed. An "isolated" or "purified" polynucleotide or protein, or biologically active portion thereof, is substantially or essentially free from components that normally accompany or interact with the polynucleotide or protein as found in its naturally occurring environment. Thus, an isolated or purified polynucleotide or protein is substantially free of other cellular material, or culture medium when produced by recombinant techniques, or substantially free of chemical precursors or other chemicals when chemically synthesized. Optimally, an "isolated" polynucleotide is free of sequences (optimally protein encoding sequences) that naturally flank the polynucleotide (for example, sequences located at the 5' and 3' ends of the polynucleotide) in the genomic DNA of the organism from which the polynucleotide is derived. For example, in various embodiments, the isolated polynucleotide can contain less than about 5 kb, 4 kb, 3 kb, 2 kb, 1 kb, 0.5 kb, or 0.1 kb of nucleotide sequence that naturally flank the polynucleotide in genomic DNA of the cell from which the polynucleotide is derived. A protein that is substantially free of cellular material includes preparations of protein having less than about 30%, 20%, 10%, 5%, or 1% (by dry weight) of contaminating protein.

A polypeptide may also be produced by known conventional chemical synthesis. Methods for constructing the polypeptides or fragments thereof by synthetic means are known to those skilled in the art. The synthetically-constructed polypeptide sequences, by virtue of sharing primary, secondary or tertiary structural or conformational characteristics with native polypeptides may possess biological properties in common therewith, including biological activity.

The term 'non-naturally occurring' as used herein describes an entity (for example, a polynucleotide, a genetic mutation, a polypeptide, a plant, a plant cell and plant material) that is not formed by nature or that does not exist in nature. Such non-naturally occurring entities or artificial entities may be made, synthesized, initiated, modified, intervened, or manipulated by methods described herein or that are known in the art. Such non-naturally occurring entities or artificial entities may be made, synthesized, initiated, modified, intervened, or manipulated by man. Thus, by way of example, a non-naturally occurring plant, a non-naturally occurring plant cell or non-naturally occurring plant material may be made using traditional plant breeding techniques - such as backcrossing - or by genetic manipulation technologies - such as antisense RNA, interfering RNA, meganuclease and the like. By way of further example, a non-naturally occurring plant, a non-naturally occurring plant cell or non-naturally occurring plant material may be made by introgression of or by transferring one or more genetic mutations (for example one or more polymorphisms) from a first plant or plant cell into a second plant or plant cell (which may itself be naturally occurring), such that the resulting plant, plant cell or plant material or the progeny thereof comprises a genetic constitution (for example, a genome, a chromosome or a segment thereof) that is not formed by nature or that does not exist in nature. The resulting plant, plant cell or plant material is thus artificial or non-naturally occurring. Accordingly, an artificial or non-naturally occurring plant or plant cell may be made by modifying a genetic sequence in a first naturally occurring plant or plant cell, even if the resulting genetic sequence occurs naturally in a second plant or plant cell that comprises a different genetic background from the first plant or plant cell. In certain embodiments, a mutation is not a naturally occurring mutation that exists naturally in a nucleotide sequence or a polypeptide - such as a gene or a protein.

Differences in genetic background can be detected by phenotypic differences or by molecular biology techniques known in the art - such as nucleic acid sequencing, presence or absence of genetic markers (for example, microsatellite RNA markers).

Antibodies that are immunoreactive with the polypeptides described herein are also provided but not claimed. The polypeptides, fragments, variants, fusion polypeptides, and the like, as set forth herein, can be employed as "immunogens" in producing antibodies immunoreactive therewith. Such antibodies may specifically bind to the polypeptide via the antigen-binding sites of the antibody. Specifically binding antibodies are those that will specifically recognize and bind with a polypeptide, homologues, and variants, but not with other molecules. In one embodiment, the antibodies are specific for polypeptides having an amino acid sequence as set forth herein and do not cross-react with other polypeptides.

More specifically, the polypeptides, fragment, variants, fusion polypeptides, and the like contain antigenic determinants or epitopes that elicit the formation of antibodies. These antigenic determinants or epitopes can be either linear or conformational (discontinuous). Linear epitopes are composed of a single section of amino acids of the polypeptide, while conformational or discontinuous epitopes are composed of amino acids sections from different regions of the polypeptide chain that are brought into close proximity upon polypeptide folding. Epitopes can be identified by any of the methods known in the art. Additionally, epitopes from the polypeptides can be used as research reagents, in assays, and to purify specific binding antibodies from substances such as polyclonal sera or supernatants from cultured hybridomas. Such epitopes or variants thereof can be produced using techniques known in the art such as solid-phase synthesis, chemical or enzymatic cleavage of a polypeptide, or using recombinant DNA technology.

Both polyclonal and monoclonal antibodies to the polypeptides can be prepared by conventional techniques. Hybridoma cell lines that produce monoclonal antibodies specific for the polypeptides are also contemplated herein. Such hybridomas can be produced and identified by conventional techniques. For the production of antibodies, various host animals may be immunized by injection with a polypeptide, fragment, variant, or mutants thereof. Such host animals may include, but are not limited to, rabbits, mice, and rats, to name a few. Various adjutants may be used to increase the immunological response. Depending on the host species, such adjuvants include, but are not limited to, Freund's (complete and incomplete), mineral gels such as aluminium hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and *Corynebacterium parvum.* The monoclonal antibodies can be recovered by conventional techniques. Such monoclonal antibodies may be of any immunoglobulin class including IgG, IgM, IgE, IgA, IgD, and any subclass thereof.

The antibodies can also be used in assays to detect the presence of the polypeptides or fragments, either *in vitro* or *in vivo.* The antibodies also can be employed in purifying polypeptides or fragments by immunoaffinity chromatography.

Other than mutagenesis, compositions that can modulate the expression or the activity of one or more of the proteases described herein include, but are not limited to, sequence-specific polynucleotides that can interfere with the transcription of one or more endogenous gene(s); sequence-specific polynucleotides that can interfere with the translation of RNA transcripts (for example, double-stranded RNAs, siRNAs, ribozymes); sequence-specific polypeptides that can interfere with the stability of one or more proteins; sequence-specific polynucleotides that can interfere with the enzymatic activity of one or more proteins or the binding activity of one or more proteins with respect to substrates or regulatory proteins; antibodies that exhibit specificity for one or more proteins; small molecule compounds that can interfere with the stability of one or more proteins or the enzymatic activity of one or more proteins or the binding activity of one or more proteins; zinc finger proteins that bind one or more polynucleotides; and meganucleases that have activity towards one or more polynucleotides. Gene editing technologies, genetic editing technologies and genome editing technologies are well known in the art.

One method of gene editing involves the use of transcription activator-like effector nucleases (TALENs) which induce double-strand breaks which cells can respond to with repair mechanisms. Non-homologous end joining reconnects DNA from either side of a double-strand break where there is very little or no sequence overlap for annealing. This repair mechanism induces errors in the genome via insertion or deletion, or chromosomal rearrangement. Any such errors may render the gene products coded at that location non-functional. Another method of gene editing involves the use of the bacterial CRISPR/Cas system. Bacteria and archaea exhibit chromosomal elements called clustered regularly interspaced short palindromic repeats (CRISPR) that are part of an adaptive immune system that protects against invading viral and plasmid DNA. In Type II CRISPR systems, CRISPR RNAs (crRNAs) function with trans-activating crRNA (tracrRNA) and CRISPR-associated (Cas) proteins to introduce double-stranded breaks in target DNA. Target cleavage by Cas9 requires base-pairing between the crRNA and tracrRNA as well as base pairing between the crRNA and the target DNA. Target recognition is facilitated by the presence of a short motif called a protospacer-adjacent motif (PAM) that conforms to the sequence NGG. This system can be harnessed for genome editing. Cas9 is normally programmed by a dual RNA consisting of the crRNA and tracrRNA. However, the core components of these RNAs can be combined into a single hybrid 'guide RNA' for Cas9 targeting. The use of a noncoding RNA guide to target DNA for site-specific cleavage promises to be significantly more straightforward than existing technologies - such as TALENs. Using the CRISPR/Cas strategy, retargeting the nuclease complex only requires introduction of a new RNA sequence and there is no need to reengineer the specificity of protein transcription factors.

Antisense technology is another well-known method that can be used to modulate the expression of a polypeptide. A polynucleotide of the gene to be repressed is cloned and operably linked to a regulatory region and a transcription termination sequence so that the antisense strand of RNA is transcribed. The recombinant construct is then transformed into a plant cell and the antisense strand of RNA is produced. The polynucleotide need not be the entire sequence of the gene to be repressed, but typically will be substantially complementary to at least a portion of the sense strand of the gene to be repressed.

A polynucleotide may be transcribed into a ribozyme, or catalytic RNA, that affects expression of an mRNA. Ribozymes can be designed to specifically pair with virtually any target RNA and cleave the phosphodiester backbone at a specific location, thereby functionally inactivating the target RNA. Heterologous polynucleotides can encode ribozymes designed to cleave particular mRNA transcripts, thus preventing expression of a polypeptide. Hammerhead ribozymes are useful for destroying particular mRNAs, although various ribozymes that cleave mRNA at site-specific recognition sequences can be used. Hammerhead ribozymes cleave mRNAs at locations dictated by flanking regions that form complementary base pairs with the target mRNA. The sole requirement is that the target RNA contains a 5'-UG-3' nucleotide sequence. The construction and production of hammerhead ribozymes is known in the art. Hammerhead ribozyme sequences can be embedded in a stable RNA such as a transfer RNA (tRNA) to increase cleavage efficiency *in vivo.*

In one embodiment, the sequence-specific polynucleotide that can interfere with the translation of RNA transcript(s) is interfering RNA. RNA interference or RNA silencing is an evolutionarily conserved process by which specific mRNAs can be targeted for enzymatic degradation. A double-stranded RNA (double-stranded RNA) is introduced or produced by a cell (for example, double-stranded RNA virus, or interfering RNA polynucleotides) to initiate the interfering RNA pathway. The double-stranded RNA can be converted into multiple small interfering RNA duplexes of 21-24 bp length by RNases III, which are double-stranded RNA-specific endonucleases. The small interfering RNAs can be subsequently recognized by RNA-induced silencing complexes that promote the unwinding of small interfering RNA through an ATP-dependent process. The unwound antisense strand of the small interfering RNA guides the activated RNA-induced silencing complexes to the targeted mRNA comprising a sequence complementary to the small interfering RNA anti-sense strand. The targeted mRNA and the anti-sense strand can form an A-form helix, and the major groove of the A-form helix can be recognized by the activated RNA-induced silencing complexes. The target mRNA can be cleaved by activated RNA-induced silencing complexes at a single site defined by the binding site of the 5'-end of the small interfering RNA strand. The activated RNA-induced silencing complexes can be recycled to catalyze another cleavage event.

Interfering RNA expression vectors may comprise interfering RNA constructs encoding interfering RNA polynucleotides that exhibit RNA interference activity by reducing the expression level of mRNAs, pre-mRNAs, or related RNA variants. The expression vectors may comprise a promoter positioned upstream and operably-linked to an Interfering RNA construct, as further described herein. Interfering RNA expression vectors may comprise a suitable minimal core promoter, a Interfering RNA construct of interest, an upstream (5') regulatory region, a downstream (3') regulatory region, including transcription termination and polyadenylation signals, and other sequences known to persons skilled in the art, such as various selection markers.

Various embodiments, described herein but not claimed, are directed to methods for modulating the expression level of one or more of the polynucleotide(s) described herein (or any combination thereof as described herein) by integrating multiple copies of the polynucleotide(s) into a (tobacco) plant genome, comprising: transforming a plant cell host with an expression vector that comprises a promoter operably-linked to a polynucleotide. Various compositions and methods are provided for modulating the endogenous gene expression level by modulating the translation of mRNA. A host (tobacco) plant cell can be transformed with an expression vector comprising: a promoter operably-linked to a polynucleotide, positioned in anti-sense orientation with respect to the promoter to enable the expression of RNA polynucleotides having a sequence complementary to a portion of mRNA. Various expression vectors for modulating the translation of mRNA may comprise: a promoter operably-linked to a polynucleotide in which the sequence is positioned in anti-sense orientation with respect to the promoter. The lengths of anti-sense RNA polynucleotides can vary, and may be from about 15-20 nucleotides, about 20-30 nucleotides, about 30-50 nucleotides, about 50-75 nucleotides, about 75-100 nucleotides, about 100-150 nucleotides, about 150-200 nucleotides, and about 200-300 nucleotides.

As discussed herein, the expression of one or more polypeptides can be modulated by non-transgenic means - such as creating one or more mutations in one or more genes, as discussed herein. Methods that introduce a mutation randomly in a gene sequence can include chemical mutagenesis, EMS mutagenesis and radiation mutagenesis. Methods that introduce one or more targeted mutations into a cell include but are not limited to genome editing technology, particularly zinc finger nuclease-mediated mutagenesis and targeting induced local lesions in genomes (TILLING), homologous recombination, oligonucleotide-directed mutagenesis, and meganuclease-mediated mutagenesis. In one embodiment, TILLING is used. This is a mutagenesis technology that can be used to generate and/or identify polynucleotides encoding polypeptides with modified expression and/or activity. TILLING also allows selection of plants carrying such mutants. TILLING combines high-density mutagenesis with high-throughput screening methods. Methods for TILLING are well known in the art (see McCallum et al., (2000) Nat Biotechnol 18: 455-457 and Stemple (2004) Nat Rev Genet 5(2): 145-50).

Specific mutations in polynucleotides can be created that can result in modulated gene expression, modulated stability of mRNA, or modulated stability of protein. Such plants are referred to herein as "non-naturally occurring" or "mutant" plants. Typically, the mutant or non-naturally occurring plants will include at least a portion of foreign or synthetic or man-made nucleic acid (for example, DNA or RNA) that was not present in the plant before it was manipulated. The foreign nucleic acid may be a single nucleotide, two or more nucleotides, two or more contiguous nucleotides or two or more non-contiguous nucleotides - such as at least 10, 20, 30, 40, 50,100, 200, 300, 400, 500, 600, 700, 800, 900, 1000, 1100, 1200, 1300, 1400 or 1500 or more contiguous or non-contiguous nucleotides.

The mutant or non-naturally occurring plants or plant cells can have any combination of one or more mutations in one or more genes which results in modulated protein levels. For example, the mutant or non-naturally occurring plants or plant cells may have a single mutation in a single gene; multiple mutations in a single gene; a single mutation in two or more or three or more or four or more genes; or multiple mutations in two or more or three or more or four or more genes. Examples of such mutations are described herein. By way of further example, the mutant or non-naturally occurring plants or plant cells may have one or more mutations in a specific portion of the gene(s) - such as in a region of the gene that encodes an active site of the protein or a portion thereof. By way of further example, the mutant or non-naturally occurring plants or plant cells may have one or more mutations in a region outside of one or more gene(s) - such as in a region upstream or downstream of the gene it regulates provided that they modulate the activity or expression of the gene(s). Upstream elements can include promoters, enhancers or transription factors. Some elements - such as enhancers - can be positioned upstream or downstream of the gene it regulates. The element(s) need not be located near to the gene that it regulates since some elements have been found located several hundred thousand base pairs upstream or downstream of the gene that it regulates. The mutant or non-naturally occurring plants or plant cells may have one or more mutations located within the first 100 nucleotides of the gene(s), within the first 200 nucleotides of the gene(s), within the first 300 nucleotides of the gene(s), within the first 400 nucleotides of the gene(s), within the first 500 nucleotides of the gene(s), within the first 600 nucleotides of the gene(s), within the first 700 nucleotides of the gene(s), within the first 800 nucleotides of the gene(s), within the first 900 nucleotides of the gene(s), within the first 1000 nucleotides of the gene(s), within the first 1100 nucleotides of the gene(s), within the first 1200 nucleotides of the gene(s), within the first 1300 nucleotides of the gene(s), within the first 1400 nucleotides of the gene(s) or within the first 1500 nucleotides of the gene(s). The mutant or non-naturally occurring plants or plant cells may have one or more mutations located within the first, second, third, fourth, fifth, sixth, seventh, eighth, ninth, tenth, eleventh, twelfth, thirteenth, fourteenth or fifteenth set of 100 nucleotides of the gene(s) or combinations thereof. Mutant or non-naturally occurring plants or plant cells (for example, mutant, non-naturally occurring or transgenic plants or plant cells and the like, as described herein) comprising the mutant polypeptide variants are disclosed.

In one embodiment, seeds from plants are mutagenised and then grown into first generation mutant plants. The first generation plants are then allowed to self-pollinate and seeds from the first generation plant are grown into second generation plants, which are then screened for mutations in their loci. Though the mutagenized plant material can be screened for mutations, an advantage of screening the second generation plants is that all somatic mutations correspond to germline mutations. One of skill in the art would understand that a variety of plant materials, including but not limited to, seeds, pollen, plant tissue or plant cells, may be mutagenised in order to create the mutant plants. However, the type of plant material mutagenised may affect when the plant nucleic acid is screened for mutations. For example, when pollen is subjected to mutagenesis prior to pollination of a non-mutagenized plant the seeds resulting from that pollination are grown into first generation plants. Every cell of the first generation plants will contain mutations created in the pollen; thus these first generation plants may then be screened for mutations instead of waiting until the second generation. Mutagens that create primarily point mutations and short deletions, insertions, transversions, and or transitions, including chemical mutagens or radiation, may be used to create the mutations. Mutagens include, but are not limited to, ethyl methanesulfonate, methylmethane sulfonate, N-ethyl-N-nitrosurea, triethylmelamine, N-methyl-N-nitrosourea, procarbazine, chlorambucil, cyclophosphamide, diethyl sulfate, acrylamide monomer, melphalan, nitrogen mustard, vincristine, dimethylnitrosamine, N-methyl-N'-nitro-Nitrosoguanidine, nitrosoguanidine, 2-aminopurine, 7,12 dimethyl-benz(a)anthracene, ethylene oxide, hexamethylphosphoramide, bisulfan, diepoxyalkanes (diepoxyoctane, diepoxybutane, and the like), 2-methoxy-6-chloro-9[3-(ethyl-2-chloro-ethyl)aminopropylamino]acridine dihydrochloride and formaldehyde.

Spontaneous mutations in the locus that may not have been directly caused by the mutagen are also contemplated provided that they result in the desired phenotype. Suitable mutagenic agents can also include, for example, ionising radiation - such as X-rays, gamma rays, fast neutron irradiation and UV radiation. Any method of plant nucleic acid preparation known to those of skill in the art may be used to prepare the plant nucleic acid for mutation screening. Prepared nucleic acid from individual plants, plant cells, or plant material can optionally be pooled in order to expedite screening for mutations in the population of plants originating from the mutagenized plant tissue, cells or material. One or more subsequent generations of plants, plant cells or plant material can be screened. The size of the optionally pooled group is dependent upon the sensitivity of the screening method used.

After the nucleic acid samples are optionally pooled, they can be subjected to polynucleotide-specific amplification techniques, such as Polymerase Chain Reaction. Any one or more primers or probes specific to the gene or the sequences immediately adjacent to the gene may be utilized to amplify the sequences within the optionally pooled nucleic acid sample. Suitably, the one or more primers or probes are designed to amplify the regions of the locus where useful mutations are most likely to arise. Most preferably, the primer is designed to detect mutations within regions of the polynucleotide. Additionally, it is preferable for the primer(s) and probe(s) to avoid known polymorphic sites in order to ease screening for point mutations. To facilitate detection of amplification products, the one or more primers or probes may be labelled using any conventional labelling method. Primer(s) or probe(s) can be designed based upon the sequences described herein using methods that are well understood in the art.

To facilitate detection of amplification products, the primer(s) or probe(s) may be labelled using any conventional labelling method. These can be designed based upon the sequences described herein using methods that are well understood in the art. Polymorphisms may be identified by means known in the art and some have been described in the literature.

In a further aspect there is provided but not claimed a method of preparing a mutant plant. The method involves providing at least one cell of a plant comprising a gene encoding a functional polynucleotide described herein (or any combination thereof as described herein). Next, the at least one cell of the plant is treated under conditions effective to modulate the activity of the polynucleotide(s) described herein. The at least one mutant plant cell is then propagated into a mutant plant, where the mutant plant has a modulated level of polypeptide(s) described (or any combination thereof as described herein) as compared to that of a control plant. In one embodiment of this method of making a mutant plant, the treating step involves subjecting the at least one cell to a chemical mutagenising agent as described above and under conditions effective to yield at least one mutant plant cell. In another embodiment of this method, the treating step involves subjecting the at least one cell to a radiation source under conditions effective to yield at least one mutant plant cell. The term "mutant plant" includes mutants plants in which the genotype is modified as compared to a control plant, suitably by means other than genetic engineering or genetic modification.

In certain embodiments, the mutant plant, mutant plant cell or mutant plant material may comprise one or more mutations that have occured naturally in another plant, plant cell or plant material and confer a desired trait. This mutation can be incorporated (for example, introgressed) into another plant, plant cell or plant material (for example, a plant, plant cell or plant material with a different genetic background to the plant from which the mutation was derived) to confer the trait thereto. Thus by way of example, a mutation that occurred naturally in a first plant may be introduced into a second plant - such as a second plant with a different genetic background to the first plant. The skilled person is therefore able to search for and identify a plant carrying naturally in its genome one or more mutant alleles of the genes described herein which confer a desired trait. The mutant allele(s) that occurs naturally can be transferred to the second plant by various methods including breeding, backcrossing and introgression to produce a lines, varieties or hybrids that have one or more mutations in the genes described herein. Plants showing a desired trait may be screened out of a pool of mutant plants. Suitably, the selection is carried out utilising the knowledge of the nucleotide sequences as described herein. Consequently, it is possible to screen for a genetic trait as compared to a control. Such a screening approach may involve the application of conventional nucleic acid amplification and/or hybridization techniques as discussed herein. Thus, a further aspect of the present invention relates to a method for identifying a mutant plant comprising the steps of: (a) providing a sample comprising nucleic acid from a plant; and (b) determining the nucleic acid sequence of the polynucleotide, wherein a difference in the sequence of the polynucleotide as compared to the polynucleotide sequence of a control plant is indicative that said plant is a mutant plant. In another aspect there is provided a method for identifying a mutant plant which accumulates increased or reduced levels of protease as compared to a control plant comprising the steps of: (a) providing a sample from a plant to be screened; (b) determining if said sample comprises one or more mutations in one or more of the polynucleotides described herein; and (c) determining at least the protease content of said plant during or after a curing procedure.

In another aspect there is provided but not claimed a method for preparing a mutant plant which has increased or reduced levels of protease as compared to a control plant comprising the steps of: (a) providing a sample from a first plant; (b) determining if said sample comprises one or more mutations in one or more the polynucleotides described herein that result in modulated levels of a protease; and (c) transferring the one or more mutations into a second plant. Suitably at least the protease content is determined in cured leaf material. The mutation(s) can be transferred into the second plant using various methods that are known in the art - such as by genetic engineering, genetic manipulation, introgression, plant breeding, backcrossing and the like. In one embodiment, the first plant is a naturally occurring plant. In one embodiment, the second plant has a different genetic background to the first plant.

In another aspect there is provided a method for preparing a mutant plant which has increased or reduced levels of a protease as compared to a control plant comprising the steps of: (a) providing a sample from a first plant; (b) determining if said sample comprises one or more mutations in one or more of the polynucleotides described herein that results in modulated levels of the protease; and (c) introgressing the one or more mutations from the first plant into a second plant. Suitably at least the protease content is determined in cured leaf material. In one embodiment, the step of introgressing comprises plant breeding, optionally including backcrossing and the like. In one embodiment, the first plant is a naturally occurring plant. In one embodiment, the second plant has a different genetic background to the first plant. In one embodiment, the first plant is not a cultivar or an elite cultivar. In one embodiment, the second plant is a cultivar or an elite cultivar. A further aspect relates to a mutant plant (including a cultivar or elite cultivar mutant plant) obtained or obtainable by the methods described herein. In certain embodiments, the "mutant plants" may have one or more mutations localised only to a specific region of the plant - such as within the sequence of the one or more polynucleotide(s) described herein. According to this embodiment, the remaining genomic sequence of the mutant plant will be the same or substantially the same as the plant prior to the mutagenesis.

In certain embodiments, the mutant plants may have one or more mutations localised in more than one region of the plant - such as within the sequence of one or more of the polynucleotides described herein and in one or more further regions of the genome. According to this embodiment, the remaining genomic sequence of the mutant plant will not be the same or will not be substantially the same as the plant prior to the mutagenesis. In certain embodiments, the mutant plants may not have one or more mutations in one or more, two or more, three or more, four or more or five or more exons of the polynucleotide(s) described herein; or may not have one or more mutations in one or more, two or more, three or more, four or more or five or more introns of the polynucleotide(s) described herein; or may not have one or more mutations in a promoter of the polynucleotide(s) described herein; or may not have one or more mutations in the 3' untranslated region of the polynucleotide(s) described herein; or may not have one or more mutations in the 5' untranslated region of the polynucleotide(s) described herein; or may not have one or more mutations in the coding region of the polynucleotide(s) described herein; or may not have one or more mutations in the non-coding region of the polynucleotide(s) described herein; or any combination of two or more, three or more, four or more, five or more; or six or more thereof parts thereof.

In a futher aspect there is provided but not claimed a method of identifying a plant, a plant cell or plant material comprising a mutation in a gene encoding a polynucleotide described herein comprising: (a) subjecting a plant, a plant cell or plant material to mutagenesis; (b) obtaining a nucleic acid sample from said plant, plant cell or plant material or descendants thereof; and (c) determining the nucleic acid sequence of the gene encoding a polynucleotide described herein or a variant or a fragment thereof, wherein a difference in said sequence is indicative of one or more mutations therein.

Zinc finger proteins can be used to modulate the expression or the activity of one or more of the polynucleotides described herein. In various embodiments, a genomic DNA sequence comprising a part of or all of the coding sequence of the polynucleotide is modified by zinc finger nuclease-mediated mutagenesis. The genomic DNA sequence is searched for a unique site for zinc finger protein binding. Alternatively, the genomic DNA sequence is searched for two unique sites for zinc finger protein binding wherein both sites are on opposite strands and close together, for example, 1, 2, 3, 4, 5, 6 or more basepairs apart. Accordingly, zinc finger proteins that bind to polynucleotides are provided.

A zinc finger protein may be engineered to recognize a selected target site in a gene. A zinc finger protein can comprise any combination of motifs derived from natural zinc finger DNA-binding domains and non-natural zinc finger DNA-binding domains by truncation or expansion or a process of site-directed mutagenesis coupled to a selection method such as, but not limited to, phage display selection, bacterial two-hybrid selection or bacterial one-hybrid selection. The term "non-natural zinc finger DNA-binding domain" refers to a zinc finger DNA-binding domain that binds a three-base pair sequence within the target nucleic acid and that does not occur in the cell or organism comprising the nucleic acid which is to be modified. Methods for the design of zinc finger protein which binds specific nucleotide sequences which are unique to a target gene are known in the art.

In other embodiments, a zinc finger protein may be selected to bind to a regulatory sequence of a polynucleotide. More specifically, the regulatory sequence may comprise a transcription initiation site, a start codon, a region of an exon, a boundary of an exon-intron, a terminator, or a stop codon. Accordingly, the invention provides a mutant, non-naturally occurring or transgenic plant or plant cells, produced by zinc finger nuclease-mediated mutagenesis in the vicinity of or within one or more polynucleotides described herein, and methods for making such a plant or plant cell by zinc finger nuclease-mediated mutagenesis. Methods for delivering zinc finger protein and zinc finger nuclease to a tobacco plant are similar to those described below for delivery of meganuclease.

Plants suitable for use in genetic modification but not claimed include but are not limited to, monocotyledonous and dicotyledonous plants and plant cell systems, including species from one of the following families: Acanthaceae, Alliaceae, Alstroemeriaceae, Amaryllidaceae, Apocynaceae, Arecaceae, Asteraceae, Berberidaceae, Bixaceae, Brassicaceae, Bromeliaceae, Cannabaceae, Caryophyllaceae, Cephalotaxaceae, Chenopodiaceae, Colchicaceae, Cucurbitaceae, Dioscoreaceae, Ephedraceae, Erythroxylaceae, Euphorbiaceae, Fabaceae, Lamiaceae, Linaceae, Lycopodiaceae, Malvaceae, Melanthiaceae, Musaceae, Myrtaceae, Nyssaceae, Papaveraceae, Pinaceae, Plantaginaceae, Poaceae, Rosaceae, Rubiaceae, Salicaceae, Sapindaceae, Solanaceae, Taxaceae, Theaceae, or Vitaceae.

Suitable species may include members of the genera Abelmoschus, Abies, Acer, Agrostis, Allium, Alstroemeria, Ananas, Andrographis, Andropogon, Artemisia, Arundo, Atropa, Berberis, Beta, Bixa, Brassica, Calendula, Camellia, Camptotheca, Cannabis, Capsicum, Carthamus, Catharanthus, Cephalotaxus, Chrysanthemum, Cinchona, Citrullus, Coffea, Colchicum, Coleus, Cucumis, Cucurbita, Cynodon, Datura, Dianthus, Digitalis, Dioscorea, Elaeis, Ephedra, Erianthus, Erythroxylum, Eucalyptus, Festuca, Fragaria, Galanthus, Glycine, Gossypium, Helianthus, Hevea, Hordeum, Hyoscyamus, Jatropha, Lactuca, Linum, Lolium, Lupinus, Lycopersicon, Lycopodium, Manihot, Medicago, Mentha, Miscanthus, Musa, Nicotiana, Oryza, Panicum, Papaver, Parthenium, Pennisetum, Petunia, Phalaris, Phleum, Pinus, Poa, Poinsettia, Populus, Rauwolfia, Ricinus, Rosa, Saccharum, Salix, Sanguinaria, Scopolia, Secale, Solanum, Sorghum, Spartina, Spinacea, Tanacetum, Taxus, Theobroma, Triticosecale, Triticum, Uniola, Veratrum, Vinca, Vitis, and Zea.

Suitable species may include Panicum spp., Sorghum spp., Miscanthus spp., Saccharum spp., Erianthus spp., Populus spp., Andropogon gerardii (big bluestem), Pennisetum purpureum (elephant grass), Phalaris arundinacea (reed canarygrass), Cynodon dactylon (bermudagrass), Festuca arundinacea (tall fescue), Spartina pectinata (prairie cord-grass), Medicago sativa (alfalfa), Arundo donax (giant reed), Secale cereale (rye), Salix spp. (willow), Eucalyptus spp. (eucalyptus), Triticosecale (tritic wheat times rye), bamboo, Helianthus annuus (sunflower), Carthamus tinctorius (safflower), Jatropha curcas (jatropha), Ricinus communis (castor), Elaeis guineensis (palm), Linum usitatissimum (flax), Brassica juncea, Beta vulgaris (sugarbeet), Manihot esculenta (cassaya), Lycopersicon esculentum (tomato), Lactuca sativa (lettuce), Musyclise alca (banana), Solanum tuberosum (potato), Brassica oleracea (broccoli, cauliflower, Brussels sprouts), Camellia sinensis (tea), Fragaria ananassa (strawberry), Theobroma cacao (cocoa), Coffe31ycliseca (coffee), Vitis vinifera (grape), Ananas comosus (pineapple), Capsicum annum (hot & sweet pepper), Allium cepa (onion), Cucumis melo (melon), Cucumis sativus (cucumber), Cucurbita maxima (squash), Cucurbita moschata (squash), Spinacea oleracea (spinach), Citrullus lanatus (watermelon), Abelmoschus esculentus (okra), Solanum melongena (eggplant), Rosa spp. (rose), Dianthus caryophyllus (carnation), Petunia spp. (petunia), Poinsettia pulcherrima (poinsettia), Lupinus albus (lupin), Uniola paniculata (oats), bentgrass (Agrostis spp.), Populus tremuloides (aspen), Pinus spp. (pine), Abies spp. (fir), Acer spp. (maple), Hordeum vulgare (barley), Poa pratensis (bluegrass), Lolium spp. (ryegrass) and Phleum pratense (timothy), Panicum virgatum (switchgrass), Sorghu32yclise32or (sorghum, sudangrass), Miscanthus giganteus (miscanthus), Saccharum sp. (energycane), Populus balsamifera (poplar), Zea mays (corn), Glycine max (soybean), Brassica napus (canola), Triticum aestivum (wheat), Gossypium hirsutum (cotton), Oryza sativa (rice), Helianthus annuus (sunflower), Medicago sativa (alfalfa), Beta vulgaris (sugarbeet), or Pennisetum glaucum (pearl millet).

Various embodiments are directed to mutant tobacco, non-naturally occurring tobacco or transgenic tobacco plants or plant cells modified to modulate gene expression levels thereby producing a plant or plant cell - such as a tobacco plant or plant cell - in which the expression level of a polypeptide is modulated within tissues of interest as compared to a control. The disclosed compositions and methods can be applied to any species of the genus *Nicotiana,* including *N. rustica* and *N. tabacum* (for example, LA B21, LN KY171, TI 1406, Basma, Galpao, Perique, Beinhart 1000-1, and Petico). Other species include *N. acaulis, N. acuminata, N. africana, N. alata, N. ameghinoi, N. amplexicaulis, N. arentsii, N. attenuata, N. azambujae, N. benavidesii, N. benthamiana, N. bigelovii, N. bonariensis, N. cavicola, N. clevelandii, N. cordifolia, N. corymbosa, N. debneyi, N. excelsior, N. forgetiana, N. fragrans, N. glauca, N. glutinosa, N. goodspeedii, N. gossei, N. hybrid, N. ingulba, N. kawakamii, N. knightiana, N. langsdorffii, N. linearis, N. longitlora, N. maritima, N. megalosiphon, N. miersii, N. noctiflora, N. nudicaulis, N. obtusifolia, N. occidentalis, N. occidentalis subsp. hesperis, N. otophora, N. paniculata, N. pauciflora, N. petunioides, N. plumbaginifolia, N. quadrivalvis, N. raimondii, N. repanda, N. rosulata, N. rosulata subsp. ingulba, N. rotundifolia, N. setchellii, N. simulans, N. solanifolia, N. spegazzinii, N. stocktonii, N. suaveolens, N. sylvestris, N. thyrsiflora, N. tomentosa, N. tomentosiformis, N. trigonophylla, N. umbratica, N. undulata, N. velutina, N. wigandioides, and N. x sanderae.*

The use of tobacco cultivars and elite tobacco cultivars is also contemplated herein. The transgenic, non-naturally occurring or mutant plant may therefore be a tobacco variety or elite tobacco cultivar that comprises one or more transgenes, or one or more genetic mutations or a combiantion thereof. The genetic mutation(s) (for example, one or more polymorphisms) can be mutations that do not exist naturally in the individual tobacco variety or tobacco cultivar (for example, elite tobacco cultivar) or can be genetic mutation(s) that do occur naturally provided that the mutation does not occur naturally in the individual tobacco variety or tobacco cultivar (for example, elite tobacco cultivar).

Particularly useful *Nicotiana tabacum* varieties include Burley type, dark type, flue-cured type, and Oriental type tobaccos. Non-limiting examples of varieties or cultivars are: BD 64, CC 101, CC 200, CC 27, CC 301, CC 400, CC 500, CC 600, CC 700, CC 800, CC 900, Coker 176, Coker 319, Coker 371 Gold, Coker 48, CD 263, DF911, DT 538 LC Galpao tobacco, GL 26H, GL 350, GL 600, GL 737, GL 939, GL 973, HB 04P, HB 04P LC, HB3307PLC, Hybrid 403LC, Hybrid 404LC, Hybrid 501 LC, K 149, K 326, K 346, K 358, K394, K 399, K 730, KDH 959, KT 200, KT204LC, KY10, KY14, KY 160, KY 17, KY 171, KY 907, KY907LC, KY14xL8 LC, Little Crittenden, McNair 373, McNair 944, msKY 14xL8, Narrow Leaf Madole, Narrow Leaf Madole LC, NBH 98, N-126, N-777LC, N-7371LC, NC 100, NC 102, NC 2000, NC 291, NC 297, NC 299, NC 3, NC 4, NC 5, NC 6, NC7, NC 606, NC 71, NC 72, NC 810, NC BH 129, NC 2002, Neal Smith Madole, OXFORD 207, PD 7302 LC, PD 7309 LC, PD 7312 LC, 'Perique' tobacco, PVH03, PVH09, PVH19, PVH50, PVH51, R 610, R 630, R 7-11, R 7-12, RG 17, RG 81, RG H51, RGH 4, RGH 51, RS 1410, Speight 168, Speight 172, Speight 179, Speight 210, Speight 220, Speight 225, Speight 227, Speight 234, Speight G-28, Speight G-70, Speight H-6, Speight H20, Speight NF3, TI 1406, TI 1269, TN 86, TN86LC, TN 90, TN 97, TN97LC, TN D94, TN D950, TR (Tom Rosson) Madole, VA 309, VA359, AA 37-1, B13P, Xanthi (Mitchell-Mor), Bel-W3, 79-615, Samsun Holmes NN, KTRDC number 2 Hybrid 49, Burley 21, KY8959, KY9, MD609, PG01, PG04, PO1, PO2, PO3, RG11, RG 8, VA509, AS44, Banket A1, Basma Drama B84/31, Basma I Zichna ZP4/B, Basma Xanthi BX 2A, Batek, Besuki Jember, C104, Coker 347, Criollo Misionero, Delcrest, Djebel 81, DVH 405, Galpão Comum, HB04P, Hicks Broadleaf, Kabakulak Elassona, Kutsage E1, LA BU 21, NC 2326, NC 297, PVH 2110, Red Russian, Samsun, Saplak, Simmaba, Talgar 28, Wislica, Yayaldag, Prilep HC-72, Prilep P23, Prilep PB 156/1, Prilep P12-2/1, Yaka JK-48, Yaka JB 125/3, TI-1068, KDH-960, TI-1070, TW136, Basma, TKF 4028, L8, TKF 2002, GR141, Basma xanthi, GR149, GR153, Petit Havana. Low converter subvarieties of the above, even if not specifically identified herein, are also contemplated.

Embodiments described herein but not claimed, are also directed to compositions and methods for producing mutant plants, non-naturally occurring plants, hybrid plants, or transgenic plants that have been modified to modulate the expression or activity of a polynucleotide(s) described herein (or any combination thereof as described herein). Advantageously, the mutant plants, non-naturally occurring plants, hybrid plants, or transgenic plants that are obtained may be similar or substantially the same in overall appearance to control plants. Various phenotypic characteristics such as degree of maturity, number of leaves per plant, stalk height, leaf insertion angle, leaf size (width and length), internode distance, and lamina-midrib ratio can be assessed by field observations.

One aspect relates to a seed of a mutant plant, a non-naturally occurring plant, a hybrid plant or a transgenic plant described herein. Preferably, the seed is a tobacco seed. A further aspect relates to pollen or an ovule of a mutant plant, a non-naturally occurring plant, a hybrid plant or a transgenic plant that is described herein. In addition, there is provided a mutant plant, a non-naturally occurring plant, a hybrid plant or a transgenic plant as described herein which further comprises a nucleic acid conferring male sterility.

Also provided is a tissue culture of regenerable cells of the mutant plant, non-naturally occurring plant, hybrid plant, or transgenic plant or a part thereof as described herein, which culture regenerates plants capable of expressing all the morphological and physiological characteristics of the parent. The regenerable cells include but are not limited to cells from leaves, pollen, embryos, cotyledons, hypocotyls, roots, root tips, anthers, flowers and a part thereof, ovules, shoots, stems, stalks, pith and capsules or callus or protoplasts derived therefrom.

A still further aspect, relates to a cured plant material - such as cured leaf or cured tobacco - derived or derivable from a mutant, non-naturally occurring or transgenic plant or cell, wherein expression of one or more of the polynucleotides described herein or the activity of the protein encoded thereby is modulated.

Suitably the visual appearance of said plant (for example, leaf) is substantially the same as the control plant. Suitably, the plant is a tobacco plant.

Embodiments are also directed to compositions and methods for producing mutant, non-naturally occurring or transgenic plants or plant cells that have been modified to modulate the expression or activity of the one or more of the polynucleotides or polypeptides described herein which can result in plants or plant components (for example, leaves - such as green leaves or cured leaves - or tobacco) or plant cells with modulated levels of proteases.

In another aspect, there is provided but not claimed a method for modulating (*eg*. increasing) the amount of protease in at least a part of a plant (for example, the leaves - such as cured leaves - or in tobacco), comprising the steps of: (i) modulating (*eg*. increasing) the expression or activity of an one or more of the polypeptides described herein (or any combination thereof as described herein), suitably, wherein the polypeptide(s) is encoded by the corresponding polynucleotide sequence described herein; (ii) measuring the protease content in at least a part (for example, the leaves - such as cured leaves - or tobacco or in smoke) of the mutant, non-naturally occurring or transgenic plant obtained in step (i); and (iii) identifying a mutant, non-naturally occurring or transgenic plant in which the protease content therein has been modulated (*eg*. increased) in comparison to a control plant. Suitably, the visual appearance of said mutant, non-naturally occurring or transgenic plant is substantially the same as the control plant. Suitably, the plant is a tobacco plant.

In another aspect, there is provided but not claimed a method for modulating (*eg.* increasing) the amount of protease in at least a part of cured plant material - such as cured leaf - comprising the steps of: (i) modulating (*eg*. increasing) the expression or activity of an one or more of the polypeptides (or any combination thereof as described herein), suitably, wherein the polypeptide(s) is encoded by the corresponding polynucleotide sequence described herein; (ii) harvesting plant material - such as one or more of the leaves - and curing for a period of time; (iii) measuring the protease content in at least a part of the cured plant material obtained in step (ii) or during step (ii); and (iv) identifying cured plant material in which the protease content therein has been modulated (eg. increased) in comparison to a control plant. An increase in expression as compared to the control may be from about 5 % to about 100 %, or an increase of at least 10 %, at least 20 %, at least 25 %, at least 30 %, at least 40 %, at least 50 %, at least 60 %, at least 70 %, at least 75 %, at least 80 %, at least 90 %, at least 95 %, at least 98 %, or 100 % or more - such as 200%, 300%, 500%, 1000% or more, which includes an increase in transcriptional activity or polynucleotide expression or polypeptide expression or a combination thereof.

An increase in activity as compared to a control may be from about 5 % to about 100 %, or an increase of at least 10 %, at least 20 %, at least 25 %, at least 30 %, at least 40 %, at least 50 %, at least 60 %, at least 70 %, at least 75 %, at least 80 %, at least 90 %, at least 95 %, at least 98 %, or 100 % or more - such as 200%, 300%, 500%, 1000% or more.

A reduction in expression as compared to a control may be from about 5 % to about 100 %, or a reduction of at least 10 %, at least 20 %, at least 25 %, at least 30 %, at least 40 %, at least 50 %, at least 60 %, at least 70 %, at least 75 %, at least 80 %, at least 90 %, at least 95 %, at least 98 %, or 100 %, which includes a reduction in transcriptional activity or polynucleotide expression or polypeptide expression or a combination thereof.

A reduction in activity as compared to a control may be from about 5 % to about 100 %, or a reduction of at least 10 %, at least 20 %, at least 25 %, at least 30 %, at least 40 %, at least 50 %, at least 60 %, at least 70 %, at least 75 %, at least 80 %, at least 90 %, at least 95 %, at least 98 %, or 100 %.

Polynucleotides and recombinant constructs described herein can be used to modulate the expression of the proteases described herein in a plant species of interest, suitably tobacco. A number of polynucleotide based methods can be used to increase gene expression in plants and plant cells. By way of example, a construct, vector or expression vector that is compatible with the plant to be transformed can be prepared which comprises the gene of interest together with an upstream promoter that is capable of overexpressing the gene in the plant or plant cell. Exemplary promoters are described herein. Following transformation and when grown under suitable conditions, the promoter can drive expression in order to modulate (for example, reduce) the levels of this enzyme in the plant, or in a specific tissue thereof. In one exemplary embodiment, a vector carrying one or more polynucleotides described herein (or any combination thereof as described herein) is generated to overexpress the gene in a plant or plant cell. The vector carries a suitable promoter - such as the cauliflower mosaic virus CaMV 35S promoter - upstream of the transgene driving its constitutive expression in all tissues of the plant. The vector also carries an antibiotic resistance gene in order to confer selection of the transformed calli and cell lines.

In a preferred embodiment, a promoter and regulatory sequences are derived from one or more of SEQ ID Nos: 1-80. These regulatory sequences can be used in conjunction with cognate or non-cognate expression sequences to increase expression of said sequences in a tobacco plant during the curing procedure.

The expression of sequences from promoters can be enhanced by including expression control sequences, including enhancers, chromatin activating elements, transcription factor responsive elements and the like. Such control sequences may be constitutive, and upregulate transcription in a universal manner; or they may be facultative, and upregulate transcription in response to specific signals. Signals associated with senescence and signals which are active during the curing procedure are specifically indicated.

Various embodiments, described but not claimed herein, are therefore directed to methods for modulating (for example, increasing) the expression level of one or more polynucleotides described herein (or any combination thereof as described herein) by integrating multiple copies of the polynucleotide into a plant genome, comprising: transforming a plant cell host with an expression vector that comprises a promoter operably-linked to one or more polynucleotides described herein. The polypeptide encoded by a recombinant polynucleotide can be a native polypeptide, or can be heterologous to the cell.

A tobacco plant carrying a mutant allele of one or more polynucleotides described herein (or any combination thereof as described herein) can be used in a plant breeding program to create useful lines, varieties and hybrids. In particular, the mutant allele is introgressed into the commercially important varieties described above. Thus, methods for breeding plants are provided but not claimed, that comprise crossing a mutant plant, a non-naturally occurring plant or a transgenic plant as described herein with a plant comprising a different genetic identity. The method may further comprise crossing the progeny plant with another plant, and optionally repeating the crossing until a progeny with the desirable genetic traits or genetic background is obtained. One purpose served by such breeding methods is to introduce a desirable genetic trait into other varieties, breeding lines, hybrids or cultivars, particularly those that are of commercial interest. Another purpose is to facilitate stacking of genetic modifications of different genes in a single plant variety, lines, hybrids or cultivars. Intraspecific as well as interspecific matings are contemplated. The progeny plants that arise from such crosses, also referred to as breeding lines, are examples of non-naturally occurring plants of the invention.

In one embodiment, a method is provided but not claimed for producing a non-naturally occurring tobacco plant comprising: (a) crossing a mutant or transgenic tobacco plant with a second tobacco plant to yield progeny tobacco seed; (b) growing the progeny tobacco seed, under plant growth conditions, to yield the non-naturally occurring tobacco plant. The method may further comprises: (c) crossing the previous generation of non-naturally occurring tobacco plant with itself or another tobacco plant to yield progeny tobacco seed; (d) growing the progeny tobacco seed of step (c) under plant growth conditions, to yield additional non-naturally occurring tobacco plants; and (e) repeating the crossing and growing steps of (c) and (d) multiple times to generate further generations of non-naturally occurring tobacco plants. The method may optionally comprises prior to step (a), a step of providing a parent plant which comprises a genetic identity that is characterized and that is not identical to the mutant or transgenic plant. In some embodiments, depending on the breeding program, the crossing and growing steps are repeated from 0 to 2 times, from 0 to 3 times, from 0 to 4 times, 0 to 5 times, from 0 to 6 times, from 0 to 7 times, from 0 to 8 times, from 0 to 9 times or from 0 to 10 times, in order to generate generations of non-naturally occurring tobacco plants. Backcrossing is an example of such a method wherein a progeny is crossed with one of its parents or another plant genetically similar to its parent, in order to obtain a progeny plant in the next generation that has a genetic identity which is closer to that of one of the parents. Techniques for plant breeding, particularly tobacco plant breeding, are well known and can be used in the methods of the invention. The invention further provides non-naturally occurring tobacco plants produced by these methods. Certain emboiments exclude the step of selecting a plant.

In some embodiments of the methods described herein but not claimed, lines resulting from breeding and screening for variant genes are evaluated in the field using standard field procedures. Control genotypes including the original unmutagenized parent are included and entries are arranged in the field in a randomized complete block design or other appropriate field design. For tobacco, standard agronomic practices are used, for example, the tobacco is harvested, weighed, and sampled for chemical and other common testing before and during curing. Statistical analyses of the data are performed to confirm the similarity of the selected lines to the parental line. Cytogenetic analyses of the selected plants are optionally performed to confirm the chromosome complement and chromosome pairing relationships.

DNA fingerprinting, single nucleotide polymorphism, microsatellite markers, or similar technologies may be used in a marker-assisted selection (MAS) breeding program to transfer or breed mutant alleles of a gene into other tobaccos, as described herein. For example, a breeder can create segregating populations from hybridizations of a genotype containing a mutant allele with an agronomically desirable genotype. Plants in the F2 or backcross generations can be screened using a marker developed from a genomic sequence or a fragment thereof, using one of the techniques listed herein. Plants identified as possessing the mutant allele can be backcrossed or self-pollinated to create a second population to be screened. Depending on the expected inheritance pattern or the MAS technology used, it may be necessary to self-pollinate the selected plants before each cycle of backcrossing to aid identification of the desired individual plants. Backcrossing or other breeding procedure can be repeated until the desired phenotype of the recurrent parent is recovered.

In a breeding program, successful crosses yield F1 plants that are fertile. Selected F1 plants can be crossed with one of the parents, and the first backcross generation plants are self-pollinated to produce a population that is again screened for variant gene expression (for example, the null version of the the gene). The process of backcrossing, self-pollination, and screening is repeated, for example, at least 4 times until the final screening produces a plant that is fertile and reasonably similar to the recurrent parent. This plant, if desired, is self-pollinated and the progeny are subsequently screened again to confirm that the plant exhibits variant gene expression. In some embodiments, a plant population in the F2 generation is screened for variant gene expression, for example, a plant is identified that fails to express a polypeptide due to the absence of the gene according to standard methods, for example, by using a PCR method with primers based upon the nucleotide sequence information for the polynucleotide(s) described herein (or any combination thereof as described herein).

Hybrid tobacco varieties can be produced by preventing self-pollination of female parent plants (that is, seed parents) of a first variety, permitting pollen from male parent plants of a second variety to fertilize the female parent plants, and allowing F1 hybrid seeds to form on the female plants. Self-pollination of female plants can be prevented by emasculating the flowers at an early stage of flower development. Alternatively, pollen formation can be prevented on the female parent plants using a form of male sterility. For example, male sterility can be produced by cytoplasmic male sterility (CMS), or transgenic male sterility wherein a transgene inhibits microsporogenesis and/or pollen formation, or self-incompatibility. Female parent plants containing CMS are particularly useful. In embodiments in which the female parent plants are CMS, pollen is harvested from male fertile plants and applied manually to the stigmas of CMS female parent plants, and the resulting F1 seed is harvested.

Varieties and lines described herein can be used to form single-cross tobacco F1 hybrids. In such embodiments, the plants of the parent varieties can be grown as substantially homogeneous adjoining populations to facilitate natural cross-pollination from the male parent plants to the female parent plants. The F1 seed formed on the female parent plants is selectively harvested by conventional means. One also can grow the two parent plant varieties in bulk and harvest a blend of F1 hybrid seed formed on the female parent and seed formed upon the male parent as the result of self-pollination. Alternatively, three-way crosses can be carried out wherein a single-cross F1 hybrid is used as a female parent and is crossed with a different male parent. As another alternative, double-cross hybrids can be created wherein the F1 progeny of two different single-crosses are themselves crossed.

A population of mutant, non-naturally occurring or transgenic plants can be screened or selected for those members of the population that have a desired trait or phenotype. For example, a population of progeny of a single transformation event can be screened for those plants having a desired level of expression or activity of the polypeptide(s) encoded thereby. Physical and biochemical methods can be used to identify expression or activity levels. These include Southern analysis or PCR amplification for detection of a polynucleotide; Northern blots, S1 RNase protection, primer-extension, or RT-PCR amplification for detecting RNA transcripts; enzymatic assays for detecting enzyme or ribozyme activity of polypeptides and polynucleotides; and protein gel electrophoresis, Western blots, immunoprecipitation, and enzyme-linked immunoassays to detect polypeptides. Other techniques such as in situ hybridization, enzyme staining, and immunostaining and enzyme assays also can be used to detect the presence or expression or activity of polypeptides or polynucleotides.

Mutant, non-naturally occurring or transgenic plant cells and plants are described herein comprising one or more recombinant polynucleotides, one or more polynucleotide constructs, one or more double-stranded RNAs, one or more conjugates or one or more vectors/expression vectors.

As described herein but not claimed, the plants described herein may be modified for other purposes either before or after the expression or activity has been modulated according to the present invention. One or more of the following genetic modifications can be present in the mutant, non-naturally occurring or transgenic plants. In one embodiment, one or more genes that are involved in the conversion of nitrogenous metabolic intermediates is modified resulting in plants (such as leaves) that when cured, produces lower levels of at least one tobacco-specific nitrosamine than control plants. Non-limiting examples of genes that can be modified includegenes encoding a nicotine demethylase, such as *CYP82E4, CYP82E5* and *CYP82E10* which participate in the conversion of nicotine to nornicotine and are described in WO2006091194, WO2008070274, WO2009064771 and PCT/US2011/021088 and as described in detail herein. In another embodiment, one or more genes that are involved in heavy metal uptake or heavy metal transport are modified resulting in plants or parts of plants (such as leaves) having a lower heavy metal content than control plants or parts thereof without the modification(s). Non-limiting examples include genes in the family of multidrug resistance associated proteins, the family of cation diffusion facilitators (CDF), the family of Zrt-, Irt-like proteins (ZIP), the family of cation exchangers (CAX), the family of copper transporters (COPT), the family of heavy-metal P-type ATPases (for example, HMAs, as described in WO2009074325), the family of homologs of natural resistance-associated macrophage proteins (NRAMP), and the family of ATP-binding cassette (ABC) transporters (for example, MRPs, as described in WO2012/028309, which participate in transport of heavy metals, such as cadmium. The term heavy metal as used herein includes transition metals. Examples of other modifications include herbicide tolerance, for example, glyphosate is an active ingredient of many broad spectrum herbicides. Glyphosate resistant transgenic plants have been developed by transferring the *aroA* gene (a glyphosate EPSP synthetase from *Salmonella typhimurium* and *E.coli*)*.* Sulphonylurea resistant plants have been produced by transforming the mutant ALS (acetolactate synthetase) gene from Arabidopsis. OB protein of photosystem II from mutant *Amaranthus hybridus* has been transferred in to plants to produce atrazine resistant transgenic plants; and bromoxynil resistant transgenic plants have been produced by incorporating the *bxn* gene from the bacterium *Klebsiella pneumoniae.* Another exemplary modification results in plants that are resistant to insects. *Bacillus thuringiensis* (Bt) toxins can provide an effective way of delaying the emergence of Bt-resistant pests, as recently illustrated in broccoli where pyramided *cry1Ac* and *cry1C* Bt genes controlled diamondback moths resistant to either single protein and significantly delayed the evolution of resistant insects. Another exemplary modification results in plants that are resistant to diseases caused by pathogens (for example, viruses, bacteria, fungi). Plants expressing the *Xa21* gene (resistance to bacterial blight) with plants expressing both a Bt fusion gene and a chitinase gene (resistance to yellow stem borer and tolerance to sheath) have been engineered. Another exemplary modification results in altered reproductive capability, such as male sterility. Another exemplary modification results in plants that are tolerant to abiotic stress (for example, drought, temperature, salinity), and tolerant transgenic plants have been produced by transferring acyl glycerol phosphate enzyme from Arabidopsis; genes coding mannitol dehydrogenase and sorbitol dehydrogenase which are involved in synthesis of mannitol and sorbitol improve drought resistance. Other exemplary modifications can result in plants with improved storage proteins and oils, plants with enhanced photosynthetic efficiency, plants with prolonged shelf life, plants with enhanced carbohydrate content, and plants resistant to fungi; plants encoding an enzyme involved in the biosynthesis of alkaloids. Transgenic plants in which the expression of S-adenosyl-L-methionine (SAM) and/or cystathionine gamma-synthase (CGS) has been modulated are also contemplated.

One or more such traits, as described herein but not claimed, may be introgressed into the mutant, non-naturally occuring or transgenic tobacco plants from another tobacco cultivar or may be directly transformed into it. The introgression of the trait(s) into the mutant, non-naturally occuring or transgenic tobacco plants of the invention maybe achieved by any method of plant breeding known in the art, for example, pedigree breeding, backcrossing, doubled-haploid breeding, and the like (see, Wernsman, E. A, and Rufty, R. C. 1987. Chapter Seventeen. Tobacco. Pages 669-698 In: Cultivar Development. Crop Species. W. H. Fehr (ed.), MacMillan Publishing Co, Inc., New York, N.Y 761 pp.). Molecular biology-based techniques described above, in particular RFLP and microsatelite markers, can be used in such backcrosses to identify the progenies having the highest degree of genetic identity with the recurrent parent. This permits one to accelerate the production of tobacco varieties having at least 90%, preferably at least 95%, more preferably at least 99% genetic identity with the recurrent parent, yet more preferably genetically identical to the recurrent parent, and further comprising the trait(s) introgressed from the donor parent. Such determination of genetic identity can be based on molecular markers known in the art.

The last backcross generation can be selfed to give pure breeding progeny for the nucleic acid(s) being transferred. The resulting plants generally have essentially all of the morphological and physiological characteristics of the mutant, non-naturally occuring or transgenic tobacco plants of the invention, in addition to the transferred trait(s) (for example, one or more single gene traits). The exact backcrossing protocol will depend on the trait being altered to determine an appropriate testing protocol. Although backcrossing methods are simplified when the trait being transferred is a dominant allele, a recessive allele may also be transferred. In this instance, it may be necessary to introduce a test of the progeny to determine if the desired trait has been successfully transferred.

Various embodiments provide mutant plants, non-naturally occurring plants or transgenic plants, as well as biomass in which the expression level of a polynucleotide (or any combination thereof as described herein) is modulated to modulate the protease activity therein.

Parts of such plants, particularly tobacco plants, and more particularly the leaf lamina and midrib of tobacco plants, can be incorporated into or used in making various consumable products including but not limited to aerosol forming materials, aerosol forming devices, smoking articles, smokable articles, smokeless products, and tobacco products. Examples of aerosol forming materials include but are not limited to tobacco compositions, tobaccos, tobacco extract, cut tobacco, cut filler, cured tobacco, expanded tobacco, homogenized tobacco, reconstituted tobacco, and pipe tobaccos. Smoking articles and smokable articles are types of aerosol forming devices. Examples of smoking articles or smokable articles include but are not limited to cigarettes, cigarillos, and cigars. Examples of smokeless products comprise chewing tobaccos, and snuffs. In certain aerosol forming devices, rather than combustion, a tobacco composition or another aerosol forming material is heated by one or more electrical heating elements to produce an aerosol. In another type of heated aerosol forming device, an aerosol is produced by the transfer of heat from a combustible fuel element or heat source to a physically separate aerosol forming material, which may be located within, around or downstream of the heat source. Smokeless tobacco products and various tobacco-containing aerosol forming materials may contain tobacco in any form, including as dried particles, shreds, granules, powders, or a slurry, deposited on, mixed in, surrounded by, or otherwise combined with other ingredients in any format, such as flakes, films, tabs, foams, or beads. As used herein, the term 'smoke' is used to describe a type of aerosol that is produced by smoking articles, such as cigarettes, or by combusting an aerosol forming material.

In one embodiment, there is also provided cured plant material from the mutant, transgenic and non-naturally occurring tobacco plants described herein. Processes of curing green tobacco leaves are known by those having skills in the art and include without limitation air-curing, fire-curing, flue-curing and sun-curing as described herein.

In another embodiment, there is described tobacco products including tobacco-containing aerosol forming materials comprising plant material - such as leaves, preferably cured leaves - from the mutant tobacco plants, transgenic tobacco plants or non-naturally occurring tobacco plants described herein. The tobacco products described herein can be a blended tobacco product which may further comprise unmodified tobacco.

The mutant, non-naturally occurring or transgenic plants may have other unclaimed uses in, for example, agriculture. For example, mutant, non-naturally occurring or transgenic plants described herein can be used to make animal feed and human food products.

Herein are described but not claimed methods for producing seeds comprising cultivating the mutant plant, non-naturally occurring plant, or transgenic plant described herein, and collecting seeds from the cultivated plants. Seeds from plants described herein can be conditioned and bagged in packaging material by means known in the art to form an article of manufacture. Packaging material such as paper and cloth are well known in the art. A package of seed can have a label, for example, a tag or label secured to the packaging material, a label printed on the package that describes the nature of the seeds therein.

Compositions, methods and kits for genotyping plants for identification, selection, or breeding can comprise a means of detecting the presence of a polynucleotide (or any combination thereof as described herein) in a sample of polynucleotide. Accordingly, a composition is described herein but not claimed, comprising one of more primers for specifically amplifying at least a portion of one or more of the polynucleotides and optionally one or more probes and optionally one or more reagents for conducting the amplification or detection.

Accordingly, gene specific oligonucleotide primers or probes comprising about 10 or more contiguous polynucleotides corresponding to the polynucleotide(s) described herein are disclosed but not claimed. Said primers or probes may comprise or consist of about 15, 20, 25, 30, 40, 45 or 50 more contiguous polynucleotides that hybridise (for example, specificially hybridise) to the polynucleotide(s) described herein. In some embodiments, the primers or probes may comprise or consist of about 10 to 50 contiguous nucleotides, about 10 to 40 contiguous nucleotides, about 10 to 30 contiguous nucleotides or about 15 to 30 contiguous nucleotides that may be used in sequence-dependent methods of gene identification (for example, Southern hybridization) or isolation (for example, *in situ* hybridization of bacterial colonies or bacteriophage plaques) or gene detection (for example, as one or more amplification primers in nucleic acid amplification or detection). The one or more specific primers or probes can be designed and used to amplify or detect a part or all of the polynucleotide(s). By way of specific example, two primers may be used in a polymerase chain reaction protocol to amplify a nucleic acid fragment encoding a nucleic acid - such as DNA or RNA. The polymerase chain reaction may also be performed using one primer that is derived from a nucleic acid sequence and a second primer that hybridises to the sequence upstream or downstream of the nucleic acid sequence - such as a promoter sequence, the 3' end of the mRNA precursor or a sequence derived from a vector. Examples of thermal and isothermal techniques useful for *in vitro* amplification of polynucleotides are well known in the art. The sample may be or may be derived from a plant, a plant cell or plant material or a tobacco product made or derived from the plant, the plant cell or the plant material as described herein.

In a further aspect, there is also provided but not claimed, a method of detecting a polynucleotide(s) described herein (or any combination thereof as described herein) in a sample comprising the step of: (a) providing a sample comprising, or suspected of comprising, a polynucleotide; (b) contacting said sample with one of more primers or one or more probes for specifically detecting at least a portion of the polynucleotide(s); and (c) detecting the presence of an amplification product, wherein the presence of an amplification product is indicative of the presence of the polynucleotide(s) in the sample. In a further aspect, there is also provided but not claimed the use of one of more primers or probes for specifically detecting at least a portion of the polynucleotide(s). Kits for detecting at least a portion of the polynucleotide(s) are also provided but not claimed. Said kits comprise one of more primers or probes for specifically detecting at least a portion of the polynucleotide(s). The kit may comprise reagents for polynucleotide amplification - such as PCR - or reagents for probe hybridization-detection technology - such as Southern Blots, Northern Blots, in-situ hybridization, or microarray. The kit may comprise reagents for antibody binding-detection technology such as Western Blots, ELISAs, SELDI mass spectrometry or test strips. The kit may comprise reagents for DNA sequencing. The kit may comprise reagents and instructions for determining at least the proteasae content. Suitably, the kit comprises reagents and instructions for determining at least protease content in plant material, cured plant material or cured leaves.

In some embodiments described herein but not claimed, a kit may comprise instructions for one or more of the methods described. The kits described may be useful for genetic identity determination, phylogenetic studies, genotyping, haplotyping, pedigree analysis or plant breeding particularly with co-dominant scoring.

The present invention also provides but does not claim a method of genotyping a plant, a plant cell or plant material comprising a polynucleotide as described herein. Genotyping provides a means of distinguishing homologs of a chromosome pair and can be used to differentiate segregants in a plant population. Molecular marker methods can be used for phylogenetic studies, characterizing genetic relationships among crop varieties, identifying crosses or somatic hybrids, localizing chromosomal segments affecting monogenic traits, map based cloning, and the study of quantitative inheritance. The specific method of genotyping may employ any number of molecular marker analytic techniques including amplification fragment length polymorphisms (AFLPs). AFLPs are the product of allelic differences between amplification fragments caused by nucleotide sequence variability. Thus, the present invention further provides a means to follow segregation of one or more genes or nucleic acids as well as chromosomal sequences genetically linked to these genes or nucleic acids using such techniques as AFLP analysis.

In one embodiment, there is also provided but not claimed cured plant material from the mutant, transgenic and non-naturally occurring plants described herein. For example, processes of curing tobacco leaves are known by those having skills in the field and include without limitation air-curing, fire-curing, flue-curing and sun-curing.

In another embodiment, there is described tobacco products including tobacco products comprising plant material - such as leaves, suitably cured plant material - such as cured leaves - from the mutant, transgenic and non-naturally occurring plants described herein or which are produced by the methods described herein. The tobacco products described herein may further comprise unmodified tobacco.

In another embodiment, there is described tobacco products comprising plant material, preferably leaves - such as cured leaves, from the mutant, transgenic and non-naturally occurring plants described herein. For example, the plant material may be added to the inside or outside of the tobacco product and so upon burning a desirable aroma is released. The tobacco product according to this embodiment may even be an unmodified tobacco or a modified tobacco. The tobacco product according to this embodiment may even be derived from a mutant, transgenic or non-naturally occurring plant which has modifications in one or more genes other than the genes disclosed herein.

The invention is further described in the Examples below, which are provided to describe the invention in further detail. These examples, which set forth a preferred mode presently contemplated for carrying out the invention, are intended to illustrate and not to limit the invention.

### EXAMPLES

The following examples are provided as an illustration and not as a limitation. Unless otherwise indicated, the present invention employs conventional techniques and methods of molecular biology, plant biology, bioinformatics, and plant breeding.

### Example 1

A 48h time-point following the curing start was selected to screen for curing-activated genes based on Affymetrix data essentially as described by Martin et al. (2012) BMC Genomics, 13:674).

In brief, exon candidates from genomic DNA and from EST contigs were joined and the genomic candidates were cleaned for redundancies (98% threshold). This resulted in a set of 312,053 exon candidates, 12,925 of which were represented by ESTs, but were not included in the genome assembly. Data sets were verified as described by the manufacturer (Affymetrix). In addition, quality checks included probe-level models, Normalized Unscaled Standard Error (NUSE) and Relative Log Expression (RLE) plots, and the analysis of DABG results as described by the manufacturer.

As the exon array design had no mismatch probes, summarization was performed using Robust Multi-array Average (RMA) method. A total of 272,342 probeset expression values were generated, and DABG P-values were computed to assess the significance of the signal obtained for each probeset. This involved the background probes that are spread over the chip. These random probes have a varying GC content. Quality checks involved a combination of Affymetrix Power Tools (APT) and Bioconductor packages, for which the Tobacco Exon Array (TobArray520623F) cdf environment was created. Once the expression values were available, differential gene expression analysis was performed using moderated t-statistics in linear model LIMMA.

### Example 2

***Differential expression.*** The tissue samples were sequenced using RNA-seq; reads were mapped to the genomes of the 3 varieties using Tophat2. Previously published gene models were used as the basis for the differential gene expression analysis. Expression changes during curing were calculated using the Cuffdiff2 software based on the mapped reads. Genes were considered up-regulated if their expression levels increased significantly during the first 48h of curing, and not if the change was insignificant or decreased. Tobacco proteins were identified by a BLAST search against a database of transcripts for the 3 varieties and equivalent genes in the 3 varieties were identified by a mutual best BLAST hit search of the transcripts of the 3 varieties Burley, Virginia and Oriental (e-value cutoff 1e-80).

The data (Figure 2) shows the number of senescence-activated genes in the 3 cured varieties.

### Example 3

The proteasae genes identified in Example 2 were analysed for membership of known protease families. The results are set forth in table 1.

The 80 curing-activated protease genes were found to belong to 21 different protease families.

In the table, AC, air-cured; FC, flue-cured; SC, sun-cured. AC+FC+SC, up-regulated in all three types of tobacco ; AC+FC, up-regulated in air-cured and flue-cured tobacco ; AC+SC, up-regulated in air-cured and sun-cured tobacco ; FC+SC, up-regulated in flue-cured and sun-cured tobacco ; AC, FC and SC, up-regulated only in the respective tobacco type.

**Table 1.**

| | | | | | | | |
|---|---|---|---|---|---|---|---|
| | | | | | | | |

| **Protease coding genes** | **AC+FC+S C** | **AC+F C** | **AC+S C** | **FC+S C** | **AC** | **FC** | **SC** |
|---|---|---|---|---|---|---|---|
| Alpha/beta-Hydrolases superfamily protein | - | - | - | - | 1 | - | - |
| Aspartic proteinase A1 (APA1) | 2 | 1 | - | - | 2 | 1* | - |
| CLP protease/crotonase family protein | 1 | - | - | - | 1 | - | - |
| Cysteine proteinases superfamily protein | 3 | 2 | 3 | 1 | 2 | 1 | 2 |
| DegP protease 3 | - | - | - | - | - | 1 | - |
| Eukaryotic aspartyl protease family protein | 4 | 1 | 1 | 1 | 3 | 2 | 2 |
| FTSH protease 8 | - | - | - | - | - | - | 1 |
| Gamma-glutamyl transpeptidase 4 | - | 1 | - | - | - | - | - |
| Heat shock protein 101 | 1 | 1 | - | - | 1 | 1 | - |
| Ion protease 1 & 3 | - | - | - | - | - | 3 | - |
| Metallopeptidase M24 family protein | - | - | - | - | - | 1 | - |
| Papain family cysteine protease | 1 | - | - | - | - | - | - |
| Peptidase M20/M25/M40 family protein | - | 1 | - | - | - | - | 1 |
| Protease-related | - | - | - | - | 1 | 2 | - |
| SAG 12 | - | 1 | - | - | - | - | - |
| Serine carboxypeptidase-like | 1 | 1 | 1 | - | 1 | 1 | 1 |
| SERPIN | - | 1 | 1 | - | - | 1 | - |
| Signal peptide peptidase | - | - | - | - | 1 | - | - |
| SITE-1 protease | - | 1 | | - | - | - | - |
| Subtilisin-like ser endopeptidase fam prot. | 3 | 1 | | - | 1 | 3 | - |
| Ubiquitin-specific proteases | - | - | - | 1 | 3 | 2 | - |
| **Total** | **16** | **12** | **6** | **3** | **17** | **19** | **7** |
| | | | | | | | |

### Example 4

*APA1* is encoded by a single gene in *Arabidopsis thaliana* and 4 in Tomato. The *APA1* gene activated in flue-cured Virginia tobacco (see Table 1) is close to *APA1-Tomato-1.* Two gene copies from both ancestors *N. sylvestris* (S) and *N. tomentosiformis* (T) exist in *N. tabacum.* Affymetrix data confirmed the activation of the S form (upper panel) and apparently not the T form during Virginia flue-curing (lower panel).

### Example 5

Table 2 illustrates the differential up-regulation of SEQ ID NO:1 to 80 in the three tobacco types air-cured Burley (AC), flue-cured Virginia (FC) and sun-cured Oriental (SC).

**Table 2**

| **SEQ ID NO:** | **AC** | **FC** | **SC** | **AC-FC** | **AC-SC** | **FC-SC** | **AC-FC-SC** |
|---|---|---|---|---|---|---|---|
| **1** | | | | | | | **x** |
| **2** | | | | | | | **x** |
| **3** | | | | | | | **x** |
| **4** | | | | | | | **x** |
| **5** | | | | | | | **x** |
| **6** | | | | | | | **x** |
| **7** | | | | | | | **x** |
| **8** | | | | | | | **x** |
| **9** | | | | | | | **x** |
| **10** | | | | | | | **x** |
| **11** | | | | | | | **x** |
| **12** | | | | | | | **x** |
| **13** | | | | | | | **x** |
| **14** | | | | | | | **x** |
| **15** | | | | | | | **x** |
| **16** | | | | | | | **x** |
| **17** | | | | | **x** | | |
| **18** | | | | | **x** | | |
| **19** | | | | | **x** | | |
| **20** | | | | | **x** | | |
| **21** | | | | | **x** | | |
| **22** | | | | | **x** | | |
| **23** | | | **x** | | | | |
| **24** | | | **x** | | | | |
| **25** | | | **x** | | | | |
| **26** | | | **x** | | | | |
| **27** | | | **x** | | | | |
| **28** | | | **x** | | | | |
| **29** | | | **x** | | | | |
| **30** | | | | **x** | | | |
| **31** | | | | **x** | | | |
| **32** | | | | **x** | | | |
| **33** | | | | **x** | | | |
| **34** | | | | **x** | | | |
| **35** | | | | **x** | | | |
| **36** | | | | **x** | | | |
| **37** | | | | **x** | | | |
| **38** | | | | **x** | | | |
| **39** | | | | **x** | | | |
| **40** | | | | **x** | | | |
| **41** | | | | **x** | | | |
| **42** | | | | | | **x** | |
| **43** | | | | | | **x** | |
| **44** | | | | | | **x** | |
| **45** | **x** | | | | | | |
| **46** | **x** | | | | | | |
| **47** | **x** | | | | | | |
| **48** | **x** | | | | | | |
| **49** | **x** | | | | | | |
| **50** | **x** | | | | | | |
| **51** | **x** | | | | | | |
| **52** | **x** | | | | | | |
| **53** | **x** | | | | | | |
| **54** | **x** | | | | | | |
| **55** | **x** | | | | | | |
| **56** | **x** | | | | | | |
| **57** | **x** | | | | | | |
| **58** | **x** | | | | | | |
| **59** | **x** | | | | | | |
| **60** | **x** | | | | | | |
| **61** | **x** | | | | | | |
| **62** | | **x** | | | | | |
| **63** | | **x** | | | | | |
| **64** | | **x** | | | | | |
| **65** | | **x** | | | | | |
| **66** | | **x** | | | | | |
| **67** | | **x** | | | | | |
| **68** | | **x** | | | | | |
| **69** | | **x** | | | | | |
| **70** | | **x** | | | | | |
| **71** | | **x** | | | | | |
| **72** | | **x** | | | | | |
| **73** | | **x** | | | | | |
| **74** | | **x** | | | | | |
| **75** | | **x** | | | | | |
| **76** | | **x** | | | | | |
| **77** | | **x** | | | | | |
| **78** | | **x** | | | | | |
| **79** | | **x** | | | | | |
| **80** | | **x** | | | | | |

### SEQUENCE LISTING

SEQ ID NO: 1 
ATGGCTCTTCGTTTCTCTTTAATTTTCCTATTTCTCTTTTCTTAACGACGTCGTTATTGTT
GTCCGTTAACGGCAACATTAACGGCGGTGAAGATGACGATATTTTGATCCGTCAAGTCG
TAGGCGACGACGACGATCACTTGTTAAACGCCGATCATCACTTCACGATTTTTAAGAGG
AGGTTCGGCAAAACCTACGCGTCCGATGAGGAGCATCATTACAGATTCTCGGTGTTCAA
GGCTAACTTGCGCCGTGCAATGCGCCACCAGAAGCTTGATCCCTCCGCCGTTCACGGT
GTGACTCAGTTTTCCGATTTGACTCCGGCCGAGTTCCGCCGGAATTTTCTAGGAGTTAA
CCGTCGGCTCCGGCTTCCTTCTGATGCCAATAAAGCTCCTATTCTTCCTACTGAGGATC
TCCCTTCAGGTTTCGATTGGAGAGATCACGGTGCCGTCACGTCAGTAAAGAATCAGGTA
CTAGTATATATCAATGTTTGTGTAAAGTTTATCTTTTTTTGGATAGGCGAAGTGTTCGTCA
TTAATGAATAATTACATAATTTCTATTTGTATCGATTGAAAAACTAGGGTTCATGTGGCTC
GTGCTGGTCATTTAGTACCACTGGTGCGTTAGAAGGTGCCACCTATCTTTCTACAGGGA
AGCTTGTAAGCCTCAGCGAGCAACAACTTGTGGACTGTGATCACGAGGTTTGACGTTCT
TCCTCTTTATCTTAGCTTAAAATCATGAATATATTGTCAATAGAGTTACTGTTTTTCTTTTT
TCTTTTTTTCTGGACGTTTGAATGTGTAAAATAATTTCGCTGTGGTGTGTCACAGGAT
TTGGTCCATAGCTGTCATCTTTTTCTAGTTAAAGAAAATTGATAGCGTGAAGGACACTAA
CCGCATAAATTAAAGTGCTTTCTCTGATTCCGTCTCACTTTAAAGTTTAAGAACCCGTTT
GGCCATGAAATTTCTTTTTTTTTTCCGTAAAATTTAACTTTTCTTCTAAATCAATGTTTGGC
CATCAAATTTTTTATTTTCACTTGAAGATAATTTTACAATTTTTCAAAAATTTGAAAAACTT
CAAAAACTGTTTTTCAAAATTTTGAATATTGTTGTTGATGTAAAAAACAGACACTAATTTA
TAAGAGTAATCTCCTCTTCTTTGTTTGGTGGATGGCCAGGGGTGGGGACTGGGGACCC
ATCTTAAGGGAGCGGAGGAAAAGTTGTTTTATTATTAGTTTATGGCTGGTTATGAAATTC
AACTAATTGATACTCTGAGGATAACCACGGACAAAATTGTTTGGATGATGAGGAAATCG
CATCCAAAAATTGTCTGCATCTGAATATACTTTTAACATTACTTGAAGTTTCAAGTTTAAG
CTCGTGTATGCAACGTGGTGGGAGATGTACAAGGATAAATAGAAAGGCGTTGAGTTATT
GAGATAGGTTTGTAAAACTCTTCTTAAATTTTCCATTGTTTGATTGCCATTATATAATCAT
TTGTATAATTTCCAACTTGGAAAAAGCTGTTCAAACTCAAAATAAGGTTTAGGCTTGAAC
TTATTGCTATTTACGGTGTCTGCCATTTTATAATCAGAAATGGGATTGAATACAGAGTTA
ATAAGACCACTGACTCGCCTTATTTACCTCACTCGTCTCAGATGAATTTTATACTTCCAA
ATTTCAGTGTTCCCCATCTCCCTGAAAAATGTATAATTTGGCCTTGCATTTATCTGCAGT
GTGATCCAGAAGAAAAAGATTCATGTGACGCAGGGTGCAATGGTGGCCTAATGAATAG
TGCCTTTGAATACACTCTGAAAGCTGGTGGACTTATGCGAGAAGAAGATTATCCATACA
CTGGCACCGATCGTGGAACCTGCAAATTTGACAACACCAAGGTTGCTGCTAAAGTTGCT
AACTTTAGCGTTGTCTCCCTTGACGAAGAACAAATCGCTGCTAATCTTGTCAAGAATGG
TCCTCTCGCTGGTAAATAGTCTCTCAAAACACTTTTCAATTTGCCTATCATTATGCTTCTT
CTTTGTCCTTACTTGATATTGTCAAAGTATATACTTGGATTGTCATATTTATGCACTGGAA
TGTAAAAGGTATTTACACAATTAAGTCACTTATTAGGTAATTACAAGTAACTATTTTGATA
AGTTTTAATTAGTAATGTGTTAAAATGATAATTAACTTGCTATTTAAATTCACTGATAGCC
GTAACAAAATCTTTTAACTATTAATATATATAATATAAATATTTGTTTTTAATAAACAACA
AATATTATTTGTGAAAGATCCAGTTATGTAGCTTGAAACTACATTTTGGGATTTTGAATTA
TGTACTACTCTTCTTATGCTAATGGTTTTCAATTTTTCACTGATGTAAACTTCTGAAAGCA
TTTTTGTTGCTTGGCTTGCAGTGGCGATCAATGCAGTGTTCATGCAGACATACGTTGGC
GGAGTTTCCTGCCCATATATATGCTCTAAGAAGTTGGATCATGGTGTCTTATTAGTTGGT
TATGGTACTGGCTTTTCTCCCATTAGAATGAAAGAGAAACCATACTGGATCATCAAGAAC
TCATGGGGAGAGAAATGGGGTGAAAACGGATACTACAAAATCTGTAGAGGCCGCAATG
TTTGCGGAGTGGATTCAATGGTTTCAACAGTTTCAGCTGTTAGTACCAGCTCACAC
SEQ2 
TTAAGCTGCTTCAGCAAATCCAACTCTGAGTTTGCCATAATCGAAGACTGTGTGATATC
GACCCATGAAAACATCACCCAAGATCCTGTAACCAAAGGAATACCATAGAGAACTCAGT
GAACAAAAGAACTGCAGGCTCAGGTTTAATTGTGCTGTAGCTCTATAGTTCGGATTAAA
CTTATATTTGGATTAACTGCATTGCTGATATTTATCTCTAAAACATAATTATAAACTAAAAT
AGAGAGAACATATAAAGATAACTTTACCAGAGTGGTCCGCGGGAGGAGGAATGTCCA
AGCCAGTGAAACCACTAATACACTGTGCCTTAGCACCCTCGCCCACCTTGAGTATGTAC
TGATCACGGGAAAAGAATCCAAAATTAGAACATAGATCAATCTAGGTCAGCAATCTAAA
CAGACAACTGAAACAAGTAAAAGGGAACCACACATCGGATTACAACTTCACTCTTTCAA
TCTTGAAAAAATTTGTTGAAAGGGTGGGAAAAGACTAGAGTGATAGTCTAGTAGAGAAA
AGTTTTGCATATGGTCAAGGGGTTTGGTGTATCACTTGGATTTTTTCCTTTGTAAGATGT
GGTCTATCCTGAATTATTCAAAGCTCAACCTCTTTATGTTACTAAACCACAAAACAAACA
AATTCAGAAAAAATGCAAATGATCAAATTGATTTGGTGTACACTTGATGAATTTCTTCTTT
GTAAGATTTGTTCTATACTGAATCCTTCAGACATAAAAAAAAATATTTTTTTTTGGGGGG
NCGGCCTGAATTAGCAAGGTCAGCAAGTAATACACTTCCATAAAAATAGCAAAGGGTAA
CTTTTTCACGGCACAAAGATCTTATGCAGGTTTTCTTAGATTACTTAGCTGGAAAATGAG
ACATCTAAATTTAAGTAAAGTCGAAATACTCACAACCTCAAAATATAGAAGTACTTCTTGA
TGACAACAAACATCTACTTCTCTGTAGAAACTGAAAACCTTAAACACTAGAATCGGTTTT
GTAATATGACAATTAGTTGTAATGCCACAAAAGGACTCTATGATGAGCCACTTAATTTTT
TCTCTCTTTGACAATGTTGAATTAGAAGAGGAATAGCAATGTTTATTACTGTCAAAGACC
ATTATAAAGCATACCTCCTTCGGGACGAGGTCAAAAACTTTGCCACCAATTGTGAAAGA
GACTGTAGGCATTGAAGAAAGCTTTCCACAGTCAACAGCTGATTCCCCCAATGGGCTTG
GGAGACGCTCGCAAAGCTGTCAGTCAGTCAGCAATCTTTTCAAGAAAAGAAGAAAATTG
CAGTGACAGATGTTTTACCTCATTCACATAGTTTAATATGCGATCTTGAGTCTGGTTTTG
TCTCAGTTGATTCTCCATCCATATGACCGCCATTTCACAAGCAGAGCACATACCATCCT
GCAGTCCTGTGGATCTGCCAGCTTTCTCGTCTACAACACTCTCAATTCCCATACTGCAG
AAAAAAGGCCACAGAATTATTCAGTATTTATATCAACATTATGGATTAACCAAATGCACT
ATATGTTCACATGAAGGGGCAAAGAGAGCCTGAAGACTAACCTAACTCCGCGGTTTCCA
TCGAAAGTGCATACTCCAACCTGTGAGCAAATCTTCTTTGGATGTGCCTGTTTAAATACT
GACCAATTAGATAACCGGGAAAGGCAACTAGATTGCCAAGTGTCATTTTGCTGTAACTG
CACAGGAAACTGCATATCAAACAAATGAAAATGCAGTTACACAGTTGAATGCTCACCTC
TGCTAACAGCAAATCCATGATTGTCTGCCCGTACTGCTCCACTACAGATTTGCATTGTT
GGCTAGCAACTCCAGAGGCTCCAATGGCTTGATTAATCATAGTGATTATGGTCTGTACG
GAAAGGGGTGGGTTTAAGATTGCTCAACCTTGGAAGTGTTTTAATCGTACAATTGTAGA
GACAAAAGGCAGCAGATTTTTACTTAATTTATATTGTCAACATTTCCAAGCCAACAGGAT
AAAACTTGGCTACAGTTTTCGGGTTGGATAATTTTCTTTTCAAATAGAAGAGGGGTAAAT
AAATAAGTCGACAGAAGACCAGGACTACAGCAGAAGTAAAAGCATCATCCTCATTGAAA
CGTAATAAAAGCAAGTAACACAAAACAACAAGTACCTACTGAGGCAGCTTTAAACATATT
AAACTGAAAGACAGGGAAGAAAAAGCAGATTTACAGACTTCGGCCCAGTGATAAGCTAA
GATGGTATATCCAAAGGTAACCTCAGAAATGAAAAACCAATTTCACTACCATCCTCTCTG
TGATGAAAAATTAAAACACAACACAGATCAGATGATGGATTCGTGCTATTTAACTCATGA
ATCTTAGGAAAATGTTACTTTTCTTGCTGAGCTGTTGAAGGTTCAAAGGAACAAGGAAAT
CAATAATCGAATTGCGGTTGACTTTGATGATGCAAACAAATAACAAAAACATAACAAACA
AGCGATATGTCCCAAATCAAGGCTATAGATAATACCGTTGGACCAGCCAAGAGAGAAGT
CCCTGAATCCGCTATTGCAGAGCACCCACTTTCACAGTAACCTACACCCGATATGTTCA
TGTTAAAAACTCAAAGGAAGGGAAAATTCTATATCCAGGCACATAGCCTTCATCTATATT
CCCGAAATTCGGCAATCCAATTCAAAAGGTACATAGCAAAACATACCAGTAGCTTTACC
CTCGATAAGAACATCACCCATATCAAACTGCCAATCATATTACTAAGATCAAGATAGCAT
TTGTACAAAAAAATGAACATACATAGTATCGAATTGACCGAATGACAAACCTGCCAATAAC
 
CTTTGTGTGTGACTGGGACATAAGTGATTTCTCCCTTATAGTGATTAGGATCAACCCCA
CCAAACACGATTTCTCCGCCTTGTTCTTCCTCTGTATTTCGGTTGAGCCAAAATGAGAA
GACAGGATCCTTGATAAGACCCTGTTGGACCATGTTGTACCTGGAAAAGACAGGAGAT
GCTGCCCAGATGAATGTCAAATCAAATTTAAACAGAAAGAGACATCCAGCCTATCCTGC
ATTTATGGAAATCTAATCCTTCAATGTGTTAAACCTCTTCTGGAAAGGAAATTGTCTAGA
GCTTTAATTTGGTTTGTGGGAAAGAAATAGAGCAAACTAAATACCGCCCACGTACCAAA
CTGGAACAGCATTGCCAACTGAAATCTCCTGGAATCCAAGACCCAATATACCGTCAAAC
TTGGCTACCAAAAATGTCACGCTGGGTTCTCTGGTTGCCTCAATAAATTCCTAGTACAT
GAACACCTTGAGATATAAGATTTCCACTTTCAAGAGATTTAAAACAAGTGAGGAGCCTCA
CTAACCTGATCTGTTACAACAAGGTCACCAACTTTGACGTTGTCTTGACTGAAGAATCCA
GAAATAGCTCCACTACCATACTGAATTGCAGCAGACTTCCCTGTATGGCAAATCAAAAA
TTTATCACGAACTAAATCACATTAAATTACAATGCCAAATACGATCTCAGTCTTGTGGAA
ACATTCGATAAGATCTTAATGTTGTTCATTAAGGTAGGAGTGACCTAGTGTTCTTAAAAG
CAAAACGTGCAAAAAAATAAAACAAGGTCCACGGACTTGCATTGAATTGCGAGAAGTGA
AGCGCAAAATTAACAGGAAACAAGAAAATATCGAACATTTATGAATTTACTCTACCATAA
AAATTAAACTGCAGGTTAAATAACTAATTTCGGCATCCAATATACAATAATCCCAGTATTA
ATTCAACTCCTCAAAATTGAGATTCAAAGAAGCAACCAATTCTAGTTGGAATCACTTTGT
GCACCATTATTTGAAGCGCAACTTCTCTAAAGCGCATGGCTTCAGCAATGAAGTGATAG
CCCTTGCTGCATCGCTTCATAACTTTAAACGACCAAGCAATGGCTTTCAATAACACTGG
AGTGAACTCACCTGGCAAACCCATTCAACTGCTATAGACTGTTCAATCCATTTTCTTTGA
GCAACATATATAATTGTAATAGAACAAAAAATAAAGAATAACTAGTTCTCTGCGGAAAAT
TTCTTATGTCACAGACCTACATGGATACAAACCGAGTTAATAGGGAAGAAGAAAGACCA
TCTAAAAAGGCATTGCATAGGTTAAGACTTAAGACTATACAAGGTGCAACGAAAAGGCA
CTAATCGCAGAGAGATATAAGGATATTGATGTTTCTTTTCCAAAACCTCACTAGTTACAG
TAATATACTAAGAAACACAACATAAACATTAAACAGCCTCGTTTTATGTCTTAACAGTCAA
CTACATGTACTCGTCAATTAACCTTTCCAAGGGAATCCCTTGATGCACCGTGAGAAACA
CATAAGGACAATACAAAAGATGTTCCATAATGAACAAGATGGCACGTATTCTAAACAATA
ACAGGCATTAGAAGGAAGCATATGTTTCATGCAGCAATAAACAAGCAAATGGTAGAGAG
AAACAATTTGCATCAACATACAGAAATGGAAACATAACATACCATTCTTCTTATAAGTACT
TGATTCGCTTGATTTGAACTTGGAATGAAAGAAACAGGGAACCTACACCAAGATAGGCA
GTCATCAAATTTTACATCACTCAAGATGGATGTACAATGCTATGCTTTGTATCATTTGCAT
GTATAGAAGCTTACAGAGAAATAGCACTTCGACGACGGCACCCACAAATTCGAGCTACC
AGTGTCAAAGATTACAGTGAACTTCTGAGGTGGAGTGCCTACACCAATCTCCCCAAAAT
ATTGAGCATCCATATAGTTCTTCAGTGCTACAATGTCTGTATCCTCAGAGTCCCCGAGTT
TACCACGGAAGTTATACTTCCTAATAGACGCCCTCAAAACGTCCCCTTCCTTTGACTCAA
TGCGTGCAGCAAGCCGGTTATTTTGATCAAATTTCATTTTTTTCAAGCCAATTCTCATCA
AGCCATCATTGGATGAGGAGGCCAAAGGAAAGAGCAGTGCTGAGAGAAACAGGGCAA
CAAGAAATACTTTTGCTCCCAT
SEQ 3 
ATGGGTTCTTTCCTCTGTTTCTCCGTCATTGTTGTTCTCCTTGTTCTTCAGCCATGTTTA
GCCAAGAAAGTTTACATTGTTCACATGAAAAATCACCAAATACCTTCTTCTTTTGCTACC
CATCACGATTGGTACAATGCTCAGCTCCAATCTTTGTCCTCTTCTTCTACCTCTGATGAA
TCATCCCTTCTTTACTCTTACGACACTGCTTATTCTGGCTTTGCTGCTTCTCTTGACCCA
CATGAAGCTGAACTACTCCGTCAATCTGATGATGTTGTTGGAGTTTACGAGGATACTGT
TTATACACTCCATACAACAAGGACTCCTGAGTTTCTGGGGTTGAATAATGAGCTCGGCC
TTTGGGCTGGTCACAGTCCACAGGAACTCAACAACGCTGCTCAGGATGTTGTTATCGG
AGTTCTTGACACCGGCGTTTGGCCGGAGTCGAAGAGCTATAACGATTTCGGTATGCCC
GATGTGCCGTCGAGGTGGAAGGGTGAATGTGAATCGGGTTCCGATTTCGATCCGAAAG
 
TACATTGCAACAAAAGCTGATAGGTGCTCGTTTTTCTCCAAAGGTTATCAAATGTCGG
CCTCTGGCTCGTTCACGAACCAACCTAGACAGCCGGAGTCACCTCGTGACCAAGACGG
TCATGGCACCCACACATCCAGCACCGCCGCTGGTGCACCTGTGGCGAACGCTAGCCTT
CTCGGGTACGCTAGTGGGGTCGCGCGTGGTATGGCACCTCGAGCGCGTGTAGCTACG
TACAAGGTATGCTGGCCTACTGGTTGTTTTGGTTCTGATATTCTAGCTGGTATGGAACG
TGCTATTTTAGATGGAGTTGATGTACTTTCATTATCTTTGGGTGGTGGATCGGGTCCTTA
TTATCGTGATACAATTGCTATTGGTGCTTTCTCTGCTATGGAAAAAGGAATTGTTGTTTC
CTGTTCAGCTGGAAATAGCGGTCCAGCTAAAGGCTCACTTGCAAATACAGCTCCTTGGA
TCATGACCGTTGGTGCTGGTACCATAGATCGTGATTTCCCTGCATTTGCTACTTTAGGT
AACGGAAAAAATTACCGGAGTTTCGTTATACAGTGGAAAAGGAATGGGTAAAAAGGT
AGTTCCATTAGTTTACAGCACAGACAGTAGTGCAAGTCTTTGTTTGCCGGGTTCACTTG
ACCCGAAAATGGTCCGAGGGAAAATAGTGTTATGTGATAGAGGGACAAATGCGAGAGT
AGAAAAGGGTTTAGTAGTGAAGGAAGCTGGTGGAGTTGGGATGATATTGGCTAATACG
GCGGAGAGCGGCGAGGAATTGGTGGCGGATAGTCATTTGTTGCCGGCGGTAGCTGTA
GGTAGGAAATTGGGAGATTTTATAAGGCAGTATGTAAAGAGTGAAAAGAATCCGGCCG
CCGTGCTCAGCTTTGGTGGGACGGTGGTGAATGTGAAACCGTCGCCGGTGGTGGCTG
CGTTTAGTTCAAGAGGGCCCAATACTGTAACTCCACAGATTTTGAAGCCCGATGTTATT
GGGCCTGGAGTTAATATTTTGGCTGCTTGGTCTGAGGCTATTGGGCCCACTGGCTTG
AAAAGGATACCAGAAGGACCAAGTTCAACATCATGTCTGGTAAGTATTACCAACAACGG
CTAGTTTCTTAATTTAATCTTTTTCATGCTTAGCTTAATTATGGCCTTAATTATATTTTTAT
TAGATCTCGCAATTATTAATACTAACCGTACACACTTAAAAAGGAAAAGAGGAACGCGTA
GAATAAAGACACCTGTGGGTGATCTGGAATTATGTACTATGCACATTCCTAAACTTTAGA
GGGGTTCACATGTGTAGCATTGATAAGTTAATCCTAAATTACATTAGTTATAATTAAATAT
TAATGCAGTTTCCAAGAAAATAGATGGACTAAAATTTAGACTTATTTGTATGATGTGACG
TGTGGAATTAAATTTAAAAACTGCCCAAGCCTATATCAAATTTATGGCTAAAATAGCAAG
AAACGTCCCTTTAATAGGCACAGAAGAAATCCAAGAGGGGCTCGCTGTAGGAGTGTTA
AGAGTTTCGATATGAACAAGGTCTAGAGAAGAATTTATTAATTAATTTCAATAATATACGC
TAATGGTATTTGAAAACAATATATTGTAATTTATCGTAACAAGTTACTAATTTCGCTTATTA
TAGACCATTATTGTGAAGTTATTTCTATAGATAAGCCAATAGCATAAAATTCATCCGTCG
GAATGTGCAAGGTGTAGTGGTAGGAGTGCTACTCATGATGTGACAAGTGCATGTCACG
GGGTTTGAATCATAATGCAAACAAAAGCCTGATATGTTAGTGAAAAATGATAGAGGGAC
GGGTTCATTATTCACACAAAAGCTTGATATTTAAGTGAAAAATGATAGGGGAACGAGTTC
ATTATCCAAAGAGTTTCGAACCTAACCTTACACCATGGCCCTTCTTGGTATAATTTACAC
TAGTTTTATGAGGCTCCTTTTTGTCTCACAAATTTGTGGATCCCAATCTTACACTTCTGG
GTCCACAAAATTGTGAGACAAAAAAGTTACCTCATACAACTAGTCTCGGTTGTGACTAGT
TGTATGAGACACAAAATAAAATTTTCCGAAAAAGTAGTATGGTCTGTCTTCCGCTATGAG
ACTAGTTGTTTGAAACACAAAATAAAATTTTCCGAAAAAGTAGTATCGTCTGTCTTCCAC
TAGTGGGTCCTGGTCCCCTTGGAATCCCAGATTATTGGTCCCTACATAACTATAAAGGT
CATAACCTTATCATGGATTTAACATCAACCCTTTGCCCCATCTGAGCACTCTGGACCTAC
CTTAATCACTTTATTGGCTGGAAATAAGTTGATGAACTTTTTGAATTTTTCTTGAAAAAC
AACAACAAAAAACCACTTGTGATCCCACAAGTGGGTCCGGGGATTAGTGTGTTATAAAG
AGGATGTTTCGGATAGACTTTCGGCTTAGGAAAGATCAATAAAGTAGTAGAAACAAGCA
ATAACAATAGCAAAATACTGAATTTTTCTTGAAAATCCTACACAAATCTCATACTTTGAAA
ATTGTATTTTGTTACATAATTTGATCATTTTTCACTTCGAACTCTTGTAGGCACATCCATG
TCCTGTCCTCATATCAGTGGCCTAGCTGCACTGCTGAAAGCAGCACATCCTGAATGGA
GTCCAAGCGCGATCAAATCTGCACTTATGACGACTGCCTATGTTCGCGACACCACCAAC
TCTCCTCTCCGCGACGCTGAAGGTGGCCAACTCTCCACTCCTTGGGCTCATGGATCAG
GTCATGTTGATCCCCATAAGGCACTTTCCCCCGGTCTAATCTATGATATTACCCCAGAG
GACTACATCAAATTCTTATGCTCCTTGGACTATGAGTTGAACCACATACAAGCCATTGTC
AAGCGCCCGAATGTCACTTGTACTAAGAAATTTGCAGATCCTGGGCAGATTAACTACCC
TTCATTCTCAGTTTTGTTCGGGAAATCAAGGGTTGTTCGTTACACCCGTGCAGTCATCAA
 
TGTAGGAGCTGCAGGATCCGTCTATGAGGTGACCGTTGATGCTCCCCCGTCTGTTACT
GTAACCGTGAAGCCATCAAAACTTGTATTCAAAAGGGTAGGAGAGAGGCTGCGTTACA
CCGTTACATTCGTGTCAAAGAAGGGTGTTAACATGATGAGAAAGAGTGCATTTGGCTCC
ATTTCTTGGAATAATGCTCAAAACCAAGTTAGGAGTCCAGTTTCATATTCCTGGTCACAA
CTATTAGAC
SEQ 4 
TCAAGCATCAGCACATCTTGTTGGTGCATATCCCAGTCTGGACCTTTTGGTGTCATATAA
GATATGAAAATTCTGCTGCTGATAGTTTCCAATTATCGACAAAGCAGATCGAGGAGTCC
CTAAAACTGCCAAACAAACGATATCCTCTGGTTCGAGTTTGATAAAGTAGTTCTCTACTG
GAAAATTCCATACAGCTCCATCACCAAACACGATCCCAAACGAGGGAAATTCCAAGTTC
TTCACACCAGACACATTGTAACACGGATTCAAAATAGGAAAGTCTTGTACAATGGGATAT
CCCTTAACCTTATTGACAAATGCCTCTTTTATAATCTCATAAGCAGGATCCGCGAAATAA
CTCAATGTGGTACCTGAATCAATGATTGCACCACCAAGACCTTCTAGCGATAAATTCCA
CGTCTCCTCGGGTATATTCAGTACCTCTCCTCCAACTATGACAGACTTTATCTGCACATA
GTAGAATGTTTCCACTTCTTTGCCTCCAACCAATGAAGTAAAATTCAACTGTGGATGTTT
CAAAAGTTCCTTATCTTCACCAAAAATCAACTTACTACTAACACTAGAATTGCTATTCCTA
TCAACAAGACAATACGAAAACGAATGACCATATAAAGATTGAAGCTGAGAAGCAAACGA
AAGCGGCCCTCTCCCTAATCCTAACAAACCAGCAGCACCATGAAATAATCCTCTATTCC
AATGACCACAACCAAACATCACATTTTCCACCTTCCTAAATTCACTCCCACTCGTCGTCG
TGAGGTTAACAGTAAATGTCTCTAGCGCGAAATCGCCAGTAGTATTAGAACTATCACCA
TACCAATAGTAATAAGGACAAGTTTGATTCTCGGATTTACAAAGCTGAGGAGGATCAGG
GGATGTAACAAATTTACACCTAGGATCATGACAACTTATATTTCTAAATGAAGTAGAGTC
TTGAGGATTATAATGAGGTCCATTTTGTTCAAAACAATCAAAACAAGGAACACATTGAAT
CCAATTAAGATCACTACCAGTATCAAGAATTAAAGAAAAATGCTTAGGTGGTGTACCAAC
AAACACATCCATAAAATACTCACCAGAGCCAAGGCTTACACCTGACTCCAAAGTCGCCA
TTAGTTTGCCGGAAAGTTCATAAGATTCCAGCGAAACTGCTGCCGGAGCAATCACAGG
CTTATGTTTGTCCACATGTTTTTCATTACTTTTTGCAAGTCTTGAATTGTAATTCTGATTTT
TCTTCTCAACAATTCTTGTATGGAGTGTCTGAATTCTGCTTAAATCCCTTGCTCTTGACT
CAAAGACTGAATCCTTAGCCTCAATTTTTTTACCAGCTGATCTGTGCCTTAACTGAAACT
TTACAGCTTCTTTTTTCTGGTTTCCAAAAATGGAAACTTCTTCATTTTCTCCATTTTTTACA
TCAACACCATCTACTTCTTGAGCTATTGAATGGGTTTTTGATTTTTGAGAAACTCCATAG
TTGCAATCTGAGTCAGCTGAAGAAGAAACAGCATTAAAGCTTGGATGGTTAGGGAATTC
AATACCCGAAACACTAGAATTTAGATTTCTGAAGCTGTAAAATCCTCCACAGGCAACAAA
ACCAGAGGAAAACAAGAATATAAACAACAAAATGAAAAGAATGAACTTTGTCCCCAT
SEQ 5 
TTACATTGAGGCATCAAGGAAAGCATTAGAGACATCATCTAATTCCACATTCAGATTTTT
GGCTGAAGGCAATCCAGCCACCACATTATGTTCAATGCCACATTCATTTGTTCCTCTTCT
GATCTTGAAGTAACCATCCTGTTATCATAGAGATAGACATACATTTAGACAAGAAGCTTA
TACAAATAGAGTTTAACTTTTGTGTATTGATAGTTTTCAGTTTGTTTAATCATTCAGGCTA
AGATGACTTATGCAACTGTCTAAAGTAATTCTAATTTAGTAACTTTAAGAGTGCAGTAAAT
AACTTGCTTTAACTTTTAAGATACACAGATAGTGTAAAAATTATTTACGCTGTCAGTATAG
TTAGCGCATCTACAACAAGGAGTTAGGATGTCGATGGTTTAAGGAAATTTGTGCTCATA
TATGAAAGCATAAGGAGAAAATAGAGTACATACATCACCCCAGCCTCTGTTCCAAGAAT
TAGCAATAAGCTGCAGAAGATAATTAAAAAAAAAGGTGATGATTAGATTTTGATAAAATG
ATATGATTTTAGATAGAAAATAAGACACCACAACTTGAACACATCAACATACCCAATAGT
 
CCTCTCCCTGCTCACTGGTTCCCCATCCGATAAGCTTAACAGCATGGCCTCCCATACTT
TGCCCTGTTACATGCTTGTAAACTCCAGACTTGTAGTGAGCAAAATCCTGTGATTGACA
AAAAAGTTTTAAGTCATTGGGTTAGCGGAAAGCATTGCTAAGAAAAAGAGAAAATAACAA
TTATAATCAACGAGAACAATCAAATTCAAACAAGCATTAGTTTATACTAGAGAAACTGAG
AAACTAACAATTATAACCAGATAACTTCTCATATTGTGCTAAGCATTAAATTATCTCATTT
ACAAGTATTAATTTGCAAGTAAATCTCGGACAACATAAAATGAGAAGGTACATCGGTAGT
TAAAGTTTCTCAATTATCATAATAGTTACTATCAGGTCACTTAAAAGATCATTACATGTAT
CACATTATGGGATTGGCAGGTAATTGTTTCTATGTACACATACCACAGGACCAGCATAT
AAGTTTACCAGCTCAGACAATGTCATGTGTGAAAACTATATCAAATAATTTTAGTATCAG
AACTTAACCCTCCCCCTTATCCTCATTACCTCGTAGACGGTAAAAGAGACCTCGACTGG
TCCATTTTTGTAAATTTCTGTCATGATACTGTTGGGATCATGGTGGATCCTGTATGCATT
GACACCATAATGCTTTGATTTCCCCCATAGTAGAATCTCCTTCACACACTTCCTCTGACA
CTTTGGGGTGGGATATCCTGGTTCACAACCAGGGTGGGAACATCCCTCATTATCAAAGT
AAGGGTCACACTGCCAGAAAAAACAACTTTATTAGTGATTGATCATAAAGATCCACGGT
AGCTAATGGTTTTAGAGGAAGCTGTAATCTCTTTTGGTGAAAATAAGTCACCATTTACCT
CTTCTGTGACCACACCCCTACGGATAAAGTATCGCCATGCTGTTATTGGATATCCACCA
TCACAACCACTCCCACATAAAAAGCCACAGCATGCTAACAGATCATTTACAGACAGAGA
GATATTCTGCATTACACATTAGAAGTTTAACATCAGTGACCATAACTACAGAAATAGATT
CACATACGTTTTGTGCTAGGTGAGATGGTTTCCACATGCTTAGACCATGAAAAAGAATC
ATGAGGCTGGCACGTGAGAGCACTTGCTGAAGATATATAATTAAGTACATAAAAATGTG
TCATCTAAAGCTTTTAAATGAGGCGGTCACACAGTTCAACAAAATTTACCAAGTTATGAT
GGATACAGAAACGATCAGACAGCGATTCAACAGCACCAAAAGCCCAACAAGAACCGCA
ATGTCCCTGATCTGTGACGCAAATTTGTCCCGGTGATTGATGTGCAAAGACGGAAAGCA
TTAGGATCACTATCATAGAATTATAATTCAATAGTAGTAAGCAAGAACAAGAAGAGACTG
ACCCAGAATTCTTCCGATAGTACTACATTGAGGCCAAGCTTTTCGTGCATCAAACTCTTT
TGGTAGCTCCAAAAGTTTTGGATGAGTTAGAATAGGAATTCCCTCCAAATCACCTTCTCT
TGCGGGCTTAACTCCAAGAAGGCGCTTAAATTGCGAAACCTGTGAAAACCAAAGAAAAA
CATAATTATAAATCGACGTTTTCGGTGTATGGCTGCAAAATAAGCAACTGTTATTGATGG
TTAGAGAAAGCGCTAAGCCATATGATGATCTGACCGTGAAATTCGAGAATCGAGGGTTG
AATGCAGCTTTCCACCCAGCTTTGGCATTTTCATTAACCTCTTTAATGATTGATTCCTAC
ATGATTATCCAAAAAGCTCTCTTAGTTTTAAATTTGAAGCAACAAGGGCAATAACATCTT
CTCTAAACATGAAAAGAAGAATATACCTGAAGGATTGCAGATTCAACTTTAGCTTCAGAT
ATTGGCTGCTCTGCAACAACCTGTTTTCCATAAGAACAAAGAGATTCTACTCATCAAAAG
ACATCCTTAAAGCTTTTAGGAAACAGAGCTGCAACTCCAGGAACAAAAGCATGACACAA
TGAGTGACAAACGAAGAACTTCGGGCTCGTTTGGTACGAGGGATAAGGATAATTAATC
TCGGGATTAAATTTGAGATGAGTTTATCCCATGTTTGATTGTAGTGTTATTTTAATAATTA
TGGGAGGGTGGGATAAACAATCGCGGGATAACTAATTTCGGGATAATTAATCCTGCGAA
CCAAACAATCCCTAAAGGTTTCACTTTAATCAAGATGAAACTCTTCCACAACTTTTATTTT
CAACATTATAATACTATTAGCCTGGAAAATTAATCAAAAGTTTGTAGGAAATTCATCATAT
GTCTAAAGCACTATAACGTAGAGGAAAAAGAATCATAGAACAAGCAGAAATTGTAATTA
GTCCATTATTTCTCCTCCTTCTTCTCCCCTTTCTGTATTTCTTTGTGAAGCAATACTTCCT
CTCATGTTATTATATTTCGACAAGTAAGTTAGCTAACACATAACATAAGTAATTTGCATCA
AACCATATAATTAACTTCAGAAACATGTGTATACTTCTCTTTTCTCATTCTCACTAGGTAA
TGAGAAAATCATTAAAATTTGCTTCTACTCATGATTTCTAGTCAACGCTTAACTAAAGCAT
AAGAAGTCCAAAATACCCAACAATATTTGATCTTTCTGAAGAAACACAAAAAGGCTAATC
CTTGTGTTCATCAAAAGCTATACAAATCAAATCAATACGCTAAATCCACCTAAAACAAAA
TCATCAATTCAATAGGCAAGAACTACCCATAAGACATACTCCTACTGTGAAAGGTTCAAA
GAATGAAGAAACAAACCTGCAATATAAGGATAAACAAAGCACCAAAAAGCAAAGGAGTT
GCTAAAGACTTCAGGGTCAAGGCCAT
SEQ 6 
ACATTAGTCCTCCATACTTCTTTCTATCTTCTTCTGTCAGTCGCATCTCCCGGCGACTGT
CTCCTCCTCTCCATTTTTCCTTTCTCTTTTTCCTCACCGAGATATTTTCCCTATAAACAAA
ACACCGTAAAAATCATCTCCTCTAATTTCCTATTTTCCCCATTTTTCCAAATGGGTTCTTT
CCTCTGTTTCTCTGTCATTGTTCTTTTCCTTGTTTTCAGCCATGTTTTTCCAAGAAAGTT
TACATTGTTCACATGAAAAACCACCAAATACCTTCTTCTTTTGCTACACACCATGATTGG
TACAATGCTCAGCTCCAATCCTTGTCCTCTTCTTCAACCTCTGACGAATCATCACTTCTT
TACTCTTACGACACTGCTTATTCTGGCTTTGCTGCTTCTCTTGACCCACATGAAGCTGAA
CTACTCCGTCAATCTGATGATGTTGTTGGAGTTTACGAGGATACTGTTTATACACTTCAT
ACAACAAGGACTCCTGAGTTTCTGGGGCTGAATAATGAGCTCGGTCTTTGGGCTGGTC
ACAGTCCGCAGGAACTCAACAACGCTGCTCAGGATGTTGTTATCGGAGTTCTCGACAC
CGGTGTTTGGCCGGAGTCGAAGAGCTTTAACGATTTCGGTATGCCCAATGTGCCGTCG
AGGTGGAAAGGTGAATGTGAATCGGGTCCTGATTTCGATCCGAAAGTACATTGCAACAA
AAAGTTAATCGGTGCTCGATTTTTCTCCAAAGGTTACCAAATGTCGGCTTCTGGTTCATT
TACGAACCAACCTAGACAGCCGGAGTCACCTCGGGACCAGGACGGTCATGGACTCA
CACATCCAGTACCGCCGCTGGTGCACCGGTGGCGAACGCTAGCCTTCTCGGTTACGCT
AGCGGGGTCGCGCGTGGTATGGCACCGCGAGCGCGTGTAGCTACGTACAAGGTGTGC
TGGCCTACTGGTTGTTTTGGTTCTGATATTCTAGCTGGTATGGAACGTGCTATTTTAGAT
GGCGTTGATGTACTTTCTTTATCTTTGGGTGGTGGATCGGGTCCTTATTATCATGATACA
ATTGCTATTGGTGCTTTCTCTGCTATGGAAAAAGGAATTGTTGTTTCCTGTTCAGCTGGA
AATAGCGGTCCAGCCAAAGCTTCACTTGCAAATACAGCTCCTTGGATTATGACCGTTGG
TGCTGGTACCATAGATCGTGATTTCCCTGCTTTTGCTACTTTAGGTAACGGAAAAAGA
TTACCGGAGTTTCGTTGTACAGTGGAAAAGGAATGGGTAAAAAGGTAGTTCCCTTAGTT
TACAGCACAGATAGTAGTGCAAGTCTTTGTTTGCCGGGTTCACTTGACCCGAAAATAGT
CCGTGGGAAAATAGTGTTATGTGATAGAGGACAAATGCGAGAGTAGAAAAGGGTTTA
GTAGTGAAAGAAGCTGGTGGGGTTGGGATGATATTGGCGAACACGGCGGAGAGCGGC
GAGGAATTGGTGGCGGATAGTCATTTGTTACCGGCGGTAGCTGTAGGGAGGAAATTGG
GTGATTTTATAAGGCAGTATGTGAAGAGTGAGAAGAATCCGGCCGCCGTGCTCAGCTTT
GGTGGGACGGTGGTGAATGTGAAACCGTCGCCGGTGGTGGCTGCGTTTAGTTCAAGA
GGGCCCAATACTGTAACTCCACAGATTTTGAAGCCCGATGTTATTGGGCCTGGAGTTAA
TATTTTGGCTGCTTGGTCTGAAGCTATTGGGCCCACTGGGCTTGAAAAGGATACTAGAA
GAACTAAGTTCAACATCATGTCTGGTAAGTATTACCAACAACGGCTAGTTTTGTCATAA
TCTTTTTATTTATGCTTAGATTAATTATGGCCTTAATTATATTTTTATTAGATCTTGCAATT
ATTAATACTAATCGTACACACTTGAAAGGAAAAAGAGGAACATGTTTAATTAGTGCGTAG
TGATCTGGAGCACATGCCTAAAGTTTAGAGGGGTTCACATGTGTTGCATTGATAAGTTA
ATCCTAAATTACATTAGTTATAATTAAATATTAATGCGCTTCCAAGAAAAAGTTGACTAAA
TTTATCATATATTTCCAAATTTGTTTTGAAAAATATGATTTTGGTGAAGTTTGGCTTGAAG
ATGAAAATGTGTTTGGACATCAATTTTCAAAACATATTTCCCAAATTTATTTTGGAAAAAC
ATGAAACATTTCTTATACCCACAAGTTTAAAAAACTATCACAAATATCCAACGGTACCATT
ATCAATAACATTCATTATATTATCTCAAACCATAATCCTGAATATAAATAAATTTGGCACA
ATATTATCATTTTTATAATTAACTATATGATACACTATTAGATGATCGAGAATACGAAGCA
ACATCGTTTCAAAATAATAAATGAAAAATGGTGGACTCTTTTATATAATACAAAAGTTTGG
AATAATTTTTAAAAAATATAATAATGATATTTTGACCCAAAACCAACATGTAGTCAAAATC
TATGACCAAACATGTGTTTGCCAAATAAAACCCAAATTTATTTTGACAAAATATATGGCC
AAACGGGGCTTAGTTGTATGATGTGTCGTGTGATATTAATAAAAGAACTGCCGAAGCCT
ATACCAAATTTATGGCTAAAATAGCAAGAAACGTCCCTTTAACAGGGACAGAAGAAATC
CAAGAGGGGGCTCGCTGGTCTAGAGAAAAATTTATTAATTAATTTCAATAATACGCTGAT
GGTGTAAAAAATATTGACACCATCAATATATTGTAATATATCGTAAAAGTTTATTAATTTC
ACTTATTATAGACAATTATTTGAAGTTATCTTTATAGCTAACCCAATAGTGTAAAATTCAT
CCGTTGGAATGTGCAATATGATGTTTGTTTTCAGCTTTTGTGCAGTAGTGATTTTAAATA
 
GGTATTACTTGGAGCTTTTGTGCGATGTGACAAAGTGCATGTCACAAGGTTAGAGTCAT
AATGAAGGCAAAAACATGATATTTAAGTGAAAAGTGATAGAGGGACGAGTTCATTGTCC
GCACAAAAGCCTGATATTTAAGTTAAAAAAAATAGGTGACCAGCTTGATATTTAAGTGAA
AAAGGATAGAAAGACGGGTTCATTATCCACCGAAAGTCGAACCTAACCTTTTGCCATGG
CCTTTCTTGGTCATCAAAATAATTTAGGAGACTACCTAGGAAAAGTAGTATGGTCTGTCT
TCCACTAGTGGGTCATAACCTTAATATCATCCCCCTTGCCCCGTTGAGTACTCTGGACC
TATCTTAATCACTTCATTAGCTGGAAATAAGTTGATGAACTTTTTGAATCATTCTTGAAAA
TTCACAAATTCGAACCGTGGAAACAATCTATTACAGGAATGCAGTCTAAGTCTTCGTACA
ATAGACCCTGTGGTCCGGCCCTTATAGCAGGAGCCTACTGCACTGGGCTGACCTTTTT
CTTTAAAATCTTACAGAGCTCAAAATTTGGACTTTGTACTGTTTCGTTACATTATTTGATC
CTTTTTGTACGTCAAACTCTTTCAGGCACATCCATGTCCTGTCCTCATATCAGTGGCCTA
GCTGCACTTCTGAAAGCAGCGCATCCCGAGTGGAGTCCAAGCGCGATCAAATCTGCAC
TTATGACGACTGCCTATGTTCACGACACCACCAACTCTCCTCTCCGTGACGCTGAAGGT
GGCCAACTCTCCACTCCTTTCGCTCATGGATCAGGTCATGTTGATCCCCACAAGGCACT
TTCCCCGGGTCTCATCTATGATATTACTCCAGAGGACTACATCAAATTCTTATGCTCCTT
GGACTATGAGTTGAACCACATACAAGCCATTGTCAAGCGCCCGAATGTCACTTGTGCTA
AGAAATTTGCAGATCCCGGGCAGATTAACTACCCTTCGTTCTCAGTTTTGTTTGGAAAT
CAAGGGTTGTTCGTTACACCCGTGCAGTGACCAATGTAGCAGCTGCAGGATCCGTTTAT
GAGGTAGTCGTTGATGCTCCCCCATCCGTTCTGGTGACCGTGAAGCCATCAAAGCTTG
TGTTCAAAAGGGTAGGAGAGAGGCTGCGCTACACCGTTACATTTGTGTCCAACAAGGG
TGTTAACATGATGAGAAAGAGTGCATTTGGTTCCATTTCTTGGAATAATGCTCAAAACCA
AGTTAGGAGTCCAGTCTCATATTCCTGGTCACAACTATTAGAC
SEQ 8 
ATGAATCCTGAAAAATTCACCCACAAGACTAACGAGGCCCTTGCTGGGGCACACGAGC
TAGCACTATCCGCAGGGCATGCTCAATTTACGCCTCTGCATATGGCTGTGGCCTTAATA
TCTGATCACAATGGTATTTTTCGACAAGCGATTGTCAATGCTGGTGGGAATGAAGAAGT
AGCTAATTCAGTGGAGCGGGTATTGAATCAAGCGATGAAGAAGCTACCTTCTCAGACAC
CGGCTCCAGACGAAATCCCACCTAGCACTTCCCTTATCAAGGTGTTACGACGAGCACA
ATCGTCGCAGAAGTCTTGTGGTGACAGCCATTTAGCAGTGGATCAGTTGATTTTAGGAC
TGCTAGAAGACTCTCAAATCGGAGATCTTTTGAAAGAAGCTGGGGTGAGTGCATCAAGA
GTGAAATCAGAGGTAGAGAAACTTAGAGGAAAGGAAGGAAGAAAAGTGGAAAGTGCTT
CAGGGGATACCACATTCCAAGCACTCAAGACTTATGGCCGTGATCTTGTGGAACAAGC
AGGAAAGCTTGATCCCGTGATTGGTAGGGATGAAGAAATTAGAAGAGTCGTTCGGATTT
TATCAAGGAGGACGAAGAACAACCCGGTTCTTATTGGAGAGCCTGGTGTGGGTAAAAC
AGCAGTTGTTGAAGGGCTAGCACAGAGGATTGTACGTGGTGATGTTCCAAGTAATTTAG
CTGATGTTAGGCTTATAGCATTGGATATGGGAGCGCTAGTTGCTGGAGCTAAGTACAGA
GGTGAATTTGAAGAGAGGCTGAAGGCTGTGCTGAAAGAAGTTGAAGAAGCAGAAGGGA
AAGTGATACTTTTCATTGACGAGATACATTTAGTCCTTGGTGCTGGTCGGACAGAAGGG
TCTATGGATGCTGCTAATCTGTTTAAGCCAATGCTAGCCAGAGGTCAATTACGGTGTAT
TGGTGCAACTACACTTGAAGAGTACAGGAAGTATGTTGAGAAGGATGCTGCATTCGAGA
GGCGTTTCCAGCAGGTGTATGTGGCTGAGCCTAGTGTTACTGACACTATTAGTATTCTC
CGCGGGTTGAAGGAGAGGTATGAAGGGCATCATGGTGTTAAAATTCAGGACAGAGCTC
TTGTGGTGGCTGCCCAGCTCTCATCTCGGTACATTACAGGTATCTATACTTTTGCTATTT
TTACATAGCACCTTGTTTTGATGTCTTTTCTCCGTCAATAACTAAGCATGTATATGCACTA
CTTTTTCCTCGTGCATTTCATTAACTCTATAAATCAGAATGGGACTTAGATTCGGTTAAG
CGAATGAAGGTGAATTTTAACCTAAAATGTTATGGTGTCGGAGCTATAGATGTATATTTG
TCTGGTACTAAAATGACTTCTTGAAGCAGTAGCCAGAATTTTGATTCATTTAAGCAGGTA
GGGCATGAGACTTAATTAGCATATCATTGTCTGCACTTCCTTCTGGACCTTTACCAGTGT
 
ATGAGTTGTTTTGTGTTACAAGCTGCTCCCCATCTGGATAATGGTGGATTAAGACTTAT
ATGATTGTCAGAAGTGTACTAAAACTTCTTGAGGATAATTAAAAATTGCTCAAATCAAAT
CCGTAGCTCGTTTTCCACTGTCAGTTTTGCAAAATGCTTTTTATGTCTGTGTCGTGACA
AATTAAGCAGTCAGCCAGTTAAATTTTGGCAGTTTGGCATGCAAATTGTCTTTGCTGCAC
ATTTCAGGTGCAAAAATCACTAACCTCTTTGTATTTTCAGGTCGACATCTGCCAGATAAG
GCTATTGACCTAGTTGATGAAGCTTGTGCAAATGTTAGAGTTCAGCTTGATAGTCAACCT
GAGGAAATTGACAATCTCGAAAGGAAGAGAATTCAGCTAGAAGTTGAACTTCACGCTCT
CGAGAAGGAAAAAGACAAAGCTAGCAAAGCACGTCTAGTAGAAGTAAGTATTATATACT
ACCAATGCTTTTACTGGTAATTGCTCTATTTTCTAAAAGATATGTTAAGAATTATACTGAC
TCGAATTATACTGACACTGGTCCAGGTGAGGAAAGAACTTGATGATTTGAGAGACAAAC
TCCAGCCCTTGATGATGAGGTACAAAAAAGAGAAGGAAAGGATAGATGAGCTTCGCAG
GCTCAAGCAAAAGCGCGATGAGCTCATTTATGCTTTACAAGAAGCTGAAAGGAGATATG
ATCTGGCTAGGGCAGCAGATCTGAGATATGGGGCAATTCAAGAAGTGGAAACTGCAAT
AGCAAATCTTGAGAGTACCTCAGCTGAGAGTACAATGCTAACAGAGACTGTGGGTCCT
GATCAGATTGCCGAAGTTGTGAGTCGCTGGACTGGTATTCCGGTCTCAAGGCTTGGGC
AGAATGAGAAAGAGAAACTGATTGGTCTTGGCGATAGGTTGCACCAAAGAGTGGTCGG
GCAAGATCATGCAGTTAGAGCTGTTGCTGAAGCCGTGTTAAGATCTAGAGCTGGTTTAG
GAAGGCCACAGCAACCAACTGGTTCATTCCTTTTCTTGGGGCCAACTGGTGTTGGAAA
GACGGAGCTCGCTAAAGCTCTTGCAGAGCAGCTCTTTGATGATGATAAACTGATGATCA
GAATAGACATGTCCGAGTACATGGAACAACACTCTGTTGCCCGGCTGATTGGTGCTCC
ACCAGGGTAAGTTTGAATCTAATTCTTTTCTTTTAATGTCATGTCATATTATTACAGTATT
CAATCACAGATTCTCATGTGTTCCACATCTGCAGTTATGTTGGGCACGATGAGGGAGGA
CAACTTACTGAAGCTGTTAGGAGGCGGCCTTACAGTGTTGTGCTCTTTGATGAAGTTGA
GAAAGCCCATCCTACGGTGTTTAATACATTGCTTCAAGTTTTGGATGATGGAAGGTTAA
CAGATGGTCAAGGCCGCACAGTTGATTTCACCAACACCGTGATTATTATGACTTCAAAC
TTGGGAGCAGAGTATCTGTTGTCTGGATTAATGGGAAAATGTACGATGGAGACGGCTC
GTGAAATGGTAATGCAGGAGGTAAATAGTCTCAAACTAGTAACTTCCCCTTTGCTGATA
AAACTGGAAGAATACAGTGAAATAGTTTACCTTATTAGCTAGAATGACAACTGTTTACAT
GTGTGTATGCTTTGTGATAGGTGCGAAAGCAGTTTAAGCCCGAGCTCCTGAATCGGCT
GGATGAGATTGTTGTGTTTGATCCCCTGTCCCACGAGCAGTTGAGGCAAGTATGCCGC
TACCAGATGAAAGACGTTGCACTACGGCTGGCTGAGAGGGGTATTGCATTGGGTGTTA
CTGAGGCAGCTCTAGATGTCATACTCTCAGAGAGTTATGACCCGGTAAGTGTTATATCT
TGTAATCTAGTCCAATATTTTAGGATTATTTTGCGAACTTGTACTTATTGTGGTGATCATG
GCATTCAGGTTTATGGTGCAAGACCTATTAGGAGATGGTTGGAGAGGAAGGTGGTGAC
CGAGCTATCCAAGATGCTTGTGAAGGAGGAGATTGATGAGAACTCAACGGTTTACATAG
ATGCTGGTGTCGGCAGGAAAGATCTAACCTACAGGGTGGAGAAGAATGGAGGTCTTGT
GAATGCTGCCACCGGGCAAAAATCTGATATATTGATTCAGCTTCCTAATGGTCCCAGGA
GTGATGCTGTCCAAGCAGTCAAGAAAATGAGGATTGAAGAAATTGAAGAAGACGAAATG
GAAGAT
SEQ 9 
TTATGTAAATGCTTCACGTTGCTGTGTAGGTAGCTCCAGTTCAGGCTCAAATGCTGTTA
GCCGAGAAAAATACTGATCTACCATGTCATCTGTTACTTTATCCAAGCTTGGAGGATCC
CACTGCACGTTTTAACATAAATCAAGAAACTCTCTCTAAGGTTAAAGTTGACTCTTAGGA
AAATTCCTCCAGGAAGGGGCTCATAATTCATAAAAATAGCATATTAGTTCGCAATAATAT
TGATTTACCTTAGGTGCAAAATCTCTATCCACAAGCCGTGCACGAACCCCCTGATAGGA
AGATATTAGTACACTGTGATGATGATCTGTGGAATTTAGTACTGAGACTTTTTTTAGCAG
CATATATTACCTCACAGAAGTCATTAGTTATTTGTCCAGAGAAAGCTTGTACTGACATTC
GATACTCACGAATTAGACACTGGTCCAGAGTCTGATGTCTGCCTTCGCGTATCTGATCA
 
GAAGCATTATTGGAAGGTCATCTCACAATGCCCTGGTGGCAGAAGGGAAAAGGAGAAC
ATTCTATCTAGAGAAACTTACAGATCTCAGTGAAACCTTCAAGCTCAGTGGGGCTGTTT
CTTGTAGTTTTCGCAATGTTGAAACACACCATGCATCTTGCTTCTTGGCTGCCTCACTTT
CCTGGCAACATTTAAATTTCTTTTTAAAAATGTAATCACACCTGTCCTTAAAAACTTCCAC
ACATGTAGCAGACTTTATATGTTTACTTAAATACTTTACTGTCAGTATTTTAAAAACAAGA
GAAAATCCATCTCGCTCGAAAGAGCACCCAGAGGTCGTAGATGCAAACGCAATGACTC
AGCTTGTACTATGGTGCAACAAAGTGCAGCACTTAAAGGCTTCTCCGTGCAATTTATAC
GTGATGTTAGAATCTCCACAATATGCTTTGCCCAAAGAATGATAGATATAAGTTAGTGTG
ACAACAGCCCCAGGACCTATAGGTACTTTTTGAACCTCAAGGAGCAAACAAAGAGTATG
CTGAGCAATGATCATTGCATTGTTTCAGATTTTCAAATAGTGGCAACTTGTGATGGCCG
AGGTATTAAAAGCAAAGCAATCCGATGCTGAACATTATTGCATTGATCAACTAAAATATG
TTTCCACTGGCACCTAAGGGGGTAGACTGTAGCCAAATAGTCAATGAAGTCGGTGAAAA
ACACGGAGACTAGGTTTCAAATCTAGCAAAGAGTCGGAGAGAGTCAGAGACAGAAAG
GCTAGGTGATTTCTTCCTATCTGCCTAAAATTTGGTAGATAGCAAGTACCTGTTGGAATA
GTCGAGGTGCGAGCAAATTGGCCCACACCACCGTTATAGAAAAGAATAATGTTTCTACT
GGCCACACTTTGATTGTATGCCCATCCGATCGAGTTTCTTACCAGGTAATAGCCTTCAC
ACCCCAGGCTAGTGAATTAAGATGTGCTATCCTATAAAGACTTACTTCGTCCTCCACTAA
TATGATCGTATCTTGTTACTGGAGTCGAAGATGGTATGTTGGCGAGGCTTCCACCTTCT
GAATACCACATGTTTAATCCCTGTTCTTTCTTTTCTCACTAGAGATATTAAGCAAATTAAA
ATCAATTAACATCAGAATTTTGTATCTTTCAGAGACCTTTTCAGTATATTTGGGAAACAAG
TTACACACTCAGGAACTATATGCTCACCAAAGCATCAATAATTTCTTCGACTGTGTCATG
GCTGAAACATTTATTGAGAGTTTCAATCCTAAAGAAAGAGAAAAGATGGATTAGATTAAG
CTATCAAATACTCATTGATCTGCAAGCAAGATCACACAAACTTGATAAGAATTTATTTTTA
TAAAAACAAAAGGTTAAAGAACAAGACTAAATACAAAAACCAAAAGATCCATGTGAAACT
ATGACAAAAATTATCATGAACCACAAAGTTTTAAGAATGTAGTGTGAAGTTTTAGAGAGC
TGAAGTTTGTTCCACTAGTTGATGTATTTTATATAACTCATGGAAAAGATGGTGCCACTA
CCAGTTTCTCTATAATGGAAAAAACAATTTCGCCACTTACAAATCTAAGTGAAGCTAAAT
ATGAAGAGCTAATTGCAGGATTTTGTGTTTACTGAATTAAACAATTTACCAAAAATTACCA
ATTTGATTCCTCGAAGAAGAAAGTAAGAGATTGATTTGCAATTCTTTCTTTTTGAGTAGTA
TGAAAAATGTTATGAAATGAGGGCAAGTGTCTCATCCAATTAAACCTGTAACTTGCTTTC
CAACAGCAGGTAAAATATTGAAGCAAGGCTGCAGCGTGTCCACTTATGATTTCAATTAG
CCAAGGGATTCTTTCGATGTTCTATAAGAAACGTGAACGGGATTCCCCGAAGATGTAGG
TAGGGAATCCGCTGACTGGTGTGGTATATATGTTTGGTTAACAACTAGAAAAGGTGTTT
CAAATCCAAAAGCAGCCCTTAACATTAAACGGTAATATGTATCAGTCCACCCCCTTTCAA
ACTGTAGCAGGAACTAATATATTTATGGACATTCCAATTTCCATATTTAGCAAGGATGAC
AGGTACCTGTGAAGTACACTTGTTGGATCTGGATGGACAATCTCTCCACAATTTTCAAG
AGATCTTTCAATCACTGAAGGATCATCAGTCATCAATTTACCAAGTTGTTCCTCAATTAA
GGGAAGCTTCTGAGAGGAGTGGAGAAGGCAGAGAAGAAGTTTTCAGAGCAATAGTCAA
ATAACCTAAGTAGGCTATTGGTACCTAGGTGCAAAGCAAATTAAGCAAAGGTAGTAGAC
TTACTGCACTGTGTAAGTAGTGAGTAGCAAGCCCACAGGATATCATTTCTGCTCCATTG
ATCTTGTCTCCAGTTAGGGCCAGGTACTCTCCTGCAAATTGAAAACATTTAAATAGCTTT
CTTCCATGCTGATTTTTTCTTTCATTTACGCAGTAGCAAATGTCCAAACAGATGAGAGAC
ACAGAGAGAGGCAGCATACTAAAAACATATTCCACTTGAACCACAAACTAGTATAGCAT
AAGATAGATAGGGTTTACATGAGCTGACATGCGTGCATCAGGTTGCTTAACATTTATAT
GCCATAGAATATGAAGTCATCAAAGAGTCAGCCATGTAAATGCAGTGCTACATGATCTA
GCATGAGTTATCAGTTATCTACAAAAAAAAGGCTTGTAACCCACTAATTTTAGTCCGCCA
CCATTTAAAGCTAAATATTAAATAGATAGAACCACATATTAACATCACCGTATGAAGTTAA
ATTTTTAAAAAAAAAGGCAGTCCAGACAAATTCCGAACATAGGTAGAATTCAAAGTCATA
ATCTCCAAAATACATGAAACAGGAGGAGGAACACATGCTTACTTCCCAATGAGTTAGGG
AAAAATAAGATGAAGTAAAAAGAACATCACGTTAGTACAAATATTTTGATAACAAGCATA
TAAGAAGGGAAACGTCCAGATATGACATGCTTATTTTTTCCCCATAGAAGATGCGCATC
 
TCCCCAGAGAAGAGGAGATCGGGGAAGGGGGTGTGTGTGTGTGTGTAGAGAGAGA
GAGAGAGAGAGGGGGCGCGGGGATCAGAAAAGTAGTTTCCAACTAGATAATTCATCAT
CAAAGACGATCAGCTACATGACATGATAGAAGTTACTGATGGATCCAAACCACAGCATG
AGATCAGCAAAAGAATATACCCAAATAACCAGGGAGGTGTGAAAGGTAAAATGACGCC
CCAGCATCGGGATGGTAACCAATCAATGTTTCTGGTGTGGCAAAAACCTACATTGATAC
AAGACCCAATACCCTTGTAAGAACGGTGAAAAATCGATCTAACAGCACTTAAAACACCT
AAACCATCACCTCGTGCACCGGCCAAATTTGATAGACCTCTTTGACAATTAAGTTCGATA
AATTTTTTATTTCCTGCACATTCCTTTAGTTCTTGAGTAATTTAAAATCAGGTTATAGCATT
TCAGTAAGACCAAGATATCTTAAATTTTTCAGCACTTTAAACGCTAAGCCTGTGGTTATA
AGTCATATATAACCGAAATAAACAAGCTTCATCACGAAAAGTCAAAAGAATTGATAATAT
ACAATTGAAATTACTATTTAACTTATTGGTACAAAGAAACTTCATCAATATAGAGCATTCA
ATTTAAGAAGATTAAAAGATGAATTTTATCAAATTAACTCGGTACGAACTCATAAATAGAT
AATAATAACTGATAAGGCCAAAACTATCAATAAAGGAAAAGCAAGAGAAGGGGCAGAGG
AAAAATTTGCTGAATAAGTTTGTAGCACTAGGATTTGAACTTCTCTCTGAAGAGAAATAA
TTAAGATTTAAAGAACAGAAAATTCATGATATGTGAAGTCACTAAGCTGTATATAAGAAT
GAGCACAAATGGAACTTCACATTACTTAGCAATGTAGTCTAGCAGTTCTTGAAGTAGGA
GAATTTATTCTGAACCAACAAATGAAAAGCTTAAACAAATAAAGCATGACTAATCTTTTCC
ATACAGTTTTCTCAGTTGCAACACGGAAAGTTCCAGGAATTGAGATGCCAGCCCCACCA
CCCATGGTAATTCCATTCAAAAGAGCAACCTGCATATGGCATCTGAGGATTAATATCTAT
ACTTGGGAACCGCATCCAGGGACCAGCTAATGTCAACAAAAGTATGACAAGTACTGAAT
ATGCAATGACTGAATTCCTTCAACATGGAATTGAGGAGGTGAGAGCGAGAAGTAACTCA
AATCCGAAAGTGCGCAAAAAGCTCTAATCGATAAAAAGATACAAAGAAATATGGATGAT
AACTACTGATAAATGCACACCGGGTTTCTGAACTAAAAAAAGACGTATATTATGATTAAT
AAAAGAATGAAAAGACCATTATGGCTGCATCTTGTCAAAGCAAAAATGATCACATGATTT
TAACAAAATAAACAACTTCCAAAATTGAGGAGATATTATTTCATGGGCAAAAAGGAACAG
ACAAACTATAGGTCTAAACAGGTGACCACTTTGTTGAGCATCAACAACAGCTTATCCCA
AATCTTTCATTTCAGAAGTCAAAGAACACAGCAGCAAGAGAACTATGGATGAACTAAGA
AAATGGAGTTGAATATCTATGAATAATTGGATCAAATTTGCATTTGTCCTAAGGAAGTTC
TTTAAGCTTATGAATCATAATGTTAATGTGACTGATTATGTTTTCCTCAACAGGCATCGTT
ATGAAGATTGTATAGCAGCCAGCATGATAAAGTAGTGTTGCTACCATTTCAAGTTTATGA
ATAAGATCCAATCAAACTTTGGCAAGACACATCTAACCTCTCTGAATACCATTTTAATTC
AGAAAGAAGTGATTGGTGTAATTAACTGTCGAGATGCTGTCTTTGCAGCAGCTTAGTCA
CAGTAAGATGAGAGAGAATCCAATTAACAGAACAAGCGATCTTCTAATAAATCCAGATAT
TTCTATAAGAGTATCTTTAAAACTGCCAGCATAAAGTACAAAGGTGTTGAAATTTCAATA
AGCAATGGCGAATATAGGATGAAAATGTTGATTCAACATCTAATAGAACTTACATATATT
TTTTAGAAATTCTTAGGTTCCAACTACAGAAGCATATTTTATTATGCGGAAGTCTAGACG
CACAGTTAGATCACAACATAAAAGACATGTAACTACAAAATTTATAAGACGCTGGCCTCT
ATCAAGGTTTAGTAAATACAACAAAAGTTCCTGATACATATATAAGAGGCAGAAAACAGA
AGAAAATTCAAGTCAACAGTTCTTTTAGTATCTGATGTTTGAACAATAATGATACTTACAT
GTGGCTTCAAGAGTGTGCCGACAACGTATACTAAGTTATTTATTGTCCAACAAAAATCTT
TACAGTCTTGAAGATTCCCTGCATCTATCCAATACAAAAAATATTACACAAAGAATGGAG
AAGATCAAATTAAAATTGAGGTAATAGTAAATTGGTAGCATGAAGATGCCGGCTGCAGT
TTTTAAAACGATGTAGAAACGTTTTAATTGTTCCCAAAAATAAATAGATATCAATACCCTA
CTCAATAAAAACAACAGAACATCCAGCTATCAGCACAAATAGCTTAATTAAGATAATTCA
CATCTAAACAACTTCTTCACACCCCAATAATACAATTATCGACAATGTTTAAATATTTAAA
ATTCCTTTAACCATGTATCACTTGCAACATAGCAGAATATGAACAAGTTCGCCGTGAAGT
ACAACAGTCAAAATGACACAACATACCTTGTTTTAACAAATTATAAATAGTGACAATGTC
ACCCCCAGCAGAAAATGCCTTGCCACTTCCCTTCAAAGTTAAAGAATAACTGAGTTGCT
ACTCAGTAGATTAACATATGTAACTTCACCTTAAGATAATCCAGAAGAACAACAATAGGA
AAAGCCATAAAATAATTCAAACAATAATTTACTTAACAAATTACCTTCAATACCACGAATC
CAATATCAGGATCATCTTCCCAATTTTTGTACAGCTTTAGCAACCTATCCACCTAAACAA
 
CAGTATCATGCAGAGTTTTTATTTATTATACAAGGTGGAAATTAAGATTGCCAACCAACT
GGAATATCAAGATTCCCCAGTGTCATGTAGGGTTCATTCCCAAAGCCAAAACAAAATCA
ATTCAGCATAAAAACACGATTTTGATGGTTGGATATTAATTAAAACACGATTTTACTTGGT
GGGTATCAATAAAAAAAATCCAAAAAGAGAGCAATAGATTTCAAAAATGATCTTCTTGTG
CGTACACACTGTAGATTTCAAAAATGGCAAAAACAGAAGCAAAAAGTAAAGGTCTTTAAA
AGGCAGGAAACTAACAACTGAAAAATTGAGAGCATTTAACGCATGGGGTCTGTTAAGGA
TTGCTGTTCTCGAAGAAGCTTTTCCCTCCACTAACACCTGAAAAATGCAAACTCATATTT
TATAACAAAATGGAAATTTTGATAATGAATCATAAATTGGATTGACAAATTTTCTTTTAAA
AAAAATTCAGAACTCACAGTGCTTTGGGATTCATCAACAAGGGCATTGGTAGAGACACT
GCAAAAGCTTCTGGAGTGAGAAACCAAGCGCGAATTCTGCAGTAAGCGCCTCAAAATA
CTTGCTGATTTGAAGCTCTGCAT
SEQ 10 
ATGGCCTTGACTCTGAAGTCTTTAGCAACTCCTTTGCTTTTGGGTGCTTTCTTTATCCTT
GTATTGCAGGTTTGCTTCTTCATTCTTTGAACCTTTCTACAGTAGGAGTATTTCTAATTAT
GGGTAGTCCTTGCCTATTGAATTGATGATTTTGTTTAGGTGGATTTAGCGTATTGGTTTA
ATAGCTTTTGATGATTTTACAATTTATATGGATTACCCTTTTCGTATTTCTTCAGAAAGAT
CAAAGATTATTGGGTATTTAGGACATCCTATGCTTTAGTTAAGCGTTGACTTGAAATCAT
GAGTAGAAGTAAATTTTAATAATTTTCTCATTACCTAGTGAGAATGAGAAAAGAGAAGTA
TAGACATGTTCCTGCAGTTAAATATATGGTTTGATGCAAATTACTTATGTGTTAGTTAACT
TACATGTTTCTATATATAACATGAGAGGAAGTATTGCTTCACAAAGAAATACAGAAAGGG
CAGAAAATGGACGAAAAACAATGGACTAGTTACAGTTTCTGCTTTGCTCTATGATTCTTC
CTCTACGTTATAGTGCTTTAGACATATGATGCATTTGCTACAAATTTTTAATTAATTTTCC
AGGTTAATAGTATTATAATGTTGAAAATAAAAATTGTGGAAGTGTTTCATCTTGATTAAAG
TGAAACCTTTAGTTCTGCGTTTGTGACCCACTGTGTCATACTTTTGTTCCTGGAGTTTCA
ACTCTGTTTCCTAAAAGCTTTTAGCTTGTCTATTGATGAATAGAATTGATGTGTTCTTATG
GAAAGCAGGTTGTTGCAGAGAAGCCAATATCTGAAGCTAAAGTTGAGTCTGCAATCCTT
AAGGTATATTCTTCTGTTCATGATTAAAGAAGATGTTAGTGCCCTTGTTGCTTCAAATTTA
AAACTTAAGAGCGCTTTTTGGATGATCGTGTAGGAATCTATCATCAAAGAGGTTAATGAA
AATGCCAAAGCTGGATGGAAAGCTGCATTCAACCCTCAATTCTCGAATTTCACGGTCAG
ATCATCATATATCTTAGCGCTTTCTCTAACCATCAACAACAGTTGCTTATTTTGTTGCTAT
ACACTGAAAACATGCATTTATAATTATGTCCATCTTTGGTATTCACAGGTTTCACAATTTA
AGCGCCTTCTTGGAGTTAAGCCCGCACGAGAAGGTGATTTGGAGGGAATTCCACTTCT
AACTCATCCTAAACTTTCGGAGCTACCAAAAGAGTTTGATGCACGAAAAGCTTGGCCTC
AATGTAGCACTATCGGAAGAATTCTGGGTCAGTTTCTTCTTGTTCTTGCTTACTACTATT
GAATTATAATTCTATGATAGTGATCCTAATGCTTTCCGTCTTTGCACATCAATCACTGGG
ACAAATTTGCATCACAGATCAGGGACATTGCGGTTCTTGTTGGGCTTTTGGTGCTGTTG
AATCGTTGTCTGATCGTTTCTGTATCCATCACAACTTGGTAAATTCTGTTGAACTGTGTG
ACCACCTCATTTAAAAGCTTTAGATGACGCATTTTTATTTACTTATTTATATATCTTCAGC
ATACTCTCTCATGTGCGAGCCCTGATTCTTTCTCATGGGCCAAGCACGTGGAAACTATC
TTATATTAGCACAAAATGCTTGTGAAGTTTTCACTATAGTTAATGTCACTAATGTTAACTT
TTAATGTGTAATGCAGAATATCTCTCTGTCTGTAAATGATCTGCTAGCATGCTGTGGCTT
TTTATGTGGATCCGGTTGTGATGGTGGATATCCTATATCAGCATGGCGATACTTTATCC
GTAGGGGTGTGGTCACAGAAGAGGTAAATGTTGTCTTATTTTCACCTCAAAAGAGATTA
CAGCTTTCAGTAAAACCATTAGTTACCGTGGATCTTTATGATCAATCACTAATAAAGTTG
TTTTTATTCTTGCAGTGTGACCCTTACTTTGATAATGAGGGATGTTCGCACCCGGGTTGT
GAACCAGGATATCCCACCCCAAAGTGCCAGAGGAAGTGTGTGAAGGAGAACCTACTAT
GGGGGAAATCAAAGCATTATGGTGTCAATGCATACAGAATCCACCGTGATCCCTACAGT
ATCATGACAGAAATTTACAAAAATGGACCAGTTGAGGTCTCGTTTACAGTGTACGAGGT
 
AATGACGATAAGGAAGAATGTTAAGTTCTGATCCTAAAACTATTTGATACAGCTTTCCGT
ACATGACATTATCTGAGCTGGTAACCTTATATGTGGTTGCCTACCTATCCCAAAATGAGA
TACATGTAATTATTTTTAGGTGACCTATAGTGTAACTGTTATGATAATTGAGAAACTTTAA
CTACCGATGTACCTTCCCAATTTATGTTTGCCCGAGATTTACTTGCAAACTAATATCTGT
AAATGAGATATTTAATGCTAACCACAAGACAATATCAGAAGTTACCTGTTGTCGTAAAAC
TGCATCATCTCTTTCTCGGTGCAAGTAGATTTGTTTAGATTTTGTTTGTTGTCTTTGATCA
TAACTGTTATCATCTCTTTTTCTCAGCAATGCTTTCCTCTAACCAATGAGTCAATTTTTTT
TATTTTTTTTTGTCAATCACAGGATTTTGCTCACTACAAGTCAGGAGTTTACAAGCACG
TAACAGGTCAAAGTATGGGAGGCCATGCTGTTAAGCTTATCGGATGGGGAACTAGTGA
ACAGGGAGAGGACTATTGGGTATGTAGATGTGTTCAAGTTCTGGTGTCCTGTTTTCTAT
TTAAAAGCATATCTTTTTGTCAAAATCTAATCACCTTATATATCATCTGCAGCTTATCGCA
AATTCTTGGAACAGAGGCTGGGGTGATGTATGTCCTTAAATTCATCCCTATGTTTTCATA
TATGAGCAAAAAGTCCTTAGACATAGGCATGCTAGCTTCTTGTTGTTGATGCACTAACTG
GCACATCAATAAATGGATTTCAACTTATATAAACTAACAACGTAAACAATTTTTGCACTAT
ATTTCAACTGGTAAAGTTATCTCTGTGTGACCTATTGGTCACGGGTTCGAGCCGTGGAA
GCAGCCACTAATGCTTGCATTTGGGTAGGCTGTCTACATCATACCCCTTGGGGCTACG
GCCCTTCCCAGGACCCTGCGTGAACGCGGGATGCCTTGTGCACCAGACTGCCCCTTTT
ATATTTTAACCAGTTAAGGCAAGTTATTTACTGCATTTTTTGAAGTTACTCATTTAGGATT
ACTATAGAGAGTTACATGCCGTCGTATGTCATTTAACCTAATGATGCAAATAAATTGTAT
ACTATTTTAATGCACAGAAGTTAAAGTAGCTTCTTCTCTAAATGAATGTATATCTCCAATA
TGACAGGATGGTTACTTCAAGATCAGAAGAGGAACAAATGAGTGTGGCATTGAACATAA
TGTGGTGGCTGGATTGCCTTCTGCAAAAAATCTGAATGTGGAACTTGATGATGTATCTG
ATGCTTTCCTTGATGCCTCAATG
SEQ 11 
CTATACCATCATACCCATGTTGGAATGTGCCACTCTGACAACAAGTGGAGATGTTACCG
ATGTTCTTTTGTTCCTCCATGTCAAAGAACCAAATACATATCCCTGTGTTGGTGCAGCCA
CCTTGAAGTTCACTGTAAAATTCATCTTCTGGTAATATCTAGTGAAGGCTAATCTTCGGG
GAACCACAGTGACATTGACACCCGTAGGTGCATAGACAACTGCCTTGTAAATGCTTCTT
GCTTTTCCCACGTTAGTAACAGTTCGAGTTACTGAATATGTGCTTCTGAGGTTTGGTATT
GTGATGGAGGGATAATTTAGTCCATTTGGTGATGCAAAGGTTTGATCACAGGTGCTATT
GTCCCTTGTAATCAGATGCAGAGATTTCTCATCATAACCAATTGAACAAAGAAATGCTCT
GTAATCTGCTGGCTGTGCATCGTATATAAGACCAGGATCCAGGACATTCGTAGGGTTAA
CAAAGCCAGAACCAAAATCAAATGGAGTAGCTCTCTTCCCTTCAGGATCTACTATTATG
GGTTTGTGATGCTTATCTGACAGTTTAGCTGCATTAATATTGATGAGATTAGTGCATTGA
AGGCTTGAATGAAAGAGTTAGATTATGTAAAAGCTTTTATTCTACCTGTCGTCATGATCG
CGGATTTAATTGCAGAGGGAGACCAAGATGGATGCACAGCTTTTAACAAGGCAACAACT
CCTGTTATGTGGGGGCAAGCCATAGAAGTTCCGGATAGTACATTGAAGTTCAACTTAGT
AGAAGCTGCTGGAGACCATGCTGCCAGGATATTTAATCCAGGAGCTGCAATATCAGGC
TACAAAGGCAAATCAGCCAGGAAATTACTTGCAAGAAAAAGCCAAATCCTCAAATAAGG
TAAGAACCAAAGAAAATGCAAAGTAACAAAGAACTAATCCACAACCACATTGACGATCC
AGGAAGTAAATATCAGGATGCAACATAAAACTTTGTTGGTCTACATGACAAAGGCAGAG
AGAGATCATTGTTGAAAACAAGTGGCAGTTGAAATTAAGTCCCTATATTACTATTTTTAG
CGCACAAATTACCTTCAAAATTTCTGGTGTTACAGAATTAGGACCTCTTGAAGAAAATGC
TGCTACTCGAGGAGCAGGTTGAGCTCCCAAAACGGTTCTAGCAGAGAGAATCCTTGCC
ATGGGGAGGCTGTTAATATAATAAGATGTGAGATTGGGAAGAGATGATAGTTAACAACA
TCAAAAAGTCAAGAAGAAGGAAGTGAACCGTGTATTGTTAATGTAAGCTAGGATCTTGT
TTCCAATCTTTTTCCCAACAGTTGCTGCAGGAATGACAAAGGGATGGCCACACCCTTG
TCTGCGTCATCTATAAGGATCATCCCAACTCCACCGGCTTCTTTAACTATAATGCTTTTC
 
TCCATCTTTGACTCACTTGAGCTTCCAGCATGTAGGCACACAAGCACCTTCCCTTTGGC
CTTAGTTCTATTCAAAGAACTATCTAAGCAATAACTAGTGGAAAAGAGAGGAAGGAAA
AAAGTAAATTAAGATAATTGTCAATGCATACATATCTACATTTAACAAACAGCGAAAGTA
CCTTATGATTCACCTGGATTGATAGGGAGTGAAGTATCCAGCATAAGCTTCAGAAGCAG
GTATGATTCTTGTAGATGTATTCATTTGAGATAAGCTAAGACTTTCACCCTTCCAAATATC
CACAACTTAATTAGAAAATAGAAATTGAATAATAATAACATGTAAACTTGTCGAAAACTG
GGATTACCTTGAGCCGAACTCCATTTCCTAGTAAAATATCAGAAGTAAAATCTCTATCAG
TTGAACTGGCTGCAACTGTGATCATCCAAGGAGCTAAATTTGTGGCTGAACCAGTGCTG
CCTTCATTTCCAACTGAAGCCACCACAAGTATTCCGCGGCTAACAGCATGATATGACCC
CACAGAAATGGCATCATTGAAATAATCTCCTTGGGGAGCATCAGGGCCCAAAGATAGA
GAAATGACATGAACCCCATCTCTAATTGCATCATCAAATGCAGCCAATAAATCAACATCA
TAGCAACCAGAACTCCAGCAGGTTTATACACTGCTATCCTGGCCATTGGGGCACCACC
TCTGGCTCCTCCATTTGCCAAACCTTTGTAATTCATATTAGCTACGTAACGCCCCGCTG
CTGTTGAAGCTGTGTGACTCCCATGACCAGAACTGTCCCTAGCAGACTTGTAAAACATG
GTCTTCCCATTTTCTTCTTCAGCTTCATAGCCACTCATATAATATCTTGCCCCAATTATTT
TCCTACATAAGACAAATCATATTGCACTTATCATCTAAATAACAAAAGAAGAGATGGTTG
CCAATCAAAAGAAAACCTGTTGCATATAGAGGCATTGAATGCTTCTCCTGATTGGCATT
GTCCTTTCCATCCAGCTGGCACTGGAGGCATGTTGGTATCACTAAAACTTGGAGACTCA
GGCCAAATTCCTGTTTCATTAAATATCTTAAGAGCTTAACCTCAAGTTCTAATTAGCTCG
AAAAACAAGGGAAACAGTGGAGCTGGGGACCAGGTTGAGGATAAACTGATAAGGTTGT
GAACAGAGATAATATTTGCATTGAAACATGTCACTCTAATGTATAGTGGCTCTTCCCATA
AAGTAACATTTACAAGTAGTTCAAGCACACTGTTAGGCATAAATGCAAATGGCAAAATAT
GGGAAGAGGTGAGAAAGATGAACTGGGAAGCTAAGAAATTGCATAAACTTGAGTTTTAA
AAAAATCTAAGCAAACATATTTCATCATTCGAAAATGATTGAAACAAGAATTGATTGATGA
AAGGAACTACTTTCCTCAGGTTCAGCCATATGTACCCAAATGACAAACTTAGCACTTTTG
CAAAGTCATGTTATTGTACTCTTCTTAAGAAAACTAACAGAGACAAGAGCCCTTTTAAGT
GACAATACATTAAATGAAGGGACCAACACTAACTTGGTTGGTGCATTCCATCATTAAAC
GATCATATCTTTCACCTAACTCGGAAAAGATTGCTAGAATTTAAGATAATTAAAGCAAAA
GGAACAGAGAAACCACCTGTATCAATGAAACCAATGATTACATTAATTTGGTTCTTGGTA
GAAAAACCTGGAATTTCCATTGTTTCATCATCACTGAGCCCCATAAAATCCCATGAATGA
GTTGTGTGTAGGCTCCTCTTAGTATTTGGAAACACGGATACCACTCCAGGCATTTCTGT
TTTATTTTAACATTAAAGACAAATTTCTCAGTACTTATTCATATCATTACCTTAAAGAAAAA
CATTTGGCATGGGCTTACTGGATATTTCAGAAGCCTGTGCCTCAGTCAACTTGGCTGCA
AAGCCTTTAAAACCATGCCTATAACTATATACATGTGAAGTCTTGGCTTGTTCAATGCTG
TAAAGTTACAACTTCAGTTTTTTTGCTAAAAGCAACCAGTGTAAAACCCAATGAACCAGC
TCAAAAAAGGGAAAAACCCTCATAGCTAAAGGTAACAAGAACTGACCTTCCTTTATGAAT
AGCAGTCAGCATTTGATGGTTTTGCCTCAAAATCTCATCTGGGTGTTCATCACTATCTTT
GCTTCCCATGTACACCACATATAACTGGAAAAAAAAAAAAACCAAGAACACAACTTTACT
AACTATTCATCATTTCAAAACATCATTCAAATATACACCCTCAATAACAGCACATAAAAAC
CCATATCAACATACAGACTACAGAGCCAAAGTTTATTTACCTTGGAAGAAAAGCAGAGG
CTAATATCTCCAAGAAAAACACAAAGAAAGAGTAAAAGAAGAGTCTTTTTTAGAACACCC
AT
SEQ 12 
ATGGGAGCAAAAGCATTTCTTGTTGCTATGTTTCTCTCAGCACTGTTATTTCCTTTTGCC
TCCTCATCCAATGATGGCTTGATGAGAATTGGCTTGAAAAAAATGAAATTTGATCAAAAT
AATCGGCTTGCTGCACGCATTGAGTCAAAGGAAGGGGATGTTTTGAGGGGGTCGATTA
GGAAGTATAACTTCCGTGGTAAACTGGGGGACTTTGAGGATACAGACATTGTAGCATTG
AAGAACTATATGGATGCTCAATACTTTGGGGAGATTGGTGTAGGCACTCCACCTCAGAA
 
GTTCACTGTAATCTTTGACACAGGTAGCTCGAATTTGTGGGTGCCGTCGTCGAAGTGCT
ATTTCTCTGTAAGCTTCTATACATGCAAATGATACAAAGGATAGCATTGAACATCCATCT
TGAGTGATGTAAAATTTGATGACTGCCTATCTTGGTGTAGGTTCCCTGTTTCTTTCATTC
CAAGTACAAATCAAGTGAATCAAGTACTTATAAGAAGAATGGTATGTTATGTTTCCATTTT
TGTATATTGCTTCTCTCTACCATCTGGTTGTTTATTGCTGCATGAAACATATATATGCTTC
CTTCTAGTGCCGGTTATTGTTTAGAATATGTGCCATCTTGTTCATTTTAGAACACTTTTTG
TATTGTCCTTATGTGTTTCTCACGGTGCATCAAGGGATTACATTGGAAAAGTTAAATGAT
GAGTACATGTAGTTGACTGTTGAGACATAAAAAGAGGCTGTTTATGTTATGTTTCTTAGT
ATATTACTGTAACTAGTGAGGTTCCAGAAAAGAAACACCAATATCCTTATCTCTCTCTGC
GATTAGTGCTTTTTGGTTGCGAGTTGTATAGTTTTAACCTCTGCAATGCCTCTTTAGGTG
GCCTTTCTTCTTCCCTATTAACTAGGTTTTGTATCCATGTCTTGGCATGTCTGTGACATA
AGAAATTTTCCGCAGAAAACTAGTTATTCTTGATTTTTTTGTTCTATTACAATTATATATGT
TGCTCAAAGAAAATGAATTGAACAGTCTGTAGCAATTGAATGGGTTTGCCAGATGAGTT
CACTCCAGTGTTATTGAAGGCCATTGCTTGGTCGTTTAAAGTTATGAAGCGATACAGCA
AGGGCTATCGCTTCATCGCTGAAGCCATGCACTTTAGAGAAGTTTGCACTTCAAATAAT
GGTGCACAAAGTGATTCCAACGAGAATTTATTGTTTCATTGAACATCAACTTTTAGAAGT
TGGATTAATGTTGGCATTTTCACAAAGTGATTCCTGTTGGGTATAAAAATAATATTCACG
GTATTAGTGATAAACGCGGAACACTAAGTTATGCTTAAATCAGTAAGAATAAAAATGCAG
CAAAAATGACACCAAGATTTTACCTAGAAACCCTTCTGAATAAGGGAAAAACCACGGCC
AAGAAGAGCAACTGATATCACTATAGCGAGGATTTTACACTGTGTAGTAACGAGTACGA
ATACTCCTAAGACCACTACACCCTCAAAAGAAATAAACACTCTTTTGCTTTTTCACCTCA
CTACAATATCTCTCACACTCTATTTTCTTTACAAACTATTTTCTTATAGTTTATGGAATACC
TTGCTCTCTCTTTTTTCTCTCTTTGTTGGTGTGTAGAAATGAGAGTTAAAGCTCTCCTTTT
ATAGCCAAAACCTCACTCTCTAAAGCCTACAATATTTGACAATTTGCACTCCCTTTTCAC
AATTTCAACAAGGTTGGCTACCAAACCAAACCAAATCAATAAAATTGTCTACCAAATCAT
ACCAATTCAACAAGGTTGGCTACCAAACCAAACCAAGTCAACAAAATTGGCTACCAAAT
CATTTGAATGAGATGGAAACCATATCAATCTCCCCCTCCAGTCTCATTCATCTAGAGGA
GGTAGCACTGTCTTCTAGTCTGAGTGCATGCCGACAAGTTCTTTGCATAGCTCGAACTT
GTCTCTTGGTACCACCTTGGTCAACATATCAGCAGGATTTTCACTTGTGTGAATCTTTTT
GACCTGAAGTGATTCGTTCTCCACTTGCTCACGAATCCAATGATATCTGACGTCGATGT
GTTTTGTTCTCGCATGGTACATGGAGTTTTTGCTTAGGTCTATTGCACTCTGACTGTCGC
AATAGACAACATACTCCATCTGTTGCAATCCAAGTTCTTGAAGAAATCTCTTGAGCCATA
TCATCTCTTTGCCAGCTTCAGTAGCCGCAATATACTCTGCTTCAGTTGTAGATAGTGCG
CACTTCTGCAACTTCGACTGCTATGATATAGCTCCCCTTGAAAAAGTAAACAAATATCCG
GTAGTGGATTTTCTGTTATCAAGGTCACCTGCCATATCAAAATCTGTATAACCTTTCAAA
ATTGGATTTGATCCTCCAAAACATAAGCATTCACGAGAGCTTCCTCTTAGATACCTGAGT
ATCCACTTTACAACTTCCCAATGCTCTTTTCCTGGATTTTCGAGAAATTTGCTAATAACAC
CGACTACATGAGCAATATCTGGTCGATTGCATACCATTGCATACATCAAACTTCCGACA
GCAGAAGAATAAGGAATCTTGGCCATTCTCTCTTTTTCCTCCGTTGTTGTAGGACACATC
TTCTTACTCAACTTCAAATGACCAGGAAGGGGTGTGCGAACCAACTTAGCACTTTTCAT
ATTGAAGCGCTCCACTACACGTTCTATGTACTTCTCCGGTGACAAGTAAAGCTTTCTTTC
GTTTCTCGAACGAGTAATTCTCATGCCCAAAATCTGCTTAGCATGACCCAAGTCTTTTAT
TGCAAAAGACTTATTCAACTGTTTCTTCAACTCGTCAATCTTGGATGCATTCCTGCCCAC
AATCAACATACCATCCACATATAGCAAGAGGATGATAAAATCATCATCAGAAAATCTTTG
TACAAATACACAGTAATCTGAAGAAGTCTTCTTGTAGCCTTGCTCCCCCATAACAGACTC
AAACTTCTTGTACCACTGTCTGGGAGCTTGCTTCAATCCATATAGACTCTTCTTAAGTTT
GCATACAAGATTTTCTTTACCTTTTGCATTGAAGCCTTCAGGTTGTTCCATATAAATCTCC
TCTTCTAAGTCACCGTGAAGAAAAACAGTCTTCACATCCATCTGCTCAATCTCCAAATCA
AGATTGGCAGTTAAACCAAGAACTGTCGGAATGGAGGACATTTTCACGATAGGAGAAAA
TATTTCGTCAAAGTCAATACCTTTCCTTTGACCAAATCCCTTGACAACCAATCTAGCTTT
GTATCTGGGCTTCAAACTATGTTCTTCAGCTTTAACTTTGAACACCCACTTGTTCTTCAA
 
AGCTCTCATGCCCTTAGGCAATTTCACCAACTCATAAGTATGGTTCTCATGCAGAGATTT
CATCTCATCTTGCATGGCTTCAATCCATTGATCCTTGTGCTCATCTTCTATGGCCTCCGC
ATAACATTCAGGTTCTCCCCCATCAGTGAGTAATACATATTCATTGGGTGAATAACGGG
AGGAAGGAGTACGAGGTCTAGAAGACCTCCTGAGTGGAATATCTAACTCGTCCACAGC
TTCGTGAGTAGGAGCATCTACCTCATCAACATTAGCATTGTTATCACCATCACCATCAAT
ATGCTGATCCAGAATATGGTTCTGGGCATCACCATCATCATTGAGCCCACCAACGTCAT
CCACATTTGTATGAGGAACTTGATCAAGATTAACTAAACCTTCAGAACTTGAAGATTTTA
GTTTCTCCGCTTTGTTAATATCTTCAATGGTTTGATCCTCCACGAAGATAACATCACGGC
TTCTCACGACCTTCTTCTCAATTGGATCATATAACTTGTAACCAAACTCATCAAGGTCAT
AACCAATGAAGATGCATTGCCTTGTCTTGGCAGTTAATTTTGACCTCTCATCTTTAGGCA
CATGTACAAAAGCTTTGCAACCAAACACTTTCAAGTGGTCATAGGAAATATCCTTGCCAT
ACCAAACTTTGTTTGGAACATCACTTTGCAAAGCAACCACAGGGGAAAGATTAATAACAT
GTGCGGCGGTCAACAAAGCCTCACCCTAAGAGGAATTCGGCAACTTTGCTTCAGAAAA
CAAACATCTGACTCTTTCCATCAAGGTCCTATTCATCTTTTCTGCTAAACTATTAAGCTGA
GGAGTCTTAGGAGGAGTCTTCTGGTGTCTGATACCCTGTTGTTTGCAGTATTCGTCAAA
CAGTCCACAATATTCACCACCGTTATCAGTACGAATACACTTCAGCTTCTTTCCAGTTTC
TCTTTTAGCTGAAGCCTAGAACTGCTTAAAGACACCCAACACTTGGTCTTTAGTCTTCAA
GATGTAGACCCAAAGTTTCCTTGAGCAATCATCAATAAAGGTAGCAAAATAAAGTGCAC
CACCCAAAGTCCTTGTCTTCATTGGACCACATACGTCTGAATGCACCAACTCAAGCAAC
TCGTCTTTCTTGAAAGAAGATGAGACTGGAAAGAAACTCTTTTTTGTTTTCCAGCCAAGA
AGTGCTCACATTTTTCTAATTTTGCACTTTCAAAATTTGACAACAATTTCTTCTTGGCTAG
AACATTTAGTCCTTTCTCGCTAATGTGGCTAAACCTCTTATGCCATAACGTTGAAGAGTT
ATGGCTCTCAACGGCATTCACCATATCAACACAGGTAGAGGTCGTAGTCCAATATAGAC
CACGACGCTTTTCCCCACGAGCCATAATCATGGAGCCCTTAGTGAGCTTCCACTTTCTA
GCACCATTGGTACTGACATATCCCTCATCATCCAAAACACCAACAGAGATCAAGTGCAA
ACGAACATCAGGTGCGTGCTTTACATTGTTTAAAACTAGTTTAGTTCCAATACTAGTTTC
CAAACAAATCATTCCAACACCAGTCACCCTAGATAAGTTCCAAAGTCACCCTGAGTATA
GGATGAGAAAATCCTTCCTTGATGTCACATGAGATGCGGCACCACTATCCACAACCCAG
CTTGACTCATCACAAGCAATATTTATCAAATCCGCATCAAGGACAATAACAAGATCTTCT
GTAGTGACGGTGGCCATACGATTGCCATCTTCTTTCTGTTCTTCCTTGTCTCTATTCTCC
TTTTTCAAAATCCGCAGAACTTCTTTGTGTGCCCTTTCTTCCCGCAATGATAACACTTAA
TATCTTTAAGTCTGCTTCTGGATTTGCTTCTATTATGTTCTCTATTTTGAGAACCCCGATT
CTTGCTTCTCCCCCTAGAGTCAGTCACCAAGACATCTGATGGGGAGGAACCTTGAGATT
TTCTTCTCATCTCTTCATTTAAAAGACTGCTTTTGGCAAGATCCATAGAGATCACACCAT
CCGGAGCAGAATTTGATAATGAAGTTCTAAGAATTTCCCAAGAACTTGGTAGGGAACCA
AGTAGAAACAGGCCTTGAATTTCTTCATCAAATTTAATGCTCATAGCAGATAACTGGTTC
ATGATCCCCTGAAAATTATTCAGATGATCTGTCATCGCAGAACCATCATGGTATTTTAAA
CCCAACATCTGCTTTATCAGAAACATCTTGTTGTTTCCAGTTTTCCGAGCATACAAACTT
TCAAGGTGCTCCCATAGGGTCCGAGCATGTGTCTCCCCAGAAATATGGTTCAAAACATT
ATCGTCAACTCACTGTCTAATAAAGCCGCAAACCTGCCTGTGTAACAGATTCCACTCTT
CATCTGATTTATTATCAGGCTTTACAGTGGCGAAGACAGGTTGATGAAAATTCTTGACAT
AGAGCAAATCTTCCATTTTGCCCTTCCAAATGGCATAATTTGTGCCATTCAAAGTAACCA
TTCTACTAGTGTTGGCTTTCATCGTTTATCACAAATACAAATACTATTTATTATGAGACCA
AAGTAATTCTTTTCTGATGTGGAAGTTCAGACTGTGCTGCAACCACAGAGCATACTCAN
NNNNTATTTATTATGAGACCAAAGTAATTCTTTTCTGATGTGGAAGTTCAGACTGTGCTG
CAACCACAAAGCATACTCAAACAGAACCTTGGCTCTGATACCACTTGTTGGGAATAAAC
CCCGTAAAAATAATATTCACGATATTAGTGATAAACGCGGAACACTAAGTTATGGTTAAA
TCAGTAAGAATAAAAATGCAGCAAAAATGACACCAAGATTTTACGTGGAAACCCTTCTGA
ATAAGGGAAAAACTACGGCCAAGAAGAGCAACTGATATCACTATAGCAAGGATTTTACA
CTGTGTAGTAACGAGTACGAATACTCCTAAGACCACTACACTCTCAAAAGAAATAAACA
CTCTTTTGCTTTTTCACCTCACTACAATATCTCTCACACTCTATTTTTCTTCACAAACTATT
 
TTCTTATAGTTTATGGAATATCTTGCTCTTTTTCTCTCTTTGTTGGTGTGTAGAAATGAG
AGTTAAAGCTCTCCTTTTATAGCCAAAACCTCACTCTCTAAAGCCTACAATATTTGACAA
TTTGCACATCCTTTTCACAATTTCAACAAGGTTGGCTACCAAACCAAACCAAGTCAATAA
AATTGGCTACCAAATCATACCAATTAGTAATGGTGCACAAAGTGATTCCAACGAGAATTT
ATTGTTTCATTGAACCTCAACTTTTAGAAGTTGGATTAATGTTGGCATTTTCACAAAGTGA
TTCCAACTAGAATTGGTTGCTTCTTTGAACCTCAATTTTGAGGAGTTGCATTAATACGGG
GATTATTGTATATTGGATGCTGAAATTAGTTATTTCAACTGCAATTTGATTTTTATTGTAG
AGTAAATTAATAAATGTTTGATATTTTCTTGTTTTCTGTTAATTGTGCGCCTCACTTCTCG
CTATTCACTGCAAGTCTGTGGACCTTGTTTTATTTTGTTGCACGTTTTGGTTTTAAGAAC
ACTAGGTCACTCCTACCTAGGGGTGTCAATGGATATTAGAAAACCGACTTAACCGACCG
AACCGTACCGTACCGAACCGATTTTTAGGTTTCTTTTAAAGAAACCGTAGGTTTTTATAT
AAATCTATAATCGTACCGATAATTAGGGTAGGTTTTTTATTTTATAAAAATAAACCGAAAA
AATACCGAACCGTACCGAATAAGTTTTACATATGAAAAATATATTCATATAGTAAGTTTAA
AACTAGTAAAGTATTAAATTTTTCATTGGGTCTTGGAATTATGAAAACTGTTACAAGCCAA
TAAGTAATTAAACTCAAAATACTAATTCCTAAAACNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNCTTTTATATAATTTAGATTTATCTTTTTAAATATTTAATATAGACTTTA
TTCTTGAGTCCCAACTTGGTTAATATCTTTCCACTCGTGTGATTTATATCTTCTTTGCTTT
TACTTGGTTTCTTTTCGTTGGTGTCGAATAGTTGTGTATTTATACTCTAGCCATCTTTCAT
GTTTTTTAATTCATTATCCTTTAAACAGTAAAAATGTCTAGAGAGTTTCGCTAAGTCCTAT
AAAAGAACGTACGTTATTGCATTCTATTTTTACTGGTGAATTTTATATGACATTTAAAAAA
TACCGAAAATTAACCGAACCGTACCGATACCGAAGTGAAACCGACATGATTGGGACGG
TTTCGAAAAGTCTAGTTTTGGTTATACATAATAAAATAACCGAAAAATTGGTATGGTATAA
ATTTTATAAAATAAGCCGAACCGAACCATTGACACCCCTACTCCTACCTTAATGAACAAC
ATTTATAACTTATCGAATGTTTCCACAAGACAGAGATCGTATTTGGCATAGTAATTTTAAT
GTGATTTCATTCGTGATAAATTTTTGATTTTCCATATAGGGAAGTCTGCTGCAATTCAGT
ATGGTAGTGGAGCTATTTCTGGATTCTTCAGTCAAGATAACGTCAAAGTTGGTGACCTT
GTTGTAACAGATCAGGTGAGTGAGGCTCCTCACTTGTTTTAAGTGTCTTGAAGGTAGAA
ATCTTACATCTCAAGGTGTTCATGTACTAGGAATTTATTGAGGCAACCAGAGAACCCAG
CGTGACATTTTTGGTAGCCAAGTTTGACGGTATATTGGGTCTTGGATTCCAGGAGATTT
CAGTTGGAAATGCTGTTCCAGTGTGGTACGTGGACAGCATTTAGTTTGCTCTCTTTCTTT
CCCACAAACCAAATTAAAGATCTAGACAATTCTTTTTTCCAGATGAGGTTTAACACATTG
AAGGATTAGATTTCCATAAATGCAGGATAGGCTGGATGTCTCTTTCTGTTTAAATTTGAT
TTGGCATTCATCTGGGCAGCATCTCCTGTCTTTTCCAGGTACAACATGGTCAAACAGGG
TCTTATCAAGGATCCTGTCTTCTCGTTTTGGCTCAACCGAAATACAGAGGAAGAACAAG
GCGGAGAAATCGTATTTGGTGGGGTTGATCCTAATCACTATAAGGGAGAAATAACTTAT
GTTCCAGTCACACAGAAAGGTTATTGGCAGGTTTGTCATTCCGTCAATTCGTTACTATGT
ATGTTCATGTTTTGTACAAATGCTATCTTAATCTTAGTAATATGATTGGCAGTTTGATATG
GGTGATGTTCTTATCGATGGTAAAGCTACTGGTATGTTTTGCTCTGTACCTTTTGAATTG
GATTGCTGAATTTTGCGAATATAGATGAGGGCTATGTGCCTGGATATAGTCTTCCTTTGA
GTTTTTAACATGAACATATCGGGTGTAGGTTACTGTGAAAGTGGGTGCTCTGCAATAGC
GGATTCAGGGACTTCTCTCTTGGCTGGTCCAACGGTATTATCTATAACCTTGATTTTGGA
CATATCGCTTTTTTTGTTATGTTTTTGTTATTTGTTTCCACCATCAAAGTCAACCGCAATTC
GATTATTGATTTCCTTGTTCCTTTGAACCTTCAACAGGTTGCTCAGCAAGAAAAGTAACA
TTTTCCTAAGATTCATGAGTTAAATAGAACGAATCCAGTGTCTGATCTGTGTTGTGTTTT
AATTTTTCATCACAAAGAGGATGTTAGTGAAATTGGTTTTTCATTTCTGAGGTTACCTTTG
GATATACCATCTTAGCTTATCACTGGGTCGATGTCTGTAAATCTGCTTTCTCTTTCCTGT
CTTTCAGCTTAATATGTGTAAACCTGCCTCAGTAGGTACTTGTTGTTTTGTGTTACTTGC
TTTTATTACGCTTCAATGAGGATGATGCTTTTACTTCTGCTTTAGTCCTAGTGTTCTGTC
GACTTATTTATTTACCCCTCTTCTATTTGAAAGAAAATTATCCAACCCGAAAACTGTAGC
 
CAGGTTTTATCCTGTTGACTTGGAAATGCTGACAATTAAATGAAATAAAAATCTGCTGCC
TTTTGTCTCTACAAATTCAGGGGCGAACGTTTACCCAAACGGTGTCATCCGACACCGCT
TGGTCGAAATTTTTTACTGTATAGACATATATATGTGGAAAAAACAGTACGCAATATAA
ATTATAAATGACCATTTATGCGTGTAGCTGTCTCTGGTATAATGGTCAAGTGCTGTTTTT
TCCCTACTTGACTTGATGTCGTGGGATTGACCTCAACGGATGGCATTTTTTTATTTCAAA
TTTTTTAGCATGGTCCTTTTAAAAACTAGAGTTTATATAGAGTTGAACCTTCAATATCATC
TTAAAAAAACAACTAAATCTGGGGGACATGACGGCTTCCACGTTATAAGTGATAAAAAAT
TTATTTGAATGCATAAAGGAAGCTGTTTAAGCATACATAATATAATTTAGAATAATAATTT
TTAAAAAATTATTCGACACCGCTTACTAAAAGTTCTGCGTACGCCCTTGTCTACAATTGT
ACGATTAAAACACTTCCAAGGTTGAGCAATCTTAAGCCCACCCTTTTCCGTACAGGCCA
TAATCACTATGATTAATCAAGCCATTGGAGCCTCTGGAGTTGCTAGCCAACAATGCAAA
TCTGTAGTGGAGCAGTACGGGCAGACGATCATGGATTTGCTGTTAGCAGAGGTGAGCA
TTCAACTGTGTAACAGCATTTTCATTTGTTTGATATGCAGTTTCCTGTGCAGTTACAGCA
AAATGACACTTGGCAATCTAGTTGCCTTTCCCGGTTATCTAATTGGTCCGCATTTAAACA
GGCACATCCAAAGAAGATTTGTTCACAGGTTGGAGTATGCACCTTTGATGGAAACCGCG
GAGTTAGGTTAGTCTTCAGGCTCTCTCTGCCCCTTCACGTGAACATATTGTGCATTTTGT
TAATCCATAATGTTGATATAAATACTGAATAATTCTGTGGCCTTTTTTCTGCAGTATGGGA
ATTGACAGTGTTGTAGACGAGAAAGCTGGCAGATCCACAGGACTGCAGGATGGTATGT
GCTCTGCTTGTGAAATGGCGGTCATATGGATGGCGAATCAACTGAGACAAAACCAGAC
TCAAGATCGCATATTAAACTATGTGAATGAGGTAAAACATCTGTCACTGCAATTTTCTCC
TTTTCTTTGAAAAGAATGCTGACTGACTGACAGCTTTGCGAGCGTCTCCCAAGCCCATT
GGGGGAATCAGCTGTTGACTGTGGAAAGCTTTCTTCAATGCCTAAAGTCTCTTTCACAA
TTGGTGGCAAAGTGTTTGATCTCTCCCCAAATGAGGTATGCTTTATAATGGTGTTTGCG
AGTAATAAACTTTGCTATGCCTCTTGTAATTCAACATTGTCAGAGAGAAAAATTAAGTGG
CTCATCATAGAGTCTTTTTGTGGCATTACAACTAATTGTCATATTACAAAACCGATTCTAG
TGTTTAAGGTTTTCAGTTTCTACAGAGAAGTAGATGTTTGTTGTCATCAAGAAGTACTTC
TATATTTTGAGGTTGTGAGTATTTCGATTTTACATCAATTTAGATGTCTCATTTTCCAACT
AAGTAATCTAAGAGAACCTCCGTAAGATCTTTGTGCCGTGAAAAAGTTACTCTTTGCTAT
TTGTATGGAAGTGTATTACATGCTGACCTTGCTAATGCAAGCCGCTTGAGAAGGCCCAA
TGAATCGAGATTTAGATGTCGTCCCCCCCACCCCCCGGGCCCCAAAAATAATAATAATT
TATATCTGGATGATTCAGCATGGAACAATTCTCTACCTTCATAGACAGGGGTAAGGTCT
GCGTACACACTACCCTCCCACACCCCACTTGTGGATTCCACTGGGTTGTTGTTGTTGTT
GTTGATTCAGCATAGAACAAATCTTACAAAGAAGAAAATTCATCAAGTGTACACCAAATC
AATTTGATCATTCACGTTTTTTCTAAATTTGTTTGTTTTGTGGTTTAGTAAACATAAAGAG
ATTGAGCTTTGAATAATTCAGGATAGATCACATCTTATAAAGGAAAAAATTCAAGCGAGA
CACCAAATCACTTGACCATACGCAAAACTTTTCTTTACCAGACCATCACTCTAGTCTTTT
CCTGCCCTTTCAACAAATTTTATTCAAGAATGAGAGTAAAGTTGTGATCCGATATGTGGT
TCTGTTTTACTTGTTTCAGTTGTTTGTTTAACTTGCTGACCTAGATTGATCTATATTCTAA
TTTTGGATTCTTTTCCCGTGATCAGTACATACTCAAGGTGGGCGAGGGTGCTAAGGCAC
AATGTATTAGTGGTTTCACTGGCTTGGACATTCCTCCTCCCCGCGGACCTCTCTGGTAA
AGTTATCTTTATATGTTCTCTCTATTTTAGTTTATAATTATGTTTTTTTGAGATAACTATCA
GCAATGTAGTTAATCCAACAATAAGCTTAATCCGAATTATAGAGCTACGGCACAATTAAA
CCCGAGCCAGCAATTCTTTTGTTCACTGAGTTCTCTATGGTATTCCTTTGGTTACAGGAT
CTTGGGTGATATTTTCATGGGTCGATATCACACAGTTTTCGATTATGGCAAACTCAGAGT
TGGATTTGCTGAAGCAGCT
SEQ 13 
ATGACTTTTTTCAGGTCGTTCTTATTCTTTCTTCTCACCTTATTTGTTATTTCATCTGCACT
CGACATGTCCATCATTAGTTACGACGAACAGCACGGCCAGATGGGGACAACACATCAT
 
CGTACTGACGATGAAGTCAGAGAATTGTACGAATCGTGGCTTGTTAAGCACGGAAAGAA
TTACAATGCCATCGGAGAGAAAGAGAAGATTTGAGATTTTAACGATAATTTAAGATT
CATCGACGAGCACAACGCTGAGAACCGCTCATATAAACTTGGGTTGAATCGATTCTCTG
ATCTTACCAACGAGGAATACCGTGCCATGTTCGTAGGTGGACGGTTGGATAGAAAGAC
GAGGTTGATGAAGAGCCCTAAAAGTAACCGTTACGCTTTTCAGGCCGGCGAAAAGTTG
CCGGAATCCGTTGATTGGAGAGAGAAAGGCGCCGTTGCCCCTGTTAAAGATCAAGGCC
AATGCGGTGAGTTTTTTTCTTCTTCAAAACTTTCCTACTATAAAGGAAAGCTCTGCTCTTT
ATCGTAAACATGTACTTTTGTTTTGTCTGCTTACGGAGTGAGACCAAGAGGAAGAGTTT
GGATAGATTGTTGAAAGGAGTCATATGTAGGTCAAAAGTTTTTGATTTTTAGGTTGTTTT
TTGACCTATGTTGTCGTCTTATACGGTCAATGATCTGTTATTGGGTAACTAATGATTCTG
TTTTCATGTTTATTTCAGTCAACAAATTGGAGAATAAATTAATTGCTGCTCTGTCTGGTAG
TTAATCTTCATGATATACACCTAAAGCTTACATCCTGATTTAGTATTTGGTGTCTCCAATT
GGAATGTTTATTTGCTTTGCTAGTGTTTCCTCTCTCTCTCTCTAGGGTAAATATAAAAAG
ATCTAAAATTTAGAGGTACCTGGTGTATATCTTAATATATTCCATGTACAAACTTTAAAAA
ATTATTTAAGCTTCCCCTAATTTGTTTAATACGCTGATAAGGGGTAATCAAAAAGCATAA
AGATTAGATTGAACGGACACAGTATATATTTTGCTTTTGCAAGTTGATCAGTTTCTTTCTC
CATTCTAAATCGGAATCGACCAGAATTTAAAGCGGTATAACTTAAGATTAAGCCATGAAG
ACATATTTGGCTATTCTAGGTGTTATAAATTTTAACCCAAGTGTCCTAGGGAATTGATGT
TTAATCTTGCTTTGATTATGACGAAACCCATATCTCGATTGGTTAGATATCAGTATATCTA
TGTTATGTATAGAATCCTCGTTTGAAATTTGAGATTTTCTTATGAAGGGAGTTGTTGGGC
ATTCTCAACGGTTGGCGCTGTTGAAGGAATAAATAAAATTGTAACGGGTGAATTAATTAG
TCTGTCAGAGCAAGAGCTTGTTGATTGTGATAGGAGTTATAACCAGGGATGTAATGGCG
GTCTCATGGATTACGCCTTTGATTTCATCAAAAATAACGGTGGCATTGACACTGAAGAT
GACTACCCTTACCATGCTCAAGATGGCACTTGTGATCCATACAGGGTAAGTAATTAACC
ATACTATCAAGAAAACATCCAAATATTAATTATGTACTATTTCAGAATGTAAGTCTATATA
GCAAGTAATTAATAGTATTTGCTGACAAAATTTGGTCATTCAGAAAAATGCCCGTGTTGT
CTCCATTGAAGGGTATGAAGATGTTCCAGAAAACGATGAGAAGTCGTTGATGAAGGCA
GTGGCAAATCAACCAGTTAGTGTTGCTATTGAAGGTGGTGGCAGAGCTTTCCAGCACTA
CTCTTCGGTATGGTGGGCGGATCTTGACTAATATATCCTTCTGAATATATATGTTATTTG
TGTCTGAACTCACTGGCCCTAAATTCTGGATTCGTTATTGCATTTTAGTATGCCTGTGTC
CCTAATCTGCAAACACGGCTGCATTGTGCCTTGTTTTACTACTTAAAGCTAGTATACTCA
TTTACCCTTCCAATTTTTATCAAATCATGCAGGGTGTTTTCACTGGATATTGTGGAACGC
AACTAGACCATGGTGTAGTTGTAGTTGGCTATGGAACAGAAAATGGCGAAGATTACTGG
ATTGTGAGGAATTCATGGGGTGCTAACTGGGGAGAAAGTGGTTACATCAAGCTTCAGC
GCAATTTCGCTAATTCTACAACTGGAAAGTGTGGAATTGCAATGCAGGCATCTTATCCT
CTTAAGTCTGGCGCAAATCCTCCTAATCCTGGTCCATCTCCTCCTACTCCTGTAACACC
ATCAACTGTTTGCGATGAGTACTATAGCTGCCCACAGGGCACTACTTGCTGCTGCATTT
ATCAATATGGCGAATACTGTTTTGGCTGGGGATGCTGTCCTTATGAGTCTGCTACCTGT
TGTGATGATAACTACAGCTGCTGTCCCCATGATTATCCTGTATGTGATGTTGATGCTGG
CACTTGCCTTATGGTAAATATTTTTTCCCTCCCATTCTGCTTTTTTCTCCTTTATAATAAT
GATCGTCAATTTCACTTATTACGTGTAATATTCTACCAGCACAGGATTAATTAGATAACT
CTGTCTACCAAAACTTTGGCAGATATTTAAACCTTCGTCTTCACTCGTTTATTGACCGCT
AGACCCACGTACAGATTCAACCTTTTATAGGTTTAATCATCAATGCAAGACTACTTATCA
CAATCTTTTTTCTTTTTATGTGACAGAGCAAGGACAATCCATTAAAAGTAAAAGCATTGA
AGAGAGGTCCAGCTAGAGTAAACTGGTCAGGGATGAAATCTAACAGGAAAGTGAGTTA
CGTT
SEQ 14 
TCATGAAGAAACAATGATCAAATAATAGCTAAAAGGGAAAACAGAGCCATCATAAGTT
GGCAAATGTAGGAATTGAAATGTGCTGGTGCATGGTTTATTGCAGGTCTAGATGACGGA
ACAGATGGAAACGAAGTAGCGGGTTCATTTCCACTTCCATTTCCCTTGGTGGCCTCTGG
CACCACACTGGAGGGCGACGGCGCTTCAGTAGAATTACGTTTGTTCACTGGCAGAGTT
GTCGATTTGTCGTTGGATTCTCTAGAATCATAACCTGACAAGAATGGTTTCTAGTTAAAA
TATGGACAGGTGTGCACACTAAAAAGGTCATACTCATGAATGCAAACTCACAATCGGAT
GGTTTCCAACCCAAAACCATCTTCTCTCGATCAAAAACCACGCGATAGCCTGTCATAAA
ATTTTCTGCAAAAAGATCCATGTATTAGTTTTCTGTCATAAATTCCAAATACAGAGACAAA
ATCGAAGTAAATCAACAAATAGCTCAGATTTTTGACTATGTAACCAGTTTTACTAGTTGG
TTATGGACTTGATTCTTACTAATTTACTTTGTCACATTCACATTTCCAATAATATATAAGCT
AGAAGTATGTAAAAAACTTGATAGGAACCAAAACTTCTAAAAGTTGGTAATTGTGAGATC
ATTAGCTGGCATGGTGCAAGTTATATTGCAAAATTCCATGGATAGAAACAAAATCTACGA
GCAAGCAACTGATAGATACTTACGTCCAATGATGTTGACATCCCCACTTTTCACAACAG
CTAAGCAAAATGCGCGAGAACCATCCTGATGATCATGACTGGGTATTAGAATATTCTAA
AAGAGAACTTCTGTAATAAAGAAGGAGCAGAAACCATCTTACCTGGAGCGAGAGCATAA
TTATCGGATCGAAAAGAAAAAACTGGTTGCCGCCTTTCATTGTCAAATTTAAATCAGGAA
CTTCGAATGTAGTTTGATTTGCACTGCGTATGTAGCATGTTAATCCTGAGAGTTCAAAGG
ACATCAAGAAAGTAAAAGTGATGAAGATTATAAAAGATGGTTCACCTTAGCCCGTAGCA
GTATTCAAAAGGAATTTCGCCATCAGGTTGAATACGTAGCTGTTTTGCTTGAGAATCAAA
CTGAAAAAAGAAACAAAATATCTTCAGTTTCACAATACAAAGAACAAACTCCCAACTAAC
AAATCATACAGTCAGCCTGTCACTCACGTTCTCTGTAATGACTTTGTAAGCTGGGTCGTT
CAAGTATGTGAATGAGGTGCCAGAGTCAAAAATGGCTGTGAAATCAACATCAGTGATCT
TGTTTCCCACTGTTATTCCTGTCAAGCTGATGTTATAGGTTGGGCTGCAATTCAAGGAA
GGATAAGAGTGAATACATATTTGGTCGAATTCTCCTGAATCAAGCCAGGAACAGAGGCA
AACGCTAAACCTAGAATATCAAATTGACTGCTTACTGTAGTTGATCAAGATTGAGTGGTG
TTTCTCCTTGGTCTGGACTCCCTTTATCTCCAAACACTATTCTTCCAATACCATCAGGGC
CAAAGCACATGGAGAAAGAATTTGCAGCAAGACCTTTACTTGCTAACATGCTCGGAACA
GATATACTTTCCAAGCCAAGTCCAAATAGACCATTAGGAGCAGCGCCACTTAAAAATGC
ACCGGTTTGTCTTATCCCACACCTGAAATTGAACAAGTAGAAAATTAACATCATGGTGGA
TAAAGATTGCATCCAAATTACACTGCATTTCTCTCAAACCATACCCACCCTAGAGCAATT
GGAGCCTCAACACTTTTTTGTTGAGCATTATCTGTCTCTAAGTGCAAGATGTCTTCCACC
AGTACCCCTGATGATGAGGTATTATTGGAGAGATATGCAACTCCATAAGCACATGCGTT
TTGTGAAGATAAGCATCGCCTCCTTTGTCCACACAGAGTGCCGTTGCAAGGAACAATCT
GACCCGTTGACGACGTATTAGGGCTGTAAATATTGAGATTTATTCGCTGTTTCAAAGCA
AACAAAGGATTATAAGTGTCAAATAATACATAAAGAAACATTAAACGGAAAAGGCAAAAG
AGCAAAGAAAATTTTGTTCCTGAAGGTGAAATCCTGTGGAGTATGAAGTAATTGAGCTA
GTAAGAATCTCAAAATTCTTCAATGACAAACAAGCCACGGAACAATATGGATCAGATATT
TCATTTCAGAGTCAGTATATTACATGCATCAAACTCACTTTGCAAATATTGATTATGTTGA
ATTTTGCGCTAGTTATGTATCTTCCTTAACAGAAAAACAATTTTCTCAATACATTCTCCAC
CCCATATCTTGTTAACTAAGAGAATATATTATTGTCATAATGACGGAAAAGACAGTTGA
AGAGAAATCCACTGTGTTACAGATTCAGCCATTGTGTTATCAGCAGGTTCCTTTCTGAAA
AAGGTTATCAGACGAGGATGATCATCTTCCTTGTAGCAGAAGGCAACATACAGACGAG
GATGATCATCGAAAAAGAAAAACTCTTTAAACATTTGAAAGTAGAAAGAAAAAGGTACTA
GAATAAAGCAAACATACTCGTCCAGAGCGTGTCTCGAGGGCGCGCACACAATTGCTGC
AATCACAGGGTAGCCAAAACAAGTCACTGCCAGTGTCAAGTGCCACCAGAAATGATAG
CCCAGGAGTGCCCACTGTCACATTTGCATAATGCAAACTGCAAATTGGCAAAGAGTATT
AGTCACACACCTTAAGAAGAAAAAATCACAACTACAGATACTACATATTTTGCATTCAAC
TCTATCTTTATAACATATAAATTACAACATGCTACTGCAAGGAATTTTCAGAACAATTCCT
TGTCTAGAGAGGAGATAAGTGGCAGCAGAGGAACGGAAAATCAGAAAAAAAAAAATGG
AATTTATTTCCGGAGCATGGAATTCGAACTAGAAGAAGACTATAATTAAATTTAGAGTCA
GTACTTTTAATATAGGAGTGAAATCGCCAAAATTCCAGTCCGATGAAACACACAAACAG
 
AATTAAAGAACAGAAACAGGCCTAAATCTTTCTTTTTTTTGTTTATCATATTTTCCTCCA
CATGAATCTCGTAAGAACTATTAATGGTACATGGAATTTATTTAAGTTAGGTAACCTATTT
TTCCTGAACTGACACATCCAACTAAACAGACAAAAACAAACGCAAAGCTCAGTCAACTC
TAACATCACACTAAACGGACAAAAGTAAAAGACAGTAAACAAGAATTTCCAAAAACGTAC
TAGTAGTTCACAATCAGGAATAACAACAAAAATATTTTAAAAAAAAAATAGAGCAGCAAA
TAAACAACTGCAAAAGCAATCAGAAAAGAAAAATAGAGTGAGCTTACAATCCCAAAGAA
CTGAGGCGGAAAGTTTCATTTCCTCCGGAAAAAGAGAGGAGTGGGATTAGTTGTAT
CAGCAAGGCGGCGACCTTTGATAAAGCGATCACGCTGAGTCCAAGCTGAATAATACTC
AACACTTCCCTTCTCAGGCAATCCATGAAGGTCCAAAATACCCTTCACCGGATCCGAAT
ACCGGTGATGGATATCAAACCCGAACGTCCCAAACCCATCGCTGCTCTGCAATTGCAAT
CCCAGAATCGCCAAGAAAATAATAGGGGCAAGGAAAAAATTAAAACTTGTATAAGAATT
AGCCAT
SEQ 15 
ATGGTGACAAAGTTTAGTATTTTTATTTTGGTGGTGTTGTTGAGGTTATTTTCATTTGGTT
CTGTAGCCTCAAGGGAAATTCACAATTCTGGTCTTAATCTGAATTCTAGTGCTTCTGGTA
TTGAATTCCCTCAACATCCAAGTTTCAACTCAGTTACTGCTTCTGGAAATTCAGATTGCA
GTTATGGAACATCCAAGAAATCAACAACCACCCATGTAATAACTCAAGAAGAAAATAGAT
CTGATGAAAAAGAAGATGAAGATTTAATGGTATCTAAAAACCAGCCAAGAGAAGCAGTC
AAGTTTCACCTAAGGCACAGATCAGCTGGTCAAAATATAGAGGCCAAAGACTCAATATT
TGAGTCCACAACAAGGGACTTAGGTAGAATTCAGACATTGCATACAAGGATTGTAGAGA
AAAAGAATCAGAACTCTATTTCAAGGCAAACAAAAAATAGTGAAAAACCTACACAATCTT
CTTCATTTGAATTCTCAGGCAAGCTCATGGCAACATTAGAGTCAGGTGTAAGTCATGGT
TCAGGGGAGTATTTCATGGATGTTTTTGTCGGTACACCTCCTAAGCACTTCTCTTTGATT
CTTGATACTGGTAGTGATCTTAATTGGATTCAGTCTGTTCCTTGTTATGATTGTTTTGAAC
AAAATGGTCCTCATTATGATCCTAAGGATTCTATCTCTTTCAAAAATATAAGCTGCCATG
ATCCTAGGTGTCACCTTGTTTCATCTCCTGACCCTCCACAGCCTTGCAAGTCTGAAAAC
CAGACTTGCCCTTATTACTATTGGTACGGAGACAGCTCGAACACGACTGGTGATTTCGC
GCTTGAGACGTTTACGGTTAATCTCACAACCCCTAGTGGGGATTCAGAGATCAAGAAGG
TGGAAAATGTGATGTTTGGTTGTGGACATTGGAATAGAGGCTTGTTTCATGGTGCTGCT
GGTTTGTTAGGACTTGGTAGAGGACCGCTTTCGTTTTCGTCTCAGCTTCAATCTTTATAT
GGCCATTCTTTTTCGTATTGTTTGGTTAATAGGAACAGCAATTCTAGCGTAAGCAGCAAA
TTGATTTTTGGTGAAGATAAGGAACTCTTGAAACACGCGAATTTGAACTTCACTTCACTG
GTTGGTGGGAAAGAAAATCATTTGGAAACATTCTACTATGTGCAGATAAAATCAGTTATA
GCTGGAGGTGAAGTGCTGAATATACCTGAGGAGACATGGAATTTGTCTACAGAAGGTG
TTGGTGGAACAATCATTGATTCAGGAACTACTTTGAGCTATTTTGCAGAACCAGCATATG
AGATTATAAAACAGGCATTTGTTAACAAGGTGAAGCACTATCCTGTTTTAGAAGATTTTC
CAATTTTGAAACCATGTTACAATGTTTCTGGAGTGGAGAAACTTGAATTGCCTTCATTTG
GGATAGTTTTTGGTGATGGAGCTATATGGAATTTTCCAGTAGAGAACTACTTCATCAAAC
TTGAACCAGAGGATATTGTTTGTTTGGCAATGTTAGGAACTCCTCATTCGGCCATGTCG
ATAATTGGCAACTACCAACAGCAGAATTTTCATATCTTATATGACACCAAAAGGTCAAGG
CTGGGATTTGCACCAACAAGATGTGCTGATGCC
SEQ 16 
TCACATAGGAGCAAGATGACCTTCTTTAGACAATTTATCTTGCATCCACCTCTGAAGCAT
TTCCATTGCTGCCTTAGGCTGATCCATTGGAACCATGTGACCTGCATCATGGACCTTAA
GGAAAGTTAAAGGTCCATAGTTTTTTTGAACTCCTTTCTCTACACCATCTACTGCAAAAG
 
AAACTTGTGTGGCTTTTCCAAAGGCTTTTTGCCCTGTCCATTTCATTGCATGCACCCATC
TCGAATTCCCTGTCCATATAACAAGAAATAGAGTTTTATAATATTATTGTTAGTTGGTAGC
TTTGAATTGACTTATAAACAATATGATAGAGTTCAACTTCTATATTTTGACAGACGTAAAA
GATAACTTGAAGTAACTTTTGTTAAAAGTAGAATTACTAGCATGAAAAAATAAGGTAGGT
TAAAATACACTAATATAGTATGAAAATCCTCTTTTGTGTATATAAGTTAAATCCATATGAT
AATATAAAGCTTACCAAGCCAATTGCAGATAAGGTCATATTCCCCAGCATACACTAGTAG
CTTGATACCATCCTCAAGGAGTGAAGGAATTCCCAATTCAAGATTCCTCATCCAGTCCA
ACTGCATTGCCTGGTAAACTTCAGAGCTACATGAAACAAACTCAATATCCCCAACACCA
AGAGCCTTTTTAACTTGTTGATCATTGAGGAAAGTTTCCATTTTGGAGAAATCATAGCAT
AGATCGCCCTCACATCTCTTCCGCACATCATAGTACTGCAACTCGGAAATTACAAAATTC
ATATGACTTTAACTTTTGTATACTGACAGTGTAAAAAAACTTTATTCTATCACGTCACTT
AAAGGATTGTAACTATAGATACCCGTTCATTATAAGTGAGATCAGTAATTTGGAAAATAA
GACAAGTAATATGTTGTACATCATTAGCTCAGAGAAAATGAGATTGGTCTTTCTTACGTT
TTTGTCACCAGCAATGTCCATAATCTTGTTGAAGATGCTTGTACAAACAAGATATGCAGC
CATGCAAGCAGTTCCGCCATCTTTTCCTAATATTATTTGCATAGAAGAAAGCTAATGTAA
AGACTAGCTGCTGCTATATGAAGAAGGAAAAGGACATTGAGGATAAACAAAATGAATTA
CCACAAAGCTTAATTGCTAGTTGACATTTTGGATATGACTTCTCTATGGCATTGTAATCA
GATTTTTTGATCAATTTCATATCCAGAGCATAGTCAGTGTAGGCTTTGTATTGAATTTCTG
GATCAGTGAGTCCATTACCAATAGCAAATCCCTAAAAAAAATTGTACTTTGTTAAGTCAT
TGGCATGACGACAAATTCAAATTAAACCTAACTAAAGGTAATTACTGGATAAAGAAAAAG
GGATGATATATGGTAAGAGTTAGAAATACCTTGAGATTTACGTAGATTCCTTCTTTATTTT
TGTTTCCTTGGTGAACCCGAGAAGCAAATGCAGGAATGTAATGCCCAGCATATGATTCT
CCAGTAATATAGAAATCATTTTTTGCATACTGTGGATGTGCCTTGAAGAAGGCCTATCAT
CAAAAGAATTTGAAAAAGTTTGAATTAAATTTTATTAATTATATCAGTTAAACTTTAGAGAT
TTATCACGAGCTAAAAAAAAGGAATGAAAGAATAGGATCAACCTGCAAGAAGTCATAGA
GATCATTGCTTACGCCCCTTTCATCGTGACGAATATCATCATCGTTTGAACTATAACTGA
AACCAGTTCCAGTTGGCTGATCGACGTATATAAGATTTGAGACCTGTAAAATTGCAATTT
ATCATATGTTATCATTCTTCAACTAACAAAGGAAAGTTGCATGTTTGATTATAGGATTTAA
CCGGTGTAAACGATTTTTACACTATTGTTATATTTTAACATGTTGTAACATGTTGTATTCG
TCCCACTTAAATAAAGTGAAGAGAAGCGTAGTAGTCATTGATGTCAATAAACGTTGAACT
ACTTTCGAATTTTTGAAATTCTACAAGTCACAGCTAATGAACAACAAGTGTTAAAGAAAA
AAATGCTAGTAGGTAAAAAGGTATTTTGCATGATGGAGAAAGGTTGAATAACAAATAAAA
ACATGGAGGGAATTCTTTTAGATTTTTACCATATTCAAAAGATCTAACTGACGTTTCTTGA
GAAATTAATTGGGTAAAATAAAAAGAATAAACTGAAAAAAAGAGAGGAAAAACAAAAGA
AAAAGCAAAAGGAAGAAAACAAGAACCTTGTCCCAGCCGAAATCATTCCAGACAAGAGA
CATGTTATCTGCAATTTTGAATGGTCCATTTTCATAAAACACAGCCAATTCACTGCTACA
TCCTGGCCCTCCAGTTAGCCATATAACTACTGGATCATTCTTCCTGCTCCTCGATTCAAA
GAAAAAGTAAAACATCCTGCCAAAAACAGATAATTTAGCATTAATTAATAATACCCATAAA
TTCATTTTTTTACCAAAATGAAGCAAGAAGAACATTTAATCCAATTCAAACCTTGCATCTT
TAGTATGTGGAAGACGATAATAACCAGCGTGATGACCCAAGTCTTGAACTGTAGACCCA
GAATTACCAACATAAGATAAATTCAATTTCTTTTCAAAAAGTCTCTGTTCAGTAACTGCTG
CAGAATCCCCTGTTGCTGCAGCCTTGTTGATATCATGCTTAGGGAATAAATTAAGCTGT
CTGATTAGCTTTTCTGCCATTGTTAATGGGAATTTTGGAGTAGAAGATAGGAAAAAATCA
TCATCATTAGAATTTAAAGTTGATGAGAAAGATAAGGAAATAGAAGCAAGAAGCAGAGT
AAGAAAGAGAAGAGAGAAAGATGAAGGCAT
SEQ 17 
TCACACGACACTTTGGGGTGGATATCCTGGTTCACAACCCGGGTGTGAAATCCCTCA
TTATCAAAGTAAGGGTCACACTGCCAGAAAAAACAACTTTATTAGTGGTTGATCAAAAAG
 
ATCCACAGTAGCTAATGGTTTTAGTGGAAGCTGTAACCTCTGTGAGGTGAAAATAAGTC
AGCATTTACCTCTTCTGTGACCACACCCCTACGGATAAAGTATCGCCATGCTGATATAG
GATATCCACCATCACAACCACTCCCACGTAAAAAGCCACAGCATGCTAACAGATCATTT
ACGGACAGAGAGATATTCTGCAATACACATTAAAAGTTTAGCATCAGTGACCATAACTAC
AGAAATACTTCACAAACATTTTGTGCTAATTAAGATAAGATGGTTTCCATGTGCTTGGCC
CATGAAAAAGAATCAGGGCTCGCACGTGAGAGAGCATGCTGAAGATATATAAATAACTA
AATAAAAATGTGTCATCTAAAGCTTTTAAATGAGGCGGTCACACAGTGCAACAGAATTTA
CCAAGTTATGATGGATACAGAAACGATCAGACAGAGATTCAACAGCACCAAAAGCCCAA
CAAGAACCGCAATGTCCCTGATCTGTGATGCAAATTTGTCCCGGTGATTGATGTGCAAA
GATGGAAAGCATTAGGATCACTAAAATAGAATTATAATTCAGTAGTAGTAAGCAAGAACA
AGAAGAAACTGACCTAGAATTCTTCCGATAGTGCTACATTGAGGCCAAGCTTTTCGTGC
ATCAAACTCTTTTGGTAGCTCCAAAAGCTTTGGATGAGTTAGAATCGGAATTCCCTCCAA
ATCACCTTCTCTTGCGGGCTTAACTCCAAGAAGGCGCTTAAATTGTGAAACCTGTGAAT
ACCAAAGATGGAGATAATTATAAAAGCATATTTTCATTGTATAGCTGCAAAATAAGCAAC
TGTTATTGATGGTTCGAGAAAGCGCTAAACTATATGATGATATGACGACAAGAGGGGGT
TGCTCCGATGGTCAGCATCCTCCACCTACGACCCCAGGATTGTGGGTTCGAGTCACCA
AAGGAGCAATAGCTCCAACAAAGAGGATCACAGGGGAATCAAAAGGGGAGGGGAATTT
TAAAAAAATGATGATCTGACCGTGAAATTCGAGAATCGAGGGTTGAATGCAGCTTTCCA
TCCAGCTTTGGCATTTTCATTAACCTCTTTGATGATTGATTCCTGCATGATCATCCAAAA
AAGCTCTCTCAGTTTTCGAATTGAAGGACAAGGGCTATAACATCTTGAATCATGAAAAGA
AGAATAATGTACCTGAAGGATTGCAGATTCAACTTTAGCTTCAGATATTGGCTTCTCTGC
AACAACCTGCTTTCCATAAGAACACATCAATTCTATTCATCAATAGACAAGCTAAAAGCT
TTTAGGAAACAGAGTTGCAATTCCAGGAACAAAAGTATGACAGTACTGTGACAAACAAA
GAACTAAAGGTTTCACTTTAATCAAGATGAAACGCTTCCAACATTTCTTATTTTCGACATT
ATAATCCTCTTAACTTAGGAAAATTAATAAAAAATTTGTAGCAAATGCATCATATGTCTAA
AGCACTATAACATAGAGGAAGAACCATAGAACAAGCATAAATTGTAACTATTCCATTATT
TGTCCTCCTTTTTCTCCCCTTTCTGTATTTCTTTGTGAAGCAATACTTCCTCTCATGTTAT
ATATAGAAACATGTAAGTTAGCTAACACATAAGTAATTTGCATCAAACCATATATTTAACT
TCAGAAACATGTCTATACTTCTGTTTTCTCATTCTCACTAGGTAATAAGAAAATCATTAAA
ATTTATTTCTACTCATGATTTCAAGTCAACGCTTAACTAAAGCATAAAAAGTCCAAAATAC
CCAACAATATTTGATCTTTCTGAAGAAATACAAAAAGGGTAATCCATGTAATCATCAAAA
CCTATATAAATTAAACCAATAATCTAAATCCATCTAAACAAAGAAATACTCTTACTGTAGA
AAGGTTCAACGAATGAAGAAACAAACCTGCAATATAAGGATACAAAAAGCACCCAAAAA
CAAAGGAGCTGCTAAAGACTTCAGAGTCAAGGTCAT
SEQ18 
ATGTTCCGACTAGTAATGGTGACAAAGTTTAGTATTTTTATTTTGGTGGTGTTGTTGAGG
TTATTTTCATTTGGTTTTGTAGCCTCAAGAGAAATTCACAATTTTGGTATTAATCTGAATT
TTAGTGCTTCTGGTATTGAATTCCCTCAACATCCAAGCTTCAACTCTGTTACTGCTTCTG
GAAATTCAGATTGCAGTTATGGAACATCCAAGAAATCAACAACCACCCATGTAATAACTC
AAGAAGAAAATAATTCTGATGAAAAAGAAGATGAAGATTTAATGGTATCTGAAAACCAGC
CAAGAGAAGCAGTCAAGTTTCACTTAAGGCACAGATCAGCTGGTCAAAATATAGAGGCC
AAAGACTCAATATTTGAGTCCACAACAAGGGACTTGGGTAGAATTCAGACATTGCATAC
AAGGATTGTAGAGAAAAAGAATCAGAACTTTATTTCAAGGCAAACAAAAAATAGTGAAAA
AACTACACAATCTTCTTCATTTGAATTCTCAGGTAAGCTCATGGCAACATTAGAGTCAGG
TGTGAGTCATGGTTCAGGGGAGTATTTCATGGATGTTTTTGTTGGTACACCTCCTAAAC
ACTTCTCTTTGATTCTTGATACTGGTAGTGATCTTAATTGGATTCAATCTGTTCCTTGTTA
TGATTGTTTTGAACAAAATGGTCCTCATTATGATCCTAAGGATTCTATCTCTTTCAAGAAT
ATAAGTTGCGATGATCCGAGGTGTCACCTTGTTTCATCTCCTGACCCTCCACAGCCTTG
 
CAAGTCTGAAAACCAGACTTGCCCTTATTACTATTGGTATGGAGACAGCTCGAACACGA
CTGGTGATTTCGCGCTTGAGACGTTCACGGTTAATCTCACAACCCCTAATGGGGATTCA
GAGATCAAGAAAGTGGAAAATGTGATGTTTGGTTGTGGACATTGGAATAGAGGCTTATT
TCATGGTGCTGCTGGTTTGTTAGGACTTGGTAGAGGACCTCTTTCGTTTTCGTCTCAGC
TTCAATCTTTATATGGCCATTCCTTTTCGTATTGTTTGGTTAATAGGAACAGCAATTCTAG
TGTAAGCAGCAAGTTGATTTTTGGTGAAGATAAGGAACTCTTGAAACACCTGAATTTGAA
TTTCACTTCATTGGTTGGTGGGAAAGAAAATCATTTGGAAACATTCTATTATGTGCAGAT
AAAATCAGTTATAGTTGGAGGTGAAGTGCTGAATATACCTGAGGAGACATGGAATTTGT
CTACAGAAGGTGTTGGTGGAACGATCATCGATTCAGGAACCACTTTGAGCTATTTTGCA
GAACCAGCATATGAGATTATAAAACAGGCATTTGTTAACAAGGTGAAGCGCTATCCTATT
TTAGATGATTTTCCAATTTTGAAACCATGTTACAATGTTTCTGGAGTGGAGAAACTTGAA
TTGCCTTCATTTGGGATAGTTTTTGGTGATGGAGCTATATGGACTTTTCCAGTAGAGAAC
TACTTCATCAAACTTGAACCAGAGGACATTGTTTGTTTGGCAATTTTAGGAACTCCTCAT
TCGGCCATGTCGATAATTGGCAACTACCAACAGCAGAATTTTCATATCTTATATGACACC
AAAAGGTCAAGGCTGGGATTTGCACCAAGAAGATGTGCTGATGCC
SEQ 19 
TTACAAAGGTTGCTGAGCTATCCATCTTTTAAACATATGAAAGCTCTCTTCACGTTTGTA
CTCAGGAGCTGTGTGCCCTCCTCCCTAAAGAGAAAAAAAGACAAGAAGGAAGAACAAA
AACATCTGAGAACTGTGAAAATGTGAGCACAAGACAATTTTACTATCGAGTGTGAGTAC
AAAATTCTAACCTTTACTGTTGCATATGTCATATGATTAGAGAAAGATCTTGTGTAACTG
CACATCAAAGCTATAAATTTAGAATTTCATAACAATTATATCGTCTATCCAACTGGAATAG
AGATCCAGAGAAATTAAAATGGAGAATCATACCCTGCAACTTGACCATCAATTGTCCAA
GGGCGCCAATCATCAATGATAGAATAATTTAGATACTTTATCCATGCTTGCGTCGATTGG
AAAGGAACAACCATGTCATGATCACCACTGGGCAAAAAAAAAAAAAAAAAAAAAAAACTT
TGTTATGTATATAATAACTTCAGCTAAATCTTTTTTGCATAAAGCACAAATTAGAGAACTA
CAAACATTGGTTTAGGTTTAAAGTATCAAACCTGTATATGAGTGATCGATAACCTTTGGA
ACTAAGGTTAACATGGTAAGGTATACTATTCATGAAAGTAACTCTATAAGTTGTACCCAT
AATACTTTGCCTACATCTCGCCCACGCTCTTCTTATAGTTCCCTAATCAAAGAAGAACCA
AAAAGAAAAGAAAAAAAGCCTTTTAATATCTTGCACCTTAATGCCAAGCAACCTTCACTA
TGTACAAGTACAAACAATTGAAATACCTTTCTAACATGGAGAGCCTCTTGAACACTAGG
GTCATTTGCCCAATGATTGGAGAGCTTGCGTGTCGCAACCTAAAATCAGAAAGCATATG
TATATCTAAGTATCCTACATTTATCTGTTTATCCCGAGAAATCATGAAAAAGAACCGGGC
ATATATGCCTCTTTTAACATAAATAGGTATATATCTTAAAATTATTAAACAATATTCAAACT
TTATTCGGTGTAAAGGCATTTAGTTGCTGCAATATTCAAACTTTATATAGTAATATTTGAG
GGACTTACACGACTTTCTCGACAAATGAAGTCATCATGTTTTAGAAAGATAAAATCCTCT
TCAAGAGATCTCCTTTCACCAGACAATTGGCGTGGGTTTGGCGATTCTGAGTCCGTTCC
ACAAAAAGGCTCTAGTATTTGCTGATCATTTATACTGCTTACAAGCTACAAAGTTGCATT
ACATGAACATTGTGGTATTACCTAATTACAAACTTAGTACCAATTCAAAACATTAAAACAA
GAATATGTAAGAAGGAATATAACCTTACCTTCTTGAACATTTTAAAGTTTTCTAAACATAG
TTTATTGGTAGGATCAATGTTTCGGCAATCGCCTTTGCAAGTCTCCTTCAGTGACTGATA
CAACAAGATCCAAATTATCATTTTTGTGCCAAACTGTGTTGGTATGATACAAAGTTAATAT
ATGAATTGAAGTAAGAGATGAACCAACCTCATAAAGTTCATTAGATATTAGTCCCATACC
ATGACAGAAAGGAATTTGGTAATTGCTTTCTTCAGGAAATGTTAGCGGATTGCCAAGTG
AATAACCCTGAAAGGATATTAATTACCATTAAAAATAATAATTTTTCATGTATGAGATATA
TTTTAAATAAAAGGAAAGGAATAGAAAACCTTAAGGTTGATTAGTGGCTTTTTGCCTGCT
TCAATTCCTGCATTTTCATATCACTCTTGAAGTGTCAACAGAGAACACTTTGGCTAGAAA
TGTTTTGAGAATCTTCTCTTCAACCAAGTGAGGCTACACATTCAGTTTTGAAGAGACAAC
AAAAGAATGTAGTGTTCTATATATCTTATCTAATATCTGTGGAAGTAATGATCAATAATAA
 
CAAAGCATAAACTTGGCTTAGAGCCCTAGGAATGGAGAACCTTTGATCAATAGAGTCAC
TTTACTCCCCCTAGTGGACCAATTCAGCATTAATTGCCAGCGGCTTCAAATAACGAAT
GGCTAAACCGGAAAAATAATAATAACAAAGCATACACTAAAGAGTGATGGGGTGATGAG
AACGATCACAACCGCACATTATATGAAAATACTAAATGACACTTACCATCTGATATTAGT
TGAACAATAACTGGAACTGTAATGCCTGAATATGAGTCCCCAGAAACATAGAAAGGGTT
GGAAATGAATTCTGGATGATTATTGAACCACTGCAAAATAAATCACAACTTATTTCGCGA
TACATTTTTGTTATATTTGGTTGCTATTTAATTATTACTGTCTTCATTTCTTCACGGTTCAT
TTGATCAATCCACCAAGATACACATATATTAGGATTTAGGTGGGTGGAGGGAATTCAAA
TACGTATATATTGAGCCTAAAAAATTTATATCCTAGATCCTCTACCAATGAACAATGCAAA
ATGAGAAGTATATTGAGGAATACATGAAAATTCAGAGTTAACTCTGCTTACTAATTCAAT
CTAAAAAATACTCAGTTTACTAATTCATATGTATCTAACTAAATGAACTTGAAATGATTTT
CAAATACCTTTAGTAGAAATTCATAGACCTGGTCGCACGCTTGTAGATCAGTACACTTG
GATGCCGCTGAAGTTGTTGCATATGAGAACCCAGTATTTACAGGCTGTTCCAAGAAAAG
TATGCTCGCAAACTACCAAGTTTAAGGAACACATTAATTTGATGATCTAATGTTAACCAT
AAGAGGATAAAAGAGAACATGAAGTGTGGATTAGAATATATATGCAGTTTGTTGACAGC
AAAATGAAGTGTGCAATAGAAAAATAACATGCTCTTCAGTGTTACCTTTGTCCAGGAATA
TGGAGTTGAAACAAGAATTGGTAGGCTCCCATTGTATGCCTTCTGACCAAAAGCCAATG
GCCCTACAAAAGAGAATTCAGAAGTTAATTTTCTCCAACTATGAGTTACACGTAATACCA
AGCTTTTCCACACCTATATATTTACTACTAATTGACTAATTAGAAACATCGACTAAAAGTA
AATTGCTTTTGTGATACTAACAAAAGCATTTCTCAAATGAGTTAAGTTAATCACAAAAGGT
TAAACTCTATTTGACAAAAATTACATTTGAACACAAACAATAATGGTAACTGTTGGGATAA
AGATAACCTCCAGACTATGATTACATATTTAAGGTGAGTTACTGATAAACATACTTGATA
ATACAATAGTAGTATAACTAACATACTATCATAGGTTAAATAATATTTTATAAAAAATATTT
ACATTGTCAGTATATACAATATAAATATTTACCTACTTCATACGCCACACCCGTGAAGGA
TGAGCAACCAGGCCCTCCCGTTAGCCATAGCAAGAGTGGATCTTTTTTAGGGTTGGATT
CTGATTTGACAAAGTAATAGAATAGTTGCACTTCCTCGGATTTGCCAACTCCAATATATC
TAACAATTGTATTCAAAATACATCACTTCAACAAACTTGTTTTACTACTCCACTATATATG
TAGCCAGTATGTTCTGAATGAAGTAAATTACCTAAGAAAAAGTTTAGATTCTTTTTATACT
AATTGATCTTTTGATCAAATACAAGTTAAAATTCAAAGGGTGGTAAAATTAACGTACCCA
GTCTCAAGATAAAAAGGAAGAGGGCCATCAAAACCAGGAAGAAACTCAACAGTTGAGC
TATTCTGAGGAAGACTTTGTACATATTGTAGAAAGAGAGTAAGAGGAAGAAGAAGATGA
AACAATAGTGGCAGGCGAAAACCAGACAT
SEQ 20 
TTAACCAGCTAGAGGATTCATCACACTGCCAATGAATAACACCGCACCAGTAGATTCGT
CTCTTATAAGGAATAGGAATGGATGGTCCGCAACAAAATCCATTTCCTTCTCAATAATCA
AGGACATGGTCATTATTACAGTAGCGGTAACAGCTGCAGCTTCGGTTCCTTCCTCATTT
ACCTCAATGAAAGACTTGTGAAAAACCTGTGAAACAGACAGGTTCTGAGGCATAGGAGA
ATCAACCATCTCAGTGAGGCTACCACCACAAAAAGGCAACGTGAGGCCGAGTCCCTTT
AGAATGTTGGAAGCTTCAAATCCAAAAGTTATTTTAAATTTAGGGATAAGAAACTTGCGC
GCTCTAACTTTTCCATATGGAACATGGTTATTTAAAAATCCTGGTTCTAAGCTGATTTTTT
CCAGTAAAGCAGGTAATCCATCATGGGCATCTGGGAGAATGAAATACATACAGAAGCG
ACGCGTATCCGTGCCTTGTTTATAAGGAAGCCTCAATATTTTAAAGCAATCAAACGCTG
CTATGTACTGCTTCTTCTTGCTAGTCATAAATGGTGCTTGAATAGACCCTCCATTGAGGA
GATGGAAGTCATGATCTTTCGTTTCTGACACATCGAACTTCTCATTCCATTCTCCTTTGA
AATATAGTGCATTGGACAAGATCAGCCTTGTCATGTTGTTCACTGCATCGCGAGGAAGA
ATCTCTTTGATAAGACCATTTGTCTCCAT
SEQ 21 
TTAGAAAAAAAGCCAATGCTTCTTTCTGCGCACTCTAGCTGGAACCTCTGTGGCACATT
ATGTTCAAAGTAAAGCAAAAATTAGTATTCAAGAATAGCTATGACAAAAAATTCTGAACT
CAGAAATAGTTAAAGCAAGAAGACACTTACCTCCAATTTCGTATAACGAATTGTAGTGAA
CTTCGCTCCAGAAACTCAGCCACAATTCTGAAAATCAAGAATAACAAGTCAAAAGTTTTG
TCTTCAAGAGATTAAACATATCGAGAAGAAATGTTCGTCAGCGCCACACCAACACATTT
CCGAACAAGTGATTTGGAATTTATTTACTTGTTAAGATAAGAGTTAATCTGATCAGGTTT
ACTCAGTCTTTCCTAAGATCACCATGGATGTGTTTGTTTATGAAAATATAATCAGCAGAA
CATCATCAAACATGTTAAAGGGGAGCTTTGGAGCAACGGTAAAGTTGTCTCCATGTGAC
CTATAGGTCACGGGTTCGAGCCGTGAAAGCGGCCACTAATGTTCGCATTAGGATAGAC
TGGCTACATCACACTCCTTGGGATACATCCCTTCCTCGGACCCTGCATGAACACGGGAT
GCCTTATGCACCGGGCTGCCTTTTTTAATCATTAAACATGTTCAGCATATTCAATTTCTT
GAGAAAACAATTTTAGCATAACAAAAGAGAACTATTAAAACAGGAGCGACGATCTGATG
TTACTTTATTGGCAGCGGATCAAGTTTATCGTCTGTAACAAATAACGCTTAATAAGTGCT
TGCATCAGTAAATTGTGTGTTTCACGTGAAGCAAGACTCTGGAGATAAATTCTCCTGATA
GCAACTAATACTGCTCTTAGTAAGAGCATCAGGAAACTCTGTACAAAGCTTACATTCACT
AATTGTAAACATACAAATGATCCACAGTTCACAAATGACAGCGAAGGATTCTTGTCATTA
GAATCAGCCTCGTTCAATGATGCAACAATGTCATTACAAATTAAAGGTGTTTTAGTTTTA
CTATTTAAAAATTAGTTAGTACCAATTCACTTCGACATACTTCCACTCACAATCTATTGTG
TGTGGTACCAAGCAATACGATGATACTTTCTCCTAAATGAGAAAAGCACATCCATTTGTC
AAAATAAACAAAATGATTCATTTTTGTTTTCCTTTTTTTTCATTTTTGTTTTCCTTTTTTTTT
CATTTTTGTTTTATATATGTATTCTTATAATAAAGTGGGAGCATGCTAGAGAGTTCAAAGT
AGGACCATGCTACAAAGTTCAGAAGAATACTTTTGGTTTAGTGTCTCAAACAAAACCAG
CAAGTACTATTATTTAAATTCTGGAATTTATATCATAATATCATTATTTTAGAGTTATTTGT
AAATTTCAAGTATTTATTTTATTTTTTGAGTTTAAAAACTCAAGCAGGAAGTTAAAGAACT
AATAATCAGAAGTATTGATTGTGCCATGATCATTAGATACAAATAACATAAAATGTATGTA
CCCCTAGAAGGTTGTATGTCCTTGGGAAGAATATCAATGTAGCCGTTGTCTCGGAAAGA
CGTGACCAAACATATTTTTACCCCAAACTGCAAACAAAATACAAGCAAACACAAGTACCA
TCAGTCACCCAAAACGGGAAAGCAAAAAAATAAAAATGCAGAGGGCATAAGGCGAAA
GGCTGATTAACAACACAAATATCTAAAGCAGTTCCACAAAGGTAAAAGAGCAAGAGAAT
GGTCATATTGCGGGAAATTCTTTAGGTAGTAGATCTCCTGCTTTTAAGTTGCTTTCGCGT
TTGATGCTATGTTGAACTTCAAATATATTCAAGTTTGAACCCATAATTTCTAAAGTGTAGC
AAATTTAGTGGTAAGAACCTAAAAGCTGAACCCACCAAACTTAAATCCTGAATCCGCCTT
CGTATTTGATGTCTTAGACATTTGTATGCCTTCTAAGCGTAAATGTAGGTTGACAAGATG
AAACAGGGCAAATTTAACTTTTTTTCTCATTTCTGCTCTAATATCTACAGTTAAATTTCAG
TAACCTACATGCAACACAGGCATACTGCACAAAAAGTCAGGCTAAAAAATAAGAAGATG
CCGGCGTTTGTAAGAAAGTAAGAGGATTATTAGAAATGTTCTTGGTTATTTTCTCCGACA
GCCTTGGGCAAGAATAAAGCTCTAAATTTCACCGTCAAAGAGTTCTAATCAGGAACCAT
TTTAATACATTGAGGGGTTTTCTCTTAAGGAAAAATAATTTTCATAAAAGCCAAATGGTTA
AAGGTTAACAGCTGAGCCGAATTGCCTTAATACTCATAACTCTTAATGCTACCATATTAT
ATACCAACACCAACTCAAAAATTCACTTCCTAAAATTTTTCAACTTTTTCACAGGAAACTA
TTCCACTATGTGTATTATGCAATGTTAGATACTATTAACAAAGTTGCTTCATTGTTTTTTTT
CTCATTTAAAAAGCTTAAATTTGCAATGCAACAGTTCCACTATCCAGGCAATACACCTTT
ATTGACAGTATAATTTGTTGGCTTTTCTTGTTCGTAGTGTTCAAATGCTGTCTTACATACT
AACAAGTGTACCGTATATATGCCTCAAAAGCTAGTTGAGGACAAAAGAGAAACTTTCAA
CTTACTCTATCGGCAGCGGCTTGTAGAGTGACATGATCTCCCCACTCTCCCAACCTGGT
TAGAGCAAGAATATGTTGGTAATATATTGAGATTTCCCGCAATAAGAAGCACTGAAAATT
GACCAGACAGCTTACCTCTTCATTTTCCTCAAGTAGCTTTTGTATCTCATGGGCACATAA
CCTTCATATAACTTTCTAAAGCGCTTTAGCTGAAATGGATTGCCAACTAGGATAAGAAAC
GTGTAGGACATAAACTAGACATAGGCGTCAAACTCAAGCAAGTATAGGACTCTGTTGAT
 
GTTTAATAGATCCAGAAATTAGGTTAAGCAGTTGAGCTGATGAGTCTCAAGCAAACACC
GGGATATTTTATTTTTAGTACTCTATTATTATGCACAAGTTACTTTAGTTCTACTAAAACT
CACGAAAACGAATATAAACTGCAGGATTTGTTAATCTAGGATGAACACAATCCTCAAACA
TAATCATTATGTAATCTCAACTTTTCACCCATCCTGTTTCACAACCAAAGGCAAGAGATG
AGAAGCAAATATTGAAAAAATGCAAACAGACCTGTTTAACGACCTCCTTCCTTACATGCT
TATGATACTCTGGATTATGATACAACTGATCCGAAAGGGCCCGAAACTACAATAAGCCA
AGTAGACAGTCATATAGCAGGATTAATCACCTGTGAATTAATAAGTAGCAGGTGGGTCT
CAAGTTCAATGAGAGTTTGTCGACTAGTCAAAAGAGACCGACCTGGCAATTTCCATCTC
CTTCAATTTGCATTTCAGCAAGACCATATGTCGCTAACCTGAAACAAAGAAGTTACAAAT
AGGATCCGAGTTGCCAAAAGGCGAAAAACATAGAAAAGCACCAAGAAATTTCACCCTTT
AAGTGCTAAGTGCAATTAAAGCATGAATTAAGCTATATAGAAAGACACTAATGAAGAAAA
AGATAACCTATGAGCAGAAACTATAATAATTTGGACAAAGAATTGAAAAACTAAAATTAA
TCAATAAATATGAAGTAAAAATCATGTCAATTAAGTTCCATAATACTGGATTCACAAAATA
AGTCAACCAATGACCTCAAGCTGAACAACTATAGCATGCTCAGTAGTCAGTACAATGAT
GGATAACCTGCTAGAGAGCCTCCCATGGTCTAGTGTGGCATCATTTGGGTCAGGTATCT
CCCCAATTACCCGTGGAGTGTGCTGCAAGGATAATAAGACTATAAGTAGCATGTAGAGA
TAGTGCAACATGGGTTTTCATGGGTTAAAGCTCAAAAAGGAATATATAGAGAAGCAACC
TTTGGATACATACATGTGGTCACATTATACTCATTTAATTTGAAAGAGTTGCCCCCAAAA
ATGTAAATTTAGATAATTGTTCATAAAAAGGTCAAAAGTTCCCCGGATTTCTAAACTTCTT
GAACATTCTCCTTCTTCCTCTATGGATAGGGTGACGTGACGCGCAATGGATCAGTTAGA
AAGGAGAAGAAACTGATCAAGTGTCCCCTTCGCAACAACCTTTTTCCCTTTCTCGCATC
CTTGGTCAAGCTTGAGATTTTCTTCTATCTGGATTTAGTTACCCGAACCTATGCAAAGCA
AGCCTACACTCTAGCTGGGGCCAAGCCATCTTTGCCTAAGTTCCATTACCTCATTAACT
CGATAAAGCTGGCATTAAGAGAAGCTTGGTAGCATAAAGTGTCAAGGAGGTGGTTTTCG
CCTATCTATAAGGACAGTTGGAGTTGATGGTCCTGGTTTTGTTACAGTAAGGGATGTTG
CAGGAATCACTTGCATTGATGGAACAGAAGCTCTTCCTGCTCTTGTATTCGACGAAGAA
TAGGCCCAAGGGACATCAATGGACAATCGCCAAGCACATGGTTTAGCTAATTCAATAGC
CTTCGGAGAAAAGTGATAAGTACCATCGAACAAGCAAATCACTTCTGGATTATTAACCTT
TAAGGATCCACTTATGGTGAAATTTTGGAATGATGCGGGGATAACCCAAGGAGTCAG
CTAAACAGAGACGGAAAATATCAGTACCATTCTGGTCGGCGTATGCTTGTTGAAGAGAA
CAAGCTCACTTCTAGCTAGCTAAAGGGAAAGAGAGATACTATAGAATACCTAAGTCAAT
TTATCATTGAGCTGCAAAGATCATAGCAGACACTACGAATTAATAGCAAATCTCGGCAC
AAACCTCTACATGTCAAGATAGTGAGTCCATCAGATCACAAAAGACTTAATACCAGATAC
CTTTCTAGATAAAGGGGTAAAATCACTTTCTTTCTTCCACAAGATCTCCATTTAAAGAGA
CTATCAACTGTACTTAAATGTAATCATATCAACTGTACTTAAATGTAATCATACCGGGATT
GAGTCCAAATGAGAAAGTCTCCTCCCAAGTTTATTGCCACCATATTTGAGAGCATTTTCT
TCTTCTTCAGCTAAAATTCTTGCAATGGTATGATCATCCTCAGTACCGTGTGAACTACTA
TTCAAACTAGATGTAGTAGAACTCGAGCTTGCTCTTGAATTTCCATAGGATTCATTCAT
SEQ 22 
CTAGAAAGGGTAAATGAGACCTCCGAACTTCCCAGAAAATGCTTCCTTCTGAGGCTGTT
GCACTATAGTTGCATTGCTCATTCGCTGCATTTAAAACAAATTAACTGTGAAAACTACAG
TAGCAAAAGGTTAAAGAAAACGAACATGAATAGCACGTCAAGAGAAATTGGCTTTGCTT
TAACGGTTATTTCATCTCTGTCAACAATGAAATGGCAATGAGTGAACCTTTTCAGAATAA
GTTGGCTTATCTCATTAATGAGAGACAGATAACAAGAGATGTCTCCTCTAATCTCTAAAT
TGATATTTCATGTTGTATGGATCCTAATAGGATGAGAAATGCATCAAATACAGAAAGGAA
TGGCAGAAGTGGAGATATACCTTGAGGCAGAGGTTCCGACTTGTGTCACAAATAGGAT
AATCATGTGGGCAACAGTGGCGTCCATCTTTGCAACACACTGCAGAATCCAACCCACAA
CATTTCCAAGAAACGCAAACTCCAAGAAGCCTCCACCCACAGCAGCAGGTTTCACCTTG
 
ACCACATGAGGTAAACATACTGCATTTGCTTGGACCAGGAGATGGAGGAGATGGTGGA
TTTGGGCTACTCTTAGTTGGATATGAAGCTAGCTTATTGATCCCACATATCCCTTCTTGA
TTCCCACTATTACGCTGCATGTGCATATAACCATTTATTCCCCAGCTTGTTCCCCATGAA
TTTTTTATAATCCAGTAATCAACTCCATTTTCAGAACCATAGCCCACAATCAGTACCGCA
TGATCAAGTACTGTAGAACACGGTCCAGTGAATATCCCCTGCAACACAAAGATAGCACC
TTTATATTTCTCTCCGACAAACAATTTAACTGATTAGGAGATTGGTAATTTGGAGATGGA
AGATACCTTTGAATATGATTGAAATGCTCTCTCACTGCCGCATATCCCAACACTCACGG
GTTGATTTGCCACCGCCTTTAGAAGCTTGTCCTCATCATATTGGGGAACATCAGTATATC
CATCAATGGTTACAACACGTCTTTGTAGCTGCAAAGTCGACAAGTTAAGCCAAGCAATC
ATATGTTAACGTTGTTCATTATGTTTTATCTGGAATAAACTTGTCCTAGGTTCTCTATATT
AATTATGAAATCCAGAAGCAGGAGGGCATATATAGGACAAAAAAAGATTTAGTACAAGA
ATAGGAGCAAGAAGATGAGAGAACAATTATGTACCTTGTTTTTGTTGCATGTTCCTTCTC
TTTCATTAAAGGGGTAATCCTCTTCAGTGTCAATACCACCATTCTTTTTGACAAATTCAAA
AGCATAGTCCATCAATCCACCTCCACAGCCGTCATTGTAACTTTTGTCGCAATCAATTAA
CTCCTGCTCAGAGAGACTTACAAGAGATCCAGTGACAATCTTATTGATACCTTCGATTG
CTCCAGTGGCTGAGAATGACCAGCAAGCACCTGGGAAAATGAAACAGAAGTAACTGGT
TTTAGTTACAGAAGCTAGTTGCTGAGATTAAGTATATGGAATGACAATAGAATGACAGTG
TTGTGGACAAGGGCAAATTTGATTCATATTATCTCAGAACAAATTCACAAAAAGGCTAGA
TCTTCACTTCCGTCCTATATTCAGGCTGACATTACCAGACATATCTACAGAAAATAATTA
CTTGAGAAACATAAAGGCAGTGATAAATTTTAAGAATAAACTATACTTAGTGAGAATTGT
GTGCAGTCATAAAAGTAACAAGTCTAAGTCCCTGAAGCAAATTCTGCATTGGGAGGAAA
GTATATTTCCGCGTATATGACAGCCAAATTAGTTGCTATAAAACATCACACTAGTATGTG
ACTCAATATTGACAGTAAAATTATAAAACATGTTCCTTCGATTGCACAAGCAAGTAGAGA
ATCATAGGGTACAATTGTGTACACAGTTCCAAAAACAAGAAAGAAGAGCTAAAACAATG
AATTGTGAGTCAATGATTCAATGGTCGAAAACAGGACCAAGAAATGGATCAGTGGATAT
TTATATTTATTTCTATTTTTAAAACTTAAAGGGCATGATGTGAAGACTGAAGCTGGTAATC
CAGTTTTGATTGTGATGCACATGAATGGATGTGGAAAGTAATATTCTCTGGAAGACAGA
AGACCACTAACCACCTCAGTTGCTCAGACCAAGATAGTGAGTAGATCTCCCCTAATCTA
TTCAACAAAGTTCATTGGAAAGAAACAAACAATGAAATGGCGGATCTCCGAGCAGTCTG
GTGAAGTTTATGGTCCAGTGGTTAAAAAGAATGACAACTCAACCATATTTTACTCCTCCG
ATGTGCTCCGTTCATGATCTTTTTAATATTTCTCGCTAATCCGCTAATCAATAAAGAATGA
GATACTGTATCAGTATGTCCTATTATTGTTGTCTTCCAGTCACTCTGAAGAAATGATTTTC
ACATACATAGAGACAAAAATTGAAAGTAAGAAACAACAACAACAAACCAGTGGAATCAC
ATAAGTGGGGTCCGGGAGTATAATGTGTACGCAGACCTTAGAGGTTGTTTCTGATAGAC
CCTCGGCTCAAGAACAGTGAGAAAATTGAAAGTAAGAAACAAACAGTATATTCATTCCTA
ATCAACTCATGAAAGGACGAGCTCATGAGACTAAGTTTCAACAACAACAACCATATGATT
GTTTATTCCACTTCATCTTGATTCCAATACCTAATAATTTGTCTTTTGGGGCAACTCAAG
GGTTCCTAAAGCTAAGAATTCTCTAAATCTCACACTTCTCCTTATACAAACATTCAAATCC
TAACCAAACTGAAAGTGCTCCTGTCTAATACTGATGAACTAAAGTAAGTGCTGAGGCTA
GGTTTCAATGAAGTAAATTAGTCCTGAACTTCAACCTGTCAAATAATACAAGGAAAAGCA
AAAAAGGGTAGCTCCCAGACAAGAGAAAAAGGCAAAACTAACATCACAAGTTTCCATTG
TCATTTGAGAAAAAAATCATCAAAATCCAAACTTTGTAAAAATTTCTAATGTTGGCTCTAC
TATGCACAAGTTATATATCCTCCACATAAATGAAATCACTATAAAGATACAACTAAAAGAT
AACGCAATAAACTGAGCATACCACAACTGCCTTGATTCTTGACTTTAGTAACAGCTCCTT
TCTCTCTCCAATCCAAAGAAGAAGGAATATCAACAACACCAACATCATTAAAAACTCCAG
CAGAAGACGACCCAGTTTTCAATCTAATAAAATCATTAGCAGAAGAGGACAAACCCAAA
AAAGAGTTCTTGAATTCATGGTGAGTGAGATCAGAAAAGGCATTGAGATTAAGGGTATA
AGTGGAATTCCCCTTACTATTATGCTCTATAATATAAGCATAATTTTCTTCAAACACCTCG
AGTCTGTACACCCTTTCTTGTTCAGAAGAATATGTCTTTCCATTTTGCTGACACCAACTT
TCAAAAAGATCAGAAATTGATGAACAAGTGCAAATTGGTCCTTGAAAAATTAGAAGTACA
AGAACCAAAGATGGACATAACCAACTCAT
SEQ 23 
TTAATGCTTATTCCAGAAACTCCACTTCTTCTTCTTCTTAAAGTCAAATGGCAGGAA
GTCTGGAATGGAGCATCAAACTACAGTATTAGAATAATATGATAAGGGTAGTGTGTACG
CCGGTGGCGGACCCAGGATTTTGTGCAAGCGGGTTCAATCTTAGAAGTATATAACTTTA
GTTGTAAAATAGTAGTTGTCAAGTGGGTTCAAATAAAATATTTAAACAAAATTTACGCAG
CTTTAATCCTAATTTATACATATATACAGTATTAGTTTTTGATGCTTGCCACCACGTGCGT
CCACCACTGTGTACACATACCCCTTACCCCCTACCTTGTGAGGATAGAAATCTAAATGA
AGCAAGAGCAGCAAACTTCCGGTCAACTCTCAATGTTCATCGCTTTGTCCATAAGCATG
TGATAAACAAAAAGTGTTATTCCGTAATGCCCATGGTAACCCCCCCCCCCGGGGGGG
GNGGTTTAAACATGTAATTAATCAGATATAGGCCAATTAATAATAGTTGAGCGACCATGC
TAAAACCACGGAACTCCGGAGTACCTAACCCCCCCCCCCGGGGGGGGGATGTCCAT
GCTAAGACAAACTAAACAGAAACGGACATAAAAGTACAAGCAACTACCTCCTTGAGGA
TAGATTGAGTTGTAGTGCACCTCTGCCCAGAAACTCAAGTATATGACTGCAAGAAGAGA
AAAATAAGAGAAACAATTAAATTGGTGTGAGCGAATGAATATCAAGACTTCAAAACACCG
CTCTTACAAAGTGCACATGCACAAAGAAATTGCATTCATACTTTAATTTCTCTTCCAAGC
AATCTCAAGATTTGCTTGCCCACACTTGGCTTCATAGTATAGGTATGATACAATGGCATA
GAATAAATGACATGCATACATAACCAATATAAAGCTTGCCCCCATTCATTAAACTTACAA
CCGTCTCATAATCATTCAATATGTTAAAACAGACAAATTCCGGTCTCTAAAAGGAGAATG
TGAATGTCAAAGCATCCATGTTATGAGATGGAATTTAGATTTCAAAAGAGCTAAAACGGA
CGACTCTTCAAAAATCAAAATCTCCTTCTCATGAAACGCAAAATCGAATTTGCTTAAGAT
TGTCCTTAAGGGTTCATAGTCATCCATTCATCCCTCCTTCCCCTTGCGCAATTTTTTGGT
CAAGGCAGGCCGAGGTACTAACTTTACAGTCCAAAGATCAAAAGTACTATTTGCATTCT
TCACGACTCATGTAATAAACTTATGTTGTCTCTTTAACTCCAGTGGTCCTACTTTATCAG
AGTCGTTATCTGATTTTGGACTTCTGAAAAAGTTTGATACAAAGATCGTACTAACTTTTC
CATAGTTGGCACAAATTTCAAGAATCAAATTCATCCAGTAAATCAGGTTGCTCTGGTACC
AGCTACTTCTATAATTTAATTTACTACTATTACTACAATATGCATAATCAAATTATCTGCTT
CATCTCCATGTGTAGCCTGTGTCGTCTGAAACGCCAATGGGGGAGTACTAATTTGGTG
GTTACGAATTATCATACTATCTCCCCTTTTTGAATTGTTGAATTTGGTCCTGAAAAATGTT
GCGGTTTTGGCTAAAAGTCTAAAACTGCATTGCAGAAAGTATCAATAGAACGACATAAG
AACTCGATAATGGTTTCTCAGTTAGTGGAATTACAGCTGAGGAAAGCATCTTTAACCGC
AAACTGGAAATACGTACAGCATTAAGCGACTCGTGACTTTTTATTTGAGACACATGGAAA
TTGAGAAATAGGATCTATGTCACTCCCACTTCCAAATATTTTTGTAATAAAAACTTGTTCA
ATCGCATTTTGTGAAGTAGAGGATATTACAGAAAGGTAAAAGCAATTCAAAGTTTGAGAA
CTAGCCTACCTCTGTTTGACTTCTGATTCTTCGGAAGAATCTCGATGTAACATGTATCCT
TGAATGACGTTATAACAAGAATTTTCACACCATACTGCCCACAAGACAAAAAGACAAAAT
CAATGTGCAACACAAGTAGGTTCATTCACAAACCAATGCATCCTAGTCTAGCATCATCA
CAAATAAAATCTTCATAAAAGGAGCTGCGCATATACAATAAATAAAAAATGCATCATAAC
CACTCAAAATGGAGAGTGAAAGAAAGAAATAGCAAAATAGAGGCACATGAATTAACAAA
AGCTAGTAAAGCACCCAATGGAGGCACTATACCAGGACATCCAAATTATGGTCTGGCCA
ACAATAGCTTAAGCTTCTTATATTCCAAGGTTAAAAGTAAACCAAAGTAATCAAATGGA
GAGAAAAAACCAAGGAAGCAAATAAGGGGAATAATCAATACCGAGTCAGCAGCAGCCT
GCAACGTAACATGATCGCCCCATTCCCCACTCCTGGTTCAACCAATATATAAGCAGATG
TTGATTTAAAGGAGAAAAGATAATGCAGAATTTCAAAATGGAGAAACAAAGGTCTGAGA
ATTACTTGGACATCCTCGTCAAGTACTCTCCATACTCCATTGGGACATATCCCTCATACA
TCTCCGGATGATGTTGAAACTGACAAGACAAAATGATTTTAAAAAACATACTCAGCTAAA
ATGTATGTGAAATAATTCAAAAATGAAATCGAAATATCATAAAGAAGATTATCTTATTCAC
TTAGACAAGCACCTGGCTGACTACTTGCTGTCTGACAAATTTGTGGTGCTCTGGTGTAC
GATAGAATTGATCTGATAAAGCACGGAACTGGAACAAAAGAGGACATGTGAATAGATGT
GCATTAGAAAGAAATAGGATGGAACCTTATTTCAACAAACTTAGAATCGGAGGCGAAGC
 
CTAATCCACTTGGGAAAAATAGGCAAAGTTCCCATACCCCATAAAACTAAAGAGTTGAG
AAAAAAATTGAATTATCCTTTTGTCAAACTACTAAAAATCCACAAATATTTTAATAAAGTT
GGAATGGCAAGCAGCCTGACAATACGTCACTTGAGTTTCATATTCCAATTTTTTAAAAA
TCCTATTATGAACCACTTCATTCACCATTTCACTGTCACAAACACCAACAACAAGTTTCT
ATCAGAGCCAAAGAGTTAATTCCAAAGTAAGGAATACTGATAAACCGTCATCAAACATTA
TGTTTTTTGTTTTTCATTCCCTTTCTTCTTTGAACCAGAGAGTAAGACCTCCATACCACCT
AGCACCTTGATATACTGAGATTTTTCATGAGACAAACTTAAAGAATTGGGGATCCTTCTT
TTGTTTATGGTTAAAAATTATGCTACAAGTAGTTTAAGGAAAGGGAAAACAATTTTTTTCT
TTCACTAGAAATCAAATCATGAGCGTCTCTAGACAGTTTGATATCATTACTGCAAGACAA
ATCAACCAAGTAATACAGTAACCTGGCAGTTGCCATCTCCTTGCACTTTGTGCTCCACC
AAGTCAAATAATTGCAATCTGAAGCAAGAAAAACCATAGCATGGGATCCTTAGCAAAGA
ATAACTTGCAGCAATAATATTACCATTCAATTGCATTGGCAAAAATATCAACTTCACAGG
AAACTTAGGTGGCACAAAACCTAATAAAAAACACAACAATATCAACTTCATAGGAAGCTT
AGGTGGCACAAAATCTAATAAAGAAACACAACAAAAGAAAATAAAATGAAAAACCACATG
CATTGTCCTGTGACTTTAAGAAAACAAAGGGTTAATAGTATCTATTGGAATGTGCTTCAT
AAGTGTTTATTTACAAAGCACAAAATACTAGTTGGCTCATTCCACACCAATTATTTTCTGT
GCTTGTCTCGGCTCCCATCTCTCCCATTTGATTCTCGTTTTCTCAAAATGCTTGAGGGGT
CAGATGTTACTTTTCGAATAGCAGGCAGTAAGAACCACCAGTAAAAGAATGAGTTAATA
AAGAAAGAGAAAACCTAACAAAATAAAAATAAAGAAATGTTTCTGGAGACACAGGACCC
CTATCACAATAGAGGTATGCATTTCTCACAGTGAAGCTATATTTCATTACCTTGAAAAGT
GCTCAGAATTTGGAGAGAATTAAGCAAAGCACTATCATAAGATCATATGTCATTAACTTG
TTCATACACAATTTTTATCTACTTGAAAACCTAAGTCGGAGGGATCAGGAGATACCTATT
TAGCAGCCTTTGATGATCAGAAGTTGCTTCATCGACTGAAGGTATGTCCCCATTTATTCT
AGGAACATGCTGCATGGTTAATAACACAAATTGTTATAAGAAACTAAAGCAGCCTCAAG
AAAATGGCCATAGGTGCAAAGCACCACACATGTCCTCGTACACAAAGTGAATTAGTGTT
CTCAATATACTAACAGACACTACACTTACAGGAACAGCACTCAGCTGGTTTATTCTCTTC
CCTACTTCTCCATCAAGCTCAAATTCATCTTGTATTTCCAATGTGTAGGTATACTCTTCTC
CATCGTATGATCTGTCTCCAGGACTCGAACAAGAACTTGAAGGCCCTACATCATCAGCT
TCTAGGCTGGTGTCATGCCCTGTGAATCATATATAATTAGCAAATGTTTAAACTCAAGGA
ACATCACAGAATTGAAAACAAGAAATGTACCAGCATAATACTCTCTTGGAGGAGTATGC
CAATGTTGTACACCAGTGGAGGCTTGCAAATACTGCTCGTCTGCATGTGAAGATTCAGC
ATCTTCTGCGATGGACAACTCTGACAAATCTTCTTGTAGAACATGAGCAATAGCCTCATC
ATTGTCAACATTGCAATATGATGTGTGATAGTGGTTTTCTCTGGCATATTGTTCATGACA
TATCTCAACGTCATGCTTTCTACCATCACCGTAATAGTTGGAACTAAAAAGTTGGTCCAC
ATCGAGAAAACTAAGAACGCCACGAGCAGCTTCAGATTCCGGCTCACACAT
SEQ 24 
ATGCCTTCACTTCTTCAAATTTTCCTTCCTTTGTTTCCATTCTTTTTCTTGGTTTCTTTCTC
AGTTTCTCACGGACCCTTTTTGCCAAAGGCCATTATTCTTCCTGTAAACAAAGATCTGTC
AACTTTTCAGTATGTTACTCAAGTTTACATGGGTGCTCATCTTGTTCCTACCAATTTAGTT
GTAGATCTTGGAGGTTCATTTCTCTGGACTAATTGTGGCTTAACTTCTGTATCTTCAAGT
CAGAAACTTGTCCCCTGTAATTCACTCAAATGCTCAATGGCTAAACCTAATGGTTGCACT
AACAAGATTTGTGGTGTACAATCAGAAAATCCTTTTACAAAAGTGGCTGCAACAGGGGA
ATTAGCAGAGGACATGTTTGCTGTGGAATTCATAGATGAGTTAAAAACAGGTTCAATTG
CTTCAATACATGAATTCTTGTTTTCTTGTGCATCAACTACTTTGTTGCAAGGTCTTGCTAG
AGGTGCCAAAGGAATGTTAGGACTTGGAAATTCAAGAATTGCATTGCCATCTCAGTTGT
CTGATACATTTGGTTTCCAGAGGAAATTTGCTCTCTGTTTGTCTTCTTCAAATGGTGCTA
TAATATCTGGTGAAAGTCCTTACTTGTCACTTTTGGGTCATGATGTTTCAAGATCTATGC
TTTATACACCTTTGATTTCATCTAAAGATGGTGTTTCAGAAGAGTATTATATCAACGTTAA
 
ATCCATCAAAATTAATGGCAAGAAACTGTCGTTAAACACATCTTTGTTTGCAATGGATGA
AGGTGTTGGAGGGACAAAGATTAGTACAATTCCCCCTTTTACCACCATGAAAAGCTCAA
TTTATAAGTCATTTATTGAAGCTTATGAGAAATTTGCTATTTCCATGGAATTGAATAAAGT
GGAAGCTATAGCACCATTTGAGCTTTGCTTTAGCACAAAGGGGATAGATGTCACAAAAG
TGGGGCCAAATGTGCCAACTACGGATCTTGTGTTGCAAAGTGAAATGGTTAAGTGGAG
GATTTATGGGAGAAATTCAATGGTGAAAGTAAGTGATGAAGTGATGTGTTTGGGATTCT
TGAATGGAGGGGTGAATCAAAAGGCTTCAATTGTTATAGGGGGTTACCAGTTGGAGGA
TAATCTTTTGGAGTTTAACTTGGGAACTTCTATGCTTGGATTTACTTCTTCACTTTCAATG
GCAGAAACAAGCTGTTCTGACTTTATGTTCCATTCTGTATCAAAAGATTCAGCTTTTGAT
TCT
SEQ 25 
TTAAGAAGAATGAGAAGTAAACTTATTTGTTGAATTTAAGAGGTAAGAATATGCAAAAGT
AGCATGAATTGCAGCACCAATTGGAAGGACATCCTCATCAATGATGAAATGTGGATTGT
GTGGAGGGTAAATAGCACCAATCTTTTCATTTTTTGTTCCCAAAAGGAAGAAGGAACCA
GGAACTTTCTCTAAAAACACTGCAAAATCTTCACTTCCCATGAAGCTAGGTGCTATTTTG
AAACTCTCTTCCCCAACAATCATTTTTGAAACTTTTCGGGCATGTTCGTATATTCTCTCAT
CGTTTATTGTTGGAGGAAGTGTTGGATTTTCTCGACCATCAAAGTCAATCTCGACCGTA
CATCGATGTACTGCTGCTTGTGCTCGTATCACCTGAAAATTTTACCAATAAAAAGTTTAA
TTACCAAATATTGAATATAATAATATGTTCTAAAAATAACATGGATGTCTATTCCTAATTAT
TAGCAAGTTATTTCATTCTCCCTAGTTGATTAGTGAATTACTGAAAGGTTATGGCGTTCT
GATATTGTTAAATGTACCACTTATTTTATTGAAAAGTTATATTGCATCTCTAAAGAACTGA
AAAGTCATTTGACCTCTTGGCTCGTGACCCTTCTCAAAAAACAGTTCTTTGGCTATAAAT
AAGATTATTTTGTGTTGAATGAATATATCAAGCAACTTGAAAATATTAAAACCTCTTTCCC
GAAACAAATCCTACAATTTCCTCAAGTACTCGTCTTTTCAAACAAAGTATTAATGAAAAA
GAAACGTAACTTGTTTGAATAAATAAAATTTGCACATATAAACTTTGTAAAGAGACGTGT
GGGACTTTGAATATTGGTCAAAGTATCCAAGATTTTTGTTCTAAATTACAAGTAGTTTAC
CTCTTCAATTCTTTTCCTCAAACCGTAGAAACTCTTCTTACTGAATGCTCTATAGGTCCC
GGAAATTGTAGCTAATTCTGGTATGATATTAAATGCATGCCCCCCTTCAATCATGGCAAC
AGAAACTACCTGAAATTTCAAAAACTAAATATATAAGAATTGATAAAATAAAAATTTAAAA
TTTGTTTGAATAAGTTTGGAAAAGAAAATTGTTAATCTCTATGTTTCAAAAAGATTGTTCT
AGTTTGACTTGACACAAATTTTAATAAGGAAGAAAAGACTTTGAGATATGTGGTCCTAAA
TAAACCATATCATTTGTGTGACTGTAAAACTTTTGAAACTTGTGATCTTAAACTTACTATA
ACATTTGTGTAACTATAAATGCTTCTAATAAAAAAAATATTAAAATTTGTCAATTTTTTTGA
AACAGACCAATAAATAAATAGTGTCAATGCTTTTGAAACGGAGGTAGTACCTGGGATTC
AAGAGGATCAGTCTCTCTAGAGACAATACTTTGCAAACTAATAACAGAAGTAGAAGCAG
CCAAAATTGGATCAACAGAATCGTGTGGAACAGCAGCATGACCTCCTTTTCCTCTAATT
GTAGCTTTAAAGCTTCCACATCCAGCCAAGAATTCACCAGGCCTAGATGCAACTACTCC
ACTTTCATACTTATGAACTAAGTGCATTCCAAAAATGGCTTCCACATTTTCAAGAACTCC
TTCTTCTATCATATCTTTAGCCCCATGCCCTCGTTCTTCAGCTGGTTGAAAAATTAACAC
CACTGTTCCCTGCAATATTATTATACACGGTAATTAAATTCATTACTTCAACTAATCCATT
AGCTTAGAAGTATGTATTTAGAGCTTAATTAAGGGTTTTATTTAACCTGTAAATTGTGTCG
GAGTTGTTGTAATATCTTGGCAGCACCAAGAAGCATGGCAGTATGGGCATCATGAGCA
CAAGCATGCATTTTTCCATCAACTTTGCTCTTGTGCTCCCATTTCGCCAATTCCTACATT
AGAAGAATTCAACTTTGACTCACGACTCTTTTATTGATCAAATTATTCACTTTATAGATTT
TTGAAGAATTGATTAATCGAGAATAAATATAGAGTCCTACTGTAGAGGCATATTATATGA
TATTGACCTCTACAACTTATAAAACCCGACTTATGATCTTTATTTTCTTTTTCTGTTGTTTA
GATCACAATTGATATTTGATGTTCAAATTAAATGTTTTAGCGGTGTAATATTATTACTTAT
GGTACTTTCGGCCATCCTATCCAATTTTACTACTAGGAAAATAAAAAACGTGTTGACCCT
 
TTATTCCACAACATATGAAACTAAAAGTAAAAAGAGATGGTCACCATAGAAGAAAACTAG
CTAAAGTATATACCTACGAATTGAAGTGTTTTCTCTTTCCCAATGAAGTTCCAAAATTCAA
GAATCTCTTTGTTTTAGGTATAATTAAGCTGTTTCGAACTCTATACTTAATTCAATATTAA
GAGAGATCTGATTTATTACTTTCCTTTCATGGCTTAAATATTACCGCCGCCGCCGCCATT
TCTGACAAAAACGGAAAGTAAACTGCCGCAAGTAATTTCTTCTTCTGCCATAGTTAATTT
AGTCGCCCACAAAATTAATAAAATGACTCAAATTTACTGCCTACACCCTAGTTCCGACCG
AATACAACATATAAATGATCCCCGTGCTGTTGTCATCTCGAACATCCTTAATAACAATCT
CCAAAACCATTAATGAAATACAGACAAAGGTAAGAAGTAAATTTGAAGATATATAGTACT
ATATTAGGCCTATAGATCTACCTCCTAAACTCCACAAACTGTTTAAAGTGAATAAAACAT
TTAAAGAGTTCATATCAATTTTTTTGATATGAAGAGTTATCCGTGGTTATAAATGAACTA
AACGTGATACTAGTATAAATATTCTTACCGTTTTTTGTTTTGAATATAATTGCAGGGTTGA
GAAAATTTCCAAGCAGAGACTACTAACCTGAATAGGCAAAGCATCCATGTCTGCTCTGA
GAGCCACAAATGGCGGCTTACCGGAGCCGATGGTGGCAACAACTCCGGTCTTAGCCA
CCGGCCACCGGTACTTTACTCCCATCCGATCAAGCTCCTCTCTGATCAAACCACTCGTC
TTAAATTCTTCATAAGCAAGTTCTGGGTTCTCGTGAATTTGTCTCCTTATTTTCATCATCC
ACTTCACTGTCTCCGTAGCATTTGCTAATTTTGTAATATAATCTTTCACGTAACAGTTTTG
ATCTACCAAAAACGGATTCAAGCACTCATCATCGCCGTGACACGAAGGAAAAACAATGA
ACATACATACAAGCACCAAAATTAGAACTTCCTTAGCACCCAT
SEQ 26 
ATGAAACTGAATCCTTACTCATGGACAAAGGTAAGTACTTGATTGTGAATTATAACTGTA
TTATGTACATAAGGTCGCTGCACAACACAAAATGTTGAAAATAAGATGGAATTATTAGGT
GGCAAGCATTATTTCTTAGACTTACCAGTAGGCACTGGATTTTCCTATGCAAGAACTCC
AACAGCTTTACAGTCATCTGATTTACAAGCAAGTGATCAAGCATATGAGTTCCTTTACAA
GGTAATTAGATTCTTCACGAAATTATTAGTTAAATGTATTTTCTCCTTTGCCCCTCAATGT
TGTTCAATATGTAGTAGAACAGTCAATAATTTTATGTTGTTTGCAGTGGTTCCTTGATCA
CCCAGAATTCTTAAAGAATCCATTGTATGTTGGCGGCGACTCATATTCAGGGATGGTTG
TTCCCATCATTACTCAAATTATAGCAACTAGTAAGACTATATTTTCCCTCAAATAGTTGTG
AAACAAGTAATGGCAGCCTAAGGTAGTAAGGTGTTCTGTTCTTGTACTATAACATTTTGT
GGCCTTGTGATAATGCAGAAAATGAGATGGGAATAAAACCTTTTGTGGATCTTCAGGTT
TGTCATTTTTCTTGTATATATTCTCTTTTCCCTACGGATAAGCAGACGGATTACATACCAA
CTCAGAATTTGTAACGAAATTGTTATGAGAATGTCACGACCCAAGCCCATAGCATGTATT
GTCTGCTTTGGGCCTAGGCTCGCACGGATTTGTCTTTCGGGCTACGCCACCTCGAGCC
CCAAAAGCGCGTGCACCATGTGAACTTGTGTCATACCTTATAAAGTTCATCACTTTCCTC
TATTATTCCGATATGGGGATTCGTCTAAGGTGACATGTGCACCGCTTATTCAGAAGTTT
GGCAGCCTAGAAGCTAGTCAGTCCTACTTAACTTGCCCTCATCAGCCCCCTCCTTCATG
GGCATCACACAGAATCAAAAGTCACTGTAGAATGTGAGTTGATTTGCAAAATGTATGAC
CTGATATCTCTCGTCAAGTGGTTTCAGGGATATTTACTCGGAAATCCATCGACTTTTAAA
GGTGAAAAGAATTATGAGATTCCATTTGCTTATGGAATGGGACTTATTTCTGATGAACTC
TATGAGGTTGGTTTTCCTTTGGTGTTATATAGTACAGTCAAACCTTTCTATAATAGCTACA
TTTGTTCCGATATTTTTTGGATGCTATAATGAAGTGTTGTTATAGAGGATATATATTAGTA
TAACATAACATACAAAATCGGCTCCGAGAAAAACTTGGCTTTATAGTAAATGACTATTAT
ATATGGATGCTGTTATACAGAGGTTTGACCGTAAGATCTTAAATATCCTCCAGTTATGCG
CTTTAATTTAGTTTGCTTACATTGTCCTTAGAACTAATTGATTTCCCTTTCTCAAATAGTC
CTTGACGAGAAATTGTAAAGGAGAGTATCAAAACACTGATCCAAGCAATACACAATGTTT
GCAAGATGTTCATACTTTTCAAGAGGTTGGATCCTATTTTGAGGAAAATCAAATATCATC
TGTTTGTTTTATGATAGGTTCATTAACATACTGACCTTATGCAGCTTCTGAAAAGAATTAA
TAATCCCCATATTCTGGAGCCCAAATGTCAGTTTGCTTCACCAAAGCCACACCTATTGTT
TGGCCAAAGAAGATCTCTTAATGTGAAGTTTCATCAACTTAACAATCCTCAACAACTCCC
 
TGCGCTAAAGTGTCGCGTGGGTACTCATCAACAAACTCTAGCATTCTTTATGCTATTGAT
TTTTTGTTTCACTGAGATACTTACGAGAATTTACAACTTGCAATTGATTTAGAATGATTGG
TACAAACTTTCTTCTCATTGGGCTGATGATGGCCAAGTTAGAGAGGCCCTCCATATCCG
AAAGGTACGTTAGTTCTTGTTGGAAGGGGAACCTTGGAGCAACGGTAAAAATATCTCTG
TGTGATCTATAGAGCACGGATTTGAGCCATGAAAGCAGTAATGCTTGCATTATGATAGG
CTGTCTATATCACACCCTTGAGATGCGGCCACCTTGCATGAATGCGTGATACTTTGTGC
ATCATGCTGCCTTTTTTTTTGAAGAACAACAAAATTTAACAAAGTGTGCTACACAAAACTA
AAAATATGATCAATTTGATTACAGGGAACTATTGGAAAATGGGTGAGATGTGCAAGTTTG
CAATACCAAAAGACAATCATGAGTAGCATACCATATCATGCAAACCTCAGTGCTAAAGG
TTACAGATCTCTTATATACAGGTTGAGTAAGATTGTTGTGTTTGCAAGATTGGAATAACT
ACATAAATAGTTGAAGATTATTATCTCTGTGAAACTATTTACTTAGTTTTCTATGTTTTTTG
AATTAAGCAGTGGAGATCATGACAAGGTTGTTACCTTCCTATCAACTCAAGCATGGATA
AAATCTCTTAACTACTCCATTGTTGATGATTGGCGACCGTGGATCGTTGACAATCAAGTT
GCCGGGTTAGTTTATGATGAAAACATTGTACGCTAGTCATAAGCTCTGTCAAGGTATAG
AAGTTAAACTCATTTTTTGTCTTTTGCATGATTGTAGTTACACGAGAAGTTACTCAAATCG
GATGACATTTGCCACAGTAAAGGCAAGATATCTCTTTCACTTGCTTTTCTCAGTTAAGTT
TGAAGATAAAAAATTTTGTTAAATAGTTGGTGTTTAAATTGCACTATTTTGTTACAGGGAG
CAGGGCATACTGCACCAGAGTATAAGCCTCGTGAATGTCTGGCCATGCTCAAAAGGTT
GATGTCTTACAAGCCTTTG
SEQ 27 
ATGTGTGAACCGGAGTCTGAAGCAACTCGTGGGGTTCTTAGTTTTCTCGATGTGGACCA
ACTTTTCAGTTCCAACTATTACGGCGATGGTAGAAAGCATGACGTTGAGATATGTCATG
AACAATATGCCAGAGAAAACCAGTATCACACATCATATTGCAATGTTGACAGTGATGAG
GCTATTGCTCATCTTTTACAAGAAGAATTGTCAGAGTTGTCCATCGCAGAAGATGCTGA
ATCTTCACATGCAGATGAGCAGTATTTTCAAGCCTCCACTGGTGTACAACATTGGCATA
CTCCTCCAAGGGAGTACTATGCCGGTACATTTCTTGTTTTCAGTTTTGTGATTTTTCCTC
GAGTTTAAACATTTGCTAATTTATATATGATTCACAGGGCATGACACTGGTCTAGAAGCT
GATGATGTGGGGCCTTCAAGTTCTTGTTCTAGTCCTGGCGACAGATCATACGATGGAGA
AGAGTATACCTACACATTGGAAATACAAGATGAATTTGAGCTTGATGGAGAAGTAGGGA
AGAGAATAAACCAGCTGAGTGCTGTTCCTGTAAGTGTAGTGTCTGTTAGTATATCAAGA
ACACTAATTCACTTTGTGTACGAGGACATGTGCGGCGCTCTGCAACTTTGGCCATTTTC
TTGTCACTGCTTTAGTTTCTTATAACAATTTGTGTTATTAACCGTGCAGCATGTTCCTAGA
ATAAATGGAGACATACCTTCAGTCGATGAAGCAACTTCTGATCATCAAAGGCTGCTAGA
TAGGTATCTCCTGATCCCTCCGACTTAGGTTTTCAAGTTGACAGAAATTTTGTGTATGAA
CAAGTTAATGACATATGATCTTATGGTAGTGCTTTGCTTAATTCTCTCTCAGATTAGCAC
TTTCCAAGGTAATGAAATATAAGTTCACTGCGAGAAATGCATACCTCTATTGTGATTAGG
TGTCCTGTGTCTCCAGAATCATTTCTGTATTTTTTTAGGTTTTCTCTTTCTTTATTAATTC
ATTCTTTTCCCGGTGGTTCTTACTGCCTGCTATTTGAAAAGTAACATCAAACCCCTCATG
CATTTTGAGAAAGAGAATCAAATGGGAGAGATGGGACCCGGACAAGCACAGAAAAT
AATTGTTGTGGAATGAGCCAACTAGTATGTTGTGCTTTCTAAATAAACACTTACGAAGCA
CATTCCAGTAGATACTGTTAACCCTTTGTTTGCTTAAAGTCACAGGACAATGCATGCGGT
TTTTCATTTTGTTCTGTTTTTTTATTAGGTTTTGTGCCACCTAAGTTTCCTAGGAAGTTGA
TATTGTTGTGTTTTTTCATTAGGTTTTGTGCCACCTAGTTTCCTATGAAGTTGATATTTTT
GCTAATTCATTTGAATGGTAATACTATTGCTATAATAACTTATTTTCTGCTAAGCATCCCA
TGCTGTGATTTTTCTTGCTTCAGATTGCAATTATTTGACTTGGTGGAGCACAAAGTGCAA
GGAGATGGCAACTGTCAGGTTATCATATTACCTGGTTGATTTATCTTGCAGTAATGATAT
CAAACTGTCTAGATGCGCTCATGATTTGATTTCTAGTGGAAGAAAAAAACTGTATTCCCT
TTCCTTAAACTACTTACAGCATAAGTATTAATCTTAAACATAATGTTTATCAGTATTCCTT
 
CCTTTTGGAATTGTTCTGGTAGAAACTTGTTCTTGGTGTTTGTGACAATGTCTTAGCTTT
CTTTATTACTTTTTAGTTATGCTTGAAAACAGTGGAAACAGTAAAGTTATCTCCATATAAA
GTTGTCTCTGTGTGACATATAGGTCATGAGTTTGAGCCGTGGAAGCAGCCATTAATGCT
TGCATTAGGTTAGGCTATCTATATCACACCCCTTGGGTGAGGCTCTTCTCGGACCCTG
CGTGAATGTGGTCGGGACCCTGCGTGAATGTGGGATGCTTTGTGCACTGGGCTGCCAT
TTTAGTTATGCTTGAAATTCTCAACTTTTTAATTTTCATATTTGGTTTTTACTTGTCTATTC
TTTCCATTAGCCTTAAGCAGTTGCTCACTGTTCATCATATTTCATTTAAGTTTGTGAAGTG
TGTGAGACCATATACAATATTGCTGAATTATGATATACATTGGGGATTGGCAATTTCATT
TAAATTGAATTCTTTAGTGATTAGTTCAATAAAGTCACAAAAAGAAAATCGGACTTGAATT
ATTGATTTGGGAGTTATTTAATTATGAAATGAATACTAGTAAGAAGCGAGTCAAGAAATT
TGAGACTGAATGTGAAAATTGGATGGAAGATGTTCACGGAGAAAAGCTGATTAATAGTA
ATGTTGGTAAAATAGGAAGGGATTAGAACTCGGATAATGAATGTAGAGCGAACTACAAA
ATATAAGAAGTTGAGAGTTCGGATGGAGTTGGGGGATGGGTGGTGAATGGAAGTGGT
TCATAATAGGATTTTGGAGAAAAACTGGAATATGAAAACTCAAGTGATATATCGGCAGGT
TGCTTGCCATGCCAAGTGCCAACTTTATGAAAATTATTTGTGGATTTTCAGTTAGTTTGA
CAAAAGGACAATTCAAATTTTTCTTAACTCTTTAGTATTATGGGGGTATGGGAACTTAG
CCCGTTTTTCCTTTCTGTGAATTAGGTTTCACCTCCGATTCTAAGTTTGTTGAGATAAGG
TTCCATCCTATCTCTTTCTAATGCACATCTATTCACCTGTCTTCTTTTGTTCCAGTTCCGT
GCTTTATCAGATCAATTCTATCGTACACCGGAGCACCACAAATTTGTCAGACAGCAAGT
AGTCAGTCAGGTGCTTGTCTAAGTGAATAAGATTATCTTCTCTATGATATTTCGGTTTTC
ATTTTTGAATTATTTCACATACATTTTAGCTGAGTAGGTTTTTAAAATCATTTTGTTTTGT
CAGCTTAAACATCATCCAGAGATGTATGAGGGATATGTCCCAATGGAATATGGAGAGTA
CTTGAAGAGGATGTCCAAGTAATTCTCAGACCTTTGTTTTTCCATTTTGAAATCGTGCAT
TACCTTCTCTCCTTTAATTTACATCTGACTTTTATATTGGTTGAACCAGGAGTGGGGAAT
GGGGCGATCATGTTACGTTGCAGGCTGCTGCTGACTCGGTACTGATTATTGCCCTTACT
TTGGTTCCTTGGTTTTTCTCTCCATTTGATTACTGCCTTTTGGTTTGTTTCTTAACCTTGG
AAATAAGAACCGTAAGCTATTGTTGGCCAAACCATAACTTGGATGTCCTCATATGGTGC
CTTCTTTGGATATTGTAGTAGCTTGTTAATTGCAAGTTGATGGTATGTAGGAAGTAACAT
GCTTCTATAGAATTTGTGATCCTGTAGTTTTTCATGAGTATGTGTTAATCCTTTATTTTGT
AGTGTGGAAGAAAATGTGTGTTTATGTGCCTCTCTTGCTATTTTTTTCTTTCAGTCTCCAT
TGTGTGGTCGTGTTGCATTTTTTATTGTATATGCTTAGCTCCTTTACGAAGATTTTGCTTG
TGATAATATTAGATGAGGATGGATTGGTTTGTGAATGACCTATTTGTGCTGCACATTGAA
TTGTCTTTTTGTCTTGTGAGCAGTATGGTGTGAAAATTCTCGTTATAACGTCATTCAAGG
ATACATGTTACATCGAGATTCTTCCGAAGAATCAAAAGTCAAACAGAGGTAACTAGTTCT
CAAATTTTGAATTGCTTTTACCTTTCTGTAATATCCTCTACTTCATAGAATGTGATTGAAC
AAGTTTTCATTAACAAAATATTTGGAAGTGGTAGTGGCATGAATTCTCAATTTCCATGTAT
CTCATATACAAATACATGTGTCACCGAATTGTGTACGTACTTCCAGTTCGCAGTTAAGGA
TTTTCCCCAACTTTAATTCCACTATGTGAGCAACCCTTACAAAGTTCTTCAAACATTCTTT
ATTGGTTATTTCTGAAATGTGGTTTTAGACTTATAGATAATACCAGAATATTATCCAGGG
CCAAATTTCAACAATTCAAACAAGGAAAGATAGTATGATCATTCTTAACACCACTGTAGT
TACCCCCCATTGAATTTTCCGATAACGAGGGCTACACATGGAGATGAAGGAGATGATCT
GATTATGCATATTGTAGTAATAGTAGTAGATAAATTTATAAAAGTAGCTAATACTAGATAA
CCCGATTTATTATCTGAGTTTGATTCTTGAAACTTGTTCGAGTTATGGAGAAACTAGTAT
GGCCTTTGTATAGGACCTTTCCGAAAGTCCCAGATCCGATAACGATCTGATAAAGTAGG
ACCACTGGAGTTATGAGACAACATTAGTCTATAATATAAATGATCAGAATATTGCAACAG
AAAACATCAGTTGTCTCTTTCCTCTCTTCGATAGAGGCAAAGGAGATTGAATCTAATTGA
TTCCGAAATGCTTCATTGGATATTCAATAGTTAAATCGAACTATTCATCTTGCAACTCTGA
AAACAGACGTGCTATACATGCAGTATAAGAGCAACATAATTAACATACACTAGGTTGGA
GGTTTACTTATCTATGTTTAGGTGGTCGGTTTATGTGCAAGTTTTCCATTTTTCAACAATT
TAGAGTTATCAGAGCTACTTATAAGACATGATACTTTTGCTGTATTTAACTTTTTTTGTAA
AGTTCAGCAAGAGTTTTTGCTAGTCCGAGTGGAAATAATTATTTGTTGACTACCCATTTT
 
CCCTTTTTACTTGAGAAAAAGATTGAGAGGGGGGAGGCAGAGCAGCATCATGAGTCAT
CTGGAGAATGCAAAGAGTACTTTTGATCTTTGGACTGAAGAGTTAGAACGTTGGCCTTC
CTTGATAACAAAATTTTCAAGGGGAGGGGGGATTAATGGATGACTATGAACCGTTATGG
ACAATCTTAAGCAAATCCGATCTTGGGTTTCATGAAAAGGAGATTCCCAAGGGTTGACC
AGTTTTGGTTGTTTTGAAATCTAAAAGATGGACTGATGAGCATCCTTATTGCCTTTTTAG
AGACCTAAAGTTGTCTACTTTAACATATTGAATGATTATGAGACAGTTCTAAGTTTAATGA
ATGGGGACAGCTTTATATCGGTCATGTATGCATGACATTTATTCCATGCCATTATATGAT
ACCTCTACTGTGAAGCCAAGGGTGGGCACAACTTAGCAATGGATACTTAAATCTGGAGA
TTGCTTGGAACATCTTTGTGCATATGCACTTTGTAAGAGCGATGTTTGAAGTCTTGATAT
TGATTCGCTTACACCAATTTAATTGTTTTGTTTCTCTCGTTTTTCTCTTGGTGCAGTCATA
TACTTAAGTTTCTGGGCAGAGGTGCACTACAACTCAATCTATCCTCAAGGAGGTAGTTG
CTTGTACATTTATCTCCTGTTCCTGTTTATTTTGTCTTAGCATTGAGAGTTGAGTGGGAG
TTTGCTGATCTTGCTTAGTTTAGAATTCCATTCTATTATCATATTATTCTAATACTGTAGTT
TGATGCTCCATTCTAGACTTCCTGCCATTTGATCTTAAGAAGAAGAAGAAGTGGAG
TTTCTGGAACAAGCAT
SEQ 28 
TCAAGAATCAAAAGCTGAATCTTTCGATACAGAATGGATCATGAAGTCAGAACAGCTTG
TTTCTGCTGTAGACAGTGAAGAAGTAAATCCAAGCATAGAAGTTCCCAAGTTAAACTCC
AAAAGATTATTCTCCAACTGGTAACCCCCTATAACAATTGAAGCCTTTTGATTCACCCCT
CCATCCAAAAATCCCCAACACATCACTTCATCACTTACTTTCACCATTGAATTTCTCCCA
TAAATCCTCCACTTAACCATTTCACTTTGCAACACAAGATCCATAGTTGGAACATTTGGC
CCCACTTTTGTGACATCTATCCCCTCTGTGCTAAAGCAAAGCTCAAATGGTGCTATGGA
TTCCACTTTAGTCAAATTCACGGAAATAGCAATTTTTTCATAAGCTTCCATAAATGTCCTA
TAAATTGAGCTTTTCATGCTAGTAAAAGGGGAAATTGTACTAATCTTTGTCCCGCCAACA
CCTTCTTCATCCATTGTAAACAAAGATATGTTTAAAGACAGTTTATTGCCATTAATTTTTA
TGGATTTGACATTGATGTAATACTCTTCTGAAACACCATTTTTAGATGAAATCAAAGGTG
TGTAAAGCATAGATCTTGAAACATCATGACCCAAAAGTGACAAGTAAGGACTTTCACCA
GATATTATAGCACCATTTGAAGAAGACAAACAGAGAGCAAATTTCCTCTGGAAACCAAAT
GTATCAGACAACTGAGATGGCAATGCAATTCTTGAATTTCCAAGTCCTAACATTCCTTTG
GCACCTCTAGCAAGACCTTGTAACAAAGTAGTTGATGCACAAGAAAACAAGAATTCATG
TATTGAAGCAATTGAACCTGTTTTTAACTCATCTATGAATTCCACAGCAAACATGTCCTC
TGCTAATTCCCCTGTTGCAGCCACTTTTGTGAAAGGATTTTCTGATTGTACACCACAAAT
CTTGTTAGTGCAACCATTAGGTTTAGCCATTGAGCACTTGAGTGAATTACAGGGGACAA
GTTTCTGACTTGAAGATACAGAAGTTAAGCCACAATTAGTCCAGAGAAACGAACCTCCA
AGATCTACAACTAAATTGGTAGGAACAAGATGAGCACCCATGTAAACTTGAGTAACATA
CTGAAAAGTGGACAGATCTTTGTTTACAGGAAGAATAATGGCCTTAGGCAAAAAGGGTC
CATGAGAAACTGAGAAAGAAACAAAGAAAAAGAACGGAAACAAAGGAAGGAATATTTGA
AGAAGTGAAGGCAT
SEQ 29 
TTAGGCCTCAATCAGTTCTCTAATTGGTTTGCTGTTTATGTTAGCTGATGGGATATTAGT
GAATTCAGAGAGTATAGCTCTGAATTCATCTCCTGTAAGAGTCTCCTTTTCTAGCAACAC
ATCCACTAATTTGTCGATTGCCTCCCTGTTGTTCCTTATGTGGTTCTTTGCAATTTCATAT
GCTCTCTCAATTATGTGCCTTACCGATGCATCAATGTCTTCTGCTAGTTTCTCTGACATT
TGATTCCTCGCCAGCATTCTCAGCACCACATCACCACTCTGTGTTGCTGGATCTGTTAA
CGCCCATGGTCCTATCTCAGACATCCCGAACATTGTCACCATCTGCTCATGATATAAAC
 
ATTGGCAAGTTAATACTTGTGTGTATTCGAATATGTTGTTCTCTTTTAATGTGGTGCAAC
AAGATGATGTGTTAAGTAAATACCTGTCTTGCTATTTGAGTTATTTGTTGCAAGTCTCCG
GCTGCACCAGTAGTGATTTCTGCTTCACCAAAAATTATTTCCTCTGCTGCTCTACCTCCT
AAGCTTCCAACTATTCTAGCAAAAAGTTGCTGCTTAGATATCAAGGTTGGATCTTCACCA
GGAATAAACCATGTAAGACCGCGAGCTTGCCCTCTTGGGATCAATGTAACTTTCTGTAC
TGCATCATGGCCAGGGGTCAATGTCCTATAAGCACAAGGACACATTCTTTAGTACTGTG
TCTTTTGATTACAAATAACAAACTGAAAAGATTGAATACTTAGACAGCTTCTTAAATTTGT
CCGGTTTTTTCATCTAAACACCTTGTCTAAGGGCCTGATATATTGAACACTTGATGTTAG
TTGAAAATTCAATAAGGAGCAAATTACTCCCTTTTTTTGCTTCATGTATAATCTAGTATAA
ATGAAAATAATGAGAGGAAAGAAATGATTGTTAACTTACGCGCAGACACCATGTCCAAC
TTCATGATATGCTACCAAAATCTTGTTTTTGCCATCTGTCATCTTGGTTCCTTCCATTCCA
GCAACAATTCTATCGATGGAATCATCAATCTCTTTCGAGGTAATCTTATCTTTTCCTCTTC
TTCCAGCTAGAATAGCAGCTTCATTCATGAGGTTTGCAAGATCTGCACCACTGAATCCT
GGAGTTCTCATTGCAATAACACTTAGAGACACATCTTTATCAAGCTTCTTGTTGTTACTA
TGAACCTTCAATATTTCTTCCCTTCCTCTTATATCAGGCAGTCCAACACTTACCTAATAAA
ATGAAATATCAATATAAGTGAAGTGTATTCTGGAAACTGTATAATACACCTCATTTTATTG
GAATTTTACAATCAAAATCTCATTTTATACCTGTCTATCAAATCTTCCAGGTCGAAGCAAA
GCTTGATCAAGAATTTCAGGCCTATTAGTGGCAGCAATGACAATGACTCCAGTGTTTCC
AGTGAAACCATCCATTTCAGTGAGAAGTTGGTTAAGTGTCTGCTCTCTTTCATCATTTCC
ACCGCCAATACCAGTTCCTCTTTGCCTCCCAACAGCATCAATCTCATCAATAAAGACTAA
ACAAGGTGAATTTTCCTTTGCCTTGTTGAATAAGTCCCTAACTCTAGAAGCTCCCACACC
AACAAACATCTCAACAAACTCTGAACCAGAGAGAGATAAGAATGGAACCTCTGCTTCTC
CGGCAATCGCCTTAGCTAGCAATGTCTTCCCTGTCCCTGGTGGCCCTACTAAGAGAACT
CCCTTTGGTATCTTTGCCCCAACTGCTGCAAACTTTTCTGGGGTTTTCAAGAACTCAACA
ATCTCTTGAAAATCTTGCTTTGCATCATCTACCCCAGCCACATCATCAAATGTTACTCCT
GTATTTGGTTCCATCTGGAATTTTGCTTTGCTCCTGCATTATTCACAAACAAATACTAGTT
ATTAGTAGTTGTTGAAGATTACATCACTAGACATAATGTTCAATCTTGATCATGTTTATGG
AATTTCTATTATAGCATACTGTTGGGTTTCTTAAAGAGATGGAAATGATTGAAATTGTCTC
TCCTAAGTTTTATTAACTATAGAGCGATTTAAATAGCCAACTTGAAAATAAAATACACAAA
TTTATAAAATATTGAAAAACCTAAAATATCTCAACAACCTAAAATATCTAACCGAAATTTA
AATTCAAACAAAGTAGACTACTTTTACCACTAAAAATTACTCCTTCTATTTCAATTTAGAT
GATACAATTTCCTATTAGTACGTTCCAAAAAGAATTATACATTTCTATAATTGAAAATAAT
TCAACTTTAAACTCTTTATTTTATCTATTTTAACCTTAATAAAAAACTTTTATAACTACACA
AATATCATGCCCCCCACAAAGCTTTTACCTCTTAAACTTTTTCAAAAGTCTTCTGTTTTTT
TTTTTTAAACTACGTGCCGAGTCAAACTAACTAATTTAAATTTAAACCGAGGAAGTATTAT
TCTAGTAAATTAACAGTAACAGAAGCTATATACAAGACATACCTTCCTAATCCAAAAGGC
AGGTTTGGCCCTCCAGGAGTATTTGAAGAAGAGGTTCTCAACAGCAAAGAGCCAAGCA
ATATCAATGGAAAAGCTAAATTCCCAAGTAAATCAAGAAGTGGCCCTATGACATTCATTT
CAGGGAGATGAGCAGCAAAATCTACATCCTTCTCTCTAAGTTTTCTCACCAATTCTGGT
GGCAATCCTGGCAACTGAACTTTAACTCTCTGGACTTTGTTAAGAGCAGGATTGAATAT
CTCAGCAACAGCACTACTCTCAAAAAAATCAACTTTTTTCACAGCACCTTCATTCAAGTA
TTCCAAGAATCTTGAATATGACATTCTACTTGAAGTTGCTTCAATTGGTGCTTCAGTTTCT
GCTCTTGCTGGTTTAGCCAAAGTCCCTGCTACAAGGCTCAAACCACTACCACTCAACAG
CTTCCTCCTATTTATTCTGGTGTCTGAATATGATTTTTGACATGGGGTTTCTTTACTAAAG
ATTTTAGGATTGTTAGTATCCTTAGAAAGATCTTGGGATTTGCATAGGGGAAATTGAATG
ACAGACAAAGAAAGGGCAGGGGACATTTTCAT
SEQ 30 
TTAGGCAGTGGGATAAGAAGCGTCCATAGCAAGTCCACAAAGGCCTTCTTTCTCATGAA
CATCCCTTTTGATGCGCATATATCCACTGTCACCCCATTTACTGCCCCATGAATTCTTTA
TAATCCAATATTTTGTACCGTCAGTTGTTGCACCATATCCCACTGCTGTAACAGCGTGGT
TAAGCCAAGTGCTGCATGATCCACTGAATACACCACTTGAATAGAACTGGAAATCGAAG
CTACTCCCGTCTATTGCCACCGAAACAGGTTGATTAGCCACTGCCTGCAATAGAGCCTT
CTCACTGTTCGCTGGCACATCTTCATATCCTGTAATAAGAGGCGTAAGTCATAATTTCAA
GCTTATGGATTCGGAATATTTATCGTTTGAAGTTGCTGGGCTAGATCATAATTAAACCAA
CTCACCCAATTGAAGATTTGTTCTACCCCTTATATTTTTATGGGCTTACCTGTAATTTTGG
CTGCTGAAAGAGCTGACTTTTTCTTGTTGCAGACACCATCTTCTCCTTTGTATGGATAGT
TTACTTCTGTTGTGAGGCCCTTGTTTTTCAGGATGAAATCAAAGGCAGTGTCCAAGAGT
CCACCGCTGCAACCTTCGTCCTCGCCTTCGACATCACAGTCTACAAGCTCTTGCTCTGA
TAAAGGGATCAACTCTCCTGTTTTCAGTTGGTGTAGCCCTTCCAT
SEQ 31 
ATGGGATGCCGCATGAAATTCTTGAATGTGGTTTTGGTGGTGGCGGCGGTGATGGCTG
CTGCCGCCGCCGTGGCCTTCGGAGCTGAGAAATTGCCGGCGGAGTGCTTAGTTTGG
AAAGGATTTTTCCTTTGAATGGGAAGATGGAGCTGGAGGAGGTTAGAGCAAGGACAG
AGCTAGGCATGCTCGAATGTTGCAGAGTTTTGCTGGTGGTATTGTTAATTTTCCTGTTGT
CGGTTCATCTGACCCTTATCTTGTCGGGTAATTACTTTGTTACGACCAATTTGATAAGAT
TATATTTGTGATGTTTTTAGTGTTTTCTTCCTTTTTCTAATGTGGAGTTATATTGCTATATT
TGCTATATTTTATTTGGTATGATGACGATGATATGGCTTGAGCTTAAATGGAGAAGTGAT
GATTGGTATAGCGGACTCCAACTTGTTTGGGACCGAGGCGTTGTTGTTGTTGAGTTTTG
TTTGGTTAATTTAGTCATTTTTTGGAAAGTTTGATTCTTTATGATGTTAAAACTTGGAACT
TTTGGTGAATGTATGGAAGCTATGGACTATTTGATGTGTTATTAACGTCTTATGAATTTG
ATCTCATGAATTCTGATGTAAATTTTGTTTTAGTTTGAGGGTAATTGATTTTAAGTGTATT
AAAGTACTTGTAACACAATGAATTTTGGTGTGCTGTTTTTCTTTTCTAGGTGCTTTCTTGT
TAATTATCGGTTGGATGGTGTTGTTGATGGTAGTGATAGTTCTGTTTGCTTTATCTTGTA
TCGTTTCTTGTTCAGGTGCAAAATATTTAGGTACAATTGAATGATGAGTTTCGTGTTGCT
CTGAATATGAACATTAGCTATATCAGTTTGGCATTTGCTTTCTGTTATTTGTGGATGAGG
GATATTCTTATTGATTTGACTATCAATTTTGTTGACCATCGTCTCTTTCTCTCTCTTCTAT
GCTTTTGGTGTATTTGTAGCCTTTATTTTACAAAAGTAAGACTGGGAACTCCACCAAGAG
AATACAATGTGCAGATCGACACTGGCAGTGATATCCTATGGGTCACATGTAGTTCCTGC
GATGATTGTCCTCGGACAAGTGGACTTGGGGTAACTCATCTTCCCTTCATCTTGTTATTA
CTTTTTTAGTTTCTTGTTTAAAGTGTGGTGAAGGAATAAACTGTTACGTGGGTGCAGGTT
GAGCTCAACTTCTATGATGCTACCATCTCGTCAACTGCTTCTCCCATTTCTTGTGCAGAC
CAAGTGTGCGCCTCTATAGTTCAAACTGCCTCCGCTGAGTGCTCTACGGAAACCAATCA
GTGTGGTTACTCCTTTCAATATGGAGATGGGAGTGGCACAACTGGCCATTACGTAGCC
GATTTACTATATTTTGACACAGTCCTGGGAACTTCTTTGATTGCCAACTCTTCAGCACCG
ATTATTTTTGGGTGAGTTCTTATTTTTTAAATACCCCTATATCTATACTTAAAATTTCATTA
GAAATAGTTGTGGGTCATTTGAACCAGAAATATCTTTGGCCCAATTTACAAAAAAACCAT
GTTTGTTTACTCAAAGCTTATACTTGGATATGATTTAAAACAGGTGCAGCACCTCTCAGT
CTGGGGACTTGACCAAGACGGACAGAGCAATTGATGGGATATTTGGGTTTGGTCAACA
GGGTCTTTCAGTAATATCTCAACTGTCTTCTCATCGGATTACTCCTAAAGTATTTTCACAT
TGCTTGAAAGGAGAGGGAAATGGTGGAGGTATACTAGTCCTTGGTGAGATTTTGGATC
CGAGAATCGTATATAGTCCCCTTGTTCCGTCACAGTACGTATTGTTACAGTACAATGAA
GTTTCTTTTCTTGCTTATGACGAATATAGAGATTTAATTGTTTTCATCTTTAGTGTGCCTT
GTGCTACATGATATAAAACAGTTGTGTTCTTTATAGTTTGTGATCCAGCTTGAGCATGTG
AAATATACCTCTCATGCGCTACATCCTGATTTTATTGAAATTTCGTCACTATATTATTGGT
TTTGCATCTACAGATATATAGTAGTTGGGTCTTGGGAAGATGACATCAATGAAACTTTAC
 
TTTGTACATATAAAAAGGGCAGCCCGGTGCACAATTTTGAGTGTTATATATATATATAT
ATATNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNTGTGTGTGTGTGTGTGTGTGTGTGTGTGTGTGTG
TGAACATGTCGATATATGTGTTTATTTTTTATGTTTTCTACATTATTTGTTTTCTAACTAT
AAGCACGGGGATTGGCTGGGCACTAGGAAAGAAAATGGTTTGTAGCAAGCTTGATTGT
ATCCGCTTTCCACTTTTGCAGGGCGCATTACAATGTATATCTGCAGAGCATTGCTGTTAA
TGGACAGTTGGTGCCTGTTGATCCATCAGTGTTTGCGACATCTGGCAATCGAGGAACTA
TTGTGGATTCTGGTACAACTTTGGCTTATATTGCCACAGAAGCTTATGATCCCTTTGTCA
ATGCTGTAAGTTCCTACATTTTGCCAATTTATTTACTCCCTCCGTCTCAACTTGAATGTC
CAATTGCTCTCTTTGTCTATACCAAAATTATATCCACATCTCCTAAATATAAAAACTGACC
ACATAACAACAAGAACAACTACGCCTGTTGGGGTTGGCGAAAAAGGGCAGATAACTGTT
GATCAAAACCCCTGAAAAATGTTTTATCATTCATCTGCAGATAACTGCTGCTGTTTCACC
ATCAGTTAGGCCAATCATCTCACGAGGAAAACCGTGCTTTCTAGTGTCCTCGAGGTTCA
TCCTTGTATAATTCAATAGATATTTACTTTGAGCTTTTAATGACAAAAATGTCTTTACCCA
ACTGCTTTGGTGACTCGAATATGACACCCTGTTTTGTTCTGAAAACAGCATAGCAGAGA
TATTTCCCCCAGTTTCTCTAAACTTTGATGGTGGTGCATCGATGGCTTTAAGACCATCAG
ACTACCTTGTGCATATGGGCTTTGTCGTGAGTACCAGAATCTGTATTGTGTTTGAATGTC
TTCTTGAAGTCTCATCTGAGACTATATCAACAATGCTATATGCAGCACTATTGTCTTTTTG
ATGAACTACAAAGCTAAGTGACTCAATTGAATGTTTCTAACAGGAAGGTGCTGCTATGT
GGTGCATCGGCTTTGAAAAACAGGATCAAGGTGTAACAATTTTAGGAGGTTGGTTCCTT
GTTTACTACAATATTTATGCCTCAACCCCAAGTGGGTTCGCTGGTGTATTTATCAACATA
TCTTCTAGGACTATGGTTACATCTAATATCTGCTGCATCATTACGTGTGCTAGCGTCCTT
AATGGGTGCTTAAGCACTCCATCACTCAAACCATCTAAAAAGGACCTAACTATACCTTGT
CCTGTATTAATTTAAAAAAAAATATAACTTGGCGAGTGTCTGCCTAGATGGAATGTACCT
TAGTGATTGCATGCTTAGATAAAGTCATCATTCCTGCAGATATCGATGTTGATGAAACAC
GGCCATCCATTTTCTATTAGAATATTAGGTCATTGATTTGCAATTTGAAAATTGTAGCTTG
AAGATTGCAGTCCCTTTGTCTTTTCTAATTTTGCATCCGTTCTTTTATTGTGATTGACCAG
AGGATAGGTATACTTGTAGTACCAGTAATTCGGTTAGATATGTTCCGTAGCGTGCCAAG
GAATTTATATTTCCTGTTTTGCCTAGTTTTTCGAAGTTTATGTCAAAGTTACTTGCTTTGG
ATTCCGTGCGTGGGACAACTATATATCTTGTAGAGGCAGTGATTGTTACTTTGATGTTGA
AAGACAGCAGAGGGTGGAATTCATAGAAAGTTAATACGTGGAAAGGGGTGTTAGACTG
GATACATGACTGCAGTCCTAAATCAAGGTTGGAGTTGCATTTAATATGGCTATTCCTAAA
GGCTAAAAGACACAGTCTCATCAAGATTGTATGTTGAGAACAAGTCTACGAGAGTGCGC
TCTGACTGTTCTCAGAGATAGGTGCCTCAGACTCTAGGGATTAAGCTAACTACGTGTTT
TGAGTGGTGTTTTGTTCTTTCTTTTCCTTTTTCACTGCTGGCTAACACAACTTGAAGACTT
GAATTCCAAACTGCTTCAAGTTTTAGGTCTATTATTCATGCCTTCTAACTTCTTAGAGTGT
TGGTTCCCTAGTCCTCTTTTTTTTCCAACTCGTGCATGCACGCTCATGCCCGTATACGTA
CATGCAAGACATCTACAGTTGTAAACACAATTTATGACCAAATATTAACGGAAAGGTATT
AATCCCTTTCTATTTCTTGTTTGTGTGTGTATGTGTAAACCAAGCACAATGTTATGACCA
ACATTAAGTGAAGAGAATTAATCTTCTTTTCTTTTGGCCCATGTCCGTGTATTGTATGCG
CATTGAATGTGTGAGCCTTTCCTTGTTTATCCTGGTTATGAGTTAATCGGTAGCATACAT
GGTGAGGTTTCAGGATGCATGATAGTTGCAGATGATAGTGTGAATTAACAAAATTAGTC
AAAAGCAGCTCCATGGTGAAACACTTAGCAAGGTTTTGGAATAAGTGGAAACAAGATAC
TATCCAGGCAATGCAAGTTTACTCCGCCTAAAAAGTATTAAGGTGAAATGATATTAGATT
ATTATGGTTCCTAAATGCTGGCAGTCAAGTTATTTAAGCTCAAATCTTCCAGGAGAATAG
AGAAATGTCGTGGTAGATGAGGAAGGAACACATAGAACTAAAATATGCTGGTTGACATG
AAAGAGTGTTGCATGAGTGTTATTAGACAGAAAGATTCCTAGAAAATTGAAATATAAGTT
AGTATAACGGTTGTGAGATCAACAATAATATGTGAGAGTGAACATTACATGTCCGCTAAT
TGAATATCATAAAAAATGCGGATGTAAAGATGGTTGTGCAATCATATAAGATTGAACATGA
 
TTAGAAACAATCACTTTTGTTGAAGGGTGCAAGTAGGGCACATATAGAGCATGAAAAGG
ACGTCACATGAGATGGTTTGGCCATATCCTATTAGTCCGCCATATGAACCGGTTATTAA
GTGTGGCAATATTGTGTTTAAAGTGCTGAAAGGAACGAGGTAGACTGATGACTACATTC
AAAAATTTGTCTCAAATGACTTAGAATCTCATGGAGTCAATACGGCTTGAACTATAAACA
AAACCTTATGGAAGAAATGGATCCATATAGGCAATACTAACTAGTTGAAATAAGGCTTAC
TTGATTTTACTCTACTGGGTGCAGTATTTGTCAGGAGTCTTTATAATTTGGTTAGAGACA
TGTTTGTAAGTGTTGGTATAAGTTAGAGATTTAGAGAACCTCTAGATTTAAGAGAACCCC
TTGTCTTAAAAAGATTATGTACTATCGGAGTGAATTATTTCAAAAAAAGAAGATTATTTAC
TATCAGAGTGAATTGGGTTCAAATAGAGCAGAATGGCCCCAAATGATATTAGACCTCAA
CTAGTTTGGGACAGAAGTGTAGTTGATTTATTGATATGCATCTTATGTGCAAATTTTATTT
TAGTCATGCGTGTGCCCTTGAGCTCCTTCCTCTTCCCTCTTCCCTCCTAGTCATTCTACT
AAATTTGGCTATTCAATTAGTTCTGGCTATGCTTTGGCATTTGAACCATGTATTCCTTGT
CAGTTGACTTATTGCATTGCTCTGCATCTTCATTTGTCGAGTATTCTGGATCCATAGATA
AACAAGAATCTAGGTTGGTCTAACTGTATTTTTCTTGATTTTCAGATCTTGTTCTGAAAGA
TAAGATCTTTGTGTATGACTTAGCTCGGCAAAGGATTGGATGGGCAGATTATGATTGTA
AGTACCTCTTTTCAAAAATGAAGTCAACATTTGCTTTTGTCTCTATTTTGCCCCCTTTTCT
TTTGGGGTGGGGAGGGGGTTGTTTGTTGTGACATGACTTACTTCTTCCCTCTGATTTTC
TCTCTATTTTTAATGGTTTTCATGATTAATGTTTTTATTATAGGTTCATCATCTGTGAATGT
GTCTATAACCTCTGGCAAGGATGAATTTATCAATGCTGGACAGTTAAGCGTGAACCGTG
CATCAGGCAGTTTGCTGTTCAATCCGCGGCACACTAGAACTATATTTCATCTGCTATCG
TTGGTTCTGATGATTGGTTCCCCATTTTTAACT
SEQ 32 
TCAAGCAACAATAGGGTATGATGCGCAAGTTGCAATACCTACAAACATATCATTAGGAT
CAGTTATTTACCTCATCAAAATCATGACTACATTGACTAGAAAGTTTTTATTATTATTTGT
GTCGATCTAAGATGAACTCAACTTGAACTCGTATATAGAATAGCATTATAGTTCAGATTT
TGTGGCCTCTTTTTTTATGGTCCAAGTTGAAAGTTATTTTTCATTAATTTATCCTAATAGA
ATAAGCTTTCACAATCCGAAAGCAGTGTGCATCCAGTATGCAGAAAGGGGGAAAGGAG
ACTTGATGATGTGGGAGAAACTTACCACACATGTTCTTTCCCATCTCCATTTTGAAGTAT
CCATTGTCACCCCATTCAGCTCCCCACGAGTTCTTGATCAGCCAATATGGAGTACCATT
ATCAACACCATATCCCACAGCAAGAACAGCATGGTTCACATCCTGTCAATGCAATAGAA
AATGTTAATGTAGTATTATGTCATTGTCTCGCATATTTAGTGAAGGTCGGACCATATACT
TTGTTGATGTCATTGATATTTAACAAAAGAAGAAAAAGAATATGTGCCTCTTCATTGCAG
CAAAATCAATCATGTCTCAGTGCCAGATTAGTTGGGTTGGGTGGGGGTATGAATCCTCT
GTGTACACTGCTCATCAGACCTTTTTATTCCAATACTACATAATCTGTATATTAAGGCAG
ATTAGGAGTTTCTAAAAGTAGAGACTATTTCAATCTTTCCTATCATCCAGCATCTTTTTTT
GGTTGACAAAGCATGGTATCTTTCCTATCAGTATTATGACACTCGAATTGTTAAAAATCA
ATAAACACAGAGAGAGAGAGACACACACACACATTACGCATAACTTAAAGATAATTGCA
GACACCCTTCTCGACGAGTAATCAAGCACTGGGTTATAACTAACTACTGGGTACAATTC
TTTCCCCTACGGTGAGTTACATGGAAAATGTGCTTCTTCATCGACAAAGAATAGCAGATT
AAAAGGGCAATATCTCAAATTCATCGGAACTAAAAACTCACCTGGGGAGTGTTGCCACA
TACGGTGCTGCTGTAAATTCCACCCTTGTACTGTTTGAAACCTTTTACCACCTGATAAGC
TACACTAACCGGTCTAATAAATGCAATCGCGTATTTTAGTTCATCTTCAGCACCCTGTAA
TTATAAATGTTTAATTGTAAGGAATCCAACCAATGCCCATTGACCTTTTCAGGTCAAATA
ACGCATATTAAAAGATCCACTCAACTGAAAATCATCAACAATGAAACACTAGAACTATGA
AAGGTTTTTCCCGTCGGAAAACTACAATAATCTTCCTTTAGTGTGTCAAATACAAGAAAT
GATAAGCACTGATGTAAGCAGTTAAAAGCTGATCCAAGACATTGCTGTTCCAGAGTAAA
GAGGTATCCTACCTTGGTAATATTAACAGAATCAACGACTTTAACAGCAACATTTTCTGA
CGAGAATTTGCATACACCAGCCTTTCCAGCATAAGGATATTCTTCTTCAGTGTCAAGAC
 
CACCACTGTATTTAATGTACTCAAAAGCCTGTGATGGAAGCCCGCCATGGCATCCAAAG
TTATTAAAAGCTCCAGCACAGTCCAAGAGCTGCTGTTCAGACAGAGAGATGTTCTTCCC
AAATGCCTGGGCATATGCTGCCTCCAGAGCACCAGTAGTGCTGCCAAAACATCATGCG
CCGACTCCGTCATTTTTCAACTTCAGAAACCCGAATAGAATGGAAAGGATATAGAATTTA
CATGTTGTTAAGAATAAGTAGTTTAGGCTTAACGCATAGTTGATTAACGATTGATAGGTA
GAATGCCATTCCAAACTGAAAATTTAGGAACCTAGTGACATGATCCTAACTACTCCTTAC
CTGAATGTCCAGCAAGACCCGCACTTGCCCTGCTTCTTCACTGGGCTTACTATCCCTGC
TTCCCTCCAGTCTTTCTGAAATGACACATTCGGAGATCAAAATTAGGAACAAGAGCTACT
TTTGGTAACCAACAATTCGTTCACTTAGCACATGCTCTTCTAGGCAGCGGCTGAATGGA
AGGCAACTGTTCCTGAACTTACTACTACTTTTAGCTTGGAAAAGAAGAAAAAGTAAATAT
ATATAGATAGACAATACACACACACGTTTTTCTAAACACACATATATACCAAATTTTTATT
TCATTCAGATACAGTGGATGCAGCAGTACTCTGCACCTCTTGACTTAAAATCCTACGTC
CAGCGTTGCTCCTAGATGCAACAGCTATCTATGTTGTCATCTCAAGAAATTCCATCATCT
TGAAAGTAATAAAAGTTAAAACATAACAAAGTGATAAAAATCCAATGAAAGAAGAATGAC
AGCAATATTAGGCTGCAAAATGGACAAGACTAAGCATTTATGAATTTTCCTTTTAGGAGA
ACATACAAGATAGAGGCACAAAGAAAGCACTGAAAGCTAGATTTCCAATAACAGGATTT
CCGGTTGGAGTGAATGGATGCAAATGGTAATGATTTTTGAGAAAGTATCATAAACAACT
GCAGAAAAAGATATAATGGAGTTCTGATTGCAAATACCGTCTCTGGTAGGTTGACATTA
GTGAGCTGAAGATCGCTCTTTGTGGTAGCGGAACAGTTTTGAGGAGCTCCTAGCCTTTC
TCTCCTAAACTCATCCCATGTTAGGTCAGTAAACTCTGCCAAGAAGCATAGTCATTGTCA
AGAGCAAAATACAGCAAGCAAAATATGCAGTGCCTTCTTGTTTCTTTATTTTGTTTGTTCT
TCTCCTTTACCTTGACCTTGGTTTTCATATTTTTATTTTCCCTTTCTTCACTCACGTACAA
GAAATCTGCTAGGAGTTCTAATTAGAAGTAACAAAAACATTAATCTACTACATTTTGAAC
CACAAATCAAGTTTTTGGCGTCGTTGAAGTCTTAAGAAGGCATCCACCTTAGCATCCGC
ACCTGGGCACAACAAGGTTGAGGGAACAGAGGGTGGACGCGCAGCATCCACCCCAGC
ATCCGATGCTGAGAAGATCAAGATGAGGCGGACGCGAAGCATCCAACTCAACATTAAT
CCCTGAAGCTGATTCGGGAAAGCAAAAGGAAAACTTTGGCCCATAACTTTTGGACGCAA
TATATAAGCCAAAAACGGCTCTTTTAGGTCATCGAACACACTTTTTGAAGGGGATTCGA
CCTAGGGAGAGCAAGGAGCCGCCGTGGAGGCCGAATTTCATCTTCTTCCGCCAAACTT
AGTAATTTTTATGTTTCTTTGTATGATTTGTTGTTTGGCTACCATGTCTATGTGGAGCTAA
ACTTCACGTTCTAATGTTCTGGTTCTTTCATGACTATTGTTATTCGAGTTGATTTTCGTTT
CTTGATTTATCATATTAGTTTATTTATTCAATCCTGCGCTTAATTATTTGATTGCTTGATCA
CCAATTAAAACTATCTACGAATCTAGAATTGAACTCGAAAGTGTGAATTCTAGATTGCAT
ATAGGATTAAATAGAGCAAGTTCTTGAACCTGGGTATCGGGGAACGGATTTGCGGTTAG
GATAAACATATATACCCGATTGCCTTGCTTGGTTGATTTACACGAATTTCAAATGCGTTC
TTGTTAGTTCTAATTCCATAGACATATTGGCGTTAGGTTAGCTTGAATAGACGAGTAAGA
ACTCGAGAGATTCTTATGAGCAATATTAACACTGTCAACCAATAAACTAGATAAATTAGT
TAGTCAATTCAATTGAAGAATACAATAGGAATGTTAGATAACTCATAACCCTAGATCGTT
TTCATTACACTGATAATATAAAAATCAGCTCTTCCTTTGTTCAGAGTTCATTATTTATTTTC
TTTTTAGTTTAGTTACTTTTGCATCACTACTTTTGGGTTTAATCCTTGTTTAGATAATTAAC
AAGTCCTCATGGGTTCGACACTCTATCTTATCACTTTATTACTTGACGACCGCATATACT
ATACAAGTCAACTTTATGTATCCACTCTATTCAGATCATTTCATGAACTATAAGTAAGAAG
AAAAACCAAAACGAAAAAGGGCAAATTGTCCATAAAGGCATATATTGTCCAGCCTATAG
CTAATAGGAAACCATATAGTATACACAAAGGCAATTACCATTGACACCAAGTTTGTAGGA
AAGTCGTTGCTTGTTGTGAGACCTAATCATCTTCAGATTGTCCAAGTATATCTCAAACCT
TTGCTTGATCTCCTCAACTGAGTCGTATCTCTTCCCATACCTTTTAAAGTTAACAGATAT
CCCAAAAATAAATAATTTTTAAGTAAAAAGGAAAACAAAGCTTATTCATTCAAATAGGAG
GAAATTAGATGAATCGAACTGACCTGCGAACAAAGCGAACGAAGGAGAGAGCACGGCG
CGTTTGGCCGATGAGTTGGAGAATTCCATTCTCCAGCTCCTGCAAACCGTCGGATACTA
CTACTTGCCTGATCGGATTATCATCGTCAAACGTCAACGCTCCGCCTTGTGCGGCGGC
GATTGAGGTCGCGATGAGTAATAGTAATAGTATAATCGAGGCGCGAGTCAT
SEQ 33 
ATGAATCCTGAAAAGTTACTCACAAGACCAATGAGGCACTTGCTGAGGCACATGAACT
AGCTATATCAGCAGGGCATGCTCAATTTACCCCCTTACATATGGCACTGGCCTTAATAT
CCGATCACAACGGTATTTTCCGGCAAGCTATTGTGAATGCTGCTGGTAGTGAAGAAACA
GCTAATTCAGTTGAAAGGGTATTCAAACAAGCCATGAAGAAAATCCCTTCTCAAACACC
AGCACCTGATCAAATCCCACCTAGCACATCACTGATTAAGGTGCTCCGACGAGCTCAGT
CGTTGCAAAAGTCTCGCAGAGACACCCATTTGGCAGTTGATCAGTTGATTTTAGGCCTT
CTAGAAGATTCCCAAATTGGTGATCTTTTAAAAGAAGCTGGGATTGGTGCAGCAAGAGT
GAAATCAGAAGTAGAGAAACTTAGGGGAAAAGATGGCAAAAAGGTTGAAAGTGCTTCA
GGGGACACTAATTTCCAAGCACTTAAGACTTATGGTCGTGATCTTGTTGAACAAGCAGG
AAAACTTGATCCTGTGATCGGTAGGGATGAAGAAATTCGAAGAGTAATTCGGATTTTGT
CGAGGAGGACGAAGAATAATCCGGTGCTTATTGGTGAGCCTGGTGTTGGTAAAACAGC
AGTAGTTGAAGGGCTAGCACAAAGGATTGTTCGAGGCGATGTCCCGAGTAATTTGTCT
GATGTTAGACTTATAGCATTGGATATGGGGGCATTAATTGCTGGAGCAAAATATAGAGG
TGAATTTGAAGAGAGGTTGAAGGCAGTGTTAAAGGAAGTGGAAGAAGCAGAAGGGAAA
GTGATCCTTTTTATTGATGAGATTCACTTGGTTTTAGGTGCTGGTAGGACTGAAGGGTC
TATGGATGCTGCCAATTTGTTTAAGCCAATGCTTGCTAGGGGCCAATTAAGGTGCATTG
GTGCAACAACTCTCGAGGAGTATAGGAAGTATGTCGAAAAGGATGCTGCGTTCGAAAG
GCGTTTCCAGCAGGTATACGTGGCTGAGCCTAGTGTTCCTGACACTATTAGTATCCTTC
GTGGGTTGAAGGAGAAGTATGAAGGGCATCATGGTGTCAAAATTCAAGATAGAGCTCTT
GTGGTGGCAGCCCAGCTTTCGGCTCGATACATTACAGGTATGTCCTTTTTTGGATTGTC
ATTGTATTTTATGAATTTTACCTTTGATCTTTAATCGAGTAAAGATGCCACTACAGGAATA
TAGCAATGTATGTAATGTTGAAATGTGATGTGTCACACGTTTGTATTGTGGTTGTCAAAA
CATTTCCTAAAATTTTGAGGAGATAGTCCCTTTCCTTTATGTCTATGCAGGATGGATGTG
AATCTAGTTTTATACTTAATTTAGCTGAATCACGTCCCATTTGAATGATAAAGTTATTTTC
TGCTTCATTGTGCTTTTCAAGGTGATAACCTCTAACCTTTGGTTTGTAGTTTCAGACTTAT
AAAAGTATGATTGGTGCGTGCTCACCTTAATTGATTGGATGGGATTATGTGTTTGCTCTC
TATTAATACGAATTTTCTTTAAAGCTTTTTCTCTCCCTTGCTATGGAGAATTGCTACTGTT
GTTTTGCGTATCATTTGCCAGTTTGCCATAATTTTGTGCATATAGGGATTACTAATCTGT
GAATTTACGTTCAGGTCGTCATTTGCCAGATAAGGCTATTGACCTAGTTGATGAGGCTT
GTGCAAATGTAAGAGTTCAACTTGATAGTCAACCTGAAGAAATTGATAATCTTGAAAGAA
AGAGGATTCAGCTAGAAGTTGAACTTCATGCACTTGAGAAGGAAAAGGACAAGGCTAG
CAAAGCTCGACTCGTTGAAGTAAGTATACATCCCGGAAATGCTTTGACCTATAATTCTAG
AACCTGTGTAGGAAATGTGGACAAATAACGTAATTACTATTTCAGGTGAGAAAAGAACTT
GATGATTTGAGGGACAAACTCCAGCCTTTGACGATGAGGTATAAGAAAGAGAAGGAAA
GAATTGACGAGCTTCGCAGGCTCAAACAAAAGCGTGATGAACTCACGTATGCTTTACAA
GAAGCTGAAAGGAGATATGATCTTGCTAGAGCAGCAGATCTTAGGTATGGGGCTATCC
AAGAAGTGGAAGCTGCTATAGCAAATCTCGAGAGTAGCACAGATGAGAGTACAATGTTA
ACTGAGACTGTTGGACCTGATCAAATCGCGGAAGTAGTCAGTCGGTGGACTGGTATTC
CTGTGTCAAGGCTTGGTCAGAATGAGAAAGACAAATTGATTGGTCTTGCTAATAGATTG
CACCAAAGAGTGGTTGGGCAGGATGATGCAGTTAGAGCTGTTGCTGAGGCTGTATTAA
GGTCTAGAGCTGGGTTGGGAAGGCCACAACAACCAACTGGTTCATTCCTTTTCTTGGG
ACCAACTGGTGTTGGAAAAACTGAACTTGCTAAGGCTCTCGCTGAGCAGCTCTTTGATG
ACGACAAGTTGATGGTCAGAATTGACATGTCCGAATACATGGAACAGCATTCTGTTGCC
AGGTTGATTGGTGCTCCACCAGGGTAAGGACCCTTTAACTATTGATAGGATAAAAGAAC
AAATCATACTTTTACGAGTAAACTGTATCTGCCATAATGAGATTGTGGATTGCACCTTTT
GTAGAACTCTGTAGCCTCATATTTGTCTAGGTACTTAATAGTTTTACGTCTGAAGTGATG
AATGCTGAACATGTTATGTGTGTGCAGTTATGTTGGACATGAGGAAGGAGGACAACTCA
CTGAAGCTGTGAGGAGGCGCCCTTACAGTGTAGTGCTTTTTGACGAAGTGGAAAAAGC
 
TCATCCCACTGTATTCAATACCTTGCTCCAAGTGCTGGACGATGGACGATTAACAGATG
GCCAGGGTCGTACCGTTGATTTTACTAATACAGTCATCATTATGACCTCAAATCTAGGA
GCAGAGTATCTCTTGTCAGGATTAATGGGCAAGTGCACCATGGAGAAGGCCCGCGATA
TGGTCATGCAGGAGGTAAGCTAGAACAGCCTATTTTCTGCTAATTTTCTGAGCATTGTTT
CCTAGTTTACATCTTTATTTGAGGAAGGATTGTTCACATATATCTTTTTGTGACAGGTGA
GGAAGCAGTTTAAGCCTGAGTTATTGAACCGGCTAGATGAGATTGTAGTGTTTGATCCT
TTATCACACGAGCAGTTGAGGCAAGTATGCCGTCACCAACTGAAAGATGTAGCAAGCC
GTTTAGCTGAGAGGGGTATCGCCTTGGGCGTTACCGAGGCCGCGTTAGATGTCATACT
TGCTCAGAGTTATGACCCTGTAAGTATCACCATCTGGTATTTCAACCTGACATTTCATGG
TGATTAGACTAGGGTCTGAGTTGAGATACCAACTATGCAGATTTTTGCATTTATCTTGCT
GTGGCGGGTTACACTTGTTTTTCAGTTGCTAATTTCACTTATTATGGAAAATTATTTGTA
GTTACATTTTAGGTGATCTAACATTCTAAAAATTATCTTAGAACCGTTGGCGTATAGAAG
CGAAATACTTTTGACAATTGATTGTGCTAACTTTTGTTACAATTACATCACAGGTTTATGG
TGCAAGACCTATTAGAAGGTGGTTGGAGAAAAAGTGGTAACTGAGTTATCCAAGATGC
TCGTGAAAGAGGAGATTGATGAGAATTCTACCGTCTACGTCGATGCTGCATCCAGTGG
GAAAGATCTAAGCTACCGAGTGGAGAAAAATGGAGGGCTTGTCAATGCTGCCACTGGG
AAAAAATCTGATATATTGATTCAGCTCCCTAATGGAGTGAGGAGTGATGCTGCTCAAGC
AGTGAAAAAGATGAAGATTGAAGAAATAGTAGACGAA
SEQ 34 
TCACTTGCTTTCAGGTATGATACTAACAAGGAGACATACTATGCCAGTAACAACAGGGC
TCGCCACACTTGTGCCAGATAATCGTTTACAACGTGTGCTGATTTTGGATCCCATAATCT
CACGCCCATATGCAACAATGTCTGGCTTTACACGACCATAACTGTCAAGGCACAGTACA
GTTAAGAATCTATGGTTTGATAGACGAGTGTAATTATTTAACTGTTATTAGAAAAGCAAG
ATCCTAAAATATTCAAAGAAAAAGAAAGAAAAAAAAATGAAGCAAAGAACCATAATTTTG
AACTTAATTCTTTTTCAAGAAAAAAAGAAGCAACAATAACAACACCAATAACAAGCCCA
GTATTTTTCCACAAGTGGGGTCTGGAGAGGGTGGAAGTACGTACCCTTACCCCTACC
CTAGAAGGACAGAGAGCTTGTTTCCGATAGACCCTCGGCTGGAGAATGGATGACAAAA
ATAATGGCAACAATAAGGAATAACAACAAGATAAAAATACTGAAGCCAAGAAAGCAGCT
AAACTCTAGGTAATAATAGCAATCTATGAATAAAAGGATATCATACTAACACTGATGCTA
GCGAACTGGGAAAGACAAAGAGATACGTTCGACTACCTACTAGCCTTCTACCCTAATTC
TCGACCTCCACACCCTCCTATCTAGGGTCATGTCCTCAGTCAACTCCAGTTGCGCCATG
TGTTGTAACCTCGCCCCAAGACTTCTTAGGCCTGCCTCTACCCCTCCCGATACCCATTG
TGGCTAACCTCTCGCACCTTCTAACTGGGGTTTCTATACTTCTCCTCTTAACATGCCCGA
ACCATCTCAACCTCGTCTCCCGCATCTTTTCCTCCACCGAAGCCACTCCCACCTTATCC
CGAATGATTTCATTCTTAATACCTAGTATGCCCACACATCCATCTTAACATCCTCATCTC
AACTACTTTTATCTTCTGGACATGAGTGTTCTTGACCGGCCGACACTCTGTGTCATACAA
CATAGTTGGTCTAACTACAGCCTTGTAGAACTTACCTTTAAGTCTCATCGGCACATTTTT
ATTACACAAGACACCGGTAGCGAGCCTCCACTTCATCCATCCAGCTCTGATACTGTGTG
TGACATCCTCATCAATCTCCACATTACCTTGTATTATAGATCTAAGGTACTTAAAACTACC
TCTCTTAGGGATAACTTGTGTATCAAGCTTCACCTCCATGTCCGCTTCCCGGGTAACGT
TGTTGAACTTGCACTCCAAGTATTCCGTCTTGATCTTGCTCAACTTGAAACCCTTAGACT
CTAGAGTCTGCCTCCAAACCTCCAGCCTCTCATTAACACCACCCCGACTCTCGTCAATC
AGAACTATGTCATCAGCAAATAACATACACCATGGCACCTCTCCTTGAATGTGTTGCGT
CAATGCGTCCAAAAAAAAAAGAAGCAAAGAGCTTAATTGTGACTTTTTCTATTTCATG
TTTACGGTTCATCTTTCTTCCTTTCCTTTTTCCTTAGAAGCTGAGTGGATTGTACAAGA
GGCATTCAACAGATGTCATGCTCCTATTCATCCATAAAGTTTTGCCATTTTCACCCATC
ATTTTCCACTCAGCAGAATTTTACTCGAAGCATCACAACCATGGATAGAATAAAGCTCAT
AGAATGCTCGTTTGTTTCACAAGAGCTGATTTTTAAAGGCATCTTTTTTAAATGAAGTTG
 
GTACATCCAACACACTCATTGACTTCCTATGTGGTCATATAGTAGAACACAACTTTAATA
CAGAGAAGGAGAGGGCAGAAAAATAAAGAATACAGACTATACTCCATTGCAAAGAGTAA
TACATAGCAAAGAAAGGAGAAAGAGATACCCATGAGGAATCTCCCAGGTACTCATTCCA
CGTGAAGAAAATGAGGCTAAATGATTACTTTGATCAATGGCACCAACACCAATAACATC
ACTTTGATCAGCAGGATTGTTAAGAGTACCATAAAGTGGTCCATCATTTCCAATAGCAGA
AACCATGATAATATTGTTGGCAGTAAGCTCCCAAACCTAGCGGAAAATATTGATTACGTC
CATCAATATAAAGCAACTATGAGGAGAGACTCCAGAGAGTAAAGGTGTTAAGACAAGTA
GAAATAAGACTGAATATATGCATGCTAAGTTTAGCAAGCATGAGAAGAGTGAAGTTGAG
GTAAGATTAGATAAGATTCCCATACCTAAAATGCACACAATTCAGATATCTAGACTCATT
TTCCATGAAATTAGTATGATCCGTGAAGATATCACATCAAATTTGAATAGAATTGGTTGT
AATGGAGGTGCGCTACAGAGGGTTATGTGATAAGAGGATACCTATCAAAGTTAAAGACA
AGTTTATATTGGGGTAAATGTTTGGCTTTGTATTAAAGATATGAGCATCACCAAAATGCA
TATGCAAGATGGGTATTATAAAAGTTTCACAAGATAAGAAATGATCAAATTTGACAGAAC
ATAAAGGATAAAATAAGAATGTCGTTTGAGATGGTCTCATCATGTCCTAAATAAATCTCC
AAAGGCACTGGTCCTTAGATGGAAACCATGATGATTGGAGGTGCTAAAAGAGATGTATA
CCTAAAATCACATGGAAGGAAGTTGTCCCAAAAGACTTATAATCTCGTTGAATTCATACA
GACTCAAAACAGAACACAACAGAAGCAAAAGTCTGTTATACGCGATATCGACTATTAAG
AATCAAGGTGTAGTCATGCTAGTGCACTTACTTTAGGTCCAATGTCTACTAGGAATCTTT
TTAGTCTGTCTGCACTTCTGAAGTTTGTATGTCAGTAGAAAAAGAACCTCCAGATTTATA
GATATCCAAACTACTGAATCTTTGATGACTCCAAATGGAGCAGAATGGATGGTGAGGAT
TCATTAGCCAACCCAACTAGCTTGGAATTAAGGAGTAATTATTCGTGTTGTTGTACATCT
CATCAATATAAAGGTGAAAAGTTCTGCTAATGTTGTTTCAGGTCCTGCTGAAAGTAATGT
TAATTTCAGTGAAATGACAGGCTTAAACAACTCCGAATCTCTTTTACAAAATTGAGTACT
AGATATAATATACAACCCTTGTGTTTAAGACATCCATGACATAGTTCAGCTTGCAAAATT
AATAGATCTCATGAAACAAACGCCCCATTAAGCTCAAGAAAGCCAAGTAAAATCCATGC
ACTGCATAAGAAATATAAGATTACATGCTGCCAATTATAATCAAACTTCTAATACTTCCGA
GACCACATATTATACAGAAACTTAGACAATAAGGGGTTATGGAACAACAGCAAGATCAT
TTCAATGCCTATGCTGTAGACAAAATGCAATCCAGTATCATACCACATAAAAACAATAAA
GATATAAACCAATAGATAAGTGACCTCACCTTTTCCACAAAAGGGAGATCCAAATAATCA
GGTCCACCTATGCTCAAATTCAGAACATCCATGTTGGTTGCAATTGCGTAATTAAATGCA
TCGAGAAACCACGATGTGTAAGAGACCTGCAATAAACTGAAGAGCCACTTCTTATAATG
CTAAATTGGTCATTACAAGATTGATCTTTTTATTTCTAACTTTTTTATAGGTCGCCTAGCG
TTGTCCTTGTCTGTAACAGTAGCTTTAGTACATGAGTTAGTGTTATTTATGTATTTTCGTA
TTCCTTGACTTATGTGATTACTTGTCGTTGCTTTCGTTCCGGCCTTCTAATTGCAATACT
CAGTTTTAGTTTTGTTCCTTTGTATTTTTTGCTTCGGTTTTCTAATTGGTGTGCTTGTTGC
TGCTCTTCCTTTTATCTTTCCTAAACCAAGGGTCTTCCGGAAATAACCTCTGCCTTCTTG
AAGGTAGGGGTAAGGTCTGTGTATGTACTACCCTCCCTAGACCCCACTTGTGGGATTAC
ACTGGGTCTGTTGTTGTTGTTGTTGATAATGATGGTGTCAAAGCAAAACTTGTCTCGACT
ATTCCAAGGATACCTGCAACCTCCCACTAGCACAGGTACCGGGTATCTCAACCCACCAA
GGCTTAGGCAGATGGGTAGATATCACCTAGCATTTTTATCTAGGCAAGGATTTGAACC
ATAGTCTCCAAAATTTTAACCCACTTCATTGAACGCTACCCAACACCCTTGGGTGCTACA
AGATTGTTCCTTTTTGTGTGAATAGACTCTCTTTCAAACCCCAACATCAAGGATTCAAAC
CCATCGAACCCATGATGTGCGTCTAACTCACACATCACTTGTTGCGCTCTTACCACTAC
ACCAAAGCCCTGGGGGTGAATACTCCATATCATCTTGATTGTCCTTACTTGCGGATATG
GTTTGTAGCTACTGAAATCAATAGATTGCACAAAGCCAATGATAGGTAGCTTAATTGGAA
AAACTGAGGTCCAAGAACGATAAGGTCTTGGCCTCAAAATCTCCATGAAAGGAAAATAG
TATTTAATGTGTGCCTTATGTAAGTAATTTTGTTTTCCAATTTACTATTAGCAACACTGTT
ACTTGTTATAGTATCTCAAACAGCGCATACAGTTTTATAATATTTCAAACTGCTTATACC
TCCCCAATAGTGGGCTAATACTAAGTATGGGCTTCCTTGAAAACAAAATAGGAATTATAG
ATTGCACATAATTCGCAGACAAGTTCCTGGCTTTTCTAAAACATAAAAGTAAACAATGTC
CTCCCTCCCCAATCCCCTGAAAAATAGTTGCAATCTTATCACTAAAGTCATAATAAGATG
 
GCAGAAGAAATATTATATGTTCAATAACATAGCATGTAACATGGACTCCACCACTAAATC
CAATCAGTGGGTTTGGCCGCTGGAAAGGGTATGAGAAAACTGTGTATATTTAGGTGCAT
ATTCTTCCAAGATATTGTAGCTTAATGATGAGAAGTTAAAGCTAGCACAAAATAAGGTGC
AGAAGCAGAACTTGTCATTTACAGAGACTAGGCAGTCTAAAGTATTTTTCTTCCATTCC
AGAGGACTTTTCACTAAAAACTATGACTGCAAGAATTTGCTATATTAGGTTCACCACTCA
TGAGGTGGATGTGGCACACTCTACTAGCAGAAAACTGGAAGGGAACGGGGAAGGAT
CTTAACACATCAAGTATTTGCTTTGCTGCAATTAACAACGAAAGGACCGTTTGATCATAG
GAATCATCATTAGCACTCAGCAAGAAGCAGACTTGTATAAAACATCAGTACAATAAATTA
GAGGCAATAATCCAAGACATCAGATTGTTGAAGATCTTCAAGTCTCAGCTTACTTAAACA
GTTTAAGAAAATAAAGCCCCGTCACCCCCCAAAGAAAAGGAATTGGAATACTCGTTCAA
AACAATCCATTACCTGTGCATCTGTAAATACATGGAAAGCATAGATTTCCGCATCTGGA
GCAAAACCGAGGCATTCTTCATCCTGACCAGCAATAACACCAGCTACAAATGTCCCGTG
TCCAACATTGTCATTCAATGTATCTTCGTTGGTCCAATTTGTGCGTTCCTGAAATTTAAG
GTACCAAGCCCACTGTCTGTTATTAATGACGTAAAGATGGTACGAAGATATATACATTG
GCATATATGTAAATGTCAAGCCTCAAACCACATTGGATTCAACTAAAGCATTTCTTTGTT
ACAGAAAATATATTTTTAAACGACAAAGACAAGATACCTTGATATTACGAAAATGTGGG
TGATCTGCACGGATGCCTGTATCAAAAATTGCCATTTTGACCTTAGCACCAGTATGCCC
TTTTGACCAAAGCTCATGTGCCCCAAAGAGGGATGTGACTCGAGATTTCTGCAAAATAA
TCATACAGGACAGACTGTATATCAAGAAAAATACAGCGAACAACAACAATATGTTCAATC
TAAAAAAGAAATAGAAAATAGAGCAGCAACATGAGACCCAGACACTCAAAGAATGCAGA
CCATGTTTTTCAAAAAGGAAGTATGCCCTGACAACCTTGAGGAAATAAGAATGACAAATT
ATAAACCTATTAACACTCTCCGATCTGAATGTTACGCTCCAACGGCCAACCAAGTTAAA
GTGAGCCACTTAATGATCTAAGGATATATAATCCATTCTCGTTAGAGGTACTTTAGTGTC
TTGAAAGACTAAAAAAACAAAACATGGTCATATATCCAGTGCAAAAGAGAATATTGGGG
CATAGCAGAGACAACTTGTGAAATTATATGGATCACAGGGTCGCACAAAGATTAAACTT
TATAGTGATCAGTAAGGTGCAGCTTCTGTGTAATAATCAAGTTTCCCTTCATATTGCGTT
GAATTCAGTGTGTCATGAAAGATATAAAAGAATATTATCAATTGTAACTTCGTCACAGAG
AAGATAATCTCAGAAGTCATTTTCACAAGTTCGTGAAGTCGAATGCTTAGATTGTAGATA
TATCCACGAGTTCTTCACTAGTCCCTGAATTAGTCACATATGTAACAAGCACTAGAAAGG
GACTGTTAGATAGTTACGGGAAAATAGCTAAATGTAAATACTTATATTTATTATAAGTGTC
CCACTTCGGGAAAACACAGGTATAGATATTATTACATTGTCAAGTGTGCCCATAAAAGG
AATCAGTTGTAAGATATTAGTCTTCAAGCATTCTCTAATCTTTCTCTTATTTTTCTCACTAT
GGAGTCTCAGCATCATGACATATTAATGGAATAACAGACACTTGATTAGACCAAAATAG
GAAAAGGACAAAGACAAAAGGGAACTGAAAGAGATTAATTTCCTTTGAACATATACCAT
GCAATAAAGTTGCAACTATCATATGTCATGAATGCAAAGAGAAGAGTTGCATACATTCCA
ATGATGTAAATTATCAGTAAACGCATACAAAATAAAACACAAATAATCAGTGGTCTTGCC
TGCATCAATAGATGTCTGCTCCAGCTAATTCTCATTATACTAGTGTTGGCCACCGCATAG
TTTTGACCTTCACTGAAGGACATAGCAGTAAAAATCTTTCCTGGCCTCTTCTTCCCATTG
GCAAAAGCCCCATTCTTCTCACTCTTCTCTTCAAGAACTATCCTTTGATAGCTCAAATCC
AACGAAACGTCTTTTACAAGATTCATTTTTCTGAACTTTTCTAGCAAGAGTTCTTTCATTG
ACTCGTCGATTTCCACCAATCCAAAGTCAGTAGGAAATCTCGCAGCCGGATTTTTCCGC
TCAATCCATTGCCAACCCTTAAATTTCAAGTTGTTTTGAAGATAATTCCAGTGATCCTCA
GGTTCCTTATAATGATAGAATCGAACAATATAATTTCTGCTATCAGATTGTTGCTTCTGG
TCATGTTGGCACTCATCGGAGCTACTAGAAATTAATGGCTCAGACTCTATTGGTGGGTT
GAAGCGGATGAGTGTATATACCGGAAGGAAGGGGACAAGTGAGAGGGTGAAGAATGA
TTTCTTAGGAGCTTCAGGCAT
SEQ 35 
TCATATTGAAGCGACCAAGTCTTCAGTCTCAGTCGTCTGCTGAGAAAGGGTGCCTCCAA
TCCACCTCTTGAGCATCTCTAAGGCAACTTTAGGTTGATCCATTGGAACCATGTGTCCA
GCATCGTGAACCTGTTATAAGACACCAAAACAGTTAGCTCAAACATCCATCAGTAGAATT
TGAACAATAACATCGACAAAGAAAGGCACCTTCAGGAAACTCAGAGGCCCATGGCTTTT
CAACAATCCAGCTTCAGAACTGTCAACTTCAAAAGGAACATCGGGAGATGCTACAAACT
CTTTCTGACCACTCCATTCCATAGCCTGAACCCATCTTGAGTTACCTGGTAAAAGAGAG
GCGTTATAATATCCGAAATATTTATGTGAAAAGTTTCCATCATTAGGCTTAGAGTTGAGT
CAAAGCTTACCAAGCCAGTTGCAAATAAGATCATATTCTCCAGCATAAACAAGCAACTTT
ATTCCATCCTCGAGCAAGGTTGGAATGCCAGCCTCAAGATTCCTCATCCAATCAACAAG
CATGGCCTGGTACACAGTAGTGCTGCATGAGACAAACTCTATATCCTCAACTCCAAGAG
CCTGCTTAACAGAGTGCATATTCAGCAATTTCTCCATGTTTGAGAAGTCATAGCAGAGT
GCTCCAACGCATTTCTTTCTGATGTCGTAATGCTGCAGGTGAAAGCTCAATGGATCAGA
AATATGGTTAATCAGTCATTTGTTCCAAACTTTGGAAGGCATGCCAATGACAATGTGACC
TCTTGAGTAATTTAACATATTCAACATGAAATGATATGGAGTAGCGATTTAGAAGAAATA
GATTTCTGGGATCATTTCTACTCTTTCTGAGGCTAGTAACACCTATTTCTCTACGAAGTA
CAGAATAAGTGTAATAGGCACTAGAATTAGTAGAAAACGGAAACAGACAGAAAGGGCT
GATAACTTACATTGATGTCAGCCCCAGCACGTGCACGAACAGCAGAGAATATAGAATTG
CAAACAAAATAGGCAGCCAAGCAAGAGATTTTCCCATCAGTACCTAAAAGAATAAAAAA
GACAGAGAAACTGAGAAGCAAAACAAAGTACAGAGAATTGATTTGCTGTGGTCAAAGAA
CATCCATTTACTTCATCAGCTCCTTCCCCTTTTCTTTTTCACCCAGGGAAAGCCCGAATG
AGTCAAATGATATGGAGGAAAGAAAGATAGTAAACAGTAAATTAAATAATGTACCACAAA
GGTTTATTGCAACTTCACAAACTGGAAGTATTTTGTTGATACGATCATGATCAGACTTTG
AAATTAATCCCATGTCCAATGCATAGTCAGTATACGCAGCGTATTGTATTTTGGGATCTG
TAAGCCCATTCCCAATGGCAAATCCCTGTTCAAATACTTTCAATGAAGTAAAGACACATG
ATTAAGGAAATAAGAATTCAATAACTGGGAAAATGAGGTACCTTTAAGTTTATATGTATT
CCTTCTTTAGCCTTGTTTCCCTTGTGTACTCTAGCAGCAAAAGCAGGAATATAGTGCCC
AGCATATGATTCTCCAGTTATGTAGAAGTCATTCTTTACAAGCTCAGGATGCTCTTCAAA
GAAAGCCTACACCATTATTAATGATCATACCAACACAAACAAGTCAGGATCATATTATCT
CTGTTACGCATCAATTTAGAAAAATGCTAACTGTGTCACATAAAAGAACGAATCTGAAAT
AGCCAATGTGTCACAAATGCTCAAGAGAATTCATACCACATTCGGCCTTCAGAATTTGC
AAAGCAGAAAAATACAACAATAAAAGCAACATATAACAATATTTCCAACTAGAAATCTGT
TGAAAAATTCACGTTCCGAATAGGTAATGTATAGTCTTAAGGCGGCTAAGCCAAGTTCT
GCTAAATATTCTGGTTTAAAAGCTGTTATACATGCTAACAAAATGCATCATGAGGGAAAC
AACTGACCAACAAGTTACCCAGCCAAAATTCAGGGATCAGCTGCAGTTTGTAGTAAAAA
ACAGGAAAACCAGCCCATGAAGAAGGGTATTGAATACTGCAAAAAGGTTGAGGGACAG
GGTTTTCTAGCAATGTGATCACATCTTTTGCCCCTAATGCATTTGGCAGATAAATGGAGT
CAAAATATTTTAACGCCTCCATTTTTGTTGGGATCAGAACCCAGGCATCAACCAACTATC
ATTTCATAAGCACAATATAAGACTCAAGTCCTAGTATATGACATCTCTCCAATTATCTATA
TGGTAAAAGTATTAAGTGACCATGTTTCTTTTGACAAGAGTGGGTTGCTCTAGTGGTGA
GCACCCTCCACTTCCAACCAAGAGGTTGTGAGTTCGAGTCACCCCAAGAGCAAGGTGG
GGAGTTCTTGGAGGGAGGGAGCCTAGGGTCTATCGGAAACAGCCTCTCTACCCCAGG
GTAGGGGTAAGGTCTGCGTACACACTACCCTCTCCTGACCCCACTAGTGGGATTATACT
GGGTTGTTGTTGTTGTTCTTGTTGTTTCTGTAAGGCTCGAGTTCTAGCATTAGAAATCAG
CCTTTTGAGCTCCTGTAGACCTATTGTACTGTACCCCGTCTTTGTATCACATGTACACAG
GTGATCAACACACACAGACAAGAGAACTGACAAGCAAACCGCACTCTGAGAGTCCGAGCA
ATTACTTGAAAGGGATCCCAGACAACCCTTACTGGCACCAGTACTTTAATTTGTCACCA
CTCACCAGGCGGCCAATTCTAGACAGGTCAGCAAGGCGATAAACAGAGGTGTCTTAAT
CATCACAATATCATCCCGCACACAGGAGTGGGGATCAGCAAACACTTCTTTTATAACTG
AGAAACTATTGTTTCCAGCATTGAAACTGTGGATAATGAGCCTGCCCCTGTACTTTCTTC
AATTTTTTTCAGAACAGGATTCAAACTACTGACATGCGCCTACCACACATCCCATGTTCG
AAATTGAAACCAAAGCTCTGGGGCACTGGAGATTAGCAAATAGGCTAGGTGATTATAAA
 
GATATCATTCAAGAGTTCTCCTACTAATTCACGGTACTTTCTACAAACCCCTCCCTCCTT
CCACAGTTGATCACAATAAGCTTGACTACTGACGTATATGTCAATACCACAGCCTCTGT
GAGATAGAAAAGCTTCCATAATGACTACTTGAAAGGAGACCAAGGGGGTTTAGAAAGTA
TTTATCATTCTGTAAGCTACTGCAACAATAATGATTTTACTTAACGGAAAGGAATGCCAT
AAATGAATTTGTATTCTTGAGGATGTTCACAACCAGGACTGAAGTTGCTTCCACCCCTA
GCTACATTCTTTATCCGTATTAAGGAAAAGTTTACCATCCTTTTTTTCCAGGTGAAATGTT
TTATTGGCCTTTAGAAGCAGGACAAATTGTCCAGTGCAAGCCTCATGAATAACATACAT
GAAACTAGGAATTGATAGGTGAAGAAAATATAGGAAACCATCCATATCATTAAGTTAAAC
AATTGACTCTCTGTCATCATACGACAATGATTACAACGGTTGATTCCAGAGGAATAAATA
TGGTTCCAAGTTGCTTTAGGGGTTTAATTTCACACAGAACAAACCTGTAGGAAGTCATA
CAAGTCGTCGCTAACACCTGCTTCACTGTGACGGATGTCATGTCTGTCAGAACTGTAAC
TAAAGCCAGTACCTGTAGGTTGGTCCACATAGATAAGGTTTGATACCTGCAGAAATAAC
ACGTCAACATCATTTCTTCAGGNCGTCAACATCATTTCTCTAGGTTTGGAAAATCTATTA
AGGTATTCTAGTGTCCTTGTAGGAGAAAAAGGAAAATCGATAATAAAAATGAAACATCTA
CTTTACAAGGAACAAATGTGGAACACAAGGCAAACTTGACACTCTAGGAGTCAGCAATA
AAAGACCCAAACCACAAAAACCAAAACTCAAGATCTTATGGAACATAAAGCACTTTCCTC
TCTGCATTCTTGAATTGCCGTGCAGGTAATTTTTTTCAGTAATGAGAAAAAAGAACATTA
AAACAGGCAGAAGCATGACATGGAATTAGGGAAGTGCAGTATCAGAGGTCTAATGAAA
AAAATATGGCTGACATGTTTCCTATGCAAAGCATTAAGATTTCAGTAAAACACAAAGCTC
CTTCCGGGAAAAAAAGTTTTCTTGCAGTCTGGTTGATGATAACTACATAAATGCTGAAAG
TGTAACTATCTAAAGGCTAAAAAGGACATTTCTCCTGACAGATGTATCATGTGCAAGAAA
GAAATGGTCCCCACAGATTTTCTTCACATTGATGAAGTATCAATTTGTGCCTGTGAATTG
GCATTGCATTCATTTCAGGTTGGTTACTGGTACATCTCGAAGAAGGATAATGGTGAAGC
TTGGATTATCAAAGCTTAATCACAAACCAGTATCCTTATACTTATGATTCTTCGATTTTAC
ACAACATTGAACTAGATATAGACATGTTATTAACTGTTTTATCTTGATTTCCTTTTCAAGT
AATCTTTTCAAAACTAAAAATGATAATAACAAAGAGGAACATCCACAACGAACAAGCTTT
TTCGCTAATTGGCACTTTACAAATAAATTAGCAGGCTGCAAAACTACTTATCATGAAAAC
GACTGGATAAAGGACATGCAAAAGATGTTGCCAATGCAAAATCTAATAATCTATACGCC
CATGCACCATTATACTCTTAACTTTCATGTACCATTGAGTAAAAGCAAGAGAAAGTCTTA
TACTATCGAGCTGAATCATCTACTAAAGAGCAAAGAAGGTAAATTGCTACTATTGCTTAA
GCAAAGCTGTGTTTACATACCACAATAAACTGCTGTTAAATAGCATAAACCATTAAGACT
ATAAGGTGGTCTTAAAGATAGTCAATTACCTTGTCCCACCCATATTCATTCCGCACAAGT
GACAAATTATTTGAAATAGAGAAAGGTCCATTTTCATAGAAAAGGGCCAACTCACTGCT
GCAACCAGGCCCTCCACTCAACCAGATGACAACAGGATCGTCCTTACTACCGCGTGAT
TCAAAGAAGAAATAGAACAACCTACAAAATGAAAGATAACACAGTTTCAAGCTAAATAAG
AGCCCAACCAACAATAAGAAGAAGCTAGTTGATACGTTAGACAGGAAGACGAAGATATA
AAGCCTTATTACTCCGGAGATGTATATTTTGGTAAATGCTCACGAAAGACAAATATTTAA
ATGAAACAATGTTGCAGCATTCCTTTCAATTCTTACAACTGATTTGGAAACTTCTTAAGC
GCACTGATTGAGTTAAACAGATAAGTGGTCAAACTGTAACAATCTGATGGTTCCCCACA
AAAGCTACATGTAATGACATTTGAAGGTAAATAAATCAAGCAGACAGAATATTTCTCCAT
TAATCAACCAATCAAATAATTCCAAATTAATTGGGTTGACTAAATGAATACTCTTTAACAA
TTCTGCTATATTTAAATCCAATTTGTTGCAATATTAAATAAATATCCTTTGAATTCATTTCC
ACACCAGTTACAACCGTTTTATTCGAATTTCCGAAGTGGAAAGTGAATTTGTAGGAGGA
TAGAGGGGAATGCAGGGGTGCACTGCACGATAGAAGACTACTGAGAACTTGGCTACAT
TTTGTAGACAACAAAAACCTATTACCCCAGTACACCACAATGCAATGCTGAATCCGACA
GTATTTTAACATAAAAGAGAAAAGAAAAAATTGAAACTATAATTTGGTTGGATGCCTATG
ATAATGCAAATCGGAACAAGAATCACCAAAAACCAGGAAGTGTTTGAAGCAACAAATAT
GGTACTAGTCTGTTCCAATTTGATAAACTTTCTATTTTTGGGACGTCCGGGAACATTTGA
CACCAGTTTGCTAGTATCTTAACAATTTTCAAGATACTCTAATTCATTTCTTTGAAAAGAA
TAGACATTAGGCCTAACTCAACATCAAAAGCTAGCTCATGAGGTGATTGATTGTTCATTC
AATATATAAGGAGACAACAGTCCACTCACTCCACCAATTTGGGACACTTTAATATTTCCA
 
CACGACGAGGCCGTGGACAACTGGAGTGTGGACAGCATAACTTGCGACCCCAACATG
GAGAAACACAATGTCGACCTTACCCTACCACAACCTCAAAAACTAGCTCACGAGGTGAG
GAACGGAGGATTGCTTGATACCATAGGAGACAACACTCCATTCCCTCAACCAATGTGAC
ACTTCAACATTATGTGTTATTTTTATCGAGTTACATTTTCTACAGTTATCGAAATATGTTTC
TTACAATACTACATCGTACATACTCTAATAATTAAATCAAATCAACATCTTAAATTCCGTG
TTCAACTAAACACTACCATAAAAATCGAGACAGAGATTGAAAAAAAAACTTAACCTAGGG
TTTGAAAAGGTACCTAGCAGCATGAGAATGCTTAATCTTATAATAACCGGCATGATGCC
CCAAATCTTCAAAGGATATAACACTCGAATTCGTCAAATTAGCGAAATTAAATCGCTTCT
CGACGATTCTAGAAGCAGCGGTGGGAAATGGATCCCGATCGACAATGTTATCGGATTC
TTTCGGGAACAAATTTAGCTCGTGTATCAACTTCTCAGCTTGCTTCGATGCTAACTTCGA
AGATATTGAAACCTTCGCGAATGAAGAAGGAGAAAAAGCAAGGAGAAGAACAAGAGAG
AGAAAAAGGGAAAGTGAAAGCTTCATTTGCGCCAT
SEQ 36 
TCAAGAAGAAGGGGTCTTCTTTCCCTCATTTCCGAAGGTTCCAATAGGTTTGGCAAGAA
TGTGCTGCATTGCTTTGACTCCTAGTGGATTGTTCTTGCTCTGTACCAACAACATTTAAA
ACACAATCACGTAAGTAAATGAAACAACCATATCCTTCTGAACAAAACTGTTAAAAGATA
AACCTTGGCCTAACTAAAGGGCAGCACGGGGCACTAAGCTCCCGCTATGCGCGGAGT
CCGGAGAAGGGCCGGACTACAAGGACCTATTGTACGCAAACTTACCCTGCATTTTTGC
AAGAGAATGTTTCTAAAGCTCGAACATATGATAGCTACTTTACCAGTTAAACCTTGGCCT
AACTCAAACAAAAATCTAGCTCATGAGATGAGAATTGCCAAAGAACCCATTCCCTTAATC
AATGTGCGACAATCTAACAATAACAAGCAGAGATCACTTACAATTGAGCAGGTGCCTGC
TTTAACATTGCAGACAGGATAATCATGTGGGCAACAACTGTTATGGTCTTTACAGCAAGT
AGCTCCTTCCATGGGACAACAACCCCAAGCAAAACAGTAGTTATAATACTTGTAGACAC
AGCAGCACGTTGTTCCGGCTGGGCATTCGTTATAATCATCACATTGAGTGGGTGGCTTG
ACTGGTGATGGAGGAGATGGAGCTGGTTTTGGGGGGTTTTGGCCTGTCTTTACAGGGT
AAGAAGCAATTGTAGCAATACCACACAAACCTTTGGGGTTGCCAATGTTTCGCTGCATC
CTGAGGTAACCATTTTCTCCCCACGAAGCACCCCATGAGTTCCTCACGATCCAATAATC
CATGCCATTTTCACTACCATATCCTACTGCAACCACACCATGGTCCACTGCTGCACCAC
ATTTTCCGGTAAAGATACCCTGGAGCATAGGCGGATCCAAGATTTAAACTTAATAGGAT
CAACATTTAAATTTTTTAATACTGAACTCATTGTGACTTTGAAAATACAGAAATATTTGTT
GAATCCGTGTAAATACTGGCTAATTCGATCAAAAAGATAACAACTTTATTGACAATAGGC
AAATCTGAAGTAATACCGATTTATAGTGCTGGAAGTCTTTGCCGCCAGCTTCGATAGCA
ACGCTGACGGGTTGACCCGCGACGGCCTTTTTCAGTGCCTTTTCATCATTAGCAGGAA
CATCTTCATACCCGTCGATGGTGACAACCTTGGCATTTTTCTATAATTGTTTCAAACAAA
ATATTAATAGTCATAACTTGTATTTAGTTCATTTCCAATAATCCCTTTTAAGACAGAGAAC
ACCATACTGACCCTTGCTTGATCGCATTTTCCATCTTTGGCTTTGTAGGGGTAGTCTTCT
TCAGTGTCTATTCCTCCATTTTGAATGACGAATTTAAAGGCATCGTCCATTAGACCCCCT
TGGCAGCCTTGGTTATCGGCAGTATCACAATCTACCAGCTCTTGTTCAGATAACGAGAT
CAGATTACCTGTCATTATCTTGTTTACTGCTTCAATTGAAGCAACTGCTGAGAAAGCCCA
GCAACTCCCTATTTCATTTCCCAATTGTAACACAATTAAGACGAAAATCATTAGTTGCTTT
ACGTAATATAAAAATATCATTATTTCTTCAGCATTTATTTATGCATAAAATGGAAGATAAT
CCAATCAATCAAGGCTGCATAATTTGCCAAAACAATTGCACAAAAATACATACTGATGAA
CATAGTGTTTTGTTCAAGGGAAAAAACATTACTTATATTGCCATAATTAGATGCGGAAAT
TGGAGCAAAAGTCCTGCTAACCTAATTTATCATACTTAACAACAACAATAACAAATCCAC
TGAAATCCCATCGTGTGGAATCTCCTTACCCCATCTAGATAAAGTATAGAGACACTTTCT
GACAGACCCCAAAAGATACAAGGTAAAGAGGAAAAAAGGCCCACGAAAGGTAAAAAG
CAGGGAATTAAATAACAATAAACAGCAATACCAACAAAGCAATCAGAAAATTTAAAAATT
AAACATGCTTAACCTTAGCTAAAATTAACAATAATGAAGGGTCAACTGAACTTGCCATTG
 
ATCTTAGGTTAAAACTTATGACACTGTGATCATTTGAATCCCTTACCCTAGCAAACTACT
AAATTTTCCGAATTTCCATCAAAGCCATCAGTAAAGAAAGCATTGACAAATGTTAAACAG
AAAATAAGTATTAACAAAAAGGAAAACAAAAACAAGTAAGAGGTGCAGAGATGAAGAAG
GCAGAGTACGAAAGGAAAATACCACATTGTCCTTGATTTTTGACGTCAACAAGAACACC
TTTCTTCCTCCAGTCAACGGAATCCGGCAAACTATCTCCGACCTTGGGGGCATAACGGT
CACTTTGGGTATACGACAACCTACTACGACCATCGGGCTTAGTACCCAAGTAGATGGAC
TTGTACTCCTCGTTGGTCAAATCTGCAAACTGAGTCAAACCCAGCTTGTAACTTTTTTCA
GGCGCAGAGTTCTGTTCATCGATGTATCTAAGGTTGTCCTTAAAGATCTGGAACCGCTT
GTCCTTTTCTCCTAAGGCGTTATACACTTTTTTATGTTCAACTAGCCAAGATTCATACAAA
GACACGATTTCATCGTCTGTTCGCCAGACCGTTGACTCGCCGTTCGTGTGATGTTTTTC
GTTGTAGCTTATAATGGACATGTCCTCCGCCGATGATGTTACGGCGGAGAACATGAGC
ATTACGAGTATGGAGATGGAGAGGGTGGAAGTATGAATCGCCAT
SEQ 37 
TCAGGAAGGCGAAACAGCAAGAGGATTCAACAAAGTGCCCACGAACAAAACAACACCA
GCAGTCTCATCTTTCACAAGGAACATGAAGGGATGATCAGCAACAAAGTCTATCTCCTC
TTCAACCTTCATCATCGAGCACCCGAACATCATTGTAGCAACAGTAACAGCTGGAGCTA
CTGGAGCCTCCTCATTTACTTCGATAAAGGCCTTGTGAAAAGCTTTTGCAGCTGGAGCT
TCTGCACCTTCCTCATTTACTTCAATAAAGGCCTTGTGAAAAACGTTTGCAACTGCCAGA
GGATAATTCTCGCCCACCATCTCAGTGAGACCACCTTTAAAAGGTAATGTGAGCTCGAG
TCCTTTTAGAACTTCTAAAGCTTCAATCCCCAAAGATATTTTGAACTTAGGGATAAGAAA
CTCGTGCACTTTAACTTTTTCATATGGAACATGGCGATCCAAAAATCCAGGTTCCGAACT
AATTTTCTCCAGTAAAGTTGGTAATCCATCACGGGCATTTGGGAGATACACATACATGTT
GAGAAATCGCTTGTCCTCGCCCTGTTTATAACGAAGCCTTAACACTTGGAAACCATCAA
AGGCCTTCACGTATTGCCTTTTTTTGCTGGTCATTAAGGGTGCTTGAACAGATCCTCCAT
TAAGGAGATGGAACTCATGGTCTTTTGTATCTGAAGCATTCAACTTTTCAGTCCATGCTC
CTTTGAAATATAGTGCATTCGCTAAGATCAGACTTGTACCGCTATTGACTGCAACAGGA
GGAAGAATTTGTTTGATAAGACCATTCGTTTTCTCTTCAGCCCACTTATTGACTTCACCA
GTAACCTCATCACCCTACATAAGAAAAATTAAAACAAACAGAGAACATAGAATCAGCAG
GCTAGTAACAGGATAGAGTTGAAAGTAAAAGAAACAAAAAAGATGTCCTATATGACATTA
GATTCGTTTGTTTGTAATCTTATTCGAAAAGCATAATAAACGTTTCTAATGTGCTTACTAA
TTTGAAAATATTCTGTTTACCAACATGCCCATAATATTTTGTTATACATTATAATACTCCCT
TTGTTTCGTACTTCGAGGGTCAAACTTTTCAATTTTAACCGTGAATTCGAACATGAAATTT
TAATTTTTGACATAAAAGTCACATATTTAGAGACTGTAAAAGTAATATAAGTCATTTATAA
GGAATATAAGAAAAATCGCAGTCAAGAAAAACTCGACTCTCGAAATCCAAAAGGTGTCA
CATAAATTGGGATGGAGGGAGTATCATGTAATTTCAAACATCAATTTCTTTTCTAGTGAG
TGAAGGATTAAGACTAACCTTGTTCCGAAAATCAACAGAAGCCGAAGCAGCCTTATAAA
CATTGTCCATAACCTGTTTGAAAGAATGCTTAAAAGACAAAGATTGGTCAACCCAGGCC
CAATTAGTGACAGACAAACGAGGACCTCCCATGGGGCTGCCATCGGCTAAGACGTCGG
TGATGACCCGAGAATAAACAGAGTTAAGTTCTTCAACAGAGTTGAATTTGAGAAAAGCC
AACAGTTGATCCAATGTGGAACCACTGGAGCCTGCTGCAATAAGAGCAAAAATTATTTG
AATGGAGACCGGGGAAAACACCATGTTTGCGTTGTTGGACTCGTCCTCGTCGGCTTTA
AACTTGCTGAAGAATACATGCTTTGAAAGAATCAATGGAACATCCATCATTTCTGATGAG
GGAAACTAAAATTGAAACAGAGAACGTAAATCCATAAGATGAGGGAGACTGAAACTGAA
ACGGAGAACAGAAATCCATAATTTCTGATGAGGGGAACTGAAACTGAAACTGAAACGGA
GAACGGAAATCCATAATTTCTGACGAGGGAAACTAAGGTACCTTCACAGTTGAACTTGA
CAGCATGGAGAGAATGCTGATGCATATAGAACTAACAGTCATGGTAGGCGACCACGAG
TCATACAGAATATCTGCAAGACAGAAAATAGTTGTCAACTGCTGCTTTTATTAGATGCAT
TCTAACTTTTTTCATGAGTCCGGTAGAAAATCTTAATTATTAAAAAAAAAAACTTATTCTG
 
GAAGATGAGAGGAATAACTGTGAATGAAAGGCGTTTTGCTTTCACTTTATTAGGCAGGT
AACTATAGAAAGTTTAAGAGATTAATAAGCAAGCCATAGGCATAGAGCAGAAACCAAAA
TTATGCAGGAAAAGAAACTAGTATGATCTCTGCAATTCTTAGATGAAATGACAAATTTGA
CGAGACCCACAGACAAACAAAAAACAGATCCAAAGTTTATTCTACTAGGGCTAAGAGTT
CTGAATCAGTAAACAACTGTGTTTTTTAATCATCCGTCAACCATCATTGACTATTACTCCA
TAATTAGATACGAATCAACAAATATTATCGGGACATGAAAGGGGAAAAGAATCAATAACA
AAAACACCAAGACCAAGTAAAAAGCTCACGGATATAGCGAGACAATCTACCATCATACC
CATCATCAACTAAGTTGGCATAGAGCATTTCCAAAACTATAATACCTGTCATCTAATCTTT
ATTAAGAGTTCATATCACAAATCATCCATAATTGCCAAGGATTTTCGTTAAATGCTTCATC
TAATGCATTCTAAATCAAGCCATTAAGCTCGAAGAGTAACCAAATAAATAAATTTCTGTT
GGTGATAAGAAATAGAGGAGACTTGAGCTGTTGAATAAATACCTAAACAAATGTGCCCG
TCCCTATAGATATCAGGGTGCAAAGGAGCTGGAGGCAAAAATATCACCTGCATCAATGT
AGTCAGCCCTAATTAGTATGATAGGAATTTCAATCAAGAACTTTAACAAGAAACCACATC
TTATATCCACCTGGGGAGCTTTAATAGGATAATGTTCAGGGAATTCGGCTTGAAGCTGA
TAAGTTTCATTAGCATACAGCGTCCCAGGAGCACCATTCACTTCAATTAACCACCTTTTC
AAAAATCAAAAGTTCAGTAATAATAGCATGGGACTTCGAATTCAATATTAGAAATTTATTG
GAAAACGACAAAAAACTTAACAACAAACAGGAAAAATGAAGACCTTTGAAGATAATCGG
AGGGTTCAAGATTGAAGCCAGACGGGGGATTGACCTGCCAGTTCCCTAGCTCTGTATG
GAGTCGATTGCATGCCAT
SEQ 38 
CTGAAAGTTGGTTCCTTTTTTCTTCTCTTATTTATTCATGCAATAAAGCATCTCCAAACT
TCTATTCTTATTCATTCTCTCTGCTTTCTTGCTTCATCGAACTGGTGAGTAGTTGTTTTGT
TTCCTTTTTCTTCTATTTAAGAAAAAATTAACTCTCTTTTTGTCGATATATTTTATCCTTTTT
TTTTCTTTTTTCTTTTTTTGTTTCTGTGGGTATTAGAGGTTTTGTGCTTCATTTCATATATT
TGTCTCATGATTTTACTACTTTCAAGGTTGGGCTTTTTCCTTAGCAAGAAAGAACTATTTC
TGTTTATGTTTCATTTTTCTTTTGGACCTTGGTTTTCTGTTCTCGAGGATTGTATCTGTTA
AAAATTGAAGTACTTTTTTTTCCCTTCATCTTTTTAATTGATGTTCTGTTTAGTGTTATTTT
CACCTTTTATGGCATTTAGCAATGTTTGTGCTTTGACGGGTTGCTGTTATAAACATAAAT
TTTGGGAAAATAATTACCAGGTAAACTTGTTATTATGCAAGTGCAATTTGTGTGCGTGTG
GTGGTTTTGTTGCTAGGGAGCAAGGCATGTGATTAGTGATAAGAGGGTTAAAAGGGGA
GTAGATAAACAAAGCTCCACTTTTTAGGCTATTGTTTTTACTTGGGTTCTTCCATTTTTTA
TTATAGCTTGATGAAGTAATATGTAGCTTATAAATTTCCCAGAATAAGAATCATCTCTTGC
CTTAGAAAAAATAATTTACCAGTAAGAGCAGAATATATGGTAGGATTCATCCACTCAACT
CCAATTAGTTTGTGACTGAGGCAAAGTTGATTGAGTGATCGATTGAGTTTAGTCTCATTA
GATTGTCATTTATCCATTAAAAACATGCAGCAGGCATAACATGAGTGATTTGATCTTCTG
AGCATTTTCTCTTGTTTGTTGAATTTAATATATCTTCACTAATTGCTTGGCCTAAATTTTAT
TAACTCAAAGTGATGATTTGCCTAGGTCAATATGGGAGCAAAAGCTTTTCTTGTCACCAT
TTTACTCTCATCGCTGTTATTTCCTTTGGCCTTGTCTACGTCAAATGATGGCTTGGTTAG
AATTGGACTGAAAAAGATAAAATTTGATCAAAACAATCGACTTGCTGCACGCGTCGAGT
CCAAGGAGGGCGAGGCTGTGAGAGCCTCTATTAGGAAGTATAATAACTTCCATGGTAAT
CTTGGGGCCTCTGAGGATACAGACATTGTAGCACTGAAGAACTATATGGATGCTCAGTA
CTTTGGGGAGATTGGTATAGGCTCTCCCCCTCAGAAGTTCACAGTCATCTTTGATACTG
GTAGCTCTAATTTGTGGGTGCCTTCATCAAAGTGCTACTTCTCAGTAAGTTATTTTTTTC
CTTAAAAGAATGCATAATAGAGAAAGCTAGTATTGGCTACATAATTTGATGATCATCAAT
ATTTATGTTTCTCTATGTTTGTGCAGGTTCCCTGTTTTTTCCATTCCAAGTATAAGTCAAG
CCAATCAAGCACTTATAAGAAAAATGGTCTGTTTCTGACCTTTGTCTATATTTGATAATTG
CAACACGACACGTGCTTTTCTCTTATACTTGTTATTTATGCTCAATGCTTGCTTGTAAGA
GAAAGCGTTCCATTATTGGCATTATACATGACATGTCTTAGGTTTTGAGATCAAAACTAT
 
TAACTCTGCTACCAACTTAGGATTTTTTAAAAGAAAATAAAGGAAACCCTCACCATTTT
TATTGTTGTCATCCAATTATGTGCCTTGTATCAAAGTTTTTTGTTGAAAAATATAATTTGG
CAAGTTTATGTTGTTGGCTTTCCCTGCCAAAAATGTGCTAATGTTATCTCTCTGATTTTTT
TTACTCATGATTTGCAATAAAAGCTTGTGCCTTTTAAACTGTTTTGTCTATCAAGGAATCT
GTTATGCTGGAGTTCCTTTATTGAGTTTTGATATCTATCATAATTTACTTTCCTGGAAAAT
TGATGTCTGCTGTGTGTTTGATATGACCTTTGAATATTCTTCTCTGTCGTTGAGTTGGTC
AACGTGTTCAATTGGTTGTTGACCTAAGAACCTGTTCATCCAAACCTTTTTCTGTTTAATA
TGCCATACAGGGAAGTCTGCTGCAATTCGTTATGGTACTGGAGCAATATCTGGATTTTT
CAGTCAAGATAGCGTTAAAGTCGGTGACCTTATTGTGCAAAATCAGGTGAATGTGGCTT
CTCACTTCCTTTTTTTAATTTTTTTTATGTTTCTTGAATATATGGTCTCTCATCTGTCGA
GATTGTTAATGACATCAGGAGTTCATTGAGGCAACAAGAGAACCCAGTGTGACTTTTTT
GGTAGCCAAGTTTGATGGTATATTGGGTCTTGGTTTCCAGGAGATTTCTGTTGGAAATG
CTGTTCCAGTATGGTATGTGGGTTTATTTTGTTTGCGTTCTCTTCTTTCCAAATGTTTCTT
CAATTTCCTATTAACCAAGTGCGTGCCTTGTGAATTTCATTATTATTGAAATGATTTTATC
TTCTGGATTGCAGAATTTCATGAACATTTTCTTCTATATAAAGTTTTAAGTGATACCGGTC
TTGACGGTTTCTTCTGTGTTTTATAGGTACAACATGGTCAAACAGGGTCTTGTCAAGGA
GCCTGTCTTCTCATTTTGGCTCAACCGAAATACAAAGGAAGACGAAGGGGGCGAAATT
GTGTTTGGTGGGGTTGATCCTAACCACTATAAGGGAAAGCACACCTATGTCCCAGTCAC
ACGGAAAGGTTATTGGCAGGTAAATATCCCTATATCTTCGGAAGATTGATGTTTTGCTTT
CTGCAACTGTTTTCTTACTCTTCAGAATATAATATGCAGTTTGACATGGGTGATGTTCTG
ATTGATGGTCAAGCTACTGGTATGTTACGTTACTTCCTTTTCTATTTTTTTGTGTGTGGA
GATTTCGAGGATATTGATGAGAGCACTTTCCCATGATTTCCCTGCTTTTTCGTTGTATTG
ACATACTGAATAATGTAGGTTACTGTGACAATGGATGTTCTGCAATAGCGGATTCTGGG
ACTTCTCTCTTGGCTGGTCCAACGGTATTCTCCAAAGCATATTCCACTTTTTGTCCCTAT
TATTCAGCTATTTTCAATAGTGAACTAGCTCAGAATATTTTTGTACCTTCTTGTTCATGT
GTAGCTTCAACAATCTTCGAGCGATGAATAGGTTTAGTTTTTGGTTGGAATATCAGTTAA
ATAATAATCAGCCATTCCTTTGAACTTTTCTCGTTTTTTCCTTTTCCTATTCAAAAAAAGG
ACGACGGAAGTGCAGTGGAATTGATGTTCATCCCAGTATCAGGACAAACTACCTTGTT
GATTGTCATACCTAAGAAATGTTTTTTTTTAACTTTTGCCTGTTGTTTCTGTCTTATTAAAT
TAATGCAACTTGAGAACTGCTTCTTTCTTCTCATCTTTAAGGCATGGTTGACAAATATGA
TACAAGGAAAAAGCTGCAGCTTTATTTGTCTAGACAATTGCAGTAGTGAAATGCTTTACT
ACTACATTTTCTAGTTCTCATCACTGTATCCTTCCTCCTCTATCTTGCAGACTGTTATCAC
TATGATTAATCATGCCATTGGCGCCTCGGGGGTTGTAAGCCAACAATGCAAAGCTGTTG
TTGAACAGTATGGACAAACAATAATGGATATGCTTTTAGCGGAGGTGAGCAATTAATTAT
TTTAGTTGATAGTTTGTTTTTGTTTTTACCAATAGTTTTCCGTGGTATCTGCAAAGAGGGT
GGTTTCGTGCTACTAGTTGCCTTCCCAATATTCTGATGGATTGGCGTCTTAACAGGCAC
ATCCAAAGAAGATCTGCTCGCAGGTTGGGTTATGCACCTTTGATGGAACTCGTGGCATT
AGGTTAGGCTAATCATTTCTTTCCTAACCTTGGCCAATCATTTGATATGTTAAATCCTATT
ATAAAATGTGTGCTGAGTGGATTTATGTCCTCCACGTGTAGTATGGGCATTGAGAGTGT
TGTAGATGAGAATGCTGGCAAATCTTCAGGACTGCATGATGCTATGTGCTCCGCTTGTG
AAATGGCGGTTGTCTGGATGCAGAACCAACTTAGACAGAACCAGACCCAAGAACGCAT
CTTGAACTATGTGAATGAGGTAAATAGCATCAGTCACATGCTTTCTCTTCTCATCTTAGG
TTAGATTACTGACCATCTTTAACAGCTTTGCGAGCGACTACCAAGCCCAATGGGACAAT
CTGCTGTTGATTGTGGAAAACTTTCTGGCATGCCTAGTGTTTCCTTCACAATTGGTGGC
AGAACATTTGACCTCTCTCCTGAGGAGGTATGTCTGATATCAATCTTCTGTAGTATACAT
GGTGTCTTCTCAACTTGTAAATGGCTTTTGATTCTTCTGAACGACGTGGTTGGTTGTAGA
ATCCTTTTGTCATGTTTCAGTTTGGCAGTTCAATTCTTTTTGGTTTTCACTAGATTAGCTA
GCAAGGTGTTACGCTGCTTTCAAGAGAAGTACACTTGTCTTGTAGAAAATTTCAACCAT
GACAGCTAAGTGTAGTTTGGATAATTAATGATATTGAATGTGTCGAGCTTCAATATCAGT
TTCTTTGCTTGATAAGTTAACTTATGATTGGATAATTAATGTCATTGAAGTGTGTCGAGCT
TTGATATCAGTTTCTTTGCTTGGTAAGTTCATATGATTGTACTAAGCTTGCATGCTTGTCT
 
TGTCACCAGTACATACTCAAGGTGGGCGAGGGTCCTGCTGCACAATGTATTAGTGGCT
TCATTGCCTTGGATGTTCCTCCACCCCGTGGACCTCTCTGGTATGTTTCTTTTCGTCTT
AACACACGTGCAGATTCTGTTATTCTAGAAAAGTTATACCAGCTCCCTTTTGATAATGCT
GTTTGCTTATGGCTTTGGTGGTGCAGGATCTTGGGGGATGTTTTCATGGGTCGATATCA
CACCGTCTTTGATTTTGGCAAACTTAGAGTTGGATTTGCAGAAGCAGCT
SEQ 39 
TCATATGGCTGCAGGTCTTCCATCTTTCCTAGGATCACTTACCGCCACAAGCATCCCAT
GAAAAACCCCGTTTTATATTCTTTCCCGCTCCTTCTTCCCAACTTTAAATGAGAATTTG
GAAGGTTTTGAACAATTAGCTGACAGATGGCTCCTCCGTTATGTGCTTCGAGTTGATGA
CCCCTCTCTTCCAAGAAATGCTTTTTCTCATCCGATAGCTCAATGTGATCGCCATCGATG
CATGTCCAGTTCTCATACAGAACTACATTCGGAATTAGCTGCAAAAAGAGGAACACGAA
TCAATACTCGGGGTTTCAGAATTCGTCCGTATCAAGAAATACGTGAATTGCTCAGTAGT
GCTTCGCCACTACGCTAGCTGGCGGAAATAGCTTAATGGTAGAGCATAGCCTTTCCAA
GGCTGAGGTTGAGGGTTCAAGTCCCTCCGCTCCTGGCTTCGTCGTTTAGTGGTAACAA
GTTCCGTGCATAAGCCACTTTAGAGATAGGTGATCCTTAAAAATACTCCCTCTGTTCCAC
TTTATGTGAGCCTTTTCGGAGCACGAGGTTCAAATTGACCAATTTTCCTTGTGGATTGAG
ACATAGAATTTTCAAAAATTACTACATAAAAAGTACTATAAGTCATACTAATAGTTAACAA
TTCAAAATAAGAAAACTTTGTCTGACTCCCTAAATAGTAATAGATTCACATAAAGTGGAA
CAGAAGGAGTAATATACATTGCTGATCAGGCAAAGGGACCGACCTCGTGGTAGACTCT
TGGACTCTGCACTGCTGCTAAAGGATCCATTCCCAAGATGAAATGGTTGATGAAAACCT
GGACCACCGCGGGGATTATTTCATGCCACCACTACCACCAATTACACCAGCCAACTGA
TTATCCTGTTAACAAGAAGAAAGAATCAATAAAAGATATACTAAACAACAACTACTG
AGGAAGTTCGGATTGTCCATATACTAAGCAGTTAAGAAAGATGACGCATTGCTCAAAGG
ATCGTTTCCTGGATTGGATACCTTGAGAACAATGATTGGAGCCATGGACGACAACGGTC
TCTTTTTTGGTTGAATAAAATTAGCCGGGGCAGGAGGGAGTTCATCAGGGGATATCTCA
CTAGGTGTTGAGAAATCTCCCATTTCGTCGTTGAGTACAATACCAGTTGATGGAGAGAG
CACACCGGCTCCAAATGGATAGTTTACTGTGGTAGTTACTGATACAGCATTTCGATCAG
AATCTACAATACAAAAGTGACTTGTTCCGTGATCTCTTAGCTGACTCCACCTACAAATCA
AGGACCAAAACCGCTTTAGAGCCATTGGTGTGCAATCAATAGGCTTGTTAGTTCTGGGC
TCAGTCTGAAATACTCCTTCTGTCCCAATTTACGTGGCGGTGTTGGATTTCGAGAATCA
AAAAAGTTTTTCTTTGACTGCGATTTTTTCATAAGCCTTTTAAATATTTTGATTTAATTATT
ATTGTGACTTATAGTACTTTTTGCGTAGTTTCCAAAGATTTAAATTTTATTTCAAGACTAA
AAGATTCTATGTCCAAATTCATGGTCAAAGTTAACTTATTTGACTCTCGAAATTCACAAAC
CGCCACATAATTGGGACGGAGGAAGTAGAATTTATCTAACCTGGGCATATAGTATTCAG
GAGGAAAGGTGGTATTGTCGAAAATCTTCTGTCGAATTGCTTTGGCAAAAGATGGGGAA
AGCATGTCTGATACAGTTTTGCTGATATTTACAAAGTCGGGATCACCGAGGTCCATCCG
AAATGCAAACATGTGTTTCATCGCCTCAATTAGTCGATGCAGACCTAAAGAACCTTCTG
CAGCATTATAGCTTTCAAGGATTTTAAGAATCTGCATTTCCCCAATCGCAATGGTTTAGT
CAGACAATTGCGTTGTATTAAGTTTAGACATAATTGAGGAAGATTGGAACCAATACGTCT
ACGTCTGTGTTATTAGAAGGAAAATAGCAGTACAAGCTAAGTTGGTTTATATACATACCA
GAGAAATCCCCAGTGTTCCACTGGACGGAGGTGGCATTCCAACGATGGTGTAGCCCAT
AGCATTAACGGTAACTGCTTCTGGAGTTTCCACTTTGTAATTCCTCAAATCGTCCATTGT
CAAAATTCCACCCGCTTTTTTCACATCTTCGACAAGCTTTTCACCAACCTCTCCATTATA
GAATGCTTCAGGCCCTTGTTCAGCAATAAGCTCTAAGCTGTGGCTAAGTTTTACATTATG
GCAAATATCACCTGCCCGTAACAATTTCCCCTCTGGTGCAATTACTTGTCGTAAACCAG
GATCTTTAAGTATCAACTTTGCTTTTGACGCAATATGATGTGCAAGATATGGAGCAACCA
CGAATCCATCTCTAGCAAGTTTAATCGCTGGTTGAAATAGGGTCTTCCACGGCAACCTG
CCATGTTTTGACCAAGCGGCGTGAAGACCAGCTAACTCACCGGGAACTCCCATGGACA
 
ATGCTCCCTCTAACTTGGATTTTCCATTATTATCATACATGTTCTGCTTGAGGACAAACA
AAACAAATTCAGAACCTGCAACTTCGTGTTGTCTATGTTTATATACTAAATACTAATTCCA
ATTCCCAATAAGAGCAGAACTGCAGTTTTCTCATGTAGACAGCTACAACAGCTATTACTA
CTATGCCTCAATTCCAAGCAAGTTGGATCAGCTACATGAATCCTCACTGTCCATTTCGCT
TCATTAAGCCACAGTTTACTTATGCCGATACAAATTAAAGAATTTAACTTCTATACACTAA
TAACCTAATTGTATTTTACAATATCAGTACTTCAATCCAAAGCAAGTTGGGATCGATTATA
CGAATCCTTAGTGTTCATGTCTCTCCATTTGAGCCAAAGTTTACTTATGTCGATACAAAT
TAAAGAATATAAACGTAATACACTAACAACCTAATTGTCTTTTTACAATATCAGTACTTCA
ATCCAGAGCAAGTTGAGATCCGCAATATGAATCCTCAATGTTCATGTCGCTCCATTTAA
GCCACAATTTACTCATGTAGGTACAAGTTAAAGAATTTTAACTTATATACACTGATAATAT
AATTCTTTTATACAATATCGGTGTATTTAACTTGGTGTAATAGGCAGCGTGTCTTATTTTC
CACATTAGTATTCCCACTATTTATGGACGATTGCATGTAATTTTCGGTTAGGTGACCTGA
TACTGTAAAAATTCATTTCTCCAAATTAAATAGTTATTTGATGATGTTTGGATGAGTTGAA
ACTTGAATGAGAAAAAGCAATGCTAAACGAGTGAATTACGAACCTGTGAAGCAGCTAAA
GGAGCAGTTTCCCTCATATCAATAGCTTGAACTTCTGATGTTGATGAAGATCTAACAACC
ATAAAACCTCCACCGCCAAGTCCGCTGGCCATTGGATTGACAACTCCAAGGCAAAGTG
CTGTGGCAACTGCAGCATCAACAGCATGTCCACCAATTTTAAGCATGGATATACCAATT
TCCGAGCATCGACCATCATCAGCAGCAACAACTGCTTGCTCCGATTCAACAACGTCAGC
ATTTTGCTGCAGTTTTCCATTATATCTCTCAACATCTCCGATTAGCCAAATACCAACGTG
TCCATGGTGTCTAAGGCCTATAACTAAATTAAGATGGAGGAAAAAAAGGAACTTAAAAAT
CAAATGACAAAGAAGTGCAAATTGCAGGTGCTAAAATAATTGTGTAAGCTCGAGCAACT
TCTTGATTCTTATTTCAGAAGATAATTAGACAATCGATGTTAAAGGTAGGAATATACATG
CCAATTCTTTATATTTTTTATAATGGAAATATGCCCGAGAGCTAATGGCGCATTGTTCGA
AACTCAATGGATAGTGGGCCCGCTCCTCTAATTCTCACTTAAAGTAGGATTTTTGTCTAT
GACAATGTTCCACTGCTAATTTTGGTTTAAAAAGTAGGAATGTACATGCCAAATTTTATT
TTTTGATAACCGACAAGCGATTATCAGAAAAGTGCAACCCGGTGCACTAAGCTTCCGCT
ATGCGCGGGGTCCGCAAAAAGGCCCTTGTGGTCTGGCCCTTTCCTGGACCCGTTGCAT
AGCGGGAGCTTAGTGCACTGAGTTGCCTTTTTTGGTAACCGACAAATCCCAGGGTCATT
AGCGTATTGTTCGAAACTCAACGGATAATGGGTCCGCTCCTCTAAATTCTCACTTAAATA
CTAGGATTTTTGTGTATGACAAGGTTTGAACCTGTGAAATGCGCACTCACACATCACAA
GTTGTGCTTTTACCACTAGACCAAAACCCCACAAGCTTGTACATGCCAATTTGCCAGGT
CAAATATTATCCAATTCAAGAAAGCCTGTAAATTTGACAATTTTTAGGCAAAAACCCAGA
ATTTGATTTTTTTTTAAAATATGTCCTTACGATAAAGGGTCAGCAAAATTTTACGCTCTAC
GGTAATTACTCAACAAATTCTTTCACAATTTCAAGATTTATAACATAACACTTCACTTCTC
TTAAGTAAAGCTAAAGAGAGAAATTCCTGTTAGGATCAAAAAGTCACGTGTCATGCGGA
AGCTAGTAACACAAATCTTGAACGACGATAAATCAAACAACAAAAGAGAAATATACCAAA
AGAGACACAAACATTTAACGTGGTTCGGTCAACTGACATACGTCCACGGCGGAGATGA
GCAATCCACTATATATAAAAGAGAGTTCAAAATATCGAGATAACAACCTCACGAAGAGG
CAAACACAAGTGATACACTAACATTTGTCCCGTAAAATTCTCCCCCTAAACACGACTCTC
AAACCTCATATGGCTACATCGTGGATGTTAGAGATAAAGTTCAATCTCTATAAGTTAGGA
TAGAAATCTCTATTAGTTAGGATAGCTATGTTCTGTTAGCTATATTTTAGGATATATGATT
GTTCTATTAGTTACCTTATCTCCCTAGTCTTCTATAGTGTGTTGTAGACTGTTGTATATAT
ATTCAACTATGTACTCAATAGAAAATCATCGAATTCTCTCAACATCATCTCTCATAATGCT
ACTGAATGGGAAAGAAAGATCTCAATTTATAGAAGTTCAAACATTTTTCTACCAGAAAAG
GGACTAGCCAACTATGGAAGCATTATATTTTCCTTCTAGGAAAAGAAAAACTGAATTATG
GTAAATATGTTGTTCTTTCCTCCGTGAAATAGGAAAATCAATTATAGTAAAAAAATCTAGA
CAAACACGTAACAATTCCATAATCATGGTGTTAATTAACTTCATTTTTCATAGCTTTTTAA
AGCCCAATTAACGAAATTCCTACAGAATTCAACTGAATATTCTGTTAACAGAATTGCAAA
TACTAAGAAAACAAAGAAGAAGACAAAAAGTCAAAGGTGAAAAACTCACATGAAATGGC
AGTGAGTGCAAATAAAAAGCAAAGAGCAAAACTCCATTTCTTCCTTCTATTGAAAGTAGC
AGGAGAAGGGTCCAACAATGGAGCTTCTAAATTCTGTTTACTCAT
SEQ 40 
TCAGTTGTGTCCTGTCAAAGGATCTACTTTTATGCTTGTGGCAACAATTGGACTTCTAAC
CACATATTTACCTTCAGTTTCTACCCAGCTCAAAGAACCATAAACCACAATATCATCCAT
AACTAATGGACCTTCTATTCTTAGCTTGTAGCTCAACTTTTCATACTTCTCTTTGAAAACC
AACTTTTCAGGTACAAGATTAACTTTAAATTTACCCATTGTGGTCAATTTTGCTGTGTATA
CTGACATACCATCTCCAATATTAGTCACGGTCCTCTGGAATTCTTGTATCCTTCTAGGAT
CCGACTCGCTGCTGTTCCCATTGAAAAATCCAATGAAAGATGGATAGTTTAAGTCCAAT
GATGGGTTGGAGCAAGTATAAGATGAGGATCTTGTGATGGTTTTTATTTGTTTGGATGT
GAAGTTCAGAGCACAGAGAAGATTGACATAATCTTGTGGTGTCGCATCATAGATAAGTC
CAGGATCTAGTGCCTTGTTTGGATCGATATGGCCAGCTCCCATGGCTAGAGGAGTAGC
AGCAGCATTCTTACTACCTGTTGAGATATAATATAATTAAATGATTAAATATATGCTCTCT
CTAACATATAAGCTTACTTGATTCAACATAGTATCAGAGCATGCAAGAGGTCCTAGGTTC
AAATCTCACCGCCACCAAAAAAGTCATAAAATAATTCCAAGTGTTTGGTCCATGAAAAAA
AATCAAACTTTTAGATGAGATGGTCACACAATTCAATATTACCTATGTCTCGGATGGGAC
TTTGTGTGTTGTCCATCGCATTGGAAGTGGTCATCATGGCAGATCGGATGGCTGCAGG
GCTCCATTCAGGGTGTGCGGCTTTTAGAAGTGCTGCTACACCAGAAGCATGTGGACAT
GACATTGATGTACCAGATATAATATTGAAGTTACTAAAAAGTTTTCCTGAGGTAACATCA
GTCACTGGTGATTGTTGTGGCCATGAAGCTAGTATTAAGGCACCAGGAGCCATGAGAT
CAGGCTTGAGGATACTTGGACAGCTCGGTGACGGTCCTCTTGAGCTATAGGTAGCAAC
TTTTGGTGCTGGTTTAGCACCAATATGTGTCACTCGGAATTCAAGTTTTCCTTTAGGTGC
AGAGTTGCTCTTAATGTACTCTAGAACTTTATCACCCTCTTGTAAGTTCAAGAACACAGC
CGGGAATTCGCTTTGGAGGTAGAATTCCAAATCAGTTATATTAGTTATGAAGACAGCCC
CAGCAACTTTTGAATTTCTCACATTGTACACATGCTCACTGACCGAATCATTCTTGTCAA
GGCAGACAACAATATTGTGTGCACTTTTTTGCAGTTCCTTGTCATCTTGGCATTCAACAT
AGACAATGGAGCTTTCACTTGAACTAGAATTCCCAGGGTAGAGCGATAAGCCAGTGACT
GAAACTCCATTTCCAAGAGTTAATGCGCCAATAAATTCGCGGTCAACTGTGCCAGCTGC
AACAGTTAGCACCCAAGGTGTTCCATTGTGCAAAGTCTCATAATAAGGCCCTTCATTTC
CTGCAGAGGTGGAAACAAATATACCTTTCTCCAATGCAGCAAATGCGGCAATTGCCACA
GGATCTTCGTGTAGTGGAATCGCGTCTATGCCTAATGACAAGGATAAAACATCTACACC
ATCTGTAATTGCTTGATCAATTGCAGCAAGAACATCAGACAAGTATACACCTTCTTCCCA
TAGAGCCTTGTACATAGCCACATGAGCCTTTGGTGCTATGCCAATAGCAGTGCCGGTG
GCATAGCCAAAATAAGATGCACCCTCGACATAACTTCCCGCAGCTGTGGAAGAAGTGT
GAGTTCCATGTCCATCTGTATCTCTAGCAGAATTCATTGAAATGTTAAGATTTGGATTGT
TGGCAAGTAGGCCTTTATTGAAGTAACGAGCGCCAATGATTTTCTTGTTACACAAAGAG
GAATTGAACTCAATGCCACTTTCACATTCTCCTTTCCATCTTGATGGTACTTCACTAATC
CCATAATCACTATAGCTTTTACTCTCTGGCCATATTCCAGTATCAACTAAGCCAATTATG
ATATCTTTACCATAGTCGGACGTTGGCCATACACCAGACTCAGAGTTTAGGCCAAGGAA
TTGGGATGTGTGAGTTGTGTCAATTTTAACTGACATATCCTTAATTGAAGAAACATAACC
TGGAGAATTTTTTATGGCTTCAAATTCAGAAGGAGAAAGACTTGCACTAAAACCATTGAT
GGCATTAGTATAAGCATAGACTAGTTTTGAGGACAAGAATTCTTTGTGATTTGTACTACT
GTCTGATAAAGAAGCAAGTGTTGTCAAGTACCAATTATGATGGCTAGCAAAAGCTTTTG
GCATGGCTGACAAATCCATATGAATGATATATGTTTCTGGCTTTGCTAGTGAAATTATAG
AAATAAAGAAGAAAAGCAACCAAATACACAAGGTAATATGACTTGCCATGTTGAGTAATA
TATTGAAGGAGGATATTTTTTTTAACAT
SEQ 41 
ATGGAATTTACCAAAAACTGGCAACATGTTCTCATTTGTCGCTTTTGTGCTTCATCCTC
TTACATTCCATTCAAGTTCAAGGTAGCTACTTTGATCAAGAATATGGTAAGCAGGTACTG
AGCTCAGCAATACAAGATAAAGATTGGTTAGTATCCATAAGAAGGATAATTCATGAATAC
CCAGAACTCAGATTCCAAGAATATAACACCAGTGCTCTCATTCGTACTGAACTTGATAAA
CTTGGCATTTATTATGAATACCCTTTTGCCAAAACTGGTCTTGTTGCTCTAATTGGCAGC
AGTTCTCCTCCTGTTGTTGCTTTACGAGCTGATATGGATGCCCTTCCTCTCCAGGTTCAT
ACACAATTTTTTACTATCAATCAATTATACCTCAATCGTCAATTAGTTGGGCAGTTATAT
GCAGTTCGGAGCTAGGTTGTTCCCTAAGGGGAATCAACATATAAAGAAGTAAAGACGAA
AAAGCCACGGAGATTCAATATATAGTGTATATACAAAAAAAAAATAAAAAAATTGACCTA
TTTACCCTGTGTAATTTTCGACCCAAAGGGTATCAGTTAACTCCCCTTGGATAAGGTTGC
TCTGCCCCTAGTTATATGAATCTTCTTGTATCTAATTGAGAGGATTCAATATAGTTAAATT
ATTTATGCACCGGTCGTCAACCTAGCACAATCCTCCAACTTTATTTGAATCTGCAACTGG
CTATGCTTTGTGAAGCTTAAATAGGTGTAGTTAGAAAGAAATATTCTTAATAGTGTGCAT
ATTTAGTTATGGAATGTCTCTAACATTATTCTCGAGTGAATATAACCATAGGAGCTTGTT
GAATGGGAGCATAAGAGCAAAGTTACTGGCAAAATGCATGGATGTGGACATGATGCCC
ACACGGCGATGCTTCTTGGCGCTGCTAAGCTGCTGAATGAGCGAAAGGACAAACTTAA
TGTAAGTTTGTTAACCTTACCCACTTCACTAATGCTGATTCATTTGGAATGTAATTTGTGC
TTGTGTGATTCTTTAACAAAAGATTTTTTGCACAATGTTGACCAATGACCAGATTGTCTT
GTTCTCAGAAGTAATAATATTAGGTTTGCGCTATAGTGATTATGCTGATCATTTTATCCG
TTGTGCTTTGACTTCTTATCTAGGTTTGCATGTACACTAGGCCTTTGGAGCTTATTCTAA
AAGGGGGTATTTCTTAAACATAGAGGACTGTAAGAAGATAGATGAAAACATTCTTTAATA
GAGGGGGGTATTAAGTGTACTTTGTCGAGATAATGAAAGAACAGACTCAAAAGGAATAG
ACCAAAAAGGATATCTTTTTGCTTTGTTATCAGATTTAGTTCACTTATTCACATGTCTCCC
TCGGAACAGTCCAAATTTCATAGCAGTGTCGCAAAAAAGGAATAGTTGTGCTGTTTGTT
ATCGATGATGCTTCTTAACTTGGATATGACCATGTTATTCTTTGATTCTTTAAATCTGAAA
CTTGGATCGTCCTTCTGTGGGTGACTAGCAGTGCCTGTGGGTAATCATTTTTGCCTTTT
CCTTAGATGAACATATAAAGTGATTTTGCCCATTGAACATAGTTGTGACCATTCATGATT
CATCAATTGTCTCGATGTGGAGAACCTAGCCCTCTGATCCTCCATGGCTTGCGAGTTCA
CATCCAGATGAAGCAACCAGAGAAACTAATTCAGGCATGACGAGAAATTTTCCGGTCAA
GAGAGAGGATCGATCAGAACCTGTTGAAGGAAATGGTAGATGACGGAGCATTGGCCCA
AATCAATTTCTCTCTGGAACCACGAAAAAGAAGCTGAGAAGACCGATAACTTCTATCTAC
ATTACAATAACAATACATGGCTGCATGTATAGGAAACGAGGAAACCATGAATGTTTTTTT
GAATTCTTTTTTGCTTGACCAATAAAAAGGAATTCAAGACTGAACCACACTTTCTAATTAC
TTGTTAGTCTGTAATTGTCTGACTGATACTATTAGATATTTCTTTTCAACTTTATAAGAATA
CATTTGTCACATGACACTCGTAAAGCACTGTTCGAATTGACTTAATCTGTTTTTGCCCTT
TGTGTGGCATCATTCATTATCTATCCATTCTTGGGGTAGTCTACAATAGAAAGTTGATTT
GTTGCTTGTCTCTATTTTTATTTTTTGAACCCGAAAAGGAACGGTAAGACTTGTTTTCC
AACCTGCGGAGGAGGGAGGAGCTGGTGCATATCATATGATCAACGAAGGGGCTCTAG
GTGATGCAGAAGCTATATTTGGAATGCATGTTGATTTTAAAAGACCTACAGGGAGCATC
GGTACTAGTCCTGGGCCGATTTTAGCTGCTGTTTCCTTCTTTGAGGCAAAAATAGAAGG
AAAAGGTGGGCATGCTGCAGAACCCCATGCTACTGTGGATCCAATACTTGCTGCATCAT
TTGCAGTTGTGGCATTGCAGCAGCTCATCTCAAGAGAAGTAGATCCCCTTCATAGTCAA
GTATGTAGCCTAATCTCAATTAGAAGTATAAATCTTTGGTTTACACACACACAGAGACAC
ACAGACACATAATTATGTAGGTACATATATTCCCTTCAGGAACATTTCTTGTTTTAGAAA
GCAGTATAGCATTTGAGACCTGAAGCCTCATTGACAGTTAAGCTGACTGAGATTGAAAT
TCTCATTTCTGCCTGAAGGTTCTTTCTGTTACTTATGTCAGAGGTGGATCAGCATCAAAC
GTAATTCCGCCTTATGTTGAATTTGGGGGAACTCTGAGGAGTCTTACAACTGAAGGCTT
GCTTCAACTTCAAAAGAGGGTGAAAGAGGTAGGTTGCTTACATGAACCTTTGACTGTTG
TTGACTATCAACATCTGCACACTAGATTGTCTGCCAGATGTCTTCAACATGTAGTTTTCT
GTTAAAAAAATTTAGTGATTTTTTTGAGTGATGTTTAATAGCCTTAAACTGAGCCTTCTTAG
GTACTGAGAGCTACGTAATCAAATTAATAAGATTAAGGGTGAATAATTCTCGAACACGTG
 
TTCACATGAATATAGAAGTCTCAGCTGAATGAATGATATAACTTGTGGTCTGCTTGCAAT
TTTCCCATGAAAATGCCATGTAACTCTAGCATTCATAACTGATCATCTTTCCCTGCTTTG
CTTCTCTTTCTTTGTCAAAATCAATTTTATGCCTGTCCTCAACATAGAAGCTTATCATTTT
TATTATTGAATCCTCTATTTCTATTTCGCATTGTTGAATTAGATGCTAATCGTCTTCAATG
TCAAGTATTGCGGCAAGATCTTACTAATTAATGTGAACAGAACCTAGATTTCTTGTGGCA
ATTTTGTGCATTTGTAACACATATTTACATGGAGCCTGCAGGTAATTGAAGGACAGGCT
GCTGTGCATAGGTGTAAGGCGTACATTGACATGAAAGAGGAGGATTTCCCAGCATATC
CAGCTTGCATAAATGATGAGCGCTTACATCAACATGTAGGGAGGGTTGGCAAACTCCTG
CTTGGTTCCGAGAACATCAAGGAAACTGAAAAGGTTATGGCAGGTGAGGACTTTGCCTT
CTATCAAGAATTGATCCCTGGAGTTATGTTTCAAATTGGAATCAGAAATGAAAAACTGGG
CTCTACCCACGCTCCACACTCCCCTCACTTCTTTCTCGATGAGGATGTCCTGCCAATTG
GAGCAGCGTTGCACACAGCCATAGCAGAGATGTATCTGAATGATTACCAACATCCCATT
GCGGTT
SEQ 42 
TTAGATTTCCTCAACTCGTCTATAAAATAGGACATAGGCGGCCGAGGTTTTGAGCTTGT
CCTGGCTGATGGGATACACATGGCTGTCATCGAAGTCATACCACCGATCAGCACCTTG
CTAGATTATTAGAAGAAAAAAACACAAAGTTAGAATATCTGGATTAAACTGGGAAGACTG
TAAAAGTCTGAATATTTGACCTTCTTGCATATTCTTCATGATGAAGAAATAAAAACAATGA
AGATGCATGACCAAAGTTAAATATATAATAGATGCACATATGTGCAATATGTGATTAATTT
AGATGCAGATGATGCATTGGAACTAAAAAATACATCAAAGGAACACTCACTACGAAATA
GGAAGTTTCTATATTTGCCCTTGGAGGTAGGTTTCAGTGACCGCCCACCACCCACCCTT
CTCCCCAAGTACTGATCTTTAGATGCCAATTTTATCAGGTATAAAAGAATCCTATTTACC
ATAGAATAGAAACAAAACCAAGAAAAAGAAGCAAGGTAATCCAAGTCGCAGCACCACTT
ACATGAACAAACGCAGTGTAGTGACCCCCTCCCATGCTTCCATAATGGTTGCTAATTGC
ATAAAGCATATACCGGTAGGAAGATTTGCCATCTTTGTAGGCCAAATATGAGGATAAAT
CAAGATCATGAGTTGGGAAGTCAACATACGTCTCCAACTTGTTCTTCAGAAACCGGTTG
TACGAGAACCTCTTCAGGTGGATGACCAGAATCTCCGGCAGTCTCCAAAGATCCAACTT
TTTAGTAGCTTGGCGATGCTGCTTGCATGCAGGGCAGTACCTAATGTTGGATATAAGAC
AAAGAAAGTTAGGCAGATCATATTCATACTTCCCAAGCGAATGACACAAAATTAGATGAT
AAGATAGAATACTAGAAAGATTTCATGAAAGAAGTCTTTTCCCCCATGAGTGGCCCCAG
GTAAATAGCTGAATTTTATTATCTCAATTACTGTCTGTAGTTGCTATGAATATACAAGAAA
CAAAGAGCAACACAAAAACTATTTCAGAAGCACAATGTGCAGAAAATCAATAGGTGTTA
CATAAGATCATCAGATGCTGTCATTGTTTCTTTAACATAACTGAACTAGAAGTTGGAAGG
GTACTACTCATGCCATGTTGCAAAGTCAAGGTCCAATTTGGTTGAGGGGAGGCTAATAA
GAATGGTTTATCCACAATAAGCAATCACGACGTGGTGGAAAATATTGACAGAAATGAAA
TAAAATGGTATAATTGGAAAAATACAATTATGTAACTACAAAAGTTGAGGGTTTATAATAT
TAGCATAAACCAAAGAAGAAGCAAAAAGGGAAGCATTACGAGAAATCTCTGAACATTCA
TATGTTATGAATAGTGAATACATAGCTTGCATATGAATGGTGTACAATCACAGAAGTGAG
GGAGTTGCATGCTTACCACATATCTTCTGGCCCTAGAGGCTCTTCCTTCAGAAATGCCT
CAAGACATTTATACAGAGAGACAGATTCTTGTGGTCTTTTGGCAAAAAACCCAGATTTAA
AAACTTCTGGCAGTGAGCTGAAAAGGCCTGTATTGTACTGTTCAAGCATTTTAGGTGAC
CAACTTACAAGTACATTTAACCGTCCAGAGATATCTGTGGACTGTAATGGCTCATTCATT
ACAATCTCGGAGCCTTTAAAGGTTGCCTTATCATCTGATAGGTAAAATTCAAAGTCCATG
TCTAAAGGTTCGGCAGTATCTTCTTCAGCAATGCTTTCTGGAACCCCGTTAACTATTGAG
TTGCCAGGTTCCATGTCTGTGCTGACTTCTGAATCTGTACATACTTCAGTAGCACTTCTA
TCACAGTTAAGAGTAGCACTTCTATCACAGTTAAGATTATCTGCTTGGGCTGTAGTGTG
GACTAAGAATGGTGTAAGTATCTGTAGATAAAGACTACGGATATAAGATCCTGTAAGAA
 
CTCTACTATGCGCGGCAAGCGGAATTCCAAATGTCTTCATATTTGAGGTCAGCTTTCCG
TATATGTAATGCCT
SEQ 43 
CTAGCTAACCTGGTTTTCACTAGCCTTGTAATACAGCATTTTCTGCACATAAAACATCAT
GTATCCTTGCGCAGCTCTCACAATACTCTCGCTCACCTGAGTTATCCAGGCATCGTCAC
ATTTGTACCATTGATTGCTTAACCTCAGATATGTTACGTAATGACCAGCATCAAGTTTAC
CAGTATGGGTGATGACAGCAAACAACTCAAATTCCGAGGACGATTCACAGGACGCATC
TTGCTCGTCCCCGTCAAAGGAGAAGATTCTATTTCCAAATCGACTCCTCAAGATAGATG
AAGAGAGATAAGGCGACATGTCCAAGGAAAAAGGAAACTGTAGGTAGTGATCAACCTT
CCTTGACATTTTCTTAATCACAGAATGCTCAAACCTTTTGATATGGAAGCAAGAAACTAA
AGGCAGTTTTCTTATGGACATCTGTTTAAGAGATTCCTGTCTCACTTGACAATGTTGGCA
GAAGAACTTCTGATCAGAACCCAATTTCTCAGGTCTTGTGAAATGATCTAAGCATCCCAT
CAACGTAGAAATTCGACCGTTTTGGCTAAACTTTCCAGATTCTGCCTCCTTTTTGTGAGT
ATTATGAGACTTCTTTGATGTCATCTTGGCGGAACTCCCCTGGCTCAGTTCCAAGTCCA
AGGAGATGTCTATACATGGATCATATGTAGTAGATGTGAAGCCACAAGCTGTACACATG
ACATCAGACCGCAAGATCCCAGAAAATACTCTATGAGCAATGCAACAGTCTCCGCTGCC
TGCACAAAAGAAAAAAATTAGTCAAGATTAGATTACAGATGACAAAGTGCAATGTGTACT
GCTCCTAATAAGTTACATACAGTAACATAGTATGCACAGCTTAAAGTGCTCCTCAACACA
TTTCATAAAAAGCAAAAGTCCAGTTATGCCATAAAGACAAAACAACAATGGTCCTATGAG
GAATGGCAACCAACAGAATGAGTAGCTACAAGCAATGGAGTACAATTGACTTTTGTAAA
AAAAACCTCAGTAAGAAAAGTATCCGCAAGACACTTTATTGCCTCAAAGGTCTATTATT
CCATTTACACAAAACCATTCAAAATCATAGATCCTTGTATTCATTATTTAACTGCAACAAC
CAGGTGTTAACTAAATTTTGCAAACTCAGGGTAGCCCTGTACTAATCCTCTAATTAAGAG
GAATAAGAATAGGTGTTAACTCTCTCAATTAAAGTACGTGTGACTTAAGCGGAATCAAA
TCAGCATATTTACAGTGGCAGAAATGAATTGTTTGCTAAGGAGTTACCCTACTAATGATT
GCTCTGCTAAAGAAATTTGAGGTACCGAGGAGGTAGACTCACAAGAATAATAAGACACA
CAGGGGCTTATAGAAAAAGTAAGCAAAAAGTTGTCAACCTAGTAAAGACTTCAAGCTT
TCTTGAAGGTTGCGATCAGCTCACTCGAGAGTAATACCCTAAAACATGGTAAAAGTGCG
AGTTAATATGAGAACTATTGTACCAGAAGAAAACTCCTATGCTGAAATCAATAAGACTAA
CCAAACTGAGACTTACTGGAAGAGTAGGAGGAAATCTTTTTCCTTTTGAGAAACATCT
CTAAGCCTGACAGCGAATATCAACTGGTAAATCGCCTATGGCAAAGAAAAGACCTAAAC
CATAACCTGCATTCAAATATTTCTATCTTTTCTCAGTGACAACGGAAGTTGGGATTGCCA
TGAGAGATGAGATGCTAGAACAACAAAGATAAGCCTATCAAGTAGGCCCTGAGTGCATT
TTAGTTAGACGTTACTTGACATCACAAAGATGCTTGAACACACTATTCTACTTCCTACAG
AAATTACTTTTTCCACCCCCTCTCCACCAACAAAAAGTTCAAAAAATTCACCTACTGAA
GTACTTGACCTTTGCAAGTAACTATACAAATTTCAGTAATCCACTTATTTGAGGTTTTTAC
TGCACTAGCCTCCCTTGACTATGTTAGATTTATGTTGCTTTATTAAAATATGCATAAACAA
TGTTGCCAATAATTTTCACAGCACAATAAAAAATATTTAATCTCAATGATTGGTCTAATTC
GGAAGAAAAGGAAAAAAGAAAGAACAAGAAACTAACTATGCAAGGTGATGGGGGGAGA
AAAGATGGGTAACTAATAGATACTCTAACGCAACAAACAAATATACCTGGACTCAACGC
CTTTCCTTTATCGTTCTGCATCCTTTCATGAATCCCATCAAGCACGGAAATGAAAAACTC
ATGAGCATCCTGCTGTTCATAACTTGCAAGATTTGATGCATGCTTCCACCAACTGTTCCA
AAGAAAGGTTTACATCCATCAGAATCAGATACAGAACAGACTGCAATTAGATACTCAAAA
AAAAAAAAAAAAAAAAANNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNCAATATAACAAATTACAAATGTATTATATCACAACCAAGTTACACACC
ACTAGCTAATTTTACTTCAATGAAGGGATAAGGGTGGTACATATAACCCCAACAATAAAA
AATTAATCTTGTCCCTACATCATTTGCAAATAAGGGCACAACCAAGCTAAATAAGGAATC
 
TTTTACCAATATATCATTATCCTTAAAAAAGAATTATAAGTATACAACAACTATGCCTCAA
TTCCAAGCAAATCTGGATCACCTATACGAACTTCTCACCCAAAAAAAAAAAAAGAACGAGT
GCACATTATAAGAAAGCCAAACATGATCTCAACAATCCAAGAAAACATGATCAAATGCA
GACCTGTAGAGGAACTTTGCAGGACTAATAGGGGTCCAATCGCCGGAGAAAACAGCAG
AAAACATTGCATCCAAATCACAAGCTAAACACAGCATTGTTGAGTTCTTATTCCCATTAT
CACTACTACTCCTTGTTATAACACTGTTATTCTTTCGCTGGCAAAAATATCTGTTATGCTT
GTCACTCAGAAAGTAATTCCTCAATGGTGGTGTATGAAGCAATGCTTGAAGCACTGAAT
TCATAAAACACGTGTTTCCAAGATTGTTAAGGCCCCTCAAACCCCATTGTACTTCTGGG
GTTGTTGAGTCATTGCCGAGCTGACTCGGCAACGGACTCGAATTCCCAACGATCAAGA
CCTGCTCTTTCACATCAGGCGTCCACGGCTTATACTCCACGCGCCTCCTCTTGCGCGT
GTTCTCCGGCTGCGGCGGAGGGTCTTGTATTGATCCGATCACGGTAGCCTCCGTCTGC
GCTAGTACTACGGCGGCGTCGAAGTCTCGATCGTATACCTGGTCCCTACACCCGCAGC
AGAACAGCTCGGCCCTGTCGATGTCCACGGCGATGCTGTGCAGCGAGGGATCGGAAG
CGTTCCCCACCGGATGCGACGGCGCGTGCACACGGCAGAATACCGCGGCGCACGTGA
CGCAGGCGTACAACCGCGGCGGCGCCTGTCCGCACGCACCACATCTCACCAGCTCAT
TCGGCGGCTCACGGCGGATGGACGCACGCCCCAATGGCCGGACCTTCACGCAGCCCC
GGAAGTTGAAAAATGGGTTCGACCCAACTCTGGATCGGAGCTCCGACAAATGCTCGCA
TGTACCACTATCGGTATAAACAGGCGACCCGTTTTTATTTTCGGATCCGGGTTGGATCT
TACCGATTGACAAATGGATCTTAAGACTGTTCCTAGACAT
SEQ 44 
TCAAGAACTGCTCTTCCTTCCTCCATTTATGAAGGCCTCAATTGGTTCGGCATGGATGT
GCTTCATTGCCTTCACTCCCAGCGGGTTGTCCTTGCTCTGTATCAACAAATGCAATCAC
GAGCATTGTTTAGAAAGAATGGCGAGCAACATGCTACAACCAGTTAAATTACACTGATA
ATATCAAAATTCTTTGGACGATCAATGTATAGAAATCAAACTTCTCTTTAGAAATAATAAG
TGCGAGTCACTTACTATTGAGCAGGTGCCTGCACGAATATTGCAGACAGGATAGTCGT
GTGGGCAACAACTGTAGTGATCTTCACAGCAAGTGGCTCCTTCAAGAGGGCAACATCC
CCAAGAAAAGCAGGAGTTATAGTATTCAAAGACACAACAGCAAGTGGTACCCTCGGGG
CATTGAGCATAATCATCACACTGAGTAGGTGGCTTGATTGGAGATGGAGGAGAAGGTC
CAGGTTTAGGGGGATTTACGCCTGTCTTGACTGGGTAAGAAGGCTCTATGGCTAATCCA
CACAAACCTTTAGAACTGGCAACGTTACGTTGAACCCGAAGGTAACCTTTCTCTCCCCA
GGAAGCACCCCAAGAGTTCCTGATAATCCAATAATCCATACCATTCTCACTACCATATCC
AACAGCAACCACGCCATGGTCCACTGCAGTTCCACATTTTCCAGTAAAGATACCCTGCA
AAATATATGTATTAGAACCATATAGCTACTAGCCAATAAGAACTTCATTATAATAAGATTT
AAATATAGAATTGTAAGTAGTGATAATTGGTAAATCATAGTTTTTGAATTATAACAGGAAT
CTATTAACTCATGCAGCCAGCGTTATCTTTTGCTACATTCTTTGGTCGAAACAAAACTAT
TATATCTGATCGAAAGGTAACGTATTACTGGAACGACGATGTCTTTCTTTGAATTCTGTG
ACTAACTAAAAGACTTCAGATTTTGCATGTTCACTTGTATTTATTACTTTCAGAAGAAAAA
GATAGGTCAATGCGAATAAAAACATTACCGATACATAGTGTTGAAAGTCGTGGCCACCA
GCTTCAATGGCAATGCTCACAGGTTGATTTGCAACAGCCTTTTGCAGTGCCTTTTCATTA
TTTGCAGGAACATCTTCATACCCATCTATGGTAACAACCTTGGCATTTTTCTGCATGACA
CAAATGTTTCAATAAATAAATATTCAACTCACTACATAAATCAATATATAAAGTGAATTTA
GTCATTTCAAGAATGCAAAATTTACCCTTGACTGGTCACATCTGCCATCACGGCCTGTG
TAAGGGTAGTCTTCCTCAGTGTCAATTCCTCCATTTTTGATGATGAAATCAAAGGCATAG
TCCATAAGACCGCCATTGCAGCCGTCATTGTAGGAAGTATCACAATCCACCAGTTCTTG
CTCCGATAGTGAAATCACATCTCCGGTGACTATCGAGTTCACTGCTTCAACGGAAGCAA
TTGCAGAGAATGCCCAACAGCTCCCTGGTTCATTAACCGATAGATTAATACACAAATATT
GAGCCATACAACATAAATAATGAATTGAGAAATTTGAACTCAGTGATTTATAAAAGAAAC
TGATATGCAGGCATTAATAAAGAATACACGTCAGTGTCACATGGTATTTAGAAGGCCCG
 
TCGGGTCACTTCGTGTAACATTTGAGCACATTAAGAGGTCGTTTGGTAGAATGTGTTAG
AGAAAATAATGCATGCATTAGCTTTGTGTATTAGTAATGCTTTGTTTGATACACTTTTTCA
ACCTATGTATAACGGATACAAGCATTAGTTATACAGTCTATTTGGTATTATCCTATGTATA
GCTAATGCATAGAAAACCATGACATTAGCTATATCGAGGCTATTAATACTTGCATTAGCA
TGGTTAAAGACAAAATTATCCTTAAAGTCCCTTAAGTAAAGAATATGGAGGGCATTTTTG
TAAACAATTAAATATCTAAAAAATTATGCAATGCATTTTAATTTTTAATACACCACACCAAA
CAATGCATAAAAAATAATCTCTGTATAACTAATGCTTGCATTACAAACCCCTGCATTACTA
ATGCACCTTATTTAGCATTATTCTTATACGCCCTACCAAACGACCCCTAAATGTTGTAGC
AACTAGCAAATATTCTAGTGCCAAAACTCAGCATCAATTGGTCAGCATTTTCTATCATTA
TCAAGTTGTGCTGTTAAGCTTTTCACCCTATAATAATTGCTCATGCGTATAGAAAGATGG
GGTCGGGGAATAATGACTAGGAAAAAAGAAAAGATGGGGTCCTGGAATAATATGATTAA
AAGGGAAATTCGGTGCCCTAAGCTCAGGGTATGTGCGAGGTGCGAAGAAGAAGTGGAT
CATAAGGATTTATTGTACGCGACCTTACCCTGCATCTTTCTATACACCAAAAAATGTAAA
GAATTGTTACATATTACTAGGTAAAATTTGATCTATAACAAATAACTCTTCATATATATAAT
ACCGGCATAGGACTATTTAAACAATAAAATAACAAACTGTTACACCCAATTAAATTACAC
TAATACTATAAAAAATTATACTATCAAAGTATAGAATTTAAACTCGAAGGAAATTGTAAAA
ATAGCACGGTATAGCCAGTTTTCGGATTGGTCATTCAAAAATAGTCAGCGTTTATCAAGT
CAATGAAAAATAGCCACTATTTTGCTGCAACAAAGACCGATCCAACATAATATACTGGAG
TTCGGTGCAACTGTGTATGAACTACAACATATTATGCTGGACCGATATACTTTGTTAGCT
CCAGTATATTACTGTAGCACCGGTGCTCCAAACTCCAGTATATTATGTTGGACCGGTAT
ACTTGATGGAACTCCAGTATATTATGCTGGAGTTCTAGTGCGCTTATGCAGATAGAGTT
CCAGCATACTTATCCTGGAACTCCAGTATAATATGTTGGAGTTCAAGTATACTTATGCTG
GAACTCCAGCATAATATACTGGCGTATTTTCTGAGTTTTAAACAGTGTTTTCGCTCAAAT
TTATCTTTACATAAAAAGTGGCTAAATTTCGATTACTTTTAAAATTTGGCTATTTTTGAAC
GACCAGCTATTTTTTATTTTCACACAAGGAAATCATAAAGGTATCCTACGTTTATGCGTA
GACACACAAATTGGCTTTCTACTCATAGAGGACTAGAACTGAACGCCACCATACTATTG
ACATGAGAGTTTATTTAAGTGCAATGAAAGTGTAAAGAACAGAAAAAATATTTAAATCTG
ATATAATAACATAGAAAATAATTCTTGATATCGATTCTAATCTAATAAAATAGGAAATAAT
AAATAACTAGTTATATTTAGAAACAATTTAACTTTAACGTTTTCATTTTATCTATTTTATCA
TTAGAGAGAAACTTTTATAATCACACGAATGTTACCCACAAATCTTTTGCCCTTGACCTT
TTAGGACCACATGATCAAAAGTTTTCTTTTCTTCTTTTTTTTTAAAAACTTTATATCAAGTC
AAATTATATCATTTAAATTGAAACGGGTAGAGTATTTATATATTTTAGCACAATAAGGCAC
GTATGATTTCCTTTGTTTGTCAAGTTCGTAGAAATACTTTCTACATATAAATTAATTATGG
TAGTGGAATACCAATAGCCTATCTCTATATGCTTTAATAACAAATTAAATCAGGAAAATAT
CATCTAAAACGCCGTCTAATTAATTATAGATCCATAACCCAAAAACCGGAGATAAACGAA
AAAGACAAAGCCATTGCTAATTAACTTACCACAGCTTCCTTGATCCTTAACTCCAACAAG
AACACCTTTCTCTCTCCAATCAACTGAGTCCGGCAAGCTATCCCCAACTTTAGGAAGAT
ACCGATCGCTTTTGTTTTTCAACAACCTGCGACGATCACTGGTCTTAGTACCTAAGTACA
TGGACCTGTACTCCTCGTTGGTCAGATCAGCAAATTTGGTTAAACCAAGCTTGTAACTC
TTGTTTGGAACGGAGTTTTGTTCATCGATGTATCTTAAGTTATCTTTAAAGATCTGAAAC
CGCTTGTCTTTTTCGTCTAAGGCGTTGTACGATTTTCCATGTTCGAGTAGCCATGACTC
GTACAAGGACATGACTTCATCGTCCGTTCGAAAGTGTTGGTTTTCGTCGTAGGTTAAGA
TGGACATGTCGGAAGCGGAAGATAAGGTGGAGAAGAAGAAGAAGAGGAGGAGAA
GTAGGGATATGGATATGGTGAGAGTGGAGCTATGAGTTGCCAT
SEQ 45 
TTAGAGTTCATCCTTAGGTGCTGTTGCAGGAGACCCTGTGGGGTTGCTTGATGAAGTCT
TGATGGGGTAGGATGGTTGCATTGCTATACCACACAATCCCTCTTCAGCATCAATCTCG
CGTTGCATCCTAATGTATCCTTTTTCTCCCCATTCAGGTCCCCACGAGTTCCTCACAATC
 
CAGTATTTGGTTCCATCAAGGGTTGTGCCATAGCCCACAATTGCCACACCATGGTCCAA
CTCAGTACCACAGTCTCCGGTGAATACACCCTGCCATATTTACAGTTCGTAAATGTTTAT
ACCTAGTAAAAAACTTTTTAACTTGAGATAAATGGTCTATCATCATTTACCTCAGAGTAGA
ACTGGAAGTCAGAACCTGAAGCTTGTATAGCTACAGAAACAGGCTGGTTGGCTACTGCT
TTAAGTAGGGAATCCTCATCATTAGGAGGAACATCCTCATATCCGTCAATTGATACCACA
GGAGAATTCCTCTGCCAATTCCATAAAATTCATGCACGTGGATTAGAAACAAGACTGGT
TCGATCTGACAGACTGACACCCTACAGATGTAACAGAATCTTACCTTTTGAATATCACAC
TCGCCACCTTCAGCCATGTATGGATAGTTCTCTTCAGTATTGATGCCTCCCTTCTTCTTG
ATGAATTCAAATGCCATGTCCATCAACCCTCCATTGCATCCTTGGTTTTGACTAGTGTCA
CAGTCAACAAGTTCTTGTTCTGATAAAGATACTAACTCATTTGTTTTGATTTGGTTTATCC
CCTCTACTGCAACGACAGTTGAAAATGCCCAGCAACTTCCTATAACAGGCAAAAGGTCA
GTTTCCATCAGCTATAATATTTTGAAAGAACATATCATATGGTTTACCCTTATGTTATTAT
GCTAGAGGTGTAAAGCGGCATAGAATTAATGACATGCTACTCTTTTCTTACCACATTTGC
CTTGGTCTTTGACAGGAGTAACAGCACCCTTCTTCCTCCAGTCAACAGAGGGAGGGAC
ATCTTCCACATTGGCGTACATGAAAGTTCCATTTGCTCGTGAAGCTCCAAGAAAAGAAC
GATGATGCTTAATCTTGGAACCAGCATAATGGTGTCTGAATTCATGGTTAGTCATGTCTG
CAAACTTGTTCAATTTCAACTTATAAGGCTTATCCTTCTTGTTGAAGTTGTGAACATAGTG
TACATTAGCCTTGAACACATTGAACCTCTTGTCTTTCTCATCAAGGCTCCTCGATACAGT
GTGATGGCTTCTCCATCTCTCATACAACTCCCACAATTTTTCCTCAGTTTCCAACTCCTT
CTCGTGGAAATCGAAACTCTCCCCAAGCCTAAGTACCAAAGCCAAAGAGAAAAGAACCA
GAAATAACTTCTTCAT
SEQ 46 
AAAACCAACCTGTGAGACATTAACATCCAACTCTTGGGCAATGAAATGGGCAAGTTCTG
GAATGCGAGGCTTCAACTCTGAGCAGTTGACACTTAGTGACAAGTAAAATGTAATGAGC
CCAACTTTAATTTCCACTGCAAGTCGACATAAAAGATGAATGTGATTACAACCATAAGTC
TTTGTAATGGAATTATCTAATTTCAATAGCCATCATATCTGCACCGAAGCCTAGCTCAAG
TTTGTGAGAATAGATGTAGTGAAGTAGAAAAGGGGACTAATACTTGCAAAACTAAATTG
AAATCTTGAAAAGTTTTACAGCAGATAAAAGTCAAAGCATTTGAGATTATGCAAACCATT
GAAGAGGTACATCAAATTGAAATAATACAAAACAGGGCTATGTTTCAACAATGCAAACA
GGAAATATTAGGCAGGAAAAATTTTGCGATTCTGTCATTACTTTAAGGTCTTGCCACAAA
TTTCTCATGCTTGTCGTTGTCCAGTCACAAAATTCACTAGAAATTTGACAATTGATTACTA
TAACTTAGTGGATGGATTTTCAGATAGTCGGTATATGGTCAATGCATGTTCACTTGGTAT
CAGTTGTCGTAGTCCTTAGAAATAACTTTTTGGTCCCTTGATTATACCATATTTGTACTTT
AGATCCCTCAACTATTCAGCTTTACACATTAAGCCTACAATTTAACGAACTTTACAGATG
TAGTCCAATAATAAACAAAACTAACTAACCCACGACATATTCATATTTCAAGTCTCCTTTT
TTAATAATACAAATTTCAAAAGGAGCATTAATGTTGTAAAAGTACCGTTGCCTACTAAAAT
ATCCCAAAAAGATGAACACGCTGCTTTGGAAATGGAACGCACCAATCACACGAGTTGC
GGATTAACAAAATCTAAAAATTCTAGTTCTAATTAAGATCTGACTAAATCTGCAAACTCGA
CAAATTAAAAGGCAGATGTCAAAGTCGGAGAGTTGGACTAAAAGTGGAAACAGGGGGA
TAACAGGGGACCAAAGATTTTCTCAGTATTCCTTGAAGTTATTATAATAAATTTCCAGTTT
AAGTAAATCTTTCTCAAACTACAAGAAGGCTGAGAATGCTGTGACATCAGTACAATTTTA
TGCAGGCAGTTGCTTCTTAGAAATTTTAAAATACCAGGGAAACAAGATGATTACAAGACT
AATTTCAGAGAAAGGTCAGATGTCAACTTGAATAGGATTATAAACAGGGATCTTTACACA
AATAGCCGGCTATATTCATGTTTACTTTTTCTAGCCATATACACAGATTATACATTGATGA
TACACAATTATGCACATATAATACATAAATTATGCATTCACACAAATACCAGCATTCTGGA
CATAAGAGACAGAATGTTGATTGCCCAAAAATGATCTAATCGAAGGCAACACATCAAAA
TCAGCATGATGCAATTCTAATTTTGATTCTCATTTTCATAAAAGAAACCACAAACCATTAT
AGTTCAAAATTGAAGGGAAAATTGAAAAGGGAATTGTATTAATCTATTAGAAAACAGAGC
 
TAGTAGAGATGCGAAAAATGAAACCAGTTCAGGTTATGACAGCATTCTAATGGGCAGAG
TAACCTTACAGTAAAAATACTATGTGAAGAAAAGCTGTCCCTTCATACCAGGTGTGTTGT
ACCCAGGGGGTCCACTAGGAGCTGATGAAGGAGACAGGTGCGCACTGGAGTTTGTGT
TATCCAAGCTTGAGACCGATGGTGAAGGTGGAGATGGAGGGGATAAATTGAGTCTGTC
CCATAACTCAGAACAATTAGTTTTCCAAAAACCAATTCTTTTATTTTCACGATCGTAAGTA
ACAAGAGTGTTGCGAACAACGATTCCTGTTGCAATTAAAAGCACGCATGTTACAGCACC
GAAGACCTAGGACGATGAACTTTGTCAAGTAGTTAGAAATACCTCCAAGAAGACTAGCT
GGATTCTTTCCATTCGGGAAAATTCCTAGGCAATAAGCACCACGTACTTTGAAGTGCTTT
ATAGCATACAAGTTACAATGAAGAAGGGGGAAATTAATCAATACTAAATCTTATAAAGAA
AAAGAAGCCAGTAGATGTGACGCCAAAATAGTGTCGGCAGATAGTTAAGAAAGAACTAA
AACAGAATAAAATGCCTACCTGAAACAAGTAATTTTCAGGAGAGAGAGTTAGTTTCTTTC
CATCGCTGAATACCATATCGACACGCGGAAAGTTCTTTGAGAGTTCTGATATGTTGCTG
GAATATATAAGACATTAAATAATAAGGGTAACACCAACAATAGGAAAAAAAAACAAATTT
AGACAAGAAAATCATAGACGTCTAATTAATTTTGGAACTCTCCTTATAAATACCTTCCAG
CACCAGAAAAGCAGATATCTTTAAAACTAGGATCTGGCCCTTCAATCTGTTTTAAAGAAT
GAAGCTCTTTCACTACCTGAAAAGAAAAGTTGAAGAGGTTAATCCCAAAAAGGAAAATA
CTTAAGTTTATCTAAAGATAATCGCCACAAGTTACATACTGTATAAATTCACAACCAGTG
ACTCCACTTTTCCTTCGCTAAATGTAACATAGCAAAAGCAATGAAAGACAACATCAAAGA
TATGCAGAAACTTGCAAGGAACTCTGTATATGTAGATAATTACTACCTGCTAACATCATT
GGAAGAAAATGTTGTGTGGTGTATTACCAAATTCAGAAGTTTAAAACAGGAAAAATCAAC
TATTATAAGTGGTTAAGCAATCTAATTAGGTCCTTTAATAGCAGAGAAACTAACAACAAA
AGAAGGGATAGAGGCCATAATATAGCCTTCCTTTCTTTTGATAAAGTACATAGCCTCCAA
CTCATCACAGGAATAAGTTAGGTGGCAGAGTTATAGGATCACAAGATACCTGGTTCTTA
CTTTTCTTCAAAAACATGTTCTAGATATCAAAGTGTTAGCTCTCAAACTATTATTGACCAA
GATGTTTTAGTTTCAGTCCCACTCATGTACAGGAATCTCCAACTATATATTTGATCCACG
TCACAATAGGACATGATGGTTTGTTTCACCAATCAGTGGTGTAAATAGAACTTTGAATGG
CAAGAAAAATTTAACAGATTGAGTTACATATAGAAAACACATTATTCAAATGAATATCTTT
TCACATCTAATTTCTTCTAGGACATTCTGCTCATCAATAGCGTGCTATTCGCCACTAGTC
ACTTTCTCAGACAGAACGAATCAAACAAACAGACAGAAACATTGGCAGATATGTAGATA
AACTATATCTTACAGCATTCTTGAAAGCTGCAAATGCTGCTTCTGGAAGGTACGCATAG
GTGGTACCACTATCAAGTATAGTCCCATGTTTTCCACCAAAAACCCGTGGATTTAGGTTT
AGCGGCTTCCCAGCGACATGTATCTCCTTCAGGTCAATATTGTAGTACGGGCTGTTGCC
ATCCGAGACTAAAGTAAATGCGTAAGATTTATCAAGTGTATTTGCTAGGAGGAGACCTA
ACATCACTGTGTCTCTAAACAATTGTTTCCAATACATACCTGTGACCAAAATCTGATTTG
GTAAAGGCCATGTCAGCAGGGGGTTTTACTCCACCAAGAACCATTGCCCCGCCACCAA
AATCCATCCCTCCATAGCACAAGGAGAAAGAATCACTAATTACATGTTTTTCAACAAGTT
GATCAACTATACTAAGATCACCTCGGCCCAAACCCATTATACCATCAGCACGTTGGCTG
TAAAGATCACCAGTTTCCGCAATTTCACATCCAAAAACAGCTCGTTGTGGTGCAAGCTC
ACTTAGATTTCCAAAAGATATGATGTCCTCTCCAAGCAACCCATAACTTGCACTCATCTC
AGCGTACCGTCTCTCATAAATACATTGCTGCCTCTTATGGTCGCAGGGACAAGCCTTAT
TGCATTTCACAGATTGATAAGTGCTTGACATTTCCGGCTGAAACTTAGGATCCTAAATAA
GACAACATACAGAGTACCACCGATCAAAAGACAAAAATCAAATGCCAAGCAATTCTAAG
TGCTATATTAAGCATTTCTCACAAAATTAATAGGCTGACTCAAAGTAAAATTACTAGTCAT
GAAGTTTCTTAAGTGCTGATATTTTCTCCATTAGGAGATCTTTTATGATTACAAGGCAAAT
TGAGCAAGAATCACATTTAAAACATCATGAAACTATACAATGGATTTGTACAGCTTATCA
ACAAAGAGAGGCTTAGAACTATTTGTATCCTAGTTAGATTGGTTTGTTCTTGTTTACCCT
CTTTCCCTTAATACTTACAAATAACTGCATCCTACTAAGCGATTTCCTCACAAACAAAAA
GTATATTAAGTAATGTTTATGAAATAGCACCTATACATAAACAGTTTCAAATTTTAATTTC
CATATCAAGCTATCAAAACACACTAACTGCAAAATTAAGATAAATATGTAACCTTTAATGT
TTATCCAGAAAAAGAAAAGAGGGAAAAAACCTGATGGTTGCCACACTTTTTACACTCAG
AGCAAGGGACATAGGTAACTGTACTCCCTGTATCAACAATAAGAGCGAACTTCTGCGGT
 
GGTGTTCCAATCCAAATATGAGTTGTATAGTATCTGCATCACAAGTTACTTGGAATCCAT
TCAACAAAATAAAAATTACTAATAAAGAAATTAGTCTGAACTAGAGGTATATAGAAATATA
TTCCACAGTGAAAACGCAAAATACGCATGTGAATCAGCAGCCAAAAGAGTTAGTAACAG
TGAATTTAAATTTTCTGAGCAAAAGCTACGAATTTGAACCCGTTGAGGAGGAGATCATCA
TGGAGAGACATGCGAGCGCTGGCAGGACTTTTCTGGAGGTGGCGACGGGAGATTTCC
GCACGGCGTGAAGTGTCTTTCGGAGGAAAGAGCGGCAGCAGCATGGTTGTGTGACGG
CTGCCGTCGGCCGGCGAAGGGAGGAAAACGGAGCTGCCGTTAGTAACATCAGATAAT
CGGAAGCCGGAAACGACACCGTAATGGATCAACAGAGAGATGATCGCGAGAATAACGG
TGAACTGTGGCCGTGCCAT
SEQ 46 
TCAATATTGTAGTACGGGCTGTTGCCATCCGAGACTAAAGTAAATGCGTAAGATTTATCA
AGTGTATTTGCTAGGAGGAGACCTAACATCACTGTGTCTCTAAACAATTGTTTCCAATAC
ATACCTGTGACCAAAATCTGATTTGGTAAAGGCCATGTCAGCAGGGGGTTTTACTCCAC
CAAGAACCATTGCCCCGCCACCAAAATCCATCCCTCCATAGCACAAGGAGAAAGAATCA
CTAATTACATGTTTTTCAACAAGTTGATCAACTATACTAAGATCACCTCGGCCCAAACCC
ATTATACCATCAGCACGTTGGCTGTAAAGATCACCAGTTTCCGCAATTTCACATCCAAAA
ACAGCTCGTTGTGGTGCAAGCTCACTTAGATTTCCAAAAGATATGATGTCCTCTCCAAG
CAACCCATAACTTGCACTCATCTCAGCGTACCGTCTCTCATAAATACATTGCTGCCTCTT
ATGGTCGCAGGGACAAGCCTTATTGCATTTCACAGATTGATAAGTGCTTGACATTTCCG
GCTGAAACTTAGGATCCTAAATAAGACAACATACAGAGTACCACCGATCAAAAGACAAA
AATCAAATGCCAAGCAATTCTAAGTGCTATATTAAGCATTTCTCACAAAATTAATAGGCT
GACTCAAAGTAAAATTACTAGTCATGAAGTTTCTTAAGTGCTGATATTTTCTCCATTAGG
AGATCTTTTATGATTACAAGGCAAATTGAGCAAGAATCACATTTAAAACATCATGAAACT
ATACAATGGATTTGTACAGCTTATCAACAAAGAGAGGCTTAGAACTATTTGTATCCTAGT
TAGATTGGTTTGTTCTTGTTTACCCTCTTTCCCTTAATACTTACAAATAACTGCATCCTAC
TAAGCGATTTCCTCACAAACAAAAAGTATATTAAGTAATGTTTATGAAATAGCACCTATAC
ATAAACAGTTTCAAATTTTAATTTCCATATCAAGCTATCAAAACACACTAACTGCAAAATT
AAGATAAATATGTAACCTTTAATGTTTATCCAGAAAAAGAAAAGAGGAAAAAACCTGAT
GGTTGCCACACTTTTTACACTCAGAGCAAGGGACATAGGTAACTGTACTCCCTGTATCA
ACAATAAGAGCGAACTTCTGCGGTGGTGTTCCAATCCAAATATGAGTTGTATAGTATCT
GCATCACAAGTTACTTGGAATCCATTCAACAAAATAAAAATTACTAATAAAGAAATTAGTC
TGAACTAGAGGTATATAGAAATATATTCCACAGTGAAAACGCAAAATACGCATGTGAATC
AGCAGCCAAAAGAGTTAGTAACAGTGAATTTAAATTTTCTGAGCAAAAGCTACGAATTTG
AACCCGTTGAGGAGGAGATCATCATGGAGAGACATGCGAGCGCTGGCAGGACTTTTCT
GGAGGTGGCGACGGGAGATTTCCGCACGGCGTGAAGTGTCTTTCGGAGGAAAGAGCG
GCAGCAGCATGGTTGTGTGACGGCTGCCGTCGGCCGGCGAAGGGAGGAAAACGGAG
CTGCCGTTAGTAACATCAGATAATCGGAAGCCGGAAACGACACCGTAATGGATCAACA
GAGAGATGATCGCGAGAATAACGGTGAACTGTGGCCGTGCCAT
SEQ 47 
ATGGGAGCAAAATCTTTTCTTGTCGCCTTTTTCCTTTCATTGCTGTTATTTCCTTTGGCCT
TCTGTACATCAAATGATGGCTTGGTTAGAATTGGTTTAAAAAAGATAAAATTCGATCAAA
ACAACCGACTTGCTGCACGCGTCGAGTCCAAGGAGGGGGAGGCTTTGAGGGCCTCTT
TTAGGAAGTATAATAATCTCCGTGGTAATCTTGGGGCCTCTGAGGATACAGACATTGTA
GCACTGAAGAATTATATGGATGCTCAGTACTTTGGGGAGATTGGTATAGGCAGTCCCCC
TCAGAAGTTCACTGTCATCTTTGATACTGGTAGCTCTAATTTGTGGGTGCCTTCATCAAA
 
GTGCTACTTCTCAGTAAGCTTTCTATTACATTTTACTGTCATAAAACATAACAGAGAAAG
CTAATGTTGGCGTATGCATAATTGACGAGCATCCATATTTATGCGTCTCTGTATTTATGC
AGGTTCCATGCCTTTTCCATTCTAAGTACAAGTCAAGCCAATCAAGCACTTATAAGAAAA
ATGGTTTGTGTCTTGACCTTTGTCTATAGCTGAAATTGCTGCATGAAAACATGCTTTTCT
CTTAAACTTGTTATTACGCTCAATGCTTGCTTGTAAGAGAAAGTGTTCAATTATTGCGTTT
TGAGATCAAAACTGTTAACCCTGCTCCCAACTTAGGAGATTTAAAAAAAAAAAAGAAAAT
AAAGAAGACCCTTACCATTCTTATTGTTGTCATCCAATTATGTGCCTTGCACCAAAGATT
TCTGTTGAAAAATATAACATGCGAGATTATGTTGTTGGCTTTCCCTCCCAAAAGATGTGC
TAATGTTATATCTCTGATTTTTTCTTTCAATTATTGGCAATAAAAGCTTGTGCCTTTTGAA
CCGTTTTGTCTATCGAGGAACCTGTTATGGTGGAGTTCCTTTATTGAGTTTTGGTATCCA
TCATAATTTACTTTCCGGGAAAATTGGAGTCTGCTGTGTGATTGACATGACATGATTTTT
GATTATTCTTCTCTGTCTGCTTTCTAAGTTTCTACATTCTCGGTAGAGGTAAGATATGCG
TACTATCTACCCTCCCCGGACCCCACTTATGGACTAGGTTTTTTTGTTGTTGTTGTCG
TCATCTACTTTCTAAGTTGGTCAACGTGTTCACTTGGTTGTTGACATAAGAACCTGTTCA
TTCAAACTTTTTTCCTGTTTAATATGCCATACAGGGAAGTCTGCTGCCATACGTTATGGT
ACTGGAGCAATATCTGGATTTTTCAGTCAAGATAGCGTTAAAGTTGGTGATCTGGTTGT
GAAAAATCAGGTGAATGTGGCTTCCCACTTTGTGTGTGTGTGTGTGTGTGTGTTTTAAA
ATGTTTCTCGAGCATATAGTCTCTCATCTTGTTAATGACATCAGGAGTTCATCGAGGCAA
CCAGAGAACCCAGTGTAACTTTTTGGTAGCCAAGTTTGATGGTATATTGGGTCTTGGT
TTCCAGGAGATTTCTGTTGGAAATGCTGTACCAGTATGGTATGTGGGTTTATTTTGTTTG
TGTTCTCTTCTTTCCAAATGTTTCTTCAATTTCCTATTATCCAAGTGCGTGCCTTGTGAAT
TTCATTATTACATTGAAATGATTTTATCTTCTGGACAGAATTTCATTAACATCTCCTTCTG
TATAAAGGTTTAAGTGATACTGGTCTTGACAGTTTCTTCTGTGTTTTATAGGTACAACAT
GGTCAAACAGGGTCTTGTCAAGGAGCCTGTCTTCTCATTTTGGCTCAACCGAAATACAG
AGGAAGATGAAGGGGGCGAAATTGTGTTTGGTGGGGTTGATCCTAACCACTATAAGGG
AAAGCACACTTATGTCCCAGTCACACGGAAAGGTTATTGGCAGGTAGATATCCCTATAT
CTTTGGGAGATTGATGTTTGGCTTTTGCAACCGTTTTCTTACTCTCAGAATATAATTTGC
AGTTTGACATGGGTGATGTTCTGATTGATGGTCAAGCTACTGGTATGTTATGTTACTTCC
TTTTCTATTTTTTTGTGTGGAGATTTCGAGGATAAGATGAGAGCACTTTCACATGATTTC
CATGCTTTTTCGTTGTATTGACATACTGAATACTGTAGGTTACTGTGACAATGGATGTTC
TGCAATAGCGGATTCTGGGACTTCTCTCTTGGCTGGTCCAACGGTATTCTCAAAAACAT
GTTCCATTTTTTGTTCCTCTTATTCAGCTATTATCAATAATGAACTGTCTCATAATTTTTTT
TGTACCGTCCTGTTCATGTGTAGGTTTAATTTTTTCGCTGGAATATGAGTTGAATAATAA
TCAGCCATTCATTTGAAGTATTCTCATTTTTTCCGTTTCTATTCAAAAAAAAAGGAGGATG
GCAAGTGCAGTGATATTGATATTCATTCCAGTATCTGGACATACTTCCTTGTTGATTTTC
ATACCTAAGAAATGTTTCTTTTTACTTTTGATCTGTTGTTTCTGTCTTCTTTGTGTGCTCTT
CTTCTTTATTAGGAAAAAAATTGTGCATCTTGAGAACTGCTTCTTAATTGTTTTCTTTTAT
GGCATGGTTGACAATATGATACAAGGAAAAACTGCAGCTTCTTTTGTCTAGACAATTGTA
GTAGTGAAATGCTTTACTACTACATTTCTAGTTCTCATCATTCTTCCCTGTATCCTTCCTC
CTCTATCTTGCAGACTGTAGTCACTATGATTAATCATGCCATTGGCGCCTCGGGGGTTG
TAAGCCAACAATGTAAAGCTGTTGTTGAACAGTATGGACAAACAATAATGGATATGCTTT
TAGCAGAGGTGAGCAATTATTTGTTTTAGTTGATAGTTTTTTGTTGTTTTTACCAATAGTT
TTCCGTGGTATCTGCAAAGAGGATGGTTTCATGCTACTAGTTGCCTTCCCAATATTCTGA
TGCATTGGCGTCTTAACAGGCACATCCAAAGAAGATCTGCTCACAGGTTGGGTTATGCA
CCTTTGATGGAACTCGTGGCGTTAGGTTAGGCTTCAGACCCTTTCTTTCCTCGCCTTGG
CCAATCATTTGATATGGTAAATCCTATTATAAAATGTGTGCTGAGTGGATTTATGTCCTC
CACGTGTAGTATGGGCATTGAGAGTGTTGTGGATGAGAATGCTGGCAAATCTTCAGGA
CTGCATGATGCTATGTGCTCCGCTTGTGAAATGGCGGTTGTCTGGATGCAGAACCAACT
TAGACAGAACCAGACCCAAGAACGCATCTTGAACTATGTGAATGAGGTAAATAGCATCA
GTCACATGCTTTCTCTTCTCATCTTAAGTTAGATTACTGACCATCTTTAACAGCTTTGCG
AGCGACTACCAAGCCCAATGGGACAATCAGCTGTTGATTGTGGAAAGCTTTCTGGCAT
 
GCCTAGTGTTTCCTTCACAATTGGTGGCAGAACATTTGACCTCTCTCCTGAGGAGGTAT
GTCTGATATCAATCTTGCGTAGTGTACATGGCGTCTTCTCATTTTGTAAATGGCTTTGAT
TTTTCTGAACAAAGTGATTGGTTGTAGAATCCTTTTGTCATGTTTCAGTTAGGCAGTTCA
TTTCTTTGTGGTTTTCACTAGATTAGCTAGCAAGGTGTTACTCTGCTTTCAAGAGAAGTA
CACTTGTCTTTTAGAAAATTTCAACCATGACAGCTAAGTGTCGTTTGGATAATTAATGAT
ATTGAAGCGTGTCGAGCTTTAATATCAGTTTCTTTGCTTGATAAGTTAACTTGTGATCGG
ATAATTAATGTTATTGAAGTGTGTCGAGCTTTGATATCAGTTTCTTTGCTTGATAAGTTCA
TATGATTGTACTAAGCTTGCATGCTTTTCTTGTCACCAGTACATACTCAAGGTGGGCGA
GGGTCCTGCTGCACAATGTATTAGTGGCTTCATTGCCTTGGATGTTCCTCCACCCCGTG
GACCTCTCTGGTATGTTTTCTTTTCGTCTTAACGCACAAATGCGTGGATTCTGTTATTAC
CAGCTCCCTTTTGATAATGTTGTTTGCTTATGGCTTTGGTGGTGCAGGATCTTGGGGGA
TGTTTTCATGGGTCGATATCACACCGTCTTTGATTCTGGAAAACTTAGAGTTGGATTTGC
AGAAGCAGCT
SEQ 48 
ATGGTTGTTGCATTTGTGGGCATAGCCAAGTCTATCGGGCAACAATGCTTGAGGCGATC
AAAACCCTACTCTTACTCTTACTTCTCCAGCTATGTTCGTTCCTCAAATTCTAAGTATGG
ACTCCAAAATTGGCAATTTCAGAGTCATAGAACTCTAATTTACAATCGGCTTCTGAATC
CGTCAAATTAGAAAGACTCTCCGATTCCGATTCCGGTAATTCCACTCACTTTGTCCTATT
TTACGGCGTACTGTTACTATTTGGGGAATCAAACTTCTTTTAATTTTGGGTACAATTGCT
TTCTGGGGTTAATTAACAGGGATTTTGGAGGTTAAATTGGATAGGCCCGAAGCGAGAAA
TGCAATAGGGAAGGATATGTTAAGAGGATTACAGCAAGCGTTTGAAGCCGTGAGTAATG
AACGTTCAGCAAATGTTTTGATGATCTGCAGTTCGGTTCCCAAAGTGTTTTGTGCTGGA
GCTGATTTGAAGGTATAACAGCTTCTCTTCATGTTGTGTTTTTAGGAAAAAATGAGGCAA
AAAAAAAACTTTTGAAATCTTGTGCAGAGTATAGGACACACTATTTGGTTAACAAAAAT
GTGTAATCAAAGGGTTCGAAGTCAGATTAAATAACAAAAATTATGGGTAAATATTAAAAA
TTTTAAAACTTTTAAAAAATCATACTCGTTCAAATTATATTAAATTTTTTCATTTCTCCTCTT
TGCTTCTTCTTCTCCTTCTTTCTTCGTTCTCCTCCTTAATTTCTTATTTCTCTTCAACTTTC
GTTGTTGTTGCTGCTGGTTCGTCCATGTCATCTTCTTCTTCTTCATTTTATTTTTCCATCT
TCGTCTCATTTTCATCTTTCTTCTTTTTTTCATTTTTTCTTTTCTTTCTAGTGGTTTAAAATA
TACGAGAAAAGAGAAAAATATGAAATTTTACAAAGTAAGTATTTTGCAAAATACCCTCG
AGATATTTTGAGACATACCCATAAATGAGTATTCTACTGAAACATCCCCGGGTTGTTGTT
TGAAACATCCTACGGGATATTTCTTCTACTTCTTTCTTTTTTTCTAGTGTTGTTCTCATTG
ATCTTTTTTTCACAGATGTTTCAAACGTTCCCACAGATGTGTGCAGATGTTTGAAACATT
TCAATAAATGTTTGAAACATTTCTTTAGATATTTGAAACATCTTTCCTAAATGTTTGAGGA
TTAGGAGTGGCGGGGATAGAGAGAGGAGCGTTGCGAGATGGAGTAACAAGGGGAGGA
GGAGGAGTGGCGGGAGGAGGAGGAGGGTTTTTTTTTTTGTAAATAAGAAAACTTTGGG
GGTTTTAAAAATAGTGTCATTAACCCTAATCACAGAAGTGTCCCTTTTACCCCATTCTTA
ACACTTTTGTCTTAAAAAGTGATATAAGTTTTGAAGTGTCTTAAAAATTTAAATGCCCCGT
GTTTTTAGTTGTAATATGTTTTTTCTTGCAAGCAGTATCACAACTTGTAGATATTACATCT
TCCTTTTCGTTATGTATCCTCTTCTCTTCATTGTGCCAACCATTGTATTTGTGTTTTCAAG
CAAGAGACAGATCCAAGATATGAACTTTATGTGTTCGAGTTCTAAATTCTGCCTAGTCCA
TTTGATTTACGGGGTTTGAAATCTATATTTGTACAAGTTTTGGTGAGTTTTTTAACACATA
TATGTTTCGTTGATTCTCTCTGTAATGTCTTGTTGTCTTCTAGTATATTTTTCTTTTGTATC
GCGTGACATGTTGACAAGAAAAACAATAAAATTTCACAAGCTAGGAGCATCTGCCAAAC
ATACAACTTTCAATGTTGAAAAGTTTCTCTGTGGTTTGATCTCTTCGCTTGTGCAGCCTC
TGTGGGACTCAAACATCTTCACAGATTCAATTTTCTTTTGTGGGGACTGCATTTATGATT
GGCATGGCATATGATTGAATAGCTGATGTTTTTGGTGTCAGATGTTGATTTACGCACTTT
AGTAGTCTTTCTACTGTTTGTTATCTGTGCTGTATTTTGTACCTACTGATAGCTAATTCAA
 
TTGATTGGCATTTGCTACATAGGAACGAAAACTATGATTCTTTCTGAAGTCCAGGATTT
TGTAAGCACTTTGAGATCAACTTTTTCCTTTTTGGAGGTACGTGATTTTTATTGATGTTTT
GTTTAATATATTAAAGATCATAGTGTCTTAAAGCTCAAGAAGAAGTTTTTTTATCAATTT
ATGAAAAGCAGAAGTTATCAGTTTATAATCTTCTGAATTCTTCCTTCAAAATGATTGGTTC
ATGAGATCATACTATGTCTCGTTTCTTCTTCCTCACTTATTACTTTATCAACCATAAAGTG
GTCCAGTTGTACATATCCTCTTCTTTTCTTTCACTCTATTTGAGTAAATATTTTCTTGCTC
AGGGTCTTCATATTCCTACAATTGCTGCCATTGAAGGTATAGCATTGGGTGGGGGCTT
GAAATGGCGATGTCTTGTGATATCCGTATATGTGGTACGTGCTTTCTTGCACTTCTGGG
TGTACCATATTTTCTCCTTCTTGCTCTCTAGTTTGATAATGTGTTAACAGGTGAAGATGC
AGTGCTGGGCTTGCCAGAAACAGGACTTGCTGTAATACCAGGGTAGGTATGCCTTAATT
ACGCATTATATGTTTGCTTATGCAAAATCCCAAAATTCTTTGAAGGATGTGTTAGCTATG
TGTGGTTTATTTCTAAATTTATCAGATCAGTGGGACGCATTTCCACTATCTTTTTGTCACT
TTGTAATCTTTTACTATTCAAAGGTTTCCAACTTCAGAAGTTGCTATAAATACTCTGTATG
CATAGATGATCTTCTTAATGGTATCCTCTTATTTCATACTGGCATTGTGCAGAGCAGGAG
GAACACAACGGCTTCCTAGATTGGTTGGAAAATCAATTGCAAAAGATATAATATTTACTG
GCCGAAAGATAAGTGGGAAAGATGCTGTATCAATAGGTACGTGTATGACTTGTCAGAGC
TCATTTGTCAAGAGACAGGACTCCTTTGTCTTTCCAAGTTCTCTCTTGTTAATATAAAGAT
AGCAGTGATGTCAGCACTTCATTACAAATTATGGGTTAACAGTGTCCTCCAAGGTTTAG
GCAGATAGAAAGAAATCATCTAATTTTTGCTTCTGCTGTAATTTTGGACCTTGATCTCCT
ATGGTTTTCTTTTCCAATTTCTTAGTGAATAATACATTGTATGCAGGGCTTGTCAATTACT
GTGTTCCTGCTGGTGAGGCTCGCCTCAAGACACTTGAACTTGCTAGGGATATTAATCAG
AAGGTTAGACTTTAGTTATTGAGATAAAGAGGATGTGATGTATTTATCCAGTGTGCCACC
CATATGACTTCCAATTGCAATTTAGTCACGAACAAAGAAGAAACATAAAAGAAGTCCAAC
TCTTCCTATAAACAAAATGATTTCAAACTGTACTGTACATAGATAATTGTAAAGATTCGTT
AGCAGTAACGTGTACTCTTTTGTACCCTTTTCACCTTTTATGAGTTATGCACCCTCTTTT
GTACCCTTTTCACCTTTTATGAGTTATGCACCCTCAAGGCCATGAAATATGCTTGTCACT
GGATTTTCTTTTCTTTTGTGTGTGTTGAATGAAGTTGAGGCTCTTGTTTGAACTTTTTATT
GTCATCCATGGACCTTAATTTAATGGCATTTACTAATCCTATGCTTGTTTGTTTTCCACTT
TGTGCACTGCACCTTCATTTTTTGTGACAAGCTTTGTTTCTTGCTTTTGGTCTTTTTCTGT
CTTGTTTTTTCTTAGGTGGAGGATCATTGACTATTGCATAGTTCCTTGCTTTGGTTTCTTT
GTTTTCCTTTTCCCCTTCTTTTTCGATTTTAGCTATTTTATGGCAGGTTCACATAGAAAA
CAAGTGTTACTCTATTGTTTTTCTTTCCCTTTTTTCTCCATGCATCTTATAGAAACCGAAG
CTTAAGGTTTCTCCACGCAAGCTGCAACGTTCTGTTTTATAAGCTTCATATATTTCTGGT
TTTCATGTAGTATGAAATGATATTGAGTGGGATTATTAGGAAGCTGAGACAGATTGAAAA
GAACAAGTAAAAGCCACATTGGTGATTCCCTCATGCTTTCAACCTTAAAAGGTCATTTCA
ATGTCCAGGGTCCAGAAAAGGGACTCACTCTATTCATGTTCTATAAGAATGGAGTAATC
CACTTGACAAGTTCTGGTGGTACTACTTCTTCATAAGGTTTTATTATACTAGTCAGGGCT
GTGTTGAAAGGATATGGTAGCATCAACTAAGTCCAATTGTTTCGTATTGTATAGAGCCTT
CCTTTTTCCTTTTCCTTTTCCCTTGTTGACTCTGCTTTTATACCTCCAAATGGAATGGTCG
TAAAGCTTTTGCTCTTTATTCAATAAGCTAAAACTTCTGATGAATAAATTTGGTTTAAGGA
TGCACGAGGAGTGAAAGTAAAATAAATATTGATGAAGGTTTTGCTAAAGATGCTCTTTTT
TATGCTCGGGTTTTGCATGTCAACTGACATATACCTTATCAATGTCGACTGACATATATT
CTCTGACAGGGTCCGGTGGCGTTAAGGATGGCAAAATGTGCTATTGACAAAGGAGTGG
AGCTAAATATGGAGTCAGCCTTAGCTTTAGAGTGGGATTGCTATGAACAACTGTTAGAC
ACAAAAGATCGGTTAGAAGGCCTTGCTGCATTTGCCGAGAGGAGAAAACCTAGGTATAA
GGGTGAA
SEQ 49 
CTAGCAGCAACCAGCTATAGGAACAAATGTGTCAGCTCGAAGTGACATTGGTTGGCAG
CTAACATCCTCACAACTCTTGTGGTAAAGCATCTTTTGTACATAGTACATCAGATAGCAT
TGTGAGGCCCTGACAACTTCTTCATCGACTTCAGTAATCCATGCATCGTCGCATTTGTA
CCATTGGTTTCTCAAACGCAAATAAGTCACATAGTGACCTGATTCTAACATCCCTGAATG
TGTGACCACAGCGAAAATTTCAAATTCCGTAGAAATATCTGATTCATCACCGTCGAATGA
AAAGATTCTGTTCCCGTATCTCTTTCGTACAATTGAAGATGATAAATATGGTTTCATGTCT
AAAGAAAAAGGAAATTGCAGGTGGCGGTCAATCTTTCTGGACATTTTTCGGGTGGGAGA
ATGTTCAAAGCGTTTTATATGAAAAGATAGCACCAGCGGAAGCTTCTTGATGGACATTT
GTTTCAATGCATCTTGCTTTTCCTGACAATTTTCACAATACAGTTTCTGATCAGATCCCAA
CTTTTCTGGTCGTGTGAAGAGGTCCAAGCAACCTACAAGAGATTCATTTGGCTTACTCG
ACTTATTAGCAAAATCCTTTGGGCTGGAGTTGCAGCTATTCAAGTCAAGAGAAATGTCC
ATACAAGGATCATGAGTTGTTGAAGTGAATCCGCACGATGTACATGTGACATCAGATCT
CAAGAGCCCATAGAAAGTCCTATGAGCAATACACTGGCAATCTCCATTATCTGTTTAAG
AAATGTTATTTTTATCAGAGAAAAAGAACTAGAGGAAGCCACAAATCTTCAGTTCTCAAG
GTTATATATGAATCATCCCTCTTCTGATCAATGCATTGACAGCAAATGGAAGTACAGGCC
AAAATTGATATCCCAGAAAGGAAATTGCGCATGTACATAGACAAGATCTGTACTATATTT
ACTCACTGCGTAGCATAATGTCCTACATAAACAAGAAGAATACCAGCCATTCTCAGCAA
AAGATACCTTTGGTTGCCAAACTAGCTTTCCCCTCTTTATCATGGATCCTGTCCATAACT
GAAATGAAGAACTCATGAGCATCCTGCTGCTCGTAGGTAGCAAGATTTTCTGAATGCTG
CCACCAACTGCAAAAATAAACAACATTTCAATTAAAAGAATCACTACATAAGTTTTCAACA
TGTCAACGTCCAATAAAAGTTAACATTTTCCACGTTTAAGCATCCAATCTAAATTAAACAA
ATGATGATATCTTAGGTGGACATAGCAGCATAGAGAAAATTTCAACAGATTTATTTTATC
ACGATAAGAGAATAGCAATCTTGCTTATTGTCTGATTTTAGCGGTAATGCACCAAATCTT
GTTTATCCAAAAACTTCAAAGTGAAAACCTATGCAGTTAGCGCTAAAAATTGTAAACATT
ATTTACAATTTTGCAGGCCATGTTATAATCAAATATCCAGTATCAATACATTAAATGGTGA
GCACAGATAAAAAGCAATTAAGAGATAATGACAGGAGATAAATCCTTTTAACACTTACAG
ACCTGTAAAGAAACCGAGCTGGACTATAAGGGGTCCGATCACCAGAAAAGACAGCTGA
GAAGATAAGGTCAATATCACAAGGCAGGCACAACCGATCCGACGACATCTTTCTGCAAA
TATCTCGGTTATGCCTATCGCTAAGGAAGTAATTTCTCAAAGGGGGTGCATGAAGTAAC
ACTTGCAACACAGAGTTCATGAAACAAGTATTCCCCAAATTGTTCAAACCCCTTAATACT
AAAGGAAAACATGATTTCGACTTCTGATCCCTCCTTAAAAACAACGTCTTCATATTCTTT
GAATCTAAATCCATCCCAAAACTCAACCTTCTCCTCTTGCTCAACCTCAACTCACTCTCC
ACAACCCCAATTTCTGTTCTAGGAAACCCCATTATGTGTTTACACATCACAACCTTATCA
AAATCAGGATCATACACCTGATCACAACACACTGAGCAATAAAGCTCAGCCCTTTCCAT
GTCTACTGAAATCTCATGCCCAGCTTTACACTGACTATGCAAAAGGGCATGGTTTGATT
CAGGTGACAAACAACATAACACTGATGAACAGATCAAACACATGTAAAATCTACCCTCAT
GTCCACTACAAATACTACATCTAGGTAGCTCTGATTTGGATATTTCTAATGTGGTCCTAC
CATATGGGGTTGTCTTAAAACATTCTTGGATCAAACTATACCCACTCATACCATTTTTCA
CCTTGTAATCTGCAAGATGCTTACAGGGCTTTGGATTTATATATAAAGAGTTACTTGAGC
ACAT
SEQ 50 
ATGAAAGAACTTCATTCTCTAAGAGAGATCGAAGGGCCTGACCCGAATTATAAAGATAT
ATGCTTTTCTGGTGCTGGAAGGTAATAAATTAACTATAGTAATGTTAGATCATTAACTTTT
TCTTTCCTTTATTTTTGGTGTTGTTTCTTGTATTGAATGTCTTGTATATTGCAGTGACATC
TCAGAGCTCTCAAAATCATTTCCTCCTATCGACATGGTATTTAGCAATGGAAAGAAACTA
TCTCTCACTCCTGAAAACTACTTATTCAGGGTAGGCATCTTAATCCACATGGTTTTTATC
TTACTACCTTGCCTTTAGAATCTGTATCTCCTTTTGGCTTCATCTCTCCTAGTGGCTACA
TTTTTTTGTCTTTGTTTTGATAAGTGGCTCCATTTTCTCTCTGTGTTTCTATATTGACTAATT
 
CTGCCCTTTTGCTTACTGTAACTGATTGCTATAAAGCACTCAAAGGTGCGTGGGGCTTA
CTGCTTGGGAATTTTTCAGAATGGGAAGGATCCAACTACTCTTCTTGGAGGTATTTGTC
ATATATATCTTTTAGAATCTTGGGAAAGTTCATCTGCCAAATTCTTCAGTTGTATAAGCTG
TAACATGCGTGCCTTTGCTTTTAATTGCAACAGGTATTGTTGTCCGCAACACTCTTGTAA
CCTACGATCGTGAAAATGAAAGGATTGGTTTTTGGAAAACCAATTGTTCTGAGTTATGG
GACCGACTAAATTTATCTCCTTCACCTCCACCTCCACCATTGCCCTCAGGCTTGGACAA
CACAAACTCCAGTGCAAATTTGACTCCAGCACTGGCACCTAGTTTACCTCTGGAGCATG
CACCTGGTACGAAGAAACTGTTCTCCTATCTTTTTGTCACCATTAGTATGCCTTTCAGTC
ATGCTTTTATCCAGTTTTGTAGTGGAACTGGTTTTATTTCAATTATTCTACCGGAAGGGG
GGAGCCTTAGAGCAACGGTAATGTTGTCTCCGTCTGGCCTATATGTCATGGGTTCGAG
AAGTGGAAGCAGCCACTATTGCTTGCATTAGGGTAGGCTGTCTACATCATACTCACACC
CCTTAGGGTACGGCCCTTCCCGGAACCACATCAATCCGAGATGCTTTGTGCATCGGGC
TGTCCATTATAATTCCGCCAGGCTGTTTTGCATCATTTCCCCCTAATATTTTTAATCCATT
TTGGTTTCTGATTTGCTATGCTGGTTTTTTGCTATATCGCCTAAGATTAGGTTAGCTTTG
ATGATTTCACATCCTTTCTTTGATTAAGGTCCATGAATGTTCCTGTGTCTCCAAATGTCA
GCTTTCAAAATGACATTTGAGCTTGCGTTTTGTATTGTTTCATCAAGTTTTGTATTCATCT
ATCTCCTTAGCATTCCAGAGTTCCTGAGAAGCACTCGCTAGTAAAGACGTATTCTGATG
TCATGACATTTTTAACCTTGTTGGAGTTTGGACCCAAACAAACTTTTTTTACAGAAGGAA
ACTATAATTTTAAGGAGTACAACAGTTGCTGTATATAGAACATGGTGAGTAACTCCACTC
TTGAGATGCCTTCTCTTCACTGAAGTCAATTTCTAAAAACCTCCGTGCTTGACACAGATT
TGTTGGTATAGATTTGTGCTCCAGAGATGCAGATGGGTCCAGCGAATTTTTCACTAGAA
TTTTTCTTTTTTTTCTCACCACCTGTCCAGCATAGTGCTGTCAAAAGTGACAAGCTTAGA
AAAAAACCATGTGCTTGGTGGGGCTTTAACTGCAACATGCTATAAAAACGTTCACTATAA
ATGTAATGTAAATAAATAACCATAAACACAAAATAGCAATTGTTGGAAAAATTGCAATTTA
GTGAAATACACGAGGTGTCAATCAAGTTCAGATCATATTGTAAGTCTTGATTCAAGGTG
CATTTTTAAATTAGTAATTTGGATCAATGATTAGTTTTCTTAGACTATAACTTTCAATTTTC
ATACTCGTAAGAACTGATATACTCATATATAATATAACGTTTTTCTTAATAACTAATAAAT
GCTTTCCTAACTATATTTATTTTTGTGCTATCCTATTAACAACAGAGCCTGTGGATGTTAG
GCACCCACTTTAAGGCCTTTTTCCTCGCACTGAAGCCCTACTTTTAAGGTTTACTGTCAC
GACCCAAAATTCCACCTTAAGGATCGTGATTTCACCTAGTCTCTAAAACTAGGTAAGTC
GATCACTTACAACAGTTAAACCATTAAAACATGATATTATGAAGCGGAGTTTAATATAAAT
GCGAAAATAAAGGTGATACAAGCCAACACGGCGTTAATCACAACAAATCCCCAAGACTA
GGTAATACAGAGTCACGAACTCTAACTGAATACATAGAAATATTTCAAAACAAAGATACA
ATACTGTTCTGGCAGATAATTGACAGTATAAAGATAAGGAAAGACTACAAGGGACTTCG
ACGATCAAGCAGCTCTACCTTGAATCCTCGTGATCAAAAAGCTAACTCTGCCTAGGTCC
TATGCCTCCAACACCTTGATTTGCACAAAATGTGCAGAAGTGTAGTTTGAATACACCATG
GTTGGTACCCAGTAAGTATCAAGGCTAACCTCGATGGAATATTGGCGAGGTTCAAGTAA
AGACACTCACTAGTCAAATAACCTGTGAAAAATATCAAAAATGGGCAAATGGAATAATAA
CATAAAGTCATAACTGTAATCTCTTCAAATTAAACGATACCTATTTAGAATAATTAAAGGT
CCCGTTCTGACAATAAGCCATCAAATAGAATCACGCACACCCGGCACCTCGTACCCACA
TTAACAATCACCCTCGCACGGCAAAGGCCTCGTGCCACAACATAAGATATACCTCGCAC
GACGAAGAGCTTGTGCCACAATATAAGTCACAACCGCATGGATAACTCATATGCCAATA
TCACAATCCGCCTGGCGTGGTCACATGCTCAATATCACAATTCGCCCGGCGTGGTCAC
ATGCTCAATATCACAATCCACCCGGTGTGGTCACATGCTCAATATCCCAATCCGGCTGG
CATGGTCACCGGCTACCTGTCCAAATGTACATGATCAATGGACATCAAGTTTCATACTC
CTGGACTGATATTAATGACATGTTATGGTATATGCATGTGCAAGTGTATTATCACAGCTT
AAATCATCTAAGTAATATCAGAGACACCAAGTGGCACATTAGGAACAACACAACAAATC
ACGTAATATGTATGACACACACAAGGAAGTCAAAAGCAACAACCAGAATACTCCTCTTT
CATCAACAACATGCCCCCAGGCCATCACATAACATCCCCTTATTGCCACCCTTATGTCA
CCACGTTGACAATATCATAATAGCCACCCGTATCGCTCCGCCTAGGCAGTATATCAATA
GCCACCCGTGTCACTGCGCGCAGACAATATATCAATAGCCATCCGTGTCACTCCGCAC
 
AATCAACAACAGTGAATTGTCATCCTTGTGCTCCGGATAACAACAATCGATCCACACAT
GTCCACATATGCCACAATATCACAGGATAGTAGTATTAGAGATTTATCACGATACAAGCT
CACCACTCATCAACAAAGTGCACAAGGACATATCATTAATATAGAATTGCTGAGGGGTA
TTCAACATTTAAGCATGAAAGCTACTCAAATTAACAAGAGTCTCACAAGCGCCCAACTTG
GCCAAATAAGGAATTAAGATCCTAAAACATGATTTGTACATGGAATATAAATAACTTAAT
GTCAAAAATAACTTGATGTCATAAATAAAAGCCATAGGAAACGATTCTGAATAATAAAGC
TTCTATCTTGAACAAGAATAAAAAGTAATCCCAAAAAGTCAACCCCGGGCCCACACCGT
GGAATCCGACAAAACTCACAAATTCCGAACACCCGTTCAAATACGAGTCCAACCATACC
AAAATCATCCAATTCCGGCCTCAAATCGGCCTTCAAATCATCAATTTATGTTTTAAAAAA
GTTTTTACTATGATCTCCAATTTCTCCCATTCAAATCATCAATCAAACACTAAAATTGAGA
TTGGAATCATGAGAATAAACAAATCCGAGTAAAAAATACTTACCCCAATCCAAATCGTGG
AAATTCCCCCAAAATCGCCCAAATCCGAGCTCTATAACTCAAAATGTGATAAAATAACCA
AAACCTTTGAAATAGAGTACTTATAGATCTGCTCCAGGTAAACCCTTCTCAATTGCAGGA
CCAGCTTCGCAATCGCAAAGCACAAACTTAACTGACCACAGAAATACCCTTCGCGTTCG
CGGTACATACCTCGCGAACGCGATGCATGGCTGAGCCAGACCTACGCGAACGCGGCG
TAGACCACGTGACCGCGAAGACAATACCACCAGCTCCCAGTTCTTCATCGCGAACGCG
TCATTGCCATCGCGAACGCATTGACCAAGCCCCACAAAGCTACGGAACGCGACCCTC
CAGTTGTGAATGCGAAGAGGAAAAACACTCAGCTCCAATCATACACTGCGCGATCGCG
GTTAGCCCCTTGCGATCGCTAAGAACGTCAGCAACAACAGAAAACCAGCAACACAACAT
GAAGGAAAATGGTCCGAAATCACCCCGAAACTCACCCGAGCCCCTCGGGGCCCCGTT
CGAACATACCAACAAGTCCCAAAACATAGACAAACCTACTCGAGGTCCCAAATGACACC
AAACAACATCAAAACTACGAATCACACCGCAAATTCAAGCCTAATGAACTAATGAACTTT
CAATTTCCAAAACTCATGCCGAACCATACCAAATCAACTCAGAATGATCTCAAATTTTGC
ATGCAAGTCCCAGATGACATAAACGGACCTATACCAACTCTCAGAACCGCAATCCGAAC
CCGATATCAACAAAGTCAACTCTCGGTCAAATCTATCAACCTTCCAAACCTTCAACTATC
CAACTTTTGCCGGTTCAAGCCAAAACAACCTAGGAGACTCCAAATCCACATCCGGACAC
ACGCTAAATCCAAAATCACCATCCAGACCTAACAGAACCATCCAAACTCTGATCCGAGA
TCAAATACGCAAAAGTCAAACTTGGTCAACTCTTCCAATTTAAAGCTTCTAAAATGAGAA
TTATTCTTCCAAATCAATCCCGAAATGCTCGAAAACCGAAACCGACCATACACGCAAGT
TGTAATACATCATATGAAGCTACTCACGACCTCGAACCACCGAACAGAAATGCAAATGA
TCAAAACGACCGATCGGGTCGTTACATTTATGTATGCTTCAAATGAGCATTCAGTGACA
CTGTTCAGCAAAAGGAGAAACTCTACTAGCCACTTGTAGCCACCTCCAGGGACCCTCTC
TGTCTCGGCCATGGATTACTTTGAGGAGTAATAGGGCTTCTCCAAGCGGAAACATTCCA
CGCATGCTGTGATCCTCCATGTTTTCTCTGCTAATCTTTGCTACTTTTTCTGGCGGTCCA
ACTGGTTGATCAATTCTACTAACACCATCCGAATGAGCCCTCAGGAATTCATCTCCCTA
CTTCTTTGCCAGATACAAGGAAGGCGTTGTCCTCATAGGGTTGGCCTTGGCATAACTCT
CTGATAATTTGACACGTTGCTTTAATTTGGTAACAATTTACAATTTTGGGGGTGCTGCAC
TTGATTCCATTGAGTTACACCATTTCTCATATTTAGGAATGGTCCTGTGCAAGATTGAAA
TGTACTGAACTCAGTTTTTCCCTGTGCAGATATTTATGATTGTTATTATTATTTCATCTTT
GACCTGAATTGGCAGGGAAAATCAAAATTGGACTCGTATCATTTGATATGTCACTGAGT
GTTGATTACTCAGCATTGAAGCCTCGTGTTCCAGAGCTTGCCCATTTTATTGCGCAAGA
GTTGGAGGTTAACGTCTCACAGGTAGTTTTTGCATGACCCAAAGTTGTGTCAGTCTGAT
GTAATCTAAAACTGTATATCCCATTTTCTTTAAGTTACTTAACTGTATTTTAATTTTGTTCA
ATATGATATGTCACTTATTGGAAGATACCTTGCAGGTTCACTTAATGAACTTTTCGACAG
AAGGAAATGATTCCCTCATTAGATGGGCCATCTTTCCTGCAGGATCTGCAAACTACATG
CCAAATGCCACTGCAACAGTAACTCTAAACATCTAGAATATGTGAGGACTATTTCTTGAT
TGAAGAACCCTTTATTCATCATTTACCTATTTGCAGGAAATAATAAACCGGTTGGCTGAG
AATCGTTTTCATCTTCCTGATACATTTGGAAGTTATAAATTAGTCAAATGGGACATTGAA
CCCCCACCAAAGAGGTATAAAAGCTATCTCCATTCTTTGCATGTTCATAAAATATTGAGT
TCTGCTGTACAAACTTTTAGCATCATAGCATTACTTATAAAATTATTCTGAATTGTCAAAA
CAAATGTGCCTTTTCTTTTCAAAATGCAAAATAAATCTCCGCATTGCATTTCAGATGGGA
 
AAAACATGACACGCATCTTTTCATCTTGCCTTAAACACATGTTTGTAAGTTACATTCTAAA
TTAGGAAACGTGAATGAGTCTACATTGCATCGCACCAGTTCGACTGCATATTCCAAGGA
TAATGATGAATAGGTGATGACTTTCGTCTCCATTTTTCATTGTTTCAATTTTTCTCAAAGT
TTCTTACTTGGATTGGTGGATAAAGTGGCAAAGCCAGAATTTTTTTATAAGGATTCGAA
AATACTAGAATGTCATAATTGAGATCTGAACTTGTGACTTGAAAGCAACTTTTGAATCCT
CTTTGCTACTAAACTAAAAAATTTCCCCTATGGCAAGGAGATTCAATAGCTTATATATAA
CCAAAAAACTTCATTTTTACCCTATTCGCATACTATAATTTGAAATGTTTTTGGTCAAAGT
TTAATTTGCTGCATCTCAAAATCTTAATAGCAAAATATTACCTTAATTAACTCTAATGTAA
AGAGATTGGATAACACACCACAACAATATTTTTGGTAGGTGAATATTACTTTAATTTTTTT
GACAATTAATTGAGATAGGAGTTCTTGATATTTTTTTTTTGGTTTTGGTAACTATCAAGTT
GTTGGTTTGATATGGTTTCATTGCAACGATTTAGGATACGATGGCAGCAAAATTACCTTG
TTGTAGTGTTTGCGCTACTAGTTGTCCTGATAATTGGATTATCAGCTTCTCTGGGATGGT
TAATTTGGAGACGAAGGCAAGAAATCCCATATAATCCTGTTGGAAGCGCTGAAACACAT
GAAAAAGAACTCCAGCCGCTAAAT
SEQ 51 
ATGGTCACAGGTCTGAACTTCCGCCATAACTTTCTCGTGTCAGCTTTACTATACTCTAAA
TTTGAACTATAATACCACATTGATGTGAAAATTCACACTTAGGTATCGATTTTTTAACACA
GAGATTTATTTTGTGTTCATGCTTTGGTTTCAAGTATTGGAGAACCTCGTAATCGTTCTC
TATAAGCTTCTGGTTTAACAGATCCTAATTTTTCTTAGAAGCTCGAATTATTTTGTATTGG
AATGAAATGAACCTGAATATTGTGGACGATACAGAGGAATTATTGTGGTATAGTTGATTG
ATTGATTGATAGTCTTAAGTAAGAAAAAGACCTATTGGAGATTATGGTGAAGCTTATACA
GGAGCAGCTGGACTTGGTTTTCACAATATCTTTTTTGTTAAGGTTAGAATAAACCTGCTA
AAATTTTTTACTTATCAAAATAAATAAATAAACTTGCTAGAATTTTTTTCAAGTTGGTGATT
GTTTAAGTTTTTTCGATTGTTTTTTCCTTTGGTAAAAACGTTTTTGGCAAGAACTATATTTT
GAAGTTGTGGTTTGAGAGTGTTTGTCAAATAATCTTTTCAAACAAACTCTCTTTTCAAATA
TCCGAACATCTTCAACTTCCACGAAATAGGTGACAACTGGATTAAATTTGGGGGGGGG
GGGGAGTGGTTGATGGTGTATTAAGTTCAAACATCATTTATCTTTTTCTCTAGGAGCAGA
TTTTTAAATCATTATAGATACATTGCTTGATGTATGTTTGAGAAATACCATTGGTGTTTCA
TTTAGGCATCATCACTGTAATAGTTTGGTTAATGTTTTGTTAATTCATCATGGTGGTTCAT
TCAGACAGCATCATTCGGCTATGATGTTGATGTTGATTTGGTTACACAAGCAACTCATTG
GTGGAAACAGTTTTCTAGGATACCCCTTTTATCATTTTCATTTGATTGTACCTCCTGTTTA
TTTTTGCACTTGGACAATTACGGGCTACAATTCTCTCCTTGCAAATCTGGTGATTGGTTG
CAGTAAGTGTAAAGTGGCAAAAAGAAGTCTATCCTGCTGTGGAAATTGACACTAGCCAG
CCTCCATATGTTTTCAAAGCCCAGCTGTATGATCTAACAGGGGTACCACCTGAAAGGCA
AAAGATAATGGTCAAAGGTGGTTTGCTTAAGGTATAAAATTTCGTTTCACTTAGCTTGTT
ATGCCATTTTTCACTTTGCAAATAAAGCACAAAACTCATTTGTGTTTTGAGGAACGCTGA
AATTCTCAGCATCTGATGCTTTGCTTGTTAATTTTGTTGTTAACTCTTCGGTTATTTCTAT
GGTTGTTTAGGACGATGCCGACTGGTCGAAAGTAGGAGTAAAAGAGGTACACGGCTAC
TCATTGAATTACTCTCTATTTTTATGCAATGAAGTGCCAATTATCTAGAAGCATCTGTTAT
TTATTATTTCCAGGGTCAAAGGCTGATGATGATGGGAACTGCAGATGAGATTGTGAAGG
CCCCCGAGAAGGGTCCTGTTTTTGCTGAAGATTTACCTGAAGAAGAGCAAGTGGTTAAT
GTAGTAAGTTTTTTGACACTGATGTTGTTGCATCAAATCGAATGATCCGGAGATGTGTGA
TTCCTTATGTTTAACTGCTTACATAGTTAGTCTTGTCTCATATGCTGTACTTATACCAGCA
CTGGATCCCTAGTAGATTTATTGGTATAACTTTACCGCAATTGCTTTGTTCATTTTTTTTC
AAAAGCAGTTGCCTTTTCCAACTTCTACATGCAAATAAGCTTTAATATATAATTCTCTATT
CTTTTTCCGCTGGCACAAGTGATTTTGTGGATGCCAAGCGCTTGTCGAATGCGTTTCTT
GTTCCGCTGGCACAAGTGATTTTGTGGATTCCAAGCGCTTGTTGAATGCGTAATTTCTT
CATTTACACATTATGAATCGGCCCTTCCCAAGACCCCGCACATAGCAGGAGCTTAGTGC
 
ACTGGGCTGCCTTTTACACATTGTGAATCAGATTACTATGTTGTTTTAGAGTCCTGTCT
AAAAGAACTGCTAACTTTTATAATGGCAAGGCTTAGTTTTGTACTTTTAATCAGTAAATG
GGTGATGAGAATTTTTATAATTTTGTTTCCTCCAGGGTCATTCTGCTGGATTATTTAATCT
CGGAAATACATGCTACATGAACTCCACAGTACAGTGCTTGCATTCAGTTCCAGAACTGA
AGTCTGCTCTAACAGAGTGAGCATTTGCTTCTTTCATCCTTTCCTTCATTTTTGGGAGTC
TTTTGGTTTAGGCTTTTTTTTGGTCCTTTTGCTTTAGCCTTGATTTCCCAAAACTTGATCA
AATTCAATATGGTTGCTTTTAAGTCTAGTTCTGAAAATATTTAGGTCTATTTTGATTGCGT
TGCACTTTTTTGGTTAGGCAATTCGATCTATTTGCACCTAATCCGTAATTCCTGTTTTTGC
TAAAATTGATAATTCTGATTTTACTGTTTATATTTGTCAAATCTATTAATCATAATTTAACT
TATATAATGTGTCGCGTTGTATACACCTAGATAGTATGTATTTACGGAGACAAAGCGGA
GAAAACAGTAATTAGTAGAGGAGACATAAATTATCCTGTTTTAATTCCTATATATCCTCC
CTTATATAAATATGGACTCGTTTCTCGGCATGTTCTCCTTTGGATGAAATCAATCCAAAA
TGTAATCCACTTTGAATCAATTTGGACTCCGAAACTGTGGATCTTTCCCGAACATTATCA
GAAAAAAGATCAAAATGGCTCCTGTTAAATACCAAGTGTAGGAGTTCCAAAAACAACTC
CGTTAGGTACATTTCTTTTTGTGTTCCTGAGATTCTGAGTTTATTTATTCTTCCTGTTAGG
TATAACCAGCTTGGTAGAAGCAATGATTTGGATCACTCATCTCATCTCTTGACAGTTGCA
ACAAGAGATCTGTTTAATGACCTGGATAAAAATGTCAAACCAGTGGCACCAATGCAATT
CTGGACGGTACTTTATTTGTCTTTATTCTACTCCTAATATTTTTGGTTACGACTTAGTATT
CCTGACTTTGTATTCTTAGAAAATGTGTTTGGATTTCGAACAAAGTTACCATACCTTTGA
AGGAGAATACGTATGCTGAGTAGGAGATAGTGTTTGCCAATAATTTCCTATTGGCAGAC
TTCAAAATATACTCGTTTAGCGTTGAACACTGAACTCGATATATTTTGTCATGACTTTTGT
GTGCAAATGGATTTGCTCTTAGAGAGCAAGGATGAAGTACTTTGTATAGGATGCGAATA
GAATGACTTAAGCTTGGGCCTGTTGTCTACATGGTCAAAACTTTGTGCATTATCATTCTT
GCACAAGGTTACTTGGATTTATATGAGAACTATAAATGTAGCCTATGTTGATATGTTTGT
CTTTTTAGTGTTTTCGTCACATGAGCTACTCGGGCATCAACATTTGATTAGGTTTATGTT
CACAGGTTTTGCGGAAGAAATATCCTCAATTTGGCCAGCAGAGCAATGGAGCTTTCATG
CAACAGGTTCCAAGCTTACCTAAGCTACCACAATGCTTCCTTGTTATTAAAAAAAAAAAA
GTGTACCACTATTGCAATTGCTATATAGAGGTCCTACTGACATGTCCTGGATAATAACAG
GATGCTGAAGAATGTTGGACGCAACTACTTTACACCCTTTCTCAGTCTCTTAAATCACCG
AACTCTAGGTACTACATCTCCTCTCGGGATATTTCTTGCAGATGAAAGTCCCTTTTCTAA
ATAATTTCCATGTTTTGTTTCGCTAGTTGTTTTCTTTGTTTCAGTTTGGACATATGGTCCC
TATTTTGTAAAAATGTGAAGGAAAACTCTCCTATATATACATTGTGCTTCTTTATGTCATG
ATTGTGACTGCTCTTTATCTCGGTACTTGCAGTGGAAGTCCGGATATTGTGAAGGCTCT
CTTCGGTATTGAGTTTGACAACAGGTATTTCTGCAGTCAAATGTTGTTTACCTTCCAGTT
ATTCTGTTACCTTATCCCCTTTGCATAGAGTTGTTCTGCACCTAAATATTATAAGAGGCA
TGTGAACTTACTGCTGTATATGTATTGAGATAGGAAGGAATGCAGCTAGTGGTCCTAGG
ATGTAGGATGTTCCCTGTTCTGACTTTGAGTATCTTCTGGGCAACCTGATGAGAATCAA
CATCCTCAACTTTTACTCTGTCATATTGTGAATCATGTAGTTGACAATAAGAGATGAATTA
CTGAAGTTGTTTTGAAAGTTGAAGCTAAAAATCATGTTTATGTTGACTTCTTTTAGTTTCT
CCTACTGTTAGTTAAGTGTACTATAGTGCTACTAGTGTGTATTTGTATTACTGCTAATGA
AAGGTCTGGCTGGTTTATGCCGTTCTAGGGTATTTTCAACAGGCTGTGCTTTTCTGCTG
TAGCCTAGACCTCTGGGCTAATATTTCTTGTCTAGGACCTGACCTACTGCAATGAGGTT
GGGAAGATCCCAATGCCCATCCCAGAGTTCTCATGTGCTCAAATTCATCTACTGATATA
CAAAATTTAATTTTCGATAGTGTGGGAAGCTGTTTATCATTCATGTCTGACTTGATCTATA
CTGTTCTGACTGGATTATGGTGGTTGTGCTAGAGTCCACTCTGTGACTATGTTCCATAT
CTGATATTAACTGCTAGATACTGAAGAAAAATGACTTAACTCTGCCCCTTATTCTCATGG
TACTGATATGGACCAGGATTCATTGTGCTGAAAGTGGTGAAGAAAGCACAGAAACAGAA
ACTGTATATTCCCTTAAATGCCACATTTCACAGGAAGTGAACCATTTGCATGAGGGTTTG
AAACGTGTAAGTTCGGTTCTTTTCCTCCTTGTATGTCCCAACTTCTAACTTTAGTCTTGTT
TCCTCCCAAATGTTTCATATTACTGCTAAGTTCTGTCTCAATTTTTTCTGTTGTGCCAATC
CAGTAATCATCCAATTTGATTAAGAGGACAGTCCCAAAGTGAAAAATGACGTATCTAATT
 
CATAGAAATTCCTTTGGTAGCTGTAACCTTTAAGGATAACTAACAGTTAGTCCTGAAATG
GTTGGTTGGATGGAGAAACTATTATATAAGATGCCTCACGGCGGCACATTGGGGGGG
GGGGGGGCTTTTNGGGGGGGTCTTTTGCTTTAGAATTTTTTCATGCCATGATTGGACC
AAATGTGGGGCCTGCATGTTGAGGTTCATGCAATAGTTTCACATCAGAGGTAGGCTGA
GCCATTGGAGCCGACTCATAATGCTTTTGGTGGGAAAAGATGCTTTGGAGATTTTGTTT
AACTTGGTGCAAAATCGTTGATGATGTTTATACTGGAAATCTGGTATTTATCCCCTCAAA
ATAATTTAAATGCACTCATATGGCCATTGCTTTTTCTGACAGGGTCTGAAATCAGAACTG
GAGAAGGCGTCTCCGTCACTTGGACGGAGTGCAGTTTATGTGAAAGACTCCCGAATCA
ATGGCTTGCCAAGGTATTAACTGGCTCGATTAAATTCCATGGCGATGTAGCGACATATG
TATGATCCGTAGCTTCTGCATATAGACTATCTTAATCCACGCCTTTCACATAACAAAAAT
GCCTTTTGATGTGTTGAAGTAGTTCACCTCATTTTTGGCATCACTTCTTTCTCATTCTCCT
TTCTTTTTTTTTTTNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNN
NNNNNNNNNNNNNNNNNNNNNNNNNNGGGGCTTTTAATTCTCACACTTTTCTGGATAGTTT
CTTTACTATTTGGTGCTGATTATTGACTGGTCTATGTCAAAATCTTCTTCTATTTTTACTA
TTTCTTTTTCATTTGATGAATAGATACTTGACCATTCAGTTCGTCCGGTTTTTCTGGAAGA
GGGAATCAAATCAAAAGGCAAAGATTTTGCGGGTATGTTGACTTACTCATCTTCCTTTTC
ACATTGATCAAATAGTTGGTCCTCCTTGAAAATGTGTCGGAAGAGGGAAAAGGATCAG
GTTACCATGTCCTGAACCGAGAAAGGGGGATGGAGAAAGGGAGTTCACTGTTTTACTTT
GTTTTGTGAGGATGAGCGGTTCTGTCATGTTTAGGTGCTCTAAAGTCTCGCCTATTCTC
TCGATTGTCAAATTCTGAACTGTATATTTTAACTTTAGGCTGGTTTGTGATGGTGTCGGT
GTAGGAGTCTTGGTCTATAATCCCTTCCCAATAAAAAATTAACTGAAACTTCTCTTAAGT
TTCTAATTGATCTTGGAGTAGGTGTAACTTGGGCACAATTACAAAGAGGAGTTCTTATGC
ACCAATGACTAGATTTCAGCTACTAGAAGTAAGAAGTAAGAGAAGGATGTGATATTAATA
CTTTGCTATTCTAGTGAGATAGTATGTACATAATATTTTTTTGAAAGAGAGTTCTGGTGA
GAAGCTGATATGTTTTTTTTTCTTTTTTCCTTTGTGTTGATACAAGATCTTCTTAACAATCA
AAAATATCTGAAAAGCTTTTTCCTGGATTCGGCCATTCGGGATAATAACCCACCCATTGC
CATAAGTTCTGGGATCTATAAAGTAGTTGTACTGGGTGTTTCAGATATCTTTGTGTTTGT
GAGAATGCACAGGAGAAATCTATTCTATTGATTAAAGTGTTGTACAACCCTATTTATATA
CAGTAATTACATAATAATAGGTATCTACTTCCCGATGTGGGACACTATAATACGAGAACC
AGTAAGAGACTTAGTGAAAATATCTGCTATGCTAGCTAATCATTCTACTTTACAAACTTT
GTAACAATATCTCCTGAGAGTATCTTTTCTCTGCCAAAGTGACAGTTGATCTCAATGTGT
TTAGTCCTCTCATGGAACACCAGATATGACGCAATATGAAGAGCAACTTGATTATCACA
CGCCAGTTCCATCGTGCTGATTTCTCCGAACTTCAACTCCTTAAGCAACCGCTTGATCC
AAACTAGCTCACACGTTGCCACAGCCATGGCCCGATATTCGGCTTCGGCGCTAGATCA
AGCAACTACATTTTGTTTCTTGCTCTTCCAAGACACCAAATTACTTCCTACTAGAACGCA
ATATCCAGACGTAGAACGTCTATCAGAAGGTGATCCTGCCCAATCAGCATCTGTGTACC
CAACAATCTGCTCGTGGCCTTGATCCTCGAATAGTAACCCCTTTGCCTAGAGCTGACTTT
ATATACCGAAGAATGCGAACAGCTGCAATCCATAACTGACTTACAACACTCACCGGAAA
AGAAATGTCAGGTCTACTCACAGTGAGGTAATTCAATTTGCCAACCAACCTCCTATATCT
TGTAGGATCTCTAAGAGGCTCCCCCTGTCCAGGCAGAAGCTTAGCATTCGGATCCATA
GGAGTGTCAACCGGTCTACAGCCCATCATTCCCGTCTCCTCAAGAATGTCTTAAGGCAT
ACTTTCGTTGTGAAATAACAATACCTGAGCTAGACTGAGCGACAGTCTCGAAGTGCTGA
AAGAGTTGCTGCTTCAGATTAGTAATACCATCCTGATCATTGCCAGTAATAACAATATCA
TCAACATAAACCACCAGATAATACACAGATTAGGAGCAGAATGCCGATAAAACACAAAG
TGATCAGGTTCACTACGAGTCATGCCGAACTCCTGAATAATTGTGCTGAACTTACCAAA
CCAAGTTCGAGGTGATTGTTTCAAACCATATAGTGACCTGCGCAATCGGCATACAAGAT
CACTAGACTCCCCCTAGCAATAAAACCAGGTGGTTGCTCCATATAAACTTCTTCCTCAA
GATCACCATGGAGGAAAGCATTCTTAATGTCTAACTGATAAAGAGGCCAATGACGTACA
ACAACCATGGACAAGAAGAGACGAGCAGACTTTAGCCGCGGGAGAGAAAGTGTCACTA
 
TAATCAAGCCCAAAAATCTGAGTGTATCCTTTTGCAACAAGACGAGCCTTAAGACGATC
AACTTGGCCATCCGGGCCGACTTTGACTGCATAAACTCAACGACAACCAACAATAGACT
TACCTGAAGGAAGAGGAACAAGCTCCCAAGTGCCACTCGCATGTAAAGCAGACATCTC
CTCAATCATAGCATGTCGCCATCCTGGATGAGATAGTGCCTCACCTGTAGACTTGGGAT
AGAGACAGTTGACAAAGATGATATAAAAGCATAATGAGGTGATGACAGACGATGATAAC
TTAAACCGACATAATGAGGATTAGGATTAAGAGTGGATCGCGCACCTTTCCAAAGTGCA
ATCGGTTTACTAGGAAGAGGCAAGTCCGCAGTAGGAGCAGGATTAGGTGCAAAACGTG
AATCAGCTGGGCCTGATGCTGGCTGCGGACGACGATGATATGTCAAGAGTGGTGTTCC
TGTGGCGGGGAATCTAGGAGGAGTCTGTGGCGAAAGGTGAAGGAGGAGCTATAGTAA
GCTCCTTAAAGGTCGATACAGGTAAGACCTCAGATATATCAAGGTGGTCAGAAGAGGTA
AAGAAAGGTTTAGACTCAAAAAATATGACGTCAGATGATATAAAGTACTTATGAAGATCA
GGTGAGTAACAACGATATTCCTTCTGAACACGAGAATAACCAAGGAAGACACACTTGAG
AGCACGAGGAGCTAACTTATCTTTCCCAGGGGCTAAGTTATGAACGAAGCAAATGCTCC
CTAAAACACGAGGAGGAACAGAGTATAAGGGTGATTGGGGAACAATACTGCATACGGA
ATCTGATTCTGGATGGGAGATGAAGGCATCCGTTTAACCAAATAACAAGCTGTGAGAAC
TGCATCAGGTGGTCAGAAGAGGTAAAGAAAGGTTTAGACTAAAAAAATGTGATGTCAGA
TGGCATAAAGTACTTATGAAGATCAGGTGAGTAACAACGATATCCCTTCTGAACACGAG
AATAACCAAGGAAGACACACTTGAGAGCACGAGGAGCTAATTTATCTTTTCCAAGGGCT
AAGTTATGAACGAAGCAAGTGCTACCAAAAACACGAGAGGAACAGAGTAGAAGGGTGA
CTGGGAAATAGTACTGCATACGGAATGTGATTCTGGATGGGAGATGAAGGCATCCGAT
TAACCAAATAACAATCTGTGAGAACTGCATCGCCCCAAAAACGCAACAGAACATGAGAT
TCAATGAGAAGTGTGCGAGCAATCTCAATGATGTGCCTATTCTTTCTCTCTGCAACCCTA
TTTTACTGAGGGGTATAAGAACAAGAGGTCTGATGAATAATTCCTTGAGAAGTCATAAAC
TGCTGAAATTGAGAGGATAAATATTCTAAGGCATTATCACTGCGAAAAGTGCGAATGGA
AACACCAAATTGATTTTTAATTTCAGCACAAAAATTCTGGAATATAGAAAACAACTCAGAA
CGATCTTTCATTAAGAAAATCCAAGTAATCTTGAATGATCATCAATGAGACTAACAAAAT
AACGAAATCCCAAGGTTGAACTGGCTCTACTAGGACCCCATATATCAGAATGAACTAAA
GAAAAAACAGACTCTGCATGACTCTCAATACTACGAGAAAAGGTTTGGGAATGTTTTCC
GAGCTGACATGACTCACACTATAATCTAGATAAACTAGACAAACTAGGCACCATCCTCT
GAAGCTTGGATAAGCTTGGATGTCCTAAACTTATGTGAATTAGGTCCGGAGGATCTGTA
GCTAGACATGTCTTGGAGGAATTGAGTGAGTTAAGGTAGTAAAGGCCTTCTGATTCAAG
TCATGTTCCAATCGTCTGTCCCGTACTACGGTCCTGCATAATAAAAGAATCATCAATAAA
ATATATACCACAATGGAGGGCACGAGTCAAATGACTAGCAGATGCAAAGGACAGCCAG
GGACATAAAGAACAGAATCTAGAGTGACAGAGGGTGGGGGATTTGCTTGTCCAACTCC
TTTTGCTTTAGTTTGAGACCCATTGGCTAAAATAATAGTGGGAAGAGACTGTGAATATGC
AATATTTGACAAAAGTGATTTATTACCAGAGATATGATCAGAAGCTGCTGAGTCCACAAC
CCATTATCCAAGAGTACTAGACTGGGAAACACAAGCAAAAGAATTATCAACAACAGAAG
TATCAGTCTGAGCAATAGAGGCTACTTGTGGAGATGTCTGCTTGCTCGATACGGAGGG
AACTCATTATATTCCCCTTCAAATAAAGAAAATCCCTGGTTACCTGTAGTCTCGGTCTGA
GCAACATAAGCATTTTTGAGTGGACGACCTTGTAAAGAATAGCACACGTCACGAGTGTG
TCCAAGTTTATGACAATAAGAGCAATTGGGCCTAGATCTTCCAAAACGACCACCTCCTC
GTCTATTCTCCATAGTTTGAGATACCTGATTGTCCACTGACTGGGATACGAGAACAGAT
GAGTCAAGTGTCTGTGATGAGCTTACTAGGTGACTTGGTATTGCAGCAAGGCGAAGTAA
TTGAGAGAATAATTCATCAACATTGGGGACAGTCGGACTAGCCAAAATCTGGTCACATA
CTGAATCAAGGTCATTAGGGAGTTCAGCGAGTGTAAGAACTAGAAACATCTTCTGTCGT
TGCTCTTGTTGCTTTTCAATACTAGCAGAAACTGACATCAATTGCTCAAATTCTTCCATG
ACTGCCTGTACTTGTCCTAAGTGTGTAGACATATCCAATTCCTGTTTCTTCAAGCTTGTC
ATTCGCGATATTACATCATAGAAACGAGATAGTCATTAGTGTATAAAGTACGAGCCTTCT
CCCAAACTAAATAACATGTCTGGAATGGATGGAACAAAGGCATCAACTTGGAATCAATA
GATCGCCACAAGATACTACATAACTGAGCATCGACCTTCTCCCAAAGTGTTTTGGCCTT
TTCATCACCGTCGCTAGCCCTTTTTGTTAAATGATCTTGAACTCCTTGACCTTTACACCA
 
CAACTCGACAGACGAAGCCCAAGCTAAGTAGTTTGAACTTCCCATTAAAGGTTCTGAGG
CAATCATAATACCATAACTTCCAGAACCCGTGTTTTAGACCCAAATACATCCACTCCCA
AAGACATTATTGGATTGAAAAGAGATCTAGCAAATTAGCACCAAATAAAACAAAGAATCA
ACTGTGGTTGCCGAAAAACTGCCGGAAAAAATACTGTAGTTGCAGGAAAATTTTCAAAG
TGCTCGGAATCAAAAAATAAAAATATGGAAGGCTCGGAATTGCAGGGCGATCAGACT
GTTCTAAAGAAGTTTTCTGAAAAAATGGACGGAACGGGCTCCACGCGCCGGCGCGTGG
AGTAGATCTTGCCGGCGAAAATTGTCTTCGGGCGGCGCGTGAGGCGGAGTCTGACGG
AGTTGTTTGCTGGGGTTTGGTCGCCGGAGGTTGGGGACCTTATGGTGGTGTTGGTTTT
TGCACAACACCGATGGAATTGGTTTTGACGAAAAAATAGCCCTAAAAGGTCACCGGGAT
GAAGCACGTCGACGACTGGGTTTTCATTCCCGGATGTTTTCTCACTGCCGCTCTGATAC
CATGTGAGAATGCACGGGAGAAAAATCTATTCTATTGATTAAAGTGTTGTACAACCCTAT
TTATATACAGTAATTACATAATAATAGGTATCTACTTCCCGATGTGGACACTAAACATG
ACTAACTACTTAACAGTGTTGAACATGGGTAACCCAGGGGGGTCTTCACCTAATTCCAA
AACTGAAGTGAAAAGAGGAAAAAGAAAGCAGGCTTGTGAGGTTCCGCGTTCCTCTCCA
TCTGATATGACCTTTCTATATATATATATTCTTTTAAATGATGCTTCCCGGCTAGCTTATG
CGCACCTCGATTATTCTATTTAGTACATGCTACCTCCCATCAGAACATGCACAAGGTAAC
TCTGTCCATCAAGGCTTAGGAAAATAGAAGAAATCACCTACTCTCTCCGTTCCAATTTAT
GTGATCCTGTTTGACTGGGCACAGAGTTTAAGAAAAAATGAAGACTTTTAGAATTTGTG
GTCCTAAACAAGTCAAAAAGGGGCCTAGATTATTTGTGTGGTTATAAAAGCTTCTCATTA
ATAGTAGAATTGTAAGTTTAAGCTAAATTGTTACCAAATTTAGAAATGGGTCATTCTTTTT
GAAACGGACCAAAAAGGAAATAGGTTCACATAAACTGGAACAGAGGGAGTAGTATTTTT
TGTTTCCATTGGGATTTGTGATTGAGATCTCATGGTTATGTATGCATATGTTGTGATGTA
GTTCCATATCTTTACTGTTTAGATGGTTGACAGGGAAATAGTAAGTTCTTTTTTAACTTAA
TTACGAAAATAAATTGTCTTCTTTTATTTAAGCTTATGTGACATTATTTCCTTTTTAGTTTG
CTTATAAAAGAATTAACCCTTTCTAAGTTTGGAGAACTAAATGTTCTCATTTTACGCTTAA
TGGCAAGCATTTATAGCCACACATTCGTTCTGCATATTTAAAACCTGAAGTTTCAAAAGT
CTTATTTAACCATAGCATGTTTAAGACTACAAGTTTCAAAAGTCATTCTTTTTTCCTTATTA
AACTTCCTGTGTAGTTAAACAAGGTCGCAATAAAATGAAATAGAGGGAGTACTATATTTA
AGAATGGTATCATACTTGGTAGTTTTTCTCTTCTCGTCTCTCCTTTTTTGGGTAGGGGAT
GCATCAAGCTGTAGGTTCAATTGTTTATAACTTTTAAATAGCTAAAGAACTTGCTCTAGT
AGTGATTTCGGGGGTAAGATAATCTTTGTGGTTAAGAATCCATTGAATATGGGAATAATA
AAAAAAAAAAAAGAAAAAGAATGCATTGGATTAGAGATCACAACCATTTTTAGCCGATAT
TGGCTGCGATGTGTTCTGGCTAATTTTTTCTTGAAAGGTAACAGAGGATCGGTTCTGGC
GAATTAACTAAGGTTCATTTTAAATATCACCCATATCCAACCTAGCCACCCATCTGTCAC
GTATAAAACTTATTGTGGACAAAAATAGAATGGTCACTTCCTTTTCCACCCACCGCTAGT
TGCGACGTGACAACAAGGAGTTGTAAATGTGATGTGATGGATTTTAGTTATAGCAGGAA
CTTAAATGATAATCTAGGAAGCTACAATTTTGCTATGCTTCAAACAACAGTCATATCTCG
TATTAAAACTCAGCAAGTCTTGCTTTTTTAGTAATATAGGTTTGCTAATATTTCAAAATCC
TATTTTATATTTTCTGCATGTATTGGATCTCTCATTGCTTTAAGATATAAGAAATGGTAAT
CTTAAACTATGTCTATGCCATCAGAAAGTGGATTACCCGCTGTCGTTGGATGTATATGAT
TTTTGTTCGGAAGACCTTCGCAAGAAACTGGAAGGTCCTCGCCAGGTACTGTCTTTTTC
CCATTGATCAATGTCTTTTAAGAAATGAGGAAAGACCAGACCCTCTTTGGCCCCTCTTTC
TCTTTCTTGTTCTGTTATTACATGACTCTAAATTTGCTGCTAGGTTTTGAGGGATGCTGA
AGGTAAGAAGGCCGGTTTAAAAACCAGTGAGAAAACTTCAAGTTCAACTGACGGCGAC
GTTAAAATGACTGAGGCTGAGGTATGAATTAATCTTTGTAATGTAGGAGTGACTTAAGG
GGATAAAGAGGGACCTTTCGGGCCCACCTATGGCGGATTGTACAAGGTGGTTTAAAGT
GGGAAATTAAGAATGTCTATAAGTAACTTCTGCCTTTTCTCCTTTTTTTTCCTATTGTTTA
TGCAGGAATCATCTAGTGGAAGTGGAGAAGCGTCTAAAACAACCCAAGAAGGTAGAGA
AACACCTCCTTTTCTTGATAACTTGATGACTTGATAAACATATGCTGCTGCTGTATTTTAA
TTGGTAACAATGTCTGGCATTAAAATTGTAATATTTGGGAGAGAAGTTATTGTCATGAAA
TTACCTTCCAACTCACATATCCTTTTCAATGCTTTAAATGAAACTCTGTTAGTTAATTGTC
 
AGATATAATTCTGCAGTAATATTGCGGTTCCATGAGGTTTTACAGTTCTTATGACAACAA
TGTTCTGCCCTGGGTATTCAAACTTCTTTTCACAAAGTCACTGTTAGTATCTTTGATTACA
AGCGATTGACCTTCTATTAACAATTTTGGGATCCCATAAAGATTATTAACTTGGATCAGA
TTTATTCCTTTTTAAATTACTATATGTCCCTAGACACCGGCGGACGTTTGCCAAGTTTCT
TTTGAAGGGGGCGCCTTTTAATTTTTAGAACTATGGAAGATCCTTAAGGTTTAGCTCTGC
TGATACAAGTACATTTTAATTTGTTTGGACATTTGTTTGTATGTAGAAAATGTAGAGAACC
TCTAGCATAGATAACCCCGTACTTGCCTTTGAATTTATATAACTACGAAAGATTCTAAAG
GTTTAGCTCTGCTGATACACTTACATTAAGTTGGTGTTTTCTCGGAGTCCTTTAATTTGTT
TAGACATTTATTTGTATGTGGAAAATGTAGAGAACCTCTAGCATTGGATAACCCCATAAA
TTGTCTTAAAAGAAAATTTTCTGAGTATTGGAATGAACTAGGGCCCAGATGGAGCAGAA
TGAATGTCGGGGATTAAATAAGGACTCTAACTGGTTCTGGATTGGGAGCAGTAGTTGT
GAATGATCAATTTTATCAACAGTTTAGTGTTTCTGATATATAAGGGGAAGTTAGTCTAAG
AGCTCAATTTTGGAATTCTGTTATGAATGAGGAGTCGCAATGATTAGTGCTTTTTTTTTTT
TAATGATGCCCGATTAGTAAACTCCATTTCAGGTGTTCTGCCTGAGAAGGAACACCACT
TGACTGGAATATATGATTTGGTGGCCGTGCTGACTCACAAGGGAAGAAGTGCTGACTCT
GGGCATTATGTTGCCTGGGTCAAGCAAGAAAACGGTCAGTTTAACTGGAAGAGATTTT
GTTCTAGTAATCGTTGCTCTTGGACTACCATCTGATACAATATATTGAAAATCTCTTTGTA
AACCACAGGAAAGTGGGTTCAATTTGATGATGACAATCCAATTCCGCAGAGAGAAGAG
GACATCCCTAAACTTTCAGGAGGTGGTAAGTGAATCACTTGTGTATTACGTCTTCGGCA
AATTTTCAAAGTCTGGCAAGCATATCCTTTCTTATAACAACAAGATGTAAAGCAGATGGA
ATATTTTGTTGCTTGTGTGCCTGAATGTGTTTTTCGTTCTGTCAGTTTATAGAAGTGCTTT
ATTTTTGGTTTCAGGTGATTGGCATATGGCTTATATTTGCATGTACAAGGCCCGTGTTGT
TCCCATG
SEQ 52 
CTATTTCACTTGATGCAAGGAAAATTGATTACTCCTGGCACTACGAAGAAATGTTTGGTT
AAAAGGACAAAGGTCAGTGCAAAAACCAGAATAGTTCATTGCTACACTAGGTCCAACTA
GCCAGATAGATGCACACAATTTGCGGCAGAGATAACTATAAGACACCACAGTGCTTAAC
TGCCTTTTAACCAAAGACCAAAAACACTCATGAAGAAGAAAAATGACAACCTTTTTATAG
CTATGGACTGTCCATTTCATACGATTCCAGTTGTTAATACTTTATGCATGAGTCAAATAG
AATTTGCATACAAAAAAATAGGTCCATTTGCAACAAATCCGAGTATAACTGAATGAACAG
ATGAGAGCCATTAAAACCTTAATGTCAAATCCTACAAAACAATTGGATCATCTCCACAAT
GCATGACGTAATTCATCTCATGCTGTAAATATATCAGTCTGTGGTTAATATGAAAGTTAT
AGATTAAAGATTCACAAGACACAAATGTGCCTCCTGAACTTCCTAGAGCAGCATATCAG
CTAAAGCAAAGAATAGAAATACACTAATACAGAACAGCAAAGAGAGACTTAATAGACCT
GAGTTCCATATTTTCAATGCGGATACGTTCAACATATAAGTCATGATCTTCTGCTTCACT
GTTGATCTCATCGTCTTTTTTGGGATCCTTTTTGCGCCGTGAACTTGATCTCCTCGAAAA
TAGTCTATCAATCCTGGCAGCATAAGAACCTAAGAGTGCTAAGACACAATGCATTCACT
AAAACATTGAAAAAAGGCCCAAAACAGAGGCAAAGTGGAGATGAACTTTAGAATTCTAT
CTTCACATTTTCACCAGAAGGGTTCAGAAAGTTATAGGAACTTCATTTCAAGTACAATGC
AAACAGTAAACAGGTTTTTACTATACTAAAGTATCTAAAAGTCACTTACCTTGTTTTTCTC
AAAATCCATGCCCAAGTGCGTCCAATAATCATGTCCACTTTTTCCATGAAAAAGTAATCA
GTAGTCCTATCAATTCCAATGCCGCGTCGAAAGATTACATACTGGAAAGAAAAGGTACG
TATATAGAGATTCAGTGGCATGATCAAACTCTTGACTCTCATCCTTTTCCCTCGAGAAAT
ATATGTTCATATTACACATTGATATACTTAATAAATTTCGAGGTAAAACATTGATATACCA
TATAAGTTGAACAGCTCAAATACAATATAATACATCCATGATACTATTTGCAGGTTCAAAA
AGATATAAGTGACAACACGTGCACAAGTCACTCTTTACCTTTGTTGAAGCTTGTGTGCA
CTATTACAGGTTTGAGGAGCAGACCTTGAACAATGATTAATCTTTTGAAAACTAGCAAGA
AGATGAGAGGATAAAGATTCCAGTTTGTGCTCAGTCATAACACACTTTTTTGAATCCGTG
 
GTGAAAATAAAAATCTTTCATCAAAGAAAAAGAAGAAGGGCCAGTAAGCACAATATAAC
TCTAAAGTTCTTCGCAATAAATGATCGCATACTTTTGCTTGTGTTTTATTGGAGTGCAAAT
TCCTTCTTGCCAAAATCATGGGCAGTAATGGACATCCTGACACCTAAGAGCTAATGTTG
TATAGGGACAGTGGTTACTTCAAGATTCACTTTAAACAGCCAATCATACTTGCTGCAAAG
CAACAATGAGATAGGGAAATAAATGGACACCATATGTGGATATCAAGGATTCAAGTCGC
ATTCTTGAACCCCAAACAATGCAAGGCACCAACAAAAAGCAGATTGGGAAATATTCAAT
TTGGTGAAAATTTCATAAATACAACCAGAAGCTACACTTGTCGACCTAGCATCTGGGAG
CCATTAACTTCATACCAAACGTTCCAGCAAAAGTAAAGGCCTCATTTCAAATTGTAACTT
GAAGCTACACTAAGAGATATACCTTGTCAGCAAACTCTGGAAGGTCCTCATGAGGATGC
TCTGCAAAATACTTCTCTAAAAGTTTTTGTCAAGCTGCATCCAGGAAAAGGTAGAGTGA
ATCGCATAGCGAACATGACAAAATGGAACTATATCAAGAGAGCCAATAATTGTCGGAAC
CAAATTATATGCGGAAGCAGCTGGTTAAGAACCTTAGATTCATCAACAGTGATTGGAAG
ATTTAGAAGATACTGTCCTGAATGTGCAACATCAATCTCTTCATCACTAGCTATTTTAAAA
TTGCTTTTGTGCATAATCTGCATCAGCATAAGAGAAATGATGATAGAAAGTGGAGAATG
AAATAAGGGTGCCAGAACACAAATGTCATACCCATTTCCCTTAATCCTCCAGCTAGATG
TGGAAAAGCATTCAGAATTCACTGCACTTAGAGTTTGATGCAAGTTTAAAATCCATTACA
GGCTTGGCCTCAATAGCATGATAATGCAGTGCAAAATAACGGAAAACGACTTTAGCGAT
AAATGAATAATATATAGCCGTTCCCCATGCTTGAAAATAAAGAAATATCCCCTTCCATTT
CGATTTTGCAACAAAACAGCATAAATATGCGAAACTTTCTTATTGTAAGGCTACTCCTTA
TGATTAAAAATGGGAAGCATGTGTTAGAAAAACAAAGTAAAAGAGAAAAAAACAGGA
AAATGAAGGACGAAGCAACTCCCACTCCCTGAAGACAAAAACCTCGACCTCTTACCTTT
CACCTCACTGGTTGTCTCCCTCCCCTGAGAAAAAACAATTATTCGCTATGTCCCAATTTA
TGTGATGCACTTTCCTTTTTAGTATGTCCCAAAAAGAATTATACCTTACTATATTTAAAAA
AAAATTAAAACTTTCCATTTTACCCTTAATGAGATAATCTATAGCCACAAAAATATCTATG
ACTTGTTTAGACCACAAGTTCCAAAAGCTTTCTTTCTTTCTTAAATTTTGTGCCCAGCTAA
ACAACATCACATAAAATGGGACGGAAGGAGTAGTTTTTCCTCTCAATTTAATCCAATAGA
ATTTTCTTCCATTTCAGTGGAGATCTTACACTTAATAACTGATGCAGGATTATATCTTTTG
TTATATTTTTATTTCCTAGGCTCGGGAAGGAGAGGATAGCATGTTTTCACCTCCGCTGG
GGATTTTCTTTTGAAGGTACATAAATGTCAGGGCCCTCATACAAGGGTTGTAGATCGAA
AGTATTCTAACTATCTTCTTTTTTTCGGGTAAATTGTTGAGCCATGGGTACACACATACA
CAAATATATAGAGAGAGATATCTTCATATAAGCAACTGCAGACTATAAATATGCACACAC
AGAAGACAGATATGAGATTGGATTTTTTATCAAATTTAAACTGGAACACCAATATCCCGA
CTAAAGATACGCGGATACAATAGAATATAGCAGCTGATGGTGTTACTGAAAGGTCCAAC
ACGGGTGCTCAAAGTCCACTTGGGCAAATGCGGCTAGGGAGTCAGAAAGAATGAGATG
GGCGACGGACTTGCCCTACTGTCAGAGCTAGTGGTGTGTTCTTGAGATGAAGGCTTAG
CATTCACCACCTTCCACGTTTCCGACTTTCTCTTATTTGAACTTTTTAGATCAGTCAACTT
GAGTAAGCAACTTGGGCCAAGGGATGAAACTCAAGTCTCTAGCTCTCAAGTATACTGAG
TAGGCAACTTGGGCTTGCATGTTATACATATTTTTTTAACTAGTATACTGAGTAGGCAAC
TGGGGCTTGCATGTTATATAATGAATCCAAACAGCCCATATAGCTGGCTCAGGTACCAG
AGGAAAACAAACTTTAGATGAAGCTGCAGATTTTTTACCTGAAACAAATATGTCAAGAAA
TTCTGTTCAAGAATGTCAATCTCTTCTGGAGATAACTTCTGTTGTTCCAATTTCTTAGCC
CCGTTCACAGGATCAAACAAAGAGTATAATTGCTGCAATCAGACAAATATTATTGATCAA
AACACAATATCAACATCAGCATATTATATTCATTTGCAGCAGACTGATGAGAAAAGATGT
CTGATCAAATAAAAAGTAGCATAAGATTATATTACCATGAGATCCTCAAATTGTAGAAGA
TACCAAGCATGAATTGTGTACTCAACCCTCTTGCAAAGCTTCAGAAATTCAGCCCGGTC
AGAACTATGTTCTGGGCAAGGAGGAAGGTCGGATCATTAGAACCTCAAAAAAGACTTTT
ACTTAAATAACCAATCATAAAAAAGCACTAAAGGGACTAGAAGCACAGATATTCATGCAG
CATTTTACAGTTTCAAGAAAAGTAGATTTTCTGAAGAAGAAAAAAAGAGTGGAAATGAGC
TGCCAAGACGCAGACATGATTTACGTATATCCAGAAGGAAAACTTGTTTCTGTTAAAAAT
TCTAAACATATAACACGTCAAAGCTTATCAGTGAAGGAAGGGTTTTGAAACCTTTTGGG
GATTTTGATCCATGACCATCAAGTGTCTCCGTAAAATTCACATGGAGGGTGTACAGGGT
 
CCCCCCTTCTTTTGAACTGGGGTCTCCTCCTTTTGAAATGTCCAGCACTAATTTACTGCG
TTGTTTTTACTGTGAGTAATACTGTTACTTATCTATCCAAAAAATTACATAGAGGGAAGTC
GTTTCAAAAGCTTACAATTTCTTGAAATCCATGAGGACTTGATGAAAAGATGGAAGCCTT
TCTATTTCGGTTAGAGTTTATATGCTCGAGTGACCGTGTAAGGAGAAGTGTAAGATTTA
GACAATTCACGGTTATAGAGAAACCCTTATTTGTCCTAAAAGACAATTTATTGAGTAGTG
GAATGAAACAAGCCAATGGAGCAGAATGATAATGAGAAAATCACATAACCAACTCCAAC
TAGTTATGAATCAGGGTGGAGTTGCAAAAGTAAAATCTCTATTAAGCATCTAATTTCTTA
AATTCAAGAGATTGTGAACTTCTGAGTTGAGTGAAAGTGACTTACTCTAAGAAAGTATTT
AATGATAAAACTCGCAATTGAGATAAGTCAAATGGTAAACCTTGGAAAATAATCTAATTT
AATCTGGGAAAATATCTAATTCACACAACAGTAAATCTAATTAATCTGGGAAAATAATCA
AGGTCAAATTGTCTGCTTTGTCACAATACAATTAGAGTGCCTCAGAAAACAACATTAAAT
TTTGCAAAATTAAACCATTAAGGGGGTTGCTGTTTACACGAAAAAAGCTAAAACTCAGAC
GTCACCAAAACTATGAGATCGTGTAAAGTTAGTAACATTTTAGGTTTAAATTCATGGCAC
GCAATAATTGCTTGACATTCAGGAGAAAGACAAAAAAGTGACTGCCGTTCCCTATAGAG
TCTAGCGTAGAAAGTTGTATTATTTGCTTCAAAGATCTCCTCTTGGAGAAAGAAGCATTG
TTTCCTTACACTGCCATTTTCTATGGTCTAGTATAGGAAGTTCTATTGTTTGCTTTAAAAG
TCTCCTCGGATTATAGCAGAGTTGTACTTGTGCTAGTCAAATTTGGTAGTTTTTCCACA
GAAGTAACAGAAAGTGAAAATATCAGAGATATTATCCAATTAAATTAGAGGAGAAGAATT
GTTTCAAAATTCAAACAACTGATCGACCACAAAATAGAGAGGGAAGAAAGAGTAGCCGG
TTGTTTGGGAGCATCCAATTTTTACCTATAAGGTCGGCGAGGGCCATGATGAGCCTGG
GTTTGAGGACTGGAATAACAGACTCGCGCTCCAAACGGATCACCTCTTTCTTCTTCTCC
AT
SEQ 53 
TCACCGGTTTGTGACAACTGGATTTCCGTTCGCATCAGACAAATGGATACTCTGGGTTG
ACTTGTTAGCGACAACAGGATTTCCAGACTCATCTAAATTAATTATAGCAACATCACCAG
GCTTGAGATCCCCAGAAAGGAAAGATTCACTCAGGAGATCTTCAACCATTTGAGTAACA
GCCCTCCTAAGAGGGCGTGCACCGTAGTTTCTGTCAAATCCTTGTTGGCATATAAGCTC
CATTACTGCTTCTGACACCTCCAAGCTTATTTCCAATGAAACAAGCCTAGCCCTCACCTC
CTGCAGCATCAGGTCTAGTATCTGGAGCATCTGAAAGGAAAAAACAAAGCAGTTACTC
GCAGTAGCCGACTGCTTTCTAAAGAGATGTGCCAGAATAAAAGATCACCCCAATGTACA
TTCAGTTATCAGATCAATGCAACTTTCCAAATCAAACAAGAGGTATATTATACGAACCTG
GGGCTTCTCTAGAGGACGGAATACTACTACTTCGTCTAGCCTATTCATCAACTCAGGGC
GGAAATATGTCTTGAGCTCTTCCATCACTATTGCTTTCATACCAGCATAGGAGGCTGCT
GATTCATCATCAGCAAGCAAGAAGCCAATAGTATTCTGTCTACCCTTTACTATGGCTGTA
GAACCCACATTAGAAGTCATCACTATCAGGGCATTCTTAAATGACACTCTTCTTCCCTGT
TGAATCAAGTTTCATAATTATCAACGCCAATCTTCCAAAACAAGGTTTACACCCCATATT
GTGGAACATTCTAGAACAATAAGCCAGATGTAAAAACGGATCTAACCTGAGAGTCTGTT
AGGTGACCATCTTCAAACAACTGAAGGAGAATATTGAATATGTCAGGATGAGCCTTTTC
AATCTCATCTAGCAGCACTACAGTGAAGGGCTTTCTTCTGATAGCTTCAGTAAGTGTTC
CTCCTTCTCCATAGCCTACATAACCAGGAGGCGATCCAATTAACTTGCTCACAGTATGC
CGCTCCATGTATTCACTCATATCCAATCTTAGCATGGCAGATTCCTGTGCAACACAAAAA
GATACTTCACTGAGTACATAGAAATTCACAAAACCAAGTGATTGTTAAACTGAAACAGAA
CCGAAGATAGCAAATATAACTCTAGCAATTTGATGATGAACTTCTAAGAAAAGTAGATGC
ATAACTCTTGATGATGAAATTCTTTTTGATAAAATCCTAGCCACAATTTTCATCTAGAAGG
TTGAACAGAGAATGTCGCTCAGATGAATTTGATTTGATTCAATCTCTGCACTGAAGTCAC
AAACCGTCTACGACCAAGAGATCCAAATTCAGTGTGATGACAAGATTAAAAGCAGCGCT
AACTATATGACTATGATCCATTATCAAAGCTCACTTAGTACTTTTCATTTATTTTGCCAAA
TTACGTGCCTCCACCATAGGTGATTAGAGACTCCTTCTGTTCTTTCTTTTGGGGCGGGC
 
TGAGAAGGGGTGGAAGGTGCAAGGATCTAGTAAATGTAACAGACTTATCAGATATATAT
ACAATGGTTGACTTCATTTCCCATTGAAATGGATGAAGGAATAATCTGATCCTGGCAACA
GGGAAAGAGATTTGAAATAAGCCAGTAATAGAGCACTACCTAGTACCTAGTATGTCTAA
ACTTGAAGTACTTATTAGTGCCCACCAATAATCAGAAGTCGTGCTCCAACAAGATTAATT
GGTGGTATTTCCAACGCTACATTTGACCATGCCGCAATAGCCATAGAAACCTAGAATGG
ACACACAGCACAGCCTCCGGTACCCTTTCCTTCTCTCCTTTTGTTTTTATTATGTGTGTC
CGACCACCTAAAACACAGTCACCACATCTTACTATCATAGTATAATACTTTCTTTACCCG
AATCACCACTACCAACAACATAGAAAATTCCCAAGAACACCAGAACCAAAAAAAGTCCT
GAGAACACCAGTACTATCACCAAAACCTATCCATTGTCACTGAAGTGGCTGGATTGCAT
AACTTCAGTGGCAAAACATGGTGACTGTCTGGTGAAACAATTAAGGCATCTAGAATGAA
AAGATGAAGCACATTTCTTATCTTACATAATAATTCTTCTCAAATTTAGACAAACTAAAAA
GAGCAAGATTGTGTTTGTGCAAATTATGCTGCCAGAACTCTTGGTCAACACGATTCAATT
TCAGAGTTCCACAATTTCTACTCAATTGCTTAATCTGGAGACGCATCTTTGGAGGAATAA
TGCAAAACAGCTCATTTTATTAATACTTACAGAACCAAAATAAGACGCTGCCAAAGCTTT
AGCTAGTTCAGATTTTCCAACTCCAGTAGGACCACAGAAGAGCATTGCCGAAATTGGTC
TATTTGGGTCCTTAAGACCAGTTCTAGATCTCTTAACAGCCCGACAAATGGCTGCAACA
GCCTCATCCTGACCAACAACCCTTTTTTAAGCTGCTCATCAAGACCAACCAGAAGCAT
TCTTTCATCAACAGTAAGCTGCTTAAGGGGAATGCCTGTCCAGAGTGAAGCAACTGCTG
CTATTTCCTCAGGTCCAACTACCGGAGGTCTAGAGTAAAAATCAAAGGATTTAAGTTAAT
TTACTTCTGATTTAAATTTATGACTTCAGACAATCTTTATTCTACTATAATGCCCTTGTAG
GAACATGGAGGCAGATACGTACTCATCTTCATCAGATGTAGAAGGTGATGCTGGCTGTA
AATGAAGTTCACTGCCATCATTCAAACGAGATGCATCATCATTTTCTGTCAGCTTGCTTG
CCAAGATCTGTTTATGGAGGGGGCACAGGTAATTGTATTTAGACCACATAAGGAAGTTC
AAATTTTTCAAGACGTGCAAAATCTAGAGAGTTTGTATAACATTGAGTACTATTACCACTT
CATGCATGGCTTGAACAGCTCTAATCTCCTGCCAATAATCACTTGGTGATTGTGAGAGT
ACAGATATCTGCTGTTCCTTTCTTCTTTTGTGAGCTTGCATACGAGATTTACTACCAGCC
TCATCAATAAGATCAATAGCTTTGTCAGGAAGATACCTATCCGGTATATATCTTGCTGAC
AGTTGCACAGCAGCATTTATGGCTTCCAAACTGTATATACACTTATGATGTGACTCATAT
TTCTCACGCAATCCCAACAGTATCTGGACAGCATCCGCCTATATAATTTAGGAAACAAG
AATATCTGTTAGGGCCTTAGACACCCACTTGAGCATACACATGCATAGAGTATTATTCAC
CTGACTTGGTTCATTAATCAAGACAGGCTGGAATCTTCGGGCAAAGGCCTTGTCCTTCT
CAATATGCAATCTGAACTCATCCATGGTGGTAGATGCAATACACTGTTACAAGAACAAAA
TTTAAGCGCAGAGTCCATAAAAGCCAACATTATGAATGCAACTACATGGAGTAATGCAT
GGAACCAGAATAGGAAATTACCTGCAGTTCGCCCCGCCCAAGTGCTGGCTTTAGCAAA
TTAGCAATGTCAAGACCAGAACCCTTATTTCCCCTTCCAACTGTACCAGCACCAACAAG
GATGTGGACCTCATCTATGAATAGAATGATATTGCCTGCAAATTATGTCACAAGCTAAGC
CAATGATCTAAGAATTTGACCAAATTTTACTTCCATTCTATGCTCACCTGACTTTTTGACC
TCCTTAATTAATGTAGTCACACGCCCCTCTAGTTCGCCCCTCTCCTTTGCACCTGAAATG
AGTAGGCCAATGTCTAAAGACATTACCCGCTTTTTCTGTGTATATAGCAAATAACCACTA
GTTAAGTAGGTGCAGCAGCTAGTGGAACAAGAGTATAGGAGATGCATATTAAATTACAA
AATAGTTCAAAGAATCCCTGGAAAAAAAATGAATTCATTGAAAAGCCCACCATTAAAAAT
GCAGGAATATTTCCCTCAGCAATGTTTATCGCCAGCCCTTCGGCTATCGCTGTTTTCCC
AACCCCAGCTTGACCAAGCAGAATAGGATTGTTTTTGGTTCGACGGCAGAGAATCTCGA
TAATTCGCTGAACTTCAATCTCTCTGCCAATTACTGGGTCTATAAGGCCCTCACTCACAC
GGGCAGTAAGATCTACACAGAATTGCTCCAGCGCATTTTTCTCTGCTGGAAATGAAATG
CATTTGAGACAGCGTGCCAAACCAAACACTAACAAGAGAGAGGAACTGGCCATACCTTT
TGCTTTCTCAGCGGATCTGTCGATAGTTATTTTTCCAGGAAAGGATTTCTCACGCGACC
TTTTGAATGAAATTGGCTCTCTACCATCTTTAGCAAGCTCTCCTTGAAGCCTGGAAACTG
CCTCAGCTGCCAAACGATTTACATTTACTCCTAACCTGAAATTAGGTCAGTCGACCATG
CAAATCTTAATTTCTTATATAAATAGCGTGTAATAGATGACAGAAAGATAATCATATTGCA
AGAGAGAAGGCATATTAAACTCAATCAGGAAGGTGAAGAAGGATCCTATTCCCAAGCCA
 
GTAGCATTGTAAGATAAATTATGCAGCAAAAAGGAAATCCGTAAGCATTTGTTTATTTAT
TAACAAGTACATCAGTATGCTTGGTTTAGAGCACAACATGTGCTCATGCATAGCGTTCC
ATCAGAAACAGAAATTGCTTATCAACAGGAGGTCAAAATATTAAGCTAGGGTTGTCAGG
CGTACAACTTGAAAAAGAATATGTACACATACATGTACACAACACAAGAAGACACCAAAA
AAAAAAGAGAGGCAGATACCATTCATAAGTTAAGAACAGAATACTAAGGTAACAACAAC
AATCCAGTGTATTCCCACAGAGGATAAGATGTACGTAGCCTTACCCCTACCCCGGAAAG
GCTGAGAGACTGTTTCCGGTAGACCCTTGGCAAACCAAAATATCTCTTGGTATTTTGTAT
ACGCTTGGGAGGGAAAACAACACCAAATGGAATGTAAAATATGGCAAGCAAGGATAAAT
GAGTGAAGGATAAATTCTACCAATAAGTTCATTGCTTCCTAGCAACAGTGGGAGACAAC
ATCTCATTCCTCCTCAATTGTAGAAGCTGTTTTGGACTCAAAATAAGCAACAATAGCTAA
TCTATTCATGATCCCAAGATTGTTTCAGCTTACCAAAAGCCTATTACTTTTGTTCTTAATA
ACCTCATCATCCACTACTAGAGGCAGATGTAGTGCTTGAGTTAAAGGTTCATCTAAACC
CATTAACTTTGGTTCAAACTTGTATGTATGTTAAAATATGCACCAAATAAGTACAAATAAT
ATATTTCAAACCAAGAATGGGCTGCGGAACGCATATTCAAATCGTTGATCTGCCTCTAA
CTACCTACTTGCTCTACCAAAAAAACTAGCAGTTCCTATACTGAAAACAAAACTACTTGC
CAAAAAAAGTTAAGAAAAAAAAAAGCATTCTTGACTGACTATCTGTAATACCTCTTGAGC
ACACGAGTGGCGTTACCATCATCAACAGTAAACAAACCAAAGGCCATATGCTCGGGAG
CAATAAAATTATGCCCCATGGTCCTTGAATACTCAACCGCAGCCTCAAAAACGCGCTTC
GTACTTGAAGAAAACGCCACATCAGTAGCCGACGTAGCAGAACCGGAGTCCTGAGAAG
CCAATTTTTCTTTATCATCCTCCACGTCATCATGCCATATGCTCCGAACAGCTTCGCGG
GCTTTATCAATTGTTATTCGAGAACCAAGGAATCCACCAGGGCTACGATCCTCTGCGAT
CAGACCCAGCAAAAGATGCTGTGTATACACCATATCTTTGCCCAAAGCCTTTGCTTCTTT
TTGAGAAAACATCACAGCTTTGATTGATCTCTCAGTAAATCTCTCGAACACTCCAGAGAC
AATATACAAAGAGCGCTTGATTTTACGAGGAATTGAGCTGCAGGGCCTATGAGAAAGG
GAAATTCCAAAAAGGGACGAAGTAGAACTGCTAGTACTACAAGCAGCGGTAGTGGCGG
TAGTAATAGTAATATGAGAGGAGGAAGAAGGGCAATATGGGAAAAGCGAAAACACGGT
TTGACATCTCTTGTGAGGGTACACAGAGCCATAGCGACGAAGCTGAGGATTGAAGCTG
ATTGTTGAGTTCACAGAAAGTGGAGAAGAACACGTTAATTCCAT
SEQ 54 
ATGAAGAATATCGAGCGTCTCGCAAATGTTGCTTTATTAGGTATGGTTTCTTTTGATTTT
GATTCATACATTATATCTTTTGATGATAGCTGAATTGCATAGTATACTTGTTTGATTTGTG
CTTGTGAAGTTCAGAAAGTAAAGTAAATCCTGTTTGATTTATAGCTTCGTTTTTTGCCCC
TTAGTTTGTGTTGGTTACTCATTCAGATCATTTTTCCGCTAGATAGATTGCTAAAGCTTTT
GATGCTAATTCTTTGTTGTTAATTGGAAGGAGGCTACCCTTCCAGGGTAGGGGTAAGAC
TACGTACATCTTATCCTCCCCAGACCCCACTCGTGGGAATTCACTGGGTTTATTGTTGTT
GTTGTTGTTAATTGGAAGGACTCAATGTAGGGAAAGGTGCTAATTATTGTGTAGTTGGA
ATTTGAGGTGTGGTTGATGGTTACCCTAAATATATCTATCCAGCATGTTGGAGTAGATTC
TATAGCGGTTGGAATGAATATTCAAATCCCCTTGGGCAGTCACATTACTACTGTTACCC
GCTTTCCTTTATGTCACAGTAGGTTCCACTTCCACAGTTCCAGTTCAATCGGTAGACAAA
GATGGTCATGTGGGTTCTTTATATCAGTTTTAGCATTTTCTTATATGTTGGATGTTTGTTT
CATCATATTGCCTTTTTGAGGACATTTCACTACGTAATAGCAGCTATGCCGTTCTTGGAA
ATTTACAATGTACGATTATTTGGTCATGGCAATTTCACATCACTTTCCAAATTTTATGTTG
ACGCAATTACCTTGAAACTCTTGCTTTTTTGGTGGATTTCAGGTTTGAGTCTGGCACCAC
TGGTGGTGAATGTGGATCCAAATGTAAATGTCATAGTAACAGCTTGCCTTACTGTCTTTG
TGGGATGCTACCGTTCTGTCAAGCCTACTCCACCTTCAGTATATCTTCTGTACTCCAAGT
TGCAGCTTCCCTTTTTCTTAGATCTGTTTTGATGTCACTTAAACATATTCTACTGCTGTTT
TCCAGGAAACAATGTCTAATGAACACGCAATGAGGTTCCCCTTGGTTGGAAGTGCAATG
CTCTTGTCATTGTTCTTGCTTTTTAAGTTCCTGTCAAAAGACCTGGTTAATGCCGTATTG
 
ACATGCTACTTCTTCGTTCTTGGCATTGCTGCACTTTCGTATGTTCTCTCCGTATGGATC
ATTCTGTGATGCTTAATATTTCTATAACAAGTTCTTGAATAGTAGTTTTTCTGTGGGTGT
ATTGGATGTCATCTCTTTCTTTGTGTCTTTGCAGGGCGACATTGTTACCTGCTATCAGAC
GATTCTTGCCCAAAAAGTGGAATGATGATCTCATAATATGGCACTTCCCATATTTCCGCT
GTAGGCACCACCTTTCTTGTCTCTTTTGAAATGCCAATTGATCCTTTAGAATCCTTGGGC
ATACAGATCTCATCTTAGTTATTTTGTTTCGTCTTTTTTCAGCTTTGGAGATTGAGTTCAC
AAGATCTCAGATTGTTGCCGCAATTCCTGGAACCATCTTCTGTGTTTGGTATGCTAAACA
GAAGCATTGGCTAGCTAACAACGTTTTGGGCCTTGCCTTTTGCATTCAGGTTTGTCGGC
ATATCCATCCAAGTTACATTCTCATTCTTCAGGATATCTCAAAATGAAAAGTTGTGTAAAA
TAGTATTATTAGTACAATGGTAATATACAATTTTGGATATTTCAAAGTGAAAAGAGTATCA
TATAAATTGGGATAGAGGAAGTACTAAGACACTTGAATGAAGAGATCATATTTCATCACT
AAAAAAGTTGCACTTATCTGTCCATACATGTTCTCGTAACCAAGCATGGTTGCTCTTTAA
TACCAGATGCAAAGGCTACCCGCCTTTATATCTAGCATTTAAATCCACGATAGCACTTGA
TGGCTTCCTCTTTAATTTGTTTGATAACTAGAATTCTCCCAGGAGTTGGCCTACATTTATT
AAACTATGGGAGTATAATAGGCCTCCTCTATCATGCTCCCACTAATATAGCGGCTCCTT
GTTAGTGATAGGGTTCTAACTCATGACGTGGACCCATATTCTGACATTGCGTCATTACAT
TGAACCGGAGCCCCAGGGGCTTACTATTTGTGATTTTCTATTTATATATACATTGAGTTA
ATGGAGATTTTTGCAAGGGAGAAAAGGTTTGATCCTCTCTTATGTCATGTCTACATCAAT
GATTGATATTGATTTTCCCATTGCGATTTTGATTTTCAGGGTATTGAAATGCTTTCACTTG
GATCATTTAAGACTGGCGCCATACTATTGGTAAGAAAGAAAATTTGTTTTCTAATTTCTAT
CTGTAATTATACATGGCTGACAGCTGTATTCTGTTTATGTGTTTCGCCTTAAACTACATAT
TGCTTGTCTTTTTGAATTTGATGCTAACCACATATCTCTTTATTCAAGCGGAAGAGAAT
TTCATGAAATGAGCTATTAATGATTGTTATTGTTGAGCTATTAATGATTTTACATACAAAA
ATACATAACATTTGCATGGATTATCCCTAATTGCAGAGTTTTTAGACATTTTTGAGGTATT
CTTTTATGTTGGCATTTTTGCTTGTTTATGCAACTATTTATATCCATTAACTTGTAGCTGA
TGTTGAATGCACATGGTTTTCGAGAATGCAGGCAGGACTTTTTGTGTATGACATCTTCT
GGGTCTTTTTTACCCCAGTGATGGTCAGTGTTGCCAAATCTTTTGATGCTCCTATCAAG
GTGTGCATACTGATTTTCTCATATAGCTATTTCTTTTGAATTTTCATTTCATGCCTTTATTA
GTTACAGAGTCCTGATTATAACTTCGCTTTCTCTGCAGCTTTTGTTCCCCACAGCAGATG
CTAAACGCCCCTTCTCAATGTTGGGTCTTGGAGACATAGTTATCCCCGGTATAACCTCC
ATTTGCGTGAAAACTCCATTCACTTTATGTGGTTAGAACAGAGAGGTTTAGCATTTTGCC
TAGCGGAGGGATCCTCCACCTCAAACCATGTGGTTTGGGGTTTGAGGCAGTAGGGAAA
CGGTGGGAAAAGCCACTGTTGGTCCCTGGAGGGGAAAAAATGGGGGTGGGTGGGGG
GATGAGGTTTACCATATTAAAAATGAAAAGCTTACTTTTTGTGAGTAGCTACTTGAATGT
ATTTTTCTGTTTCTTACACATGCTTATTATTAGCTTTTGCCATGATGCTGTATTTGTTTTCA
TTTTTCAACTTGTTTTTTGCTTGAATAGATACACTTGGTAACATTTGATCACTTCAATCAT
GCAGGTATTTTTGTTGCATTGGCCCTCCGCTTTGACGTTTCCAGAGGAAGGGGCCCC
AATACTTTAAGAGTGCATTTTTAGGATACACATTTGGTTTGGCTCTTACCATATTTGTTAT
GAACTGGTTTCAAGCTGCACAGGTTGGTGAATCAAAATAAAGCTTTTACACTTTATTTCT
CTTGCTAGAATTGCAGCGCCCTTATGTTTGACTTGGCCTTTGTTTTTCCAGCCTGCTCT
GCTATATATTGTTCCAGCAGTGATTGGATTCTTAGCCGTACACTGCATATGGAACGGGG
ACGTGAAGCCTGTACGTTTTTTCTTTTGACAATCTGTTTCAACTTCATCCACTTGCTAACT
TTACTGTTTATGTTCTTTATATGCTGTTTACTACCATTTTAGCTACACACTATTTGTAGATT
ATATTTTCTAGGAGTTAATAGATATGAGAAAATGCATCTTATGGTTTACCTTTAATTCATC
CAAAGAAAACATGCATGTCATGATTTGTTTGGAAACTATGGACAATAAGTTAAAGGTAGG
GAAGGGAAGTTTTCTCGTTTCTTCGAGTTGGAAGCAAAAAAGGACGATCAAGAACTTCT
TTTTCGCCTAACCTTTGATAGAGAGAGTACAAACCCAAACCTTCATTGCCTTTTCTAGTT
TATACGGATACAGAGTTAACGAAATTTTCGTTTATGGAAGTAAGATTGGGGTCTATTCTC
AAGTCGTAGAAAATTACATTTTCGTTTTGATGCTGAGTTTGTATTCTTGATTTTCTGTTGA
GCAGTTGTTGGAGTTCGACGAGGGAAAGACGAAAGGCGCTGAAGAAGCCGATGCCAA
AGAAAGCAAGAAGGTAGAA
SEQ 55 
CTAATTTTGTTTGAGATCTCTATAGTACTCTTCTAACCACTTTTGAATAATAGCCACTTCT
TGCTTCCTTTGCATGATCAACCAGCCAGGGTCATTCTTTGTCTCAGAACGAAAGTCAAC
ATGGTGTGCACCTAATTAAATAAGAAAAATTAAAACATGAAACTAGTAATAAAATAAATCA
ATGTCTCTAATCAACAGCAATCGTTTTTCATTATGCTTTAATTCAATGAATATCATGATAT
ACAAAAGCATATATTACACAAATAGCCGGTCATATAGCCGATGTACATAGATTATACAGT
AATTATATATAGTTATACACATTTTATATATGAATTATACATAAATTGTACATGCGCTAGTT
ATTTTTAATTTAAGAAATCAGATCAGTGGCTATTTGGGTTAACTCTTCGTACCTTTTTGAG
TTACGAGTGCCACAATGCTAGCTGATATATTTTTCAGCACACTGTCACAAAAAGAGAAA
GAAAAAAATCAATTTAATAACAATAAACAAAGACTTATTCAAGATTTAGAGTCTATATGAG
TTTATAATATAAGTCGAAAATAATAGATTCAATTAAAACAACAGGACTTTACCATCACAGA
TAGATATAGGACCACATTTAGTGGTTTTAATTGTATCTATCTATTGTTTTCCACTCCAATT
ATAAACTCATATGGACTCTAAATCTTGAATTTACGAGTCCTCACATTATTATATTTTTATTT
TTAAATTTTGAACTCACCCTCCTCTGCTCCATGGATCTTGCATTCCGTTAGAGAATATCA
TATTACTGCCAAATCTCTTAAGAACTTGCTCAATTCTCTGAAATATAAACATATTTTTTTTA
ATTTCTTATTTTCGTGAATAAAAAAATATGTAAAGAGAATTTTCATTTGTTAACAAAAAAA
GAATGTAAATTAGAAAACTTACATAGCCACCAAATTCAGTAGTGATCCAATGTGGTCGA
GGCTCTACTCCATATTTCTTTTTGCAATCTTCTTTGAATTCCTTGTAACTATAGGAAGATG
GAGGAAACATGCTTTCATTTGAACAAGTCATTGGCATAACCATCTCTGTACATGCCTTAC
TCAAAAACCATGGAAAGAAACACACTAAAATTAGGCATACATTAGACCAAGATTTCAAGA
ATCATTTAATTTTAATCACACCATAACCAAACTAAAGTTTAATATAAGTACCAGTGGCGAA
TCCAACAATGCATTTACGGATTCGATCGAACTTAGTATTTACAGTATAGAAAAATTTGTAT
ATACGACAACAACATATCAAGCCCACCAAGCAGGGGCGTACGCATGAATTTTTGTAAGT
GATGTCAAAATTTATAGAAGAACGGGAATTATAACTTTAATTATCATACTTCTAGACAAGA
AATAACTTTAATCTATTGGTTTTGTTGAGTCTTAAGTTAGAAAAAGTCCAGAATTAAATTC
TACTTCATGACAGTTTTAACCACATAGATTCTCTAAAATATTTGCAAAAAAAAAAAAAAA
AAAAAAAAAAAAAAAAAAAAAAAAAGGAAAGCAAAAGAAGCCTGTCAGTTCTCAAATTCAC
AACTTACAATTGAACAGAAACAACCTTCTTTTTTCGTNAGCAAAATCAAGCACATAACCA
ATGCATCAAGAAATAATTTATGTCAAGTGGTGTCATTTTTTTCTACTTATCTGCTTCTTCA
CAGTATTAACACATATATTTAGTAAAAAAATTCGACGAAGCGGTGTCGCGTGACACCGC
TTCGATACATCTGCATCCGCCCCTACCACCAAGTGGGATTTGGGGGGTGGGTAGGACA
TATGCAAACATTATCCCTACTTTTGTGAAGGAAAAAATATTATTTCCGGTAGACCCCCAA
CTCAAAGAAAGATAAAAAGAGATAGAAACAACAAATAGTAACAATCACAAGATAAGGAG
ATAAGGTGATAGAATAATGAGAGATAGAATAACATTTAATAGAAAAATCTGCGAATAAAA
AGCTACAAAAATACCATACTAAAAAAATACGTATAGATAGAGTTAGATATATTTTTCAAAA
TCTACACGTTTAAATTCTGAATCTGCCACTAATAAGTACAAAGAATAATAAATGGAATAG
AATAAAAGGTAATTAAGGTAAAGAATTCTAAACCTGCCAATCCCAACCACGAAGACCAT
GAGCATCATCACCACCTTCTAAATTGAAACATTTTTCTCTTTTTGTATAATTGTAATATAA
ACTTGCCGCAGCAAATGCCCGGCTGATTTTGGAAGCTCCTTTTGGTAATCCATCAATTA
TCTTGCACATCTTAATTTAAAAAAAAAAAAAAAGTTTAAGAATTTTTTCACTCGACAATATA
ATTTTTTTACACAATTAGTCATTTATAAGATAATTACAAATAAATTTTTATGATAAGTATTA
ATTGATGAACTGATAAAAATGATAAATAACCTGTTATAATATGTTAAACTACACTAATCAT
GTACAAAATCTCCACATTGTCATTGCATTAAGTCTTATGTACAAATGGTATAAAAAATATT
TATGCCAATCAGATAAATTATAAAGTACCTACAAGTAACCTTTGTGATAAACATTAATTGG
TAAATGGCTAAAAATTGTATCTAGCATGTCATATCAGATTAAACTATACCCACTTTTGTAA
AAATATTTATCCTGTCAATTAGGGGCGGACCTACGTGGGGAGGGGTCACTAGACCCCG
TCAGTCTCGACAAAAAAACTGTATATAAATTTCATATATATCTATATATACAGTAAAGACG
CCTTAAATACTTTATGCGCCCCCCTAAAAGCACAAAAACTGGACAGAGGCACTGGTTTG
TAGGAGTGCTTAATCCAAGTTCGAATCTATGCTCCTACAACTTATATTTTTTTATTTTATTT
 
TTAAAGTGGTGTCGCCGTAATACTCAAATCCCAGGTCCGCATCTGTTATCAATGTATATC
AAGTTCAACAAAAGTTGATCAGGGGCGAAGCTATATATCCCAAAGGGTAGTCAACTGAC
CACCCTTCCTCGAAAAATTTACTTTGCGTATATAGGTAACATATTAGGTTTTAGAGGTAT
ATAACATATATGAATACTCTTTATTAGAGAATTTTTCCACTTCTTTAAGTTTGAACACCC
TTGAGCCTTGAGAAAATTACTGATTTCGCCACTGATGCTGATAGAAAAAAAGGAAATGTA
ATTAAATGCAATGATTTTGAAACCAATTTAGTGCTAATTAAGCAGAATTGTCTACCTCTTG
TACTGGATATGCTGGCAATGGCATCATAAAGTTGGCTTTAGTAGGATAATTCACCATTG
CTGTATATACAAAAGCTTCCCATAGCCAATCTCTAGCTGAATAAACTGAATGTAAACCCC
TGAAATATAATATAAAACAAATCATTTTATTGAATCAAACTTGACCTTGTAATTTGCTACA
ATAAAAAATTATCATGTTTCTATTTTTATATTCAAATATACTTACTTGCAAGTTCTGAAAAG
TTTACTAACTTCAGTCAAGCCTTCTTCATGTTTTGATAAAGCATCCAACTCTGTCCAACTT
CCCTTTATCACTCTATAACAATTCAAGCTTACCTCCTATTCGTGAGGGCAAATAAATAAA
TAATAATAAGGTCTCTTATCCAAGAAAGATTTTTATGAATATATCCTATTCGAGATAAAAG
ATAATTACAGTAATCGCTCATAATAAAGTGAGATTAGTAATCTGCAAATAAGTCAAGTTA
CTTGTTACAACATGTTGAAAATATTGAGTGTAAAAATTTATTTACATTGTCAATAACGGTG
GATCCTGATGGGTTCATCCTCTAGAGTTCAAATAATTTAAGGGTTTGTTTGGCCATGATT
TTTTTTTTTACTTTTTTTTTGGAATCAGTGTTTGGCGATGAAAAATTCTAACATTTGAATTT
CTAAATTTTTTCGAATTTGAAAAACTTCAAAAAACTATTTTTCAAGATTTTCACTTCAAAAC
ACTTAAAAAAATTTAAAAACAACCCCAAATTATATTCATGTCCAAACACAATTCTAATTTT
AAAATACCATTTTCAACTTGAAAACAAAAATTACTTGTTTAAGGAATTTCACAATTCTTAT
GTCCAAACACCCACAAATTTGTTGCGCTTTTAGAAGTAGAGTTTCAAAATTTAATATTTAT
TAAAATTTACAATCTTTTTGCATTTCTTTATAAACCTAGTAAATAAGGCGATATTCGTCCC
AGTGTATATAAATTAAATCCTATTATAAAAGGAGCATCTAGGTGCATGAATGTGACCATG
TAGGATCAGAACATTTTAAACAAAGGGGCTTAATAGATTGTAAAGGCTTTACTATTACTA
ACGACAAGAGCGGGTTGCTCCAGTGGTGAGCACCCTCCACATTCAATCAAGAGGTTGT
GAATTCGAGTCACCCCAAAAGCAAGGTTAGGAGTTCTTCGAGGAAGGGAGCCGAGGGT
CTATCGGAAACAACCTCTCTATCCCAAGATAAAAGTAAAGTTTACGTACACACTACCCTC
CTCAGACCCTACTAGTAAAATTTTACCTGGTTATTATTGTTGTTGCTGCTTTACTATTACT
AACCTTGAAATCTTGGGAAACAGCATCATAAAAGCTTGACCATGGGGTGATTTTGTCAA
ACTGCAAGATTGGTGCTGAAGATGCCACTGCACCTATTGCTATATGTGGGTACTTCAAT
CTAAACCAAGAAGCCAACACTGGTTGACCAGAAAAAACAAAGTTAAAAGAGGAAAGGAA
AACAAGTATTTAAGAGTGAGAGCAATTTATTAATTATTGGATACTACTCGAGAAGAATGA
ACCTAAATAGCCGTTCACTCAACTGCTTAAACTAAAATAGCTGACGGATGTATAAAATAG
GAAAATTTTATTTTGTGTCTCAGACAACTTACACTAGTTGTATGAAGCATTTTTTTATCT
CACAGTTTTGTGGACCCGAATTTTTGTGGACCTAGAAGTGTAAGATTGGGGTCCACAAA
TTTATGAGACAACAAAGAGCCTCGCACAACTAATGTCAGTTGTGTGAGGCACACAATAA
AACTTCTCTATAAAATATATAATTCATACTCTTATATAATACACCATCCGGTCCACTTTCA
TTGATTTTTTGACTCTTTTCACATATATTAGAAAATCACATTTTAGCATTAATTCACAATGA
AATTGACCATATTAACCTTATTTTGTTCCTTGAAAATATAACAAATGCTCCTATGCTCTTT
ACTTCAAATGCAACTTTAAAAAAAAAATTAACTTATTCTTAATATCTGGAAAAAATCAAAT
ATTGTGGACCACAAAAAAAATTAAAAATTCAATTAAAATTGACCGGAGAGAGTATATGCA
TAACTATGTATAATCTATATATATCGGCTAGAAACAAACAGTAAATTGAACTGGCTATTTG
TGTAAAGATTCCTACTTAACAAATGCAAAAGTGGAAGAAAAGTTCTGTTTATTTGAATAAT
TGAATGCATCTAATGCTAATGCTAAATTCATACAAAAGAGAACTTTCCATGAACATTTAG
CAACCATAGAATGTAATTATCATTGATTCACATGGATTGGACACTCAATAAGTCAATATG
TCCACACATGTAATGTCATGTCATTTCCATCTATCATTATGTCAAGGCAAAAAATTAGCT
AAAAGTTAAAACTTTTTCACTTATATTATTACTTTTCTTTCATTACTTTTTTTTTGTTTGTTT
GTGTGGTGTTCTACTATATTAGTGGCAGTTTGGACATAAGAATTGTAAAATTTCAAAAAA
AAAAAAATTAACAAAATTTAAGTAAAAATAATATTTGAAAATTAGAGTTGTATTAGAATAT
GAACATAATTTAAAGCTGCTTTTGATTTTTTTTATGAATGATTTGAAATGAAAATTTTGAAA
AACAGCTTTTTGAAGTTTTTAAAATTTTCGAAAAATTCCAAAATTCAACTTCAAGTGAAAT
 
TTAAAATTTGCATGGCCAAACACTGATTTCGGGAAAAGTGAATGTTTTTTATGGCCAAAC
AGTTCCTTACTTACTTCCTCCATAAGAGCCACCAAAAACCACAACCGGTGATGATTCAG
AAGAAAGATTCTGCTTTAAACTCCTTATTAGAACAGCATAATCAGCCAATGCTTGCTGTG
AATTCAAGTATCCCAAAGTCTTTGGTGACTTGTAAGATTTCTTTCCAAATGGCATTGAAT
CCCCATAAAACCTATGCTAAATTATTACAATACAAAAAACCATTATCAATTTCATTCCCAA
CAAAGATAAATAATAATAATAATAAAATATAAAAAAGGTTCAATTTTACCTTTCTATAT
GAAGTATCTTAATATTACAATTCATTATACTTTGGGCCACTAATATCTTATTTTTGGAAAA
AATTCTTGTATTTGTCTTGATTCTAACGAAGTTCCAACTTGAAGTATAATAGATGGTAATT
TTAAATCATAGTGAATAGCTGGATAAATTTGGATTTTTTCTAGTAGTATTTTGATACGTAG
AATCTACCAAATCAATATTGGAGTTTCATTAAACGTAGTATAAATACGATTCGATTTAATA
ACGGCAAGAATATAAATAATCCCTTAAATAAAACGAAGTGTAAAACTAAAATACTTCGTA
TAGTACAACAACAATAAATTCAGTGTAATTTCACATGTGAAGTTTGGGGAGGATAGTGTG
TACGTAGATCTTACACATATCTTGGGAAGATAAAGAAGTTGTTTTCGATAGACCCTCGG
CTCAACGAATAGTGAAAACAAAGTAACAAACAGTAGCAACAACAACATAATATGAACAAA
AGGCAAAATACTTCGTATAGTATAGGAGTAAATTTAAATATTTTTCTCAAAAATAAATACT
TCAAATAAAAAAACATTTCAAGATTATATACATACTTCAATGAAGACTAGAAGAGCATGA
AACTTAGGAGCAATATCAAGCATAAATCCAGTATTTGCAGCAAACCAATCAATATTTCCT
TCATTTCCAGTGTAGACAAAGATAGGGCCTCCTTGTTTCCAATAATTATCATTTATGAGA
TATTTCTGTTTAAAAACTTTAGAACTCTTTGGTAGAAAAGTGAAATGGTCAAGAATTTGA
GGAAAGTAATGGACTTTAAATGGTATTTTTGACTTGACATGTTGTTTTTCTAATGAAGATT
GATAAGTTCCAGGTAGATAAATTGGCTTAATTTCTCCAACTACAAAAGAGATAATAAACA
GTAAAATCAAGAAAATGAAGAAAAATAAGAAGAAGAAAAAGCCAT
SEQ 56 
ATGTCTCGTTTCTCACTCCTATTGGCTCTCGTCGTCGCCGGTGGCCTTTTCGCCTCCGC
ACTCGCCGGACCGGCGACCTTTGCCGATGAGAATCCGATCAGACAAGTCGTTTCTGAC
GGTTTACATGAGCTGGAGAACGCAATTCTCCAAGTCGTCGGCAAGACCCGCCATGCTC
TCTCCTTCGCTCGCTTTGCTCACAGGTACGATGATCTCTACATGGAAATGAGATTTTTT
TTGTTATTTGCTTATTAATAGTAATTGTTTTATTTTGAGTTTAAGTTCTATATATGCAGTGA
GCATATTTTTTTTTTTACATAAATAAGATAATAACAAATAAATCACTTAATATGTATTAGTT
GGTAATGATAGTGTAAAAAAATATTATACTGTAATGTGTATATAACTTAAATCTTTTTATTT
TTGGGACGATATTTAAGGTATGGGAAGAGGTACGAGTCAGTTGAGGAGATAAAGCAAA
GGTTCGAGGTATTTTTGGACAATTTGAAGATGATTCGATCGCACAACAAGAAAGGACTA
TCATACAAACTCGGTGTCAATGGTATAATTAATATTATGGCATAACGCTAAGGCCCTGCT
CTTTTCCTTTTTCTCTTTTGCTTAAGTGGAGTCTTAATTTGTTGATTTGGAGGTAACAAG
TTATAGTTTTGTGGTTCCTTTACCGGAATACTCTTTGTTTTTATCTTCAGCTAAGGTAACA
GATTAAGGCGTAATTATAGTTATTATTGTAAAATAAGGTAATTTTTATTTAGAAGCTTCAA
AATTAAGTACAAGCAATTGAATACTACTCTTTGTAAGTAGACTTTGTATATATGTTTTTAT
TTCATTCTCTTTTTTCATTTGGAGAGATGTGGACAAATTAAAATTATAATATAATGCGATA
AAACATATGTTCCACTACAGTATCAGTATGGTATTTATAGTTTGCATATTTTATTAGTAAT
TAATTGGTCTAGTGCCTTATCATGTGTAGATATTTCATTCATATTGTGTGGCTAGTGGGT
ACCCTTTCTCTCTCCAATCAAAAAACTTTTTTAAAAGCTCAATTCAAAAGCTTTTCTTCA
TTACAACTGATCCTGCTTAAAGACTAAAAACAATCTAAATTGAATTCTTAATTCTTCTCTA
TTCATTCATATATGGACATAAAAACAAAATCACAGTACATGGAAAGAATATAAGCACCTA
AGCATTGGACTGCCCAAATGAAAAGTTTTTGCAACTTAATCTAGTTGTGCATAGATTCAA
CAACAAAAAGTAAAGAAATAAGTATGCATTTTATGCTTCTAAGTTCTAGTATATATGGCC
CTTATTGTTTATCGATTATTATGTTTCATGACAGAGTTTACCGACCTAACATGGACGAG
TTCCGGAGAGACAGGTTGGGGGCAGCTCAAAACTGTTCAGCCACCACAAAGGGCAATC
TCAAAGTCACTAACGTTGTTCTGCCGGAGACGGTATATGCACTCAGAACTCCTCTGTAT
 
CTATTTCTGGAGTTAGTGATCATTAGAGTTAAACTACTTTCTGATGATTTATTATTTCCAG
AATTGTGGAGTGCTCTGAGTTTAATTATGCTGTAACTATAGAAACACTAACTAAAAAGAT
CTTGAATAGGTATCCTACAACAATAAATAGAATCCTCATAAGAAATACCACTAGATCGAG
CACCAGTCATGATTTCATATCTGGTAAAAATCTTGGCTAATTGATCGAAGTGGAGTAGAC
TAGCGAGCATGTACTGAGCTAATGCACAATTGGTTGCAAAAAGAAGTTTTTTCTTTCCTA
ACCGAAATTTCCAATTTCGTAATTATAGAAAGACTGGCGGGAAGCTGGGATTGTCAGCC
CAGTCAAGAACCAGGGCAAGTGCGGATCTTGCTGGACATTCAGGTAAGAATTAGTTAG
AATCTCACATCATTGGACTCTTAAATTGTAAGTCTTGAAATTGCACTCTTAAGCTGAAATA
TAACGGAGAAGGCACTTGGCAGCACTACTGGTGCACTAGAAGCAGCATATAGCCAAGC
ATTTGGGAAGGGAATCTCTCTATCTGAGCAGCAGCTTGTGGACTGTGCTGGAGCTTTTA
ATAACTTTGGCTGCAATGGTGGGCTCCCATCACAAGCCTTTGAGTATATTAAATCCAAT
GGTGGTCTTGACACTGAAGAAGCATATCCATACACTGGCAAGAATGGCTTATGTAAATT
CTCATCAGAAAATGTTGGTGTCAAAGTCATCGATTCCGTCAATATTACCCTGGTATGATA
TCTCTTTCCTCCAGTATGCAACCAATCTTTGCCAGTGTTAATATCCAACCTTAATGGTCA
ATAAGGATTGGTTAAGTTCCTTACATACGTGTCATTACAGGGTGCTGAAGATGAACTAAA
ATACGCGGTTGCATTGGTTAGGCCCGTTAGTATAGCTTTTGAGGTGATAAAAGGTTTCA
AACAATACAAGAGTGGTGTTTACACCAGCACCGAATGCGGCAACACTCCCATGGTAAGT
CATCTGTCCCTAGGAACGTGATATGCAAATATATTGACATAGTTACCTAAATACAGGGG
AAAGCTACAGCCGACCAAGGGTCGTCAGTTGAACACCCTTCACTTCACTGTCGTGCATA
TATTAAATCTTGAACACCCTAAGTGAAATTTATAACTTCGCTAAATAGGCATATACACAAT
ATTACAAACATTGTGTGTTGCATTGGCAGGATGTAAACCATGCTGTTCTTGCTGTGGGT
TACGGTGTTGAAAATGGTGTTCCCTATTGGCTCATCAAGAATTCATGGGGAGCAGATTG
GGGTGACAATGGATACTTCAAAATGGAGATGGGAAAGAACATGTGTGGTATTGCCACTT
GCGCATCCTACCCTGTCGTTGCC
SEQ 57 
ATGGAGAAGGAACACAAATACTCTTTGTTTCTCACAAAGTTGAAGTTGTTTTTCTTGTTA
CATTAAGTACTTTCCATGGCCTTAGCCATGGCTTCCAAATGGATCAGGCACGTACATTA
ATGTCTTGGCGTCGTTCTAAAATGCATGCTCAGACAACTACTTATGCTACTAATGAGGAT
GAGACAGAAAACTTAGTATTTTCCGATGAAAAACATGTCGGAAATATGGAGGATGATCT
TATTAAAGATGGTCTTCCAGCGCAGCCTTCAAATGTGATGTTTAAGCAATATGCAGGATA
TGTTAATGTTGATGTAAAGAATGGAAGAAGCCTTTTCTATTACTTTGCTGAAGCTTCTTC
TGGAAATGCTTCTTCAAAACCTCTTGTTCTTTGGCTAAATGGAGGTAAATTATATGTGTT
GATGATTCTTTCTCAACTTAATTTTGTCTTACTAATTACTCATCTTCTCTTAATTCTTTTGT
CATGCACCTAATTTGATTAAGTACTCATTTGTTTTGTTTCGATTTAATCTAACTTACCCTT
TATGCACATATATTCTGCATCAAATTAAGTTGAATATTACTCCACGTGTTCCACATTATAT
ACTTAACATTTTTTTTTCCAATCTATTTTACACATTTTATATATTTGAATACTTTTTTAACT
TTAGACATTTCAATTTACCCTTAATAGTATATTCTTGTAGCCGATCAAATATCTATGAGAT
ATTTTTGAAGTCTTTTTTCTTCCTAAATCAAGTCAAATGTTAATGTATAAAATAAAGCAGA
GGGAGCAATAACTTTCGTTTTATGTTTGTAATTTTTCTTAATAGTGATACTCATTACTCTC
CCCGGTCCACAATAAGTGACTATTTTACTTTTTTATTTTGGTCAAAAATAAGTATCCATTT
ACCTAATCAATAAGGAATTAATTTTATTTTTCTAAAATTTACCCTTATTTACATATTCCAAC
GTGTCAAGGAAATAATTAATTAAGGTTAATTTAGTGAATATATTTTTTTTCTCTAAGAGTT
CGTATTTCTTTAATGGATGTGCCAACTATAAAATGGTCACTTATTAGGGACCAGAAGAGT
AACTCTTTATTATTTGAAATTTTGATTTCCCAAAAGGTATAAATGGTCCGAGGAACATCTA
ATTTTCGCTCATTTGCAAGAAAGTGGTTCATAGACAAATGGAGTTATTAAGTGGGGACG
CGCAACAGAGAATTATTGGTCACTTTATTCGTTTTGTTCGCTCTTTTTCTTTCTTTACTTC
TTTACTAAAGTAGAAGAGAAAAAAAGGAAGTTTAAAAAATTGTTAGTGTATATGAAGATA
AGAGCTGTCATTTTCTTCGGCTATTGAATGACGAATAAAAGCACAATTGGGTACAGGTC
 
CAGGATGTTCATCATTAGGATTCGGGGCCATGCTAGAGCTTGGGCCTTTTGGTGTAAAC
CCTGATGGTAAAACCCTTTATTCCAGAAGATTTGCATGGAACAAAGGTACATTTCATTTG
CTAAACTAATATAGACCTACTTATAATTAATGAAACTAATTTCTCAAGAAATAAGACAACT
ATTTTTTGTAATAACGTATACTCTATCTTTTTCAATTTATGTGACAACATTATGTTAAAGGT
GTCACGTGAGTAGGAAGCCAGCTGACACTTAGTAGGCAAAGAGTCTGTTAGATTAGTTG
TTAATTATACAATTAGAAATTAGTTAGAATCAGTTGGATTACATTGTATATGTATGTGTAT
AGACGGTTATTCAATACAACAGTAATTTTCTCATCTTCTCTTTTCCTCTCTAAGCTGCGAT
CTCTCTTAGCTCAATCTAGAAGCATCCACGACAGATGTTTGGCATGGTATCAGAGCTTT
GTGCGATCATTGCTCTCGTCTAATTCTCCTCTGAGTTCATGTGAACGAAACTTCAACTCG
TTCGTCTTCATCTCCTTCCCTCGAGAACATCGAATCGACGATGACGACGGAAAAAATTG
ACCACATTCATCCTCTGTTTGTGCATCCCTCAGATACTCCAAGTTTCATGTTGATTCCAG
TCCAACTCACTGGATCTGAGAATTACGAATTATGGCGGAGATCGATGAAAATTGCACTT
AGGCAAAACGAAAGTTAGGGTTCGTCAATGGCACACCCACTAAGGATCAGTTTAGGTCA
GAGCTACATGAAGACTGGGAGACATGTAATGCGATTGTGCTCTCGTGGATTATGAACAC
AGTATCTCCAAATTTACGTACTTAGTGGAATTGTGTATGCTTTTAATGCTCACCTAGTAT
GAGAAGATCTAAAGGAGATGTTTGATAAGGTGAATACGATGAGGATCTTTCAATTTCATA
GAGAAATTGCTACAATTTTCCAACGAACAGATTCAGTGTCCATGTATTTTATAAAATTGA
AGGAGCTCTGGCTGAGTATGATGCAATGGTACCCTCAACAAATTCGAAGTAGTATGCTG
ATCATCTTCAGCGGCAGAGGCTATTACAATTTCTAAGTGGACTGAATGATTCCTATGCTC
AAGCTAGAAGACAGATTCTAATGAAATCAGTAGAACCTACTTTGAATCGGCCTTATGCTC
CAATTGTTGAAGACGGAAGTCAAATGAGTACATCGGGAACTTTATCACACATTGGGCTG
AACTCAATAGCCGAGGAAAATGACATTACAACATTGTGGAGCTCAGCAGTAAAATGAGG
TTCAATCAAGAAGAACAAAAGGAATTACAGTATATTTTGTGAACATTGCAAGATGAAAGG
ACATAGTAAAGAAAATTAGTACCAGCTCATTGGTTATCCGACAGACTTTAAAGATAGAAG
AAAACAAGGAGCACCTACTGGTTACCAATGAGCACCTATTGGTCACCAAGGAACAATTG
AGGAAGATGCAATGCAAGGGAAACAGGTATGACTGTAGATTTTGGGAATCTTTATGCAG
GCATATCATATGGGGACAACAACATATGCAGGTGCAAAGGCAAGGGACTCATAATCCT
GTACACATGGAAGATGCTCAATCTCAGGGACAATCTTAGGGATATACAGGTGGTGTCAC
TGTTATATTTACTCCGGAACAGTATAGTCAAATCTTACAAATGCTCAACAAAGATTATGTT
CCAGAAACATCAGCTAATATGGCAGGTACTATTTGTTCTTTTCTGGCTAGTAAAACCGG
GCACAATTGGATAATGGACATAGGAGCAACAGATCATATGGTATCTACTCCTTAAATGTT
ATTTGATTTGAATGACTATGCTAAGCAAGGCTCACTGTTGCATTTACCTGATGGAAAAAG
TTGCCTATTAGTTATGTTGGTAAATGTAGATTGGCACAAGGGGACATCAGGGATGTGTT
GTGTGTACCAGACTTCAAGTTTAACTTGTTGTCAGTGGCTAAACTAACTAGAGAATGCA
GTGTTTCATGTCTTTCTATCTTGATTTTTTTCTGATGCAGGACCTTCACATTGGGAAGGT
GAAAGGGACTGATAGAATGCACAATGACTTGTACTATTGGAGAAATAATATAGAGAATA
AGATACCACAATCATTGGCTACTACTTTGACTCAATCTGCAGCATTGTGGCATAAGAGG
TTGGGGCATGTTCATCATAGAATACTACAACAAATGAACTTTTTTAAAGATATCAAGACA
AATACTGGCAGAACTTGTTCTATATGTCCTTTAGCTAAGCAAACTAGGCTTTCTTTTCCT
CAAAGTACTAGTGGAACTACTACACTGTTTGAGCTAGTTCATGGTGATGTATGGGGTCC
ATACAATGTACCTACATATGGTGGTCATAGATTCTTTCTTACACTTGTAGACGATTGTAG
CAGGATGGTCTGGGTTTTCTTGTTAAGGTTGAAGAGTGATGTCTCATTTGTATTAAAAGA
TTTTATGTCATTAATAAAGACACAGTTTTATAGTTCAATCAAGGTTTTCAGAAGTGATAAT
GGTACAGAGTTATTTAACTCACATTGTATAGATTTGTTCAGTGGTGCATGAATTGTACAT
CAAAACTCATGTGTTCATACTCCACAGCAGAATGAAGTTGTTGAACGAAAGCACATATAA
TTTTTTGAGGTAGGAAGAGGTTTCAGGGTTGCATTCCTCTAACTTTCTGGGGATTATGT
GTTCAGAATGCTGCGTATCTGATTAACAGGATTCCATCCACTACTGTGGCAAGAAAGTC
ACCATTTGAGGCATTCTATAGGAGGAGTCCTAACCTACAACACCTAAGGGGTGCTTATG
TTATGCCATAAGTGTGGGTGCCAAAAGTGACAAATTTGGAGCAAAAGCAATCCCAACAG
TGCATATGGGATACTCTACCACTCAGAAAGGCTATAGGTTGTATAACACAGCCAATAAA
CTGATCTTTGTCAGCAGGGATGTTTCATTTAGAGAAGATATATTTCCCTTCAAGTCCTCC
 
TACTATCAACTTAGACCACCTAATCTTGTGGAGTATTGGAATGGTCGCCATGATCCCTTT
GTTCTTGAAACTACTATTGATGCAGCTCCATTGGAGACTTCATCTATAGTTGAGCCAGTC
TTTGTCCCCTCTAGTCCTTCTATTCCTACTTCTTTGAATTTAGGAGACTCTACAGCTGGT
GTCTCTGAGAATGCTACTACTGTATCAGTCCCTGCTGCTAGTACTGATTCTCTCATTCTT
AGTAAGGCTCCTTATGATAATGTAGCAGATATTACTGTTGCTCCAGATTCTTGAGAGCTT
ACAGTCACAAGAAAGTCATGCAGAACCTCCAAGACTCCTAGTTGGCTTAGTGACTATGT
TCATAAGGGGTCCAAGCCTCTATCACATGCTGTAATGGGCACAAGTTATCCTTTATCAG
TATATATGTCATATCCTTCACTTTCAGACCCCTATTACAAGGTCATTTATAGCATCTCATC
TGTGAGGGAGCCTGATACTCATGAAGAAGCTCTTTATGATCCACAGTGGGTAGTAGCTA
TGCAACAAGAACTGCAAGCCTTTCAAGACAATCACACTTGACAGCTGGTTAATATACCT
CCTGAAAAGAGAGTCATTGGTTGTAAATGAGTATTCAAAGTCAAATACAATGCTAAAGGT
GAGGTGGATAGATACAAAGTTCGTTTGGTAGCCAAGGGATATACTCAGCAGGAGGGGT
TGGATTACTAAGAGACTTTTTCTCCTGTGGATAAGATGGTCACTGTGAGGACTATCTTAT
CCTTGGCTGCAATGCATGGTTGAAGGTTGCATCAAATGGATATATTCAATGCATTCCTC
CAGGGTGATCTTGTAGAGGATGTTTACATGGTTCTACCTCCTGGTCTTCTAGGACATGG
GGGGGAGGGNNNNNAGGGGGGGGGGATGTAGGAGAGTATGCAAGCTACATAAGTCTA
TGTGTGGCTTGAAACAAGCCTCTCGACAGTGAAATCTTAAGCTTTGTGAGGCACTTCTC
TCCTCAGGCTTTATTCTAAGTCATCATGACTAGTCCCTCTTCACTCAAAGATCAGGGAAT
GAGCTGTTCCTCATCCTAGTTTATGTGGATAACCTCCTCATCACATGTTCTTCTCCTTCT
CTCATTCATGCAGCTAACTCATGCTCCATCAGCATTTCAAGATCAAGGATCTGGGGGAG
ATGAGATACTTTCTTGGTCTTGAAATTGCAAAGAGCACAATGGAATACTAGTATGTTAAA
GAAAGTTTGCACTAGCTCCTTATTGCAGACTTATGAGTGGCTGCTTCTAAGCCTACTAG
CATACCTACGGAGGTCAATCAAAGGTTCACTAGTGAATAATTTGATCACAACTATAAGAC
TGAGGGCAATACTGATGAGTTGTTGTCCGATCCTACTGGCTATCAGAAACTAGTAGGGA
AGCTGCTATACCTAACAATGACTCGACCAGATATAAGATACACAGTGCAGAACCTGAGT
CAATTTATGCATAAACCAAAGAGATCACACGTGGAAGGGGCTCTAAGGGTGGTGAAGT
ACTTAAAGAATGCACCTGGTTTGGGCATCTTGTTACCTTCTAAGCCATCCTCACAACTTA
CAGTCTACCGTGATGCAGACTAGGCCAATTGTCCCATGACAAGAAGGTTAGTTAGTGG
CTTCATAGTCAAGCTGGGAGACTCCTTGATTTCTTGAAAATCAAAGAAGCAAAGTACAG
TGTCAAGAAGTTCAGCAGAGGCATAATACAGAAGTATGGCCAATGCAATTGCAGAAATA
GTTTGGCTCATTAGACTGTGTGAGGAACTGAAGGTGAAGCTGGAGTTGCCTGTTAAACT
ATATTGTGATAGCAAGGGAGCACTTCAAATTGCTGCTAATCCTATCTATCATGAACGAAC
GAAGCACATAGAAATCGACCGTCACTTCATTAGGGAAAAGATACATGAGGGCATTATAC
ACACAGAACATGTGTCCACAAGTTTGCAGCTGGCAGATATTCTAACTAAAGGTTTAGGA
AAGGCGCAATATGACTTCCTATTATCCAAGCTAGGAATGTTCAATTTGTTCATATTACAT
AGCTTGAGGGGGAGTGTTAAAGGTGTCACATGAGTAGGAAGCCAGATGACACTTAGCT
GGTAAAGAGTGTTAGATTAGTTGCTAATTATACAATTAGAAGTTAGTTAGAGTCAGTTGG
ATTACACTTTATATGTATGTGTATAGACGGTTATTCAATACAATAGTGAAAATAATTTTCT
CATCTTCTCTTTTCCTCTCTAAGTTGCGATCTCTCTTAGCTTAATCTAGAAGCATCCATG
ACAGATGTTTGACACATTACTATTTGGGGAGCCAAAGAGGTTCTTCTTTATCATGTGTTT
TCTTAAATGTTTTATAAATATTTTGAATTATAATTTTTTTTATGAATTATAGTACTTTTTATG
TAAAAAAAATGAATTTGTATCTAAATTTACGGTGTAAAGTAAGCTAGCGTTTGGCCATAG
ATTCCCAAATTTGTTCTGAAAAATCTGATTTGGGTGAAGTTTGGTTTGGAGATGAAAATG
CGTTTGGACATCAGTTTTCAAAACATATTTCCCAAATTTATTTTGGAAAAACATGAAATAT
GATTTATACCCACAAGTTCTAAAAACTATCACAAATACCCAACAGTACCATTATCAATAA
CATTCATTAAAAAACTTTGATTCTCGTAAAAACTTTGATTATCAATCACAAATATCCAAAT
TTATTTTGGCAAAATCTATGGTCAAACGGGTATTAAGAACTTTGATTCTCGTGCTATGTA
CCTTGCCCGGAATGGAATTGTACGGGTATTAGAAAAATACATTGTGCTGCCAAATGCAT
TGTACAATAACTATAACTACTTATAGTTATTGTATGTTTTTTCTTTCTTTTATTTATTTACAT
ATGTAATGATGGTATACAGTTGCGAATGTGATGTTTCTGGAGTCGCCGGCAGGGGTTG
GGTTCTCTTATTCCAACACTACCTCGGACTATTCAAAGTCAGGCGATAAGAGGACTGGT
 
ACACACCGAAAAATCTCGTTAATACAATAGTAATAATTGTCAGTTTCATTATTATTTTTTA
AAACAATTTTAACAGTCAAAATGATGAAATTTTACTCTTTCATTTAACTCCTCAACTTCAA
TTTCAACTTCACATGCTCTATTCGTCAACACTCAACTCCAATCAAACATTGTGCAAACAG
TTATATTATTATCGTTTGTAGTCTGTAACTATTTTTTAATTTTTTTTAAAGACTACACTTGA
GTATCGTTAAAAACATGGTCAAATCTTTTGGTCACTTAAAGTGAGGCAGAGGTGGTGTC
TTTTCTTATCAAACGAGATTTTTCATTTTTTTATTTATCATTAATTCAGTTATATATTTATTT
CTTTTCCTTAAACTATATTCTTTTTTATTGGGTGACAGCTGAAGATGCATATAGGTTTCTA
GTGAATTGGTTCAAGAGGTTTCCACATTACAAAGGCAGGGATTTCTACATCATGGGAGA
AAGCTATGCAGGTATCTAGTACAGTATCAGTAATTAACAAACGAAAAAATACAAAACAAA
AACTTTTGATATTCTTGACTTATCCTTCTAGTAGTGAGAGCCCTCATTATTGAGTTCTGTC
CAATAAAATTTGTAAGAATTAAGGACCACTGTATCAGAGACAGCTTGTGCATATTTCAGA
CCATTCACAGAAATGTCCTCCTGTACAGTCTCAGCTCAAAGCCGAAATGAGCATTCTGA
TTGAAAATTTTGGCTATATTTGTAATACAATCTTATATAAGTAGTCTATATCTAAAGTCTAA
TATTGACATGACAGCTAATATTGCTGTGACTGCTCATCACAGGATTCTACGTACCAGAG
CTAGCAGATATCATTGTCAAGAGGAACATGTTGCCTACCACAAACTTCTACATCCAATTC
AAAGGAATCATGGTATTATATCATTTAATTTGTTGACCTTTTAATTTGTTTGATCTCTCTG
TTATCAAATCTTACTTGTATACCTAGTGATGAGGGGCGGATTTAGGGGTGCAAGGGTGT
TCACCCGAATCCCTTCGCCGAAAAATTACACGGTATATATAAGAAAAAGTCTGATATTTA
CCTTTATATATTATGTTTTGAATTTCCTTTACACAGCCCAAAAGTCTACTCTATGTCATGA
CATAAATTATTTCTTTATATTGCAGATAGGGAATGGTATAATGAATGATGAAACAGACGA
GAAAGGGACATTGGATTATTTATGGAGTCATGCACTAATCTCAGACGAGACTCATCGAG
GTCTCCTACAACACTGCAAAACGGAGACCGAAACATGCCAACATTTTCAGAACATAGCA
GAGGCTGAGTTGGGAAACGTCGATCCTTACAACATCTATGGTCCCCAATGTTCCATTAA
TTCAAAGAGCAGATCTTCTTCTCCGAAACTGAAGAATGGATATGATCCTTGCGAACAAC
AATACGTTCAGAATTATCTCAATCTTCCTCATGTGCAGAAGGCCTTGCATGCTAACCTCA
CTAACCTTCCTTATCTTTGGAACCCATGCAGGTAATCCAACTAAGTAAATATTATGTATA
GCATATCGATTTAACTTATATATACCGATAGTATAAACAATTTTTACACTGTCGTTGTATA
TGTATTGATTTATATATATACACTGTTAGTGTAAAATGTGTTGTAACAAATAATCTATGTT
ATTTTTCTATTTATAAATTAAATTCTACTTTTTATAAATAATAACTTGTACTTATCTTTTTGG
TCACCTGATAGAAACTCTTTTATATCATCCAATGTGTATTTAAATCTGTTGCGGCAATGAT
ATTTCTTATTTTCAAGATTACAAAATCTCACTCTTTATGTTTAGTTTATGTCACTTTTAATA
TGTAGAAGGTAATTGAACTCATATAAAAAATAGTGTATATGATATGATATGATGATTTTTT
TTCTTTTTTTTTTTTCATTTGGTATGGTAGCAATTTGGATTGGAAGGATACTCCAGCAACC
ATGTTTCCGATATACAAGAGACTTATTGCATCTGGTCTACGTATACTTCTTTACAGGTAA
CTTTATTATGGGCTTATCTTAGACTTTGGTTTATGTTCATGATACAATATTTTTAATTGTTC
GAATAAAGAACAAGTGGATTTGTATTGTTTGGAAACAGTGGAGATGTTGATGCAGTAGT
TTCAGTTACTTCAACTCGCTATAGCCTTAGTGCTATGAACCTTAAGGTGATCAAACCTTG
GCGTCCTTGGCTTGATGACACACAAGAAGTACGTTCTTCGAATATATTTTTTAATGATAA
TTTTATATATTTGTGGTGAGAAATAAATCTTATTGTTTCGTTCTTTGTTTTTTTTTTATAATT
TAAAGGTAGTTTGTATAATTTCTGCAGGTAGCTGGATATATGGTGGTTTATGATGGATTA
GCTTTCGCAACAGTTAGGGGAGCAGGGCACCAAGTTCCACAATTTCAACCACGTCGAG
CTTTTGCTTTGTTGAATATGTTCTTTGCCAATCATTCT
SEQ 58 
ATGGCTAATTCTTATACAAGTATTAATTTTTTCCTTGCCCCTATTATTTTCTTGGCGATTC
TGGGATTGCAGTTGCAGAGCAGCGATGGTTTTGGGACATTCGGGTTTGATATCCATCAC
CGGTATTCGGATCCGGTGAAGGGTATTTTGGACCTTCATGGATTGCCTGAGAAGGGCA
GTGTTGAGTATTATTCAGCTTGGACTCAGCGTGATCGCTTTATCAAGGGTCGCCGCCTT
GCTGAAGCTGATACAGCTAATTCCACTCCCCTCTCTTTTTCAGGAGGGAATGAAACTTT
 
CCGCCTCAGTTCTTTGGATTGTAAGCTTCCCTCTATGCATTTTTCTGATTGCTTTTTGC
ACTTGTCTATATCTTTATTGTTTACTTTTTCTAGTCATATACATAGATTATATACTAATTAT
ACATAATTATACATATATAATACAAAAATTATACCTTTTAAGTGGTTGGGTGGGCGGCTA
TTTGGGTTAATTCTTCTTCTTTTTTTGTATGTGTGTTTTGTATCTGTGTTATTATTCCTGAT
TGTGAACTAGTACGTCTTTGGAAATTCTTGTTTACTGTCTTTTCCTTTTGTCTGTTTAGTG
TGATGTTAGAGTTGACTGAGCTTTACGTTTGTTTTTGTCTGTTTAGTTGGATGGCAGTTC
AGGAAAAATAGGTTACCTAACTTAAATAAGTTCCATGTGCCATTTTTAACGAGATTCAAG
TGGAGAAAATATGAAGAAGAAAAAGAATGATTTAGGCCTGTTCTGTTCTATAATTCTGTT
TGTGTGTTTGATTGGACTGGAATTTTGTCGATTTAACTACTACATAAAATACTGACTCTTA
ATTTGATTTTACTTTTCTTCTATTTCGAATTCCAAGCTCCGGAAATAAATTCCGTTCTTTTT
TCTGATTTTCCTCTCCTCTGCCGCCACTTAACTCCTCTCCAGACAAGGAATTGTTCTGAA
GTTTCTGGCAGTAGCATGTTGTAATTTATGTGTTATAAAGATAGAGTTGCAAAATCTGTA
GTATCTGTAGTTGTGATTTTTTCTTCTTAAGGTGTGTGACTAAATATTCTTTGGCAATTTG
CAGTTTGCATTATGCAAATGTGACAGTGGGCACTCCTGGACTATCATTTCTAGTGGCAC
TTGACACTGGCAGTGACTTGTTTTGGCTACCCTGTGATTGCAGCAATTGTGTGCGTGCC
CTCGAGACACGCTCTGGACGAGTATGTTTGCTTCATTCTAGTACCTTTTTCTTTCTACTT
TCAAATGTTTAAAGAGTTTTTCTTTTTTTTGATCGTCATCCTCGTCTGTATATTGCCTTCT
GCTACAAGGAAGTTGTGCATACTTCTCTTCCTTTTGTAATTATGAGACTTTCTGATAACC
TTTTTCAGAAAGGAACCTGCTGATAACACAATGGCTGAATCTGAAACACAGTGGATTTCT
CTTCAACTGTCTTTTTCGGTCATTATGACAATAATATATTCTCTTAGTTAACAAGATATGG
GGTAGAGAATGTATTGAGGAAATTGTTTTTCTGTTAAGGAAGATACATAACTAGCGCAAA
AAAGAAGATTTAAACATAATCAATATTTGCAAAGTGAGTCTGATGCATGTAATATACTGA
CTCTGAAATGAAATTTCTGATCCATATTGTTCCGTGGCTTGTTTGTCCTTGAAGAATTTT
GAGATTCTTACTAGCTCAAGTACTTCAACTTGTCACGACCCAAAAATCCCACCACAGGC
GTCGTGATGGCACCTAGTCTCTAAAACTAGGTAAGCCGATTTCAATTACATTTTGGAG
CCATTTTTTTTTTAATTAAATAAGTAACCAAAACTAACAGCGGAACAAATATGAATGTACA
ATCTCCCAAGACTGGTAGTACTAAGTCACGAACTCTAACTGAATACATGGAATGATCAC
GAGGACCGAATATACAATACTGTTTGATTAAAAACTCCACAGGAGTTCACCTTGAAGAA
CAAAATTTTCTTTGCTCTTTTGCCTTTTCCTTTTAATGTTTCTGCATGTATTATTTGACACT
TGTAATCTTTTGTTTGCTTTTGAAACAGCGAATAAATCTCAATATTTACAGCCCTAATACG
TCGTCAACGGGTCAGATTGTTCCTTGCAACAGCACTCTGTGTGGACAAGGAGACGAT
GCTTATCTTCACAAAATGCATGTGCTTATGGAGTTGCATATCTCTCCAATAACACCTCAT
CATCAGGGGTACTGGTGGAAGACATCTTGCACTTAGAGACAGATAATGCTCAACAAAAA
AGTGTTGAGGCTCCAATTGCTCTGGGGTGGGTATGCTTTAGTTTTTCTCTTTATCTTTG
GAAGAGATTATCTTTGGATCTTCTGATGCATTTCTTTATCCGCCATGATTTTTTATATTCT
ACTTGTTCAATTTCAGGTGTGGGATAAGACAAACTGGTGCATTTTTAAGTGGCGCAGCT
CCTAATGGTCTATTCGGACTTGGCTTGGAAAATATATCTGTTCCGAGCATGTTAGCAAG
TAAAGGTCTTGCTGCAAATTCTTTCTCCATGTGCTTTGGGCCTGATGGTATTGGAAGAAT
AGTCTTTGGAGATAAAGGGAGTCCAGCCCAAGGAGAAACACCACTCAATCTTGATCAAC
TACAGTAAGCAAGTCACTTTGATATTCTGGGTTTATCGGTTGCTTCTGTTTCTGGCTTGA
TTTAGGAGAATGCGACTGAATATTTATTAACTCTTACCCTTTCCTGAATTGCAGCCCAAC
TTATAACATCAGCTTGACAGGAATAACAGTGGGAAACAAGATCACTGATGTTGATTTCAC
AGCCATTTTTGACTCTGGCACTTCATTCACATACTTGAATGACCCAGCTTACAAAGTCAT
TACAGAGAACGTGAGCGACAAGCTGACTGTATGATTTTAAGTTGGAGTTTGTAACTTTG
TATTGTAAAACTGAAGATATTTTTTTTCTTTTTTCAGTTTGATTCTCAAGCAAAACAGCCA
CGTATTCAACCTGATGGCGAAATTCCTTTTGAATACTGCTACGGGCTAAGGTGAACCAT
CTTTTATAATCTTCATCATTTATTACTTTCTTGACGTCCTTTGAACTCTCAGGATTAACAT
GCTACATACGCAGTGCAAATCAAACTACCTTCGAAGTTCCTGATGTAAATTTGACAATGA
AAGGCGGCAACCAATTATTTCTTTTTGATCCGATAATAATGCTCTCGCTCCAGGTAAGAT
GGTTTCTGCTCCTTTTATATTACAAAAGTTCTCTTTTAGAATATCCTAATATCCAGTGATG
ATCATCAGGATCGTTCTGGCGCATATTGCTTAGCTGTTGTGAAAAGTGGGGATGTCAAC
 
ATCATTGGACGTAAGTATCTATCAGTTGCTTGCTCGTAAGATTTTGTTTCTATCCATGGA
ATTCTGCAATATAACTTGCACCATGCCAGCTAATGATCTCACAATTACCAACTTTTAGAA
GTTTTGGTTCCTATCGAGTTTTTTACATACTTCTAGCTTATGTATAATTGGAAATGTGAAT
GTGACAAAGTAAATTAGTAAAAACCAACTAGTAAAACTGGTTCCATTGTCAAAAGTCTGA
GCTATTTGTTGATTTACTTGGATTTTGTCTCTCTATTTGGAATTCATGACAGAAAACTAAT
ACACGGATGTTTTTGCAGAAAATTTTATGACAGGCTATCGCGTGGTTTTCGATCGGGAG
AAGATGGTTTTGGGTTGGAAACCATCGGATTGTGAGTTCGCATTCCTGAGTATGACCTC
TTTAGTGTGCACACCTGCTCATATAATTTAACTATAAACCTTTCTTGGCAGGTTATGATTC
TAGAGGATCCAACGACAAATCGACAACTCTGCCAGTGAACAAGCGTAATTCTACTGAAG
CGCCTTCGCCCTCCAGTGTGGTGCCAGAGGCCACCAAGGGAAATGGAAGTGGAAATG
AACCCGCTACTTCGTTTCCATCTGTTCAATCATCTAAACCTGCAGCAAACCAAGCACCA
GCACATTTCATTTGCCAACTTATGATGGCTCTGTTTTCCCTTTTTAGCTATTATTTGATCA
TTATTTCTTCA
SEQ 59 
ATGGCGATTCATACTTCCACTCTCTCCATCTCCATACTTGTAATGCTCATGTTCTCCGTC
GTATCATCATCGGCGGCGGAGGACATGTCCATTATAAGCTACAACGAAAAACATCACAC
GAACGGCGAGTCAACGGTCTGGCGAACAGACGATGAAGTCATGTCTTTATATGAATCTT
GGCTAGTTGAACATAAGAAAGTGTACAACGCCTTAGGAGAAAAGGACAAACGGTTTCAG
ATCTTTAAAGATAACCTTAGATACATCGATGAACATAACTCTGTGCCCGATAAAAGTTAC
AAGCTGGGTTTGACCCAGTTTGCAGATTTGACCAACGAGGAGTACAAGTCCATCTACTT
GGGTACTAAGCCCGATGGTCGTAGCAGGTTGTTAAATACCCAAAGTGACCGTTATGCC
CCTAAGGTCGGAGATAGTTTGCCGGATTCCGTTGACTGGAGGAAGAAAGGTGTTCTTG
TTGACGTCAAAAATCAAGGGCAATGTGGTATTTTCCTTTTACCCTCTGCCTTGACTCTGC
ACCTGTTGTTTTTGTTTTCCTTTTTGTTCGTACTTATTTTCTGTTTAAAGTTTGTCCATGCT
TTCTTTACTGATGGCTTTGATGGAAATTTGGAAACTTTAGTAGTTTGATAAGGTAAGATA
TTAAAATAATCACAGAGTCATGAGTTTTAATCTAAGATCAATTTTAATGGCAAGTTCAGTT
GACCCTGCATTATTGTAAATTTTAGCTTAACATTAAGTATGATTAATTAGGTCAGCACGA
TGAAGTTGACAACTTTTGCTCCAATTTCCGCATCTAATTGTGGCAATATAAGTAATGCTT
TTTTCCCTTGGACAAAACACTAGTTTCCGGAATTGAGCTATTTTATTCAATTTAAAATGAA
AATTTTCTGTTTTAATGTATTAGAACTATAAAGAAACCGAAACATTAAGTAAACTTCGGAT
TGATCTGTGTTTTTCGGGAATTTAGTTGTTAGTGGTCTAATTTTCGGTTTAAATGCAGTT
CTTAATATTGGATAGGCATTTTGGCACTTTTCTTGGCTGTCGCTTCTCTTACCTTAAAATT
AAAATTATGGAGTACCTACCAAGTTCAAGATCTTATGGTTGTAAATTGAATTTGTAAAAG
GGGTTCTTCTTCGTTTGCTCTGAGATCCTTCTTTTAGCTCGCTCCTTAAATATTTACTAAT
CAGTGGTTTGTAGCTCCAACCGAGTGTCTATCGGAAACAAACTCTTTACCCTTCTAGGG
TAGGGGTAAGGCTGCGTCACTTGTGTGAACTCACTGGGTTTGTTGTTGGTCTGTAGTCC
GATATACCCCCATCAAACACCCTTGGAGTTGTTTCACTATGTCTAGTTGTGTCAATTGTT
TTGGCAAATTATGCAGCCTTGATTGATTGGATTATCTTCCATTTTATGCATAAGTAAATG
CTGAGGAAAAAATGATATGTTTATATCACATAAAGCAACTAATAATTTTCTTCGTAATTGG
TGTTGCAATTGGGAAATGAAACAGGGAGTTGTTGGGCTTTCTCAGCAGTTGCTTCAATT
GAAGCAGTAAACAAGATAGTGACAGGTAATCTGATCTCGTTATCTGAACAAGAGCTGGT
AGATTGTGATACGTCCGATAACCAAGGCTGTCAAGGGGGTCTAATGGACGATGCCTTTA
AATTCGTCATTCAAAATGGAGGAATAGACACTGAGGAAGATTATCCTTACAAAGCCAAA
GATGGAAAATGCGACCAAGCAAGGGTCAGTATGGTGTTCTCTGTCTTAAAGGATTATA
GGAAATGAACTAAATACAAGTTGTGACTATTAATATTTTGTTTGCAGAAAAATGCCAGGG
TTGTCACCATCGACGGGTATGAAGATGTTCCTGATAATGATGAAAAGGCACTGAAAAAG
GCCGTTGCTGGTCAACCCGTCAGCGTTGCTATCGAAGCTGGTGGCAAAGACTTCCAGC
ACTATAAATCGGTATTACTTCAGATTTGCCTATTGTCAGTAAAGTTGTTTTCTTTTAATCG
 
AATTAGCTAGTGTTTACACAGGCTCAACAAATATTTCTGTATTTTCAAAGTTACAGTGAG
TTCAGTATTAAAATTTTTAAATGTTGATCCTATTAAGTTTAAATGTTGGATCCGCCTATGC
CCCAGGGTATCTTTACCGGAAAATGTGGTGCAGCAGTGGACCATGGTGTGGTTGCAGT
AGGGTATGGTAGTGAAAATGGCATGGATTATTGGATTGTGAGGAACTCGTGGGGTGCT
TCGTGGGGTGAAAAGGGCTACCTCAGGATGCAGCGAAACATTGGCAACCCCAAGGGTT
TGTGTGGTATTGCTACGATTGCTTCTTACCCTGTAAAGACAGGCCAAAACCCTCCAAAA
CCAGCTCCATCTCCTCCACCAGTCAAGCCGCCCACTCAATGTGATGATTATAACGAATG
CCCAGCTGGAACGACGTGCTGCTGTGTCTACGAGTACTATAAATACTGCTTTGCTTGGG
GTTGTTGTCCCATGGAAGGAGCTACTTGCTGTAAAGACCATAACAGTTGCTGCCCACAT
GATTATCCTGTCTGCAATGTTAAAGCAGGCACCTGCTCAATTGTAAGTGATCTCTGCTT
GTTATTGTTAGATTGTCCCGCATTGGTTGAGGGGAAGTGTTGTTGTCTCCTTATATAGTC
TTCGGCAAGTCTTTTTAACAGTTAAGGTTGTTTCCTTTACTTATGGAATCATGTTTTTGTT
GATACAGAGCAAGAACAACCCACTAGGAGTCAAAGCAATGCAGCACATTCTGGCCAAA
CCTATTGGTACCTTCGGAAATGAGGGAAAGAAGAGCCCTTCTTCT
SEQ 60 
CTAAGCACTTTCTGCAAATCCAATTTGTGAACTGCCAAAGTCGAAAACTGTGTGATATGC
TCTCAAGAATGCATCTCCAAGAACCCTGCAAAAGTAGAGTTGAATATATCATACAACTG
GATCTTCATGAATATATAATATATTATAACTTATGGCAGTGAAAATAGTCTTACCAGAGG
GGACGTCGCGGATGCGCGTTTAAAGTTGTAAATCCACTAATACAGTGGACACCTTGGCT
GTCATCAACTCTGATAACATACTATTACACAGAAAGGAAGATAGTAAGTGGAAGAAGAG
AAAGCACTGCTATTTAAAACTATAATATGTTACTAATATACGGCCTAAAAACAAGACGCT
AACGGCTTTTCCAATGTACCTTACTGGAAAACCAGTTCAAGTTGTCGAAGCAATTTTAAT
CTACATTGCTCCGATCCTCCAAAAATGCTAACCGCACTCATGTTGGATCCTCCAAAAAG
TGCAATGAGATTTTTGCGGGATCCCAGCAATCAGTGGCGAATCCAGGATTTGAATTTTA
TGGGTTCAATCTTTAAGATTTTTAGTATTGAACTCATTGTATTTTGAAGTTATTGCTTCAG
TACTACTATTTATTAGATTTGACTGAACCCGGTACTAATATGATGCATCTGCCTCTGCCA
GCAACATAAATTTTAATGGGAAGATAAGAACTTTTCTCCGATATTATGTCATCTCCGATA
AGAACAGATGTAATTCTAGCACCAGTTGCTACCTTTAACACATATTGATAATGGAGGAAT
AGTATCAGATAACAACAAACCTGATCTGGAGAAAGGGGAAAAGATTTGTCTCCAATGGT
AAATGTTATATGTGGCAGGGCAAAGACATCACAGTTGATAAATGATTTTCCCCCGGGAT
TCGGAAGCTTCTCACACAGCTTCAGCACAAATTACATGTATTAGTAGTAAGTAACTAATG
AGAACTCGAAAAACAAAAAGACACAACTGTGACATGAATACCTGATTGGCATATTGAAA
CGCTTTTTCTTTTGATCTCTCTTTTCTGATCTCTACTTGTATCCAGAACACTATCATCTCA
CAAGAAGAACATAACGACCCATTATTTGTACAGAGTCCAATTCTGTTGCATACGTTCTCC
GGTTGTAACTGCATGCAGCCAAACAAAAGGATGACATCTAAATAAGAGAACACCTAAAA
TACCCGCACACAACAATTAGAAGTTAAAAAGCAGCTACCCCTGCTATCAAGCGTTCCCA
GATCGAATCCCCATAACTCGAGACAACTTTTTTGCATTCCAAACTAATAATTCCTTCCGC
TCCAATGGCATGATTTATTTGAGTTAAAATAGTCTGAAATGGAGATGGCTGAGAAGAAAT
TGATCTTGCAAAAAAGAATTAAAATCAACAATGGTTTAAATACAGACAGTTGGACCAGCG
ATAAATGATGTCCCTGTATCCACAATAGCTGGACATCCATCCTTACAAAGGCCTGAAAG
GGAAAGAGCAGAATTACACTAGAAATGCATTGTTTTTATATAGTAAATCACTTATGTATAT
GAAGATATTACCTGTTGAATTGCTTCCTATAAAAAGATCCCCTATCTCAATCTGGAATTTT
TGAAAAAAAACTCAGTTTTTTCAGGTTACCAAGTAAACAATACAAATAAAGTTCCATTAAA
CGGAGGCGCAGGTTACCTCCCAATAACCATTTTGAGCGACTGGTACGTATGTATGCTG
ACCCCTGAAGTGAGTCCAATCCATGCCTCCAAAGATAATTTCACCCGCTATCTTAGACG
TAGGATCTCGATTTAGCCAGAATGAGAAGATTGACTTGGTAACCATATGCTGAAGCAAC
ATGTTATACCTGAAATTAACTCACACAAAAGAATGTGGATTTCAATTTCAATGACACAGG
TAAGAAATGAATGAGAGAACAAGCTCATTAAAACTATGTCAGAAAGCATAAATTACTATG
 
TCAAAAGTTATTATAATATGAAGAGATAATAATTTACCATACTGGTGTGACATTCCTTGAT
GTCGTGCTCTGATCAAATCCAAGTCCTAGTACTCCATCAAATCGTGCACGCAACAATGT
CAAGTATCCCTCCCGTGTTACCTCAGTGAAAACCTGTTTAATAAATTTTATTCAACATGT
AACTTGAAAACATATATATCTACAATTTCAGCTGCAAAGACCGGCACCTGCTGCTTTAAG
ACAGCACCTCCAACTTTCACATTGTCTTGGCTGAAGAATCCATGAACTGAACCAGTGCC
AAAAGGGATTTTGCTAGACTTTCCTATCATAAAAGCCCATGTATAGAAGTGATAAGTATT
TCGATTAAGCTATTCTACGCTTAAATATCAACAATGCTCTAACCAAAATAAAGGTTTGGA
GGTCCATTGGGTAACTACCAATTTTTGTATACGTATTTGATAGTCTTGATTTGTACCTGG
AACGAAGATAACATGCAATCTGCATTGGATAAATCCATTCAAAAGTTAGTTAGTTTCATG
TAGTGAAAATTGTTAATCCACAACTTGACAAAGACTAACCGAGAAGAAACATCTGGAAG
AAGGGACCCAAAGATTGGAACTTCCAGTATCAAACACAACAATGAAGCGTTGGGGCGG
TGAACCAATACCAATCTCCGCGAAGTACTGAACATCATGATAATTTTTGAGGTAAACTAT
CTGGTCATTCGGAGCAGCCAAATTTCTATTGCGACCCCTGAGATCTTTAGCGTAGATTC
TTGCATCGCTTATGCTAGAAAGGTCCAACGATTGCCTTTTTAGCTCAATCCTAACCATAT
CATCAGCATATACGTTGATGCAGGTTATATACCATATTACAAGTGATGCAAGAAGGATTT
TGATCTCCAT
SEQ 61 
ATGGCGTCAATTTTCGCTCTTTCATTATTTTTCATTATTATCTCTTTCTGCATCACTTCGA
TCACCATTCCCGTTCAATCCGACGGTCACGAAACTTTCATCATTCACGTTTCTAAATCCG
ATAAGCCCCGTGTGTTCGCCACCCACCACCATTGGTACTCCTCCATCATCCGATCCGTT
TCTCAACACCCTTCTAAAATCCTCTACACCTACTCACGCGCTGCCGTGGGCTTCTCCGC
CCGCCTCACCGCCGCGCAGGCCGATCAGCTCCGCCGTATTCCCGGCGTAATCTCCGT
CCTTCCCGACGAAGTACGCCACCTCCACACCACCCATACCCCTACCTTCTTAGGCCTTG
CTGACTCCTTCGGCCTTTGGCCCAACTCCGATTACGCCGATGACGTCATCATCGGAGTT
CTGGACACAGGTATATGGCCGGAAAGACCGAGTTTTTCCGATGAGGGTCTCTCTCCTG
TTCCTTCAAGTTGGAAAGGAAGTGCGCTACTGGACCGGATTTTCCTGAAACCTCATGT
AATAAAAAAATCATAGGTGCCCAAATGTTTTACAAAGGCTATGAAGCTTCACATGGCCCA
ATGGATGAATCAAAAGAATCGAAATCGCCAAGAGATACTGAAGGACATGGAACACACAC
AGCATCAACTGCAGCTGGTTCTGTAGTGGCAAATGCTAGCTTTTATCAATATGCCAAAG
GTGAAGCTAGAGGTATGGCTATAAAAGCAAGAATAGCTGCTTACAAGATTTGCTGGAAA
AATGGTTGTTTTAATTCTGATATATTGGCTGCCATGGATCAAGCTGTTAACGATGGTGTG
CATGTGATTTCACTTTCCGTTGGGGCTAACGGTTATGCTCCACATTATCTCCTTGATTCT
ATTGCAATTGGAGCTTTTGGTGCATCTGAACATGGCGTCCTCGTCTCATGTTCAGCTGG
AAATTCTGGTCCCGGCGCTTATACGGCAGTGAACATTGCCCCCTGGATTCTCACCGTTG
GTGCTTCAACTATAGATCGTGAGTTCCCTGCAGATGTTATTCTAGGAGATAATAGAATAT
TTGGTGGCGTATCATTGTACTCCGGCGATCCATTGACCGATGCCAAATTGCCGGTGGTT
TATTCCGGCGACTGTGGTAGCAAATACTGTTATCCAGGAAAGCTAGACCATAAAAAGT
CGCTGGAAAAATTGTTTTGTGCGATAGGGGAGGCAACGCTAGGGTTGAAAAAGGGAGT
GCAGTGAAGCAGGCAGGCGGAGTAGGGATGATACTCCTTAATTTGGCCGACTCCGGTG
AAGAGCTCGTCGCCGATTCACATCTTCTCCCCGCGACGATGGTAGGTCAAAAAGCAGG
AGACAAAATAAGACACTACGTAAAGTCTGATCCTTCACCGACGGCGACGATCGTGTTCA
GAGGAACCGTGATCGGAAAATCACCGGCGGCGCCACGTGTAGCGGCGTTCTCGAGCA
GGGGACCGAATCATTTGACGCCGGAGATTCTCAAACCGGATGTTATTGCACCTGGAGT
TAACATTTTGGCCGGTTGGACCGGATCTGTTGGACCGACCGATTTGGATATTGACACGA
GAAGAGTGGAATTTAATATTATTTCTGGAACTTCCATGTCGTGCCCTCACGCTAGTGGA
TTGGCTGCGTTACTTAAAAGGGCCCACCCTAAATGGACCCCAGCAGCGGTAAAGTCAG
CACTCATGACAACAGCTTACAATTTGGACAATTCTGGTAAAGTATTTACAGATCTTGCCA
CTGGCCAAGAATCTACTCCTTTCGTTCATGGATCAGGTCATGTAGACCCGAACCGAGCA
 
TTGGATCCGGGTTTGGTTTACGATATCGAAACTAGCGATTACGTGAATTTCCTATGCTC
CATTGGCTATGACGGCGACGATGTCGCCGTGTTCGTGAGAGATTCTTCTCGAGTGAATT
GCAGTGAACAGAATTTGGCTACTCCAGGAGACCTGAATTACCCGTCGTTCTCTGTTGTT
TTTACCGGTGAGAGTAACGGTGTGGTTAAATACAAGCGGGTGATGAAAAATGTAGGGA
AAAATACAGATGCTGTTTATGAAGTGAAGGTGAACGCGCCGTCGTCTGTGGAGGTGAG
TGTGTCGCCGGCGAAGCTTGTATTCAGTGAGGAAAAGAAAAGCTTGTCGTATGAGATTA
GCTTTAAGAGTAAAAGCAGTGGTGATTTGGAGATGGTGAAGGGGATTGAATCTGCATTT
GGGTCGATTGAGTGGAGTGATGGAATTCACAATGTGAGAAGCCCAATTGCAGTGCGTT
GGCGTCACTATTCTGCGGCATCCATT
SEQ 62 
TCACATAGGAGCAAGATGACCTTGTTTGGACAATTTATCTTGCATCCACCTGTGAAGCA
TTTCCAGTGCTGCCTTAGGTTGATCCATTGGAACCATGTGACCAGCATCATGGACCTTT
AAGAAAGTTAAAGGCCCATAGTTCTTTTGAACACCTTTCTCTACACCATCTACTGCAAAA
GAAACTTGTGTGGCTTTTCCAAAGGCTTTTGCCCTGACCATTTCATTGCATGCACCCAT
CTCGAGTTTCCTGCCCATTATAGCAAGAAATTGAGTTTAGTTGTCAATTACTAGGTTGTT
TTCATCTTTCAGTTTATTGTAACAAAATTATGTTTATATATCACATATAAAATAAAAATAGT
TACCGGAAATATATACTAAATCCGGTCAAAGAGAGATCATATCCATGTAACACAATGTAT
AGGAAGGCCCATTTTTTTTTGGTTGGTCGGGTAATGTTTTGTTTTGACAATAAGTAGTG
TCACATGGATATTCTGAATGTAAGGTCAGTTGCCGATATGACTATATTATTACATGTAAA
TGTTATACATTTGGCAGCCTACTTGCACTTTCTTAGAACCGGCTTTAACATTTTTTGACTT
TGTATTTTAATTTTCTGTGATACATAGGTATAATTAAGTTATTTTGCTTTACAGAAACATG
CTTAATTTTGTCCATATATGAAATTAGATGTTGGGTCAGAGATGAATGAAGCTTACCAAG
CCAATTGCAGATAAGGTCATATTCCCCAGCATACACAAGTAGCTTGATACCATCCTCAA
GGAGTGAAGGAATTCCCTCTTCAAGATTCCTCATCCAGTCCAACTGCATTGCCTGGTAA
ACCTCAGAGCTACATGAAACAAACTCAATATCCCCAACACCAAGTGCCTTTTTAACTTGT
TGGTCATTGAGGAAAGTTTCCATTTTTGAGAAGTCATAGCAGAGATCACCCTCACATCT
CTTCCGCACGTCATAGTACTGCAACTCGGAAATTACATGACTTTAACTTCTCTACACTAA
CTAACAATGTAAAGAAATTTTTATTGTTACTTAAAAGATAACTACAGATAATAAGTGAGAT
CAGTAATTTGGAAAATGAGACTGCAAGTAGCGTGTTGTACATCATTAGCTCAAAGAAAAT
GAGATTGCGCTTTCTTACATTTTTGTCACCAGCAATGTCCATAATCTTGTTGAAGATGCT
TGTACAAACAAGATATGCAGCCATGCAAGCAGTTCCGCCATCTTTTCCTAATATTATTTG
CATAGAAATAAAGCTAATTAATGTCAAGATTATATTAGCTGCTGCTATATGAAGGAGAAA
GAACTTACAGGATAAACAAAAAATTAAGAATTACCACAAAGCTTAATTGCCAACTGACAT
TTTGGATATGATTTCTCTATGGCATTGTAATCAGATTTTTTTATCAATTTCATATCCAGAG
CATAGTCAGTGTAGGCTTTGTATTGAATTTCTGGATCAGTGAGTCCATTACCAATAGCAA
ATCCCTATACAAATTAAATACACTTGGTTAAGTTATCGGCATGATGACAAATTTAAATTAA
ACCTACCTAAAAGTTTAACTGAAAAAAAAAAAGAATGGTGGAGGAGCTAATGAGTTAGA
AATACCTTGAGATTTACGTAGATGCCTTCTTTATTTTTGTTTCCTTGGTGGACCCGAGAA
GCAAATGCAGGAATGTAATGCCCAGCATATGATTCTCCAGTAATATAGAAATCATTCTTT
GCATACTGAGGGTGTGCCTTGAAGAAGGCCTATCATCAAAAGAATTTGAATTAAATTTTA
TTAATTATATCAGTTAAACTTTAGAGACTTATCACGAGCTAAAAAAAGAAGAATGAAAGA
ATAAGATCAACCTGCAAGAAGTCATAGAGATCATTGCTTACGCCCCTTTCATCGTGACG
AATATCATCATCGTTTGAACTATAACTGAAACCAGTTCCAGTTGGCTGATCGACGTATAT
AAGGTTTGAGACCTGTCAAATTGCAATTTATCTTATGTTATCATCATTCTTCAACTAACAA
ATGAAAGTTGCATGTTTGATTATAGGATTTAACCAATGTAAACGACTTTTACAGTATTGTT
ATATATATATATATATATATATATATATATTAACATGTTGTATTAGTCCGGTTACTAATCCCAC
TTAAATAAAGAGAAGCGTAGTAGTCATTGCTGTCAATAAGCGATGAACTACTTTTAAACT
TTTGAATTCTACAAGTCACAACTAATGAACAAGTGATAAAGAAAGGAAATGCTAGTAGGT
 
AAAAAGGTACTTTGCATGATGGAGCAAGGTTGAGTAACAAATAAAAACATGGAGGGAAT
TCTTTTAGACTTTTACCATATTCAAAAGATCTAACCGACGTTTCTTGAAAAATTAATTGGG
TAAAATAAAAAAAATAAAAAATAAAAAGCAAAAGGAAGAAGACAAGAACCTTGTCCCAGC
CGAAATCATTCCAGACAAGAGACATATTATCTGCAATTTTGAATGGTCCGTTTTCGTAAA
ACACAGCCAATTCACTGCTACATCCTGGCCCTCCTGTTAGCCATATAACTACTGGATCA
TTCTTCCTGCTTCTCGATTCAAAGAAAAAGTAAAACATCCTGCGAAAAACATATTAAAAA
AACACACAGATAATTTAGCATTAACTAATAATACCCATAAATGAAGCAAAAAGAGCATTT
AATCCAATTCAAACCTTGCATCTTTAGTATGTGGAAGACGATAATAACCAGCGTGATGAC
CCAAGTCTTGAACTGTAGACCCAGAATTACCAACATAAGATAAATTCAATTTCCTCTCAA
AAAGCCTCTGTTCAGAATCCCCTGTTGCTGCAGCCTTGTTAATATCATGTTTAGGGAATA
AATTAAGCTGTCTGATTAGCTTTTCTGCCATTGTTAATGGGAATTTAGGAGTAGAAGATA
GGAAAAACTCATCGTCATTAGAATTTAAAGTTGATGAGAAAGATAAGGAAATAGAAGCAA
GAAGCAGAGTAAGAAAGAGGGATGAAGGCAT
SEQ 63 
ATGTTAGTTATCAGTGATTGTTATATAAATTCTTGCAAAGCTTTCAACTTTGTGATCAATT
TGCCCGTCATGGGACACTCTCACTCTCATTCTTCTCATTCTCACTCTCACTTTCACTCAT
CTAAATCTTCCGATGATCAAAATATGGATATGGGGGAATCGATCACCACCCAAACAGAC
GTTTCTTTCATGCTCGCTAAGCATGTTTTCTCCAAAGAAGTTAAGGGCGATTCCAACCTG
GTGTTTTCTCCTCTCTCAATTCAAATAGTACTTGGCCTGATTGCGGCCGGTTCTAAGGG
GCCAACTAAGGATCAGCTGCTCTGCTTCCTCAAGTCCAAATCCATTGATGAACTCAACT
CTCTTTATTCTCATTTTGTCAGCGTCGTCTTTGTTGATGGCAGCCCCAATGGAGGTCCT
CGTTTGTCTGTTGTTAATGGTGTTTGGATCGACCAAACACTGCCTTTTAAGCCTTCTTAC
AAAAAGGTTGTGGATAAAGTTTACAAAGCAGCTTCCAATTCTGTTGATTTTCAGTGCAAG
GTTAGGCCTTTATTCGTTTGTTTCATTCAAATCTTGTTTCTTTTGTGCTGGGGTTTAATAT
TCTTTGTTCATGCTGACTGCTGAAAATTGGTTCTTTAACTAGTATAATTGACCCTGCATAT
TACTCTCATCATAAGCCCTCCAAATATATCATATAAAATGGATATACATATAGTAAACTGC
AACTAATTAACTTGGGATTGAGGTATAAATGATTGATTGACCAATCTGACTTTAAAATAAT
GAAAAGTGTTAAACAATTAGGACAGAAGCTATATTGCTTAGCCTCAAGTAGTAACAAAAC
TAAATAATGTCAACGGTTGATACTCGTTTCACAGAATTGAGGCAGTTTAAAGAGTAAAAA
GTATTGGTTGTTAATTTGAAAAGTAAGATGAAAGAGTCACAATTCATCTTCATCAATGCT
TATTGTTTAGCAGGTTAGTTGACTAGTTCGACATTTTACTGAGTGGTAATAAGCTTCTTTT
TTGTAGGTAGCTAAAGAAGCCTAAGTAGTTCTAAGCTCAACTGGATATGTGGCCGTGCT
TAATTTTGTAAAACTTCAGTTTTTGGCCTAAATCTACACCCAATCAGTGCTTAAAATATAC
CATGTAAAGCATCCAAATTCTCACTTACCCCTTGCAAGTACTGTAATCAATCTTCTTACT
GCAAACTCCCTTTGTTGGCTAAAGCATATACGTGTTAATTCTGTCGTATACTCTGTTTGT
CTTGCTAATTGAATAAGGCTGCTGAGGTTGCCAATCAAGTCAATCAGTGGGCTAAAATG
AAGACAAATAATCTCATTAAAGAGATTCTTCCTCATGGAACAGTAAACAATATGACAAGG
CTCATCTTTGCAAATGCATTATATTTTAAAGGAGTATGGAATGACAAGTTCAATGCTTCA
GAAACAAAAGACCATAAATTCCATCTCCTCAGTGGAGGGTCTATTAAAGCGCCGTTCAT
GACTAGCAAGAACAAGCAATATGCAGTAGCCTTTGATGGCTTCAAAGTGTTGGGACTTC
ATTACAAGCAAGGCAAAGATATGCGTCGTTTCTGCATGTATTTAATTTTGCCAGATGCTC
GTGATGAATTACCAGCTCTATTGGACAAGATTAGTTCAGAACCTGGTTTTATAGATCATC
ACATTCCGTTTGAAAAAGCTAAAATGCGCAAGTTTCTTATCCCTAAATTCAAAACAACTTT
TGGTTTTGAAGCTTCCAAGGTTCTAAAGGGACTTGGCCTCACATTGCCTTTCTCCAGTG
GTGGCCTCACTGAGATGGTGGATTCCCCGTTAGCTGGGAGGTTGTTTGTTTCGCAGAT
TTTTCACAAGTCCTTCATTGAGGTAAATGAGGAAGGAACAGAAGCTGCAGCTGTTACAG
CTAGTGTAATAATGACCAAGTCCTTGATAATTGAGAAGGAAATGGAGTTTGTTGCTGAC
 
CATCCATTTCTATTCCTTATAAGAGACGAATCTACCGGTGCTGTGTTTTTCATAGGGAGC
GTGCTGAATCCTCTAGCTGGT
SEQ 64 
TTAGCTTGAGCAAGCTGACTGAAGTTCAACTGCATTTTCATCATCAGTAAGGTCACTAGA
CATGGCATGAGGTATTCTATGGCGTTTCAATATACGCGATGTGGCTATTCTTGCCGATT
CATAGTTCAAAACAATCACCTTCTCATCATCCAAATCAAATCTTACATTCTTTTGGTTACC
ATCTTCCACAAGCTGACGTAAATGTTTCAAATTCAGAACTTCTACGCCATTAACCTTCTT
CACCTGTACAAAACGGTTCAAAATTTCCCCGAAAACAAAGAGGAAAAATAATTATAAGG
CGCCCTTATGTTGTCTTTTAAGGGAGAATGAAGTAATAGAAGCAACAAGTGTTTTACCTG
CAACTCGGCAAGGCGCTCATAACCAGCATTAATATCATCCATCAACACCTAATTAATTGC
AAACGGTCATATTAGCAGCATATAGCTCATTGATCTGCGAGTACAATCCTAAAGGAAAA
ACAGACCTCCGTCTCTATGTATAGGTTAGGAATCATGTTGCGAAGCAAAAGTTAAGGCA
CAAAAAATTGAAGTAAACAAATACAAAGTTTATTATTTATGAAGTTAGCCAAATGACATAG
ATTGTCAAGTAAAATAAGACATGCCTTCCTCCCATGTCAGAAATCTTACTGGAAACATTT
ACAAAAGGTTATCTAGCAGCAAATATCTTAATACGTTAACAATGTCTGCCCATTTGGATA
GACCCGTCCACCATCAGTTTTGTGTGTCTCAGTATGTATTTAGCATGTGAAATTATGGTG
GAACTTCATGGCGTCTTTATGCTTTTTTGTTTTTCTATTTTTATAAGAGATCAAATTATTTG
AGTTTTCTATTTGACAAAAGGATAAGAAACTCTAGATACCTGAGAAAGGATGATGAATTG
TTCACCAGGTTTCTTAGGTAGTTCCCGAAGGGCTCGTTCACACAACCGACGGGGTGAG
GCATTATACCAGTCTTCTCCATACTCGTGAAGGAATGGTTGAGTTAATGGAATAAAGAC
GAGACCAGCAAATATGAAATAACTCGGAAGCTTGTCAAATTGATGAACTGGAACAAGTG
GCTGCAACTGCATAATGTTAACTAGTTGTTAGATACAGTAGTCAGTTACACAGCATCAAA
GAAACCTTTAGATTACCAATCATACAAGTTGATAAAGTGTTAAATCTAAGGCTTAGCAAA
AAGTGCAGTCACCTTCCCAACACCTTACTCCTATGCATCAGATTCCCAAGAGCGACAAA
GCAAAAAATAACCTTGGTCCACTCGCACACCAAAATACTTCTAACAAAACAAAAATTTAC
CTTGCTCCAATACCGCACCAAAATACTTCTAACTACATAAATTATGTGACACCCCCAAAT
ACTTCAAGGCTTCTTTCATCTTTTCTATTTTTCTATTTTTGTAAAAGATCAGATTGTTTAAG
TTTTCCATTTAACAAAAGGATACTTGATGAGAGCAACTATAGATACCTGAAAGAGGATGA
TGCATTGTTCAACCTTCATATTATAACTTGTGCAAATGATGAATTTACTTCTACCGGAGC
GATAATGTCAATAAGTTATTTTACCAGAGTAATTCTAATTGAACAATGTAGATGCAGTGA
TTTCGATCTATTCGTCTGATTACATGCACCCAGGATATGAGCAGAAGTTTGAAAAATCAC
TGGGAGATACATTCGAAACAACAATTAATTATGAAGGGTTAATAGAAGGGTAATTATGAA
GGGTAAATAAAAGTGACTTACAGGATGAAGCGTGATTTTGAAGTCATGCACTTTGCCAT
TTCTCAAGACTTTAAGTTCAGCAGTTTCATTAGGTTTCTTCATAGATACCAGATGGTCAA
ATGTGATCCTCTCTCTGTTTCGGAAAGGAACTGCAGATCATAAGCAATCTCAAAACTTTA
GTCCAGGGAAATAAGGTTGCTAATTTTGATATGCATTTTAGTCAAAATGCAGAAGGGCA
AACTTTTCTAACAGAAAATAAGTTATTCTAGCCTTACAATTTTCATATCCTGCACACCCAC
TTATCTGGTTTCTGGTATAATTATCAGCTATTCACATGGCAAGAGAAGAAACTCAGAATT
AAAAACGACTAGACTCTAGGCTTTTCCATCTCTCAAAAGGAGGTCATTAATTTGTTACTC
ATAGTTGCTCGTTGAACATGAATACTTTTTAGTTGGTGCTGGCCTGCCTAAAAGAGCCT
CTACAGCAAGAACCACATCCATGTTTCATCCAAATCCGTCATTTTACGTCACGAAACATT
TAGAGAAAGAAAAAGGCATGTGCCATAAAATGTAAGAAAAAGCTGTGAAGAAGAATGTT
GTACTAGTTTAACTGTCGTACATAAGAATTACAAGAAATAGAGAGAAGCAAGGCAATAA
GAGAAAGCCTTACCTGTTCCATCATTTGCTATGGGTACGCCATCAAATGAGAGGATTAT
GTCGTCTTTCTTTAATACTCTAGAAGCATCAGAAAGTGGGTTGATTCGGCTAACAAGCA
CACCTGTCAATTTGGACTGCATTTGGAAGTACTCTCGAATTTGTGCATTTTCAGTAGGTT
GGCATGACAAGCCCAGAGAGCAAAACCCAATGTATTCACCCCGTTCTTCTACTCCAGCT
ATAAAATGCTTTATCACAGGAACAGGAATAATGTAGCTGCAAAATCCAAATAGAACTGAA
 
ACTTTTAAGGCCAACTATGCACACTGTAAATTTCCTTTCCAATTACAACAGTTTTCTCCA
AGGTAGATTGTTTAATCAAGGGTATCAGTTTAGATTTGATTGTTTGCAGCACTGAGGAAG
AGTCGAAATAGAATGAACTTGAATTCGAGACCGCTATTTGAAATCATAGTGAAATACTGG
AATTTTTATCTCATGTCTAAGAGCTACTAAATGTTCCCACAAGCTAAGCAAATGTTGATTA
AAACTAGTAAATGTCATCAACCAAATCTCCTTATCAACTGCATGTCATCACAACTAAAAG
CTTTCAGGCATTCCCACATATGCCATCCTTTGTAACCCCTCTGAGATGAAAAGAATAATA
TTATGAAGCTAGAGCCAAAGGGCTACAACTCAAGCTTCAAATTTGTGAATGCATGAACA
AGGACTGCGTGAAGGGAAAAATCTGAATATTATGACGAAGAAAAATGGAAGAGAAGATT
TGCAGGAGAATACATGTGTGAAGGGAAGAATAACATGCAGTCAGATTCAGGTAAAGGA
GAGAAAAATCTGAATTTTGTGATGATGCAAGTGGATATTAGAGTATATACCCCATCAATA
TTTAACTAAATAATATATGGTAGACCCAGCAATAAATGATGCAATGGAACTAAATCTGTA
TAGTAGTTCTATCCCTCCGGGGTAGGGGTAAGGTCTGCGTACACTCTACCCTCCCCAG
ACCCCACTTGTGGGATCCTACTGGGTTGTTGTTGTTGTAGTTACAGAGACACAAAG
TACCAAAATAAAATTCTAATTTACCCAATATTCTCTGCACCAGAGAGGTTTTGGAAAGCA
ACTCCAGCAACTTTGTCACCCATAATTGCTGGTCCTCCACTATTCCCTGGATTTATAGCC
GCATCAATTTGTATTGCCAATAGTTGACTAGCGCCGTGTACATATTGCGTAGGTTCTAC
CCTTGAGACAACACCTTTTGTCACGGATATATTATCTCCCCCTAGAAAAAGCAAAAATT
ATTAGAGAACTCCTCAGGTGAAGGTATTTGTGGCTTACAATTAAGTAAAGAAAAAAAAA
AGAGAACATAGTATAGTGAAGAAAACAAAAATATAACTAGACGTCAACAAAGATTAAGA
AGGATCTGCACTATTGAAGACAAGAATCTAGTATATGCAAGCTACAAATATCCAGCCTT
GCACCTAGTTGACACCAGAGAGAAACAAAATACATCATGAAAGTTTCCTTTTCACTCTTC
TGGATCTTATTTGTTCGCTGCTCGTATGAGCCCTCGAAAAGGGTACACCGAATCCGAGA
AAATAGTGCACTCTCTCGCGGAGTATAGCTCACATCCAAACATCTGATTAGGGAATGGG
GCAATGCCCATGAAGCTCTGGCGGAAAGGAAGGCATGCCAGGCCGTATGCCTATGG
GTGCACAATTCTTCGAAAAAGCGCATGCTACCTCGGAGACCTGGACCTTGGCTTAGT
AATGAATGAAGGGAAGCTCTTCGAGCTTTCTCCGCCAGCGGCTTATGTAGTGGTCGGC
CTTATAAAGCTCGCTAAGCCTCGCTTCCCTCTCCCCTTCACTTATTAATTAAGTGGAAAA
TAGTCGTCGGCATTCTATAAGCGACTTGACCGAGTCTACGAAGCTTTGCTTTTCTTGTA
GTCGGCCCGTAATGCCTCCCTTCATTTGCTTGCCTCCTTTCAGCTCAGAATGCCTAATG
CCTATTATTTAGTGCTAGAAGCTAACCGCCATAAGCTCACCCTTCGGGTCTCGCTTCCA
GCGCAGGAGGCCAAGCATTCTGCCAGACGTCCCGGCCTGGGAGCCCCGTTCGCCTTT
GGTNGCTTATTAATTAAGTGAAAAATAGTCGTCGGCATTCTATAAGCGACTTGAGCAGA
GGAGACAAGAAAAGGCTCAGTGGTAACTGTTATGAATGTGGAGGAGTGGGGAATTTTT
GTCGTTACTGTTACAATGTTCAGACGGACGAATGCTGCTAGGGGAGTGCAGAATAGAC
CAGCTGAACGTAGTCGGAGAAGCTCTAAAGGTAAGTTCAAGTCGGAAACATTGTGTTTC
CACAAGAAAAGAAGCACATATTTTTTATAGGATTTGATTTGATAAATGGAAGGAAATTGT
CAATTGAATTGGCAGAAAACTATGGTAAAGGGGAGCCCAACGTCCTCAGCAGAGAGGA
ATGGCAGATGCATTCCCCCCNAATGACATTAGCCTAATTTATTTGAACTATAGACCAAAA
AGAGCCCCATTGGAGTGAAATATATATAGTACTCATACTGCTGTTCCCAATTGATTTGGA
TTGAGGTGTAGCTGATTGGTTGATTTCGTGAGAAAACATGAATAAAAGGAGCTCACTTG
TGAATTGTGATTCATCTGCCGTTGTATTTTAAGACACGAAAAGAAGAATGTGAATTTCAT
ATTAACTCGACACCGTATCAACAACTCAGTTATTTTTATTTTTCTGAAAATTATATCAATA
ACTCAGTTATTTCGAATAGGTATAGCATCCCATTGCTAAACATGAATGTTGTATATCACA
CAAACAATAGGGGGGAAAGGGAGGTTCAGAGTTCGATTCAAGTGTATGGGGGGTTGG
GTGTTTACATTACATCTTGAATCAGTTACAAGAAAAATAATTATCTTGAGGATGACCGCT
GATTAAAAAAAAAATTACCTTGAGGATAACCAACAACAGCCACAGCTTCTTGGAGAAAT
GGAACATCACCAAGCTCCAAAGAGTTCATGCCCTCCCAGAATTCTTCACTTTCTACCAC
CAGAATAGCCAAGTCACATTCATGACCAACAGCTTGCACTGTTGCTCTATACTTGGTAG
GAGAACCATGCTTTCTTACAAGTACAAACGTATGATCAGCCACAACATGAGCATTTGTTA
GGATCCTCTTTCCCCGAATAACAAAACCTATAACATTAAGAATGCAAAATCCGAAAGTAA
GCTTTATGTTCTCTTAAATTATTAGTAGGTCAGATACTTGATAAGTTGATATGAGCTCTGT
 
ATTCTCTTCTTTTAAGAAAAAGAAATTATTACACATAGAGGGTGGGGAAGGGGAAATGG
GGGAGGGGATTACAAGTGGGGAATCGAAACCCAAACTTGTAACAAAAGCAGATAAGAG
AATTAACCTTCCAAAATCGAGTCCCCATAACAAATCAATCAACAATCCTCAATCCTAAAC
TAATTGTAGTTACAACATCAATAATTTCATGCTTTAATCCTAAACTAGTTGGCGTTGGAGT
TGGGACAACAAATCTCTAATACTTGCAGCCCTTGGGACAACAACAAACTATTTATCTCAA
TCAGTGGCGGAGTCACCTTATACCAAGGGGTGTCAATTTGACACCTGACACCCCTTCAC
GGGAAAAAAATATACTACATAGGTAGGTAAAAAAAATTATATATATGTTGACTCCCCTTA
ATTTTTTCGTCTATTTACTTATATATATTTTGACACCCCAATGAAAAGCATGCCTCCGCCA
CTGATCTCAATGAACAACAACTTCATAAAAAGTAGCCTTTTGACAAGGCTTCCTTAGTAA
ATGAAGTGCCAATGTAAGATTTTCACAATAACCAAATGGCTAGTAAAAAAACGGAAGCAT
TACACTGATAGAGAATAACATATTTAGAAAGTAAATGAAAGAGAATAAAAATACCAGAGC
CCGTAGTTTCACGCTGGGACTTGTTCTGCCATGGAAGGAAGTAATTAGGACTACTGGAA
ACAGTGAATATTTTAACTACAGAATCCAATGCTAGCTCTATTGCTAAATAAGCATCCACC
ATTCCACTACTTAATCGCTGCTCCACCGCCGCCACCGGTTCTACTTCCGATGCATTGGA
TTGAAGACTATCATTTTCCTCAGCTCTCTCGACATGAGGTGTCGTAGAAGTTGTTGAGC
TATTGCTGTTATTCAAGGTGGAAAAAAATGAGGCGGAAGCAGAAGTATTACCAACAGTG
CTGCTATAATTGCAGCGCCGAACAAATCGATGAAGCTCTTGACGTCGGTGATGTACCG
GAGCTACATCTCCGGCGATAATAGGGGATTGAAAGTGGAGATTTCTGTTTAAGAGCTTT
CGTGCTGTACGGAGACTTGGACCTATTCGTAACAT
SEQ 65 
CTAGCTAACTTGCTTTTCACTAGCCTTGTAATACAGCATTTTCTGCACATAGAACATCAT
GTATCCTTGCGCAGCTCTCACAATGTTTTCGCTCACTTGAGTGATCCAAGCATCATCAC
ATTTGTACCATTGATTACTTAACCTAAGATATGTTACGTAATGACCAGCATCAAGTTTAC
CGGTATGGGTGATGACAGCAAACAGCTCAAACTCTGAGGACGATTCACAGGACGCATC
TTGCTCGTCCCCATCAAAGGAGAAGATTCTATTTCCAAATCGACTCCTCAAGATAGATG
AAGAAAGGTAAGGCGACATGTCCAAGGAAAAAGGAAACTGTAGGTAGTGATCAACCTT
CCTTGACATTTTCTTAATCACAGAATGCTCAAACCTTTTGATATGGAAGCAAGAAACCAA
AGGCAATTTTCTTATGGACATCTGTTTAAGAGATTCCTGTCTCACTTGACAATGTTGGCA
GAAGAACTTCTGATCAGAACCCAATTTCTCAGGTCTTGTGAAATGATCTAAACATCCCAT
CAACGAAGAAATTCGACCGTTTTGGCTAAACTTTCCAGATTCTGCTTCCTTCTTGTGAGT
ATTATGAGACTTCTTTGATGTCATCTTTGAGGAACTCCCCTGGCTCAGTTCCAAGTCCAA
GGAGATGTCTATACATGGATCATATGTAGTAGATGTGAAGCCACAAGCTGTACACATGA
CATCAGACCGCAAGATTCCAGAAAATACTCTATGAGCAATGCAACAGTCTCCACTACCT
GTGCCAAAGAAAAAGATTTGATAGAATGAGATCACAGCTGAAAAACACAATGTGTACT
CCTAATAAGTTACACAGAGTAACATACTTAATATGCACAGCTTAACGTGCTCCTCGACAC
ACTTCATAACAAGTAAAAGTCCAGTTATGCCATAAAGTTCTATCAGCTATTGCATTACTA
GACAAAACAAAAATCGTCCTATCAGGAATGGCAACCAACAGAATGAGTAGCTAAAAGCA
ACGGAGTACTATTGAAATAAAAGTAAAAAACTCAGTAAGAAATGTATCCGCAAGACACAT
TTATTGCCTCAAAAGCTCTATCTATTCCATTTACACAAAACCATTCAGAATCATAGATAAT
GTGTATTCATTATTTAAATGCAATACCCAGATGTTAACTAAATTTTGCAAACTCAGGGCA
GATAGCCCTGCACCAATCCTCTAATTAAGAGGAATGAGAATAGGTGTGAACTCTCACAA
TCAAAGTACGTGTCACTTAAGTGAGAACCAAATCAGCATATTTACAATGGTAGAAATGAA
TTGTTGCTAAGGAGTTACCCCTCTAATGATTGCTCTGCTAAAGAAATGCGAGGTACCGA
GGAGCTAGACTCACAAGAATAATAAGGCACATAAGGGCTTATAGAAAGGAGGAAGCAA
AAGGCTGTCAACCTAGTAAAGATTTCAAGCTTTCTTGAAGGTTGCGATCAGCTCACTCG
AGAGTAATACCCTAAAACATGGTAAAATTGCGAGTTAATACGAGAACTATTGTACCAGAA
GAAAACTCCTATGCTGAAATCAATAAGACTAACCAAACTGAGACTTACTGGGGAGAGAG
TAGGAGGAAATCTTTTTCCTTTTGAGAAACATCTCTAAGCCTGACAGCGAATATCAACTT
 
GTAAATCGCCTATGGCAAAGAAAAGACCTAAACCATAACCTGCATTCAAATATTACTATT
TTTTCTCAGTGACAATGGAAGTTGGGATTGCCATGAGAGATGAGATGCTAGAACAAAGA
TAAGCCCATCAAGCAGGCCCTGAGTGCCTTTTAGTCAGACGTTACTAGACATCACAAAG
ATGCTTGAACACACTATTCTGCTTCTGACAGAAATTGCTTCTTCCACCCCCTCCCCACCA
ACAAAAGAAATTCAAAAAATTCACCTACTGAAGGACTTGACCTTTGCAAGTACCTGTACA
AGTTTCAGTAATCCACTTGTTTGAGGTTTTTACAATACTAGCCTCCCTTGGCTATGTTAC
ATTTATGTTACTTTAAAGTTGCTGCCATGTGACCTGGAGGTCACGGGTTCGAGCCGTGG
AAACAACCTCTGCAGAAATGCATGGTAAGGCTGCGTCCGATAGACGCCTGTGGTCCAG
CCCTTCCCCGGACCCCGCGCATAGCGGGAGCTTAGTGCACCGGGCTTCCTTTTTTTTT
TATTAAAATATATATAAACAATGTTGTCAATAATTTTCCCAGTACAACAAAAAAAAGAAAT
CTCAATGATTGGTCTAATTCGGAAGAAAAGGGAAAAAGGAAGTATAAGAAACTAATATA
GGCAAGGTGATGGGCGGAGAAACGATGGGCAACTAATAGGTACTCTAATGCAACAAAC
AAATTTACCTGGACTCAACGCCTTTCCCTTATCGTTCTGCATCCTTTCATGAATCCCGTC
AAGCACGGAAATGAAAAACTCATGAGCATCCTGCTGTTCATAACTTGCAAGATTTGATG
CATGCTTCCACCAGCTGTTCCAAAGAAAGGTTTACATCCATCTGAATGAGACACAGCAC
AGACTGCAACTAGATACTCAAAAAGTCGAAATCCACATCTAATAAAACAAATTACAAATG
TATGTATATCACAACCAAGTTACACACCACCAGTGGCGGAGCCAGGATCTCCGCGAAG
GGGGTTCAAGAAAAAAAAAATCGTAGCTAGTGGGAATTGAACCTATGACCTTTCAAAGA
TTTTGAACCCCCTTGACCACTAAGCTACACTTATGGTTGTGTCAAGGGGGTTCAAAACT
TAATATATAGAGGTAAAAAACAGATTTTGCCTTATATATACAGTGTAATTTTTCGGCGAA
GGGGGTTCGGGCGAACCCCCTTTCGCCCCCCTAAATCCGCCCCAGCACACCACTGTCT
AATTTCACCTCTATGAAGGGAAAAGCGTGGTACATACAATCCCAATAATAAAAAACTAAT
CTTGTCCCTACATCATTTTCAAAGAAGTGCACAACCCAAGCTAAATTAAGGAATCTTTTA
CCAATGTATTGTTATCCTTATAAAAAAGAATTATATACAACTATACCTCAATCCCAAGCAA
ATCGGGATCAGCTATATGAACTTCACAACACACACACACACACACACAAAAAAAAAAAA
CGTGCACATTATAACAAAGCCAAACATTATCTCAACAAACCAAGAAAACATGATCAAATG
CAGACCTGTAAAGGAACTTTGCAGGACTAATAGGGGTCCGATCGCCAGAGAAAACAGC
AGAAAACATTGCATCCAAATCACAAGCCAAACACAGCATTGTTGAGTTCTTATTCCCATT
ATCACTACTACTCCTTGTTATAACACTGCTATTCTTTCGCTGGCAAAAATATCTGTTATGC
TTGTCACTCAGAAAGTAATTCCTCAATGGTGGTGTATGAAGCAATGCTTGAAGCACTGA
ATTCATAAAACACGTGTTTCCAAGATTGTTAAGGCCCCTCAAACCCCATTGTACTTCTGG
GGTTGTTGAGTCATTGCCGAGCTGACTCGGCAACGGACTCGAGTTCCCAACGATCAAG
ACTTGCTCTTTCACATCAGGCGTCCACGGTTTGTACTCCACGCGCCTCCTCTTGCGCGT
GCTCTCCGGATGCGGCGGAGGGTCTTGTATTGATCCGATCACGGTGGCCTCCGTCTGC
GCTAGTGCCACGGCGGCGTCGAAGTCGCTATTGTACACCTGGTCCCTACACCCGCAGC
AGAACAGCTCGGCCCGGTCGATGTCCACCGCGATGCAGTGCAGCGAGGGGTCCGCAG
CGTTTCCCGCCGGATGTGACGGCGCGTGCACGCGGCAGAATACCTCGGCGCACGTGA
CGCAGGCGTACAACCGCGGCGGCGCGTGTCCGCACGCACCACATCTCACCAGCTCAT
TCGGCGGCTCGCGGCAGAT
SEQ 66 
TCATAACTTACTGTGCACGAGCTTATTTGAAAGACGTTCAACCTTGCCAAGTTGAGTTGC
GCTCTTCAGCATTGCCTCGCTGTCAGCTATAAACTTTGCAAACTTAGAACCCTTTGTATC
ACCATCACTATCTCCTCGAGGAAGCATTGGCAGATGAAGCATAGAGGGACTTTGCTTG
GAAGATAAAGGCGGAGTTAATGCCCACACAGAAGAAAGCTGTTCATCTGGTTTGTCAAG
ATATTCCAATGATAGATTCTGCATGTCTGTNTAACGGTGATAGATCCTGCATGTCTGTCA
GGAAGAACAGAACAGAAATATCAGACGTTGGTATCGTGCCTACCAAGTGTTATTCGGAA
CTGATTTCTTGAGAGTTGCAAAACTTATTAAGAGCTTGCAGATCAAATGTTACTGCTTTT
ATTCTTCATACGGGAAAAGACCACCCATTATCTGAAAATGGAAGTATCAGGAACAGTCG
 
AATAAAGTACCTTCAACAAACTTGAAGATGGGCTCTAAAGCTGCACATGGAATGCTGAA
GTTCAAATGTGGAATGACCGTCCCTCCACCATGTCTAGCATTACTGCATCAAAAATAAAA
CTACAAGACATGAAGTTTCAACAAGAAAGATATTAATAGATGGAAAAACTATAGAAAGAC
CATGATATGTGAAGATTAACATAAATGAGATGACTAAAAGATCTTCTGTATGAGATCATT
CAAATTGGACACCATTATTTTCCTGTATGAAAAGCGTATAACCAATATACATTTTTGGTA
AGGACAATTAATATACATTCAAACAGGAATATCTTTCTTCAAGGACTTCTCAAAGTACTC
CAGGACGCAGTGTACCACAATATCATTAGATTGAACTTCAAGAACAAACACATGTAAATA
TACATAAGCTGAAAAAGAAATATCCTCAATTATAAGCATCCCCAGTTGTCATCCAAAAGT
TAATTACCTTGTTACAAGGGCAATCATGTGTCCTTCTGAATTAACAACAGCTCCACCACT
ACCACCAGGGTGTACAGCAGCCGTTGTTTCAAGCATTGCCGGAAAATGTTCTCCTAAAC
TTGATTGGTTGAGCAGAGACCGCTTGGCTTCAACTACCTTAGCTATTGCACCCACACAA
GCAGATGGAAGGAAGTCTATATTATAAGAAAAAGTAAGACAAATTACAAATACAACTAAA
GAAGTCTAATATAGGTATGAAACATATGAAATAAATATACGTAGTATATCATGTTCAAAAT
GAAAGAACTTAACAAAATTATTCACATGAAAAGCAATTTAACCTCCAGAACAACATGATT
TAGTACTATTGGGCGCACAAAGATAGTCAGTTCCAGAAAATTATGTTCAGCAAAGGTTAT
GGAACAGACAAGTTATCTGTATCAACGAAAAAAGATGGAACAGACAAGTTAAGATTGCA
TCAATAAACAATAGTAGCACTTGCAACAACCTAGCTACTATTAAAATATCCTTGAGATAC
AGCCCGACTCGAATAAGTGAGTTACCAGGAATTTCCTATTCAAAAATCCCATTCTTTAAA
GCTGATCATTTGTACTTGCTTTCACAATAGAAAACATCAATTTAATGCTCCAGAAATTTAC
CTTTTTTCGTGATAACTGATAAGTGACTTCAAAACTCTAGATTTGATTCCCCAATTCCACT
TTGTTAGCATAGGTATTAGGTATATATCATTCTTATGGATGAAGATCTGAATTAGTGCCT
ATGGCTTTTATTAGCCCACGAAAGGAAAACGCTTCTTTTTAATTTCGTCTACCTTTCTCC
TTGTTCTGCTAGCCTTGTTTGAGCCCTACAACAACCTCGCTATTCTTAATCTGACGTGCA
ATTTTTTTTAACCAGAAGATCAAAACACTGACTTGGACTACAAATCAAATTCAGTATCAGT
AAACAATGTCTTCACCTAAAAGATTACCCAGTTTTGAGCCCTCCCGACCAATCTGGTTAT
TATTTCTCCATTGGAAGACACCTCAAGTTCCCTTGCGGAGGCGTCGACATCACCTCATA
ACTACTCAATCAGTCATCAAATGATCATCATTTGGTGAAGGAAGAAACATCAAGTATTCC
AGCAGTAACAAGGAGATGAAAATGATATAATACGACCCAATCCTGCAATTGATTATAATG
ACACTTCAACAAATTCTTAACACAAGAGAAGCAAGGTGGAAGAGAGAGAAATTCAAGAT
AAACAAAGTTTTTGTAGAATATTCTAAAATTTCAGATTTACTGTGATGCGTGTGTCAAAAT
AAAAGTAAAGGCAAAAATATTTTATTTAGACAACAAATCTAAAGCAAGATTTACCACATC
GTGGTCCAAATAGCCCATGTCCGAGAATGTATGCTTTTGATCCAGGGGACGGGCACAT
GAAGTCAGCAGTAATGGGACAGAGCTGATCAGGAACTAGCTCAAGTTGTAGTAATGCA
ACATCCAGAGGTCCTCTGGAGACATGAACTACCTTTGCATTTGTCCATACCCAGGGATC
CATAAAATCCAAGCGAACACGAATGATCCTACTGCCTGTCTTTGCCAGGTTAACTCTAA
AGCTACCTTGCTCATTGTCAACCAAGAAATGCGGAGTTTTTAGTTCCTTTTGAATCAAAT
GTTTATTCCTACGCTGAATGTCAAATTTCTCAACCCCTGGATGCTCAGATTGATCAGAAG
GGATGAGAACTACATCAGATTTGGTGTTATATCCTGAACCGTTTACAGATGTTTTTCCAA
ATCTCCATGGCTCTAGAAGATGAGCATTTGTAAGAAGAAGACCCTGCTTGTTGAGCAAA
ACTCCAGAAGCCCATGCTCCATCATCAACAGTGATAAGGCAGATAGATGTCATTGCCTT
CTCAATCAAGGATGGGGGAACAGGATCTATTAGGAGATGCTCTCGAGTATCATTGGAA
GGTCCATTCCGAATATTATTGGAGGGTGATTCATTTTTAACACTGATTAGGTTTCCATTA
TCAAAATGGATCTTTCTCCTAGTTTGTAGCTCTTCTTTAAGCAGGCTACCACAAGCAGAT
GTAATAGCTTCCCATGGAATCACCATCTGCCACCCAATGCTTCAATTTCAAATTAAAGCA
ATTTATGTGAAGATAGATCTCAGCAAGAACATAGAGCACATTATGAAATGTTTACCTGAA
TTTCAGCAGCAGTAGCCCTTTGTCTGAGTGGCCGAGACAGAACACCAATAAGCTCTGC
ATGTTCCCCTAACACTGGGCTACCTTCCAT
SEQ 67 
CTGGATATAAGTTTGAACATGATCCTTCCAGAGAACTTGTCTTCAAAACATCCCTTAG
GGTTGATAGAACTTCAAAAGATGTCGCCTTTATAACATCGTCATCCTTGTTGTACGGCTG
TTCCTGCAACATAATTAAGAAAGCAAATTTTAGAAACAATTTCAAAACTGAACATCAAGC
TTTATGAGTTTATCATTGCTGGGTAGAAAGAATGCACAAAAACTGAGGCATGCCTTGA
GATGATCAACTTTCACTGTAAGGGGTTCTTCACTGACCTGCAAATGAATGCCACTGTTT
CAGTCCTCAAAGTGTTACTCTTGTACATATCACAAAATGGGCTATCGAGACGGAAAAA
ACAACATGGTGAATAAAGTAAGGAAATCTGACAGTGAACATTCAAGTGTGACAAATCTA
CTGCATAGTCATCAAATTGTTACACTAGTATATTGAAGAAAATTTTATTGAAACAAAAGA
AATAACTACATAAGGGAATCTGACTGCAAACATCCAACAATAAATAAACCCAGTGTAATC
CCACATGTGGGGGTCCAAGGAGGGTAGTGTGTGCGCAAACCTTACCCTACCTTGTGAA
GGTAGAGAGGTAAACATCCAACAGTATGTATAATCTAATAGGAGAATTATTAATAAGGAG
CTCAGGGGTTGAGAGATTTAAGTCCAATAGAAGAGGATAAGAGGTCCTCAATCACAAAA
TAAAACGAAAAAATAAGAAGACATAAGACCCTCCAATGGCTAAGTAAGCCCATAAATGG
CTGACCATGCTTGAGTACTCCAGCTTTACAGCTAAAGCCAAGAATACGACTCTATACCA
AATTAGAGCTCAAACAAATAGGATTCTATTCAATCTTTTATGTGTTTTATATATGTTTAAG
CCCCAGGGCTTTGGTCTAGTGGTAAGAGTACAGCCCGTGATATGTAGGTTGGGTGCAC
ATCACAGTTCGTGCTCTGGCGCAAACAAAAGCCTAGTATTTAGGTAGACAATGGTAGAA
TGGCGAGCCCATTATCCACCGAGTTTGAAACCATGAGCCACTTACCCTCAGATTTCTCG
GTTATCAAAAGTCTAGGATTTCATTCAAACCAAATGCATCGGCAATCAAACAGATGCTAA
ACTAGCACATCATAGAAGAAACTCAATAGCTTTTCTCTTCTACAACCCCGAAGTGCAATA
AGAAAGACATTCCTATGACTTTGCAGGAAACCAGTTGTCCAAAATTATTACAAAGTGCTA
CTTTCTGTTGCAAACAAGCAATCAAGAATAACTAAAAGAGTGTTTCATTCTATTGGTAAC
ACATCATACACAACTGGGAAAAACAGCTCATTTAGCCTCAAATTGCAACAGACCTCTATC
GCTTGACAACGCAGCTGCTCAGTTATATGAAGCCAAAAGGAGAAAGACTGGGTGTTGG
GACACAAAAGGTAGGAAAGAGAGAAAAGTATACAAGTGCAATGAACCCAAGATGTCAAA
TCGGCAGTCTCAAAATCAGGAGATGATGGATATGTAGATGAAAAATACAACTAAATAGG
ACACAAAGAAAACATATGGAGCTACTTATAACCAAAAAATAACGCAGACATTTACTTACC
ACCTCTGTCATACGTATCCGCCTGTGACCAATAAGAATAACCTGGTCGTCTTTAATACTT
GTTATCTGGCTCATCATAAGTTGCATCAACCTTGATCAGAAACTTCTTCAATAACTTAAA
GCAACAGAATAACACAAGGAACAACAAGATGTACCTGAGCAAGTGTACCAACTTCATGA
AGACGGTTCAACATGTCTTTTCCTTTAAGCTCATAGATGTTTTTTTCTGTATCTGAGGCA
GACACAACATTAGGATCAGTCCCTTGCTCGTCTTTCATAAGGAAAGCGCCAGCATAAGG
TGCTTGCCTTTTTCGACTTTCCAGCAAGGCTGCTAATACCTTGGGATCCTGCAGTTAAAT
TTACGGATTATGTGGAGCCTCAGATCAATTTGTTGCATGCCATTACAGTATAATTTATCT
TTTTCTGGAAACTAACAAATGAGGCTAAAGAATATTACAATTGAGCAAAGACGCTCTCCG
AATTGAAGGTTTTAGCTTTTCAAGCTTACAACTTCAGCCAAGAACTTTCCATCAATTAAA
GTAGAGCATTCTTAAAGCTGAATGCTGGTTACAGACTTAAGAGGAAATAGGGTCCAAAA
CACTAGCTGAAACAGTACAACTCTCAGAAAGTTGACAGTAATTATACACTTAATGTTTAA
AGCTTATATCTATTTTATTTTGAAAATTACGAAGACTAAGACATGGTTTAACCCATCCTAG
ACAAGGTACCATACCTTCACATAAATATGCATATAAAACCCTGGAAATAACGGTCTGTGT
GGAAGTGGCAGTGCTAGAACCTGCACCAAGAAAACAACATGCATCATTATAAATTATAA
AGAATTAAAAAAGTACAGCCCACACAGACTTTATTTATTTTTAGTATAATAAATGACATAA
AGCCCTTAATTGCAAGTCTTACAGGTAAATCTTATCCAAAGATAAAACCATAACCAACCA
AGAAGGCAAGAAATGGGTCGCATCCATTAAATTTACAAACAGATGAATTATGAGCAAAC
AAGAAGCCTCCAAGAAACAGTGTTTTCTTTGCATTCGTTCAGTATCTCTCTTTTTTAGGG
AAGGTTGATAAATACCATTTTTACACTGATCTTGCAACAACTAGCAAAAACTGAAATACA
CATATGAATCTTCCTTTCAAAGAAAAACGAGATAGTAGGGCAACCTATTACTTTATCCTC
ACATGTACTTTCCAATGCTTTACTTCTTAAATAACCTAGAAGCTGTTGATTAAATTGAAAC
TAAAGCATGAAGTAACCCTCTCTAGGCCTCCAAATATAGGATGAAATGTTAAAAATTAAC
ATCCATCAAGGTGCCTTCTGTACGGCCAGAAGTCAGAGGCATGAGGCATAACTGGTCG
AATGTTGACTCTGAAAAGAGCATATGGGAAGGAGAGTATTCTAATTACAATGGACAAGC
 
AAACAAAAAAAAATTCTTTTGCTGAACAATGTGAATCACCAGCTTCTTGCAGGTGAGAAA
TCATTCAAAATCATTAGGAACTTTCTGAAGCTTCAGTCTTGACATAGAGCTGATCAATTT
GCCTCTACAAGGTGGGAGTTACACATGGTCCAGAGAGAGACAAGGCAGGAAGTACTCA
AGGATCGACGGGTTTCTAATATCAGGAGATTTCAAAGAAACAATCTCAACCAATACGGA
TGGTTCAACTGACAATGTGCTCAAAACAAATCAATCTTCTTGATTATGGGATTCTCTGTA
AAAAAAATAAGCCATTTAAATCTGAAAATTGTGAGACAATAGAAGAAGCAGATGAAAAGA
TAAGTTGATGAATACTTATTACATAATACATGACACTTAATTTTCAGGTGTGAAAAGAACA
ATAATCTTTGATGTTGGACTAACTTAACATTAACATTCTACTAACTAACATTGCCATTGTA
TTATGTGTCCTCGAACTCAAGGTAAGAAGAAGTGAAGAACACCTTGAATTTGCTTTTCTG
GAAAACACGGAATAGTGAACAATAGTCGTAATAGACTAATAGTAAAGCATCACCTAGAA
AGTGAACAGTTCAAATCGTGATCAGTCACCAGAACACCATAATGGATTTTTTCGCTGAAA
GTCACGAAAATAGCCCAGCTAAAGAAATCATTAATATTCAGCATAGACTAAGGGAACCG
ACTCTCAATGACCAGAGAGAACGCGATACCAGGAATCTCAAAACCAACCTTGGTGTGG
CGTAAGGTGGCAGCTAATGTGGCTCGAATTGGCGGCTAAACAGTGACTAGAATAGCAA
TGAAATAGCGTCCAAAATAGATATCTGGAACAATAATCATAATACTGGCCTGAAAGATG
GTAGAAAACTCATTGGAAGAATGGCCAAAATAGTTCTTTTACACTGTAAATGGGACATG
GTTGGGATGGTGGCAGAAACTCAATGGAAGAATGGTCAAAAAGGAGGAATTTTACAGC
TAAAAAGATGACTGAGAAAGAGGCATGATACAGTCAAATGGTCTACAGAAATAGGTATG
AGGTTAACGATCACCAAAAGTGGAAAGAATAGTAAAAAAGTTGACCAATCGGGTAGCAT
AGGCTGCCCCTTATTAAGATCACCAATGGTATGTGATACGATCTGAGAAAATAAGAAAG
AGGATGAATTGGTAACTTGATGAAAATTGGAATACAAGACACATCTATTTGTACCTAACA
ATCAAATAGAAAAGACACATCTATGTGTAGGTGAGAAATACATTAACTTTATGATATCTTT
CCAATGTGAGACTAATTTAACACTACTAAGATAACACACATTAACATTTGAACAAGAACA
AGTGGGTGCTACAAGATGGTCAAGGGATGACAAGAATAATATCACATTGCCGAAAATCC
AAGCTGTGTGATTGGAAAAAAACTTGAAATTACTTACAGATGATCTTAGAAGATGAAAAT
AGAGAGGTATATGCTTGAGCGGAGATGGAGATGAAAGAATTGTTGGAAAAATTGAAACC
TTTAGAAATCATTAGGTGAAGCAAAAGAGATGATTGCAAATGAATTACCCCTAATGGAAG
ACTTGATTAAGGAGTTGGCAGACTGTGCCTTAGTGGAGGAGGTATCAAGGAAGCAAAA
ATCAATAACATTGTGGTTGAAGGAGGGGTACAGAAATACCACATTCTTTCTTCCTCCTCT
TCTTTTTTTTTTTTGGAAGAAAGTGGAATACCACATTCTTTCATTGAAAGGGTAATGCTAA
TTCAAGCTACAATAGCATTAAAAAAGTTGCTCACTGATGAGAGGCTCTCTGACGATCCT
AAGAAGATTCAGGACAAGAAATCTTTAAAAAGACGACTAGATTTTAAAAATTTGTCAATT
GTCATTTCATTAAAATACTAATGCTAATTGAAGCTACAATAACATTAAAAGTGTCGGTCAT
CGATGTGAGGTTGTCTGAGGATCTTAAGAAGATTGATATCAAGATTCTTTACCACTACAG
CCTGATTTAAAAAAGTCTACACTACTATTCTTTCGTCTCAAACGGAATGGCTGGGTTCAA
AATATGAGAATCAAAGCATCTGATAATAAAACCGTTCTTTTTGAGACAAAGAGGGTATCA
AAAGCTGTTCTTTGGAAAATAAAATGTTGAAGACCTTCACAGCATTATATCACGTCAAGG
TTGGGAAGGCCTTTTACTAAAGAATAATTGAGTGTACAAGTCTACGAGAAATATAATCTT
GGCGATCCACCTTTTATATCCTAGAAAGTTAACTTCTTAGCCAACCATCCCTTTTGATTG
CCGGCAGAGTGTTGCAACCAAAGTTAAAAATATTTTCTATATCAAATTGATCCCAATTCT
GCGTCAATGCCAACCACAAAGATTAGAAATTTCATCATCAGCGTCCATGTTTGCATCCAT
ATGTACAAATTTGTATAATAATGAATAAAATCATATCACAACTACCCAAAGAAGACATAAA
ACCAGCACTAACAACAGAAGGAACTCACTTAATATCTTCTCTTTTGGTGCACAATCTGAC
ATGGCCACGCCGTTATAACAAAAAGTTGACATCTTTTCTTAAGGAGTCATTTTACCAGCC
ATCAAATATTGGAATAAAATGTACTTATATAATGATGGAGCAGGAAAATACTTAATTATGC
GCCAAATTATATTAGTTAATAACAATGAAGTGATAAATTGAACTGAGAATGTACATTAGTT
AACAAAAGTCATAAAATTTATTTTAAAGAGACGTGTAATTCTATTGGTGCGGGTGCAAGA
AATTATTATGAACTAACCGTAAGGCAATCTTCAGGCTTAAAAACAGTAGGAACAATAGCA
GCGGAAGCCTTAGAATCAGCATCTCCGCCTTTCTCGGCCGGCTTGGCTTCGGATGCAG
CAGCTTCGGAATTCGATTCGGACCCATCAGTAGAATCCGAACAAAAGAATCGTCGAGAC
AAATAAGGGCCCCTGCGATTCGAACTTCTTAGCGAACCTAAAACCCGAAGCAAGGGCG
 
TATTCGAGTCAGTGCCACGGCGAACTTGAGGGGTAAATGCCGTTGTGACGGCGTGGAA
ACGATTCTGCAGACATGAGGATGTGAGAGCCTTCAACAT
SEQ 68 
TCACGCAGCTTCAGCAAAACCCAGTTGAAGATTACCATAGTCAAATACAGTATGATACA
CCCCCATGAATACATTACCAAGAATCCTGCATGTACATGAATCTTTTCGTATGAAACAGA
TCGAGAAGCTTTTGAATAAATTGAGGTATTCTTGTCTATGCATGCAATTTCTATGACCTT
ATTAAAGCGAAATGGTTGTAAGAATAATAAAAACGTACCAAAGAGGACCACGAGGTGGT
GGCACATCCAAAGCAACAAACCCACTGAGGCAAATGGTAGCAATCCCCTCTCCAGTTTT
CAAAATATACTGGAAACAGAAACAAAGCCACAGTTGAAACAAGGCTGAGTATGCAACAT
AACAATGTAACATATATAAAATTAGGAAGAGAGGCATAACCTGATCTGGAGTCAGGACA
AAATCCTTATCACCAATATTGAATGTAACATTTGGCATGGATGATATGCTATTGCAGTCG
ATTACGGATTGCCCCATAGGACTTGGTAATTTCTCACAAAGCTGTGAAATACCAAACATT
AGGCATTAAGACAATGCTCTGCACTACTTAAAGAGAAAATTCACTCACCTGATTCACATA
TTCTAGCACACTCTCCTTTGTTGTCTTCTGTTTCAGCTGGTTCTGCATCCAAATTACAGC
CATCTCACAGGCAGTGCACAATGGGGCCTCTCCTATGGAACTTCCTTCATTTTCCTTCT
CAACCACACTTCTGATATTCGAGCTGCAAAATTGGTTGTAGATTGTCACATGGTTACACA
ATTCAACATGTATTGTAACAGACATAGATCCTTGGTTCAAATCTCATTACCAACCTTCAT
CATAATGAATTTCCAAGTACTAGGCCCATGAAAAGATCATACTTACACGTAAGAGAGCAT
GTCCAAGATATAATAATAATTGAATAAGATTGTGACATCTTTGACAGCTTAAGTTTTTAAA
CGAGATGGTTACACAATTCAACGTAAACAACATAGCTCTAGAGCCTTGACATATGTTCC
AAAGACAGATTGTCCAGTTCAATTTAGTAACTTCACCAAAACCACCTTAATTCACATATG
CTCCAAAGAACAGGAGAAAAGCATCCATACCTCAAATGCTGAGCTCCATTAAGATAACA
TAAACCTACTTGTAAACAAATTTGATCTGGTGTGACCTGCAGACAATTACAAGTAGCATT
ATATCTACAATGTCACATGCAGTAGTGGCCGGCTCAAATAAATAAAAGAAGAGAAATAG
AACAAACCCCTGATACTAGTAAATCCCAAATCATTTCCCCATATTGAGAAATGGTTTCTT
TACATTCCATGCTCAATACTCCTTCTGCTCCAATGGCATGGTTGACTTGTGTCACAACAG
CCTGTTATGTACTTGCTATAATTACTTTCAAGAATTTGTTCAAGATAACTAGATAGTCGAA
AGGTCTACAACTTCTGCAACAACCCAACTAAAGTAAATCATACAGTTGGACCAGCAAGC
AATGATGTTCCAGAATCAACTATAGCAGCACAACCGCCTTCACAGAAGCCTGTAAGGGA
AAAGTATTAAGACAAAAAGAAATTATTCTTATCTTAGGCTCGTAATCAACAGCAACGCTC
ACCTGTTGATTGGTTCCCAATAGAGAAATCTCCCATTTTAAACTGAAACAGAAAAAGGAA
AAGAACAGATGGGGGGACCGTGAAAAAGGCAAAAAGGAATGGAAACCATAGTGGAAA
AAACAATTCAAGTACCTGCCAGTAACCTTTCTGAGTCAAAGGAACATAAGTATGTTTATC
CTTGAAGTGTTTTGGATCAACACCACCAAAAACAAGTTCACCTCCCTCTTTTGCATTTAT
ATCGCGATTAAGCCAGAAAGAGAACACAGGCTCCTTTACGAGATCTTGCTTCACCATAT
TGTACCTGCACATATATCATGAATCAAACAGACAAGATTCCAAAAACTGAGAAGAAAGG
AAAACAAGATAGAATTGGTTAAAAACTGAACTGAACAAATCAATTGCAGGCCTTATTACC
AGACAGGTGTAGTGTTTCCAACAGCAATTTCCTTGAAACCAAGCCCAAGTATTCCATCA
AACTTTGCAACTATAAATGTAACACTTGATTCCCGTGTCGCCTCAATAAAGACCTGAAGA
AATTGATGTAAAAAATTCTCATCCATTGTGTTTTCAGAAGAGCAGAAAGGACCATAATAT
GAGGCAGTGATGACTTATTGCCAAGCAAGATTTCACCTGATCCGTGACTACAAGATCGC
CAACTTGAACATTATCTTGACTGAGAAATCCTGAAATTGATCCAGATCCATAGTGGATTG
AACAAGATTCTCCTGGATTAGAATAAACATCAAATATAATCAGAAGCCATCAATAAATAA
CTTCTTAGTCTTTCAATTAATGTGAAAGAAATATAACATTAAACTATGATATGAACATCAC
CTTTTTTTGTGTATGTACTAGACTTCCTTGCCTTGTATTTGGAATGGATCCAGCATGCAA
TCTGGAAATAATTCAAGTTTAAGGAAAAATTCTGTATAAACCGGTAATTCAACAAAGGAA
CAAATAACTAAAAGAATTCAAGTTATATACACTAAAAGTGTAAGGATTTTTGTTACTATCA
GTATAGTTTAACTTCTGATAGAAATAATTAAGTACCAATTATTGTTACCAATTAATGTTTT
 
CTTATAGAGATTTACATGTAATTACCTTATAAGTGACCTGATTATATAACTAACCTTTGCA
CCGTTAGCACATATAGAACGTAAACATTAACTAAAAAGAAGCACAACTTACAGAAATA
ACATCTTGATGATGGAACCCAGAGATTAGAACTTCCTGTATCAAATATGACAGTGAAATT
TTGAGGGGGTGAACCAATACTAATATCTCCATAATATTGAGCATCCAAGTAGTTCTTTAA
GGACACTATATCTGAATTTGTGTCAGATTTCTTCTTCTTCTTCTTCTCTATGTCCTTC
ATCACATGCTTTCCATATCTGTCTTCAAGTCTTGCTACATTGGCTACATTTAAGCTACTG
ATATCTAATTGTCGCTTCTTCAGACTAATTCTTAGCAAACTATCAGAGGAAGCAGGAAAT
ACAAAGCAGGCAATGGCCAATAAAAGGAGAGCAGCCCAAAGATGCTTCCTTTCCAT
SEQ 69 
TCAAGATCTTATTACAACATACTTCTATTACAATATCTTTTTCTTTTTGTAATGGCTTTGAT
CCTTGGATGGAAAATACTATTTATCCTTCTTTTTGTGATAATTGGGATGTGTACATCTCAA
GTCACTTCTCGTAATATTCAAGCTTTATCCATGTTAGAAAGCACGAGTTATGGATGTCA
AGTCATGGACGTACTTACAAAAATGAAGCAGAGAAGGAAAAGAGATTGAATATATTTAAA
GAGAATGTGAAATTTATTGAGTCTTTCAACAATAATGGGACTAAAAAGCCATACAAATTA
GGCATCAATGCATTTGCTGATCTTACTGCAGAGGAATTCTTGAGTTATTATACTACTGGA
CTTAAGTTGTCTAATTCCTACTCTCAAATTCAATCATCATTTAAGTATGAAAACTTGAGTG
ATGTTCCATCTGTTATGGACTGGAGAAAGAGTGGTGCTGTCACTAGAATCAAACATCAA
GGTCAATGTGGTAAGGCACAGTTTCCTATTCAAGAAAAGTTTCATATTCTCTTCTTATTA
AGTGCTGACGTAACTAGTAAAGTTGATGATATGTGACCAGCAGGTCACGGGTTCAAGTC
ATAGAAACATTCTCTTGCAGAAATGTAGGGTAAGGCTGCGTACAATAGACCCTTGTGGT
CCGGCCCTTCCCCAGACTCCGTACATAGCGAGAGTTTAGTGCACTGAGCTGCCCTTTTT
ATTAAGTATTGAGAAAGGATTTAAGTAAAATACTACATACTCCTTTCAAATTTGTGATCTT
AAACATGTTTTATCATTGTATTATAACGGAGTATCACTAAGGTTAAAATGAGAATATTAGA
AGCAAGCATACTAAATATAAAAATACATTCTTTCTGTAATAGACTAAAATGGAAAATAAGA
TATGCATAGAGTACTCTCTTCTTGTCCAATAATGTTGACAAGGCACTTAAATTATGAGTG
TGTGAAGTCTCACATTGGTAACTGAAAAAATTAGGAGTCTACATATAAGCCTACATATAA
GGTTTAGAGTTTTTTTATGGTGTGAGGTCTTTTGAAAAAAATCGTGCGGACTTAATCCAA
AGTGGATAATATCACACTATTCTAAGAGTATCTTTGAGCTGTTTTAGCTCAACAACTCGT
ATCAGATCCCAGGTTCTGCGGACGAGCATAGCGATGGCGACCTGTGGATCGTGGTAAT
AGCCACATGAAACTGGTTCGACGGGGAGACCCGTGGATCATGATCATGGTAGTGAGCC
ACATAAAACTTAGTTCGAGAGAAGGATTATTGGGTATGCAAACAAAGTCTCACATTAATA
GCTAAAAAGTTTGGGAGCCTGCATATAAGGCGTAGAGAACTTTTAATATTGTGAGTCCT
TTTGGGGAAACCGTACAGTTTGGCCAAAGCGGACTATATCATACTAAGTTAAGAGTATC
TTTGAGCCATTTTAGCCCAACAAATCATATATGATAATTTAAATTTGTTTTACACTACCAA
TAATGTATTTGACCTACTTTGCAGTATAGTTACTATTTTTGTATGTTTATCATAAAAGTTAA
CCTTTAAAACAATACAAGTGATATGATTTGTATAAATATGTGCATAGAACTTCCAACTCAT
TAATAAATTGCATGAAATATAGGATGTTGCTGGGCATTTTCAGCAGTTGCAGCCTTAGAA
GGAGCAAACAAACTCTCAACGAACAACTTGATTTCACTCTCCGAACAACAACTGTTAGA
TTGCACCACCGAAAATAACGGTTGCAACGGCGGTTTAATGACCACAGCCTACGATTTCA
TCATTCAAAATGGCGGCATTGCCACAGAATCCAACTACCCTTACGAGGAATATCAAGAT
TCATGCAAAAGCCAAGAGATGAACTCTGCAGTGAAAATCAATCGTTACGAAACTCTGCC
CTCGACTGAATCAGCATTGTTAAAAGCCGTAGCTAAACAACCGGTCTCTATCGGTATTG
CAGTGAATGAAGATTTTCATCTGTACCAAAATGGTGTTTACAATGGAAATTGCGAGGGT
CAAGAACTAAATCATGCAGTTACTGTAATTGGTTATGGACAGAAAATGATGGTACAAAA
TATTGGTTGATCAAGAATTCTTGGGGACAAGTTGGGGTGAAAATGGTTACATGAAAAT
TGCTAGAGATACTGGAATTGAAGGAGGTCTTTGTGGGATCACCACTTTAGCTTCCTATC
CTGTTCTT
SEQ 70 
TCATAACTTACTGTGCACGAGTTTATTGAAAGACGTTCAACCTTGCCAAGTTGAGTTGC
ACTCTTCAGCATCGCCTCGCTGTCAGCTATAAACTTTGCAAACTTAGAACCCTTTGTATC
ACCATCACTATCTCCTCGAGGAAGCATTGGCAGATGAAGCATAGAGGGACTTTGCTTG
GAAGATAAAGGCGGAGTTAATGCCCACACAGAAGAAAGCTGTTCATCTGGTTTGTCAAG
ATATTCCAATGATAGATTCTGCATGTCTGTCAGGAAAAACAAAACAGAAACATCAGACGT
TGGTATCGTGCCTACCAAGTGTTATTCGGAACAGATTTCGTGAGAGTTGCGAAACTTAT
TAAGAGCTTACGGATCAAAGATTACTGCTTTTATTCTTCATACGGAAAAAACCACCCAT
TATCTGAAAATGGAAGTATCAGGAATAGTCGAATAAAGTACCTTCAGCAAACTTGAAGAT
GGGCTCTAAAGCTGCACATGGAATGCTGAAGTTCAAATGTGGAATGACCGTCCCTCCA
CCATGTCTAGCATTACTGCATCAAAAATAAAGCTACAAGACATGAAGTTTCAACAAGAAA
GACATTAATCGATGGAAAAACTATAGAAAGACCATGATATGTGAAGATTAGCATAAATGA
GATGACTGAAAGATCTTCCATATGAGATCATTCAAATTGGACACCATTATTTTTTCCTGT
ATGCAAAGCGTATAATTAATATACATTTTGGTAAGGACAATTAATATACATTCAAACAGG
AATATCTTTCTTCAAGGACTTCTCAAAGTACTCCAGGACGCAGTGTACCACAATATCATT
AGATTGAACTTCAAGAACAAACACACGTAAACATACATAAGCTGAAAAAGAAATATCCTC
AATTATAAGCATCCCCAGTTGTCATCCAAAAGTTAATTACCTTGTTACAAGGGCAATCAT
GTGTCCTTCTGAATTAACAACAGCTCCACCACTACCACCAGGGTGTACAGCAGCCGTTG
TTTCAAGCATTGCCGGAAAATGTCCTCCTAAACTTGATTGGTTGAGCAGAGGCCGCTTT
GCTTCAACTACCTTAGCTATTGCACCCACACAAGCAGATGGAAGGAAGTCTATATTATA
AGAAAAAGTAAGACAAATTACAAATATAACTAAAGATGTCTAAATAGGTATGAAACATAT
GAAATAAATATACGGTATTATATCATGTTCAAAATGAAAGAACTTAACAAAATTATTTACA
TGAAAAGCTATTTAACCTCCAGAGCAACATGATTTAGTACTATTGGGCGCACAAAGATA
GTCAGTTCCAGAAAATTATGTTCAGCAAAGGTTATGGAACAGACAAGTTAACTTTATCAA
CGAAAAAAGATGGAACAGACAAGTTAAGATTGCATCAATAAACAATAGTAGCACTTCCA
ACAACCAAGCTACTATTAAAATATCCTTGAGATACAGCCGACTCGATTAAGTGAGTTACC
AGGAATTTCCTATTTTAAAACCCCATTCTTTAAAGCTGATCATTTGTACTTGCTTTCACCA
TAGAAAATATCAATTTAATGCTCCAGAAATTTACCTCTTTTCGTGATAAGTGACTTCAAAA
CTCTAGATTTGATTCCCCAATTCCGCTTTGTTAGCATAGGTATTAGGTATATGATCATTC
TTATGGATGAAGATCTGAATTAGTGCCTATGGCTTTTATTAGCCCACGAAAAGAAAACG
CTTTTTTGTTTTTTAATTTGGTCTACCTTTCTCCTTGTTCTACTAGCCTTGTTTGAGCCCA
ACAACAACCTCGCTATTCTTAATCTGACAAGTGCAATTTTTTTTAACCGGAAGATCAAAA
CGTTAACCTGGACTACAAATCAAATTCAGTATCAATAAACAATGTCTTCACCTAAAAGAT
TACCCAGTTTTGAGCCCTCCCGACCAATCTGGTTACTATTTCTCCACTGGAAGACACCT
CAAGTTCCCTTGCGGAGGCATCGACATCACCTCATAACTACTCAATCAGTCATCAAATG
GTCATCATTTGGTGAAGGAAGAAACATCAAGTATTCCAGCAGTAACAAGGACATGAAAA
TGATATAATACGACCCAATCCTGCAATTGATTATAATGACACTTCAACAAATTCTTAACAC
GAGAGAAGCAAGGTGGAAGAGAGAGAAATTCAAGATAAACAAAGATTTTGTAGAATATT
CTAAAATTTCAGATTTACTGTGATGCGTGTGTCCAAATAAAAGTAAAGGCACAAATTTTT
TATTTAGACAAGAACATATCTAAAGCAAGATTTACCACATCGTGGTCCAAATAGCCCATG
TCCGAGAATGTATGCTTTTGATCCGGGGGATGGGCACATGAAGTCAACAATAATGGGA
CAGAGCTGATCTGGAACTAGCTCAAGTTGTAGTAATGCAACATCCAGAGGTCCTCTGGA
GACATGAACTACCTTTGCATTTGTCCATACCCAGGGATCCATAAAATCCAAGCGAACAC
GAATGGTCCTACTGCCTGTGTTTGCCAGGTTAACTCTAAAGCTACATTGCTCATTGTCAA
CCAAGAAATGCGGAGTTTTTAGTTCCTTTTGAATCAAATGTTTATTCCTACGCTGAATGT
CAAATTTCTCAACCCCTGGATGCTCAGATTGATCAGAAGGGATGAGAACTACATCAGAT
TTGGTGTTATATCCTGAACCGTTTACAGATGTTTTTCCAAATCTCCATGGCTCTAGAAGA
 
TGAGCATTTGTAAGAAGAAGACCCTGCTTGTTGAGCAAAACTCCAGAAGCCCATGCTCC
ATCATCAACAGCGATAAGACAGATAGATGTCATTGCTTTCTCAATCAAGGATGGGGAA
CAGGATCTATTTGGAGATGCTCTTGAGTATCATTGGCATGTCCATCCTGAATATTATTGG
AGAATGATTCTTTTTAACGCTGATTAGGTTTCCATTACCAAAATGGATCTTTCTCCTAGT
TTGTAGCTCTTCTTTAAGCAGGCTACCACAAGCAGATGTAATAGCTTCCCATGGAATCA
CCATCTGCCACCCAATGCTTCAATTTCAAATTAAAGCAATACATGTGAATGTAGATCTCA
GCAAGAACATAGAGCACATTATGAAATGTTTACCTGAATTTCAGCAGCAGTAGCCCTTT
GTCTGAGTGGCCGAGACAGAACACCAATAAGCTCTGCATGTTCCCCTAACACTGGGCT
ACCTTCCATTCCTGAAACATAAGGTACCATACGTTATCAGGTACCCAAAGATGGAAAGG
ATTAATTAACTAAAGTTGCAAGGCAAAAGCTCAAACCAGGGAGACAACGGATGTCAGCA
ATCAACAGTGCTTTATTCTGTGGACTAGGTGGATAGCTGTTTGCAATGGACCCAACTGA
TATGCTGTATAGCAACAAAAGATCAAATTGAGCTTTCACAAGGAGATGTAACAAGAATCA
AAGTGAATATTAATCCTATGTATGATCACAACTTGGCAGACAAATAATCATCGAACAACA
TCTGCATACACCAGATACAGCTTATATAGCTTACTCAAGTAAAGATGAGCTAAAACATCT
TTAGCACTAGCAACAAGAATTACACCAGTGCTTTCATATTCAGATAGTCTATTAACACAG
CACAAATGTTCAGTCATCATACATTTTGATGCTGACAAGCAAGGATGGAAAGATTACAA
GAAAGTTGTAATTCCAACATATGATAGAAACAATACCAAAGCAACTACCTGTTGAAAAAG
TGACTGGGAGACAAGATACCGAAAGGAGAACCCATACCCAGAAGAAGATCACCTCTCC
TACTCCAGGTAGCCACTTTTAATGCAGGCAGGTCCTGAAAGGAAATACCTATGCAGTCA
TTATCATCAAAAACCTACTAAATGGAGCTTTCAAAAATTACCAGCAAAAAGCAAGCCCA
TTCAGCAAAATGCAATGCCATGCTTCATTTCACAAGTTTGATGCCACTTAGTGCAGTAAA
AGCCTGAAACCTCATATGGATTTGAAGAAACTCTGAGAAGAGCAATTCTTGTAGTTGAT
GTTCCTATCACACTTGGCAGGCTGGACTGCGCCTCCATCATTGGAGTTTGACTTGGGAA
AGAAATTTTCTCAACCTGAAATTATCAGTTGACAGTTCCTGTTAAAAAATAGTCAATCTAC
AAAATCAATCTCCGTGGTCCTTTGAGACTAGCATCTCATAGACCTTTGGCTATGAAAACT
AGAATGCTTTGTGCAAAAATTGTTTGGGCCATCTTGAACATAGTACTAGGGACAATTGA
GATGCCACCGTTGAAGAACACCATGGTAAGAGTTCCTTATGTCATGCCATTTTGCACTT
GACAGCGTGAGACTCAAATGTGCTCCTTTCCAATAACACTGCCGAATGCTTATACCATT
ACAAATATGTAACCAGAGTAGCTTACTGAGTTGGAGTTCTTAATATGAATTATTTTAAGA
AATGCCTCCAAGTTTTACGGGTGGTAGACTACCTTGTATTCAGACACGGGAAGGTGATA
TTCAATCATTAATCTTTGAATTGGAATTAGGTGTTGAGACATTCTATGATGATAAAGAGG
CAACTATTTTGGCATGAGTAGAGACATGAAACAATGTGATGTCACTCTATTGTAAATATA
GGGAGCATGAAATAAGGAACTCATTACATGAATTTCCATTATTCTCCTATAAAAAGAGGT
TACAGAACATATAAAAGGTTCTGCTTGGAAACAACTCAACTGATAATGCAATTAATGCTA
AATATATGGAGGAAACTTGCATGTTCCACAACTCGAAGAAGTGGTGTGCAACATATATT
AGTTCAAATCCATACTGCTCTTATGCCAGAAAAGAGAAGAAAAAACAGAACCCTGAAAG
TGCAAAGTGGTCATGATTAGACGTAAGACAGGAATAAAATGGCATTTCTGCTCAAAAGA
AATAGGTGCTTGATTTATTTAGTTATTTAAGAATGATAAATGATATGCCTTTCAGTTGATA
GAACTTTAAAGTGTTAGCCTGATAAAATTATAGTTATTTGATGAAGTCTTTTGAAATTTGA
ACCACAAAGTGGGATCATAGCAGAAGTTAGCTCATGAAAAATGACCAAGGATCTACAGC
ATCCAATCAAAATATGCATGCAAGAGAATTTGGCTTATCTTGGTCTCGGCGACATTTAAT
TATCTTTGAATGGAAGTATTGTCATGTTAATCCTATTTATGACTATGTTATGCATTAATGA
AACAATCACCTAATTCAATAATAACACAGCTAGATAGTGTCAGGAGTATTTAGAAAGCGA
GTGAGAGGCTCGGACACATCTATCAATAGGTTGTACCAATCATATATAGATGAAGTACA
GAGACTATGGTTACATATTTACTCATTAATACATAAAGGTACAGAGATTGTTATTGGGTA
CACATTTACTGCATGTAAGTGCCAAACAGAGGAAACATCATACCTGTGTGCGCTTAGTG
TTAGTAAATGATTGACGGGAATTTCCATAGGCAGCTAGGGACCAACCAACCTCCCACCC
ATGTTCAATTGAACTAGACGAACCTTCAACTAGGGACTGGACAGCAGCAGATGACACAG
GGATGTCAACCTTCAGCAAGAACACCAAATAGACGGTGTCAAGTGCAACTTTTACGTTA
GATCAGTAACAAAATAGCGACAAAGAAGAACACCAGCCATAAAAGTGATCAGTTAACCC
ATGCTAAAAACTAGGATAAAGTTCAAAACTAGGTATTCCTTCTCCATTTTATACGGCACA
 
CTTTCCTTTTTAGTATGTTCCAAAAAGAATAGAACCCTTCTCTATTTGGAATATCTTAAAA
CTTTAAACTTCCCACTTTACCTTAATGACATGCTCTTATAGCCATAGAAGTGTTATGAAAT
GTTTAAAACCACAACTTCTAAAGGTAATTTGGTATGTGTCAAAATCTTTTGTGCACGGGC
ACAAAACACCAGATGACATCAAACTAGAATTTATATGCATGCATCAAAATGAGAGCACTT
ATCAATTCAATATTGCAAATAAAAAACATATATAAGATAAAGTAACAGGTTTTATGATAAT
CAGCATTCAGATTACAAAATCCTTTCAGTCTCCTACTAAATCCTACCACTCTTAGGAGTT
CTGCAGGTAGCCAGTTCAAACCCTCTTTGTTGGTCACTTTGATATCATTTTGCAATGTAT
TTCCTCCCTGTGAACCACGAGCAGGACATCCAATGTCAATTTAATATATCAATGAACCTA
AAAAACGTATTTTCTTCCGTAGGCTCAAATAAATACCTCCCACAGTATATCAATTTGAGC
ACCAGGAATCAGCTCCGGCTTATCCTGCAAGATGGTACATTGTAAAAGAATTATGAAAA
AGCATAACGACAAAGAAATAATCATGTTGTTCCATTTTATACTACATCCATTCAAAAACCT
ACCTTTCTATACATAGAATTTTTTTAATCTTGTAATTCTCATTTTGCACTTAATGGCATGCT
CTTATAGGAATTGACATGGCATGCTAAAGACAACTAGATAACTTCTTACACATGTAATTA
AATATGTGACAAAAGTGGTTCTTTCTTTATTAAACTCCTTCTCCAATCAAACACCATCATA
TAAAGTGAAACCAAACAGAGGGAGTAATTATCAACTGAAAGAAGACTAAAGATCCAAAC
CTTTGATATGTCCCCTCTATCCTGTTGTACAACAAAAGGCTCAATAACAGAAGCAACTGT
TAAAACCAAGAAGTGACCTCCAAAAGAGTGCAACTTGCTTTCACCTTGAATCTGCTTAG
ACACTGAAGCATTAACAAAGGAACTGGGCAAAAGCATCCCAGATGCTGATAGTGTCGTC
TTCCCAGAACTACACCACAAAATTACAACCCAAATTTAAAACTTTCAGTTCAAAACACAT
AACATAAACAACTATAATATAGAGACAGAGAGAGCTATGTAAAATCACTTACTTGTACAG
GTGGAAAGCATGTTTTCGCATTTTTAGGCCTTTAGGGTCCTTCAAGAAGAAGAATTTATT
TGTATTTTTTCAAAAATTAATTAGTAGAATAAGCAAAGTGATTGAAAATTACAGTATCTTG
GCTTAAGAAAAGGGACTCACTGGGCCTTGAATTCTGACCATGACGGCATAATTGCGGG
CAACATCAACCACTTCAGGAAGACCCAT
SEQ 71 
ATGGATAACCCATCGGAGGATTCCTCGGATTCTCCTCAACAGCAGCCCGAATCTCCTGT
AAACGATGACCAACGTGTTTATTTAGTTCCTTACAGGTAAAATCTCCCTTCCCCGTTTTG
ACCCATTCCTCATGCAACTGTTTGTTTATGTATATCAACATAAAAGTAAAAATAAATAAAA
ATAAAGAATTGAATTCTCGGATTTTGCTTTCCCAATTGATTTTATGATTTGGTTTGATCCA
ATTCAGCTAAACCCGAATCTGAACCCATGAGATAACGAGAAAGTCGAAACAAGTTCTAG
TTTTTTTTTTCTTTTTTCTTTTTGTTTAAATTACTTATATTTTTATTTGTATTACTTGTCATTT
AGATTGGTAATTGTATTAGCTTCCCTACATTGGAATGTTGTAGTTTTTTTAATCAAGTCTT
ATTATCTGGATCAAATCGTGTTGTGAGTTTTTTTATTTTTTTTATTAGTTGCCATTTGGATT
GGTAATTGTATTAGCTTTTGTACATTGAACTAGTGTTGGTTTTTAATCAATGTTGTTGGTT
TTTGTTATCTGTTAACCGGTGGATCAAATCATGTTGTGGGTTGTATATTTTTGTTTTGTGA
GCTTAAGCATAAGAAAGTATCGGCCTTGGATTTTCAGTTGTGTTTTTTTGATGAAGTAAA
TAGTTTCACCAATGTCATCAAGAAGATGCAAGTATTACGAAAGATTAGGCCAGAGAGTA
TCAGCTTCAATTACATTGGTCTAGATTGCTAAGGAGCTGATAAAGTCCAGAAAGTTAACA
GGGTAAGTTACAATAGATAGTTTTGCCAACTAAATAAAAGTAAAAGACACCTAGCTATCA
GTTGTTAACAATGGAGAAGTAGTATAGCAAAGTGCCGGCAAGATCTGAAAGTGGTGGTT
ATAGGGACCTGTTTAATAACTTAGTAAACCTTAGAAGAAGCTGACAAATTGTTCCATCTA
CAATTTGTCAACCTTAATAGAGGTGCACACAAGCTGGTCGGACACCACGGTTATCAAAT
TTTTTGTTTAAAAAATGTTCCATCTCGATAAAATATCAATTGATTATGCATTATGTTGTCA
GTTCAAATATTGTTTCTCGCAATTATTATAAAAAGTGCATATCTGTGGAGAAGTGCTCCG
CGGGCTAGTGCGGTGGTAGGGGAGAGTGGTAAAAATGACACAAATGATGCTTTCCACT
TGCTAGTGGTTGTTAAGAAGAGAGAGAATGTTTGAGCGGGAAGGACGGGGTAAATAGC
 
ATGGAAATGTTAATTGAAAGAAGTTAAAAGTTACCCTTTGCAGCATCTTCTCTAGGTAAG
AATTTTTTGTCTGTGTTTTCCCGAGTAGAGGGTTAAAGTGTTGCACACACATATATTACA
GGTGCCACAGACACGTATATGTTTAGAGTACTATATAAGAAAGCGTGTTTGTGTTCTAG
GTGGTGGAAAGAAGCACAGGAGTCATCACCATCAGATGGGAAGTCAGTGACTTTGTAC
GCAGCGGCACCAGCTCCATCTTATGGAGGGCCAATGAAAATCATTAACAACATATTTAG
CCCAGACGTCGCATTTAACTTGAGGAGAGAGGAGGAATCTTTATCACAGAGTCAGGAG
AATGGTGAAGTTGGGGTATCTGGTCGGACTATGCTTTGGTCCCTGGCGACATTTGGC
TGCAGGCACTCAAATGGTCAGTATTTTAGAGCAGTTTCCAATTTGTATTCCTTGAAGTGT
GTTAGATAAAGCCTCTTCTGACGGAGATTTACGCCATAGTTGTTGAGCATTCTGAGGAT
ACCATTTGCATATGTGTTTTTCTCGACTTCAAATAAAACATTGATTTTTCACTTCTGGTTA
CAACAACCACTTGCAATTTGTTGTTTGGTTTCTTCTGCTTTTCAGACCATTCACATTTTCA
TTTCACATGAAAGAGGCCTCAAGCCTTTCGAGGCTTCATTGTTGTTGCTAGTCCGATGG
CAATTCCCAGTTATAAATATATATTGTTAAATGCCTTGTGAATGCATATGGAAGCTCGTTT
TTTAAAGCATTTTGAGATTTCATTCTAAAAAGACCACTGTTTATTCTTTCAGCTTTAAAGT
GCTAAGCTCAATCTATTAATTCGCTTCCTTATTTTCTTTGTCTCTTTCATATATTTTTTTTG
TGTGTGTGGGGGGTGGGGATTGGTGTTAACTTATAACTGATTATTTCACTTTCCTTTTTG
GTGTTTTTGCACATCTAAGAAAGGGAATTTGTCTTTTGATCCTAGTAACATGTTATTTAG
CACGTTAATTTCATACATCTGGCACTATGTAAAAGTTGATCTTTTGATTATAGAGTTCTGA
TTAGTTTGATTGGAATTGCTCCTTTCCATCCAGGCACAGTAACTCTAAAGCTGCGGCTA
AGAATGGAAAAAGCTTTTNCAAAATTGCTCCTTTCCATCCAGGCACAGTAACTCTAAAGC
TGCGGCTAAGAATGGAAAAAGCTTTTCAGCTACAGATGAGGATATTGCAGATGTCTATC
CTTTACAGCTGAGGCTTTCTGTTTTGCGGAAACCAGTTCCTTGGGAGTCAGGATAAGC
AAAAAGGTTAATAATAACTTTGGAATTTCTAGTTTCATCTACAATTCCCATGAGATTTGTA
CTGTCATAATATCCATAGAGTGCATAACCACATGTGATCTTTTTGGTACAGACCTTGCCA
CAATTTGTGAAGCTGACTTTCTTTTTCATCAGCTGCTTTGCCATCTTTCATCCTTCACTTT
TGTTGTTGCTGTTTATTGTTGAAACTGAAGGATCTAAGATGGACAAGTCCAAAATATCAA
CTTTAAGAACAAGGGTTATGCATGAAGCCCTCTATTCTCATCCTCATATTAATTACTCGG
AATGGGCATAGCTGTTAAGTGCTTCCATTTTTGTTTGATATTTAAATATTAACAGTAAGAG
CTTTTTATGGTTCTGGGTTGTGCAAAAAGAGGACAAGTATGTTAGCTGGATACGTATCTT
TTTGCTGACAAAGTGGGATAAATTTTGCATGGTAATTTTTGGCTTTACATGAATTTGTTG
ACAAGGATATCAATCCTATTGATATTTATGTAATTCACTTGAAGCATTTAATACTTCTATT
TCCCAATTACAGAATTGCTCATCAGCAATTTTTGACCACAGTGAGTTCTAGAGAAGCGA
GTCTTTTGAAAATGATAGTAGAAAGAGCGCTTCCTTTCTCTAGATTGTCTTTCTAGCAAA
GTAAATTAATCTAGAGTTAAATTGTATTGACAGGAATAAACAATATAAGCGGTATTTTCAT
CCAGACACCCCTCCCCTGTTTGTGGAGCAGAAAGAAACTATGTAATTGGGAATCACTTA
GTTTTGAGATAACATAGTAGACTATGTGACAGTTTTATTCTTTTAATTTAAAACTAACAAG
TTGTCTTATATCTAAAGTTTTAGCAGAATCATTTATTCTGCCTCTAATAGCTTGGAAGAAC
TATATATCATTTGAGGTCTTTACTTGCCGAAAGACATCGGAGATGAAGTTAGTTTTTTATT
AGATCAGATATGAAATTAGTTCTAGCTTTTTTTTATATACTCAAGGATGCCCTGACTTCCT
CCATCTTTATCTATTTTTGAGAAATTCTCTTTCTTGACTGCCAAATGCTAAGAGGAAATG
GTACCAAGCGGTTACATGCAAACCCTGGTCTGAAGAAGTAAAATAGCTAGAGTTCTAAT
TTTCATAAAGCTAATAGGAAATAATTTGATCACTTGTGAAAATAAGCCAAAAGAATGATG
CTCATTCGAACAAAGTTCTCTTAGAGTTACTACATATTTCGTTGTGTATACATGATCCTTC
AATGCTACTTCATATTTATTATTTACCCGAAAGTTGATGTTAATTTGAGCTCTTTTTTTCTT
AACAATGTTATTGCTGACTTGTCCGTTTACTGCCTCAGCTTGTGTACATAAAAATGTAGT
TTCCAAAATGTTGTTTGTATTTTGTAACTGTTGCATGTTAAACATTTGCAATATTGCGGTG
GACAACGTTTCTTTTTTTTTTTCTTGCAATAATATCCTCACCGATGTTCTTTTTTTTTTTTT
AACTATCCTCACCGATGCTTTGGAGTCAGTTAAGCTGCTTTATTTCCTTTAGAGTCTAGT
TTTACATTTGTCTTCTCACATTTGATTCAGGACAATACAGTTGAATGCTTTAAAAGAGCCT
GCAGAATTTTTAGTGTCGATACAGAACCCGTAAGTTTCAATACTGTTGTTAATCAATTGC
AATGGTATCTCTTTCAGGAGATTAATGGTATTTTGGTTCTCTGCAGTTACGGATTTGGGA
 
TTTATCTGGGCAGACGGCATTGTTTTTTCAGATGAAAACAATAAGATCCTCAAAGACTC
TCAGAAACAGTCAGAGCAAGATGTATGTACTTTCAACTGTGTCATACTTCATGACTAACC
AATAAACAAGTCGACCAATGCTTCTGCGGCATTCACTATTTTTCCTGTCTTTACTAAGGA
AATAATTTAGTTATGCTTTTTTCTAATTGTTTTCTAATTAAGTGTTTTTAGCAGATTTTTCC
ATTTCTTTATCTAGTGTTTGTGTCGTAAAAAGATATATAATGATTGAGGTGATGAATATGC
TTACTTAACACTTCATCTAGGAATGAAGTGAGACAATGATTTTTCTCCATTTTCTATATAA
GTGTTGTTTTTTCTTGAGCATGGACAATGCTAAGCCCACCAAAATTCAGTTTTATGCGAC
TCTCTTTCATTTTAGGGTTCGTTCTGGAGTTATTCATTTATAAGCAGTAGATGTGCTCTTT
CCTTGTACTTTCAATGATGTACACTCTAAGAAACTTTAGCTCTTTTATTACCCTGGGACA
AAAGAAACACATAATAAACGGGACTGTCATGTCTAACGACCAGCTTATACACCCATTCG
TCTTGGAGAACAGGCGGGTAAATTCAGCTTAGTTATGCTGTTTTTCAGCTGGATCTAAG
ATTACAAAAAGAGCAACTGTTTTGTTTTTTGTTTTTTTCCATTTGTGGCAGTTATTACCGG
TGTGGTTCATCATTGATTGTTTTTGTATTTCTTAGGCTGTTTATCTGTGAAACAAATTGA
AAGAGATGCCAAAGTTTTATCTGTTTTATGTTTCTTTTTTCCTTGTGGGTTACCATTTAAC
TGAGAGCAAGGTAAACCTTTACTGTTGAAGGCATTTTGCTGGTTATGGGTTGCCTTATA
GTCTTATTACTGACTCTTGAATTAACTCTAGAATTTAGTGTTTAATGGTTCGCACCGCTT
GTAAGCAAGAAATGATTTGGACAAACTTCTTATTTTGTCCTCTTATGTTTTTGCTTGCAGA
TGCTCTTGGAGTTGCAGGTCTATGGGTTATCAGATTCTGTTAAAAATAAAGTGAAAAAAG
ATGAGATGTCAATGCAATACCCTAATGGTTCTTCTTTTCTGATGAATGGTACTGGCAGTG
GTATAACCTCTAATCTCACTAGGAGCAGTTCTTCATCATTTTCTGGAGGTCCATGTGAAG
CTGGTACCTTGGGCTTGACTGGATTGCAAAACCTAGGGAACACCTGTTTCATGAACAGT
GCTCTTCAGTGCCTTGCACATACGCCAAAGCTTGTTGATTACTTTCTCGGGGACTACAA
GAGAGAAATAAATCATGATAACCCTTTGGGAATGAATGTAAGCAATCTTGAATATTTCAA
GATCATTACGTGCTGCTTTAGATGTTTTCTTCAGTTCTCTCTGAATAAGTCAATGTTGAC
ATCCCTTAACCTATTCTACATTATATGTGGTTGGAAAAGTAAAAGAAAAAGAGAAATTCA
TTTGATTACTCTCCAGGTGAGGAATTCTTTATTTACCTCCAATTGTTTTGTTAGCCCGGA
CAAAAGAAAACGATATGCTTATCCGTTCCATTCAATTTAGTAGGGGTTGAGAAAATTGAC
TCGGAGGGTATTCAATATCTCCACTTTTTGTTTCGTACCAAACAAGGGGAATAAACTTTA
CCTCTTTTACTTTTCCTCCTCCTTCCACCTCATCTCATCCCAATCAAACATTGTGTTCTAA
TCTGTCTCCTTACATATTTTATTGTCTAAGTTCCTCTCTTTAAATTCTTTCAGGGTGAAAT
TGCATCTGCTTTTGGTGACCTTTTGAAGAAATTATGGGCTCCTGGAGCGACTCCTGTGG
CACCTAGAACATTCAAATTAAAGCTTGCTCATTTTGCTCCTCAATTCAGCGGCTTTAATC
AGCATGATTCTCAGGTCCTTTCAGTCCTTCCTGTTGGATTTAGTTTCCCAGTTTTAGGTC
ACTTATTAACGCTCTCTTTTCTGTCCTCTCATTTTGTGGGCATCTTTTGACATCTAATTCT
CCTATTTATATCTGCAGGAGCTCCTAGCTTTTCTATTGGATGGACTCCACGAAGATTTGA
ACCGTGTCAAGAATAAACCTTATGTTGAAGCTAAGGATGGAGATGATCGTCCAGATGAA
GAAATTGCTGATGAATACTGGAATAATCATCTGGCTCGTAATGATTCCATCATAGTGGAC
GTTTGCCAGGTAAGTAACATCCGATGGTCTCTTGTATCTCACTAGAAGTAGGAAACATTT
GATATCACCGGCACTCAGTGGTCTCTCGTCTCTCACAGGCAAATGTGAATTATTGATCT
CATTTCAACATTGACTTGAAAAAGCAAGAAGAATGAAGTGGCATATTTTTTAAAAATATCT
GAACTCTACTGTATTTGTGCTGCGAGAGTTGTTCTAGGATGAGAGAGTAATTATACCCC
AACTGTTTGGGGAAGTTTAACCAGTGTTCCTAAAGCTTGCTTCAAATTTCTCAGATATTT
TTGTCTAGATTCTCTGCCTTTTTCATCAATAAGATTTCTTACCTTACTCAAAAGAAATTGT
AAGTAATGGAAATTGAATTCAACTCTTACCATAAGTAATGGAAATTGAATTAGTCGTCCC
TTTCAAATCAATCCGATAACCAACTTGGTTCAATAATTCGGAATAGTGGGAGTACTATTT
GTTAACAACTGACATACTATTTTCCTAGAATGCAGTCCTGAACTAAGAGCTGAATTTTGG
ATTCAGGTTTATTTTAAAACAAATTAGTTTTATTTGTAGTGCTCCCTGTTTTCTCTATATG
GTCTCCGCATTTTATCTTTGATGTCTTTTTGAGTTTTACTGAAATTTCCTAAAAGAAGAAG
AATATTCCAGCCTTTAATCTCCATAAGAAAGTTAAATTTTTGTTCTTCCATAGTTTACAAG
TTTAATTATATAAAAACTTGAACCTACCTTATCAAAAAAGAAAAAGAATTATGTGAAAACC
TTGAACATTGTGAATGTTTCTGACATTTGTGCACTCTATAGGTGTGTTTGGAAGTAATTG
 
GTCTTTTTATGTGTTATGTAGTCTATGGCGGTATGTAATTTCTAAATTCCTCTTTGGCCAA
TCATCTGTGAGATAAAGCTCTGCAATTCTAAGATAATTTCGAATACCACCAATTGATGAT
AGCTTGTCATTTTTTTTAGTTTTTTTATTCTGTAATTTTTTGCATTAATAAAGTTGACTCTT
AGCCATGTCTATCATTAGATGTCGTAAATTTTGAAATTTCACTTTAGAAACCATGTACATA
GTACATGTTTCCTAGCAGGTCGTGATTTGTCTATGACTTTTAGGAGGATTTAAAAGTTTA
CTTCATCTGACTCCCTTTTTCTGCACATTTATACAAGTTTCTTTTCTAGTCTTGCTTCTCA
GAGCTTATCTCGATTGTTGCAGGGTCAATATCGTTCCACATTGGTCTGTCCTGTTTGCA
AAAAGGTCTCCATCATGTTTGATCCTTTCATGTATTTGTCACTGCCTCTTCCATCTACAT
CTATGAGGTCAATGACTGTCACAGTTATAAAAAATGGCAGTGATATTCAGATATCTGCCT
TTACAATCACTGTTTCCAAGGATGGAAGACTTGAAGATCTTATTCGTGCTTTAAGCACTG
CATGCTCTTTGGACGCTGATGAGACCCTTTTGGTGGCTGAGGTAAAGTGCAGAATTTCC
AGTGATGAGAAATGGTTATGGATTTCAAGTTGTTGCTTTATTGTTTCCTAAATAGAACTTA
TTACATACTGTGTATTGGATAGTCAAGTAGAGTCCTTTTTCCTATTTCCAAAATTTTATTT
CCAGCTCTTGCTGGGTTGTTGTTGTTGTATTTCCAGCTCTTACTCCATTTAATGTTACAG
ATATACAACAACCGCATTATACGTTATCTTGAGGAGCCAGCCGATTCATTATCCTTAATA
AGAGATGGTGACCGACTTGTTGCTTATCGGTTGCACAAGGGTACTGAAGAAGCCCCCT
TGGTTGTGTTTACGCATCAACAGATTGATGAGTATGTCTTGACTTCATAATTTGGGCATT
ATCTTTTTTTGCTTTAAAGTTCATCAAACATTACTAGCCATTACTCAGATGTGTCTTGCAT
GCACAGCTATGTTTCATAAGTAATAAGTTGGGGAAAAAGTACTCCAAGGGTGGTGCTT
CCACATCATCACTCTTAATCATGGCAGGGTTTGGATGTGGGCGTACTTGATGACTACAT
TGCTTAAAAGAATTGACAAAATATTTTCGCAGATGACATATGTAGTAATATCTCAGTCTAT
TAGTTTGCTTTATGGAGATCGGGTGATTAATTCATGATCGACACAACTCCAGTTAGTTAA
TAGAGTAGGCTGTTAGTTGTCATATACTTCTATCTTGTATAAAGTAAAAATGTGAGGTGG
TTTATTTAGTACTGTTGAGCATCCTCAGTCTCAATTCGCTTCACTTGAATACATTACAAAT
CATTGTTATGCATGGTTCGTCGAGCAACATGTAGTTCAGATGATGTGTGATCCTTCTA
GATTATTTGGACAATCATGAAACTATTGCTTCTTCCATGCATCTTACTGCTGAAGCTGTA
TATGATATGGAATTTCATGCTGTTTTGTTTGCTGATTATGTTTAGTTTAACTTTTGATCCA
TTGAAAGATTCTCATAGTGGTCCTTGACTCATTAAATGAGATGGCTGATATTTATTTTGG
CAAAATATCATTTCCTTCTTGATTTCCTCTTCCATTCTAGCAATCTTATGAAGACTGCATG
TGCAGGCATTATATATACGGAAAGCTGACCTCAAACATGAAGACATTTGGCATTCCGCT
TGCCGCGCATAGTAGAGTTCTTACAGGATCTGATATCCGTAGTCTTTATCTACAGATACT
TACACCATTCTTAGTCCACAATACAGCCCAAGCAGATAATCTTAACTGTGATAGAAGTGC
TACTGAAGCATGTACAGATTCAGAAGTCATCACAGACATGGAACCTGGCAACTCAATAG
TAAACGGGGTTCCAGAAAGCATTGCTGAAGAAGATACTGCCGAACCTTTAGACATGGAA
TTTCAATTTTACCTATCAGATGATAAGGCAACCTTTAAAGGCTCCGAGATTGTAATGAAT
GAGCCATTACAGTCCACAGATATCTCTGGACGGTTAAATGTACTTGTAAGTTGGTCACC
TAAAAATTCTTGAACAGTACAATACAGGCCTTTTCAGCTCACTGCCAGAAGTTTTTAAATC
TGGTTTTTTTGCCAAAAGACCACAAGAATCTGTCTCTCTGTATAAATGTCTTGAGGCATT
TCTGAAGGAAGAGCCTCTAGGGCCAGAAGATATGTGGTAAGTATGCAACTCCCTCACTT
CTGTGATTGTACACCATTCATATGCAAGCTATGTATTCATAACATATGAAATTTCTCGTAA
TGCTTCCCTTTTTGCTTCTTCTTTGGTTTGTGCTAATATTATAAACCCTCAACTTTTGTAA
TTACATAATTGTATTTTTCCAATTATACCATTTTATTTCATTTCTGTCAATATTTTCCACCG
CGTCGTGATTGCTTATTGTGGATAAACCATTCTTATTAGCCTCCCCTCAACCAAATTGGA
CCTTGACTTTGCAACATGGCATGAGTAGTATCCTTCCAACTTCTAGTTCAGTTATGTTAA
AGAAACAATGACAGCATCTGATGATCTTATGTAGCACCTATTGATTTTCTGCACATTGTG
CTTCTGAAATGCTTTATGTGTTGCTCTTTGTTTCTTGTATATTCATAGCAACTACAGACAG
TAATTGAGATAATAATATTCAGCTATTTACCTGGGGCCACTCATGGGAGGAAAGACTTCT
TTCATGAAATGTTTCTAGTATTCTATCTTATCATCTAATCATTTATTTGTGTCATTCGCTTG
GGAAGTATGAATATAATCTGCCTAACTTTCTTTGTCTTATATCCAACATTAGGTACTGCC
CTGCATGCAAGCAGCATCGCCAAGCTACTAAAAAGTTGGATCTTTGGAGACTGCCGGA
GATTCTGGTCATCCACCTGAAGAGGTTCTCGTACAACCGGTTTCTGAAGAACAAGTTGG
 
AGACGTATGTTGACTTCCCAACTCATGATCTTGATTTATCCTCATATTTGGCCTACAAGG
ATGGCAAATCTTCCTATCGGTATATGCTTTATGCAATTAGCAACCATTATGGAAGCATGG
GAGGGGGTCACTACACTGCGTTTGTTCATGTAAGTGGTGCTGCGACTTGGATTACCTTG
CTTCTTTTTCTTGGTTTTGTTTCTATTCTATGGTAAATAGGATTCTTTTATACCTGATAAAA
ATGGCATCTTAAGATCAGTACTTGGGGAGAAGGGTGGGTGGTGGGCGGTCACTGAAAC
CTACCTCCAAGGGCAAATATAGAAATTTCCTCTATTGGTCTTATTCTTATTGTTCGTAGT
GAGTGTTCCTTTGATGTATTTTTTAGTTCCAATGCATCATCTGCATCTAAATTAATCACAT
ATTGCACACATGTGCATCTATTATATATTTAACTTTGGTCATGCGTCTTCATTTTTTTTATT
TCTTCATCATGAAGAATATGCAAGAAGGTCAAATATTCAGACTTTTACAGTCTTCCTAGT
TTAATCCAGATATTCTAACTTTGTGTTTTTCTTCTTCTAATAATCTAGCAAGGTGCTGATC
GGTGGTATGACTTCGATGACAGCCATGTGTATTCCATCAGCCAGGACAAGCTCAAAACC
TCGGCCGCCTATGTTCTATTTTATAGACGAGTTGAAGAAATC
SEQ 72 
TTACACTTGCCTACTACACTCTCCTTTGCCAAAACCTACTCGTCGATTTCTCATATCAAA
TTCTACCCATAAATTTTGCTGGTGAAAATTACCAATAATATTGCTTGCTATTCCAAGTGAT
TCTGACCGTCCGATTCCAACACAATGGATCCCACCTTCTACTTCATCCAACATCCTTTCC
TTATTGATCAAAATATCAACCCCGTTTTCAAATTGCAATGTCATATCACCTATCAACCGTC
CGATTTCGATCGGACGGTTATCGAAGCACATGTCGAGTGCACCACCATAAACGTAACCT
TTTTTCAATCTTGGACCTACTAACCTAACAATTTCTTCTCTGACCTTATTGTACGCTTCTT
CCACTAAGAAAGTGTACTCCGTGCCGGAATCAATGATCGTCTGGCCGGAACCACCAGC
GTTTGGCCGGAAAACCCTCCCGGAGATGTTTAATTTTTTGCCGCCAATTTTTATCCCCA
CCATGCCAACAGTAAAAGCTAGTGGATCCAAATTTGGCATGCGTTGACTTTGAGGAAAA
GTCAAAAGATTTATGTATTGAAATGTATGGGAATTAGGGTTTTGGCCTAGGTAAAATGTT
CCACTAGGTTTAACTGCATGGCTACCTTGTCTAATTGGCACGCAATATGAGAATTTTTGT
ACCTTAGCTTGGGAGGCAAAAGAAAACCGTCCAAGATTCATTCCCAAAATACCCTCAGC
ATCTTCGGACTCGGTCGCACAACCAAGAATCAAAGGAGGGGTACTTTGGGAACGTGAA
AATGTAATTTTTTCACGGACAAGATTACCCTCAGCTAAAGTACCATCAGCATAAAAGTAG
GAATAGTGGCACAAACGATTTTGGTCACAAGTAGTTGGAAGGGTAAAATCGGGAATTCT
TGGCTTACATAAAGGATGAGTACAAGGAAGAACAGAGAAAGTAGAAGACAAAGAAGGA
TCAAACGACGTCGTTGGTGGGGGTCTTTTGGGAATTTTCTTATGACATTGAATCCAAGA
AAGTTGGCTACCAGTGTCCAAAACCATTTGTTGATTTTGTGGTGGTGTTCCTATTGGTAG
TGTAACAATTAAAGCCATTGAATATTTAAAAGTTGATTTATAGTTCAAAGATGGAATTCTA
GACATAGTTTTTGTATTTTGAGTTTGTCTTCTATTATTAGAAGCCATAAAAGAAGAAAGAA
AAAGAGCTTTAGAAGAAGAGTTATGTGATAAAGATGTTGAAATAAGAGGAAATGACATA
GAAAAAGGCTTATGTTTAATGGTTTTTTGTGCTGAGATGTAGAGAAAATTGAAGATTATG
AGAAGAAGAAGAACAAAAACTCTAGAAGAAGAAGCCAT
SEQ 73 
TACACTATAATTATATTTTCGTTAAATATGAAGATTTTTTCCATATTCTCTTTGCTTCTTCT
CCTTCTCCTTCCCATCTTGGCTTCATGTCATGAAAAACAGGTACAAGCATATACAATTCT
AGTTTCTCATTGATTCTTTAATCGCAGTTCTACTTCTGTTTATTCTTTGTTTTAATTATGGG
GTTTTGTTTTGCAGGTTTATATAGTGTATTTTGGAGGACATAAAGGGGAGAAAGCATTGC
ATGAGATTGAAGAAAACCATCACTCATATCTCATGTCAGTGAAGGAAAGTGAAGAAGAA
GCCAGATATTCTCTTATTTACAGTTACAAACATAGCATCAATGGCTTTGCTGCACTTCTC
ACCCCACATGAAGCCTCCAAGTTATCTGGTATAATAACCACGAAAAAAGTTCACTCTTTC
AAAGAAAGAGTTTAAGTTACATATAGTAAAATTTAATTGGTTATAGCAGGTTATTGCTCTA
 
TTTTCTAGGTCAGAGTAACTTGTTTTCATATGTCAAATTAATCTGATAGTGTAAAAAATCC
TGTATAAGAAACACAAGGTTCTTGTATGTAGAAGAACTTACCTTATGTATTATTTGAACA
CAGAATTGGAAGAAGTGGTATCGGTGTATAAAAGTGAGCCAAGGAAATACAGATTGCAA
ACAACAAGGTCATGGGAATTTTCTGGAGTGGAAGAGTCAGTGCAACCAAATTCCTTGAA
CAAGGATAACTTGCTACTGAAAGCCAGATATGGCAAAGATGTCATTATTGGCGTTCTTG
ACAGCGGTACATACATATATATATTTGCTTACCATTATTTCCAATATGGCATTATTTTCCCTT
TGTTTTAAATTTTAAATGTATTTCCACAAAGGGCTACATAATCTAGCATGTGATTATCGTT
TCTCCAATAGTGATACAGACAATCTTATTAGTAAGACTAATGCCTTGTATGTATAATAGTA
GAAAGGGATAACACGTGAGGAATCAACCTATATATATATATATATATATATATATAAATGT
ATTTCAAAAAATACTACTTATAGACATATAAGGAAAATTGTGAGAAGCCTTGTACCAAAG
GGAGTCTAAAGTTAAAATAAAAATTCAACATGTTTAAGGATTATGGTTATATAGGATGGA
CGTGTAACTGTGTCTATCCTCCGGCTTATCACTGGCAACTGAACACGAGGGTTGCGCT
CGTTGCGGGACTCATTAATTATGAGATTATCAACTGTAACTAGTGTTAATTGACTAGTCT
GATACTTAAAAAAAAATTGGAGTATGATATTATGTGATGAATGTTGTTGGATGATTTACC
AGGGCTATGGCCAGAATCTAAGAGCTTTAGTGATGAAGGGTTGGGACCGATTCCAAAG
TCATGGAAAGGAATCTGCCAATCTGGAGATGCTTTCAACTCTTCAAACTGTAATAAGTGA
GTGTAATTCCTCTTCCATATGTTTTATATCTTTCCTTTAACTTTTTCTTTCTTTCTTTATCTT
ATCCCTTTTTATTATCTCGATGATCTGATGTCTACCTGTTTTACAATGATTTAATGTGGAT
TTTAGCCATTCTTGGGTTAGAAAATGTTCAGCTGCTCTACAACCCTAGACCACATTCTTT
TTGTTTTGGGAATTCCTGCTAAAATAAGCTGATTTACTACCTTAGACGTTTGGTTTATCAA
ATATACCAACCTATACGTATTTCTTTATTTTTCTTTTTTTAATAAACTTTATTAAATTTTATA
AGGCTGAGATGACTTTGAACGAAAAATATGATTCATTTAGTTTAAATCCAACTTATTTGG
AACTGGCATAATAGTTGTTGTTGCTATTAAATTTCATAAGTAGGCTTAATAAACATGTCAT
CAAGTTTTGTGCGCACCTATCATATGATGCCTTGTTTATCCAATTATGGATTTCAGGATT
TGCTTGGAAATGAAGTGTTTGGCACTTATCTCTTGTCTCTTATACATTGATCTAACTTCG
TAAGATTAATTGTATTTAAATGGCTTGTAATAGAAAAGGCCAAAGGTCAATTTCAAGGCC
GATTTTTGGAAGTTTTCCTTTGTCTTCTTTATCAGTTGACCCTAAACCATTCTCATAATTT
AGCTTAATTAAAATCAATTAAAAGAAAGCAGATACATGTTTAGTTTTTAATCTTGTACCT
CTCTAAAGAGTGAAAGAGAGTTTTTTTGAGAGGACAGGACCCATTGGGTGTCCATGCCT
GTCCTTTGGTGGCCTTAGGATATCAGTGTAATAATTTCAATATTGTCCATTTCAATCAAA
CCAAGAGAGGTTATGCTGACAAGTTGCTAATTGTTTTTTGGATTCTTGCTTTGCCATCTT
GTGAACTTTGTATCCTTCCAATGCTTTGTTGTGCAGTAATTTGTTTTTTGCATGTGTGTTG
TCATTATGGTTATTGTGAAGTCTATAGTGAAATTTTGTGAGGCCCTTACTTCCAGTTTTG
CACGGATATTCTCAGTAGTAGCCAGTAATATTATCCATTTTGACTATCTCATGACTTCCA
TGCAGCAGCTTTTTGACCTTTAGAAAGTTGATGATGAAATTCTACCATTTTAGAATGATA
AGTCATTTTCTAGCTGTTAAGTCACAAAAAGAGCACTAGAGCAGTAAAACTTTTGAAGTT
TCATTGTGAGGTTGGGAGGAGTGGGCCTGATTATCACATTCTTGTCCTAATTTGTTACT
GCTACTATCCTTTTTTTTTTTTTCTTATTAAGAAGAAGAAAGCCTTTTCTTCCCTTCTTTTCA
AAGGGTAGGGGGTGGGTGGAATATATTAGCCTAATTTGTCATATTTTCCTTCTCGTATAT
AACCATGCTACTATATATGTTGTACTCAAAATATAAGATTTTGTATACCTTTTCCTCTATA
TACTAGATAGTGTGATCCCCTCATGCATCATCTTCTTTTCTCTAGAAGAAAATGTTTTATT
CATGGTGACAGGGGAGGGAGAGGGTGGGAATGTTGGGATCATATCTTGATATCTTGTC
TAATTGATCATCTCAGGCAAATTTAGGGTGGTCATGTGAGTTAAACTAAATAATTTTATTT
CATAGGATCAGCCCGCCCTGATCAAGAATTACTTACTAGCTAGCCAGACTAGTGGAGC
CCTAGCCGGAGACATTCTCTAAATCATGCCTTAACGCGCCCATCTTCCAAATAAAAAAG
GGCTAGTTAGTAAGAAAGATGGAAAGACCTTTATCCATAATTCTTTCCCAGTCTACCTCC
TTCCTTAATTGTGACATGTCCCGTTGATCCCACCTACGAGCTATCTGTCTTTGCCTAGCA
AGATAATTTTTGGTCTCCTATTCTTGCCTATTTTTATAGCCTGTCTTTATCAAGCGAGATA
ATTCTAGTTCTTTTATTTTTGCCTATCATGGTAGGAAATTGGTTCGGCTTGATTGAAATTT
TTTAAAATGTTTACATATAAAAAGAGTACACGCATTCTGAACCCACCAACTCTAAATCCT
GAACTTGCTTCTCCTAATTATGTAAGATAACTTTAATATTTATTCTCCTATGCTACTTTGG
 
GACTTCTATTGCAGGAAAATAATTGGAGCTAGGTACTACATCAAAGGTTACGAGCAATA
TTATGGCCCTCTAAACCGAACTCTAGATTATCTATCTCCACGAGACAAGGATGGACATG
GAACTCATACATCATCAACAGCAGGAGGCAGAAAGGTTCCAAATGTCTCTGCCATTGGT
GGCTTTGCATCTGGCACCGCCTCGGGTGGCGCGCCACTCGCACGGCTAGCAATGTAC
AAAGTCTGCTGGGCTATTCCGAAGGAGGGCAAAGAAGATGGAAACACTTGCTTTGACG
AAGATATGTTAGCAGCAATGGATGATGCTATTGCAGATGGTGTTGATGTTATTAGCATTT
CTATTGGAACAAAAGAACCTCAGCCTTTTGATCAAGATAGCATTGCTATTGGAGCACTTT
ATGCTGTGAAGAAAAACATTGTTGTGTCTTGTAGTGCAGGGAATTCAGGACCTGCACCT
TCTACATTGTCTAACACAGCTCCCTGGATTATCACTGTTGGTGCTAGCAGTGTTGACAG
AGCATTCTTGTCACCTGTTATCCTAGGAAATGGCAAGAAATTTACGGTAACACGATAATC
TATTCATTTTCTGTACACTATTTCATCTAAAATGTTGTAACACTAGGATCATAACGTTTTC
CTTTATCTATTTAATTACATTCATATTGGAATGAAATTGAATCCATTTTTCGTTTGCTTAAT
ATCAGGGACAAACAGTTACACCTTACAAGCTCGAGAAGGAGATGTACCCTCTAGTTTAT
GCAGGACAAGTAATCAACTCTAACGTAACCAAAGATGTAGCAGGGTACTCTCCTTGCCT
CAAAGTTTCAATATTTTTAATTAATAATCATAATTTTCTTTTGGTTGATTATGTTAAACACT
ATCTGAAACTTTTTCAAAAAAAAAATTCAGGCAATGTTTACCAGGTTCCCTTTCGCCGAA
AAAGGCCAAGGGGAAGATAGTAATATGCTTGAGAGGAACGGACAAGAGTAGGAAAA
GGTGGAGAGGTGAAAAGGGCAGGAGGAATTGGTTACATACTAGGAAATAATAAAGCAA
ATGGAGCTGAATTAGTAGCTGATCCTCACTTTCTTCCAGCCACTGCAGTGGACTATAAA
AGTGCAATGCAGATTCTCAACTACATCAATTCTACAAAGTCCCCAGTGGCATATATTGTC
CCAGCTAAAACAGTTTTGCATTCTAAACCAGCACCTTACATGGCTTCCTTCACTAGTAGA
GGTCCAAGTGCAGTTGCACCTGATATCCTCAAGGTCAGAATTTACATAACAAACTTAAG
ATATTTACCTGACTTATGATTTATGCTTCCTCATCTAAATTAAATTCTGATTTTCGCTACTT
CCACAGCCTGATATCACCGCACCAGGGCTGAATATATTGGCAGCATGGAGTGGCGGAT
CTTCCCCAACGAAACTAGATATCGATGATCGTGTGGTTGAGTATAACATAATCTCAGGT
ACTTCCATGTCTTGCCCACATGTCGGTGGCGCCGCTGCACTTTTGAAGGCTATACATCC
CACTTGGAGCAGTGCTGCAATAAGATCTGCTCTTATAACCTCAGGTACCTCTCAACTAC
TTTTGAACTTAACTTATATACACTAACTACAGTATTTTAACCTGTTATAACATATATAGTTA
TTTTGCTGCAGGTGACCTGATAGTGTGTAAACATTATTTTACATTGTCGGTGTATAGAAT
TTAAACTCCTTTTTCGTCCAAAATTTTGTATTTTGAACTGATCAATCGTTATATTTTCAGCT
GGATTACGAAATAATGTTGGTGAGCAAATAACGGATGCATCAGGGAAGCCAGCAGATC
CATTCCAATTCGGAGGAGGGCATTTCAGGCCATCAAAGGCAGCAGATCCTGGACTTGT
CTACGATGCTTCCTACCAAGACTATCTTCTCTTCCTTTGCGCTTCTGGTATTAAGGATCT
TGACAAATCCTTCAAGTGTCCCAAGAAATCACATTTACCTAACAACCTAAATTATCCATC
TCTGGCTATTCCCAATCTCAATGGTACTGTTACTGTTAGCAGAAGGTTGACAAATGTTG
GTGCACCAAAGAGTGTTTACTTTGCCAGTGCTAAACCTCCATTGGGATTCTCTGTTGAG
ATTTCTCCTCCCGTCTTGTCTTTTAAGCACGTTGGTTCGAAGAGGACGTTCACTATTACA
GTGAAAGTTCGAAGTGATATGATTGACAGTATTCCGAAAGATCAGTATGTGTTTGGATG
GTATTCCTGGAATGATGGAATCCATAATGTTAGGAGTCCAATTGCAGTCAAATTGGCA
SEQ 74 
ATGGCAACACGTAGAAGCTCTAGCTCTGCTCTCACGGCCCTTGCGGCGTCTCGTTCCC
GCCTACTCTCGCGGTTTCGTCCTGCAGTTTCTCGTCTCTCTCAGAATACTTTACTCGGC
ACCGGCAGGTGTCCACCTCCCAATAGTGGATTTTTTGTTGCAGAAACAACTGCTGCACT
TTGGCCGAATTATAACGTGTTGTCCAAAAGTTTCGTGCACTCTTACTCTACTACTGCTGC
TAGCTCCGGACAGGCACGACTTTCTTCTTCCTAATTGCATTCTTCTCTGTTCAACGACTT
TTCTTCTTCCTAATTGCATTATTCTGTCCGTTCAATTGGAAGTGCTAATAGAATTAACTCT
AATTGACGTTTAGATTAAACTTGAATGAATGCTGTTGGTTCTTTTATTTAGCTTTTGATGC
GAAGTGAAGTAATCTCTATTTAGATATTGTCAGTTAGAGAACTATTTTCTCAACGTTAAG
 
GAACATCATTTCCAGCCTTTTTTTTTTTGCAGAGTGGAAGCCTTAAATTGTGTATTTTTG
GACGAGAAATAACAAAAATGGTCCCTTATATGTGGGGTAGAATAAAATAGTCCCTTAATA
TACTCCTGAGCAGTTTTGGTTCTTCAAGTTTGCAAAAAAGTGAGCAGTTTTAGTAGTCGT
CAATTATTTTAACAAACTCTGGTTGTTTAATTTGACGAATACGAAGTCGCATTTGGAGGT
GCATTTTTTGCCGTTTATGATATATTTGGTCTATTTCTGGTGTCTGATGGAGTTTCTGGG
ATTTAATGGGTCTTTCCTAGTGGTTCTAGTTAAATCCTTTTCTTTTTCATAGTTTCGCTAA
ATCTAGCAGCCAAATTTTGATAAACAGTTGACAAGATAAAAAATGCTCATGCGTGGCAAA
CATAGATCCTTCTGATAAGCGTCAAGCAGTGGAAAACACTTTTAGGTGCTGAAGTGGAT
TTTTATAAATTGGCAGTTACGTGTTAAGTGAGAAGTGGAACTGATAATCAATTAGTATGG
TTGGTAAAAAAACTGTTGATAAACACTTTTTTTGCTAAAATAACTGTAATGACCTTAAAGT
TATTTACAAATTCTATAATTTTAAAGTATTTATTACATAAAAAGACGAAAAATAGAGGTAAT
TAAAAGTTATGTTAGAAGAATATATTGGAGATTACAAAAGATCATAGGGATAAAATCGTA
AAAGGCTTGGTCAAACAAAAAATGTTTATAAGGTATAACTTTTGACTGATTTTGGCTTAC
AAGTTCTTCTCGTACGAGCACTTTTGATGTTTATCAAACGTGTAGATAAGCCAAAATGTG
CTTACAAGCTAGTAGGACCCTCTTATAGCTTAGACAAATACATGTATTTAAGAGTCTATT
TTATACCTACCTGGNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNN
NNNNNNNNNNNNNNNNNNNNNNNNAAAAAAATAAAAGTTACTACTTTTGTCATTTTCTAAT
TGTTGGACAGGCGGTTTCATTCTACTATTTTTTGACAATTTCGTGGGTCATCCTTGGGGT
AGGGTAGGCTGCCTTCGTCACACACCCTTGGGGTGCGACCCTTTCTCGGGCCCTACAT
GAATGCACGATGTTTCGTGCACTGGACTTCCCTTTGTGGAGTACACTTTATCAAAGTTA
CGTATAAACACATGCACTAAACTAGACTTTTGTGATAGTTGTTTCACTTGAGGCTGTCAG
TTTACTTCTTTTTTTGTTTGGCTGCTATTGGTGTTGCCATAAAAACTACTCTAGAGTTTAC
TATTGATGCTTAGAACTTATGGGGTGAATGAAAGGACGGGTGACTGAAAGGACATCTCC
TTCCTCAAACGTGTTATATCTTCTTAAACAAGAAATTTTGATGTACTCAAACAATACTAAG
GGGGGTTGTGCAGATAAAAACAGCTATTTATAAAGGTCCATTGGTCTCCCTGGCCTTCT
CTAGAATGAGGAGTGCCATGATTGTAGATATTTACCTATACCATATCGCCATTTGTCAAT
TCTCTTGGATTTGTCCTGATGATTACTTATCTTTAATTTTGGGTAATTTTAAATTGTTTTG
GCATATTATTTGTTGTTATGTTAATTAGTATGAAGATTTAACAGATTAATAATATGGACTA
CACCGAGATGGCTCTTGAGGGTATTGTTGGTGCTGTAGAGGCGGCACGGACTAGCAAG
CAACAAGTAGTTGAGACTGAGCACTTAATGAAAGCTCTTTTGGAGCAGAAGGATGGGTT
GGCTCGAAGAATATTCACTAAGGCTGGGTTGGACAACTCATCAGTTCTGCAAGAAACAG
ATCAATTTATATCTCAGCAGCCAAAGGTATGAAAAATGGAGACTGATTGTGGATTCTGAT
GAGTTCTTGGACTAGAGAATCAGATATTTTTTCTTGCAGGTAGTAGGTGATACTAGTGG
CCCCATATTGGGGTCACATCTTAGTTCTCTCCTAGAGAATGCGAAGAAGCACAAGAAAG
AAATGGGAGATTCCTTTGTGTCTGTGGAGCATATGTTGTTATCTTTTTTGTCAGACACAA
GATTTGGTCAAAAGTTATTCAGGAATCTCCAGCTTACGGAGAAGGCTTTGAAGGATGCT
GTCAATGCTGTTCGTGGAAGTCAGAGAGTAACTGATCCAAGTATGTATATATTTATATAG
CTTCATGTTTCGTGGCCATGTCTCTTATGATTTCATTCGTTCTGGTTGAATGGTAAATCC
CAGTTGGCGAAAGGATCTTACTTTATCACAGCATGAGATGATCCTTACTTTGGTTGATTG
GGTGGATCGTGCAATATTGTCTCTCTGGTTTGTCATGGACATTAAATTTTTCCTATTGAT
GGGGTGGGGGGGGGAGTCTTGTAAGGTGGTTAGAGGCTTAGAGCTGATAATATTTCAA
AAAGTACTTGGCAAAAAAGGCTAATTCCAAGTAGATGGTATAATACTCCATTCACATGAA
CTTAGATTTAAATCTGAATTTGAAGAATGACTTTTGATATCAAAAGGTAGAATGAGGAAT
TAGCAAAATTGCATTGAGGGAGTATGGATGTCCTTGATATATATGACAATGCATATTACT
GTCTCTTAGTGACTACATCATGAAGTTTCAAAATGATTGACCATGCTCAGTGCAAATAAG
ATATCTAATTGTGTCAAAATAGATTTTAGTATGGCTCCGATATTTATCAAGATCTTCCAGC
 
GGCTCTTCCTGTCTAGGAATGAGTTTGAAATCTCGATCCTAAGGGTGTGAACATGTTTG
CAATCTGACACGTTTGTTTACTATAGTCGTCAAAAGCATGCTTTGTGAGATAGCAATGCC
TATCTTTGATTGTGCTACCTCAATCTCCTGTAATTTTCTCAAATCCTGAGCCTTACAGCA
ATTGAACAAGTGTTGCTAGAGTTAAGAAACTATTTGGTCATTGCCTTCTATAATAAAATG
TCATCAACATAGTCCACTAGTAAATACATTAACCACATTGTTGTGAAGAGCGTGAAGTGA
GAAAAAGCGACAACCCCCATTTCGCTTAAAGCGAGAAGCGTAGCACTCACTTTTTTGAA
GTGAAGCCGAATTTTCAAAAAAAAATTAAAATAAATACTGCATAGACAACACATGTAATT
GTAAGCAAATGTTCAATACTTCAATGTAAAAACTAAATAGTAGCATCAATTAAAGCACAA
AATGAGCATCCTATTCTTCTACAAGATTGTGAAATTCTTGTATTCCACTATCATTATTATA
TTGCTCGTCATCTTCTTCAACTTCTTCTTCTTCATCAACTAGGGACAAAGTAGCCGCCTC
TTTTCCCTTCCTCTGTGAACTTGAAGTTGAGGTACTCCCCTTCAAACCATAAATCCTCTC
CCCAATTTCACACGCCTCCGCAACATCACCCCAAGTGAAATCAGAAGTTTCCTCAAATA
CTTCTTCATCTGCATGATCTTCCGGGACTCCAATTAGCCATTCATTAGCATCATTGATGT
TGTCCAAACTAATTGGATCAATTACATTGCGAGCATTGTAACGACGCCTCAATGTTCTAT
TGTACTTAATGAAGACTAGATCATTGAGACGCTTCAAGGTTACTTTGTTCCTCTTTTTGG
TGTGGATCTGCAAATAATAAGTAATTAGTAAGATTAGGACAATTGAGATATAATTTAATTA
TCTCAATATACTAAATCTTTCTTCTTAGTTCTTACATGTTCAAACACGCTCCAATTCCTTT
CACACCCGGATGAACTACATATTAGACTTAGAACCTTAATGGCAAACTTTTGTAAATCTG
GGGTGAAATGGCCATATTGCTTTCACCATTCAACTGTAGAAGTAGAACAAATTTTCAAAA
CATAATATTGATAAATTAATAACTTATTAACTATAAAACAACATATAAGCACTCGTTCACC
TGGTGACTTCGTCTTTTTTTGTCTAATCGCCATGTTTTTCCAAAAGTTGCTCAGCATTC
CTATAAATACTAAATTGCTCTGTTATTTTATCTTGCACGGATTCTTTGGGTATCAACTTCT
CAGTACATTCATAGTATCCATTCCACAAATGTTCATCTCCTAGAATCCTCTCTTCATTGTC
ATAAAACAGTTCCGGGTTCAAAATAAGTCCAGCTACATGCAAAGAGCTATGAAGCTTAC
TATCCCACCTTTTATTTATGAAATACACTTTTGTATTTTCTTTGATCACTAAAAGAGACTT
GAATAGCCTTCTTTGCCCTATCCATTGCTTCGTACATGTAGCCCATTGGTGGCCTTTGC
TCCCCATCCACCAAACAAAGCACTTTAGCTAAAGGACCACCAATCTTCAATGCATGAAC
CACATTGTTCAAGAATGAAGGAGAAAGTATAATATCTGCAAATTCTCTCCCTCGAGCTTC
CCTTCCATAGGCACCGTTAGTGTACTCATCTGAAACAAACAACTTCTTCAAATTGCTTTT
TTGCTCATACATCCTATGCAAAGTCAAGAAAGCGGTAGTAAATTTTGTCTTTGCAGTTTC
ACCAAGCTTCTTTGTTTAGTGAATCTCTTCATCATATTCAATAACAAAGGCCTTTGAACAA
TAGAGGAATGCACTCTAATTGCCTGATTAAAGACTGAATTGATGGGTCTTTCCTTGAAAA
TGTCACCGAAAATCAAATTAATGCAATGTGCTGCACACAGAGTCCAATAAATATGCGCG
TACCCAACAGACATCAAATCACCAGCTTTAACATTTTCACTGGCGTTGTCCATGACAACA
TGAACAACATTTTCTGCTCCAATAGAGTCTATTGTACTCTTGAACAAGGAGTATATTTTG
GTTGAATCAGTCAAAGAGTCGCTTGCATTAACGGACTCAAGAAACATACTTCTTTTAGGA
GAATTCACCAAGATATTGATGATCATTTTTCCATTTCTCGCCGTCCACTTATCCATCATAA
TGGAACAACCAAACTTGTTCCATTCTACTTTGTGATCCTCCCCGATTTTGTTCAACTCTG
CCGCCTCTTTTTTTTAGATATAGACCTCTAACTTCATGATAAGTTGGAGGCTTCATTCCT
GGACCATATTGGCCTACGACATCAATAAAAGCAGAAAAAGTGTCAGTATAATTAACACA
ATTAAAAGGAAGACCTGCATCATACATCCACCACGCAAACATTGTGACTGCACGAACCC
TCAAAATCGCTTTGGCATCAATTTGAGGATTACCACTTTTTCCTCCCTTATCCCCAGATT
TTTGCGGGAAGTAACAATCCATAGGACCTTTGGTCTTGCCAGTAGATCCACAGCTAGAA
GACATTGGTTGCATCCTTCCCTGCTTTTGTGATTTTGGTGGAAGCGACGAAGCATCGTC
ACCTTCTTCTGTTTCATCATCGTCATCATCATGATTATACAGTTCTTGTTCATGAATCATT
TGAGTCTTTAACTCTTTTTTTTTTTGAAGGAATGCTTTCAATTCTGCCTTCACATGCGATG
GAACTTTAGGACAATATGCGACATTTGGATCACCACCGATTAGATGCGCTATTTGACCG
ATAGATTCCCCCATTTGAAATCTTGTCACAAAAAAGACATCTAATTGCCATCTTGTTTTGT
TGGTCTCGCTAACTCTTTCAGAGTAAGTCCAAGCCGGATCTTTCCTATCTTCTTTTGGTG
CCATTAAAAGAACAACTACATAACACATAAGAAAGACAATAAGAACTAAGAATAAAGGAT
ATAAAAATTAGAGGAAGGGCAGTAATTCTCTTTTAAAAATTGGGCAGAGTTAAAAAAAAA
 
AAAACTAGGCAGTAATTCTCTGTTAAAAATTGGCATCAATTCTGAGAAAGAACCGAAGGT
TAAAAAAAAAAATTTGCCAGCATTTCGCTGCTTTTTGGACGTTTAAACAGTGAAAGAAAA
AGAAAAGAAGAAGAAGAAGGAGGAAAATCAGAACATACCTGTTGCTGTTGAAGACTTGA
ACTTGAAGTTGAAGACTTGAACTTGAAGTTGAAGTTGAACTCTTGAAGAAGAAGAAGAA
CCCCAGTCGATGCTTGTCGATGCTTTCAGAAGTTGATGAAGAAGAAGAAGTCGACTAGT
GTCTCTGCTTTAAAACCCTAGTCGCATTTGTTCGTTTAATGAAAGACCAGACATCTTGTT
TTTAATAAAATAGGGTTGAGTCTGATTTAAAACACAGAAGCGATCGCTTCTCTCGCATCG
CATCGCTTTCCTGCTTCTCGTTTTTTAGTGGGAAGCGGTCGCTTTTCTACACCTAAGTC
GCTTCACCCTGTTGAAGCGTGCACTTTCTTGCTTCGCTTCGCTTCTCGCTTAAAGCGAG
GAAGCGGACGCTTTTTTAAACACTGATTAACCATGACCATTATAATTGTATACGGGTAAA
ACCGAGCCCATGATGCACCTCGATTTCCGACAAGAGAAGCCAGGCTCGAGATGTGATG
GCAAGGGACAAATATCAAGCCGAAAGTCCCATTGAGCCAGAGCCCTGGGACACGATGC
CTGCCCTCGGGAATATCGAGGTCATAATTACAGAATCGGTCCTAACCTCGAACAACTTC
GAGGAACATTATCGGACGATCAAGCGTAGCCAACAGAAAGCCGAAATATCCATGACCG
GCCGAGTATCACGACGGGGATCTCGGCACGTATCGATAAGGAACCTTCAACCAGTTAA
TCAGAAGACCTTTTACCTTTTACAGAGTTGTACCTAAAGTAGGACTCCCCTACTATATAA
AGGGGGTTTGATAATTCATGTAACACATTGAAAACACGCGTTCCAAGGAAATATATTATC
ATTTTCTCTTTTATCTAGCTTTTTTCACTTGTTCATCAGTGTTGACTATAGCAAGCCCGGG
ATCGAGGGTGAACAATTTTACTAAGGTTGAATCTGTCTTATTCGCATGGTTTGAATTCAT
TTTATCTTTACTAGTTCAATCTAATCCAATTTATAGCTTTGTGTCAAATTAATCCGCGTAT
CCTTAAAACCACTTATAAATTCAATTGTTATCCGATTTTGAGGGTAAACAATAATGAAAG
GAAATTGAACGATCAGCTTCACTTTGGACACTTCCCATGATATTGGACCACGAACTGAA
TTAGAGAAATTCCAAGCCACACTCCCTATAAGTGTTTTAGACCATATAAGTGACTGTCAA
CAGCATACTAAGCCTGAATCCTTTGAGCAACAAGCTAGAGTGGTTGCTTCATGTACACC
TCCATGGCTAATTCTCCATGCAAGAAGGCATTTCAGATATCCTGTTTATAAAGTGTACAT
TAAAACATTGACGCTAAAAAGTTAAACAGTTGTGTGGGTGGATACCATATAAGTAGCGA
CGTCCAAAAAACACTTACCAGAATTAGCATGAGTTTCAGATTCAAATTCCTGCGGAGGC
AAAAGACACTAGGTGATTTCTTTTTGTATGTCCAAGTCTTGGTGAACATATGTGAGAAAG
AAAGAGTTTGAGAGAGAACTCAGCTTGATTATTACCTTAAGCAGTGGGCTTTAAATAGCT
AAGTGCACATATTCTAAGTATTAGATCAAGTTTGAGACCTAGTAAACTTCCACAGGAAAA
GGGATAGAGTCAGATACCTAAACCTAGTTATACTCTAAAGTTTTATGTAATTAGACCTC
CTTAGTCTCTATCCTAATGGTATAGTCTCATCTGTGCTGTCCCAAATTAATATATTGAAG
AGAAATTCAATCCCAAAGTTGTGTGTGAATTTATATGGTATCAGAAGCCATGTCGATATC
TTCCTCCTCCTCCCCTCCATCACCTACACTCGTTAACCCTCTTTCTTCGTCATCTTCCTC
GCATGCACCCCTTGACCATGCTCATCACTTCATTTCAGTTAAATTAACTTCTACGAAATT
TTTCTTTTTTGGAAGACGCAACTATTACATTTTCTTCGAGGACAAAATCTCCACAAAAACT
CCACGGCTATATTGATGGAACTAATCCTTGCCCACCATCACACACTACGGTTGAAGGCA
AAGAAATACCAAATACAACCTATGTATAATGGATCTAACAGGACCAATTGATTCTTAGCC
TGTTGATTTCATCACTTTCCAAAGAAATGTTGCCCATGAAAATTGGTTTAAATACCTCCA
AAGCAGTTTGTGATGCACTTGAGGCAGCCCTATCCGAACCTTCAAATGCACGAATCCTC
AATCTTCATATGCAACTTTAAAACTTGAAGCAAGAAGATCTTTCGGTTACTCAATACTTG
CACAAGGCCAAACTCATCTCCGACGAGTTGGCAGTTGCTGCAAGGCCCCTTCGTCTTG
CCGATCAAAATGTGTACATCTTTAAGGGACTGAGATCTGATTTCAAGGACATTGTTACAA
CTCTCTCAGAACGACATGAACCAATCACATTCTCAGAACTTCACAGCCTCTTGCTTAACC
ATCAATTTAGACATGGTTCCTCTATCTCCTCACTTTCCTTAACCACCCCAAAACCACCTG
CTCTTCAACAATACCCACAGCTAACTTCAATCAACGAACTACAAATCTGATCGTAATAAT
GGTTTCAATTCAAATAGGGGACGAGGCAGATCTTCGTGTGAAAGAGGGGGTAGAGGTG
GTTGTTCATCCTCAAGGAATTTCTCTAACAATGGACAATCTTGGTCTCAATATGATCAGC
GAACCCGGTGTCAAATATGCAATGGTACCAACCATCTTGCATCAACTTGCTTCCAGAGG
TACAATCACTTGATTAACCCTATGGCTTATTTGTCTAACCAAGCTCCTTTACCCTCAACTT
TGCAATGGTTTGCGGACATTGGAGCCACTCGCTACATCACTTTGGATCTCACAAATATT
 
CATCAAGTTGAAGATTATAGGGGTTCAGATCAGGTCCAAATTGGCTATAGACATGGCCT
TTCTATCCATCGCACTGGTAACTCCTCTCTCTGATCACCCTCTTGGTCTCTCTATCTTAA
GAATATCCTTCATGTTCCTTCAATTACCAAACGTTTACTCTTTGTTCAACTTTTGCTCGTC
ACAATAATGTCTTCGAACTTCATCCCTTTCATTTTGTTGTCAAGGATCTACAATCCAGGA
CACCTCTTTTTACAGGGCAGAGTGATGGCGATTTATACACACTTCCATCCAAGTCTTCTT
CTTCTTCCATCTCCCAGCCAGTTCCAGCCTCTCCAACAGCTTCTCTATCCATCAACACAT
CACCTTCATGCTGACATCTTCATCTTGGTCACCCCCATCAACTAGTACTTACGCAGATTC
TTAGGACCTACTACAATCTGAAATGAATGCTTTGCTACGAAATAATACCTGGTCTTTGGT
TCCTCATAATCTTTCAATGAATGTTTTAGGATGCAAATGGGTGTTTCTCATTTAAAAAAAT
TTCTATTGGGGCAATTAAGAGATGAAAAGCCCATCTTGTGGCTAAAGGTTTTCATCAACT
TGAAGGCCAGGACTACTCTAAGACTTTCAGTCCAGTTGTAAGGCTGCAACCATTTGCAT
TGTTCTATTTTTAGCAGCTTCACATGGGCGGTCTCTCCAACAATTTGATGTGCAAAATGC
AGTTTTACATGGTGAGCTTCAAGACCATGTGTTCATGAGCCAGCCTTCAGGTTTCATCC
ATCCTCTTTTTCCTCATCATGTTTGTCAACGTAAGAAGTCACTATACGAGCTCAAATGGC
TCCCAAGGCATGGTATATGCGTCTCCATAAGTTCTTGCTCAGCGTAGGCTTCATCACCT
CTAGATCGGACACTTCCCTGTTTGTCTGCAACTCAAATGGTGTTGTCGCCTACCTCTTA
GTATACGTTGATGATACATAGTCACTGGCAGTGGTACCTCCTTTTTAGAATCCATTTTCC
TCAAACTTGGAGATGTCTTTTCCATATGTAATCTTGGTCCTCTCAGTTTCTTTCTTGGTCT
TCAGGTTTCACGTGATCACCATGGCATCTCTATGTCCCAAGCTGAACACATTAAGACTA
TTCTTGCAAGAGCACGTATGTAGCACTGCAAACCTTTAATTACTCCCATGGAAGTGAAT
GTCAAACTTCACAATGGAGAAAGTCTTAGCTTTCATGATCCTACCTTGTACTGTCATATT
GTGGGCCTTACAGTATGTTACTCTCACTTGGCCGGACTTAGCTTTTGTGGTGAATAAAG
CTTGTCAATTCATGCACAATCCTACTATGAGTCAGTGGGCAGCAGTCAAGCGCATACTC
TGCTATTTGATGCATACCCAACGTATGTGTTTTCACATTCCTAGGTCTCTTACACTCACT
TTTCAAGCCTTCACACACTCAGATTGGGCAGGTTCACTCGATGATCGTAAGTCCACTAC
GGTTATGCCATTATCTTGGGTGAAGCTATTCTCATGGTCGTTCAAAAAGCGGCGCATTG
TAGTAGATCTTCCACAGGTTCAGAGTATAAAGCTTTAGTAGATGCAGCTGCCGAGCTGA
CTTGGATTCTGTCTCTCTTGTTTGAGCTTGGTGTTTAACTTCCCAAAGCTCCAATTCTAT
GGTTTGACTACCTATCTTTCGGTAATCCTGTGTTTCATGCACGAACCAAGCATGCGGAA
ATTAATTTTCACCTTGTTAGACAAAGTAGCTCGAAAGGATCTCACAGTTCAATTTTTATCC
TCCAAAGATCAGCTTGGTGATGTCTTCACAAAGCCACTAGCTTCCTCTAGATTTGAGTTC
CTTTGGTCGAAGCTCAATGTGGTTTATCCACCTCAGCTTGCAAGGGAGTATTGTATCAA
CTTTGAGTCCTGGTAAACTTAGGATATAGTCGGGTACCTAAACCTAGTTATACTTTGAAG
TTCTTATGTAGTTAGATCTCCTTAGTCCCTATCCTATAGTATATACTCATCTCTATAAATG
TACGACCGCTGTACCAAATTAATACATTGAAGAGAAATTCAATCACAAAGTTGTGTGTGA
ATTTATACTAAAAGGAAAGAGAATTAATCACAATGAAAATACAATCAAGCTATACTCTATT
TACAAGATCCTAGAATATTCTAGAATATTGACAAGATTCTAATAAGAGTTAGCCCGTATC
TGTGCTGGTGGGAGGTAGCAGGTATCCTTTAGAATTAGTGGAGGTGTGCGCAAGCGCC
AGAACACCGTGGTTATTAAAAAAAATCCCTACGAATTATTGGTATGAATAACAACAATCT
CCTCTATCATGGAATGACTCCATATTAGATGTATCAATAATTTTCCAACGTCTTTTAAATG
TGGAAACACTAGAAAAAGTTGACACATTGATTAAGAAAGTAACAAAATGATACGGGAAG
TACGAATATCTTTGCGTGAGGTAATGAATGGAGTTGTTATGTCTGTTGAAGGCATGATTA
GGATTAGTGATGGAGGAGGAGGAGAAAGTCATTGAATGCTGGCGATGTACTTGTATCA
TGAGAAAAATCTCATCTGTATTAGTAGGAGGATGTTTATGAACTGATTAGACTCGGACA
GGACAAACTTGTGTTTTAAGAAGTTAGGGACTAGTAGATGTCAGGAGGGAAATTTTATTT
CATAGAATTTGGTGAAAAGTATAGAGATGAGTGGGAGGTTACATTTGCTGGGACGAACT
TTACTTTTTGGGGGATTATAAACATTTGCAACTTTTCTTGAAAATGGGTGAGTAAAAAAA
GACACAATTCTGATTAGGATTGAGGTCTGTCTTTTCCAGTAGTGTGATCATGAAGGAAG
CAAGTACATCCAAGCACTTCCAATTGTAGATGGTGGAGATTTTGATCTGGAAATTTTAGT
GTGGACAGTAAAGTTGCAACATAGAAAAGATTGATTTGTACATTTGCCATCGATAACAAT
GCTTCTAGCAACATGATTCTAGGGTGAATGGGTTTGTTGGGGCTCTATGAGTCTTCCTC
 
GAGTGGCGGTGAGGTCTAGAAGTCGATGCAAAGAATTGAGTGGTACAGTCAGTGAC
TATGACTCATTCTGTCAGCCTCTGTAATTTTTTGCCGCTATAGCTTTTCAGTAAGTAGTTT
CTTCCCTGGTTTTAATGTCATGAAAACTAGAAATAAAAGAATGAAAAATGATGGAAAAAC
TTGATTATTTCCTCAAGTTTGATTAAGAACTTAAACTAGTTACAATGTTGGTAAAATACAG
AATATGGTGAAGTAATTCTCCTAATGTGACTCATTCATAGCAACTGAGTTAACCCAATAA
ATGCAAATGACTGGACGATCTCGACAAATCCAAGTCTATCAATTTCAACAAGTTTCGCTG
CCTTAATCATGACAATATATTTAAGTGATGGTCATTAAATTGGAAAGAGTTGCTGTTGCT
CTTGTTTTTGCCATCATTCAGCTGTTCACTGTGGTAGATTATGGTTTCCTACCAAGTCCA
ATGAAACTGAGCAGTCTTGACAATGCCTGATGTTCAGTTTCTAAAGTCTGTTCTCTCTCC
AAAAGTAGAGGAACATAATGTTATCTGATTGGCTCGGAAAAAGTTGTATGTAGGGTGA
AACCTTAATGACATAATGAAACATGTAATGGTCTTTGTGCCTTTGGTTCATTATGTCTGC
TACTAGATACTGAAATTGCTGCTGAAAGTGCTTTTTGAGGTGTCACTCATTTTTTCTTGC
TGCTATTAATACATAGCGTTCTGATTCTTTTCAGACCCAGAGGGAAAGTATGAGGCACTT
GAGAAATATGGAAATGACTTAACTGAACTTGCCAGACGTGGAAAACTTGACCCGGTGAT
AGGAAGAGATGATGAAATACGGCGCTGCATCCAAATATTAAGTCGGAGGACAAAGAATA
ATCCTGTTATTATTGGTGAGCCTGGAGTGGGAAAACTGCAATTGCCGAAGGTATGAT
CTCTAGCCTTTTTTGGTCTCACGGGGTGATGTATGAACATGTTTTTCCTTATATTTATTTG
TCTGGATCCTGGTTCAGTTGAAAACAATCCAAAAACAGTAATGGAATAGCAGATCTGTG
GAGGACATTTTTATTATTACTTGTCTACAATGATATTCTTTTGGTTGGATATGCTGTTTTA
ATTAGTTTTGTTGAATAATGCTGCCTGGCAAACCAAACATTGAACTTTAAAAGAGTTATG
TTTCTAGAAAAGATATGCTCGGAATAGCATGATTATCCACTCAGAGAGGTTTGTGATATT
TAGCAGACTGTATGTGGGGTTTACAGGAAAAAATCATTGCTAATACATATTGTTTCTGGA
CAATACGTTATTCCTGTTAACTATTTGAATTGTGAGATGGGTCAGGGTGTGTTATGTGGA
GTTGTTGAAAATTAGTAATTGTAGGATGGAATGAAACAATTATAATCTTTTTTGTTTACCA
TGGTTCCTTGTGTTTATTTTTAATGAGCAGTGGCGTGGTAATGAAATGTAGTTATAGAAC
TTCTTACTAAGGGGTCTGCCTATATGTTGCAGAATCAGGATCGCTGACAAGGTTCAGTG
AAGAGTTCTGATGGATGCATTCAACATTTCTTGAAAATATATATCAAAATAATGCTGTTGT
TAGTGAATCACTGAATTTGTGTTAACTTCCCTTTGATTTGAAACTACCTTACCCGTAAGC
CTGTAAAATTGAGGTGGCTTGCAGAGCCTTTGAATGATCAAATTTCTATTTAATAATTAAT
ATAATGCCAAAATTGTGGCAGCTTGTAGAGCCTCTGTGTGACCTCGTTTTAACTTTTTAC
ATAGGTCCAAGGTAGTGTTAGCTGTAATATTTGCGTGTACTTAAGAGTCCTGTTCAAGT
GGTGGCATTTTTCACGTCATCTACTTTATGCAATATGTTATGTTTCCAGCCTTCGTGAAA
TGGGGATGTGTTTTTGACAGATTAACTGATAAAAGTCAATCAGTTCTGTCTCTTGTAATG
ATCTTTTTCCAGCAAAGGAGGCTTTTTATTTATTACTTGTTACCAGTACGTTATGAGATTT
AGAGCCTTTGTGGTTTGGATATTTAAAAAGTTAATCAATACTTACTTTTTATTAGATCAGT
TGTGCTAGTTGTAACTTACTTATCTTACATATCGGAATAATTAGTTTGGTTTAACTGCCAG
ACGAATGTGTTTCTGCACCGGAAGTGAATAATTCTAAATTGATATTGGATAGTGACTTAT
TTGAGTGTATCTGCAATATAGTTTTTTTTTGCTTAGACGTAGAGAAAGCATATGATGAGT
TGGGAAAAAAATGTCGTTTAGTGGGTGCTTGAGAACAAAGAAATTTCTACTCAAAATATAT
AAGTACCATAAAAGATATATATGAAGGAGTGGTCACAAGTGAGGGACCAGTGGCGGA
GATATTGAGGAGTTCCTTGTAACCGTGAGTCCATAATAGGGAGCTGCCCCATAGTTGCT
TACCCTGGCTATGGAATAAGTTATTCAGTAGCAAATAGGACGGCATTATGGGATATGCT
ATTTGTCGATGATATTTTGTTATTTGACGAGGGGCAGGGCAAGAAAGTAACAATCATAC
CCGAGAGAGTCAATTGTTAATTGACGAAATTAGTTAGGAGCCGATCAAAAGTTGGAATT
ATGGAGAAGCACGCTAGAGAATAAGGATTTTACAATAGGTAGAAGTTAAACAGAAGATA
TGCAATGCAAGTTTAGCTTTTGAAGTGAGGTTAGATAGGATACTAGTGTCGAGACAAAA
AATTCAGATATCTAGACTCGATCTTTCAATAGAATGACATGATAGATGAATATGTAACAC
AATAATATGATGGCCGAATTGAAGGAAGCCCATGGAAATGCTTTGCGATAATGATATAG
CTACTAAAGTAGAACATAAGGTCTATGGAAACTGGTGATATAAACAGGGTTATATAGGA
GTAAAGGCTGGAATTTTAAGACCCACAATATCGGCAAGATTAGCATCGTAAATATGTGG
TGTCAGATGGATGTACTATACGACCCAAAAATGGTTGAGTCCAAGTCACTACATTGACA
 
AGATGAGCATCGCAAATATGTTGCGTCAGATGGATGTATACACACCAAAAATGGTTGTG
TCCATGTGAAGGTGCATGTAGCACACAGTGATAGTAAATTGAGAGATGGCCACATTTCC
ATCATGTCTTTCCTAGGCCTTCCAAGTGCATTGGTTTATTGGTGAGACTATGATGACTAA
AGCTGTTGAAAAGGTATGAACTAGATCTAAAATTACATGAAGAGAAGTCATCTAGAATCA
CCTACAATCTCACAGAATCTGCGTGGATTCATTATGAACATAGCACAATGAAAGCAAATT
ATCAAAAGCATGCAATAGTAATTAGCTAGAGTTGAAGCCTAGTCGCTATTGCTCTTACAT
TAGGTTGTGTGTTTTCCAGGAGCTTTTAATTTGGTTAGAGATTTGTATATCAAATGTGAG
ATATAGAGACCCTCTAGTTTTAACAAACACCCTTAAGTTATCTCAAAAGAGAGCAAAATA
GATAGATGATTCATATAACGATCCCAACTAGGTTGGATTTGAGGTATTGATTAGAATGAT
TGATATAGTTATCCAAATTTTAAAAATCAACCTTATTAGTAAGGCAAAGATGCTCTTAACA
TGTTAAAAAGAAGTCGAACAAGAAATTGTTCTTCCTTTCCTTTGATATAAGATATTTCTCT
CCCACCATCCTGGAAGGAGAAGGTAAGGATTGTGAGAATGCATGGGAGAAAAATCTAT
CTTTTTAAATATATGATACAACGAGCCCTATATATAATATATATTCTACTCCTACTACATAT
AGGACTAGGACATATTCTACTCCCACTGACTGGAATGATGCACACAACGGCTAGAATAG
CCTCCAGAGAGGGTGGAGCAGCAACCGAAGAAAATGTTGGCGGGTTGGGCTGCCGGT
AGACAGAAGTTATTGAGTCTCTACTAAGAGAATAGAGGTTTTGGTATCATGAGAAAAGA
AGAGAGTACTTATTGACTTGATTATTGACACAATGAGAGTGTTTTTTATAAAGGATTCTTA
TTCTAGTGATATAAGCTTAAGTATTTATATTATGCTAATGATATGAATAGTGATTTTTCTCT
TGTAGTTAAGTAAAGAATACTCCAAAATATCATATAAAATATTTTACATATTCTCCTATTCA
GATACAATGAGACTAGGCAATATTAACCTATAATTACTTTGATTCTTTTGGTATTTTAACA
CGGTTTTTAGTCTTTAAAAGAAATCAGGAAAAAGAGAGGTGAAAGATGGTATCAGCCAA
ATCTATATGACAATTAACCTCGGAATGATTATGCTTTCTGCTCCTCATCCATGCAGGTTA
GCTCAAAGGATAGTCCGCGGTGATGTTCCTGAACCTTTGATGAATCGGAAGGTTATAAC
TTCTCTTCCTTGGTCTTAATTTGATTGACTTTATTTTATTGTGAAAAGGTCAAGTGATTGT
GCATATGCAAGATCTTGGAAGATGGCCTTTGCGTAGTGTGCATTGGATTGCTTTCTCTT
CAATTTGAAAAAGATAGTATCTGGGAAAGGCATGTCTTGAAGAAATAAATCAATGGTTGA
AAACCTGCTGCCTTTCTCTTGAGAACCATGAGCCGTAACTTGTTGCTTACTGCAAATAGT
TCTGTTTCTCGTGTTGCAGTGATTCATTTGTATGACGCCAGTATTATTGGATCATAATTTT
CTTTTCAAGATATGAGTCTGCTAAAGACAGTTGATGCAGTCTTTACTCAAATGAACGACC
GTGGTTAAGTAACATATTGTTTTAATCATTTCTGGGAGCAATTTCAGTCGAGTTTGATAC
TAAGTGAGATATGCATAACATTGCACTCAAATGTCAAACTAAATTTTGATCATCTAAGCT
AGTCCGATGAGTTAGTCTCTTTTCTGAAACCCCCAACCCCTCACTCAGCAAAGGGGTAG
GAGAAAAGGAAACGAACCACCTCATTCTCTAAAAAGAGTGGAACTAAATTATGATGCCT
CCTAAAGTAAAGTAGTTGGACATAAGTTTCTGAAGTTTCAAATTTGAGAGTGGGATTCCG
AGGAAAGCCTTCTGTAGCTTGAACCTTAATTGTCTACTAGGATTTGTTAATTTCGACTTT
TAAGTGCTAATACTGTAATTTGATTTCCTTTTTCCTTTTCATTTTATTTAGTTGATGTCTCT
CGATATGGGTGCCTTGCTTGCTGGTGCAAAGTACCGTGGAGATTTTGAGGAAAGGCTG
AAAGCTGTTTTAAAGGAAGTCTCTTCATCCAATGGGCAGATAATATTGTTTATTGATGAG
ATACACACTGTAGTTGGTGCAGGTCTGGTACTTTTTTTTTAATATCCATTTCTCCATGAA
GGAAGAAGTTTATTTCTACCGACTGGTTAGAAAATTTGCCAAATGTATTCTTTCTCTCTA
AGATCAAATCTCTATTATTTATAGAGTTCGATATTAAAGAAAAGTGCTGACTCAAACTGC
TTGTCTGCTTTCAAATCTCATGGCGTGTGGACAGCAAGTAGTGACTGCTCTTTTTGTTG
GATGATTTTTATCCTTGCTTAATTCAATCTTAAACAATCCAAAGTTCATGATTTAATTCATT
TGATGTCACTGGGAAACACTTGTTCTTCATTCTAGTGAGGTTAAAAAGCACTATTGCTTC
GTGATTGTGTTTTAGGTGATCCATTTTTAAATTTGAGCCTGGAGCTGGGAAATGCTTGAA
CTAAAGTTTACTTTTCTGTTAATTTGAGCCTGGACCTTGGAAATGATTGGAATAGGTTTA
TTTTTCTGTTCCATAAAGCTGATAAAACATTACTGATGATGTCCTTTATGTATGCAACCGA
GTAAAGGAGAAGGTACCTTTACACCCAAGATATAGTTTCTGTTTGTAGCTGGCTTATATT
AGTAACTAATTCAGCGAATGTTACTTGGTCAAATTATGTTGATACGTTATATTCTAAAACT
TTTGTTTGTTTATACCTTCCTAATCATTGTGTTTGCTTATGCTTTGTGTCTAGGAGCTACT
AGTGGGGCCATGGATGCAGGGAATTTGTTGAAACCCATGCTTGGTCGGGGTGAACTTA
 
GATGTATCGGAGCAACCACTTTGAATGAATATAGGAAGTACATTGAGAAGGACCCTGCT
CTGGAGCGCAGATTTCAACAAGTATATTGTGGCCAACCATCTGTGGAAGATGCAATTTC
CATCCTCCGTGGATTGCGTGAACGATATGAGCTGCATCATGGTGTTAAAATATCAGACA
GCGCTCTTGTATCAGCTGCAGTTCTTGCAGATCGATATATCACTGAGCGATTTTTGCCG
GACAAGGGTAGGCTAATGTATCCTTAGAACTGCAAGTTGTCTGAAATACTTGCTTTTCAT
TCCTATAAAATTCTTGTGAACGTTTTTCATGATATCTTCAAATAATACAGCAGCCTAATGT
TACTTTTACATAATAAGAAAGTTACAGGGTTACAAGTAGCTTATTTTTATGGCTTCTTTAC
ATGTTTTATTGCATTGAGTGGATCAATGGGTCCAGATTTTCAAGCTTCTTCTAAATGTTTT
TAGCTGTGCGTGATCTGACATACGTTACTTGGGGCTTTTCACTTATGCTCAGTTCTTTCT
TTTCAGCCATTGATCTTGTTGATGAAGCTGCTGCAAAACTAAAAATGGAAATTACTTCAA
AGCCAACTGAATTGGATGAGATAGATAGGGCAGTGCTAAAGTTGGAAATGGAGAAACT
CTCCCTGAAAAATGACACGGATAAAGCATCTAAAGAAAGACTTAACAAGCTAGAAAGTG
ATTTGAAGTCCCTTAAGGCAAAGCAGAAAGAGTTAAACGAACAGTGGAACGCGAGAA
AGATCTGATGACACGTATACGTTCTATAAAGGAGGAGGTAAATTGCATCTTTCATTGATG
AGGTCAAATCAAAGTTGCAGTTTTTCTTTGTTTTCTCATGATTACTGTTCAATTTTTTCCG
TTGCGTAGATTGACAGGGTGAACTTAGAGATGGAAGCTGCTGAACGTGAGTATGACTT
GAATCGTGCTGCTGAACTCAAGTATGGCACCCTAATCTCCCTTCAACGGCAGCTAGGA
GAAGCAGAGAAAAACCTGGCAGACTACCGGAAGTCTGGGAGTTCGTTGCTTCGTGAAG
AAGTAACAGATCTTGATATTACTGAAATTGTTAGCAAGTGGACGGGTATACCACTATCAA
ACCTTCAGCAGTCTGAGAGGGACAAGCTTGTCTTTCTAGAGAATGAACTTCACAAAAGA
GTTGTTGGTCAGGATATGGCAGTAAAATCTGTGGCTGATGCAATCAGGCGATCTCGGG
CAGGCCTGTCCGATCCAAATCGGCCCATTGCAAGCTTCATGTTCATGGGTCCCACTGG
AGTTGGCAAAACTGAACTTGGAAAAGCTCTTGCTGCGTACCTTTTCAATACTGAAAATG
CTCTGGTGCGTATTGACATGAGTGAATACATGGAAAAACATGCTGTTTCACGGTTGGTT
GGTGCACCACCAGGTTATGTTGGATATGAAGAGGGTGGGCAACTCACTGAAGTGGTCC
GTCGGAGGCCTTACTCTGTGGTCCTTTTTGATGAAATTGAGAAAGCGCATCATGATGTT
TTTAACATTCTCTTACAGTTGTTGGATGATGGAAGAATAACTGATTCTCAAGGGAGGACT
GTTAGTTTCACAAACACTGTTGTAATAATGACATCAAACATCGGGTCACATTACATTCTT
GAGACGCTGCAAAACACTCGAGATAGCCAGGAGGCAGTTTATGATGCGATGAAAAAGC
AGGTTATTGAATTGGCAAGACGGACTTTCCGGCCTGAGTTCATGAATCGGATTGATGAA
TACATTGTTTTCCAACCTCTGGACCTTAAGCAAGTTAGCAGAATTGTTGAGCTCCAGGTA
ATACAGATCTGTAATCTGTTGAATTCTGATTCTCCTGACTTCATACGTTTTTCTTCTGTGT
TGTTTTCTGTTTGCTGCGGTGTCATCTGCTTTCTGATTACTTTGACTTTAAGAGTTTTATA
AGCACTACAGCAGATTACTGTTTGTGCGTTATCTCTGTAAATTTCAGTTTTTCTGTGTGA
GAACAAAAAAATGTTTTAGTGTGCATTAGATCTCAAAATTACACATAAGTACATCTCATTT
GCTTGGTGGTCGTCGTCCTAGTTTGTCCTCCTTGCTGCTTTCTGATGAGTGCATGGTTG
AGTATGTCAAGCTTGAGAACTGCAGCGCACTGCGCATCCTGTCTAATGTCTGCTCTTGC
AGTAGTTTTCTAACAGAGTATAATGTAAAATATATCATTTCATCTGGTGGTTAAGCTTTCT
CCAAGATGAAACATAATTTGATATCTGTTCTTTGTGGTTCTTAATTTGGGGAAAGTGTTT
GGCTATTTCTTATTTTAACCTTATCATCGCATCTGCAGCCATAGCATAACCTTGGTGAGG
TTCATGGGAAAGAAAGTTACCGAGGCTACCTGACAATATCGTTAACTGATGAAAATATTT
GTAGAACAAAACTTTGTGTTTTCATTATCATTACTATATTAGTTGCTCTCTTTTATCTTTTT
TTCGTCCATCTTTTCTGTTTGAAGAGATTTTTCTTCTGTTTCATGAGATAATTCGAGGTG
GAACTGCTGAGTGCTATGTATTACATGCGGCTGATTATCTATTTCATTTTCTATAAAACC
TTCTCTCTATCGTGAGAAAGCGAAGGTCTCCCTCTGAAGTTATACTGCTGATATTAGAGT
TTCTTAGAACCTGACGACTAGTTCTCTTTTTCTCTTCGCAGATGAGAAGGGTGAAAGAC
AGACTCAAACAGAAGAAAATTGATCTTCATTACACGCAGGAAGCTATCAGTCTACTGGC
AAATATGGGCTTCGACCCTAACTATGGAGCTCGACCCGTTAAACGAGTGATTCAGCAGA
TGGTTGAGAACGAAGTAGCAATGGGTGTTTTAAGAGGAGATTTTTCGGAGGAAGACATG
ATTATCGTTGATGCTGATGCTTCTCCTCAGGGGAAGGACCTTCTTCCCGAGAAGAGACT
GTTGATACGAAGAATTGAAAATGGTTCCAACATGGATGCCATGGTTGCCAACGAT
SEQ 75 
GTGAATGTGAAATGTTTCTTTGTTTCTTTCTTTTTTCTTTTTCTTGTATGTCACTTTTTTTT
TTGCAAGGCTGGAACTTTGAAACTTTTTGTTTGAAAACACAATCATTCGCAGTAACAAAC
AAGAACCACCGTCCCCATCTTCACTCCCATCACTCTTCTTTTCTTTGTTTTCACACTTCAT
ATTTACTCTTCTTTCTCATCCTTTATATTTACATAGCAAAAACAACGTCAAGATTTGCAAA
AACACAGCAACCCCCCCAAAAAATGTCAAGATTTACAATGCTAGTAGTTCTTGTTCTTCT
TCTTCTATGTCTATGCCATTTATCAGTAGCAACAATAGGAAGTAGTAGTAATAAGAAGAG
TACTTACATAGTACACGTGGCAAAATCCCAAATGCCGGAGAGTTTTGAAAACCATAAAC
ACTGGTATGATTCATCACTAAAATCAGTTTCTGATTCAGCAGAAATGTTGTATGTTTACA
ACAACGTTGTACATGGTTTCTCAGCAAGACTGACTGTTCAAGAAGCAGAATCACTTGAG
AGACAAAGTGGGATTCTGTCTGTTTTGCCGGAGATGAAATATGAACTTCACACGACAAG
AACACCATCTTTTCTGGGTCTTGATCGAAGTGCTGATTTTTCCCAGAATCAAATGCTAT
GAGTGATGTGATTGTTGGGGTTCTTGATACTGGAGTTTGGCCAGAAAGTAAGAGTTTTG
ATGATACTGGACTTGGACCTGTTCCTGATTCTTGGAAAGGAGAGTGTGAATCTGGTACC
AATTTCAGTTCTTCAAATTGCAATAGGAAATTAATTGGTGCAAGGTAAAACTTTTCTAAAA
GTTTATGCGGTTAGAGACAAGACATTTTAAGTTAGTTAATTATATTATATCTCAAATTGT
GGTCGCGAGGATTCATATTGCTTACTTCAACTTTTTTGGGACTGGGACGTAGCAGTTGT
TATTATATGTTAAACTCGTCCTCTCGCATGTTGGTCTGATTAATTTTATGATTTCTCTAGT
TGGCAGTGAAATTTGAATCTGGGATTTTTTGCTTGGTTTGATACCATGTTGAGTTGTCTG
ATTAGTTGCATAACTAAGTGGTAACTGGTAAAGCTGCTCCCATATGATCGGAAGGTCAC
GGGTTCGAATCGTGAAACCAGCCTCTTGCTGAAATGCACGGTAAGGCTGTATACAATAA
ACCTTTTGTGGTCCGGTCCTTACCCGGATACTGCTATGGTATAGCGGGAGCTTAGTGCA
CGGGGCTCCCTTTTGGCATAGCTAAGTGTGTTGAAGAGGGTTTAGTTAAATCCCATTCA
TCAGAGTTGTACTGTACAAATAAGCTAAAAATGAATTATTTTTGTGTATGTAAATTGGTGT
ATCCCATTGATAATACAGGTTTGTACTTTTTTGAATTTCCTTGTTAGAATTATTTTAAAAAA
AAATAAAAAATATCATGGCTCTGCCACTGTTGTGCTCAACTTATCTAAAAGCTAAAACTA
TTAGAGATAAGATATACTTTTAATTACTTAATCATATTATGTCTGTTGATAGGTACTTCTC
GAAAGGTTATGAGACCACTTTGGGTCCAGTTGATGTATCCAAAGAGTCGAAATCTGCGA
GGGACGATGACGGACATGGAACACACACTGCTACTACTGCAGCTGGTTCAATTGTTCA
GGGCGCTAGTCTCTTTGGTTATGCTTCTGGAACTGCTCGTGGAATGGCAACACGCGCT
AGAGTTGCTGTGTACAAAGTTTGCTGGATTGGTGGTTGTTTTAGTTCTGATATATTAGCA
GCTATGGACAAAGCAATTGATGATAATGTGAATGTGCTTTCTTTGTCACTTGGTGGTGG
CAATTCAGATTATTATAGAGATAGCGTCGCAATTGGAGCATTTGCTGCTATGGAGAAAG
GGATTCTAGTCTCTTGCTCTGCAGGTAATTATGCTAGTCGGAAAATATGAAGAACTTCTA
GTACTTCTTAATTATTACATTTTATTTTATACTAGACCAGACTAGTTTAAAACTGAGCGAC
ATTAACAATGAAGATTCATTCATATTGCCGATTCTAACTTGCTTGGGATTGAGACGTAAT
TGTTGTTGTTGCTCTGCAGGTAACGCTGGTCCTGGTCCCTATAGTTTGTCCAATGTAGC
GCCGTGGATAACTACTGTGGGTGCAGGAACATTGGACCGTGATTTTCCTGCATATGTAA
GCCTTGGCAATGGTAAGAATTTCTCTGGTGTTTCACTTTACAAAGGGGATTTGTCGCTG
AGTAAAATGCTTCCGTTTGTGTACGCTGGTAATGCTAGTAATACTACAAATGGAAATCTT
TGCATGACGGGTACCTTGATTCCTGAGAAGGTTAAAGGAAAAATTGTTCTATGTGACCG
CGGGATAAATCCCAGGGTCCAAAAAGGTTCTGTGGTAAAAGAAGCTGGTGGGGTCGGT
ATGGTTTTGGCTAACACTGCCGCCAACGGGGATGAGCTGGTGGCTGATGCCCATTTGC
TTCCAGCAACGACAGTTGGTCAGACGACAGGGGAAGCAATCAAGAAATACTTAACCTC
GGATCCTAATCCAACCGCTACAATTCTTTTCGAGGGAACTAAGGTGGGGATCAAACCAT
CACCAGTGGTTGCTGCATTTAGCTCCAGAGGACCAAACTCAATCACGCAGGAAATTCTC
AAACCGGACATCATAGCACCAGGTGTTAACATTCTCGCAGGGTGGACAGGTGGTGTTG
GACCAACAGGGTTGGCCGAGGACACGAGACGTGTCGGGTTCAACATTATCTCGGGCA
CGTCTATGTCTTGCCCGCACGTGAGTGGTTTGGCTGCTTTGCTTAAAGGAGCGCACCC
 
CGATTGGAGTCCAGCGGCTATTCGCTCGGCTCTTATGACCACGGCTTATACAGTGTACA
AGAACGGCGGTGCACTCCAAGATGTCTCGACGGGAAAGCCATCCACACCATTTGATCA
TGGTGCAGGACATGTAGACCCTGTTGCAGCACTAAACCCCGGACTTGTTTACGACTTGA
GGGCTGATGATTATCTGAATTTCCTCTGTGCCTTGAACTACACATCAATCCAGATTAATA
GCATTGCTAGAAGAAACTACAACTGTGAAACAAGTAAGAAATACAGTGTCACTGATTTG
AATTACCCTTCATTTGCTGTTGTTTTTCTAGAACAAATGACTGCAGGCAGTGGAAGCAGT
TCTAGCTCCGTTAAATATACACGAACGCTTACTAATGTTGGACCAGCAGGAACATACAA
AGTTAGTACTGTTTTTTCATCAAGCAACTCAGTAAAAGTCTCGGTTGAGCCTGAAACATT
GGTTTTTACTCGTGTGAACGAGCAGAAGTCATATACTGTGACTTTCACTGCTCCTTCAAC
TCCATCAACTACGAATGTGTTTGGTAGAATCGAGTGGTCAGATGGCAAGCATGTAGTTG
GTAGTCCAGTGGCCATTAGTTGGATA
SEQ 76 
ATGTTGAAGGCTCTTACATCCTCATGTCTGCAGAATCGTTTCCACGCCGTCACAACGGC
ATTTACCCCTCAAGTTCGCCGTGGCACTGACTCGAATACGCCCTTGCTTCGGTTTTAG
GTTCGCTAAGAAGTTCGAATCGCAGGGTCCCTTATTTGTCTCGACGATTCTTTTGTTCG
GATTCTACTGATGGGTCCGAATCGAATTCCGAGGCTGCTGCATCCGAAGCCAAGCCGG
CCGAGGAAGGTGGAGATGCTGATTCTAAGGCTTCGGCTGCTATGGTTCCCACTGTTTTT
AAGCCTGAAGATTGCCTTACGGTTAGTTCAAAATAATTCTTTGCACCCGCACCGATAGA
TTTAGACGTGTCTTTAAAATAAATTGTATGACTTTTGTTAACTAATGTACATTCTCAGTTC
AATTTATCACTTCATCATTATTAACTAACATAATTTGGTGCATAATTATGTATTTTCCTGCT
CCATCATTATATAAGTACATTTTATGCTAATATTTGATAACTGCTAAATGACTCCTTAAGA
AAAGATGTTAACTTTTTGTTATAACGGTGTGGCCATGTCTGCTTGTGCACCAAAAAAGAA
GATATTAAGTGAGTTCCTTCTGTTGTTAGTGCTGGTTTTATGTCTTCTTTGGGTAGTTTT
GATATGATTTTATTCATTATTATTCAAATTTGTACATATGGATGCAAACATGACGCAGAAT
TGGGAACAATTTGATATAGAAATTATTTTTAACTTTGGTTGCAACACTCTGTTAAGATTTC
GGCAATCGAAAGGGATGGTTGTTTAAGAAGTTAACTTTCTAGGATATAAAAAAGGTGGA
TCACCAAGATTATATTTCTCGTAGATTCGTACACTCAATCATTCTTTACTAAAAGACCTTC
CCGACCTTGACTTGATATAATGCTGTGAAGGTCAACATTTTATTTTTCCAAAGAGAGGCT
TTTGATACCCTCTTTGTCTCTAAAAGAATGGTTTTATTATTAGATGCTTCGATCCTCATAT
TTTGAACCCAGCCATTCCGTTTGAGATGAAAGAATAGTATTGTAGACTTTTTTAAAATCA
GGCCGTAGTATTAAAGAATCTCGATATCAATCTTCTTAGGATCCTCAGACACCTCACATC
GATGACCAACACTTGTAATGTTATTGTAGCTTCAATTAGCATTAGTATTTTAATGAAATGA
GAATTGACAAATTTTTATAATCTAGTCGTCTTTTTAAAGATTTCTTGTCCTGAATCTTCTTA
GGATCATCAGAGAGCCTCACATCAGTGAGCAACTCTTTTAATGCTATTGTAGCAATGTA
GCTTCAATTAGCATTACCCTTTCAATGAAAAAATGTGGTATTTCAGTACCCCTCCTTCAA
CCACAATGTTATTGATTTTTGCTTCCTTGATATCTCCTCCACTATTTATTCTGCTTTTATAT
GGCTTTTTGGTACTATCCCTTCTTGTCTATATTTTCATTAATGTGGTGCTTATGCTTTCCT
GAGCCGAGGGTCTATTGGAAACAACCTCTCTTTCATCACAAGGTAGGGGTAAGGTCTG
CGTACACACTACCCTCCCCAGACTCCACGGGGTGGGATAAGACTGGGTATGTTGTTGT
TGTTGATACTTCCTCCACTAAGGCACAATCCGCCAACTCCTTAATCAAGTCTTCCATTAG
GGGTAATTCATTTGCAATCATCTCTTTTGCTTCACCTAATGATTTCTAAAGGTTTCGATTT
TTCCAACAATTCTTTCATCTCCATCTCCACTCAAGCATATACCTCTCCATTTTCATCATCT
ATGATCAGCTCTAAGTAATTTCAAGTTTTTTCCCAATCACACAGCTTGGATTTTCGGCAA
TGCACTTGTTCTTGTTCAAATGTTAATGTGTGTTATCTTAGTAGTGTTAAATTAGTCTCAT
TGGAAAGATATCATAAAATTTATGTATTTCTCACCTACACATAGATGTGTCTTTTCTATTT
GATTGTTAGATTTTCTCAGATTGTATCACATACCCTTGGTGATCTTAATAAGGGGCAGCC
TATGCTACCCGATTGGTCAACTTTTTCACTATTCTTTCCGCTTTTGGTGATCTTTAACCTC
ATCCCTATTTCCGTAGACCGTTTGACAGTATCATGCCTCTTTCTCAGTCATCTTTTTAGC
 
TATAAAATTCCTCCTTTTTGACCATTCTTCCAGTGAGTTTCTGCCACCATTCCAACCATG
TCCCATTTACAGTGTAAAATTACTATTTTGGCCATTCTTCCAATGAGTTTTCTACCATCTT
TCAGGCCAGTATTATGATTATTGTTCCAGATACTATTTTGGACGCTATTCCATTGCTACT
CCAGTCACTGTTTAGCCGCCAATTTGAGCCACATTAGCTGCCACCTTACGCCACACCAA
TGATGGTCTTGATATTCGTGGTATCGCGTTCTCTCTGGTCATTGAGAATCGGTTCCCTTT
TTTTTTTGATAAGGTAAATTGTATTAATCAAAAGGAGAAAAAAAAAACTCCCGCATACA
AGAAGTATACAAAAAGTAGAGAATTTACATCAGAACATGATTCTCTACAAATGACGCCCA
ATCTTCTACGCAAGTAGGGGCTACATGTGTGCACCATTAAGCGATCAAAGATAAAAGAC
TATTCTTAAGATGTGAAAACGGAGACTCAATCCCCTCAAAAACTCTCTTATTTCTCTCTC
CCCAGACTAACCACATGAAAGCTAACGGACGAACTTCCACGCCTTTTGCTTGCTTCTT
CTACGGAAATTGGCCCAGCTATATAGCATTTCCTTTACAGTGTTTGGCATCACCCATTGA
ACACCAAAAAGATTCAGGATTGCCCTCCATAAACCCCAGGACACAAGGCAACGCATCAT
AAGATGATCAACTTCTTCCCCTGAGCTTTTACACATGTAGCACCAACTAACATGTGTAAT
TCCTCTCTTTCACAGATTTTCAGATGTCAAGATCACTTCCCTCGCTGCTAGCCATGCAAA
GAAGCACACCTTCGTGGGCGCCTTAGGAATCCAGATCGATGAGTATGGGAAAGTAGCT
TCCTGTCTCNTGCCGCATCCCAGAGAAAATCCCTTTGAAGCCGCTCCAGTTTTTCTGTG
ATGCTCACAGGTGCTTGCAACAGGGATAAGTAATAGGTAGGGATACTTGACAAAGTGCT
TTTAATAAGCACTTCCTTACCGCCTTTTGACAAATACCGTTTCTGCCAGCCTGCTAATCG
TTTTTCAACCCTTTCAATGACTGGATTCCAAACNGAATTTGTTGGACCATCAATTCCTTC
TGGTTTGAAACTCTGAAGACGTGGGGGAAAGAGACCCTCAGAGTAGCCTCTCCACACC
ACCTGTGATTCCAAAAGCTGATTCTCCTTCCATCACCCACCTTGTAAGTGATGTTGCCAT
AGAAAGCTTCCCAGTTCTTCATGATGTTCCTCCACATGCCACACCCGAACGGTGTTGTG
ATTGCCTTGGTCCTCCAACCCCCTCCCGTGGAATCATACTTTTCTGCTATGACCTCCCT
CCATAGAGCATGCTCTTCTACCCCGAATCTCCACAGCCACTTCCCCAGCAAAGCTCTGT
TGAATACCCTGAGATCTTTTACTCCAAGTCCACCCCACTTCTTTGGGGAAGTGACTGTC
TGCCAATTCACTAGATGAAACTTTCTAGTTCCATCTGCCGCATCCCAGAGAAAATCCCTT
TGAAGTCGCTCCAGTTTTTCTGTGATGCTCACAGGTGCTTGCAACAGGGATAAGTAATA
GGTAGGGATACTTGACAAAGTGCTTTTAATAAGCACTTCCTTACCGTCTTTTGACAAATA
CCGTTTCTGCCAGCCTGCTAATCGTTTTTCAACCCTTTCAATGACTGGATTCCAAACAGT
AGTATCCTTTTGCAAAGCACCCAATGGTAGACCCAGGTAGGTAGTGGGGAGAGAGCCC
ATCTTGCATCCGAGAACATGAGACAAAGCATCAATGTTAGCAACCTCATCCACCGGGAA
AATCTCACACTTGCTGAGGTTGATTTTGAGTCCTGATACTATCTGAAATCACTGCAGTAG
CTGCTTCAGGCAGGTCAACTGATCCATATCGGCATCACAGAAAACTAGGGTGTCATCC
GCAAAAAGCAAATGAGAGACCCTTCGGGCACTGAGCACCTCGATCGGAGCTGAGAAAC
CTCTCAAGAAGCCTCCACTCGCTGCACGATCCATCATTTTACTCAGAGCATCCATCACT
AAAATGAATAGCATGGGGATAAGGGGTCACCTTTCCTAAGCCCCCTGGAGCTGCCAAA
GAAACCACACGAGCTACCATTAACCAGGACAGAGAATCTGACTGATGAAATGCAAAACT
TGATCCATCCCCTCCATCTTTCCCCAAACCCCATCCGTTTCATAATGAAGTCCAGGAAC
TCCCAATTGACATGATCAAAGCCTTCTCAAGGTCCAACTTGCACAGTAATCCGGATTCT
CTATTTTTCCTTTTGGAGTCTACAAGTTCATTTGCCACCAGAGCAGCATCCAGGATCTGC
CTACCTTCCACAAACGCATTCTGGGAGGACGAAACAGACACGTCAAGAACCTTCTTTAG
TCTGTTAGAGAGCACTTTAGAAATAATCTTGTAAATGCTCCCCACTAGACTGATAGGCCT
ATAGTCTCTGATACAAGATGCACCTTCTTTCTTAGGCACAATGGTAATAAAAGAAGCATT
GATGCTTCTCTCGAAAGCACCATTCACGTGGAAGTATTCGATGGCTTCCATCAACTNNN
NNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNAGCATC
 
GTGCACCTCCTCCTCCTCGAAAGCTCTTTCTAGCCACTCATTGTCTCCCTCCCCTATGT
GGTTGAACTCTATTCCCCCCAAAGTAGGCCTCCAACTAACTTCCTCTTTATATAAGTTCT
CATAAAACCCCACTATCGTCCCGTTTACCTCCTCCTCACCCTCGATCCTCACCCCATCT
ACAACTAAGGACTCTATGAAGTTTCTCCTTCGATTTGCAATCGCAACCCTGTGAAAAAC
TTAGTATTCGAATCCCCCTCCTTTAACCAGAGCGCCCTTGATTTTTGTCGCCAACTAGTC
TCCTGAGCTATTGCTAGCTCGACTATTTCACTCTTAACCTCCCTCAATCTCACTTTCTCA
GATTCATCTAGCTCTCTCGCCCCTTCCCCGCTCTCTAATTCCCCCAATTCATGCATAAG
CTCCCTCATTTTGACCTTCACCCTCCCGAAGACCTCCTTGTTCCACCTAATAATATCTCC
CTTGAGCAATTTGAGTTTCTTCAACAGACGGAATGAGGGTGACCCTGATACCCCGTAGT
TGGTCCACCATTCTTTCACTTTGTCACAAAATCCTGGCACTTTCAACCACATGTTCTCGA
ACCGGAAGGGAGCACGGACCCCCCTCCCTCTGCATCCATCTAGCAAGATAGGCAAGTG
ATCCGAAGTCAGCCTAGGTAAAGGATCTGCAACACATNTTGCAACACATTCGGTACCA
GCTCATCCCAGGAGGACGATATCAAAAATCTATCGAGTCTGGACCTTGAGTTTGAATCC
TCCGCCCTGGTCCAAGTAAACCTTTCCCCTGACAGAGGAAGATCAATGAAAAAGTGATC
ATTGATGAAGTCAGAAAACTCCTCCATGGCCGTCGAAATCAAATTGCACCCTAATCTCT
CCTCCGGGAATCTAATTGTGTTAAAGTCTCCCCCTAGAACCCATGGAATGTCCCATTCC
CCCATAATATTAGCAAGTTCCTCCCAGAAAGACACTTTGGACTCTTCCCCTACCGGCCC
ATACACTCCCCCAAATCTCCACTCCACCCCACTCACTCTATCTTTGACGAGAGCTGCCA
AAGAGAAAGCTCCCTTCCTAATCTCCTTTACCTCCAAACTTCTATCATCCCACATAAGTA
GAATACCCCTGGCACTTCCTGCTGCAGGGACCCAGTCATATTTCACCCAACAACCCCC
CAGACACTTCTCACGATCGCATCCGACAAAACTTCCATTTTGGTCTCTTGAAAACAAACT
AGGTTGGCACCCCACTCCTTAACTCCCACTTTGATGATGGCCCTTTTGTTTGGGTCATT
CATCCCCCTGACATTCCATGAAAGGATCTTAACTTCCATAAGCAACAATAGCCCCCTTTT
CCCCACCCCCCTAGACGAGATCCCTTTCTCCATGTCACACACTCGACCCCTTCTCTGAT
ACACCACTACATCCCCTATTTATTTTGATTAACACCTTTACCCAACTCCCTCCCCGCGC
CTTCGTAACAAGGCTGTTGCTCAATCTCCCTCAACAGTTCTAGCACCCTATCTTCCTTCC
CTTCAAAAGAAACACCTAAGAACTTCCCAAAGTTTAATAGTTTATCCTCCATCCATAAGG
ACATGTCCACTTCTCCAATCCGTGACGAAATTGGACATGCGTCTTCTGTAATTACCTTTT
CCTTGCCCCTACCGGAGGAATGCAAGACCATAGCATTGCTAGTAGTAGGAACTCTGGG
TGTCGGGTGGATCACGTCGTTTCGTTGAGAGGCACCAACCTCAGGAATTAATGCCTCT
CTTGGAGGAGATTGTATTGTAACCTCAGCACATCTAAGCACAGTGGGAGATGAGGGGG
CGATGGAGCTGGGTCCATCAGAGGCGGGGGGCCGTGGAATAACACCGCTAGCTTTTG
CCGGTGCTGCCCCTGGAACAGTGGCTGAAGAGGAGCACAAGTCTCTAGTGTTTGGTAT
AGCACTGTTTGAGGCATCATTGGCAGAAGGAGGAGGAGTGACTTGCTCCCATATGTTTA
GGCGCCTTATCGGACGTCGCTTGATATAATTTGGGCCCTTTTGGATCGATTCTAATAGA
ATTGGGAAAAGTTGCTGGGCCCATGTGTAGAGGCCCAGACCTATGCTTGCTGAAATCTT
GCCCGACCCTATGATAGTTAAGAGAGGACGCATCACTATCCCATTCACGTCTTTTCCTC
CTGTTAAAATTTTGTCTTCTTCTCCCAAGTTCAAACCTCCCGTCACTTCTCGATCTACCG
TCTGATTCCAGCTTAGTCCACTGGTCCGGTGAGCCCTCCCCTCCTCTCAGAAACTGGTT
TCCCCTTCCCTCCCTTCTATTTGACCTTTCCTTTTCTTCCGCCAAAATCGCAGGCATAAA
GACAGGGCGAAATTCAGGGCTCAACCAAACCCTGTAGCTCAAGTAGCCATCTTCCACA
ACAGTGGAGACAGGGATTCGGCCCCCTTTCCTCACCACAATCCTTACTCGCGAGAGAT
CGTGCAAGCTGCAAGTGACATCAATGAAACCCCCACAGATTTCCCCAACCTTCTTGAAT
AGGTCAAGACACCAGAGATGCACCGGGAGACCCACCATGTTGACCACCAATGTTTCGG
CAGGGAAACGAGGGTCGAGACACCCATCGTGCTCCACCCATCTGTCTAACTTTAAGAA
ATTCCCATCAAACCACCTGTTTCCTCTTACGAGAATTTCTGAAGCTTGAGACTCGGTGA
CAAATCGAAAGAGATATTGCGTGTCACCCAGTTTCGATATTTTCAGCCCGTCCTTGACG
TTCCATGCCTCTGCTGTCCAGTTCCTAACAGTTTCTGGAGAACCAACCCATTTGGAGAA
AGACCCAATCAAACAAGAGTTCAAGAAACCTTTACGCCTGAGAGTGTGCTCCTCGGACA
GCGAGAAGTGTGGCTCCTGCCCTACGTCAAGCTTTCGCTGGCATCTTCCCCCAAGTTC
GCGGGCTGGCCAAGATGCAGCTTTGCTAAAAGACATGCTTTCTAATTTTCTAGATTCTA
 
GGGAGAAGTAGGAGCTTCTCTCTGTAGAGTCCGTCATAGCTAAACCAAGAGCCTCGCA
AGGAGACTGACTGAAGAAGGGAAAGTGAGAGGGAGTGAAATCTGGCCGGAAAAAGAC
ACCTCCGTCGCCGAAAAATAGCAAAAGTGGTCGGAAGTTGGAGGTTGATGGATGGGTG
AGGTCGGAATAGCCTCGCGAAGAGGCTGTCTGAAGGAAAAAAGTTGGATCTGGCCTG
AAAAGGCGTCACGTGCCGGCGCGTGGGGTGTCAGATCCCGGCGATTCTTTCTGGGGT
AGTGTCGCGACAGGGTTGGCTCTCGGATGGTGGTGGTGGAATCCTAGGATTCATGGTT
CATGGTGGTGGTGAAGGGGTGGGATGGGGTGTCTGTGTGCTACAGTGGTTTTTTTCCG
CTTCATGCACATTGAGTTCTGGTGACTGATCTCGTTTTGAACTGTTCACTTTCTAGGTGA
CGCTTTACTATTACGACTATTGTTCACTATTCCGTGTTTTCCAGAAAAAGCAAACTCAAG
GTGTTCTTCACTTCTTCTTACCTTGAGTTCAAGGACACATAATACAATGGTAATGTTAGT
TAGTAGAATGTTAGTGTTAAGTTAGTCCAACATCAAAGATTATTGTGCTTTTCACACCTG
AAAATTAAGTGTCATGTATTATGTAATAAGTATTCATCAACTTATCTTTTCATCTGCTTCTT
CTATTGTCTCACAATTTTCAGATTAAAATGGAGTATTTTTTTACAAAGAATCCCATTAGC
AAGAAGATTGTGATTGTGTCTTGGGCACATTGTCAGTTGAACCATCCGTATTGGTTGAG
ATTGTTTCTTTGAAATCTCCTGATATTAGAAACCCGTCGATCCTTGAGTACTTCCTGCCT
TGTTTCTCTCTGGACCATATGTAACTCCTACCTTGCAGAGGCAAATTGATCAACTCTATG
TCAAGACTGAAGCTTCAGAAAGTTCCTAATGATTTTGAATGATTTCTCACCTGCAAGAAG
CTGGTGATTCACATTGTTCAGCAAAAGATTTTTTTTTGTTTGCTTGTCAAATTAACATTG
TAATTAGAATACTCTCCTTCCCATATGCTCTTCTCAAAGTCAGCATTCAACCAGTTATGC
CTCTTACTTCTGGCCGTACAGAAGGCACCTTGATGGATGTTAACTTTTAACACTTCATCC
TATATTTGGAGGCCTAGAGAGGGTTACTTCATGCTTTAGTTTCAATTTAATCAACAGCTT
CTAGGTTATTTAAGAAGTAAAGCATTGGAAAATACATGCGCGGTTTAAGTAATAGGATTG
CCCTAGTATCTCATTTTTCTTTGAAAAGAAGTTTGATTCATATGTGTATTTCAGTTTTTGC
TAGTTGTTGCAGGATCAGTGTAAAAATGGTATTTATCAGCCTTCCCTAAAAAAAGAGAGA
TACTGAACGAATGCAAAGAAAACACTGTTTCTTGGAGGCTTCTTATTTGCTCATAATTCA
TCTGTTTGTAAGATTTATGGGTGCGACCCATTTCTTGCCTTCTTGGTTGGTTATAGTTTT
ATCTTTGGATAAGATTTACCTGTAAGACTTGCAATTAAGGGCTTTATGTCATTTATCATG
CTAAAAATAACATAAAGTCTGTGTGGGCTGTACTTTTTTAATTCTATATAATTTATAATGA
TGCATGTTGTTTTCTTGGTGCAGGTTCTAGCACTGCCACTTCCACACAGACCGTTATTTC
CAGGGTTTTATATGCATATCTATGTGAAGGTATGGTACCTTGTCTAGAATGGGTTAAACT
ATGTCTTAGTTAGTCTTCGTGATTTTTGAAATAGAATAGATATAAGCTTTAAACATTAAGA
GTAAAATTACTGTCAACTTTCTGAGAGTTGATACTGTTTCAGCTAGTGTTTTGGAGCCTA
TTTCCTCTTAAGTCTATAAAACCAGCATTCAGCTTTAACAATGCTACTTTAATTGATGGAA
AGTTCTTGGCTGAAGTTGTAAGCTTGAAAAGCTAAAACCTTCAATTAGGAGAGCGTCTG
TTGCTCAATTGTAATATTCTTTAGCCTCATTTGGTAGTTTCCAGAAAAATATAAATTCTGC
TGTAATGGCATGCAACAAATTGATCTGAGGCTCCACATAATCCATAAATTTCACTGCAG
GATCCCAAGGTATTAGCAGCCTTGCTGGAAAGTCGAAAAAGGCAAGCACCTTATGCTG
GCGCTTTCCTTATGAAAGATGAGCAAGGGACTGATCCTAATGTTGTGTCTGCCTCAGAT
ACAGAAAAAAACATCTATGAGCTTAAAGGAAAAGACATGTTGAACCGTCTTCATGAAGTT
GGTACACTTGCTCAGGTACATCTTGTTGTTCCTTGTGTTATTCTGTTGCTTTAACTTTATT
GAAGAAGTTTCTGATCAAGGTTGATGCAACTTATGATGAGCCAGATAACAAGTATTAAA
GACGACCAGGTTATTCTTATTGGTCACAGGCGGATACGTATGGCAGAGGTGGTAAGTA
GTTGTCTGCTTTATTTTTTGGTTATAAGCAGCTCCATATGTTTTCTTTTTGTCCTATTTAG
TTCTATTTTTCATCTACATATCCATCATCTCCTGATTTTGAGACTGCCCAATTGACATCTT
GGGTTCATTGCACTTGTATATTTTTCTCTCTTTCCTACCTTTTGTGTCCCAACACCGAGT
CCTTCCCCTTTGGCTTCATATAACTGAGCAGCTGCATTGTCAAGCGATAGAGGTCTATT
GCAATTTGAGGCTAAATGAGCTGTTTTCCCCAGTTGGTTATGACGTGTTATCAATAGAAT
GAAACCCTCTTTTAGTTATTCTTGATTGCTTGTTTGCAAGAGAAAGTAGCACTGATTTTG
TAATAATTTTTGGACAACTGGTTTCCTGCAAAGTCATAGGAATGTCTTTCTTATTGCACTT
CGGGGTTGTAGAAGAGAAAAGCAATTGAGTTTCTTCTATGATGTGCTAGTTTTGCATCT
GTTTGATTGCTGATGCATTTGGTTTGAATGAAATCCTAGACTTTTGATAACCTAGAAATC
 
TGAGGGTAAGTGGCTCACGGTTTGAAACTCAGTGGATAATGGGCTCACCATTCTACCAT
TGTCCACCTAAATACTAGGCTTTTGTTTGCGCCACGGCACGAACTGTGATGTGCACCCA
ACCTACATATCACGGGCTGTACTCTTACCACTAGACAAAAGCCCCGGAGGCTTAAACAT
ATATTAAAACACATAAAAGTTTGAATAGAATCCTATATATTTGGGCTGTAATTTGGTATAG
AGTCGTATTCTTGGCTTTAGCTGTAAAGCTGGAGTACTCATGCATGGTCAGCCATTAAT
GGGCTTACTTAGCCACTGGAGGGTCTTATGTCTTCTTATTTTTTCGTTTTATTTTGTGATT
GAGGACCTCTTATCCTCTTCTATTGGACTTAAATCTCTCATCCCCTGAGCTCCTCAGTAA
TAATTCTCCTATTATACTATACTTACTGTTGGATGTTTACCTCTCTACCTTCACATGGTAG
GGTAAGGTCTGCGTACACAGTACCTTTCTTGGACCCCACCTGCACGATTACACTGGGTT
TGTTTGTTGTTGGATGTTTGCAGTCAGATTTCCTTATGTAGTCATTTCTTTTGTTTCAATA
AACAATTTCTTCAATATACTAGTGTAACAATTTGATGACTATGCAGTAGATTTGTCACACT
TGAATGTTCACTATCAGATTTCCTTACTTTATTCACCATGTTGTTCTTTTTCCGTCTCAAT
AACCCATTTTGTGAAATGTACAAGAGTAACACCTTGAGGACTGAAACAGTGACATTCATT
TGCAGGTCAGTGAGGAACCCCTTACAGTGAAAGTTGATCATCTCAAGGCATGCCTCAGT
TTCTTGTGCATTCTTTCTACCCAGCAATGATAAACTCATAAAGCTTGATGTTCAGTTTTGA
AATTGTTTCTAAAATTTGCTTTCTTAATTATGTTGCAGGAACAGCCGTACAACAAGGATG
ACGATGTTATAAAGGCGACATCTTTTGAAGTTCTATCAACCCTAAGGGATGTTTTGAAGA
CAAGTTCTCTCTGGAAGGATCACGTTCAAACTTATATCCAGGTGTTAGTCATTTCTTTCT
AAATGTTAAGTCCTAATTGTTTGATTTGGTGATAACTCCAAAAAAAAAAATTTACTCTCT
CAAAATGCATTGCCTTTTGATATTCTAGCCCACACTATTGTGTGAAGCTCCACAATGCCG
GTGGATTGTGTTAAACTTAGATCATGCCCTAAGTTAGGCATTTCATGCCATTCTTTTTAA
TGGAAAATTATGTTCGTGTTGGTGGTAATCAAATCCATGACTAGATTCTCTGCCTTATAG
TTGATGCCCTGCTGAAAGAGGAGACTAATTAAAGATGTCAAGACTGCTAGGAAATGGAA
GGTCATAAATGTTCTCTGAACCAACTGTATACATATTGTTATACATATTTCTAAAGAGAC
GCTAGGAAGTTTATACAACTGAACAACTGTATAAGATGTATTTGGAGCTAGGAAGTTTAT
ACAATACCTAATTCTCTCTCTGCTGAATTTCTAAAAAGATGCTACTGATTGTTATGAATAG
TGTCAAAATCAAAAGATAATATTGTGGCATGTTTTGCTGAAGAAGTGGGAGTGGAGGGA
TAAAGGGATAGGGACTTACTGAATACTTCTAAGGGAAGCATTTGTGTCCCTCCAAGTTA
TTGGTACACTAGTAATTGGAGTAAGAGACATTACTCCTTTCGACCAATTTCAGAGGGGT
TATCAAGTCTTCAAAGCGTGGCGATATTTGTGGGGGTTGGCTGGAAATTGAAAAAAAGA
CAACTCTAAGAAATCATCCTAGATGGGCTTCGATCAAGGAGAGGAGGCAGATGGAGAA
GATTTCGAAAACCCTAGAAGTGGAGAAGAAGGGTTTTCTTTATAAATTACATGTTTGGAG
TGTAGCGTTGGTAACAATTATCTCCAATCGACGAAATGGAGAAGAAGATGGACCTGCTA
GGTACTATGATGAAGTTTGAGATAGGCCTGCTACCGTAGAAAAGATCGGTCATGTGGG
GGTTGGGAACAATATTTCAAATTCTAAAAAAGTGTGGACAGATGTGGGAGTGGCACGTG
GAAAGGTCTTAAGGTAGAATGGGTCAGTAGTAAAGGAAAGGGCTCCAGAATTGGGCCA
CTTATGTCATGATGTTTGGAAGCTTAACATAACTTTTGGGTCATAAAAAATAATAGATATA
GGCTCAGATGAGTCAACCTTTATTTTCAATAAGGGGCTTGGTCTTTTAGCCATGAACCTT
TAGGGTCTCAGTTTTGATACTCTTTTAGTGGAGATTAATGTCATTAATTTGTCTGAAACAT
GATACATTTTAGACTCCTTTCACAACACCATGATCGGCAAACCGATGAAAGAGCAGGAA
AGCCCTAGTCTGCCACAAATTTCACAGAACTCACATGCAGCTGATTACACAAAAATTGT
CATTCAAGATTAAGAAAACTCTTTCTTCGTACAATCGAAAGAGGCTGACCAAAATCAGAT
AGTTTTTTGAATTTGAAAGAACTAAACCATGGCTTATCGAAGAGGCTACTCAAGCTTTCT
TATGGCTGCATAATAATGTTACAAAGATGAGCAAACAATATGGATTGAATTTTTGTGAAT
GTGAGATTGAGGGTCTAGCTCTGTTCACGAAGCTTGACAGGAGAAGGCATAAGAGAAA
TGAAGCTACCATGTCCAGATTCACAATTCCAAAAGTGATAGGTATAAAGGAGCTCCAAA
AACTGTTTTTTAATGTGAATTATGGGGAGCTCGGATCAATGATGGGAAGGGGGATCACA
AACACTAGGTACCCATGAAGCTGAATATTCTCACTTGGAATATTAGGGGGTTGAATGAC
AGGGAAAAGATAAAGGTGATAAAAAGTTAATCCATAAATTGAAGGCAAATATTTATTGCT
TTCAAAGACGAACTTAGAAGGGGGTGTGGAAATACTAGTTAAACAAATGTGGTCAGACC
CATCTCAAGTGTTGTTTGGAGTCCAACGACAGGAAAGGGAAAATATTGGTGATGTGGGA
 
AAAGAATGTTTGGACAAGAGAAACTATCAACAAGGATGTATACTATCACTCGCAAAATT
TCCTCATTATCACAGAATTTCTCCTGGCACCTCACAGGGGTGTATGAATCACACTGCAA
GTTGGAGAAACAAGAACGCTGGTGGAGATAGTAGCATCTAAGGCATTTGTGCAGGGC
CTTGGGTGGTGTATAGAGATTTTAACACTAATAGATTCATAGCAAAAAGAAAGAACAACA
ATAAACTCACTAGGGCTATGATGGACTTCTCTAATTTTATAGATCATTAGAAGCTTGTAG
ATTCTAATCTTAATGGGGCTCCTTTTACTTGGACAAAGGGTAATAATCAGGAAAACTCTT
CAAGATTGGATAGATTTTTTTCCCGGCTAAATGGGCTGAGGAATTAAAGAACAAAAGGC
AAGCAGTACTCCCCAGTGTATTTTCTGATTGTACTCCCGTTTCTTTTCAATGCGGAGATG
GGGAAGTTTAAAATCTTACTTCAAGTTTGAAAGCTGGTGGTTGGGTGTTGAGAGATTCA
ATGAAATGGTGAAAAGCTGTGGAACTCTTTTGAAGTACAGGGTAGACTAGACTCCATTC
TTTCAAGCTAACTGAAGTTGTTGAATACAAAGCAGTGGGATTGGGAATCTTCAGACTAT
GACAGTGTTGTGAAGAGCCTGAAGCGAGGAAAAGCGATAAGCCCCTTTTCGCTTAAAG
CGAGAAGCGAGAAGCGAAGCGCTCGCTTTTTTGAAGTGAAGCGGTTTAAAAAGATATTA
AAATAAATAATGCATAGACAACACATGTAACTGTAAGCAAATGTTCAATACTTCAATGTA
AAAACTAAAGAGTAGCATCAATTAAAGCACAAAATGAGCATCATATTCTTCTTCAAGATT
GTCAAATTCTTGTATTCCACTATCATTATTATATTGCTCGTCATCTTCTTCAACTTCTTCTT
CTACATCAACTAGAAATGAAGTAACTGCTTCTTTTCCCTTCCTCTGTGAGCTTGAAATTG
AGGTACTCCCCCTCAAACCATAAATTTCTCCCCAATTCCACGCGCCTCCGCAACATCA
CCCCAAGTGAAATCAGAAGTTTCCTCAAATACTTCTTCATTTGCATGATCTTCCGGGACT
CCAATTAGCCATTCATTAGCATCATCGATGTTGTCCAAACTAATTGGATCAATTACATTG
CGAGCATTGTAACGACGCCTCATTGTTCTATTGTACTTAATGAAGACTAGATCATTGAGA
CGCTTCAAGGTTAGTTTGTTCCTCTTTTTGGTATGAATTTGCAAATAATAAGTAATTAGTA
AGATTGCATGCGCATAACTGTCTGTCATCATTCAACATTCTAACTTCTTGAAGTTGCAGC
ATATTGGTGATTTCAATTATGCAAGGTTAGCAGATTTTGGAGCAGCAATATCTGGAGCC
AACAAGCTACAATGCCAGCAAGTGCTTGAAGAGCTAGATGTAAGTCCGTGGTTCAAGAA
GTTAGATATTCCCTCTGTTTCAATTTAGATGACACACTTTCCTTCTTAATCCGTTCCAAAA
AGAATGACACATTTCTACAATTGAAAATAATTCAACTTTAAACTTTTCATTTTACCCATTTA
CCCTTAGTGAGAAGTTTTTATAACCACACAAATGTTATGCCCCCACAAAGCTTTTACCCC
TTAAGCTTTTAAGTCCACAAGTTTCAGAAGTCTTTTTTCCTCTTAAACTTCCTGCCAAGTC
AAACTACCTCATCTAAATTTAAACGGAGGGAGTACATAATATTTTCTGTATGTGTGCCTT
TTTCAATCCAAACCTCAAAGAGAGAGGTTTACCAGTATTGTGAGTTCTAACCACTCTGGA
AGAGAGCGAGGTTTTATTACTAGGAGGTAGATCTAAGAATTTTTCCTTGCCATTTGCTTC
CTATTGTAACAAAAAAAAATATATGTTTGGTCAGTGCTTCCCTGCTCTGAAAAGGAAAA
TGGTTGAAAGTTAAAAAAAGGAGGATCAAAGCGTATTAGCGGTAAAGCTTCATTTCTT
GCTGATGGGTAAAATGGCTTCTCATTGGTACTCTTGCTCGAAAAATTAGGAATCTAAAAA
AGTTATCAGTTTGTTCACCACTTCATAAAAAGAAATTGTCAGTTTGTTCAAGTGGAGCTA
CTTATTTTGGGAAAATTAGAAAGGGAAATATTCAGTTTAGAACAGCTAGCAATATTTTGT
ATTTCCTCGTAAGAATCATAAACTTGCATTACAATGGTTATTTCATGATTTTCATCGAACT
GAGCCTTTTGTCTGCTCAGGGATCAGAATATAGTGTGTGCATGAGATAAAATGAATGTT
TCCCGCTTGTTTCATAGCTAACACCATAATACCTGCATCAGGTGCATAAGCGGCTACAG
CTTACCCTGGAGCTAGTGAAGAAAGAAATGGAGATTAGTAAGATTCAGGTAAATGCACA
TCAAGACGCATACCTGAACTTTAAATAGGTGCTATGCATGCATTTAGCATTTTACGTCTT
TTCTGTTGTCTGCAGGAATCAATAGCAAGAGCAATTGAAGAAAAAATAAGTGGAGAGCA
ACGCCGTTATTTGTTGAATGAACAATTAAAGGCCATAAAGAAGGCATGTGGATTATGTG
CAGCTTTTTTTGTGTTATCATCCTTAAACTTAGTACTTACATATGTTTATTCCTAGATAATA
TATATGTTAAGCTTCTGATTCTTATGTTTGATTCACAGTTTAAACAAAGAGTTGATTTGAA
AAAAAAGTGCTTCCTTATGACGTGATTTTGATTGGCTTAATGCAGGAACTAGGTTTGGA
GACTGATGACAAGACAGCTCTTTCTGGTTCGTTGTCTCTTAATTACTACTGAAATGAATA
ATGTTCTTTTTGGATTTATTACGGCCAGACGTGTTTTCCCATCTGGCCAATGAAACATCT
TATGTTGGCCCTGGAAAAATTCCATGTAGCAGTAGCATAGAAAAGGCCATTTGCAATAC
GTTTTGCCCTTTCTGTTGTAACAGTTTTATGGTGGCTGATTGATCTCCTGTTTACCATTG
 
AAAATCTTCACTAGAAGCATGAATAGCATTCAGGCGATAATTGGCTTTACAGATAGGAAA
TTGAAGGTTGAATTTTCTTTGTACACGGTCCAACCAACTTTATTGACATGCTTTCTAAAG
CTATTTGAGACATACCAAACTTTTGACAGATAAGAATACATTGTACCTATAGGGGGCTCT
TTGTGTTTTCACTAAAGCACTTAATAAGACAATACTGGAATCCTTCAACTTCAATTTCCGA
AAGGTTACTGTCTACCATATTTGGTAATGTGGCACTAAATCAACATGGATTTACTGGAAG
GCCACATTGTTAGCACTGCATTGTTGTTTATTCAAGTTATGGACTGCTCATGATTCAGAT
TTATCTTCCTCCTCCAATGTTTCTTTTTCTCTTGAACTCTGTCCTGTTTCCCTTTTCAATG
CACTTCCTTATTAGAAGCCTACTTGCTTTTTCTGTCATGTACTCTTTCTTCTCCCCTTTGC
TCCTGTTGAGGAGTGTAATGCTTATGAAGGTGCTTCTTCCAGGTTTTGGTGATAATAGA
CATTACTGCAAACCCTAAAACGCGGTTCTCATTGTTTTTCTCTATAATTTTCACAAAAAT
AGAGTTAAGCTGCGTGTTGTAACATTGTTTTGGTAGACCTGGTTTTGGTGTTGAAATTGA
GAAGAAACTGCCTAATTATTAGGTTTGCTTTAAGGGCTTCGATTTTTCTGGTCAATACTA
ACGAAAACCTGCAGTTTTTTGTTCCCTTTTTTATTACATCTAATGGTGTGACACTATATTT
GTTTATTACTGCAGCAAAGTTCAGGGAAAGATTGGAGCCTAATAAAGAAAAAATACCAG
TACATGTTATGCAAGTTATTGAAGAAGAACTGACAAAACTGCAACTGTTGGAAGCTAGTT
CCAGTGAATTTAACGTAACACGTAATTATCTTGATTGGTTGACTGCCTTGCCATGGGGT
AATTACAGGTTTGTTGTCTATCGATTCTGCCTTACATTGTCTTGGGTTCAACCCAACTGA
TGTTATCCTTATCCTTGGCTAGCTGTACTAGAGGAATCTGTTTGAGAAGCTGGCTAAAC
AGTCCAGCGAGAAATAAAAATGTTATTCTCTGAAATTTGCTGCTTCCAAGTTAACCTTAC
TGCCTAGTGATGTGACTTGCCTAAATATCTATCGAGTAATATCCATTTGTCTTTAACTTTT
CTTTCTCCTCCAGTTCCTTATTTTGGGTTCTTACATGTCATGCTTCTGGCTTTGAGGATG
CTTACTTGACATCCCAATGTATGAGTTTAGACCAGGATCTCATGAGAGCAGCAAAACTA
GGATTGTACTTATGATGAGCTCCTTAAGATGGGGGCTTGATTTGCCGTAGTTCGTGTTG
TTTGCTGCTGATGGTGGTGGTGTTGGCTTTATAGTTTTGTTCTCTGCCATGGGTGTATT
GCATTGGTTCCTGAAGTTTTCTTTTTATGATCCAAATGCAGTGATGAAAACTTTGATGTA
CTACGGGCAGAACAAATTCTTGATGAAGACCACTATGGGTTAACCGATGTTAAGGAAAG
GATCTTGGAATTTATAGCTGTGGGAAAACTCAGAGGAACCTCGCAAGGTTGGTAAATGC
CTTTTTTTAAAAATAATAACCCTCATTTTTATTAAAAAAAATCCTATTTTATAAGGTTCAGC
CATAATCATATTAAAAGAACGGAAAATGATCCAGCCATCTCCTTACTGTCCATTGTCATA
ACATTATAATGGACCAATGGAAAATATATCCATAGAACATGAGATTTATGGTTCCCAAAT
ACTTTATTGACATCAAATTGAAACGAGTAAACGGAAAGAAGTGAACATTTTAGGGAATTT
GAGAAATATTTATTGGTCAAACTTAGGTAATACTTTTTGTGTCAGTCTAGAGTTCCTCCA
ATGTTTTCTTGTGATTATCTGTGGAGTAAAGAAATATATCTTGAGCTTAATTTCTTCCCTT
GAAAAGCAACTAATGTGAATTAAACTGCTGCACCTTGGGCCATAGTTTGTTGGTGTTCTT
CTTACATTCTGATTTTGTGCTGTCCATGATTGGGCACTCGCTGTGTGGTATTCGATTGAT
AACTTACTTTCACCACCAGTTGTACTTGTATATCTTTGGGACATTGAACTTGAGATGTAG
TTGTTTGTTGAGGATAATCTTTGGAAACTATGAAGTGTTGAGAAAAAAACAGGTTGAATG
AAAGTTAACAATATAATCCAAAGACAAAGGTTAACTCTAGAAAAATGTGAATTGCATCAT
AGCTAGGACAATATTAGGTTCAAATGATAATATTAATCCCAATTATACACTGGCACTGTC
TTTCATATGTGCGGCTTCACCTGATTTATCTCAGTTTAATTTTGAATCTGAGTCAGGAAC
TAGAAACAGACTCATTGCTTATTTTTGTTTGAACAGGGAAAATCATATGCCTCTCTGGCC
CTCCTGGGGTGGGCAAAACCAGTATAGGTCGTTCAATTGCACGTGCATTGAACCGCAA
ATTTTACCGATTTTCTGTTGGAGGGCTGTCTGATGTTGCTGAAATAAAGGTAATGGGAAT
ATCTGGCCAGCTAAAACAGAGTTGTTTTGTGGCGCACAGAATCTTGAACTTTCATGACT
AACTTTGGGATACACTTCAAGGGACATCGACGAACTTATATCGGTGCCATGCCGGGGA
AGATGGTGCAATGTTTAAAAAGTGTGGGAACCGCTAATCCTCTTGTTTTGATAGACGAA
ATTGACAAGGTATTTTATGGTTTGTGAGTTCATGCTTCAATTGTATGGCTTTGACTATGA
GAGGAAGTCTAACTTCTTTTTCACCATTTAATCTCGTTTTTCTGTATATGACCACTGGAA
GAATCTTGAGCCTGAACATATTATGTTTTTGCTTGGATTTCCTCTGCAATCTAAATGTTTG
AAGAAAGTGTTTATCGATCAGTTTAATAATAGCCTTGTATTTTTTCTTCTATGGCAGTTGG
GAAGAGGACATGCTGGTGATCCAGCAAGTGCTATGTTGGAGCTTCTTGATCCAGAACA
 
GAATGCAAATTTCTTGGATCATTATCTTGATGTTCCTATTGACTTATCAAAGGTAGTTGTT
TTCTGGAGCACTTATCAAATTATTGTGGCTGTTGATTGGCCCTTATGAAATGCCCTCTAA
CATCATTTGATGAATGGGGACTAATGTGATATTAAAAATCTTGCAAATATCTACTATCATT
TTGCTTTTTCACCTTTTGATTTCCCCCCCTTTTTCTTTACTGATTGATGTTCTCTTTCGTC
TCTTACCTTAGTTAAGTTTGGAAAAGGTTTCGTGACTGAGCCTGTTTCTTTTATTGTCCA
TAATGGAAGGTTATCTGGAAGTATTTTATTTCACGTCTGTGTTACCTTTGTCTCTGTCATT
ATGACTGTAATATGATTAGTGTAAGTAGAATGTTGTTCTCTTGATACTTGAGAAAAAACT
AGGTTTCATTGGCATTGGTTTTGGTGATCATTCAATAGGAAAAAGGTTGATGCATTGAGT
TTTCCTACTGTCTGTTCATATTATCTTTGATGGCTTTACTGATGAGGGATATGGTTTTATA
CCTCTTGGAGTTACCGATAATTGGAGCGATAGACTTTGAGTGTTGAGTGTTAGTTTACC
TATTGATTAAAGAATTTGGCCCATAATTCAAATATGACAGTTATCGATACTATTTCATTTT
ATTATATCAAACTTCAGCAAATCAACATGGCTACAGAGAGAAGGTTGATGGGTATATTC
GCATACAATTTCATTTATTTTGTCCTTTAATGTGATAATTTACTGTGTGTTCCTATGTCTTT
TCATAATGGTAAATATTTGTGTTATATTTCCAAAGGGCTTTGTCAAAAGAAGATAGCATC
TTTCGATTATTTTGGTAGTATTTTGGGTCTGACTTGGTTATGGGGAGGGAGGCTATATCA
AAGCCCTGTTATGGATGTTGAGTTATATATGAACCTGAAGAAATTATAAAATCCAACCTC
TAGGTTTTAATGTACTTCTAAAATTTGAAAAAATCGAGCTTTGGCACCTTCAGTGTTTTCT
ATCCTCTTTGCTTAATACAGTTACCTATTCCACTGTAGTTTATACGAGAATGACATCTGC
ACATTTATCAATTTTTTGAGTGCATAATTTGAGGTCGATCATTTTCCTTTTTGCGGCTGCA
GGTTTTGTTTGTCTGCACAGCCAATGTTGTAGAAATGATACCTAATCCTCTTTTGGATAG
AATGGAAGTAATTTCAATTGCTGGTTACATTACGGATGAGAAAATGCACATAGCCAGGG
ATTATTTGGAGAAAGCTACTCGTGAAACATGTGGGATCAAGCCTGAGCAGGTATGTCTT
ATAGAAACATCTCTAGTTGCATCTCTTTCATTATCTCCTGTGCATATTCATTATCGGGTG
AATAGTTTTGTATTTTTTCCGCTCCCAATTTTTGACATTCAGAAGCAGACATTGGTCTGT
CGGGAAAATGTTCGAAACATGATTGGGCAAGAATGTAACTTCTTAAAGTAAAATGTGAA
AAGCTTTATTCAGTTAGAAGTTTATGGAATAAAACATATGATTGGAACATTCAGGGGATC
AAGCTCTTTACACCCTTCCATATAGAACATGGCTAAAGGAAAAAATCGCAGTAAATCTCA
TTATAGGTTTTTTGCCATATGCTTTAATATTTCCTAGAAAATATACTGTTTGATGTATTGAT
ATCATTTTCATCTTAAGCCTCTACATTTAGAATATACTTGCCTCCAAAGCTAATGGATTAT
AAAGTTTAATACAAACTCTTCCATCACTATCCTTTTCAAGAAGTATTTAAGTGGTTCACAT
AAACAACTGAACGAGTCCACATTGCTGTATATATCCTCTCAAGTGCTGCTTTCTCTTTGA
GGAATGTTTGCTTGACCGAGCCAAACTGTGACTGCCTATGCTAGCCAATGTGCTCGTG
CATCATTAACACAACCACTGTGCCACACTTTGCTGGGAGTATAAACGCAATTGAAGGTG
GTTCTGTCGTGGTAGTCGCCAATCTCTATTAGTAAATGGAGGTATAATGAGTTTCTTAGT
GGTAGGGATACATGTGCCTGGTGGGTGGGGTTGCTGGATGGTATTGTCTAACAGATTT
GAGCGGGGCGTGAACTAAAGCAGGATGGATGTTTAAAATATGCCAAGTGTTTTGGTGTT
TTTGCTGTTGACTTCAATTTGACCATCGTGTAAAAGAGAAACTAGACTAGTTTGTTACAC
AGTAGTGAATTTTTTATTACTGTACAAGGAATCATCATACGATGCCAAGATTGTGGTGAT
GACTCCAAAAAACACTTGACAATGTCTATTGAGTGAGCTTAAGTTATAATATGTCTGCAG
TAAACATGTTCTCCCACAGCCTTAAAGATGAGATCCGTGGAATTCGGAGAACCTTATATT
TAGCTCTCTAATGTGATCTTCAGTTGAGTTACATGCCATATGGATCTCTGGTGAAGAAAA
GGGAGCTGAAGACTTACTGATTAAGGGCATCAAAATGACATTTGACCTCATCTAACTCT
GTGGTGTCATGTGGTGTCATTTACTGTGCATATATACTCTAATAATTAACGTTTTCCACTT
TTCCGGGAAAAAACACCCTAAATATGGGTGTCAGAAGTTAATAAGTTGATATGTGGGAG
GGCTGATGGCTACTTTGGTTTGTTTTGGCCCTCCATAGCGGATAGGAGCAGTTCTAATT
TTTGAAGTATCTTGTAGGCTGTTGGTACTTTTTCATGACATAGTAGTGGCGTTATGCTTA
TCTAATGGCAAATGTATGACAGGACGAGGGTAGAGCTTTGGGTACCATTTCTCGGGAAA
TGTTAACTCCCTAATGCTCCTGTAAAATTACTCGAGTTGATATGTAATCAAGTTGAAGAG
ATGCAACGTTTGAGCCCATGTATATGTCATCTCTCTGTCTCTCAGGCTTTTAACATGCTG
AGGGCCGTTTCTCAGATGCAGAGATGTGATATCAAGCCGGAAAATTAAGTGTGCATGAT
TTATGTTAGCAATTGATTGCAATTCAAAGCCTTGAATGGTTGATGTTTGGTTTAAATATTT
 
CAACACGTATAACCATCCTGGCAGTTAGTATATGTGGTTATAGGAAGACTTTCAATTGAT
GCCTTGATGTTGTTTTCTAAATATTTCACAAGTTACACTGAAGCGTTACTTGCAATAAG
CTAATTTTATTGGCTTCATGAATTCTTTATTTCAGGTTGAAGTGACCAATTCAGCTCTTCT
TGCTTTAATAGAAAATTACTGCAGAGAAGCTGGTGTACGCAATCTGCAGAAGCAGATTG
AAAAGATTTATCGCAAGGTTCTGTGACCTGTCCTCTTTTGTATAATACATCTATGATGAA
ATTGTCTCGAGTCTCGTACCTAGTCAACTTTTGATGAAAACTGATGGTTATTTCATGAAA
TCCAATACGAACACTTTGAATCGTTTTCATGCTCTGATACTTTGCACATCCTGTTCTTGC
TTTTCAGATAGCTCTAAAGCTTGTCAGGGAAGATGGAGAGATTGAGCCTCAGAATGCAG
AGGTAGGTGAGGTAGAAGCAGAATCTATCCATCTATCAGACGAAATCAAGTCTAAGGAA
GAAATTCAAGCTGGAGCTGAGTCCGCAAACGGTAGCAATGATGACAAGGCCTCTGAAA
ATAATGCTGAAGCTGAAGCACAGGGAGCACCAGTGAATCAAACACAGAAATCTGCTAAT
GAAGATGCTTGTTTACAGGTAAATGAAAAACATTAAAAGCAAAATTATAATGTTTAGTA
CTTCAGGTGATTCTTGCCAGTTGTAACTATGTATGCTACAATGTATTTTAATGCTTCTAA
GTTTTATCTATGCTCAAAATAAAAATACAAGATGCAGTGTAATAGGTAGTTTTGTAGGCA
CAGAAGTGTCTTTTTACAACTTGTCTTACTGTCACATGTTGGATTAAATATGGTTAACAAA
TGAATGTAAGAAATCATTTATCTGGCAATATAACACCAAACAAGCAGGGGAAGCACTTT
CTTTTGAAATGTTAACTAAGAACAAGCAAGGGAAGCGCTTGGTATTCATTTTCTACGCTA
AGTCCCCCCTGTATCTTAAAAAGGTATCTATGCATAATTTGCATTTATATCAGACTGTAA
GACAAGAGTGGGTTGCTCTAGTGGTGAGCACCCTCCACTTCCAACCAAGAGGTTGTGA
GTTCGAGTCACCCCAAGAGCAAGGTGGTGAGTTCTTGGAGGAGGGAGCCGAGGGTC
TATCGGATACAACCTCTCTACCTCAGGGTAGGGGTAAGGTCTGCGTACACACTACCCTC
CCCAGACCCCACTAGTGGGATTATATTGGGTTGTTGTTGTTATCATACACTGTAATCAGT
TAAAATCAAATTCTTTGGGATGGAGGGGTTTCTTTAGTCTGGCTAATTAGACTTGATAGT
CAAATTTGTTTTAAGAAGTTGTGCCAAATGTTAACTCACTTGTTATAATCTAGCGCGCGC
ACACACATTTTGATATGTTTGATGTTAAGCTGCTAATGAATAACGTCCTTATATTTTCCTT
GACATTGCATGATTTTATGTTTACATATTTATCTGCGTTTAATTTAATGCTATTGTGTCCT
TGCAACTGAATTTTGGTTCTGAGTGGTTGTGATCTTAACAGGATACTCAAGAAACTGAG
AAAGCAACAGAAAGTGAAGCGAGTAAAACAGTAAATAAAGTGGTTGTTGACTCGCCAAA
CCTAGCTGATTATGTTGGCAAACCTGTTTTCCATGCGGAGCGCATATACGATCAGACAC
CAGTTGGAGTTGTGATGGGTCTTGCTTGGACTTCAATGGGTGGCTCAACACTCTATATA
GAAACATCTCTGGTGGAGCAAGGAGAAGGAAAGGGGCTCTCAATGTAACAGGACAAC
TAGGCGACGTTATGAAAGAAAGTGCCCAAATTGCCCATACGGTTGCCAGGACCATTTTG
CAGGAAAAGGAGCCTGATAACCAATTCTTTGCAAATAGTAAGCTTCATCTTCATGTTCCT
GCAGGTGCTACCCCTAAGGATGGCCCTAGTGCTGGTTGTACTATGATAACGTCCTTGTT
GTCTCTTGCCATGAAAAAGCCTGTTAAAAAGGACCTGGCAATGACAGGGGAAGTCACG
CTAACTGGCAAAATTCTTCCTATCGGCGGGGTATGTTAACAATTCTTACACCTCTCCTTA
TAATTTCATGCAGCTTTTGTGTCTGATCATCTATCATGTTTTCTTTTTATTTTTCGTTGATT
TTGTCTTTAATGTTCTTTATGCTTTAATTATTTCCTGTGTCTGTATGTGTTACATGCATGC
GCATGTAAGCATAATAAGAGTGGTCTTTTCTTTTTGACCCAACCAGTGTTGGGTTCCTTT
CTTGATTTTACAAAAGCTTTACCTTTTGGTTCAATAATAGGTCAAGGAGAAAGCCATAGC
AGCGCGAAGAAGTGATGTGAAAACTATAATATTCCCTTCAGCCAATCGCAGAGATTTTG
ACGAGCTTGCTCCTAATGTCAAGGAAGGCCTTGATGTACACTTTGTGGATGACTACAAG
CAAATATTTGATTTGGCATTT
SEQ 77 
ATGCAGTTTTTCCGAAGAAACCCATCACTTCACAGAATCTCCTCCAGATTCCTTAATCAA
GTTCGTTTTCTTTTCTTTTCCTTTTCCGAAGTATAACTAGCTTTTCAATTTTTGTTTGGCTT
TTCGATTATCTTACTAGTGTAACATATATTTCATATGTCTTGAGGTTCATTGCAAAAACTC
GTTATATTTCTAAATGGGGTTGAGCACGTGGTCCAATACAATTCAGTAGGACTTACAGC
 
TGTAGCCTGTAGTTAGAGGACATTGGATTAATTAATTATATGGCTGCAATTCAGATATTC
AAAACGTTTCTTTTCCCTGTTTGCAATTTTTTCCTCCAAGTAGTGAAACAGTGGAATTTTC
TCCCCATTCTTAGGTCAAGCTATCATCTTTTTGCTTAAGAGTTGGTTTGGATGTTTACATT
TATTTTCTAACACAATTTGTTTTGGTTTGCTGCTGATATCTCATGAATTGGATACGATAGG
TAGTCAAAACCAGTGCATATTCAACCAAGAAAGTTTACAATGCTGGGCAGCCGACTGCT
GCTACTCACCCTCAGGTACTTCTACACTGCATATGTAGTATTGACATTTGGTAGCATAGA
ATTAAGACCGTTGACATTAACAAATGATAAATGTGCAGTAAATTTAAAATCTTGTTTTCTT
GGTGGTTGTTTTCCTTGTATGGGACATACTTCGCTGTCCTTTGGAGCTCCTTTGTGAATT
TCTGTTAAATGTTGTTACATCACTGCATGCAGTTAATGAAGGAAGGGGAGATTACTCCT
GGCATTACCAGTGAAGAATATATGCAGAGAAGGAAGAAATTATTGGAGTTTCTTCCGGA
GAATAGCTTAGCAATTGTTGCAGCCGCTCCCATAAAAATGATGACTGATGTTGTACCAT
ACAATTTTAGGCAGGATGCTGACTATTTGTACATCACCGGATGCCAACAACCTGGTGGT
GTTGCAGTTCTAGGGCATGACTGTGGTTTATGCATGTTCATGCCAGAACAAAGCCCCCA
GGTATTTCAGGAACCATTCACTTGCTTCCTTCTTGTTGACAAGAAGCTGTTAATAAGAGA
AAAGCTTCGTCCTATAATTTAGTGACATTTTTCTTTAGATTCAGTTACTACCATGATTTTT
TGGTAGTTAGTATACATTGTAGCAAGTTAAAGATTGTTTCCATACTAAAAGTGAAAAAGT
ATTTTTAGGACGCTCTTTGGCAAGGAGAAACTGCTGGAGTTGATGCAGCTCTACAGATA
TTCAAGGCTGACCTTGCTTACCCTATTAACAGATTGCCTCAGGTAAATCTTTTTTAAAAT
CATATCTCCAACTGCAAATAAGTTTGAGATTCTTTTAGAAGCGAATACCTCTCACACTG
ATAAGTAAAAGGGCATATGATAACATCCCTTCTTTTATTCCTTTCAATAGGACAATGAAG
TACTTTATCTAAAAAGGGAGTGGAGACCTATTTGTCTCCTTTCCACTTGATTATAGAAAT
TTATGTCAGAGAATTGAATCTGTTAGAGTTGGCTTGTAGATACCTTTTGACTGTTGATGC
AATTCTTAATATGCGTAAAAGAATTGTTTTTCTCCTTTTTCTCTTTTCTTGCCGGGGAAAA
GAATTGTTTTCCTCCTTTAATATGCGTAAAAGGTATAGAGGGAACAAAGTGGATGGAAG
TTAGAGTTTTCACCTAAGTTGCTCCGACACGGCAATTTAGGTGCCGCACCCATATCGAC
ACGACACTAGTATGGGTGTGGGTATGGGATCCGTACCGGATCTGGTCAAACAATTTTG
GGTACTTTGACCACGACGGATGGAAAAATTCGAGACGAGATACAATTTGATTCCCAAAA
TCAGAATCTAAGGTAAATTTAAATAAAATAATATACCTTATCTAGAAAATCAATCCTTTAC
TTATCTATAACTTGAAAATAAAAAGGAAATCCACACTTTACAAGCTATACGTAAGTAATCC
ACAAAATTTCTCATAATTTAAAAATATTTTTATTTTTTTTGAATTATTTTTAGTCGGATCCC
CGCACCCATATCTGTACTAGGATCTGTATCCCCGAATCTTAGAATTTACATCTCGAAGG
ATCCAACCTCTAGATTCGCACCCATGTCGGACACCCGCACCCGTGTCCGAGCAACTTA
GGTTTTCACATATATAGGAGTCGGGCCTGGCTTATTACTATAAATTCATGTTTGATAGGA
CCTATTACTGGATGTAGCCTTTCCTCATAATTTTGAAAATCAAGCAGGCATCACGCTAGG
ATCGGTTGAGAATATAATATTATGGGTAAGAAATTGAAGAATGAAGGTTAACAGAAAGTG
GACACTGTGTTCCAAATGGAAACTAGGTAAATGATTTAGGCAGACGGAATATTTTTTGGT
TGGCTGTATTTGGCTCTCAGGTTAACCGTTTGACCACTTAATGGTAATTTACTATTTAAA
TCGGCAACACAGAGAACAGGAAGTGAAGATGTATATATGACTGTGTTATTTGTAGAGAA
CCAGTTTATGGTGTAGGTTTTCTAGTTATTGTAGAGCACTTGCGTACAAGAGTTTAAATT
CCGCAACATCGATAAATTCTTACCTTATAAAAAAGAGCAGGAAATGAAGACGAGATGCC
GATATCATGATTAGATCTATGTCAGCAAAGAAAAAATGTCAATTATTTCCTTCTAAGCTGT
CTTTCCTGTACATGGCTTCAATGTAGTGACTTTGTTTCACTTTCTCCATGTTCCAATTTCT
CTTCTCTAATTTTTGCTGGACTTGTCAGATTCTCTCCAGGATGATAGAAAGTTCTTCCAC
TGTGTTCCATAATGTGAAGACAAGGACTTCATCCTACCTGGAGCTTGAGGCCTATAAAA
AAGCAGTTAGCAATTACAAAGTGAAAGATTTCTCTGTGTACACTCATGAAGCCCGATTTG
TGAAGTCTCCAGCAGAGCTGAAATTGATGAGAGATTCTGCATCTATAGCTTGTCAGGTA
ATGGTAGTTCTTTCATTTTTGTCAGGTTCATGGGTTAGAGTGGTAGTTCTTACTCATAGA
GGTTCTTGTTTTTATTGGACATGGAAAAGCAGTCTCTGGGTTATAGAGATGGAAAGATA
GAGTGTACACTTGACACTACTTTTGTATGTTTATTTGTTTTCCATTGAAGTTGATACTCTT
CACACAGTTAACATGTGACTAAGGTATTGATATGCCAGCGAGGTGTTTTCAGAATTTTAA
AAAGCTTATTTGCAGGTCAGCTGTTATACAATCTTAAGTAACATGTTTGTCATTTTGCTAT
 
ACGACAAAATTTTTAGAAAGGTAAAATAGGTATTTGCATTTCCCTTTTTTCCTCTTCTT
CTTTCATGTCTAGGGTGGTGTCTTCAGGTTGAAGATACTACACTTCTGAGGATCTAAAAA
ATATTTCACAAAAGGAAAAAGGGTACAGTCAGATAAAAGGATCACCAGTCTAAAAGAAG
ACGGTTCTTAATATTCCAAAAGTTGGAGTCCCAGCTTTCTTACTTGGGTCAACATATTCT
TGGTCTAATTGTGAAGGAACAGTTCTTGCATGTACAATCCTTTCTTTGATAATGTGCTTC
TGTGTTAAGTAGTTCAGAAGCTCTAGGCATGCTTAACCAAAAGATGTGTATATACTACTC
ATTCATTCTATTTCACAATCATGATTTGCATGTTTTCTTATGAGAGACTGGTCTAGAAAAT
GCTTCTTCCTATTCCTGGATTTGTATGCAGTTGCCTAGCAATAAAGTTGCCAGTTATATG
GGAGTTGAGATATTTTCCTTTCACTAATTCAGTCCTTTTTTATACTGTATAAAGGATATTT
TTTATTTCTTGATCTTTTAATGTCTGTCTTGTCTTTCGGAAACAGCCTCTCTACCCCTCG
GGGTAGGGGTAAGGTCGGCGTACACACTACCTTCCCCAGACCCCACTAGTGGGATTTC
ACAGGGTCGTTGTTATTATTGTTGTTCTTTTAATGTATATATTTTTGGTAGGCACTTGTCC
AGACCATGTTGTACTCGAAGTTGTTTCCTGATGAAGGAATGCTGTCAGCCAAATTTGAA
TATGAATGCAGAGTTAGAGGTGCCCAAAGAATGGCGTAAGCTTTTTCTTGTAATAATTTT
TGGAAGTTTGTATATAGAGAGGAGCACGTTGCAATTTCTAAGTATTTTAGTCTAACATGA
GTTGCAGGAGAGTAAATCAAAATGCCACTAAGACCTCATGTGTAAACATGCAATTGATTT
TCTTTTTCTTCTATTCTCTGCGATTCTGATAATTTGTTGTTTTTCCTGACTGTTAGTTTTG
GTCATACTTCTGGTTGAGATAGTTTCAAGGATTATACATTTTTCTTTTCCTGTTCCACAG
GTTTAATCCTGTTGTTGGTGGCGGACCTAATGGCAGTGTCGTGCATTATTTTCGTAATG
ACCAGAAAGTATGTTTACTGTCTTTAAGCACAGTTGAATTTGAATATCAAGCATATTGAG
TAGTAGTATCTAATTTGTTGTTTTAACAGATTGAAGATGGGGACTTTGTTGTTTTAACAGA
TTGAAGATGGTAACCTTGTCCTCATGGATGTTGGATGCGAGCTCCACGGTTATGTCAGT
GATCTTACTCGTGTTTGGCCGCCCTTTGGAAAATTTTCTTCTGTTCAAGTAAGTATAGAA
TCCATGATTTTCTTCTCCGTTTTCCCCTTAAAACTCAAGTCAAACCCCACTCCTCTGGGT
AAAAACCCTGTTAGCTGATCAAAGTCATAGACAACCTTCCATTTCAGAAAGAATGCACTG
ACCTAAATTGAAACCCCAGACTACCAAAAGCAATCTAGAAAGACAAACGGTAAAATGAA
AATCATCAGGTAATGTAGCCTAGCAGCTAGCTTCACCCTCCAGTGGTATGAGTTATGAA
TCTTAATTCCAGATCCTCAATGGCCTTGCTGATTTGATGTGGATGTGAAGAATGAGAAT
GATATAATCATATAAAGTTCCTCCTTATTAGAAAAACAAAATTTCAATTTTACTTCTAAGC
TACAGATTATGCTTGAGAAATAAATTCTTCCCTTGGTATGGTTTTAAATTGCTACTTTTCT
GTGATATAGTCTCTATACATTATTGTCTGGAACTTAGTGATACGCTCCAAGATACATTTC
AGGAGGAACTTTATAATCTTATTTTGGAGACAAACAAGGAATGCGTGGAGCTGTGCAGA
CCTGGCACAACCATCCGAGAAATACACCACTACTCGGTACTATTTTAGTTAATCCATCTC
GTAATTTCTTTTGGTTTATATTCAAGGGGTAGCTGAGAGTAGGAATTTAATTTTTTTTCTC
TTGCCTTTCATAGACTTAGACCCAGTTATATTGCCAAGTTACATTGGTAGTCTCGGTGAT
AGAAATTTGGGTACAGTTGTGAAGGCCCTACTCTTGCTTTATGTTTTGCCTAATTCTCAA
GTTACACTGACTTCCACCTCCTATTGTGAATAGCAATGTTGCTTCAAAGTTTTCGTTCCT
ATGCATGAGCCGAGGAAAATGAGGCTTGATGGCATTTTCGAAGATAAGGAAAAAAGTTT
AATTCCTTTTAAGCCTTTGAGTATAGAGGTGTTGGGAAGAGATAGATGCCTTTTGATTGC
CCTCCCTTGATTTGAAAATAGTATTTGTTCCCCCATTTCTTCATATATGATGAATAATGCT
TTGTAAAATAAGCCAGTAAGGTAATGATTAGAGGTGTCTAATTAGTGTAGTGTGTGGTAA
TTGATTCTCAGTGAAGGGTAGTATTACCTGGTTGATGCAAATGCTTTATGAATTAAGGGT
CATTCTCTGCATTTGTTGTTAGGGCAGCTGTTAAAGGTCTTATTACTGTTCAAGATATGG
GCGTATATGGTATATATTCTGTAGTGAGTAATTGCCCTATATCAGCATGCTCTTTTCTTTA
GATACTTGAGGACTGCCAAGGTCTCATTCTTTTTTTTTTATTTGATGTGTATAGGTAGAA
ACGCTGCGAAGAGGATTCAAAGAAATTGGGATACTAAAAAATGATCGGCGTGGAAGATA
TGAAATGTTAAATCCTACAAATATAGGTCTTTCCTTTTAACCCTTACTCTTCCGCTGCAG
ATATAAGTACAAATGCATGTGCAATAGCAGCAGAAACCTGCTCCCTCTCAATTGTCTTCA
CAGTTGCTAATGCTATTCCTATTATGCTTTTTGCTGAAAAGAGAAATGATTTCTTGTACC
GCTGCAACCATCTCTGAATGAATTTGGTTTGTTCAATTATATTTCCAGGTCACTATCTAG
GAATGGACGTTCATGATTGTTCTACAATTGGAAATGATCGACCTCTGAAACCTGGTGTA
 
GTAAGTTTCCTTCCTTACTGATGATTGCTTTGATATTTGAAAATAATCGGGAAACTGCTA
GGTTTGCAAAAGAAATTGGTTGTCATTATTTTGAAATCCTCCTAACGAATGAGGACCAGT
GACTTGCTCATTTGGAAACAAATGAGCTTTGCCATAATGCATCACTCTCTTTTAGCAATT
TACAATGAGTTTTCCTCCATAGGATAGTTCAGTCATTCTCTCTTTGCTTCTTGACTGGAT
TACAATATGAACCAACTAAGAATGCTTATGTTTTTAGAGCCATGTGGTCAAATTTCCCTT
TTCCTTCACTTTTTTCATTTTTATAGAGATGCAAAGGGTTAAAAGAGAGGATTGAATGAT
GATATATATGAATATTTTCTAAACTGCTTTTGTCATATGCACCTCTTTGGTCCTATTGCAG
CTTCTAATTTCATCACTTCTACTAGTAGTTACATGGAGAAAGTTAAATTCAGAAATGAAGT
TGGTTAGGTTACATGGAGAAAATTAAATTCAGAAATGAAGTTGGTTAGGTTTATTTCTAT
TGAAGGATGTGTACATCAGGGTAGTGCGTGTATTTGCATAAAAATTATGTTTGAAATATC
TGACGGTCCAATTAGCAGAAAGATCAAAGTATCTTTTGCTTCTCTCTAGATTCTATATAG
ACCTTTGTTTTGATTGATTAATTTGAAATATTTGAAATGATTAATTCCTCGACCGCTTAGT
TCATTAACCCCGGCTTCATACACGATGCAATTTTTGTTATTGATAAAGGTTTCGTTCCTG
GTATGTCTACTCTTTATCAAAAAGTAAAGCTCTTGTATATCTTTTCATTCTGAGTCACAAG
GAATGTAATATTCCTGCAGAATGTGTAATGTTATATGCAATTGTAGTAAAATTTCTCAGTA
GCTCGCGCATCTTGTTTTCATGTTGGTACTGCAAACTGTTAACTTATACTTTATGTATAAT
CCATTTCATGACAAGTACCTTGCTCTTGTGAAAGGTACTTAACGTCCAACATGCTTGCTT
CCATGTATGAAAAATTATTAGTTATCCCATTTTGCTCCTTTTTCCTTCATTCTTCTAATCAT
AAAAAATTGGAATATGCTCCCGACCTGTCTGATATGACAATAAAAACATACACAATATAT
ATCAAGTCAGCTGTTATATGCAAAATTACTGAAGGTAATTCCAGTAATACAGCTTATTGG
TGTCAGCGGTAGATTTATGTTAAATTATGCTTTAACTGAGGTCTATTTTGCCAGTGATAT
CTGTATCCATGCATTTGTTTTTAGTCCTTACAAAAAACAATTTCAGAACTACTATGTTTTT
GAATAAGAAGCACCATGAACATGCTACTTAGAGGTCTTAGTTTGTATAATTTATGTTGAT
CATATCTGTGGAGAAATATGCTAATTTTGCGTGGGCCGTGGCATTTGTATTTGAAAGAA
GTACATGACTTTTGATTGTTCTGAGATTATGTGCAGAGTCTAGTTCTTTGACTTCGGGAC
TATGGAAAACTTTGTGTTTACTCTACATTCATACATCTAGACAAGGTCATGGCCAATTGC
GAAACATGCTTTACGTTTTTTTAAAAGTGACGGAGACGCATATAGCTCAGAATGATGAG
ATGAAATTGGACAAAGCTAATAGTCTAGGATAAAATTGCCTGGTCATTGTTTAGACATTT
GTAAACTCCTGTTCCCTCTGTTTGTTTCTACATTAACCTTGATGAGGCGGTCATTGATAA
GAAGAATCTCTGACCCCATAAATAGATGAGTCCTTTTCATCTTAGCTTCCAATTACTTGT
GATTTCTGCAAGAACTTGTGATCAATCTTCATGGACTGTTATATGTAGGTCATCACAATT
GAACCAGGAGTATACATCCCTTCATGCTTTGATTGTCCAGAAAGGTAATACTTGTTACCT
CATCAAATTAATGTTCCTTTTGGCATGCATTCAGAAGTTACTGTATCTTAGATCATCCTC
CAGATTCTTGGTTTATTGAACTGGTTCTATCTGCCAAAATGTATTATGGTGGATGGACGA
AGAGTTACTCTTTCATGCAGAAAAGATTGAGAATCATATAAAGATGTCTGTTCTAGGTGG
GTCAGGTAGATCTTGTAGGTCTAGTCAACATGTTAATCTAAGGACATTGTAGCAGAAGT
AGTCGTATCGTGCAAAGGAGTCCCAGTTTGGTTGGGCTTTCAGTGAATGACTGATGGCT
ACTTGTATGTTCAACCGAGAATGGGATCAGGAACCTTTACTTCGAAATTTCTTTTGCAAG
GTTCGAGAAAACTTCCCAGAAACAAATAGCTCGAAAGAAATATGAAAGAAATATTCCAC
GTATCAATGGCATATTCTGGCTGTCCCATTCTAGGGAAAATTGTTGCTTTTGTCATAAAT
CTTAGGGAGAAAGAATTATTGCTCACTTGAACTAAGAAGCTTCATCTGCTGATTCATCTA
TTGTTAAGGAGCTAGATTATTCTCTTACCCCAATAAGGAACAACGTGTCTGTTTCCTTAG
AATTCTTGATTTTTACTCACTTATATATGTCTATATTCACTTATGCTCATTCAGGTTCCAA
GGCATTGGATTTAGGATTGAAGATGAAGTCCTTATTACAGAATCAGGTTATGAGGTATA
GTTACAGAAATCGTTCAATTGTTTGAACAACCGAGTTATACAAGTACCAGTTCATATGAT
CTCTGATACTTTGATCACTTCCGACACTTGTTAGCATTCAAGACCTGATTTTCTGCCCTA
CTGGAAACAGGTACTTACTGCATCCATACCGAAGGAAATTAAACACCTCGAGTCCTTGT
TGAACAACTTTGGCAGTGGGAGAGGAACAGAAATTAGAGCTGCTCTCAGT
SEQ 78 
CTACTCACCTCTCACAAAAACCATATAATTCTCCTTCCCTTTCTTCTCTACAAAATCTTCA
TTTCTCTCCAAAAACAAACTCTCATGGCTTCTTCTACTAGAGTTTTTGTTCTTCTCCTTCT
CATAATCTTCAACTTTCTCTACATCTCAGCACAAAAACCATTAAACATAAGCCTTTTTCA
ATGTCATTTCCTCTTACTTCAACATCTTTATCACATAACTCTTCTTCTAAAGCTCTTTTTCT
TTCTTCCCTTTTGGCTTCTAATCAAAGAAAACAAGCTCCAAACACAAAAACTGTGTCTAG
AATTCCATCTTTGAACTATAAATCAACTTTCAAATATTCAATGGCTTTAATTGTTACACTT
CCAATAGGGACACCACCACAAAATCAACAAATGGTTTTGGACACAGGCAGCCAACTTTC
TTGGATTCAATGTCACAAGAAAATTCCAAAAAGACCCCCACCAACGACGTCGTTTGATC
CTTCTTTGTCCTCCACTTTTTCTGTTCTTCCTTGTACTCATCCTTTATGTAAGCCAAGAAT
TCCCGATTTTACCCTTCCAACTACTTGTGACCAAAATCGCTTGTGCCACTATTCTTACTT
TTATGCTGATGGTACTTTAGCTGAGGGTAATCTTGTCCGTGAAAAATTACATTTTCACG
TTCCCAAAGTACCCCTCCTTTGATTCTTGGTTGTGCTACGGAGTCCGAAGATGCCGAGG
GTATTTTGGGAATGAATCTTGGACGGTTTTCTTTTGCCTCCCAAGCTAAGGTACAAAAAT
TCTCATATTGCGTGCCAATTAGACAAGGTAGCCATGCAGTTAAACCTAGTGGAACATTTT
ACCTAGGCCAAAACCCTAATTCCCATACATTTCAATATATAAATCTTTTGACTTTTCCTCA
AAGTCAACGCATGCCAAATTTGGATCCACTAGCTTTCACTGTTGGCATGGTAGGGATAA
AAATTGGCGGCAAAAAATTAAACATCTCCGGTAGGGTTTTCCGGCCAAATGCTGGTGGT
TCTGGCCAGACGATCATTGATTCCGGCACGGAATACACTTTCTTAGTGGAAGAAGCGTA
CAATAAGGTCAGAGAAGAAATTGTTAGGTTAGTTGGTCCAAGATTGAAAAAAGGTTACG
TTTATGGTGGTGCACTTGACATGTGCTTCGATAACCGTCCGATGGAAATCGGACGGTTG
ATAGGTGATATGACATTGCAATTTGAGAACGGGGTTGAGATTTTGATCAATAAGGAAAG
GATGTTGGATGAAGTAGAAGGTGGGATCCATTGTGTTGGAATCGGACGGTCAGAATCA
CTCGGAATAGCAAGCAATATTATTGGTAATTTCCATCAGCAAAATTTATGGGTAGAATTT
GATATGAGAAATCGAAGAGTAGGTTTTGGCAAAGGAGAGTGTAGTAGGCAAATG
SEQ 79 
ATGGCTGCACTCAATTTCTTCATAATCTTCACATCACTAGTCTTACCAATTGCATCTGAT
CCTCTGTTGTCAACTTATGTTGTCCATGTTGACACCAAAGCCAAGCCATCTCATTACTTA
ACTCAAGATGAATGGTATAATTCAGTGGTTGAGTCAGTTCTTGCAAACAAAATGGACTCA
GATTCTACTTCTCCAAGATTGTTCTACTCATATGATGTAGTGTTACAAGGTTTTGCAGCA
AGATTGACTGATCAAGAATCTGAAAAACTAAATAAATTTCCAGAAGTCATTCACATTTTCA
AAGATCAGTCTAGAATCAAGCTTGACACAACACGTTCGCCGAATTTTCTTGGCCTAAAC
ACAGGTTATGGTCTGTGGCCACAATCTAACTTTGGAGATGATGTTATAATTGGCCTTGTT
GATACAGGGATTTGGCCTGAGAGTGAGAGTTTCAAGGACAATGGTATTGGTCCTATTCC
AACAAGGTGGAAAGGTAAATGTGTTGATGGAATTGAATTCAACGCGACGAGTAGTTGTA
ACAGAAAACTTATTGGTGCTAGGAATTTCGTTAAGGGTGTTGAGAATGACTATCATCATC
AATCGGCACGAGATCAAAATGGACATGGAACACATACTGCTTCAACTGCAGCAGGTACA
GAGGTAAATGGTGCCAATGTATTTGGTTTTGCTAAAGGGAAAGCACGAGGGATTGCGA
GTAAAGCTAGGATTGCAATGTACAAAGCTTGTGGGAGTAGTTCTTGTGCAGAATCTGAT
ATTTTAGCAGCTATTGAAAGTGCTATAAAAGATGGCGTAGACATACTTTCGCTCTCTTTA
GGATACGATGATGCTCCGTTTTATGAAAATCCAGTGGCAATTGCAACATTTGCTGCTGT
TAAAAGGAACATATTTGTTGCTTCTTCAGCTGGAAATCTTGGACCTTATCCATTTTCAGT
TCACAATACAGCACCTTGGGTTACAACAGTTGGAGCTGGATCACTTGATCGCGATTTCC
CCGTTGAAATCAACTTATCAAACAACAAGACTTTTGTTGGTTCTTCTCTTTATCCAGGGA
GAATCAGTGGTAAAAGTTACTCTCTTGTTTATATTGAAAATTGTTCTATAATGACAATCGA
TCGTTCTAAAGTTGAACGAAAGATTGTAGTTTGCAACACTAGTAAAATCGAAGCTCTTAG
AAATGGGATTTTAATTCAGAAAGCAGGTGGTTTTGGACTGATTCAATTAAATCTTCCAAC
TGAAGGAGAAGGGATTAGAGCAATGGCTTACACATTGCCTTCTGCAACATTGGGTTATA
AAGAAGGTATAGAGCTTCTTTCTTATATCAAATCCAATGCTAATCCAAGAGCAGGGTTCG
 
TACGTCGAAAGGATACAGTAATTGGGAAAAAAGTTAGAGCTCCAATTGTTGCTAGCTTTT
CTTCAAGAGGGCCTAATGTTGTTGTTCCTGAAGTCCTCAAACCTGACCTCATTGCTCCG
GGTTTGAACATTCTTGCTGCATGGCCAGGTAACCAGAGACGGATCCAGGATTTATACCT
TATGCATTCAACCTTTATTCTTTACCATTGACCCCACGACACTTTTAAACTTATGAGGTA
GGAATTTTATACTTTTTGAAATTGTTGTGATTTTTCATATTGCGTGGAAGCAACCACTAAT
GCTTATGGTAGGATAGGCTGTCTACATCACACTCCTTAAGTGCGGCCCTTCGCCCGAC
CCTGCGTGAGCAAGGGATACTTTATGCACTAGTCTACCGCTTTTCTTTATTTAGTGATTC
TTCACATTGTGTGTGTCTATGCAGGTGACATTTCCCCAACACGTCTCAAGATGGATCCA
AGGAGAGTGAAGTTCAATATAAACTCGGGAACATCAATGGCGTGCCCTCACATAGCCG
GAGTAGCTGCATTAGTCCGCGCTGTTCATCCAGATTGGTCCCCGGCTGCTATAAAATCC
GCACTCATGACTACATCCACAGCATTCGACAATGCACAACTCCCTATCATAAAACACGA
AGACATGGAGCTAGCAACTCCGATCAGCATTGGAGCCGGGCACGTGAACCCTGAATCG
GCTATTGATCCGGGCCTAATATACGACACTGATACATCAGACTACATCAACCTACTATG
CAGCTTGAATTACACAGAGAAACAAATGAAACTTTTCACGAACGAGTCAAATCCTTGCTC
GGGTTTCACTGGATCTCCACTTGATCTTAACTATCCATCACTTTCTGTTATGTTCAGGCC
TGATTCCTATGTTCATGTAGTTAAGAAGACACTGACACATGTCGCGGTATCTAAGCCCG
AGGTGTACAAAGTAAAGATAGTGAATCTGAATTCTGAAAAGGTGAGTTTAAGTATAGAG
CCAAGGAAGCTGATTTTCAATGAATCTTTACAGAAACAAAGCTATGTGGTCAAATTTGAG
AGCCATTATGCATTCAACAGCAGCAGGAAAATAGCTGAGCAAATGGCGTTTGGTTCGAT
ATTGTGGGAGAGTGAAAAGCACAATGTTAGGAGCCCCTTCGCTGTTATGTGGGTTCAG
CAAAATTTCAATAACAGTAGATTATACAAA
SEQ 80 
TCAAAATGCCAAATCAAATATTTGCTTGTAGTCATCCACAAAGTGTACATCAAGGCCTTC
CTTGACATTAGGAGCAAGCTCGTCAAAATCTCTGCGATTGGCTGAAGGAATATTATAG
TTTTCACATCACTTCTTCTCGCTGCTATGGCTTTCTCCTTGACCTATTATTGAACCAAAA
GGTAAAGCTTTTGTAAAATCAAGGCACTTCAGAAAAGGAACCCAACACTGGTGGGGTCA
AAAAGAAAAGACCACTCTTATTATGCTTACATGCACATGCATGTAAACACATACACACAG
AGGAAATAATTAAAGCATAAAGAACATTAAAGGCAAAATCAACAAAAAATAAAAAGAAAA
CATGATAGACGATCAGACATAAAAGCTGCATGAGATTATAAGGAGGTGTAAGAATTGTT
AACATACCCCACCAATAGGAAGAATTTTTCCAGTTAGTGTGACTTCCCCTGTCATTGCCA
GGTCCTTTTTAACAGGCTTTTTCATGGCAAGAGACAACAAGGACGTTATCATAGTACAA
CCAGCACTAGGGCCATCCTTGGGGGTAGCACCTGCAGGAACATGAAGATGAAGCTTAC
TATTTGCAAAGAATTGGTTATCAGGCTCCTTTTCCAGCAAAATGGTCCTGGCAACCGTAT
GGGCAATTTGGGCACTTTCTTTCATAACGTCGCCTAGTTGTCCTGTTACATTGAGAGCC
CCTTTCCCTTCTCCTTGCTCCACCAGAGATGTTTCTATATAGAGTGTTGAGCCACCCATT
GAAGTCCAAGCAAGACCCATCACAACTCCAACTGGTGTCTGATCGTATATGCGCTCCG
CATGGAAAACAGGTTTGCCAACATAATCAGCTAGGTTTGGCGAGTCAACAACCACTTTA
TTTACTGTTTTACTCGCTTCACTTTCTGTTGCTTTCTCAGTTTCCTGAGTATCCTGTTAAG
ACCACAACCACTCAGAACCAAAATTCAGTTGCAAAGACACAATAGTCATTATATTAAATG
CAGATAAATATGTAAACATAAAATCATGCAACGCCAAGGAAATACAAGGACATTATTCAT
TAGCAATTTAAATTGGTAGAATTCTATATATTTTTCTTTCAGTACAGACCAATATGAGGGT
AGAAATAACATCAAACATATCAAAATGTATGTGCGCGCGCTAGATTATAACAAGTGAGTT
AACATTTGGCACAACTTCTTAAAACGAATTTGACTATCAAGTCTAATTAGCCAGACTAAA
GAAACCCCTCCATCCCAAAGAATTTGATTAACTGATTACAGTGTATGATATAAATGCAAA
TCATGCATAGATACTTTTTAAGATACAAGGGGGACATAGCATAGAAAATGAATACCAGG
CGCTTCCCTTGCTTGTTCTTAGTTAACATTTCAAAAGAAAGTGCTTCCCCTGCTTGTTTG
GTGTGATATTGCCAGATAAGTGATTTCTTACGTTTTGTTAACCATATTTAATCCAACATGA
GACAGTAAGACAAGTTGTAAAAAGACACTTCTGTGCCTACAAAACTACCTATTACACTGC
 
ATCTTGTATTTTATTTTACGCGTAGATAAAACTTAGAAGCACTAAAATACATTGTAGCAT
ACATAATTACAAATAGCAAGAATTACCTGAAGTACTAAACACTATATTTTTGCTTTTTAAT
GTTTTTCATTTACCTGTAAACAAGTATCTTCATTAGCAGACTTCTGCGTTTGATTCTCTGC
TCCCTGTGCTTCAGCTTCAGCATTATTTCAGAGGCCTCATCATCATTGCTACCGTTTGC
TGACTCAGCTCCAGCTTGAATTTCTTCCTTAGACTTGATTTCGTCTGATAGATGGATAGA
TTCTGCTTTTACCTCATCTACCTCCGCATTCTGAGGCTCAATCTCTCCATCTTCTCTGAC
AAGCTTTAGAGCTATCTGAAAAGCAAGAACAGGATGTGCAAAGTATCAGAGCATGAAAA
CGATTCAAAGTGTTCGTGTTGGATTTCATGAAATAACCATCAGTTTTCATCAAAAGTTGA
CTAGGCACGAGACTCGGACAATTTCATCATTAGATGTGTTATACAAAAGAGGACAGGTC
ACAGAACCTTGCGATAAATTTTTCAATCTGCTTCTGCAGATTGCGTACACCGGCTTCTC
TGCAGTAATTTTCTATTAAAGCAAGAAGAGCTGAATCGGTCACTTCAACCTGAAATAAAG
AATTCATGAAGCCAAATAAAATTAGCTTATTGCAAGAAACACTTCAAGTGTAACTTGTGA
AATATTTTGAAAAACAACATCAAGGCATCAATTGAAAATCTTTCTACAACCACATATACTA
ACTGCTAGGATAGTTACACGTGTTAAAATATTTAAACCAAACATCAACCATTCAAGGCTT
TGAATTGCAATCAATTGCTAATATAAATCATGCACACTTAATTTTCCAGCTTGATATCACA
TCTTTTTTTTGATAAGGTGAAGATTTTATTAAAAACAGTATCAAGCTGATACTGTAAAAAT
ACAAGGACACTGCTGGCTTAAAAACATTAAAATCCTAAGCGGTCTAGCATGTCCAGCTT
GATATCACATCTCTGCATCTGAGAAACAGCCTCAGCATGTTAAAAGCCTGAGAGACAAA
GAGATGACATATACATGGACTCAAATGTTGCATCTTTTCAACTAGATTACATATCAACTC
GAGTAATTTTACAGGAGCATTAGGGAGCTAGCATTTCCCGAGAAATGGTACCCAAAGCT
CTACCCTAGTCCTGTCATATGTTTGCCATTAGATAAGCATAAAGCCACTACTATGTCATC
AAAAGTACCAACAGCCTCAAGATACTGAAAAAATTAGAACTGCTCCTATCCGCTCTGGA
GGGCCAAAACAAACCAAAGTAGCCATCAGCCCTCCCACATATCAACTTATTAACTTCTG
ACGCCCATATTTAGGGTGGATTTTTTTTTTTTTTTTTTTTTTTTTGGGGGGGGGGGGGG
GTAGGCACAGTAAATGACACCGCAGATATAGATGAGGTCAAATGTGTCATTTTGATGCC
CTTAATCAGTAAGTATGCAGCTCCCTTTTCTTCACCAGACATCCATATGGCATGTAACTC
AACTGAAGATCACATTAGAGAGCAAAATATAAGATCCACCGAATTCCATTGATCTCATCT
TTAAGGCTGTGGGAGAACATATTTACTGCAGTCATCTTATAACTTAAGCTCCCTCAATAG
ACATTATCAAGTGTTTTTTGGAGTCATCACCACCAATCTTGGCATTGTATGATGATTCCT
TGTACAGTAATAAAACATTCACTACTGTGTACTAGTCTAGTTTTTCTTTTACACGATGGTC
AAATTGAAGTCAACAACAAAAAAAACAAAACACTTGGCATATTTAAACATCCATCCTGC
TTTAGTCCATGCCACACTCAAATCTGTTAGACAATACCATCCAGCAACCCCAGCCCACC
AGGCACATGTATCCCTACCAGTAAGAAACTCATTATACCTCCATCTACCAATAGAGATTG
GCGACTACCACGACAGATCCACCTTCAATTGCGTTTATATTCCCAGTAAAGTGTGGCAC
AGTGGTTGTGTTAATGATGCACGAGCACATTGGCTAGCAAAGGCAGTCACAGTTTGGCT
CGGTCAAGCAAACATTCCTCAAGAGAAAGCAGCACTTGAGAGGATGTATACAGCAATGT
GGACTCATTCAGTTGTTTATGTGAACCACTTAAATACTTCTTGAAAAGTATAGTGATGGA
AGAGTTTGTATTAAACTTTATAATCCATTAGCTTTGGAGGTAAGTATATTCCAAATGTAGA
GGCTTAAGATGAAAATGATATCAATACATCAAACAGTATATTTCCTAGGAAATATTAAAG
CATATGGCCAAAAACCTATAATGGGATTTACTATATCCGTGCGATTTTTTTCCTTTAGCC
ATGTTCCATATGGAAGGGTGTAAAGAGCTTGATCCCCTGAACTTTCCAATCATATGTTTT
ATTCCATAAACTTCTAACTGAATAAAGCTTTTCACATTTTACTTTAAGAAGTTACATTCTT
GCCCAATCATGTTTCAAACATTTTCCCAACAGACCAATGTCTGCTTCTGAAATGTCAAAA
ATTGGGAGCGGAAAAAATACAAAACTATTCACCCGATAATGAATATGCACAGGAGATGA
TGAAAGAGATGCAACTAGAGACGTTTCTATAAGACATACCTGCTCGGGCTTGATCCCAC
ATGTTTCACGAGTAGCTTTCTCCAAATAATCCCTGGCTATGTGCACTTTCTCATCCGTAA
TGTAACCAGCAATTGAAATTACTTCCATTCTATCCAAAAGAGGATTAGGTATCATTTCTA
CAACATTGGCTGTGCAGACAAACAAAACCTACAGCCGCAAAAAGGAAAATGATCTACCT
CAAATTATGCACTCAAAAAATGGATAATGTGCAGATGTCATTCTCATATAAAGTACAGTG
GAATAGGTAATTGTATTAAGCAAAGAGGATAGAAAACACTGAAGGTGTCAAAGCTCCTT
TTTTTCAAATTTTGGAAGTACATTAAAACCTAGAGGTTGGATTTTATAATTTCTTCAGGTT
 
CATATATAACTCAACATCCATAACAAGGTTTGATATAGCCTCCCTCCCCATAACCAAGC
CAGACCCAAAATGCTACTAAAATAATCGAAAGATGCCATCTTCTTTCGACAGAGCCCTTT
GGAAATATAACACAAATATTTACCATTATGAAAAAGACATAGTAACACACAGTAAATTATC
ACATTAAAGGACAAAACAAATGAAATTGTATGCGAATATACCCATCATCCTTCTCTCTCT
AGCCATGTTGTTGATTTGCTGATGTTTGATATAATAAAATGAAATAGTACCGGTAACTGC
AAAGTCGTCATATTTGAATTATGGCCAAATTCTTTAATCAATAGGTAAACTAACGCTCA
ACACTCAAAGTCTATCGCATAATTGTAAAGTCGTCATATTTGATTTATGGCCAAATTCT
TTAATCAATAGGCAAACTAACGCTCAACACTCAAAGTCTATCGCTCCAATTATCGGCAAC
TTCAAGAGGAATAAAACCATATCCCTCATCAGTAAAGCCATCAAAGATAATATGAACAGA
CAGTAGGAAATCTCAATGCATCAACCTTTTTCCTATTGAATGATCACCAAAACCAATGCC
AATGAAACCTAGTTTTTCTCAAGTGTTAGATATAGAAATGTAGTTGTCCCACATTGGAAT
AGGTGTAGTATGCCTTTGTATAGAGTAGCTATAAATAAGCCCATCTTGTATTGCATTAGA
CACACAATATCAATATATCATATTTCTCCCGTGTCTTCTCACATGGTATCAAAGCAATC
GTGAGAGATTTATCGTTGTGCATAAATTCCAGCGACTCCGGGAAGGAAAATCAGTTGAC
CGGAAGCCTTTTCCGGCAGGTCTGCCGCAAGTAAAAAAAAAGCCACTTCGTCAGTGTT
GTGCAAAAAAACCAACACCACCACGAAGTAGATCGGGCTCTGGCAACCAACCCATAAA
AAAATCTCCGTCAGAATACCCTCCACGCGCCGTCACTTGCTACCGGAAGAAAATTTTCC
GGCGAAGTTCCGACGTCGCGTGGGCCACCTTCCAGCCATTTTTTGGCGACGACTCTTC
AGGACAAATTATTCCCCTTGCAATTCCGAGCCTACCCATCCAGGTTACACCAAATTCCA
GACAACTTATATATTTTTTCCAGCATGCATAGTGATTTCAAAAGTGGACTTCCGGCAATT
TTTTGAAAACGTTTCTTCAGAACAGTTGGGTCATCTGGTAATTCCGATCCTACCCCTACT
GTTTTTATTTCATTCCGACCACTTTGAATTTTCCCGGCAGCTACAGTACTATTCCGACTG
CTACAGTAATATTCCGATAGCTACAGTATTTCCTTATTCTGTTTCACTGTTCCTTACTCTG
TTTTAGTGGATTAAATTTGATTATTTCTATAATTTGGTAATAATTTGCAACGATGTCTATG
GGAATTGATGCTTTTGGGTCTAAAAACATGAGTTCTGGAAGCTCTAGTGTTATGATTACT
TCAAAACCTTTAATGTGAGGTTCAAACTACTTAGCTTGGGCTTCATCTGTCGAGTTGTGG
TGTAAAGGTGAAGGTGTTCAAGATCATCTAATTAAACAGTCTAGCGAAGGAGATGAAAA
GGCGATAGCGCTTTGGGCAAAGATTGATGCTCAATTATGTAGCATCTTGTGCCGTTCTA
TTGATTCCAAGTTGATGCCTTTGTTTCGTCCATTCCAGACATGTTATTTGGTTTGGGCAA
AGGCTCGTACCTTATACACTAATGACATATCTCGCTTCTATAATGTGATATCACGGATGA
CAAACTTAAAGAAGCAAGAATTAGATATGTCTACTTAATTGGGTCAAGTACAAGCAATCA
TGGAGGAATTTGAGACATTAATGCCAGTTTCTGCTAGTGTGGCAAAACAACAAGAGCAG
CGACAAAAGATGTTCTAGTTCTTACACTCGCTAGACTTCCTAATGATCTTGATTCAGTGC
GAGACCAGATTTTGGCTAGTCCGACTGTTCCCACAGTTGATGAATTATTCTCTCGATTAC
TCCGCCTTGCCGCACCACCAAGTCACCCAGTGATCTCATCACAAATACTTGATTCCTCT
CTCACATCGCAGACGGTGGATGTTCGGGCGTCTCAAACTATGAAGAACAGAGGAGGAC
GAGGTCGTTTTGGGAGATCTAGACCCAAGTGTTCTTATTGTCACAAACTTGGATACACT
CGTGAAATGTGCTATTCCTTACATGGTCGTCCACCCAAAAATCTTACGTTGCTCAGACT
GAGACTACATGTAACCAAGGTTTTTCTGTATCTAAAGAAGAATATAATGAGCTCCTTCAG
TATCGAGCAAGTAAGCAGACATCTCCACAAGTAGCCTCAATTGCCCAGACTGATACTCC
AGTTGTTGGTAATTCTTTTGCTTGTGTTTCCCAGTCTAGTACTCTTGGACCATGGGTCAT
GGACTCAGGCGCTTCTGATCACATCTCTGGTAATAAATCACTTTTGTCGAATATTGTATA
TTCACAGTCTCTTCCCACTGTTACTTTAGCCAAGGGATGTCAAACTAAGGCACAAGGAG
TTGGACAAGCTAACCCATTGTCTTCTATCACCCTAGATTCCGTTCTTTATGTCCTTGGTT
GTCCTTTTAGTCGTGCATCTGTTAGTCGTTTGACTTGTGCCCTCCATTGTGGTATATATT
TATTAATGATTCTTTTATTATGCAGGACCGCAGTACGGGACAGACAATTGGTACAGGAC
GTGAATCAGAAGGCCTTTACTACCTTAATTCACTCAGTCCTTCCACAACATGTCTAGTTA
CTGATCCTCCGGACCTAATCCACTGTCGTTTAGGACACCCAAGTTTATCCAAACTTCAG
AAGATGGTGCCTCTTTTAGGACACCCAAGTTTATCCAAACTTCACAGTCTACATTAGATT
GTAAGTCGTGTCAGCTTGGGAAACATACCTGAGCTTCCTTTCCGCGTAGTGTTGAGAGT
CATGTAGAGTCTGTTTTCTCCTTGGTTCATTCTGATATATGGGGTCCTAGTAGAGTCAGT
 
TCAACCTTGGGATTTCGTTATTTGTTAGTTTCATTGATGATTACTCAAGATGTACTTGGC
TTTTCTTAATGAAAGATCGTTCTGAGTTATTCTCTATATTCTAGAATTTTTGTGCTGAAAT
AAAAAATAAATTTAGTGTCTCTATTTGCATTTTTCGTAGTGATAATGCCTTAGAATATGTA
TCTTCTCAGTTTCAGCAATTTATGACTTCTCATGGAATTATTCATCAGACATCTTGCCTTA
TACCCCTCAGCAAAATGGGGTTGCAGAGAGAAAGAATAGGCACCTTATTGAGACTGCT
CGTACACTTCTAATTGAATCTCGTGTTCCGTTGTGTTTTTGCGGCGATGTAGTTCTCACA
GCTTGTTATTTGATTAATAGGATGCCTTCATCTCCCATCAAGGATCAGATTCCGCTTTCA
GTATTGTTTCCCCAGTCAGCCTTATACCCTCTTCCACCTCGTGTTTTTGGGAGCACATAT
TTTGTTCATAACTTAGCCCCTAGGAAAGATAAGTTAGCTCCTCGTACTCTCAAGTGTATC
TTCCTTGGCTATTCTCGTGTTCAGAAGGATATCGTTGTTATTCACTTGATCTCCGTAGG
TATCTTATGTCAGCTGACGTCACATTTTTTGAGTCTAAACCTTTCTTTGCTTCTGCTGAC
CACCATGATATATCTGAGGTCTTACCTATACCGACCTTTGAGGAGTTTCCTATAGCTCCT
CCTCCACCTTCGAACACAGAGGTTTCACCCATACTAACCATTGAGGAGTCTAGTGTTGT
TCCTCCTAGTTCCCCAGTCACAGGAACATCACTCTTGACTTATCATCGTCGTCTGCGCC
CTACATCAGGCCCAACTGGTTCTCGTCCTGCACCTAACCCTGCTCCTACTGCGGACCC
TGCTCCTAGGACACTGATTGCACTTCGAAAAGGTATACGGACCACACTTAACCCTAATC
CTCATTATGTTGGTTTGAGTTATCATCGTCTGTCATCTCCCCATTATGTTTTTATATCTTC
TTTGTCCTCGGTTTCCATCTCTAAGTCTACAGGTGAAGCGTTGTCTCATCCAGGATGGC
GACAGGCTAGGAGTGATGAGATGTCTGTTTTACATACAAGTGGTACTTGGGAGCTTGTT
CCTCTTCCTTCGGGTAAATCTACTGTTGGCTGTCGTTGGGTTTATGCGGTCAAAGTTGG
TCCCGATGGCCAGATTGATCGACTTAAGGCCCATCTTGTTGCCAAAGGATATACTTAGA
TATTTGGGCTCGATTACAGTGATACCTTCTCTCTTGTGGCTAAAGTGGCATCAGTCCGC
CTTTTTCTATCCATGGCTGCGGTTCGTCATTGGCCCCTCTATCAGCTGGACATTAAGAA
TGTCTTTTTTCACGGTGATCTTGAGGATAAGGTTTATATGGAGCAACCACCTGGTTTTGT
TGCTCAGGGGGAGTCTCGTGGCCTTGTATGTCGCTTGCGTCGGTCACTTTATGGTCTTA
AGCAATCTCCTCGAGCCTGGTTTGGTAAGTTCAGCACGGTTATCCAGGAGTTTGGCATG
ACTCGTAGTGAAGCTGATCACTCTGTATTTTATCGGCACCCTGCTTCAAGTCTATGTATT
TATCAGGTAGTCTATGTTGATGATATTGTTATTACTCGCAATGATCAGGATGGTATTACT
AATCTGAAGAAGCATCTCTTCCAGCATTTTCAAACTAAGGATCTAGGCAGATTGAAGTAC
TTTCTAGGTATTGAGGTTGCTCAATCTAGCTCAGGTATTGTTATTTCTCAAAGGAAATAT
GCTTTAGACATTCTTGAGAAGATAGGGATGATAGGTTGCAGACCTGTTGATACTCCAAT
GGATCCGAATTCTAAACTTCTGCCAGGACAGGGGGAGCCGCTTAGCGATCCTGCAAGC
TATAGGCGGTTGGTTGGTAAATAAAATTATTTCACAGTGACTAGACCCGACATTTCTTAT
CCTGTGAATGTTGTAAGTCAGTTTATAAATTCTCCCTATGATAGTCATTGGGATGCAGTC
GTCCGCATTATCCGGTATATAGAATCGGCTCCAGGCAAAGGATTACTGTTTGAGGATCG
AGGTCATGAGCAGATCGTTGGGTACTCAAATGCTGATTGGGCAGGATCACCTTCTGATA
GACGTTCTACGTCTGGATGTTGTGTTTTAGTAGGAGGAAATTTGGTGTCCTGGAAAAGC
AAGAAACAGAATGTAGTTGCTCGGTCTAGTGCAGAAGCAGAATATCGAGCAATGGCTAT
GGTAACATGTGAACTAGTCTGGACCAAACAATTGCTCAAGGAGTTGAAATTTGGTGAAA
TCGGTTAGATGGAACTTGTGGAACTTGTGTGCGATAATCAAGATGCCCTTCATATTGCA
TCAAATCTGGTGTTTCATGAGAGAACTAAACACATTGAGATTGATTGTCACTTCGTAAGA
GAGAAGATACTTTCAGGAGATATTACTACGAAGTTTGTGAGGTCGAATGATCAACTTGC
AGATATTTTCACCAAGTCCTTCACCGATCCTTGCATTGGTTATATATGTAACAAGCTCGG
TACATATGATTTGTATGCTCCGGCTTGAGGGGGAGTGTTAGATATAGATATGTAGTTGC
CCCACATTGGAATAGGTGTAGTATGCCCTTTGTATAGAGTAGCTATAAATAAGCTCATCT
TGTATTGCATTAGACACACAATATCAATATATCATATTTTCTCCCGTGCCTTCTCACATCA
AGTATCAAGAGAACAACATTCTACTTACGCTAATCTTATTACAGTCATAAAGACAGAGAC
AAAGGTAACACAGACGTGAAATAAAATACTTCCAGATAACCTTTCATTATGGACAATAAA
AGAAACATGTTCAGTCACGAAACTTTTTCCAAACTTAATTAAGGTAAGAGACGAAAGAAA
ACATCAATCAGTAAAGAAAAAGAAGGGGAAATTAAAAGGTGAAAAAGCAAAATGATATTA
GATATTTGCAAGATTTTTAATATCACATTAGTCCCCACTCATCAAATGATGGTAGAGGGC
 
ATTTCATAAGGGCCAATCAACAGCCACAACAATTCGATAAGTGCTCCAGGAAACAACTA
ACCTTTGATAAATCAATAGGAACATCAAGATAATGATCTAAGAAATTTGCATTCTGTTCT
GGATCAAGAAGCTCCAACATAGCACTTGCTGGATCACCAGCATGTCCTCTTCCCAACTG
CCATAGAAGAAAAAATACAAAGCTATTATTAAATTTGATCGATAATTATACACATTCTTTA
AACATTTAGATTGCAGAAGAAATCCAAGCAAAAACATAATATGTTAATGCCCAAGATTCT
TCCGGTGGTCATACACAGAAAAGCAAGATTTAAATAGTGGAAAAGAAGTTACACTTCAC
TCATAGTCAAAGCATATAATTGAAGTATGAACTCACAAACCATAAAATACCTTGTCAATTT
CATCGATCAAAACAAGAGGATTAGCGGTTCCCACACTTTTTAAACATTGCACCATCTTCC
CCGGCATGGCACCAATATAAGTTCGTCGATGTCCCTTGAAGTGTATCCCAAAGTTAGTC
ATGAAAGTTCAAGATTATGTGCACAACAAAACAACTCTGTTTTAGCTGGTTAGATATTTC
TGTTACCTTTATTTCAGCAACATCAGACAGCCCTCCAACAGAAAATCGGTAAAATTTGCG
GTTCAATGCACGTGCAATTGAACGACCTATACTGGTTTTGCCCACCCCAGGAGGGCCA
GAGAGGCATATGATTTTCCCTGTTCAAACAAAAATAAGCAATGAGTTTGTTTAGTTCCTG
ACTCAGATTCACAAATTAAACTGAGATAAATCAGATAAAGCCGCACATATGAAAGACAGT
GCCAGTGTATAATTGCGATTAATATCATCACTTGAATCTAACATTGTCCTAGCTATGGTG
CAATTCACATTTTTCTAGAGTTGCCCTTTGTCTGTTTTTTCCTCAACACTTCATAGTTTTC
AAAGATTTTCCTCAACAAACAACTACATCTCAAGTTCAAATGTCCCAAAGATATACAAGT
ACAACTGGCAGTGAAAGTAAGTTATCAATAAAATACCATAGAGCGAGTGCCCAATCATG
GACAACACAATATCAGAATTTAAGAAGAACACCAACAAAGTATGGCCCAAGGTGCAGCT
ATTTAACTCACATTAATTGCTTTTCAAGGGAAGAAATTAAGCTCAAGATATATTTCTTTAC
TCCACAGATAATCACAAGAAAACATTGGAGGAACTCTCGACTGTCACAAAAAGTATTAC
CTAAGTTTGACCAATAAATATTTCTCAAATGCCCTAAAATGTTCACTTCTTCCAATTTACT
CGTTCCAATTTGATGTCAATATAGTATGTGGGAACCATAAATCCCATGTTCTATGGATAT
ATTTTCCATTGGTATATTATAATGTTATGAAAATGGACGGTAAGGAGATGGCTGGATCAT
TTTCCGTTCTTTTAATATGATTATAGCTGAACCTTATAAAACTGAGGATTTATTAAAAAT
GAGGGTCATTATTTTTAAAAAATAAGGCATTTACCAACCTTGTGAGGTTCCTCTGAGTTT
TCCCACAGCTATAAATTCCAAGATCCTTTCCTTAACATCGGTTAACCCATAGTGGTCTTC
ATCAAGAATTTGTTCTGCCCGTAGTACATCAAAGTTTTCATCACTGCATTTTGGATCATA
AAAGAAGAAAAGTTCAGGAACCAATGCAATACACCCAGGCAGAGATCAAAACTATAAAG
CCAACACCACCACCATCAGCAGCAAACAACACGAACTATGACAAATCAAGCCCCCATCT
TAACCTACATAAGGAGCTCATCATAAGTACAATCCTAGTTTTGCTGCTCTCATGAGATCC
TGGTCTAAACTGATACATTGGGATGTCAAGGAAGCATCCTCAAAGCCAGAAGCATGACA
TGTAAGAACCCAAAATAAGGAACAGGAGGAGAAAGAAAAGTTAAAGACAAATGGATATT
ACTCGATAGAAATTTAGGCAAGTCACATCACTAGGCAGTAAGGTTAACTTGGAAGCCTA
GCAAATTTCAGACAATAACAATTTTATTTCTCATAACTGTTTAGCCAGCTTCTAACAAACA
GATTCCTCTAGTACAGCTAACCAAGGATAAGGTTAACATCAGTTGGATTGAACCCAAGA
CAATGTAAGGTAGAATAGATAGACAACAAACCTGTAACTACCCCATGGCAAGGCAGTCA
ACCAATCAAGATAATTACGTGTCACGTTAAATTCACTGGAACTAGCTTCCAACAGTTGCA
GTTTTGTCAGTTCTTCTTCAATAACTTGCATAACATGTACTGGTATTTTTTCTTTATTAGG
CTCCAATCTTTCCCTGAACTTTGCTGCAGTAATAAACAAATATAGTGTCACACCATTAGA
TGTAATTAAAAAGGAACAAAAAACTGCAGGTTTCCCTTAGTATTGACCAGAAAAATCCA
AGCCCTTAAAGCGAACCTAATCATTAGGCAGTTTCTTCTCTCAATTTCAACACCAAAACC
AGGTCTACCAAAACAATGCTACAATGCGGAGCTCAACTTTATCTTTGTGAAAATTATAGA
GAAAAAATAATGAGAACCGCATTTTAGGGTTTGCAGTAACGTCTATTATCACCAAAACCT
GGAAGAAGCACCTTCTTAAGCATCACACTCCTCAACAGGAGCAAAGGAGAGAAGAAAG
AGCACATAACAGAAAAAGCAAGTAGGCTTCTAATTAGGAAGTGCACTGAAGGAAACAG
GACAGAATTCATGAGAAAAAGAAGACATTGGAGGAGGAAGATAATCTGAATCATGAGCA
GTCCATAACATGAATAAACAACAATGCAGTGCTAAAACGGAAAATGTGGCCTTCCAGTA
AATCCATGTTGAATTTGTGCCACATTACCAAATATGGTAGACAGTAACCTTTCGGAATTG
AAGTTGAAGGATTCCAGTATTGTCTTATTAAGTGCTTTCGTGAAAACACAAAACAGCCCC
CTATAGGTACAATGTATTCTAATCTGTCAAAAGTTTGGAATGTCTCAAATAGTTTTAGAAA
 
GCATGTCAATAAAGTTGGTTGGACTGTGTACAAAGAAAATTCAACCTTCAATTTCCTATA
TGTAAAGCCAATTATCGCTTGAATGCTATTCATGCTTCTAGTGAAGATTTTCAATGGTAA
ACAGGAGATCAATCAGCCACCATAAAACTTTTACAACAGAAAGGGCAAAACATATTGCA
AATGGCCTTTTCTATGCTACTGCTAAATGGAACTTTACCAGGGACAACATAAGATGTTTC
ACTGGCCAGATGGGAAAACACGTCTGGCCATAATAAATCCAAAAAGAACATTATCCATT
TCAATAATAATTAAGAGACAACTAACCAGAAAGAGCTGTCTTGTCATCAGTCTCCAAACC
TAGTTCCTGCATTAAGCCAATCAAAACCACGTCATAAGTACTTTTTTTTCAAATCAACTCT
TTGTTTAAACTGTGAATCAAACATAAGAACCAGAAGCTTAACATATATATTATCTAGGAAT
AAACATATGTAAGTACTAAGTTTAAGGATGATAACACAAAAAAAGCTGCACATAATCCAC
ATGCCTTCTTTATGGCCTTTAATTGTTCATTCAACAAATAACGGCGTTGCTCTCCGCTTA
TTTTTTCTTCAATTGCTCTTGCTATTGATTCCTGCAGAAAACAAAGACGACGTAAAATGC
TAAATGCATGCATAACAACTATTCAAAGTTCTGGTATGCGTCTTGACGTGCATTTACCTG
AATCTTACTAATCTCCAT
**SEQ 81 to 160 are putative protein SEQ related to SEQ 1-80**
SEQ 81 
MALRFSLIFLFSLFLTTSLLLSVNGNINGGEDDDILIRQVVGDDDDHLLNADHHFTIFKRRFGKTYA
SDEEHHYRFSVFKANLRRAMRHQKLDPSAVHGVTQFSDLTPAEFRRNFLGVNRRLRLPSDANK
APILPTEDLPSGFDWRDHGAVTSVKNQGSCGSCWSFSTTGALEGATYLSTGKLVSLSEQQLVD
CDHECDPEEKDSCDAGCNGGLMNSAFEYTLKAGGLMREEDYPYTGTDRGTCKFDNTKVAAKV
ANFSVVSLDEEQIAANLVKNGPLAVAINAVFMQTYVGGVSCPYICSKKLDHGVLLVGYGTGFSPI
RMKEKPYWIIKNSWGEKWGENGYYKICRGRNVCGVDSMVSTVSAVSTSSH
SEQ 82 
MGAKVFLVALFLSALLFPLASSSNDGLMRIGLKKMKFDQNNRLAARIESKEGDVLRASIRKYNFR
GKLGDSEDTDIVALKNYMDAQYFGEIGVGTPPQKFTVIFDTGSSNLWVPSSKCYFSVPCFFHSK
FKSSESSTYKKNGKSAAIQYGSGAISGFFSQDNVKVGDLVVTDQEFIEATREPSVTFLVAKFDGIL
GLGFQEISVGNAVPVWYNMVQQGLIKDPVFSFWLNRNTEEEQGGEIVFGGVDPNHYKGEITYV
PVTHKGYWQFDMGDVLIEGKATGYCESGCSAIADSGTSLLAGPTTIITMINQAIGASGVASQQCK
SVVEQYGQTIMDLLLAEAHPKKICSQVGVCTFDGNRGVSMGIESVVDEKAGRSTGLQDGMCSA
CEMAVIWMENQLRQNQTQDRILNYVNELCERLPSPLGESAVDCGKLSSMPTVSFTIGGKVFDLV
PKEYILKVGEGAKAQCISGFTGLDIPPPRGPLWILGDVFMGRYHTVFDYGKLRVGFAEAA
SEQ 83 
MGSFLCFSVIVVLLVLQPCLAKKVYIVHMKNHQIPSSFATHHDWYNAQLQSLSSSSTSDESSLLY
SYDTAYSGFAASLDPHEAELLRQSDDVVGVYEDTVYTLHTTRTPEFLGLNNELGLWAGHSPQEL
NNAAQDVVIGVLDTGVWPESKSYNDFGMPDVPSRWKGECESGSDFDPKVHCNKKLIGARFFS
KGYQMSASGSFTNQPRQPESPRDQDGHGTHTSSTAAGAPVANASLLGYASGVARGMAPRAR
VATYKVCWPTGCFGSDILAGMERAILDGVDVLSLSLGGGSGPYYRDTIAIGAFSAMEKGIVVSCS
AGNSGPAKGSLANTAPWIMTVGAGTIDRDFPAFATLGNGKKITGVSLYSGKGMGKKVVPLVYST
DSSASLCLPGSLDPKMVRGKIVLCDRGTNARVEKGLVVKEAGGVGMILANTAESGEELVADSHL
LPAVAVGRKLGDFIRQYVKSEKNPAAVLSFGGTVVNVKPSPVVAAFSSRGPNTVTPQILKPDVIG
PGVNILAAWSEAIGPTGLEKDTRRTKFNIMSGTSMSCPHISGLAALLKAAHPEWSPSAIKSALMT
TAYVRDTTNSPLRDAEGGQLSTPWAHGSGHVDPHKALSPGLIYDITPEDYIKFLCSLDYELNHIQ
AIVKRPNVTCTKKFADPGQINYPSFSVLFGKSRVVRYTRAVINVGAAGSVYEVTVDAPPSVTVTV
KPSKLVFKRVGERLRYTVTFVSKKGVNMMRKSAFGSISWNNAQNQVRSPVSYSWSQLLD
SEQ 84 
MGTKFILFILLFIFLFSSGFVACGGFYSFRNLNSSVSGIEFPNHPSFNAVSSSADSDCNYGVSQKS
KTHSIAQEVDGVDVKNGENEEVSIFGNQKKEAVKFQLRHRSAGKKIEAKDSVFESRARDLSRIQT
LHTRIVEKKNQNYNSRLAKSNEKHVDKHKPVIAPAAVSLESYELSGKLMATLESGVSLGSGEYF
MDVFVGTPPKHFSLILDTGSDLNWIQCVPCFDCFEQNGPHYNPQDSTSFRNISCHDPRCKFVTS
PDPPQLCKSENQTCPYYYWYGDSSNTTGDFALETFTVNLTTTSGSEFRKVENVMFGCGHWNR
GLFHGAAGLLGLGRGPLSFASQLQSLYGHSFSYCLVDRNSNSSVSSKLIFGEDKELLKHPQLNF
TSLVGGKEVETFYYVQIKSVIVGGEVLNIPEETWNLSLEGLGGAIIDSGTTLSYFADPAYEIIKEAF
 
VNKVKGYPIVQDFPILNPCYNVSGVKNLEFPSFGIVFGDGAVWNFPVENYFIKLEPEDIVCLAVLG
TPRSALSIIGNYQQQNFHILYDTKRSRLGYAPTRCADA
SEQ 85 
MALTLKSLATPLLFGALFILILQVVAEQPISEAKVESAILQESIIKEVNENAKAGWKAAFNPRFSNFT
VSQFKRLLGVKPAREGDLEGIPILTHPKLLELPKEFDARKAWPQCSTIGRILDQGHCGSCWAFGA
VESLSDRFCIHHNLNISLSVNDLLACCGFLCGSGCDGGYPITAWRYFIRRGVVTEECDPYFDNE
GCSHPGCEPGYPTPKCQRKCVKEILLWGKSKHYGVNAYRIHHDPNSIMTEIYKNGPVEVSFTVY
EDFAHYKSGVYKHVTGQSMGGHAVKLIGWGTSEQGEDYWLIANSWNRGWGDDGYFKIRRGT
NECGIEHNVVAGLPSAKNLNVELDDVSNAFLDASM
SEQ 86 
TLVLHTSFYLLLSVASPGDCLLLSIFPFSFSSPRYFPYKQNTVKIISSNFLFSPFFQMGSFLCFSVIV
LFLVFQPCFSKKVYIVHMKNHQIPSSFATHHDWYNAQLQSLSSSSTSDESSLLYSYDTAYSGFA
ASLDPHEAELLRQSDDVVGVYEDTVYTLHTTRTPEFLGLNNELGLWAGHSPQELNNAAQDVVIG
VLDTGVWPESKSFNDFGMPNVPSRWKGECESGPDFDPKVHCNKKLIGARFFSKGYQMSASGS
FTNQPRQPESPRDQDGHGTHTSSTAAGAPVANASLLGYASGVARGMAPRARVATYKVCWPTG
CFGSDILAGMERAILDGVDVLSLSLGGGSGPYYHDTIAIGAFSAMEKGIVVSCSAGNSGPAKASL
ANTAPWIMTVGAGTIDRDFPAFATLGNGKKITGVSLYSGKGMGKKVVPLVYSTDSSASLCLPGS
LDPKIVRGKIVLCDRGTNARVEKGLVVKEAGGVGMILANTAESGEELVADSHLLPAVAVGRKLG
DFIRQYVKSEKNPAAVLSFGGTVVNVKPSPVVAAFSSRGPNTVTPQILKPDVIGPGVNILAAWSE
AIGPTGLEKDTRRTKFNIMSGTSMSCPHISGLAALLKAAHPEWSPSAIKSALMTTAYVHDTTNSP
LRDAEGGQLSTPFAHGSGHVDPHKALSPGLIYDITPEDYIKFLCSLDYELNHIQAIVKRPNVTCAK
KFADPGQINYPSFSVLFGKSRVVRYTRAVTNVAAAGSVYEVVVDAPPSVLVTVKPSKLVFKRVG
ERLRYTVTFVSNKGVNMMRKSAFGSISWNNAQNQVRSPVSYSWSQLLD
SEQ 87 
MASSCLHAILLCFLLFITSTTAQNQTSFRPKGLILPITKDASTLQYLTQIHQRTPLVPVSLTDLGGQ
FLWLDCDQGYVSSSYKPARCRSAQCSLAGAGSGCGQCFSPPKPGCNNNTCSLLPDNTITRTAT
SGELASDTVQVQSSNGKNPGRNVTDKDFLFVCGATFLLEGLASGVKGMAGLGRTIISLPSQFSA
EFSFPRKFAVCLSSSTNSKGVVLFGDGPYSFLPNREFSNNDFSYTPLFINPVSTASAFSSGEPSS
EYFIGVKSIKINQKVVPINTTLLSIDNQGVGGTKISTVNPYTILETSIYNAVTNFFVKELVNITRVASV
APFGACFDSRNIVSTRVGPAVPSIDLVLQNENVFWRIFGANSMVQVSENVLCLGFVDGGVNPRT
SIVIGGYTIENNLLQFDLAGSRLGFTSSILSRLTTCANFNFTSIT
SEQ 88 
MNPEKFTHKTNEALAGAHELALSAGHAQFTPLHMAVALISDHNGIFRQAIVNAGGNEEVANSVE
RVLNQAMKKLPSQTPAPDEIPPSTSLIKVLRRAQSSQKSCGDSHLAVDQLILGLLEDSQIGDLLKE
AGVSASRVKSEVEKLRGKEGRKVESASGDTTFQALKTYGRDLVEQAGKLDPVIGRDEEIRRVVR
ILSRRTKNNPVLIGEPGVGKTAVVEGLAQRIVRGDVPSNLADVRLIALDMGALVAGAKYRGEFEE
RLKAVLKEVEEAEGKVILFIDEIHLVLGAGRTEGSMDAANLFKPMLARGQLRCIGATTLEEYRKYV
EKDAAFERRFQQVYVAEPSVTDTISILRGLKERYEGHHGVKIQDRALVVAAQLSSRYITGRHLPD
KAIDLVDEACANVRVQLDSQPEEIDNLERKRIQLEVELHALEKEKDKASKARLVEVRKELDDLRD
KLQPLMMRYKKEKERIDELRRLKQKRDELIYALQEAERRYDLARAADLRYGAIQEVETAIANLES
TSAESTMLTETVGPDQIAEVVSRWTGIPVSRLGQNEKEKLIGLGDRLHQRVVGQDHAVRAVAEA
VLRSRAGLGRPQQPTGSFLFLGPTGVGKTELAKALAEQLFDDDKLMIRIDMSEYMEQHSVARLI
GAPPGYVGHDEGGQLTEAVRRRPYSVVLFDEVEKAHPTVFNTLLQVLDDGRLTDGQGRTVDFT
NTVIIMTSNLGAEYLLSGLMGKCTMETAREMVMQEVRKQFKPELLNRLDEIVVFDPLSHEQLRQ
VCRYQMKDVALRLAERGIALGVTEAALDVILSESYDPVYGARPIRRWLERKVVTELSKMLVKEEI
DENSTVYIDAGVGRKDLTYRVEKNGGLVNAATGQKSDILIQLPNGPRSDAVQAVKKMRIEEIEED
EMED
SEQ 89 
MQSFKSASILRRLLQNSRLVSHSRSFCSVSTNALVDESQSTVLVEGKASSRTAILNRPHALNALN
FSVVDRLLKLYKNWEDDPDIGFVVLKGSGKAFSAGGDIVTIYNLLKQDAGNLQDCKDFCWTINNL
VYVVGTLLKPHVALLNGITMGGGAGISIPGTFRVATEKTVFATPETLIGYHPDAGASFYLSHLPGY
LGEYLALTGDKINGAEMISCGLATHYLHSAKLPLIEEQLGKLMTDDPSVIERSLENCGEIVHPDPT
 
SVLHRIETLNKCFSHDTVEEIIDALESEAAKKQDAWCVSTLRKLQETAPLSLKVSLRSIREGRHQT
LDQCLIREYRMSVQAFSGQITNDFCEGVRARLVDRDFAPKWDPPSLDKVTDDMVDQYFSRLTA
FEPELELPTQQREAFT
SEQ 90 
MALTLKSLATPLLLGAFFILVLQVVAEKPISEAKVESAILKESIIKEVNENAKAGWKAAFNPQFSNF
TVSQFKRLLGVKPAREGDLEGIPLLTHPKLSELPKEFDARKAWPQCSTIGRILDQGHCGSCWAF
GAVESLSDRFCIHHNLNISLSVNDLLACCGFLCGSGCDGGYPISAWRYFIRRGVVTEECDPYFD
NEGCSHPGCEPGYPTPKCQRKCVKENLLWGKSKHYGVNAYRIHRDPYSIMTEIYKNGPVEVSF
TVYEDFAHYKSGVYKHVTGQSMGGHAVKLIGWGTSEQGEDYWLIANSWNRGWGDDGYFKIRR
GTNECGIEHNVVAGLPSAKNLNVELDDVSDAFLDASM
SEQ 91 
MGVLKKTLLLLFLCVFLGDISLCFSSKLYVVYMGSKDSDEHPDEILRQNHQMLTAIHKGSIEQAKT
SHVYSYRHGFKGFAAKLTEAQASEISKMPGVVSVFPNTKRSLHTTHSWDFMGLSDDETMEIPG
FSTKNQINVIIGFIDTGIWPESPSFSDTNMPPVPAGWKGQCQSGEAFNASICNRKIIGARYYMSG
YEAEEENGKTMFYKSARDSSGHGSHTASTAAGRYVANMNYKGLANGGARGGAPMARIAVYKT
CWSSGCYDVDLLAAFDDAIRDGVHVISLSLGPDAPQGDYFNDAISVGSYHAVSRGILVVASVGN
EGSTGSATNLAPWMITVAASSTDRDFTSDILLGNGVRLKGESLSLSQMNTSTRIIPASEAYAGYF
TPYQSSYCLDSSLNRTKAKGKVLVCLHAGSSSESKMEKSIIVKEAGGVGMILIDDADKGVAIPFVI
PAATVGKKIGNKILAYINNTRLPMARILSARTVLGAQPAPRVAAFSSRGPNSVTPEILKPDIAAPGL
NILAAWSPAASTKLNFNVLSGTSMACPHITGVVALLKAVHPSWSPSAIKSAIMTTAKLSDKHHKPII
VDPEGKRATPFDFGSGFVNPTNVLDPGLIYDAQPADYRAFLCSIGYDEKSLHLITRDNSTCDQTF
ASPNGLNYPSITIPNLRSTYSVTRTVTNVGKARSIYKAVVYAPTGVNVTVVPRRLAFTRYYQKMN
FTVNFKVAAPTQGYVFGSLTWRNKRTSVTSPLVVRVAHSNMGMMV
SEQ 92 
MGAKAFLVAMFLSALLFPFASSSNDGLMRIGLKKMKFDQNNRLAARIESKEGDVLRGSIRKYNF
RGKLGDFEDTDIVALKNYMDAQYFGEIGVGTPPQKFTVIFDTGSSNLWVPSSKCYFSVPCFFHS
KYKSSESSTYKKNGKSAAIQYGSGAISGFFSQDNVKVGDLVVTDQEFIEATREPSVTFLVAKFDG
ILGLGFQEISVGNAVPVWYNMVKQGLIKDPVFSFWLNRNTEEEQGGEIVFGGVDPNHYKGEITY
VPVTQKGYWQFDMGDVLIDGKATGYCESGCSAIADSGTSLLAGPTAIITMINQAIGASGVASQQC
KSVVEQYGQTIMDLLLAEAHPKKICSQVGVCTFDGNRGVSMGIDSVVDEKAGRSTGLQDGMCS
ACEMAVIWMANQLRQNQTQDRILNYVNELCERLPSPLGESAVDCGKLSSMPKVSFTIGGKVFDL
SPNEYILKVGEGAKAQCISGFTGLDIPPPRGPLWILGDIFMGRYHTVFDYGKLRVGFAEAA
SEQ 93 
MTFFRSFLFFLLTLFVISSALDMSIISYDEQHGQMGTTHHRTDDEVRELYESWLVKHGKNYNAIG
EKERRFEIFNDNLRFIDEHNAENRSYKLGLNRFSDLTNEEYRAMFVGGRLDRKTRLMKSPKSNR
YAFQAGEKLPESVDWREKGAVAPVKDQGQCGSCWAFSTVGAVEGINKIVTGELISLSEQELVD
CDRSYNQGCNGGLMDYAFDFIKNNGGIDTEDDYPYHAQDGTCDPYRKNARVVSIEGYEDVPEN
DEKSLMKAVANQPVSVAIEGGGRAFQHYSSGVFTGYCGTQLDHGVVVVGYGTENGEDYWIVR
NSWGANWGESGYIKLQRNFANSTTGKCGIAMQASYPLKSGANPPNPGPSPPTPVTPSTVCDEY
YSCPQGTTCCCIYQYGEYCFGWGCCPYESATCCDDNYSCCPHDYPVCDVDAGTCLMSKDNPL
KVKALKRGPARVNWSGMKSNRKVSYV
SEQ 94 
MANSYTSFNFFLAPIIFLAILGLQLQSSDGFGTFGFDIHHRYSDPVKGILDLHGLPEKGSVEYYSA
WTQRDRFIKGRRLADTTNPTPLSFSGGNETFRLSSLGFLHYANVTVGTPGLSFLVALDTGSDLF
WLPCDCSNCVRALETRSGRRINLNIYSPNTSSTGQIVPCNGTLCGQRRRCLSSQNACAYGVAYL
SNNTSSSGVLVEDILHLETDNAQQKSVEAPIALGCGIRQTGAFLSGAAPNGLFGLGLESISVPSM
LASKGLAANSFSMCFGPDGIGRIVFGDKGSPDQGETPLNLDQLHPTYNISLTGITVGNKITDVDFT
AIFDSGTSFTYLNDPAYKVITENFDSQAKQLRIQPDGEIPFEYCYGLSANQTTFEVPDLNLTMKG
GNQFFLFDPIIMLSLQDGSRAFCLAVVKSGDVNIIGQNFMTGYRVVFDREKMVLGWKPSDCYDS
RESNDKSTTLPVNKRNSTEAPSPSSVVPEATKGNGSGNEPATSFPSVPSSRPAINHAPAHFNSY
ICQLMMALFSLFSYYLIIVSS
SEQ 95 
MVTKFSIFILVVLLRLFSFGSVASREIHNSGLNLNSSASGIEFPQHPSFNSVTASGNSDCSYGTSK
KSTTTHVITQEENRSDEKEDEDLMVSKNQPREAVKFHLRHRSAGQNIEAKDSIFESTTRDLGRIQ
TLHTRIVEKKNQNSISRQTKNSEKPTQSSSFEFSGKLMATLESGVSHGSGEYFMDVFVGTPPKH
FSLILDTGSDLNWIQSVPCYDCFEQNGPHYDPKDSISFKNISCHDPRCHLVSSPDPPQPCKSEN
QTCPYYYWYGDSSNTTGDFALETFTVNLTTPSGDSEIKKVENVMFGCGHWNRGLFHGAAGLLG
LGRGPLSFSSQLQSLYGHSFSYCLVNRNSNSSVSSKLIFGEDKELLKHANLNFTSLVGGKENHL
ETFYYVQIKSVIAGGEVLNIPEETWNLSTEGVGGTIIDSGTTLSYFAEPAYEIIKQAFVNKVKHYPV
LEDFPILKPCYNVSGVEKLELPSFGIVFGDGAIWNFPVENYFIKLEPEDIVCLAMLGTPHSAMSIIG
NYQQQNFHILYDTKRSRLGFAPTRCADA
SEQ 96 
MPSSFSLLFLTLLLASISLSFSSTLNSNDDDFFLSSTPKFPLTMAEKLIRQLNLFPKHDINKAAATG
DSAAVTEQRLFEKKLNLSYVGNSGSTVQDLGHHAGYYRLPHTKDARMFYFFFESRSRKNDPVVI
WLTGGPGCSSELAVFYENGPFKIADNMSLVWNDFGWDKVSNLIYVDQPTGTGFSYSSNDDDIR
HDERGVSNDLYDFLQAFFKAHPQYAKNDFYITGESYAGHYIPAFASRVHQGNKNKEGIYVNLKG
FAIGNGLTDPEIQYKAYTDYALDMKLIKKSDYNAIEKSYPKCQLAIKLCGKDGGTACMAAYLVCTS
IFNKIMDIAGDKNYYDVRKRCEGDLCYDFSKMETFLNDQQVKKALGVGDIEFVSCSSEVYQAMQ
LDWMRNLELGIPSLLEDGIKLLVYAGEYDLICNWLGNSRWVHAMKWTGQKAFGKATQVSFAVD
GVEKGVQKNYGPLTFLKVHDAGHMVPMDQPKAAMEMLQRWMQDKLSKEGHLAPM
SEQ 97 
MTLTLKSLAAPLFLGAFCILILQVVAEKPISEAKVESAILQESIIKEVNENAKAGWKAAFNPRFSNFT
VSQFKRLLGVKPAREGDLEGIPILTHPKLLELPKEFDARKAWPQCSTIGRILDQGHCGSCWAFGA
VESLSDRFCIHHNLNISLSVNDLLACCGFLRGSGCDGGYPISAWRYFIRRGVVTEECDPYFDNE
GFHTRVVNQDIPPQSVV
SEQ 98 
MFRLVMVTKFSIFILVVLLRLFSFGFVASREIHNFGINLNFSASGIEFPQHPSFNSVTASGNSDCSY
GTSKKSTTTHVITQEENNSDEKEDEDLMVSENQPREAVKFHLRHRSAGQNIEAKDSIFESTTRDL
GRIQTLHTRIVEKKNQNFISRQTKNSEKTTQSSSFEFSGKLMATLESGVSHGSGEYFMDVFVGT
PPKHFSLILDTGSDLNWIQSVPCYDCFEQNGPHYDPKDSISFKNISCDDPRCHLVSSPDPPQPC
KSENQTCPYYYWYGDSSNTTGDFALETFTVNLTTPNGDSEIKKVENVMFGCGHWNRGLFHGA
AGLLGLGRGPLSFSSQLQSLYGHSFSYCLVNRNSNSSVSSKLIFGEDKELLKHLNLNFTSLVGGK
ENHLETFYYVQIKSVIVGGEVLNIPEETWNLSTEGVGGTIIDSGTTLSYFAEPAYEIIKQAFVNKVK
RYPILDDFPILKPCYNVSGVEKLELPSFGIVFGDGAIWTFPVENYFIKLEPEDIVCLAILGTPHSAM
SIIGNYQQQNFHILYDTKRSRLGFAPRRCADA
SEQ 99 
MSGFRLPLLFHLLLPLTLFLQYVQSLPQNSSTVEFLPGFDGPLPFYLETGYIGVGKSEEVQLFYYF
VKSESNPKKDPLLLWLTGGPGCSSFTGVAYEVGPLAFGQKAYNGSLPILVSTPYSWTKFASILFL
EQPVNTGFSYATTSAASKCTDLQACDQVYEFLLKWFNNHPEFISNPFYVSGDSYSGITVPVIVQL
ISDGIEAGKKPLINLKGYSLGNPLTFPEESNYQIPFCHGMGLISNELYESLKETCKGDCRNIDPTN
KLCLENFKMFKKLVSSINDQQILEPFCGTDSESPNPRQLSGERRSLEEDFIFLKHDDFICRESRVA
TRKLSNHWANDPSVQEALHVRKGTIRRAWARCRQSIMGTTYRVTFMNSIPYHVNLSSKGYRSLI
YSGDHDMVVPFQSTQAWIKYLNYSIIDDWRPWTIDGQVAGYTRSFSNHMTYATVKGGGHTAPE
YKREESFHMFKRWIAQQPL
SEQ 100 
METNGLIKEILPRDAVNNMTRLILSNALYFKGEWNEKFDVSETKDHDFHLLNGGSIQAPFMTSKK
KQYIAAFDCFKILRLPYKQGTDTRRFCMYFILPDAHDGLPALLEKISLEPGFLNNHVPYGKVRARK
FLIPKFKITFGFEASNILKGLGLTLPFCGGSLTEMVDSPMPQNLSVSQVFHKSFIEVNEEGTEAAA
VTATVIMTMSLIIEKEMDFVADHPFLFLIRDESTGAVLFIGSVMNPLAG
SEQ 101 
MNESYGNSRASSSSTTSSLNSSSHGTEDDHTIARILAEEEENALKYGGNKLGRRLSHLDSIPHTP
RVIGEIPDPNDATLDHGRLSSRLATYGLAEMQIEGDGNCQFRALSDQLYHNPEYHKHVRKEVVK
 
QLKRFRKLYEGYVPMRYKSYLRKMKRLGEWGDHVTLQAAADRFGVKICLVTSFRDNGYIDILPK
DIQPSRELWLSFWSEVHYNSLYEIGEVPARVRRKKHWLFF
SEQ 102 
MSWLCPSLVLVLLIFQGPICTCSSISDLFESWCQQNGKTYSSEQERVYRLEVFEENYAYIIEHNS
KGNSTYTLNLNAFSDLTHHEFKNSFLGLSSSANDFIRLKTGSSSAGVFNDVGVVDIPSSLDWREK
GAVTKVKNQGSCGACWSFSATGAIEGINKIVTGSLVSLSEQELIDCDKSYNDGCGGGLMDYAFE
FVKKNGGIDTEEDYPFNEREGTCNKNKLQRRVVTIDGYTDVPQYDEDKLLKAVANQPVSVGICG
SERAFQSYSKGIFTGPCSTVLDHAVLIVGYGSENGVDYWIIKNSWGTSWGINGYMHMQRNSGN
QEGICGINKLASYPTKSSPNPPSPPSPGPSKCSMFTSCGQGETCCCGWRLLGVCVSWKCCGL
DSAVCCKDGRHCCPHDYPICDTSRNLCLKRMSNATIVQQPQKEAFSGKFGGLIYPF
SEQ 103 
MCEPESEAARGVLSFLDVDQLFSSNYYGDGRKHDVEICHEQYARENHYHTSYCNVDNDEAIAH
VLQEDLSELSIAEDAESSHADEQYLQASTGVQHWHTPPREYYAGHDTSLEADDVGPSSSCSSP
GDRSYDGEEYTYTLEIQDEFELDGEVGKRINQLSAVPHVPRINGDIPSVDEATSDHQRLLNRLQL
FDLVEHKVQGDGNCQFRALSDQFYRTPEHHKFVRQQVVSQFQHHPEMYEGYVPMEYGEYLTR
MSKSGEWGDHVTLQAAADSYGVKILVITSFKDTCYIEILPKNQKSNRVIYLSFWAEVHYNSIYPQ
GDFLPFDFKKKKKKWSFWNKH
SEQ 104 
MPSLLQIFLPLFPFFFLVSFSVSHGPFLPKAIILPVNKDLSTFQYVTQVYMGAHLVPTNLVVDLGG
SFLWTNCGLTSVSSSQKLVPCNSLKCSMAKPNGCTNKICGVQSENPFTKVAATGELAEDMFAV
EFIDELKTGSIASIHEFLFSCASTTLLQGLARGAKGMLGLGNSRIALPSQLSDTFGFQRKFALCLS
SSNGAIISGESPYLSLLGHDVSRSMLYTPLISSKDGVSEEYYINVKSIKINGKKLSLNTSLFAMDEG
VGGTKISTIPPFTTMKSSIYKSFIEAYEKFAISMELNKVEAIAPFELCFSTKGIDVTKVGPNVPTTDL
VLQSEMVKWRIYGRNSMVKVSDEVMCLGFLNGGVNQKASIVIGGYQLEDNLLEFNLGTSMLGF
TSSLSMAETSCSDFMFHSVSKDSAFDS
SEQ 105 
MGAKEVLILVLVCMFIVFPSCHGDDECLNPFLVDQNCYVKDYITKLANATETVKWMMKIRRQIHE
NPELAYEEFKTSGLIREELDRMGVKYRWPVAKTGVVATIGSGKPPFVALRADMDALPIQELAKW
EHKSKVDGKMHACAHDAHTAMLLGAAKILQQLRHNLQGTVVLIFQPAEERGHGAKDMIEEGVLE
NVEAIFGMHLVHKYESGVVASRPGEFLAGCGSFKATIRGKGGHAAVPHDSVDPILAASTSVISLQ
SIVSRETDPLESQVVSVAMIEGGHAFNIIPELATISGTYRAFSKKSFYGLRKRIEEVIRAQAAVHRC
TVEIDFDGRENPTLPPTINDERIYEHARKVSKMIVGEESFKIAPSFMGSEDFAVFLEKVPGSFFLL
GTKNEKIGAIYPPHNPHFIIDEDVLPIGAAIHATFAYSYLLNSTNKFTSHSS
SEQ 106 
MKLNPYSWTKVASIIFLDLPVGTGFSYARTPTALQSSDLQASDQAYEFLYKWFLDHPEFLKNPLY
VGGDSYSGMVVPIITQIIATKNEMGIKPFVDLQGYLLGNPSTFKGEKNYEIPFAYGMGLISDELYE
SLTRNCKGEYQNTDPSNTQCLQDVHTFQELLKRINNPHILEPKCQFASPKPHLLFGQRRSLNVK
FHQLNNPQQLPALKCRNDWYKLSSHWADDGQVREALHIRKGTIGKWVRCASLQYQKTIMSSIP
YHANLSAKGYRSLIYSGDHDKVVTFLSTQAWIKSLNYSIVDDWRPWIVDNQVAGYTRSYSNRMT
FATVKGAGHTAPEYKPRECLAMLKRLMSYKPL
SEQ 107 
MCEPESEATRGVLSFLDVDQLFSSNYYGDGRKHDVEICHEQYARENQYHTSYCNVDSDEAIAH
LLQEELSELSIAEDAESSHADEQYFQASTGVQHWHTPPREYYAGHDTGLEADDVGPSSSCSSP
GDRSYDGEEYTYTLEIQDEFELDGEVGKRINQLSAVPHVPRINGDIPSVDEATSDHQRLLDRLQL
FDLVEHKVQGDGNCQFRALSDQFYRTPEHHKFVRQQVVSQLKHHPEMYEGYVPMEYGEYLKR
MSKSGEWGDHVTLQAAADSYGVKILVITSFKDTCYIEILPKNQKSNRVIYLSFWAEVHYNSIYPQ
GDFLPFDLKKKKKKWSFWNKH
SEQ 108 
MPSLLQIFLPLFPFFFFVSFSVSHGPFLPKAIILPVNKDLSTFQYVTQVYMGAHLVPTNLVVDLGG
SFLWTNCGLTSVSSSQKLVPCNSLKCSMAKPNGCTNKICGVQSENPFTKVAATGELAEDMFAV
EFIDELKTGSIASIHEFLFSCASTTLLQGLARGAKGMLGLGNSRIALPSQLSDTFGFQRKFALCLS
SSNGAIISGESPYLSLLGHDVSRSMLYTPLISSKNGVSEEYYINVKSIKINGNKLSLNISLFTMDEE
GVGGTKISTISPFTSMKSSIYRTFMEAYEKIAISVNLTKVESIAPFELCFSTEGIDVTKVGPNVPTM
DLVLQSEMVKWRIYGRNSMVKVSDEVMCWGFLDGGVNQKASIVIGGYQLENNLLEFNLGTSML
GFTSSLSTAETSCSDFMIHSVSKDSAFDS
SEQ 109 
MKMSPALSLSVIQFPLCKSQDLSKDTNNPKIFSKETPCQKSYSDTRINRRKLLSGSGLSLVAGTL
AKPARAETEAPIEATSSRMSYSRFLEYLNEGAVKKVDFFESSAVAEIFNPALNKVQRVKVQLPGL
PPELVRKLREKDVDFAAHLPEMNVIGPLLDLLGNLAFPLILLGSLLLRTSSSNTPGGPNLPFGLGR
SKAKFQMEPNTGVTFDDVAGVDDAKQDFQEIVEFLKTPEKFAAVGAKIPKGVLLVGPPGTGKTL
LAKAIAGEAEVPFLSLSGSEFVEMFVGVGASRVRDLFNKAKENSPCLVFIDEIDAVGRQRGTGIG
GGNDEREQTLNQLLTEMDGFTGNTGVIVIAATNRPEILDQALLRPGRFDRQVSVGLPDIRGREEI
LKVHSNNKKLDKDVSLSVIAMRTPGFSGADLANLMNEAAILAGRRGKDKITSKEIDDSIDRIVAGM
EGTKMTDGKNKILVAYHEVGHGVCATLTPGHDAVQKVTLIPRGQARGLTWFIPGEDPTLISKQQ
LFARIVGSLGGRAAEEIIFGEAEITTGAAGDLQQITQIARQMVTMFGMSEIGPWALTDPATQSGD
VVLRMLARNQMSEKLAEDIDASVRHIIERAYEIAKNHIRNNREAIDKLVDVLLEKETLTGDEFRAIL
SEFTNIPSANINSKPIRELIEA
SEQ 110 
MEGLHQLKTGELIPLSEQELVDCDVEGEDEGCSGGLLDTAFDFILKNKGLTTEVNYPYKGEDGV
CNKKKSALSAAKITGYEDVPANSEKALLQAVANQPVSVAIDGSSFDFQFYSSGVFSGSCSTWLN
HAVTAVGYGATTDGTKYWIIKNSWGSKWGDSGYMRIKRDVHEKEGLCGLAMDASYPTA
SEQ 111 
MGCRMKFLNVVLVVAAVMAAAAAVAFGAEKLPAGVLSLERIFPLNGKMELEEVRARDRARHAR
MLQSFAGGIVNFPVVGSSDPYLVGLYFTKVRLGTPPREYNVQIDTGSDILWVTCSSCDDCPRTS
GLGVELNFYDATISSTASPISCADQVCASIVQTASAECSTETNQCGYSFQYGDGSGTTGHYVAD
LLYFDTVLGTSLIANSSAPIIFGCSTSQSGDLTKTDRAIDGIFGFGQQGLSVISQLSSHRITPKVFS
HCLKGEGNGGGILVLGEILDPRIVYSPLVPSQAHYNVYLQSIAVNGQLVPVDPSVFATSGNRGTI
VDSGTTLAYIATEAYDPFVNAITAAVSPSVRPIISRGKPCFLVSSSIAEIFPPVSLNFDGGASMALR
PSDYLVHMGFVEGAAMWCIGFEKQDQGVTILGDLVLKDKIFVYDLARQRIGWADYDCSSSVNVS
ITSGKDEFINAGQLSVNRASGSLLFNPRHTRTIFHLLSLVLMIGSPFLT
SEQ 112 
MTRASIILLLLLIATSIAAAQGGALTFDDDNPIRQVVVSDGLQELENGILQLIGQTRRALSFVRFVR
RYGKRYDSVEEIKQRFEIYLDNLKMIRSHNKQRLSYKLGVNEFTDLTWDEFRRERLGAPQNCSA
TTKSDLQLTNVNLPETKDWREAGIVSPVKKQGKCGSCWTFSTTGALEAAYAQAFGKNISLSEQQ
LLDCAGAFNNFGCHGGLPSQAFEYIKYSGGLDTEEEYPYAGKAGVCKFSSENVAVKVVDSVNIT
KGAEDELKYAIAFIRPVSVAYQVVKGFKQYKGGIYSSTVCGNTPQDVNHAVLAVGYGVDNGTPY
WLIKNSWGAEWGDNGYFKMEMGKNMCGIATCASYPIVA
SEQ 113 
MNPEKFTHKTNEALAEAHELAISAGHAQFTPLHMALALISDHNGIFRQAIVNAAGSEETANSVER
VFKQAMKKIPSQTPAPDQIPPSTSLIKVLRRAQSLQKSRRDTHLAVDQLILGLLEDSQIGDLLKEA
GIGAARVKSEVEKLRGKDGKKVESASGDTNFQALKTYGRDLVEQAGKLDPVIGRDEEIRRVIRIL
SRRTKNNPVLIGEPGVGKTAVVEGLAQRIVRGDVPSNLSDVRLIALDMGALIAGAKYRGEFEERL
KAVLKEVEEAEGKVILFIDEIHLVLGAGRTEGSMDAANLFKPMLARGQLRCIGATTLEEYRKYVEK
DAAFERRFQQVYVAEPSVPDTISILRGLKEKYEGHHGVKIQDRALVVAAQLSARYITGRHLPDKAI
DLVDEACANVRVQLDSQPEEIDNLERKRIQLEVELHALEKEKDKASKARLVEVRKELDDLRDKLQ
PLTMRYKKEKERIDELRRLKQKRDELTYALQEAERRYDLARAADLRYGAIQEVEAAIANLESSTD
ESTMLTETVGPDQIAEVVSRWTGIPVSRLGQNEKDKLIGLANRLHQRVVGQDDAVRAVAEAVLR
SRAGLGRPQQPTGSFLFLGPTGVGKTELAKALAEQLFDDDKLMVRIDMSEYMEQHSVARLIGAP
PGYVGHEEGGQLTEAVRRRPYSVVLFDEVEKAHPTVFNTLLQVLDDGRLTDGQGRTVDFTNTVI
IMTSNLGAEYLLSGLMGKCTMEKARDMVMQEVRKQFKPELLNRLDEIVVFDPLSHEQLRQVCR
 
HQLKDVASRLAERGIALGVTEAALDVILAQSYDPVYGARPIRRWLEKKVVTELSKMLVKEEIDEN
STVYVDAASSGKDLSYRVEKNGGLVNAATGKKSDILIQLPNGVRSDAAQAVKKMKIEEIVDE
SEQ 114 
MPEAPKKSFFTLSLVPFLPVYTLIRFNPPIESEPLISSSSDECQHDQKQQSDSRNYIVRFYHYKEP
EDHWNYLQNNLKFKGWQWIERKNPAARFPTDFGLVEIDESMKELLLEKFRKMNLVKDVSLDLS
YQRIVLEEKSEKNGAFANGKKRPGKIFTAMSFSEGQNYAVANTSIMRISWSRHLLMQKSRVTSL
FGAHELWSKGHTGAKVKMAIFDTGIRADHPHFRNIKERTNWTNEDTLNDNVGHGTFVAGVIAG
QDEECLGFAPDAEIYAFHVFTDAQVSYTSWFLDAFNYAIATNMDVLNLSIGGPDYLDLPFVEKVW
ELTANNIIMVSAIGNDGPLYGTLNNPADQSDVIGVGAIDQSNHLASFSSRGMSTWEIPHGYGRVK
PDIVAYGREIMGSKISTRCKRLSGTSVASPVVTGIVCLLVSIIPESK
SEQ 115 
MAQMKLSLSLFLSLVLLLAFSPSSFAKVSISSKLASKQAEKLIHELNLFPKESDNIVDRDPFPTAAS
RIVEKRFNFANLTNSSVISFEDLGHHAGYYKIKHSHAARLFYFFFESRGSKDDPVVIWLSGGPGC
SSELALFYENGPFSISNNLSLVRNEYGWDKVSNLIYVDQPTGTGFSYSSDRHDIRHSEAGVSDD
LYDFLQAFFEEHPELVKNDFYITGESYAGHYIPAFAARVHKGNKAKEGIHINLKGFAIGNGLTDPKI
QYAAYTDYALDMGLISKSDHDRINKILPVCEVAINLCGTDGKISCLAAYFVCNSIFSAVRARAGADI
NHYDIRKKCVGALCYDFSNMEKLLNMHSVKQALGVEDIEFVSCSTTVYQAMLVDWMRNLEAGIP
TLLEDGIKLLVYAGEYDLICNWLGNSRWVQAMEWSGQKEFVASPDVPFEVDSSEAGLLKSHGP
LSFLKVHDAGHMVPMDQPKVALEMLKRWIGGTLSQQTTETEDLVASI
SEQ 116 
MAIHTSTLSISILVMLMFSAVTSSAEDMSIISYNEKHHTNGESTVWRTDDEIVSLYESWLVEHKKV
YNALGEKDKRFQIFKDNLRYIDEQNSAPEKSYKLGLTQFADLTNEEYKSIYLGTKPDGRSRLSYT
QSDRYAPKVGDSLPDSVDWRKKGVLVDVKNQGQCGSCWAFSAVASIEAVNKIMTGNLISLSEQ
ELVDCDTADNQGCQGGLMDDAFKFVIQNGGIDTEEDYPYKAKDGKCDQARKNAKVVTIDGYED
VPANDEKALKKAVAGQPVSVAIEAGGKDFQHYKSGIFTGKCGAAVDHGVVAVGYGSENGMDY
WIVRNSWGASWGENGYLRMQRNIGNPKGLCGIATIASYPVKTGQNPPKPAPSPPSPVKPPTQC
DDYNECPAGTTCCCVYKYYNYCFAWGCCPMEGATCCKDHNSCCPHDYPVCNVKAGTCSISKN
NPLGVKAMQHILAKPIGTFGNEGKKTPSS
SEQ 117 
MACNRLHTELGNWQVNPPSGFNLEPSDYLQRWLIEVNGAPGTLYANETYQLQAEFPEHYPIKA
PQVIFLPPAPLHPDIYRDGHICLDILYDSWSPTMTVSSICISILSMLSSSTVKFPSSEMMDVPLILSK
HVFFSKFKADEDESNNANMVFSPVSIQIIFALIAAGSSGSTLDQLLAFLKFNSVEELNSVYSRVITD
VLADGSPMGGPRLSVTNWAWVDQSLSFKHSFKQVMDNVYKAASASVDFRNKGDEVTGEVNK
WAEEKTNGLIKQILPPVAVNSGTSLILANALYFKGAWTEKLNASDTKDHEFHLLNGGSVQAPLMT
SKKRQYVKAFDGFQVLRLRYKQGEDKRFLNMYVYLPNARDGLPTLLEKISSEPGFLDRHVPYEK
VKVHEFLIPKFKISLGIEALEVLKGLELTLPFKGGLTEMVGENYPLAVANVFHKAFIEVNEEGAEAP
AAKAFHKAFIEVNEEAPVAPAVTVATMMFGCSMMKVEEEIDFVADHPFMFLVKDETAGVVLFVG
TLLNPLAVSPS
SEQ 118 
LKVGSFFSSLIYSCNKASPNFYSYSFSLLSCFIELVNMGAKAFLVTILLSSLLFPLALSTSNDGLVRI
GLKKIKFDQNNRLAARVESKEGEAVRASIRKYNNFHGNLGASEDTDIVALKNYMDAQYFGEIGIG
SPPQKFTVIFDTGSSNLWVPSSKCYFSVPCFFHSKYKSSQSSTYKKNGKSAAIRYGTGAISGFFS
QDSVKVGDLIVQNQEFIEATREPSVTFLVAKFDGILGLGFQEISVGNAVPVWYNMVKQGLVKEPV
FSFWLNRNTKEDEGGEIVFGGVDPNHYKGKHTYVPVTRKGYWQFDMGDVLIDGQATGYCDNG
CSAIADSGTSLLAGPTTVITMINHAIGASGVVSQQCKAVVEQYGQTIMDMLLAEAHPKKICSQVG
LCTFDGTRGISMGIESVVDENAGKSSGLHDAMCSACEMAVVWMQNQLRQNQTQERILNYVNEL
CERLPSPMGQSAVDCGKLSGMPSVSFTIGGRTFDLSPEEYILKVGEGPAAQCISGFIALDVPPPR
GPLWILGDVFMGRYHTVFDFGKLRVGFAEAA
SEQ 119 
MSKQNLEAPLLDPSPATFNRRKKWSFALCFLFALTAISFIGLRHHGHVGIWLIGDVERYNGKLQQ
NADVVESEQAVVAADDGRCSEIGISMLKIGGHAVDAAVATALCLGVVNPMASGLGGGGFMVVR
SSSTSEVQAIDMRETAPLAASQNMYDNNGKSKLEGALSMGVPGELAGLHAAWSKHGRLPWKT
LFQPAIKLARDGFVVAPYLAHHIASKAKLILKDPGLRQVIAPEGKLLRAGDICHNVKLSHSLELIAE
QGPEAFYNGEVGEKLVEDVKKAGGILTMDDLRNYKVETPEAVTVNAMGYTIVGMPPPSSGTLGI
SLILKILESYNAAEGSLGLHRLIEAMKHMFAFRMDLGDPDFVNISKTVSDMLSPSFAKAIRQKIFDN
TTFPPEYYMPRWSQLRDHGTSHFCIVDSDRNAVSVTTTVNYPFGAGVLSPSTGIVLNDEMGDF
STPSEISPDELPPAPANFIQPKKRPLSSMAPIIVLKDNQLAGVIGGSGGMKIIPAVVQVFINHFILGM
DPLAAVQSPRVYHELIPNVVLYENWTCIDGDHIELSDEKKHFLEERGHQLEAHNGGAICQLIVQN
LPNSHLKLGRRSGKEYKNGVFHGMLVAVSDPRKDGRPAAI
SEQ 120 
MLKKISSFNILLNMASHITLCIWLLFFFISIISLAKPETYIIHMDLSAMPKAFASHHNWYLTTLASLSD
SSTNHKEFLSSKLVYAYTNAINGFSASLSPSEFEAIKNSPGYVSSIKDMSVKIDTTHTSQFLGLNS
ESGVWPTSDYGKDIIIGLVDTGIWPESKSYSDYGISEVPSRWKGECESGIEFNSSLCNKKIIGARY
FNKGLLANNPNLNISMNSARDTDGHGTHTSSTAAGSYVEGASYFGYATGTAIGIAPKAHVAMYK
ALWEEGVYLSDVLAAIDQAITDGVDVLSLSLGIDAIPLHEDPVAIAAFAALEKGIFVSTSAGNEGPY
YETLHNGTPWVLTVAAGTVDREFIGALTLGNGVSVTGLSLYPGNSSSSESSIVYVECQDDKELQ
KSAHNIVVCLDKNDSVSEHVYNVRNSKVAGAVFITNITDLEFYLQSEFPAVFLNLQEGDKVLEYIK
SNSAPKGKLEFRVTHIGAKPAPKVATYSSRGPSPSCPSILKPDLMAPGALILASWPQQSPVTDVT
SGKLFSNFNIISGTSMSCPHASGVAALLKAAHPEWSPAAIRSAMMTTSNAMDNTQSPIRDIGSKN
AAATPLAMGAGHIDPNKALDPGLIYDATPQDYVNLLCALNFTSKQIKTITRSSSYTCSNPSLDLNY
PSFIGFFNGNSSESDPRRIQEFQRTVTNIGDGMSVYTAKLTTMGKFKVNLVPEKLVFKEKYEKLS
YKLRIEGPLVMDDIVVYGSLSWVETEGKYVVRSPIVATSIKVDPLTGHN
SEQ 121 
MEFYQKLATCSHLSLLCFILLHSIQVQGSYFDQEYGKQVLSSAIQDKDWLVSIRRIIHEYPELRFQ
EYNTSALIRTELDKLGIYYEYPFAKTGLVALIGSSSPPVVALRADMDALPLQELVEWEHKSKVTGK
MHGCGHDAHTAMLLGAAKLLNERKDKLNGTVRLVFQPAEEGGAGAYHMINEGALGDAEAIFGM
HVDFKRPTGSIGTSPGPILAAVSFFEAKIEGKGGHAAEPHATVDPILAASFAVVALQQLISREVDP
LHSQVLSVTYVRGGSASNVIPPYVEFGGTLRSLTTEGLLQLQKRVKEVIEGQAAVHRCKAYIDMK
EEDFPAYPACINDERLHQHVGRVGKLLLGSENIKETEKVMAGEDFAFYQELIPGVMFQIGIRNEK
LGSTHAPHSPHFFLDEDVLPIGAALHTAIAEMYLNDYQHPIAV
SEQ 122 
RHYIYGKLTSNMKTFGIPLAAHSRVLTGSYIRSLYLQILTPFLVHTTAQADNLNCDRSATLNCDRS
ATEVCTDSEVSTDMEPGNSIVNGVPESIAEEDTAEPLDMDFEFYLSDDKATFKGSEIVMNEPLQS
TDISGRLNVLVSWSPKMLEQYNTGLFSSLPEVFKSGFFAKRPQESVSLYKCLEAFLKEEPLGPE
DMWYCPACKQHRQATKKLDLWRLPEILVIHLKRFSYNRFLKNKLETYVDFPTHDLDLSSYLAYK
DGKSSYRYMLYAISNHYGSMGGGHYTAFVHQGADRWYDFDDSHVYPISQDKLKTSAAYVLFYR
RVEEI
SEQ 123 
MSRNSLKIHLSIGKIQPGSENKNGSPVYTDSGTCEHLSELRSRVGSNPFFNFRGCVKVRPLGRA
SIRREPPNELVRCGACGQAPPRLYACVTCAAVFCRVHAPSHPVGNASDPSLHSIAVDIDRAELF
CCGCRDQVYDRDFDAAVVLAQTEATVIGSIQDPPPQPENTRKRRRVEYKPWTPDVKEQVLIVG
NSSPLPSQLGNDSTTPEVQWGLRGLNNLGNTCFMNSVLQALLHTPPLRNYFLSDKHNRYFCQR
KNNSVITRSSSDNGNKNSTMLCLACDLDAMFSAVFSGDWTPISPAKFLYSWWKHASNLASYEQ
QDAHEFFISVLDGIHERMQNDKGKALSPGSGDCCIAHRVFSGILRSDVMCTACGFTSTTYDPCID
ISLDLELSQGSSAKMTSKKSHNTHKKEAESGKFSQNGRISTLMGCLDHFTRPEKLGSDQKFFCQ
HCQVRQESLKQMSIRKLPLVSCFHIKRFEHSVIKKMSRKVDHYLQFPFSLDMSPYLSSSILRSRF
GNRIFSFDGDEQDASCESSSEFELFAVITHTGKLDAGHYVTYLRLSNQWYKCDDAWITQVSESI
VRAAQGYMMFYVQKMLYYKASENQVS
SEQ 124 
MATHSSTLTISISLLLLFFFFFSTLSSASDMSILTYDENQHFRTDDEVMSLYESWLLEHGKSYNA
LDEKDKRFQIFKDNLRYIDEQNSVPNKSYKLGLTKFADLTNEEYRSMYLGTKTSDRRRLLKNKSD
 
RYLPKVGDSLPDSVDWREKGVLVGVKDQGSCGSCWAFSAIASVEAVNSIVTGDVISLSEQELVD
CDTSYNDGCNGGLMDYAFDFIIKNGGIDTEEDYPYTGRDGRCDQSRKNAKVVTIDGYEDVPAN
NEKALQKAVANQPVSIAIEAGGHDFQHYVSGIFTGKCGTAVDHGVVAVGYGSENGMDYWIIRNS
WGASWGEKGYLRVQRNVASSKGLCGLAIEPSYPVKTGVNPPKPGPSPPSPIKPPTQCDDYAQC
PEGTTCCCVFEYYNSCFSWGCCPLEGATCCEDHYSCCPHDYPVCNIRAGTCSISKDNPLGVKA
MKHIHAEPIEAFINGGRKSSS
SEQ 125 
MKKLFLVLFSLALVLRLGESFDFHEKELETEEKLWELYERWRSHHTVSRSLDEKDKRFNVFKAN
VHYVHNFNKKDKPYKLKLNKFADMTNHEFRHHYAGSKIKHHRSFLGASRANGTFMYANVEDVP
PSVDWRKKGAVTPVKDQGKCGSCWAFSTVVAVEGINQIKTNELVSLSEQELVDCDTSQNQGC
NGGLMDMAFEFIKKKGGINTEENYPYMAEGGECDIQKRNSPVVSIDGYEDVPPNDEDSLLKAVA
NQPVSVAIQASGSDFQFYSEGVFTGDCGTELDHGVAIVGYGTTLDGTKYWIVRNSWGPEWGEK
GYIRMQREIDAEEGLCGIAMQPSYPIKTSSSNPTGSPATAPKDEL
SEQ 126 
MARPQFTVILAIISLLIHYGVVSGFRLSDVTNGSSVFLPSPADGSRHTTMLLPLFPPKDTSRRAEIS
RRHLQKSPASARMSLHDDLLLNGYYTTHIWIGTPPQKFALIVDTGSTVTYVPCSECKKCGNHQD
PKFQPEMSSTYQSVKCNKACPCDHKRQQCIYERRYAEMSASYGLLGEDIISFGNLSELAPQRAV
FGCEIAETGDLYSQRADGIMGLGRGDLSIVDQLVEKHVISDSFSLCYGGMDFGGGAMVLGGVK
PPADMAFTKSDFGHSPYYNIDLKEIHVAGKPLNLNPRVFGGKHGTILDSGTTYAYLPEAAFAAFK
NAVVKELHSLKQIEGPDPSFKDICFSGAGSNISELSKNFPRVDMVFSDGKKLTLSPENYLFQHFK
VRGAYCLGIFPNGKNPASLLGGIVVRNTLVTYDRENKRIGFWKTNCSELWDRLNLSPPSPPSPS
VSSLDNTNSSAHLSPSSAPSGPPGYNTPVEIKVGLITFYLSLSVNCSELKPRIPELAHFIAQELDV
NVSQVGF
SEQ 127 
MGAKSFLVAFFLSLLLFPLAFCTSNDGLVRIGLKKIKFDQNNRLAARVESKEGEALRASFRKYNN
LRGNLGASEDTDIVALKNYMDAQYFGEIGIGSPPQKFTVIFDTGSSNLWVPSSKCYFSVPCLFHS
KYKSSQSSTYKKNGKSAAIRYGTGAISGFFSQDSVKVGDLVVKNQEFIEATREPSVTFLVAKFDG
ILGLGFQEISVGNAVPVWYNMVKQGLVKEPVFSFWLNRNTEEDEGGEIVFGGVDPNHYKGKHT
YVPVTRKGYWQFDMGDVLIDGQATGYCDNGCSAIADSGTSLLAGPTTVVTMINHAIGASGVVSQ
QCKAVVEQYGQTIMDMLLAEAHPKKICSQVGLCTFDGTRGVSMGIESVVDENAGKSSGLHDAM
CSACEMAVVWMQNQLRQNQTQERILNYVNELCERLPSPMGQSAVDCGKLSGMPSVSFTIGGR
TFDLSPEEYILKVGEGPAAQCISGFIALDVPPPRGPLWILGDVFMGRYHTVFDSGKLRVGFAEAA
SEQ 128 
MVVAFVGIAKSIGQQCLRRSKPYSYSYFSSYVRSSNSKYGLQNWQFQSHRTLILQSASESVKLE
RLSDSDSGILEVKLDRPEARNAIGKDMLRGLQQAFEAVSNERSANVLMICSSVPKVFCAGADLK
ERKTMILSEVQDFVSTLRSTFSFLEGLHIPTIAAIEGIALGGGLEMAMSCDIRICGEDAVLGLPETG
LAVIPGAGGTQRLPRLVGKSIAKDIIFTGRKISGKDAVSIGLVNYCVPAGEARLKTLELARDINQKG
PVALRMAKCAIDKGVELNMESALALEWDCYEQLLDTKDRLEGLAAFAERRKPRYKGE
SEQ 129 
MCSSNSLYINPKPCKHLADYKVKNGMSGYSLIQECFKTTPYGRTTLEISKSELPRCSICSGHEGR
FYMCLICSSVLCCLSPESNHALLHSQCKAGHEISVDMERAELYCSVCCDQVYDPDFDKVVMCK
HIMGFPRTEIGVVESELRLSKRRRLSFGMDLDSKNMKTLFLRRDQKSKSCFPLVLRGLNNLGNT
CFMNSVLQVLLHAPPLRNYFLSDRHNRDICRKMSSDRLCLPCDIDLIFSAVFSGDRTPYSPARFL
YSWWQHSENLATYEQQDAHEFFISVMDRIHDKEGKASLATKDNGDCQCIAHRTFYGLLRSDVT
CTSCGFTSTTHDPCMDISLDLNSCNSSPKDFANKSSKPNESLVGCLDLFTRPEKLGSDQKLYCE
NCQEKQDALKQMSIKKLPLVLSFHIKRFEHSPTRKMSRKIDRHLQFPFSLDMKPYLSSSIVRKRY
GNRIFSFDGDESDISTEFEIFAVVTHSGMLESGHYVTYLRLRNQWYKCDDAWITEVDEEVVRAS
QCYLMYYVQKMLYHKSCEDVSCQPMSLRADTFVPIAGCC
SEQ 130 
MKELHSLREIEGPDPNYKDICFSGAGSDISELSKSFPPIDMVFSNGKKLSLTPENYLFRHSKVRG
AYCLGIFQNGKDPTTLLGGIVVRNTLVTYDRENERIGFWKTNCSELWDRLNLSPSPPPPPPLPSGL
 
DNTNSSANLTPALAPSLPLEHAPGKIKIGLVSFDMSLSVDYSALKPRVPELAHFIAQELEVNVSQV
HLMNFSTEGNDSLIRWAIFPAGSANYMPNATATEIINRLAENRFHLPDTFGSYKLVKWDIEPPPK
RIRWQQNYLVVVFALLVVLIIGLSASLGWLIWRRRQEIPYNPVGSAETHEKELQPLN
SEQ 131 
MVTVSVKWQKEVYPAVEIDTSQPPYVFKAQLYDLTGVPPERQKIMVKGGLLKDDADWSKVGVK
EGQRLMMMGTADEIVKAPEKGPVFAEDLPEEEQVVNVGHSAGLFNLGNTCYMNSTVQCLHSV
PELKSALTEYNQLGRSNDLDHSSHLLTVATRDLFNDLDKNVKPVAPMQFWTVLRKKYPQFGQQ
SNGAFMQQDAEECWTQLLYTLSQSLKSPNSSGSPDIVKALFGIEFDNRIHCAESGEESTETETV
YSLKCHISQEVNHLHEGLKRGLKSELEKASPSLGRSAVYVKDSRINGLPRYLTIQFVRFFWKRES
NQKAKILRKVDYPLSLDVYDFCSEDLRKKLEGPRQVLRDAEGKKAGLKTSEKTSSSTDGDVKMT
EAEESSSGSGEASKTTQEGVLPEKEHHLTGIYDLVAVLTHKGRSADSGHYVAWVKQENGKWV
QFDDDNPIPQREEDIPKLSGGGDWHMAYICMYKARVVPM
SEQ 132 
MEKKKEVIRLERESVIPVLKPRLIMALADLIEHSSDRAEFLKLCKRVEYTIHAWYLLQFEDLMQLYS
LFDPVNGAKKLEQQKLSPEEIDILEQNFLTYLFQIMHKSNFKIASDEEIDVAHSGQYLLNLPITVDE
SKLDKKLLEKYFAEHPHEDLPEFADKYVIFRRGIGIDRTTDYFFMEKVDMIIGRTWAWILRKTRID
RLFSRRSSSRRKKDPKKDDEINSEAEDHDLYVERIRIENMELSARSNQFSLHQVK
SEQ 133 
MELTCSSPLSVNSTISFNPQLRRYGSVYPHKRCQTVFSLFPYCPSSSSHITITTATTAACSTSSST
SSLFGISLSHRPCSSIPRKIKRSLYIVSGVFERFTERSIKAVMFSQKEAKALGKDMVYTQHLLLGLI
AEDRSPGGFLGSRITIDKAREAVRSIWHDDVEDDKEKLASQDSGSATSATDVAFSSSTKRVFEA
AVEYSRTMGHNFIAPEHMAFGLFTVDDGNATRVLKRLGVNVNRLAAEAVSRLQGELAKDGREPI
SFKRSREKSFPGKITIDRSAEKAKAEKNALEQFCVDLTARVSEGLIDPVIGREIEVQRIIEILCRRTK
NNPILLGQAGVGKTAIAEGLAINIAEGNIPAFLMKKRVMSLDIGLLISGAKERGELEGRVTTLIKEVK
KSGNIILFIDEVHILVGAGTVGRGNKGSGLDIANLLKPALGRGELQCIASTTMDEFRLHIEKDKAFA
RRFQPVLINEPSQADAVQILLGLREKYESHHKCIYSLEAINAAVQLSARYIPDRYLPDKAIDLIDEA
GSKSRMQAHKRRKEQQISVLSQSPSDYWQEIRAVQAMHEVILASKLTENDDASRLNDGSELHL
QPASPSTSDEDEPPVVGPEEIAAVASLWTGIPLKQLTVDERMLLVGLDEQLKKRVVGQDEAVAAI
CRAVKRSRTGLKDPNRPISAMLFCGPTGVGKSELAKALAASYFGSESAMLRLDMSEYMERHTV
SKLIGSPPGYVGYGEGGTLTEAIRRKPFTVVLLDEIEKAHPDIFNILLQLFEDGHLTDSQGRRVSF
KNALIVMTSNVGSTAIVKGRQNTIGFLLADDESAASYAGMKAIVMEELKTYFRPELMNRLDEVVV
FRPLEKPQMLQILDLMLQEVRARLVSLEISLEVSEAVMELICQQGFDRNYGARPLRRAVTQMVE
DLLSESFLSGDLKPGDVAIINLDESGNPVVANKSTQSIHLSDANGNPVVTNR
SEQ 134 
MKNIERLANVALLGLSLAPLVVNVDPNVNVIVTACLTVFVGCYRSVKPTPPSETMSNEHAMRFPL
VGSAMLLSLFLLFKFLSKDLVNAVLTCYFFVLGIAALSATLLPAIRRFLPKKWNDDLIIWHFPYFRS
LEIEFTRSQIVAAIPGTIFCVWYAKQKHWLANNVLGLAFCIQGIEMLSLGSFKTGAILLAGLFVYDIF
WVFFTPVMVSVAKSFDAPIKLLFPTADAKRPFSMLGLGDIVIPGIFVALALRFDVSRGKGPQYFKS
AFLGYTFGLALTIFVMNWFQAAQPALLYIVPAVIGFLAVHCIWNGDVKPLLEFDEGKTKGAEEAD
AKESKKVE
SEQ 135 
MAFSSSYFSFIFLILLFIISFVVGEIKPIYLPGTYQSSLEKQHVKSKIPFKVHYFPQILDHFTFLPKSS
KVFKQKYLINDNYWKQGGPIFVYTGNEGNIDWFAANTGFMLDIAPKFHALLVFIEHRFYGDSMPF
GKKSYKSPKTLGYLNSQQALADYAVLIRSLKQNLSSESSPVVVFGGSYGGMLASWFRLKYPHIAI
GAVASSAPILQFDKITPWSSFYDAVSQDFKEVSLNCYRVIKGSWTELDALSKHEEGLTEVSKLFR
TCKGLHSVYSARDWLWEAFVYTAMVNYPTKANFMMPLPAYPVQEMCKIIDGLPKGASKISRAFA
AASLYYNYTKREKCFNLEGGDDAHGLRGWDWQACTEMVMPMTCSNESMFPPSSYSYKEFKE
DCKKKYGVEPRPHWITTEFGGYRIEQVLKRFGSNMIFSNGMQDPWSRGGVLKNISASIVALVTQ
KGAHHVDFRSETKNDPGWLIMQRKQEVAIIQKWLEEYYRDLKQN
SEQ 136 
MSRFSLLLALVVAGGLFASALAGPATFADENPIRQVVSDGLHELENAILQVVGKTRHALSFARFA
HRYGKRYESVEEIKQRFEVFLDNLKMIRSHNKKGLSYKLGVNEFTDLTWDEFRRDRLGAAQNC
SATTKGNLKVTNVVLPETKDWREAGIVSPVKNQGKCGSCWTFSTTGALEAAYSQAFGKGISLSE
QQLVDCAGAFNNFGCNGGLPSQAFEYIKSNGGLDTEEAYPYTGKNGLCKFSSENVGVKVIDSV
NITLGAEDELKYAVALVRPVSIAFEVIKGFKQYKSGVYTSTECGNTPMDVNHAVLAVGYGVENGV
PYWLIKNSWGADWGDNGYFKMEMGKNMCGIATCASYPVVA
SEQ 137 
MEKEHKYSLFLTKLKLFFLVTLSTFHGLSHGFQMDQARTLMSWRRSKMHAQTTTYATNEDETE
NLVFSDEKHVGNMEDDLIKDGLPAQPSNVMFKQYAGYVNVDVKNGRSLFYYFAEASSGNASSK
PLVLWLNGGPGCSSLGFGAMLELGPFGVNPDGKTLYSRRFAWNKVANVMFLESPAGVGFSYS
NTTSDYSKSGDKRTAEDAYRFLVNWFKRFPHYKGRDFYIMGESYAGFYVPELADIIVKRNMLPT
TNFYIQFKGIMIGNGIMNDETDEKGTLDYLWSHALISDETHRGLLQHCKTETETCQHFQNIAEAEL
GNVDPYNIYGPQCSINSKSRSSSPKLKNGYDPCEQQYVQNYLNLPHVQKALHANLTNLPYLWN
PCSNLDWKDTPATMFPIYKRLIASGLRILLYSGDVDAVVSVTSTRYSLSAMNLKVIKPWRPWLDD
TQEVAGYMVVYDGLAFATVRGAGHQVPQFQPRRAFALLNMFFANHS
SEQ 138 
MANSYTSINFFLAPIIFLAILGLQLQSSDGFGTFGFDIHHRYSDPVKGILDLHGLPEKGSVEYYSA
WTQRDRFIKGRRLAEADTANSTPLSFSGGNETFRLSSLGFLHYANVTVGTPGLSFLVALDTGSD
LFWLPCDCSNCVRALETRSGRRINLNIYSPNTSSTGQIVPCNSTLCGQRRRCLSSQNACAYGVA
YLSNNTSSSGVLVEDILHLETDNAQQKSVEAPIALGCGIRQTGAFLSGAAPNGLFGLGLENISVPS
MLASKGLAANSFSMCFGPDGIGRIVFGDKGSPAQGETPLNLDQLHPTYNISLTGITVGNKITDVD
FTAIFDSGTSFTYLNDPAYKVITENFDSQAKQPRIQPDGEIPFEYCYGLSANQTTFEVPDVNLTMK
GGNQLFLFDPIIMLSLQDRSGAYCLAVVKSGDVNIIGQNFMTGYRVVFDREKMVLGWKPSDCYD
SRGSNDKSTTLPVNKRNSTEAPSPSSVVPEATKGNGSGNEPATSFPSVQSSKPAANQAPAHFI
CQLMMALFSLFSYYLIIISS
SEQ 139 
MAIHTSTLSISILVMLMFSVVSSSAAEDMSIISYNEKHHTNGESTVWRTDDEVMSLYESWLVEHK
KVYNALGEKDKRFQIFKDNLRYIDEHNSVPDKSYKLGLTQFADLTNEEYKSIYLGTKPDGRSRLL
NTQSDRYAPKVGDSLPDSVDWRKKGVLVDVKNQGQCGSCWAFSAVASIEAVNKIVTGNLISLS
EQELVDCDTSDNQGCQGGLMDDAFKFVIQNGGIDTEEDYPYKAKDGKCDQARKNARVVTIDGY
EDVPDNDEKALKKAVAGQPVSVAIEAGGKDFQHYKSGIFTGKCGAAVDHGVVAVGYGSENGM
DYWIVRNSWGASWGEKGYLRMQRNIGNPKGLCGIATIASYPVKTGQNPPKPAPSPPPVKPPTQ
CDDYNECPAGTTCCCVYEYYKYCFAWGCCPMEGATCCKDHNSCCPHDYPVCNVKAGTCSISK
NNPLGVKAMQHILAKPIGTFGNEGKKSPSS
SEQ 140 
MEIKILLASLVIWYITCINVYADDMVRIELKRQSLDLSSISDARIYAKDLRGRNRNLAAPNDQIVYLK
NYHDVQYFAEIGIGSPPQRFIVVFDTGSSNLWVPSSRCFFSIACYLRSRYKSRLSNTYTKIGKSSK
IPFGTGSVHGFFSQDNVKVGGAVLKQQVFTEVTREGYLTLLRARFDGVLGLGFDQSTTSRNVTP
VWYNMLLQHMVTKSIFSFWLNRDPTSKIAGEIIFGGMDWTHFRGQHTYVPVAQNGYWEIEIGDL
FIGSNSTGLCKDGCPAIVDTGTSFIAGPTTILTQINHAIGAEGIISLECKKVVSSYGDSIWERLIAGL
QPENVCNRIGLCTNNGSLCSSCEMIVFWIQVEIRKERSKEKAFQYANQLCEKLPNPGGKSFINC
DVFALPHITFTIGDKSFPLSPDQYVIRVDDSQGVHCISGFTTLNAHPRRPLWVLGDAFLRAYHTV
FDFGSSQIGFAESA
SEQ 141 
MASIFALSLFFIIISFCITSITIPVQSDGHETFIIHVSKSDKPRVFATHHHWYSSIIRSVSQHPSKILYT
YSRAAVGFSARLTAAQADQLRRIPGVISVLPDEVRHLHTTHTPTFLGLADSFGLWPNSDYADDVI
IGVLDTGIWPERPSFSDEGLSPVPSSWKGKCATGPDFPETSCNKKIIGAQMFYKGYEASHGPMD
ESKESKSPRDTEGHGTHTASTAAGSVVANASFYQYAKGEARGMAIKARIAAYKICWKNGCFNS
DILAAMDQAVNDGVHVISLSVGANGYAPHYLLDSIAIGAFGASEHGVLVSCSAGNSGPGAYTAV
NIAPWILTVGASTIDREFPADVILGDNRIFGGVSLYSGDPLTDAKLPVVYSGDCGSKYCYPGKLD
HKKVAGKIVLCDRGGNARVEKGSAVKQAGGVGMILLNLADSGEELVADSHLLPATMVGQKAGD
KIRHYVKSDPSPTATIVFRGTVIGKSPAAPRVAAFSSRGPNHLTPEILKPDVIAPGVNILAGWTGS
 
VGPTDLDIDTRRVEFNIISGTSMSCPHASGLAALLKRAHPKWTPAAVKSALMTTAYNLDNSGKVF
TDLATGQESTPFVHGSGHVDPNRALDPGLVYDIETSDYVNFLCSIGYDGDDVAVFVRDSSRVNC
SEQNLATPGDLNYPSFSVVFTGESNGVVKYKRVMKNVGKNTDAVYEVKVNAPSSVEVSVSPAK
LVFSEEKKSLSYEISFKSKSSGDLEMVKGIESAFGSIEWSDGIHNVRSPIAVRWRHYSAASI
SEQ 142 
MPSSLFLTLLLASISLSFSSTLNSNDDEFFLSSTPKFPLTMAEKLIRQLNLFPKHDINKAAATGDSE
QRLFERKLNLSYVGNSGSTVQDLGHHAGYYRLPHTKDARMFYFFFESRSRKNDPVVIWLTGGP
GCSSELAVFYENGPFKIADNMSLVWNDFGWDKVSNLIYVDQPTGTGFSYSSNDDDIRHDERGV
SNDLYDFLQAFFKAHPQYAKNDFYITGESYAGHYIPAFASRVHQGNKNKEGIYVNLKGFAIGNGL
TDPEIQYKAYTDYALDMKLIKKSDYNAIEKSYPKCQLAIKLCGKDGGTACMAAYLVCTSIFNKIMDI
AGDKNYYDVRKRCEGDLCYDFSKMETFLNDQQVKKALGVGDIEFVSCSSEVYQAMQLDWMRN
LEEGIPSLLEDGIKLLVYAGEYDLICNWLGNSRWVHAMKWSGQKAFGKATQVSFAVDGVEKGV
QKNYGPLTFLKVHDAGHMVPMDQPKAALEMLHRWMQDKLSKQGHLAPM
SEQ 143 
MLVISDCYINSCKAFNFVINLPVMGHSHSHSSHSHSHFHSSKSSDDQNMDMGESITTQTDVSFM
LAKHVFSKEVKGDSNLVFSPLSIQIVLGLIAAGSKGPTKDQLLCFLKSKSIDELNSLYSHFVSVVFV
DGSPNGGPRLSVVNGVWIDQTLPFKPSYKKVVDKVYKAASNSVDFQCKAAEVANQVNQWAKM
KTNNLIKEILPHGTVNNMTRLIFANALYFKGVWNDKFNASETKDHKFHLLSGGSIKAPFMTSKNK
QYAVAFDGFKVLGLHYKQGKDMRRFCMYLILPDARDELPALLDKISSEPGFIDHHIPFEKAKMRK
FLIPKFKTTFGFEASKVLKGLGLTLPFSSGGLTEMVDSPLAGRLFVSQIFHKSFIEVNEEGTEAAA
VTASVIMTKSLIIEKEMEFVADHPFLFLIRDESTGAVFFIGSVLNPLAG
SEQ 144 
MLRIGPSLRTARKLLNRNLHFQSPIIAGDVAPVHHRRQELHRFVRRCNYSSTVGNTSASASFFST
LNNSNSSTTSTTPHVERAEENDSLQSNASEVEPVAAVEQRLSSGMVDAYLAIELALDSVVKIFTV
SSSPNYFLPWQNKSQRETTGSGFVIRGKRILTNAHVVADHTFVLVRKHGSPTKYRATVQAVGHE
CDLAILVVESEEFWEGMNSLELGDVPFLQEAVAVVGYPQGGDNISVTKGVVSRVEPTQYVHGA
SQLLAIQIDAAINPGNSGGPAIMGDKVAGVAFQNLSGAENIGYIIPVPVIKHFIAGVEERGEYIGFC
SLGLSCQPTENAQIREYFQMQSKLTGVLVSRINPLSDASRVLKKDDIILSFDGVPIANDGTVPFRN
RERITFDHLVSMKKPNETAELKVLRNGKVHDFKITLHPLQPLVPVHQFDKLPSYFIFAGLVFIPLTQ
PFLHEYGEDWYNASPRRLCERALRELPKKPGEQFIILSQVLMDDINAGYERLAELQVKKVNGVE
VLNLKHLRQLVEDGNQKNVRFDLDDEKVIVLNYESARIATSRILKRHRIPHAMSSDLTDDENAVEL
QSACSS
SEQ 145 
ICREPPNELVRCGACGHAPPRLYACVTCAEVFCRVHAPSHPAGNAADPSLHCIAVDIDRAELFC
CGCRDQVYNSDFDAAVALAQTEATVIGSIQDPPPHPESTRKRRRVEYKPWTPDVKEQVLIVGNS
SPLPSQLGNDSTTPEVQWGLRGLNNLGNTCFMNSVLQALLHTPPLRNYFLSDKHNRYFCQRKN
SSVITRSSSDNGNKNSTMLCLACDLDAMFSAVFSGDRTPISPAKFLYSWWKHASNLASYEQQD
AHEFFISVLDGIHERMQNDKGKALSPGSGDCCIAHRVFSGILRSDVMCTACGFTSTTYDPCIDISL
DLELSQGSSSKMTSKKSHNTHKKEAESGKFSQNGRISSLMGCLDHFTRPEKLGSDQKFFCQHC
QVRQESLKQMSIRKLPLVSCFHIKRFEHSVIKKMSRKVDHYLQFPFSLDMSPYLSSSILRSRFGN
RIFSFDGDEQDASCESSSEFELFAVITHTGKLDAGHYVTYLRLSNQWYKCDDAWITQVSENIVRA
AQGYMMFYVQKMLYYKASEKQVS
SEQ 146 
MEGSPVLGEHAELIGVLSRPLRQRATAAEIQMVIPWEAITSACGSLLKEELQTRRKIHFDNGNLIS
VKNESPSNNIRNGPSNDTREHLLIDPVPPSLIEKAMTSICLITVDDGAWASGVLLNKQGLLLTNAH
LLEPWRFGKTSVNGSGYNTKSDVVLIPSDQSEHPGVEKFDIQRRNKHLIQKELKTPHFLVDNEQ
GSFRVNLAKTGSRIIRVRLDFMDPWVWTNAKVVHVSRGPLDVALLQLELVPDQLCPITADFMCP
SPGSKAYILGHGLFGPRCDFLPSACVGAIAKVVEAKRSLLNQSSLGEHFPAMLETTAAVHPGGS
GGAVVNSEGHMIALVTSNARHGGGTVIPHLNFSIPCAALEPIFKFVEDMQNLSLEYLDKPDEQLS
SVWALTPPLSSKQSPSMLHLPMLPRGDSDGDTKGSKFAKFIADSEAMLKSATQLGKVERLSNKL
VHSKL
SEQ 147 
MLKALTSSCLQNRFHAVTTAFTPQVRRGTDSNTPLLRVLGSLRSSNRRGPYLSRRFFCSDSTD
GSESNSEAAASEAKPAEKGGDADSKASAAIVPTVFKPEDCLTVLALPLPHRPLFPGFYMHIYVKD
PKVLAALLESRKRQAPYAGAFLMKDEQGTDPNVVSASDTEKNIYELKGKDMLNRLHEVGTLAQI
TSIKDDQVILIGHRRIRMTEVVSEEPLTVKVDHLKEQPYNKDDDVIKATSFEVLSTLRDVLKTSSL
WKDHVQTYIQ
SEQ 148 
MERKHLWAALLLLAIACFVFPASSDSLLRISLKKRQLDISSLNVANVARLEDRYGKHVMKDIEKKK
KKKKSDTNSDIVSLKNYLDAQYYGDISIGSPPQNFTVIFDTGSSNLWVPSSRCYFSIACWIHSKYK
ARKSSTYTKKGESCSIHYGSGSISGFLSQDNVQVGDLVVTDQVFIEATRESSVTFIVAKFDGILGL
GFKEIAVGNTTPVWYNMVKQDLVKEPVFSFWLNRDINAKEGGELVFGGVDPKHFKDKHTYVPL
TQKGYWQFKMGDFSIGNQSTGFCEGGCAAIVDSGTSLLAGPTAVVTQVNHAIGAEGVLSMECK
ETISQYGEMIWDLLVSGVTPDQICLQVGLCYLNGAQHLSSNIRSVVEKENEGSSIGEAPLCTACE
MAVIWMQNQLKQKTTKESVLEYVNQLCEKLPSPMGQSVIDCNSISSMPNVTFNIGDKDFVLTPD
QYILKTGEGIATICLSGFVALDVPPPRGPLWILGNVFMGVYHTVFDYGNLQLGFAEAA
SEQ 149 
SRSYYNILLLQYLFLFVMALILGWKILFILLFVIIGMCTSQVTSRNIQALSMLEKHELWMSSHGRTY
KNEAEKEKRLNIFKENVKFIESFNNNGTKKPYKLGINAFADLTAEEFLSYYTTGLKLSNSYSQIQS
SFKYENLSDVPSVMDWRKSGAVTRIKHQGQCGCCWAFSAVAALEGANKLSTNNLISLSEQQLL
DCTTENNGCNGGLMTTAYDFIIQNGGIATESNYPYEEYQDSCKSQEMNSAVKINRYETLPSTES
ALLKAVAKQPVSIGIAVNEDFHLYQNGVYNGNCEGQELNHAVTVIGYGTENDGTKYWLIKNSWG
TSWGENGYMKIARDTGIEGGLCGITTLASYPVL
SEQ 150 
MGLPEVVDVARNYAVMVRIQGPDPKGLKMRKHAFHLYNSGKTTLSASGMLLPSSFVNASVSKQ
IQGESKLHSFGGHFLVLTVASVIEPFVVQQDRGDISKDKPELIPGAQIDILWEGGNTLQNDIKVTN
KEGLNWLPAELLRVVDIPVSSAAVQSLVEGSSSSIEHGWEVGWSLAAYGNSRQSFTNTKRTQV
EKISFPSQTPMMEAQSSLPSVIGTSTTRIALLRVSSNPYEDLPALKVATWSRRGDLLLGMGSPFG
ILSPSHFFNSISVGSIANSYPPSPQNKALLIADIRCLPGMEGSPVLGEHAELIGVLSRPLRQRATAA
EIQMVIPWEAITSACGSLLKEELQTRRKIHFGNGNLISVKKESFSNNIQDGHANDTQEHLQIDPVP
PSLIEKAMTSICLIAVDDGAWASGVLLNKQGLLLTNAHLLEPWRFGKTSVNGSGYNTKSDVVLIP
SDQSEHPGVEKFDIQRRNKHLIQKELKTPHFLVDNEQCSFRVNLANTGSRTIRVRLDFMDPWV
WTNAKVVHVSRGPLDVALLQLELVPDQLCPIIVDFMCPSPGSKAYILGHGLFGPRCDFLPSACV
GAIAKVVEAKRPLLNQSSLGGHFPAMLETTAAVHPGGSGGAVVNSEGHMIALVTSNARHGGGT
VIPHLNFSIPCAALEPIFKFAEDMQNLSLEYLDKPDEQLSSVWALTPPLSSKQSPSMLHLPMLPR
GDSDGDTKGSKFAKFIADSEAMLKSATQLGKVERLSNKLVHSKL
SEQ 151 
MDNPSEDSSDSPQQQPESPVNDDQRVYLVPYRWWKEAQESSPSDGKSVTLYAAAPAPSYGG
PMKIINNIFSPDVAFNLRREEESLSQSQENGEVGVSGRDYALVPGDIWLQALKWHSNSKAAAKN
GKSFSATDEDIADVYPLQLRLSVLRETSSLGVRISKKDNTVECFKRACRIFSVDTEPLRIWDLSGQ
TALFFSDENNKILKDSQKQSEQDMLLELQVYGLSDSVKNKVKKDEMSMQYPNGSSFLMNGTGS
GITSNLTRSSSSSFSGGPCEAGTLGLTGLQNLGNTCFMNSALQCLAHTPKLVDYFLGDYKREIN
HDNPLGMNGEIASAFGDLLKKLWAPGATPVAPRTFKLKLAHFAPQFSGFNQHDSQELLAFLLDG
LHEDLNRVKNKPYVEAKDGDDRPDEEIADEYWNNHLARNDSIIVDVCQGQYRSTLVCPVCKKVS
IMFDPFMYLSLPLPSTSMRSMTVTVIKNGSDIQISAFTITVSKDGRLEDLIRALSTACSLDADETLL
VAEIYNNRIIRYLEEPADSLSLIRDGDRLVAYRLHKGTEEAPLVVFTHQQIDEHYIYGKLTSNMKTF
GIPLAAHSRVLTGSDIRSLYLQILTPFLVHNTAQADNLNCDRSATEACTDSEVITDMEPGNSIVNG
VPESIAEEDTAEPLDMEFQFYLSDDKATFKGSEIVMNEPLQSTDISGRLNVLVSWSPKILEQYNT
GLFSSLPEVFKSGFFAKRPQESVSLYKCLEAFLKEEPLGPEDMWYCPACKQHRQATKKLDLWR
LPEILVIHLKRFSYNRFLKNKLETYVDFPTHDLDLSSYLAYKDGKSSYRYMLYAISNHYGSMGGG
HYTAFVHQGADRWYDFDDSHVYSISQDKLKTSAAYVLFYRRVEEI
SEQ 152 
MASSSRVFVLLLLIIFNFLYISAQKTIKHKPFSMSFPLISTSLSHNSSSKALFLSSFMASNNRRQTQ
NTKTMSRIPSLNYKSTFKYSMALIVTLPIGTPPQNQQMVLDTGSQLSWIQCHKKIPKRPPPTTSF
DPSLSSTFSVLPCTHPLCKPRIPDFTLPTTCDQNRLCHYSYFYADGTLAEGNLVREKITFSRSQS
TPPLILGCATESEDAEGILGMNLGRFSFASQAKVQKFSYCVPIRQGSHAVKPSGTFYLGQNPNS
HTFQYINLLTFPQSQRMPNLDPLAFTVGMVGIKIGGKKLNISGRVFRPNAGGSGQTIIDSGTEYTF
LVEEAYNKVREEIVRLVGPRLKKGYVYGGALDMCFDNRPIEIGRLIGDMTLQFENGVDILINKERM
LDEVEGGIHCVGIGRSESLGIASNIIGNFHQQNLWVEFDMRNRRVGFGKGECSRQV
SEQ 153 
YTIIIFSLNMKIFSIFSLLLLLLLPILASCHEKQVYIVYFGGHKGEKALHEIEENHHSYLMSVKESEEE
ARYSLIYSYKHSINGFAALLTPHEASKLSELEEVVSVYKSEPRKYRLQTTRSWEFSGVEESVQPN
SLNKDNLLLKARYGKDVIIGVLDSGLWPESKSFSDEGLGPIPKSWKGICQSGDAFNSSNCNKKII
GARYYIKGYEQYYGPLNRTLDYLSPRDKDGHGTHTSSTAGGRKVPNVSAIGGFASGTASGGAP
LARLAMYKVCWAIPKEGKEDGNTCFDEDMLAAMDDAIADGVDVISISIGTKEPQPFDQDSIAIGAL
YAVKKNIVVSCSAGNSGPAPSTLSNTAPWIITVGASSVDRAFLSPVILGNGKKFTGQTVTPYKLE
KEMYPLVYAGQVINSNVTKDVAGQCLPGSLSPKKAKGKIVICLRGNGTRVGKGGEVKRAGGIGY
ILGNNKANGAELVADPHFLPATAVDYKSAMQILNYINSTKSPVAYIVPAKTVLHSKPAPYMASFTS
RGPSAVAPDILKPDITAPGLNILAAWSGGSSPTKLDIDDRVVEYNIISGTSMSCPHVGGAAALLKAI
HPTWSSAAIRSALITSAGLRNNVGEQITDASGKPADPFQFGGGHFRPSKAADPGLVYDASYQDY
LLFLCASGIKDLDKSFKCPKKSHLPNNLNYPSLAIPNLNGTVTVSRRLTNVGAPKSVYFASAKPPL
GFSVEISPPVLSFKHVGSKRTFTITVKVRSDMIDSIPKDQYVFGWYSWNDGIHNVRSPIAVKLA
SEQ 154 
MATRRSSSSALTALAASRSRLLSRFRPAVSRLSQNTLLGTGRCPPPNSGFFVAETTAALWPNYN
VLSKSFVHSYSTTAASSGQINNMDYTEMALEGIVGAVEAARTSKQQVVETEHLMKALLEQKDGL
ARRIFTKAGLDNSSVLQETDQFISQQPKVVGDTSGPILGSHLSSLLENAKKHKKEMGDSFVSVEH
MLLSFLSDTRFGQKLFRNLQLTEKALKDAVNAVRGSQRVTDPNPEGKYEALEKYGNDLTELARR
GKLDPVIGRDDEIRRCIQILSRRTKNNPVIIGEPGVGKTAIAEGLAQRIVRGDVPEPLMNRKLMSL
DMGALLAGAKYRGDFEERLKAVLKEVSSSNGQIILFIDEIHTVVGAGATSGAMDAGNLLKPMLGR
GELRCIGATTLNEYRKYIEKDPALERRFQQVYCGQPSVEDAISILRGLRERYELHHGVKISDSALV
SAAVLADRYITERFLPDKAIDLVDEAAAKLKMEITSKPTELDEIDRAVLKLEMEKLSLKNDTDKASK
ERLNKLESDLKSLKAKQKELNEQWEREKDLMTRIRSIKEEIDRVNLEMEAAEREYDLNRAAELKY
GTLISLQRQLGEAEKNLADYRKSGSSLLREEVTDLDITEIVSKWTGIPLSNLQQSERDKLVFLENE
LHKRVVGQDMAVKSVADAIRRSRAGLSDPNRPIASFMFMGPTGVGKTELGKALAAYLFNTENAL
VRIDMSEYMEKHAVSRLVGAPPGYVGYEEGGQLTEVVRRRPYSVVLFDEIEKAHHDVFNILLQL
LDDGRITDSQGRTVSFTNTVVIMTSNIGSHYILETLQNTRDSQEAVYDAMKKQVIELARRTFRPEF
MNRIDEYIVFQPLDLKQVSRIVELQMRRVKDRLKQKKIDLHYTQEAISLLANMGFDPNYGARPVK
RVIQQMVENEVAMGVLRGDFSEEDMIIVDADASPQGKDLLPEKRLLIRRIENGSNMDAMVAND
SEQ 155 
VNVKCFFVSFFFSFSCMSLFFLQGWNFETFCLKTQSFAVTNKNHRPHLHSHHSSFLCFHTSYLL
FFLILYIYIAKTTSRFAKTQQPPQKMSRFTMLVVLVLLLLCLCHLSVATIGSSSNKKSTYIVHVAKS
QMPESFENHKHWYDSSLKSVSDSAEMLYVYNNVVHGFSARLTVQEAESLERQSGILSVLPEMK
YELHTTRTPSFLGLDRSADFFPESNAMSDVIVGVLDTGVWPESKSFDDTGLGPVPDSWKGECE
SGTNFSSSNCNRKLIGARYFSKGYETTLGPVDVSKESKSARDDDGHGTHTATTAAGSIVQGASL
FGYASGTARGMATRARVAVYKVCWIGGCFSSDILAAMDKAIDDNVNVLSLSLGGGNSDYYRDS
VAIGAFAAMEKGILVSCSAGNAGPGPYSLSNVAPWITTVGAGTLDRDFPAYVSLGNGKNFSGVS
LYKGDLSLSKMLPFVYAGNASNTTNGNLCMTGTLIPEKVKGKIVLCDRGINPRVQKGSVVKEAG
GVGMVLANTAANGDELVADAHLLPATTVGQTTGEAIKKYLTSDPNPTATILFEGTKVGIKPSPVV
AAFSSRGPNSITQEILKPDIIAPGVNILAGWTGGVGPTGLAEDTRRVGFNIISGTSMSCPHVSGLA
ALLKGAHPDWSPAAIRSALMTTAYTVYKNGGALQDVSTGKPSTPFDHGAGHVDPVAALNPGLV
YDLRADDYLNFLCALNYTSIQINSIARRNYNCETSKKYSVTDLNYPSFAVVFLEQMTAGSGSSSS
SVKYTRTLTNVGPAGTYKVSTVFSSSNSVKVSVEPETLVFTRVNEQKSYTVTFTAPSTPSTTNVF
GRIEWSDGKHVVGSPVAISWI
SEQ 156 
MLKALTSSCLQNRFHAVTTAFTPQVRRGTDSNTPLLRVLGSLRSSNRRVPYLSRRFFCSDSTDG
SESNSEAAASEAKPAEEGGDADSKASAAMVPTVFKPEDCLTVLALPLPHRPLFPGFYMHIYVKD
PKVLAALLESRKRQAPYAGAFLMKDEQGTDPNVVSASDTEKNIYELKGKDMLNRLHEVGTLAQI
TSIKDDQVILIGHRRIRMAEVVSEEPLTVKVDHLKEQPYNKDDDVIKATSFEVLSTLRDVLKTSSL
WKDHVQTYIQHIGDFNYARLADFGAAISGANKLQCQQVLEELDVHKRLQLTLELVKKEMEISKIQ
ESIARAIEEKISGEQRRYLLNEQLKAIKKELGLETDDKTALSAKFRERLEPNKEKIPVHVMQVIEEE
LTKLQLLEASSSEFNVTRNYLDWLTALPWGNYSDENFDVLRAEQILDEDHYGLTDVKERILEFIA
VGKLRGTSQGKIICLSGPPGVGKTSIGRSIARALNRKFYRFSVGGLSDVAEIKGHRRTYIGAMPG
KMVQCLKSVGTANPLVLIDEIDKLGRGHAGDPASAMLELLDPEQNANFLDHYLDVPIDLSKVLFV
CTANVVEMIPNPLLDRMEVISIAGYITDEKMHIARDYLEKATRETCGIKPEQVEVTNSALLALIENY
CREAGVRNLQKQIEKIYRKIALKLVREDGEIEPQNAEVGEVEAESIHLSDEIKSKEEIQAGAESAN
GSNDDKASENNAEAEAQGAPVNQTQKSANEDACLQDTQETEKATESEASKTVNKVVVDSPNL
ADYVGKPVFHAERIYDQTPVGVVMGLAWTSMGGSTLYIETSLVEQGEGKGALNVTGQLGDVMK
ESAQIAHTVARTILQEKEPDNQFFANSKLHLHVPAGATPKDGPSAGCTMITSLLSLAMKKPVKKD
LAMTGEVTLTGKILPIGGVKEKAIAARRSDVKTIIFPSANRRDFDELAPNVKEGLDVHFVDDYKQIF
DLAF
SEQ 157 
MQFFRRNPSLHRISSRFLNQVVKTSAYSTKKVYNAGQPTAATHPQLMKEGEITPGITSEEYMQR
RKKLLEFLPENSLAIVAAAPIKMMTDVVPYNFRQDADYLYITGCQQPGGVAVLGHDCGLCMFMP
EQSPQDALWQGETAGVDAALQIFKADLAYPINRLPQILSRMIESSSTVFHNVKTRTSSYLELEAY
KKAVSNYKVKDFSVYTHEARFVKSPAELKLMRDSASIACQALVQTMLYSKLFPDEGMLSAKFEY
ECRVRGAQRMAFNPVVGGGPNGSVVHYFRNDQKIEDGNLVLMDVGCELHGYVSDLTRVWPPF
GKFSSVQEELYNLILETNKECVELCRPGTTIREIHHYSVETLRRGFKEIGILKNDRRGRYEMLNPT
NIGHYLGMDVHDCSTIGNDRPLKPGVVITIEPGVYIPSCFDCPERFQGIGFRIEDEVLITESGYEVL
TASIPKEIKHLESLLNNFGSGRGTEIRAALS
SEQ 158 
LLTSHKNHIILLPFLLYKIFISLQKQTLMASSTRVFVLLLLIIFNFLYISAQKTIKHKPFSMSFPLTSTSL
SHNSSSKALFLSSLLASNQRKQAPNTKTVSRIPSLNYKSTFKYSMALIVTLPIGTPPQNQQMVLD
TGSQLSWIQCHKKIPKRPPPTTSFDPSLSSTFSVLPCTHPLCKPRIPDFTLPTTCDQNRLCHYSY
FYADGTLAEGNLVREKITFSRSQSTPPLILGCATESEDAEGILGMNLGRFSFASQAKVQKFSYCV
PIRQGSHAVKPSGTFYLGQNPNSHTFQYINLLTFPQSQRMPNLDPLAFTVGMVGIKIGGKKLNIS
GRVFRPNAGGSGQTIIDSGTEYTFLVEEAYNKVREEIVRLVGPRLKKGYVYGGALDMCFDNRPM
EIGRLIGDMTLQFENGVEILINKERMLDEVEGGIHCVGIGRSESLGIASNIIGNFHQQNLWVEFDM
RNRRVGFGKGECSRQM
SEQ 159 
MAALNFFIIFTSLVLPIASDPLLSTYVVHVDTKAKPSHYLTQDEWYNSVVESVLANKMDSDSTSPR
LFYSYDVVLQGFAARLTDQESEKLNKFPEVIHIFKDQSRIKLDTTRSPNFLGLNTGYGLWPQSNF
GDDVIIGLVDTGIWPESESFKDNGIGPIPTRWKGKCVDGIEFNATSSCNRKLIGARNFVKGVEND
YHHQSARDQNGHGTHTASTAAGTEVNGANVFGFAKGKARGIASKARIAMYKACGSSSCAESDI
LAAIESAIKDGVDILSLSLGYDDAPFYENPVAIATFAAVKRNIFVASSAGNLGPYPFSVHNTAPWV
TTVGAGSLDRDFPVEINLSNNKTFVGSSLYPGRISGKSYSLVYIENCSIMTIDRSKVERKIVVCNT
SKIEALRNGILIQKAGGFGLIQLNLPTEGEGIRAMAYTLPSATLGYKEGIELLSYIKSNANPRAGFV
RRKDTVIGKKVRAPIVASFSSRGPNVVVPEVLKPDLIAPGLNILAAWPGDISPTRLKMDPRRVKFN
INSGTSMACPHIAGVAALVRAVHPDWSPAAIKSALMTTSTAFDNAQLPIIKHEDMELATPISIGAG
HVNPESAIDPGLIYDTDTSDYINLLCSLNYTEKQMKLFTNESNPCSGFTGSPLDLNYPSLSVMFR
PDSYVHVVKKTLTHVAVSKPEVYKVKIVNLNSEKVSLSIEPRKLIFNESLQKQSYVVKFESHYAFN
SSRKIAEQMAFGSILWESEKHNVRSPFAVMWVQQNFNNSRLYK
SEQ 160

MEISKIQESIARAIEEKISGEQRRYLLNEQLKAIKKELGLETDDKTALSAKFRERLEPNKEKIPVHV
MQVIEEELTKLQLLEASSSEFNVTRNYLDWLTALPWGSYSDENFDVLRAEQILDEDHYGLTDVK
ERILEFIAVGKLRGTSQGKIICLSGPPGVGKTSIGRSIARALNRKFYRFSVGGLSDVAEIKGHRRTY
IGAMPGKMVQCLKSVGTANPLVLIDEIDKLGRGHAGDPASAMLELLDPEQNANFLDHYLDVPIDL
SKVLFVCTANVVEMIPNPLLDRMEVISIAGYITDEKVHIARDYLEKATRETCGIKPEQVEVTDSALL

ALIENYCREAGVRNLQKQIEKIYRKIALKLVREDGEIEPQNAEVDEVKAESIHLSDEIKSKEEIQAG
AESANGSNDDEASENNAEAEAQGAENQTQKSANEDTCLQDTQETEKATESEASKTVNKVVVD
SPNLADYVGKPVFHAERIYDQTPVGVVMGLAWTSMGGSTLYIETSLVEQGEGKGALNVTGQLG
DVMKESAQIAHTVARTILLEKEPDNQFFANSKLHLHVPAGATPKDGPSAGCTMITSLLSLAMKKP
VKKDLAMTGEVTLTGKILPIGGVKEKAIAARRSDVKTIIFPSANRRDFDELAPNVKEGLDVHFVDD
YKQIFDLAF

## Claims

1. A mutant tobacco plant cell or a transgenic tobacco plant cell comprising:
(i) a polynucleotide comprising, consisting or consisting essentially of a sequence encoding a functional protease and having at least 96% sequence identity to a protease selected from:
at least one of SEQ ID NO: 1 to 16; or
at least one of SEQ ID NO: 30 to 41; or
at least one of SEQ ID NO: 17 to 22; or
at least one of SEQ ID NO: 42 to 44; or
at least one of SEQ ID NO: 45 to 61; or
at least one of SEQ ID NO: 23 to 29;
or
(ii) (ii) a construct, vector or expression vector comprising the isolated polynucleotide set forth in (i),
and wherein the mutagenesis or transgenesis results in the insertion of one or more mutations into the polynucleotide set forth in (i) and/or into a regulatory region thereof, to create a mutant tobacco plant wherein expression or activity of said protease is up-regulated or down-regulated as compared to a control tobacco plant cell, wherein the control tobacco plant cell has the same genotype as the starting material for the genetic alteration that resulted in the mutant tobacco plant cell or the transgenic tobacco plant cell.

2. The mutant tobacco plant cell or the transgenic tobacco plant cell according to claim 1, wherein the expression or activity of a protease selected from at least one of SEQ ID NO: 30 to 41 is up-regulated or down-regulated in an Oriental type tobacco.

3. The mutant tobacco plant cell or the transgenic tobacco plant cell according to claim 1, wherein the expression or activity of a protease selected from SEQ ID NO: 17 to 22 is up-regulated or down-regulated in a Virginia type tobacco.

4. The mutant tobacco plant cell or the transgenic tobacco plant cell according to claim 1, wherein the expression or activity of a protease selected from at least one of SEQ ID NO: 42 to 44 is up-regulated or down-regulated in a Burley type tobacco.

5. The mutant tobacco plant cell or the transgenic tobacco plant cell according to claim 1, wherein the expression or activity of a protease selected from at least one of SEQ ID NO: 45 to 61 is up-regulated or down-regulated in a Virginia or Oriental type tobacco.

6. The mutant tobacco plant cell or the transgenic tobacco plant cell according to claim 1, wherein the expression or activity of a protease selected from at least one of SEQ ID NO: 23 to 29 is up-regulated or down-regulated in a Burley or Virginia type tobacco.

7. The mutant tobacco plant cell or the transgenic tobacco plant cell according to any preceding claim, wherein said mutation(s) is a heretozygous or homozygous mutation.

8. The mutant tobacco plant cell or the transgenic tobacco plant cell according to any preceding claim, wherein the expression of one or more proteases is increased by about 10% to about 1000%.

9. The mutant tobacco plant cell or the transgenic tobacco plant cell according to claim 8, wherein the expression of one or more proteases is increased by at least 10%, at least 20%, at least 25%, at least 50%, at least 100%, at least 200%, at least 500%, at least 750% or up to 1000%.

10. A mutant tobacco plant or a transgenic tobacco plant or component or part thereof comprising the plant cell according to any preceding claim.

11. Plant material consisting of biomass, stem, flowers or leaves, from the tobacco plant of claim 10.

12. A tobacco product comprising the tobacco plant cell of any of claims 1 to 9, the tobacco plant of claim 10 or the plant material according to claim 11.

13. A method for preparing a mutant tobacco plant with modulated levels of protease as compared to a control tobacco plant, wherein the control tobacco plant has the same genotype as the starting material for the genetic alteration that resulted in the mutant tobacco plant, said method comprising the steps of:
(a) providing a tobacco plant comprising (i) a polynucleotide comprising, consisting or consisting essentially of a sequence encoding a functional protease and having at least 96% sequence identity to a protease selected from:
at least one of SEQ ID NO: 1 to 16; or
at least one of SEQ ID NO: 30 to 41; or
at least one of SEQ ID NO: 17 to 22; or
at least one of SEQ ID NO: 42 to 44; or
at least one of SEQ ID NO: 45 to 61; or
at least one of SEQ ID NO: 23 to 29; or
(b) inserting one or more mutations into said polynucleotide of said tobacco plant to create a mutant tobacco plant.

14. The method according to claim 13, wherein the tobacco plant in step (b) is a mutant tobacco plant, preferably, wherein said mutant tobacco plant comprises one or more mutations in one or more further sequence encoding a functional protease and having at least 96% sequence identity to a protease selected from:
at least one of SEQ ID NO: 1 to 16; or
at least one of SEQ ID NO: 30 to 41; or
at least one of SEQ ID NO: 17 to 22; or
at least one of SEQ ID NO: 42 to 44; or
at least one of SEQ ID NO: 45 to 61; or
at least one of SEQ ID NO: 23 to 29.

15. A method according to claim 13 or claim 14, wherein the genome of a cell of a tobacco plant is modified by a genome editing technology or by genome engineering techniques selected from CRISPR/Cas technology, zinc finger nuclease-mediated mutagenesis, chemical or radiation mutagenesis, homologous recombination, oligonucleotide-directed mutagenesis and meganuclease-mediated mutagenesis.

16. A method for producing cured plant material, preferably cured leaves, or flowers with an altered flavour profile as compared to control plant material comprising the steps of:
(a) providing a tobacco plant according to claim 10 or the plant material according to claim 11;
(b) optionally harvesting the plant material therefrom; and
(c) curing the plant material for a period of time such that the levels of at least one protease are modulated compared to control cured plant material.

17. The use of
(i) a polynucleotide comprising, consisting or consisting essentially of a sequence encoding a functional protease and having at least 96% sequence identity to a protease selected from:
at least one of SEQ ID NO: 1 to 16; or
at least one of SEQ ID NO: 30 to 41; or
at least one of SEQ ID NO: 17 to 22; or
at least one of SEQ ID NO: 42 to 44; or
at least one of SEQ ID NO: 45 to 61; or
at least one of SEQ ID NO: 23 to 29; or
(ii) a construct, vector or expression vector comprising the isolated polynucleotide set forth in (i),
for the up-regulation or down-regulation of the expression or activity of one or more proteases in a mutant tobacco plant or a transgenic tobacco plant during a tobacco curing procedure as compared to a control tobacco plant, wherein the control tobacco plant has the same genotype as the starting material for the genetic alteration that resulted in the mutant tobacco plant or a transgenic tobacco plant,
wherein mutagenesis or transgenesis results in the insertion of one or more mutations into the polynucleotide set forth in (i) and/or into a regulatory region thereof, resulting in either up- or down-regulation, or complete knock-out, of the protease activity encoded thereby, under the curing conditions.

18. Use according to claim 17, wherein the curing procedure is selected from the group consisting of air curing, fire curing, smoke curing and flue curing.

## Patentansprüche

1. Mutante Tabakpflanzenzelle oder transgene Tabakpflanzenzelle, umfassend:
(i) ein Polynukleotid, umfassend, bestehend oder im Wesentlichen bestehend aus einer Sequenz, die eine funktionelle Protease codiert und wenigstens 96 % Sequenzidentität mit einer Protease aufweist, ausgewählt aus:
wenigstens einer der SEQ ID NO: 1 bis 16; oder
wenigstens einer der SEQ ID NO: 30 bis 41; oder
wenigstens einer der SEQ ID NO: 17 bis 22; oder
wenigstens einer der SEQ ID NO: 42 bis 44; oder
wenigstens einer der SEQ ID NO: 45 bis 61; oder
wenigstens einer der SEQ ID NO: 23 bis 29;
oder
(ii) (ii) ein Konstrukt, einen Vektor oder einen Expressionsvektor, das bzw. der das in (i) angeführte isolierte Polynukleotid umfasst,
und wobei die Mutagenese oder Transgenese zur Insertion einer oder mehrerer Mutationen in das in (i) angeführte Polynukleotid und/oder in eine regulatorische Region davon führt, um eine mutante Tabakpflanze zu erzeugen, in der die Expression oder Aktivität der Protease im Vergleich zu einer Kontroll-Tabakpflanzenzelle hoch- oder herunterreguliert ist, wobei die Kontroll-Tabakpflanzenzelle denselben Genotyp aufweist wie das Ausgangsmaterial für die genetische Veränderung, die zu der mutanten Tabakpflanzenzelle oder der transgenen Tabakpflanzenzelle führte.

2. Mutante Tabakpflanzenzelle oder transgene Tabakpflanzenzelle nach Anspruch 1, wobei die Expression oder Aktivität einer Protease, ausgewählt aus wenigstens einer der SEQ ID NO: 30 bis 41, in einem orientalischen Tabak hochreguliert oder herunterreguliert ist.

3. MutanteTabakpflanzenzelle oder transgene Tabakpflanzenzelle nach Anspruch 1, wobei die Expression oder Aktivität einer Protease, ausgewählt aus SEQ ID NO: 17 bis 22, in einem Virginia-Tabak hochreguliert oder herunterreguliert ist.

4. Mutante Tabakpflanzenzelle oder transgene Tabakpflanzenzelle nach Anspruch 1, wobei die Expression oder Aktivität einer Protease, ausgewählt aus wenigstens einer der SEQ ID NO: 42 bis 44, in einem Burley-Tabak hochreguliert oder herunterreguliert ist.

5. Mutante Tabakpflanzenzelle oder transgene Tabakpflanzenzelle nach Anspruch 1, wobei die Expression oder Aktivität einer Protease, ausgewählt aus wenigstens einer der SEQ ID NO: 45 bis 61, in einem Virginia- oder orientalischen Tabak hochreguliert oder herunterreguliert ist.

6. Mutante Tabakpflanzenzelle oder transgene Tabakpflanzenzelle nach Anspruch 1, wobei die Expression oder Aktivität einer Protease, ausgewählt aus wenigstens einer der SEQ ID NO: 23 bis 29, in einem Burley- oder Virginia-Tabak hochreguliert oder herunterreguliert ist.

7. Mutante Tabakpflanzenzelle oder transgene Tabakpflanzenzelle nach einem beliebigen vorhergehenden Anspruch, wobei die Mutation(en) eine heretozygote oder homozygote Mutation ist/sind.

8. Mutante Tabakpflanzenzelle oder transgene Tabakpflanzenzelle nach einem beliebigen vorhergehenden Anspruch, wobei die Expression von einer oder mehreren Proteasen um etwa 10 % bis etwa 1000 % erhöht ist.

9. Mutante Tabakpflanzenzelle oder transgene Tabakpflanzenzelle nach Anspruch 8, wobei die Expression einer oder mehrerer Proteasen um wenigstens 10 %, wenigstens 20 %, wenigstens 25 %, wenigstens 50 %, wenigstens 100 %, wenigstens 200 %, wenigstens 500 %, wenigstens 750 % oder bis zu 1000 % erhöht ist.

10. Mutante Tabakpflanze oder transgene Tabakpflanze oder Bestandteil oder Teil davon, der die Pflanzenzelle nach einem beliebigen vorhergehenden Anspruch umfasst.

11. Pflanzenmaterial, bestehend aus Biomasse, Stängel, Blüten oder Blättern, der Tabakpflanze nach Anspruch 10.

12. Tabakprodukt, umfassend die Tabakpflanzenzelle nach einem der Ansprüche 1 bis 9, die Tabakpflanze nach Anspruch 10 oder das Pflanzenmaterial nach Anspruch 11.

13. Verfahren zur Vorbereitung einer mutierten Tabakpflanze mit modulierten Proteasewerten im Vergleich zu einer Kontroll-Tabakpflanze, wobei die Kontroll-Tabakpflanze den gleichen Genotyp aufweist wie das Ausgangsmaterial für die genetische Veränderung, die zu der mutanten Tabakpflanze führte, das Verfahren umfassend die folgenden Schritte:
(a) Vorsehen einer Tabakpflanze, umfassend (i) ein Polynukleotid, bestehend aus oder im Wesentlichen bestehend aus einer Sequenz, die eine funktionelle Protease codiert und wenigstens 96 % Sequenzidentität mit einer Protease aufweist, ausgewählt aus:
wenigstens einer der SEQ ID NO: 1 bis 16; oder
wenigstens einer der SEQ ID NO: 30 bis 41; oder
wenigstens einer der SEQ ID NO: 17 bis 22; oder
wenigstens einer der SEQ ID NO: 42 bis 44; oder
wenigstens einer der SEQ ID NO: 45 bis 61; oder
wenigstens einer der SEQ ID NO: 23 bis 29; oder
(b) Einfügen einer oder mehrerer Mutationen in das Polynukleotid der Tabakpflanze, um eine mutante Tabakpflanze zu erzeugen.

14. Verfahren nach Anspruch 13, wobei die Tabakpflanze in Schritt (b) eine mutante Tabakpflanze ist, wobei die mutante Tabakpflanze bevorzugt eine oder mehrere Mutationen in einer oder mehreren weiteren Sequenzen aufweist, die eine funktionelle Protease codieren und wenigstens 96 % Sequenzidentität mit einer Protease aufweisen, ausgewählt aus:
wenigstens einer der SEQ ID NO: 1 bis 16; oder
wenigstens einer der SEQ ID NO: 30 bis 41; oder
wenigstens einer der SEQ ID NO: 17 bis 22; oder
wenigstens einer der SEQ ID NO: 42 bis 44; oder
wenigstens einer der SEQ ID NO: 45 bis 61; oder
wenigstens einer der SEQ ID NO: 23 bis 29.

15. Verfahren nach Anspruch 13 oder Anspruch 14, wobei das Genom einer Zelle einer Tabakpflanze durch eine Genom-Editing-Technologie oder durch Genom-Engineering-Techniken modifiziert wird, die ausgewählt sind aus CRISPR/Cas-Technologie, Zinkfinger-Nuklease-vermittelter Mutagenese, chemischer oder Strahlen-Mutagenese, homologer Rekombination, Oligonukleotid-gerichteter Mutagenese und Meganukleasvermittelter Mutagenese.

16. Verfahren zur Herstellung von getrocknetem Pflanzenmaterial, bevorzugt von getrockneten Blättern oder Blüten, mit einem veränderten Geschmacksprofil im Vergleich zu Kontroll-Pflanzenmaterial, umfassend die folgenden Schritte:
(a) Vorsehen einer Tabakpflanze nach Anspruch 10 oder des Pflanzenmaterials nach Anspruch 11;
(b) optionales Ernten des Pflanzenmaterials; und
(c) Trocknen des Pflanzenmaterials über einen bestimmten Zeitraum, sodass der Gehalt an wenigstens einer Protease im Vergleich zu dem getrockneten Kontroll-Pflanzenmaterial moduliert wird.

17. Verwendung
(i) eines Polynukleotids, umfassend, bestehend oder im Wesentlichen bestehend aus einer Sequenz, die eine funktionelle Protease codiert und wenigstens 96 % Sequenzidentität
mit einer Protease aufweist, ausgewählt aus:
wenigstens einer der SEQ ID NO: 1 bis 16; oder
wenigstens einer der SEQ ID NO: 30 bis 41; oder
wenigstens einer der SEQ ID NO: 17 bis 22; oder
wenigstens einer der SEQ ID NO: 42 bis 44; oder
wenigstens einer der SEQ ID NO: 45 bis 61; oder
wenigstens einer der SEQ ID NO: 23 bis 29; oder
(ii)ein Konstrukt, einen Vektor oder einen Expressionsvektor, das bzw. der das in (i) angeführte isolierte Polynukleotid umfasst,
für das Hoch- oder Herunterregulieren der Expression oder Aktivität einer oder mehrerer Proteasen in einer mutanten Tabakpflanze oder einer transgenen Tabakpflanze während eines Tabaktrocknungsverfahrens im Vergleich zu einer Kontroll-Tabakpflanze, wobei die Kontroll-Tabakpflanze den gleichen Genotyp aufweist wie das Ausgangsmaterial für die genetische Veränderung, die zu der mutanten Tabakpflanze oder einer transgenen Tabakpflanze führte,
wobei die Mutagenese oder Transgenese zur Einfügung einer oder mehrerer Mutationen in das in (i) angeführte Polynukleotid und/oder in eine regulatorische Region davon führt, was unter den Trocknungsbedingungen entweder zu einer Hoch- oder Herunterregulierung oder einem vollständigen Knock-out der dadurch codierten Proteaseaktivität führt.

18. Verwendung nach Anspruch 17, wobei das Trocknungsverfahren ausgewählt ist aus der Gruppe bestehend aus Lufttrocknung, Feuertrocknung, Rauchtrocknung und Heißlufttrockung.

## Revendications

1. Cellule de plant de tabac mutante ou cellule de plant de tabac transgénique comprenant :
(i) un polynucléotide comprenant, constitué ou essentiellement constitué d'une séquence codant pour une protéase fonctionnelle et ayant au moins 96 % d'identité de séquence avec une protéase choisie parmi :
au moins l'une de SEQ ID N° : 1 à 16 ; ou
au moins l'une de SEQ ID N° : 30 à 41 ; ou
au moins l'une de SEQ ID N° : 17 à 22 ; ou
au moins l'une de SEQ ID N° : 42 à 44 ; ou
au moins l'une de SEQ ID N° : 45 à 61 ; ou
au moins l'une de SEQ ID N° : 23 à 29 ;
ou
(ii) (ii) une construction, un vecteur ou un vecteur d'expression comprenant le polynucléotide isolé indiqué en (i),
et dans laquelle la mutagenèse ou la transgenèse a pour résultat l'insertion d'une ou de plusieurs mutations dans le polynucléotide indiqué en (i) et/ou dans une région régulatrice de celui-ci, pour créer un plant de tabac mutant dans laquelle l'expression ou l'activité de ladite protéase est régulée à la hausse ou régulée à la baisse comparativement à une cellule de plant de tabac témoin, dans laquelle la cellule de plant de tabac témoin a le même génotype que la matière de départ pour la modification génétique qui a abouti à la cellule de plant de tabac mutante ou à la cellule de plant de tabac transgénique.

2. Cellule de plant de tabac mutante ou cellule de plant de tabac transgénique selon la revendication 1, dans laquelle l'expression ou l'activité d'une protéase choisie parmi au moins l'une de SEQ ID N° : 30 à 41 est régulée à la hausse ou régulée à la baisse dans un tabac de type oriental.

3. Cellule de plant de tabac mutante ou cellule de plant de tabac transgénique selon la revendication 1, dans laquelle l'expression ou l'activité d'une protéase choisie parmi le SEQ ID N° : 17 à 22 est régulée à la hausse ou régulée à la baisse dans un tabac de type Virginie.

4. Cellule de plant de tabac mutante ou cellule de plant de tabac transgénique selon la revendication 1, dans laquelle l'expression ou l'activité d'une protéase choisie parmi au moins l'une de SEQ ID N° : 42 à 44 est régulée à la hausse ou régulée à la baisse dans un tabac de type Burley.

5. Cellule de plant de tabac mutante ou cellule de plant de tabac transgénique selon la revendication 1, dans laquelle l'expression ou l'activité d'une protéase choisie parmi au moins l'une de SEQ ID N° : 45 à 61 est régulée à la hausse ou régulée à la baisse dans un tabac de type Virginie ou oriental.

6. Cellule de plant de tabac mutante ou cellule de plant de tabac transgénique selon la revendication 1, dans laquelle l'expression ou l'activité d'une protéase choisie parmi au moins l'une de SEQ ID N° : 23 à 29 est régulée à la hausse ou régulée à la baisse dans un tabac de type Burley ou Virginie.

7. Cellule de plant de tabac mutante ou cellule de plant de tabac transgénique selon l'une quelconque des revendications précédentes, dans laquelle ladite ou lesdites mutation(s) est/sont une mutation hétérozygote ou homozygote.

8. Cellule de plant de tabac mutante ou cellule de plant de tabac transgénique selon l'une quelconque des revendications précédentes, dans laquelle l'expression d'une ou de plusieurs protéases est augmentée d'environ 10 % à environ 1000 %.

9. Cellule de plant de tabac mutante ou cellule de plant de tabac transgénique selon la revendication 8, dans laquelle l'expression d'une ou de plusieurs protéases est augmentée d'au moins 10 %, d'au moins 20 %, d'au moins 25 %, d'au moins 50 %, d'au moins 100 %, d'au moins 200 %, d'au moins 500 %,d' au moins 750 % ou jusqu'à 1000 %.

10. Plant de tabac mutant ou plant de tabac transgénique ou composant ou partie de celui-ci comprenant la cellule de plant selon l'une quelconque des revendications précédentes.

11. Matière végétale constituée de biomasse, de tige, de fleurs ou de feuilles, provenant du plant de tabac de la revendication 10.

12. Produit de tabac comprenant la cellule de plant de tabac de l'une quelconque des revendications 1 à 9, le plant de tabac de la revendication 10 ou la matière végétale de la revendication 11.

13. Procédé de préparation d'un plant de tabac mutant avec des taux modulés de protéase comparativement à un plant de tabac témoin, dans lequel le plant de tabac témoin a le même génotype que la matière de départ pour la modification génétique qui a abouti au plant de tabac mutant, ledit procédé comprenant les étapes consistant à :
(a) fournir un plant de tabac comprenant (i) un polynucléotide comprenant, constitué ou essentiellement constitué d'une séquence codant pour une protéase fonctionnelle et ayant au moins 96 % d'identité de séquence avec une protéase choisie parmi :
au moins l'une de SEQ ID N° : 1 à 16 ; ou
au moins l'une de SEQ ID N° : 30 à 41 ; ou
au moins l'une de SEQ ID N° : 17 à 22 ; ou
au moins l'une de SEQ ID N° : 42 à 44 ; ou
au moins l'une de SEQ ID N° : 45 à 61 ; ou
au moins l'une de SEQ ID N° : 23 à 29 ; ou
(b) insérer une ou plusieurs mutations dans ledit polynucléotide dudit plant de tabac pour créer un plant de tabac mutant.

14. Procédé selon la revendication 13, dans lequel le plant de tabac à l'étape (b) est un plant de tabac mutant, de préférence, dans lequel ledit plant de tabac mutant comprend une ou plusieurs mutations dans une ou plusieurs séquences supplémentaires codant pour une protéase fonctionnelle et ayant au moins 96 % d'identité de séquence avec une protéase choisie parmi :
au moins l'une de SEQ ID N° : 1 à 16 ; ou
au moins l'une de SEQ ID N° : 30 à 41 ; ou
au moins l'une de SEQ ID N° : 17 à 22 ; ou
au moins l'une de SEQ ID N° : 42 à 44 ; ou
au moins l'une de SEQ ID N° : 45 à 61 ; ou
au moins l'une de SEQ ID N° : 23 à 29.

15. Procédé selon la revendication 13 ou la revendication 14, dans lequel le génome d'une cellule d'un plant de tabac est modifié par une technologie d'édition de génome ou par des techniques de génie génomique choisies parmi la technologie CRISPR/Cas, la mutagenèse induite par nucléase en doigt de zinc, la mutagenèse chimique ou par rayonnement, la recombinaison homologue, la mutagenèse dirigée par oligonucléotide et la mutagenèse induite par la méganucléase.

16. Procédé de production d'une matière végétale séchée, de préférence des feuilles ou des fleurs séchées ayant un profil d'arôme modifié comparativement à une matière végétale témoin comprenant les étapes consistant à :
(a) fournir un plant de tabac selon la revendication 10 ou la matière végétale selon la revendication 11 ;
(b) éventuellement récolter la matière végétale à partir de celui-ci ; et
(c) sécher la matière végétale pendant une durée telle que le taux d'au moins une protéase est modulé comparativement à la matière végétale séchée témoin.

17. Utilisation
(i) d'un polynucléotide comprenant, constitué ou essentiellement constitué d'une séquence codant pour une protéase fonctionnelle et ayant au moins 96 % d'identité de séquence avec
une protéase choisie parmi :
au moins l'une de SEQ ID N° : 1 à 16 ; ou
au moins l'une de SEQ ID N° : 30 à 41 ; ou
au moins l'une de SEQ ID N° : 17 à 22 ; ou
au moins l'une de SEQ ID N° : 42 à 44 ; ou
au moins l'une de SEQ ID N° : 45 à 61 ; ou
au moins l'une de SEQ ID N° : 23 à 29 ; ou
(ii)une construction, un vecteur ou un vecteur d'expression comprenant le polynucléotide isolé indiqué en (i),
pour la régulation à la hausse ou la régulation à la baisse de l'expression ou de l'activité d'une ou de plusieurs protéases dans un plant de tabac mutant ou un plant de tabac transgénique pendant une procédure de séchage de tabac comparativement à un plant de tabac témoin, dans laquelle le plant de tabac témoin a le même génotype que la matière de départ pour la modification génétique qui a abouti au plant de tabac mutant ou à un plant de tabac transgénique,
dans laquelle la mutagenèse ou la transgenèse aboutit à l'insertion d'une ou de plusieurs mutations dans le polynucléotide indiqué en (i) et/ou dans une région régulatrice de celui-ci, ayant pour résultat une régulation à la hausse ou à la baisse, ou une inhibition complète, de l'activité de protéase codée par celui-ci, dans les conditions de séchage.

18. Utilisation selon la revendication 17, dans laquelle la procédure de séchage est choisie parmi le groupe constitué par le séchage à l'air, le séchage au feu, le séchage à la fumée et le séchage à l'air chaud.
